# EUROPEAN PATENT SPECIFICATION

(11) **EP 1 781 661 B1**
(45) Date of publication and mention of the grant of the patent: **07.06.2017**
(21) Application number: 05773384.2
(22) Date of filing: 24.08.2005
(51) Int. Cl.: C07D 487/04, C07D 267/14, C07D 265/36, C07D 215/08, C07D 498/14, C07D 513/14, A61K 31/5517, A61K 31/553, C07D 223/16, C07D 417/10, C07D 231/38, C07D 231/42, C07D 267/16, C07D 333/44, A61K 31/55, A61K 31/495, A61K 31/38, A61P 3/00, A61P 23/00, A61P 35/00

(54) **VASOPRESSIN V1A ANTAGONISTS**
VASOPRESSIN V1A ANTAGONISTEN
ANTAGONISTES DE LA V1A VASOPRESSINE

(30) Priority: 24.08.2004 EP 04104062; 24.08.2004 US 603557 P
(43) Date of publication of application: 09.05.2007
(60) Divisional application: 10176764.8
(73) Proprietor: Vantia Limited, Southampton SO16 7NS (GB)
(72) Inventor: ANDRZEJ, Roman, Batt, Southampton SO17 2LJ (GB); BAXTER, Andrew, John, Romsey, Hants SO51 7HU (GB); HEENEY, Celine, Southampton SO14 6TQ (GB); STOCKLEY, Martin, Lee, Southampton, SO16 8FY (GB); ROE, Michael Bryan, Ringwood, Hampshire BH24 1QU (GB); HUDSON, Peter, DK-2300 Copenhagen S (DK); HANDY, Rachel, Chandler's Ford, Hamphshire, SO53 2PD (GB)
(74) Representative: Hallybone, Huw George
(86) International application number: PCT/DK2005/000540
(87) International publication number: WO 2006/021213

(56) References cited:
- EP-A- 0 186 817
- EP-A- 0 620 216
- EP-A- 1 449 844
- WO-A-01/29005
- WO-A-02/00626
- WO-A-02/04403
- WO-A-95/34540
- WO-A-03/016316
- WO-A-2004/037788
- US-A- 3 987 108
- US-A- 5 760 031
- US-A- 5 843 952
- US-A- 5 968 930
- US-B1- 6 583 141
- POCIECHA D ET AL: "NEW MESOGENIC COMPOUNDS HAVING FORK-LIKE OR CYCLIC AMIDE TERMINAL GROUPS" LIQUID CRYSTALS, TAYLOR AND FRANCIS, ABINGDON, GB, vol. 29, no. 5, May 2002 (2002-05), pages 663-667, XP001102373 ISSN: 0267-8292
- DATABASE BEILSTEIN BEILSTEIN INSTITUTE FOR ORGANIC CHEMISTRY, FRANKFURT-MAIN, DE; XP002357863 Database accession no. BRN: 2173418 & DE 24 18 295 A (CIBA-GIGY) 1974
- DATABASE BEILSTEIN BEILSTEIN INSTITUTE FOR ORGANIC CHEMISTRY, FRANKFURT-MAIN, DE; XP002383169 Database accession no. BRN:2886892 & US 3 960 886 A 1 June 1976 (1976-06-01)
- DATABASE BEILSTEIN BEILSTEIN INSTITUTE FOR ORGANIC CHEMISTRY, FRANKFURT-MAIN, DE; XP002383170 Database accession no. BRN:2131233 & ARZNEIMITTEL FORSCHUNG, vol. 14, 1964, page 324,

## Description

### Field of the Invention

The present invention relates to compounds with vasopressin V₁ₐ antagonist activity and to pharmaceutical compositions comprising such compounds. The present invention also relates to the use of vasopressin V₁ₐ antagonists for the treatment of certain physiological disorders, such as Raynaud's disease and dysmenorrhoea (primary dysmenorrhoea and/or secondary dysmenorrhoea).

Priority is claimed from EP patent application number 04104062 and US patent application number 60/603557, both filed 24 August 2004.

### Background

The neurophyseal hormones vasopressin (VP) and oxytocin (OT) are cyclic nonapeptides secreted by the posterior pituitary gland.

Only one OT receptor has so far been well characterised, while three VP receptors are known. These are designated the V₁ₐ, V_{1b} and V₂ receptors.

Vasopressin acts on the blood vessels, where it is a potent vasoconstrictor, and on the kidneys, where it promotes water reuptake leading to an antidiuretic effect.

The V₁ₐ, V_{1b}, and V₂, as well as the OT receptors, are members of the super-family of seven transmembrane receptors known as G-protein coupled receptors. The V₁ₐ receptor mediates phospholipase C activation and intracellular calcium mobilisation. Localisation of the receptors includes blood platelets, blood vessels, hepatocytes, brain and uterus-cervix. Thus a V₁ₐ antagonist may have effects on any or all of these tissues. For example, selective V₁ₐ antagonists have been cited as having clinical utility in dysmenorrhoea, pre-term labour, hypertension, Raynaud's disease, brain oedema, motion sickness, hyperlipemia, small cell lung cancer, depression, anxiety, hyponatremia, liver cirrhosis and congestive heart failure.

With respect to dysmenorrhoea it has been proposed that myometrial activity is markedly increased in women with dysmenorrhoea during menstruation. It is proposed that the myometrial ischemia caused by increased uterine contractility might explain the menstrual pain. Furthermore, on the first day of menstruation, higher plasma concentrations of vasopressin have been measured in dysmenorroeic women than in controls.

In healthy women without dysmenorrhoea, intravenous infusion of lysine-vasopressin resulted in decreased uterine blood flow, increased uterine contractility and slight to moderate dysmenorrhoea-like pain, these effects being inhibited by a selective human V₁ₐ preceptor antagonist. (Bossmar, T. et al., BRITISH JOURNAL OF OBSTETRICS AND GYNAECOLOGY (1997 Apr), 104(4), 471-7). Also, it is known that vasopressin contracts human uterine arteries in a dose-dependent and V₁ₐ-mediated fashion.

The above evidence suggests that a V₁ₐ antagonist would be an appropriate and effective treatment for dysmenorrhoea (primary dysmenorrhoea and/or secondary dysmenorrhoea). Further evidence is taken from the clinical study carried out on the selective V₁ₐ antagonist SR49059 ("Effect of SR49059, an orally active V₁ₐ vasopressin receptor antagonist, in the prevention of dysmenorrhea". Brouard, R. et al., BRITISH JOURNAL OF OBSTETRICS AND GYNAECOLOGY (2000), 107(5), 614-619). It was found that there was a dose-related decrease in pain and a dose-related decrease in the amount of additional pain-killer taken compared to patients taking placebo.

The International Patent Application WO 03/016316 A1, published 27 February 2003, discloses a number of compounds which are claimed to be oxytocin agonists and to find use in the treatment of male erectile dysfunction. No V₁ₐ antagonist activity is reported. The European Patent Application EP 1 449 844 A1, published 25 August 2004, discloses a number of compounds which are claimed to be V₁ₐ antagonists and to find use in the treatment of primary dysmenorrhoea.

There exists a need for treatments for conditions which are associated with the V₁ₐ receptors. There further continues to exist a need for alternative V₁ₐ antagonists.
Simple synthesis and oral availability are additional desirable characteristics.

### Disclosure of the Invention

The invention is defined by the appended claims.

According to an aspect, the present invention relates to compounds as defined in the claims, preferably VP antagonists, and in particular specific antagonists of the V₁ₐ receptor, and pharmaceutically acceptable salts thereof.

According to another aspect, the present invention relates to pharmaceutical compositions comprising these compounds, which compositions are useful for the treatment of, inter alia, dysmenorrhoea (primary dysmenorrhoea and/or secondary dysmenorrhoea).

According to another aspect, the present invention relates to the use of the compounds for the manufacture of a pharmaceutical composition for treatment of dysmenorrhoea.

Also disclosed herein is the medical use and the use of the above mentioned compounds and compositions in therapy and to therapeutic methods wherein the above mentioned compounds and compositions are used.

### Detailed Description of the Invention

According to an aspect the invention concerns a compound according to general formula 1a, or a compound which is a tautomer or a pharmaceutically acceptable salt thereof, 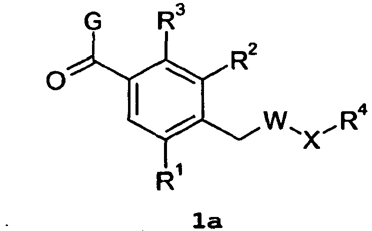 wherein G is a group selected among general formula 3a, 4a, 5a, and 6a. 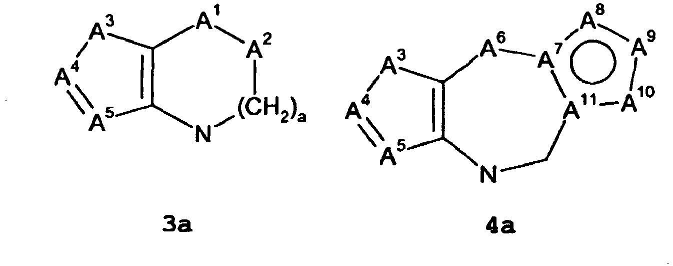 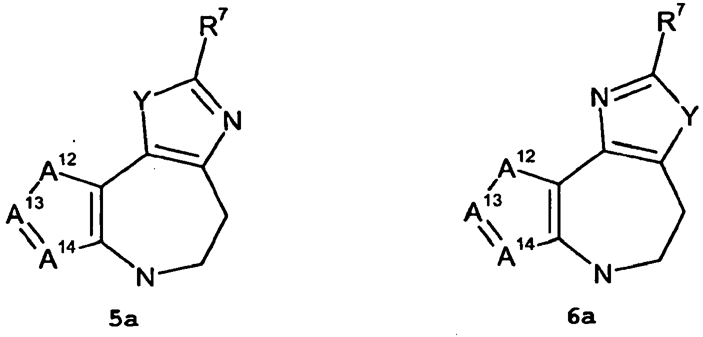 wherein:
A¹ is selected among CH₂, CH(OH), NH, N-alkyl, O and S;
A² is selected among CH₂, CH(OH), C(=O) and NH;
A³ and A¹² are independently selected among S, NH, N-alkyl, -C(R⁸)=CH-, -C(R⁸)=N-, -N=C(R⁸)- and -CH=C(R⁸)-;
A⁴ and A¹³ are independently selected among C(R⁹) and N;
A⁵ and A¹⁴ are independently selected among C(R¹⁰) and N;
A⁶ is selected among CH₂, NH, N-alkyl and O;
A⁷ and A¹¹ are independently selected among C and N;
A⁸ and A⁹ are independently selected among CH, N, NH, N(CH₂)_{b}R¹¹ and S;
A¹⁰ is selected among -CH=CH-, CH, N, NH, N-(CH₂)_{b}-R¹¹ and S; wherein the ring constituted by A⁷, A⁸, A⁹, A¹⁰ and A¹¹ is aromatic;
R¹, R² and R³ are independently selected among H, alkyl, O-alkyl, NO₂, F, Cl and Br;
R⁴ is selected among H, alkyl, aryl, heteroaryl, -(CH₂)_{c}-R¹², and 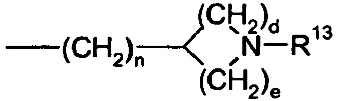
R⁷ is selected among H, alkyl, aryl, heteroaryl, and -(CH₂)_{g}-R¹⁴;
R⁸, R⁹ and R¹⁰ are independently selected among H, alkyl, alkoxy, F, Cl, Br, CN, NH₂, NO₂, NH(alkyl), N(alkyl)₂; with the provisos that when G is **3a,** and R⁴ is H, alkyl, aryl, heteroaryl or - (CH₂)_{c}-R¹², then the ring containing A³, A⁴ and A⁵ is substituted in at least one position by alkyl, alkoxy, F, Cl, Br, CN, NH₂, NO₂, NH(alkyl) or N(alkyl)₂; and
when G is **4a,** and R⁴ is H, alkyl, aryl, heteroaryl or - (CH₂)_{c}-R¹², and A⁸ is NH, NCH₃ or S, then the ring containing A³, A⁴ and A⁵ is substituted in at least one position by alkyl, alkoxy, F, Cl, Br, CN, NH₂, NO₂, NH(alkyl) or N(alkyl)₂;
R¹¹ and R¹² are independently selected among H, alkyl, aryl, heteroaryl, F, OH, O-alkyl, S-alkyl, O-acyl, NH₂, NH-alkyl, N(alkyl)₂, NH-acyl, N(alkyl)-acyl, CO₂H, CO₂-alky CONH₂, CONH-alkyl, CON(alkyl)₂, CN and CF₃;
R¹³ is selected among H, alkyl, aryl, heteroaryl, -(CH₂)ₕ-R¹⁵ and Z-R¹⁶;
R¹⁴ and R¹⁵ are independently selected among H, alkyl, alkenyl, aryl, heteroaryl, F, OH, O-alkyl, S-alkyl, O-acyl, NH₂, NH-alkyl, N(alkyl)₂, NH-acyl, N(alkyl)-acyl, CO₂H, CO₂-alky CO-alkyl, CO-aryl, CONH₂, CONH-alkyl, CON(alkyl)₂, alkenyl-CO₂-alky, alkenyl-aryl, CN and CF₃;
R¹⁶ is selected among H, alkyl, alkenyl, aryl, heteroaryl, O-aryl, -(CH₂)ᵢ-R¹⁷, cyclopropyl-aryl and O-(CH₂)ᵢ-R¹⁷;
R¹⁷ is selected among H, alkyl, aryl, heteroaryl, F, OH, O-alkyl, S-alkyl, O-acyl, NH₂, NH-alkyl, N(alkyl)₂, NH-acyl, N(alkyl)-acyl, CO₂H, CO₂-alky, CONH₂, CONH-alkyl, CON(alkyl)₂, CN and CF₃; W is selected among O and NH;
X is selected among (CH₂)ₘ, C(=O) and S(=O)ⱼ
Y is selected among O, S, NH and N-alkyl;
Z is selected among -C(=O), -C(=O)-O and -S(=O)ₖ;
wherein alkyl is selected from saturated hydrocarbon residues including linear groups up to 10 atoms (C₁-C₁₀), branced groups of between 3 and 10 atoms (C-C₁₀), cyclic groups of between 3 and 8 (C₃-C₈) atoms and combinations of linear, branched and cyclic groups;
a is selected among 1 and 2;
b and c are independently selected among 0, 1, 2 and 3;
d and e are independently selected among 1 and 2;
g, h and i are independently selected among 1, 2 and 3;
j and k are independently selected among 1 and 2; and
m and n are independently selected among 0, 1 and 2;
wherein at least one of R¹, R² and R³ is other than hydrogen and wherein the compound according to gerneal formula 1a is not (4-Aminomethyl-3-methylphenyl)-(5*H*,11*H*-benzo[*e*]pyrrolo[1,2-*a*][1,4]diazepin-10-yl)methanone

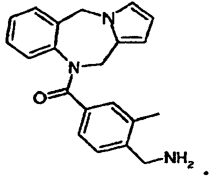

According to an aspect, R¹⁶ may further be selected among O-alkyl and O-alkenyl, and Z is different from -C(=O)-O.

It appears that the compounds of formula **1a** of claim 1 are subject to the following constraints. When G is **3a,** and R⁴ is H, alkyl, aryl, heteroaroaryl or -(CH₂)_{c}-R¹², then the ring containing A³, A⁴ and A⁵ is substituted in at least one position by alkyl, alkoxy, F, Cl, Br, CN, NH₂, NO₂, NH(alkyl) or N(alkyl)₂. When G is **4a,** R⁴ is H, alkyl, aryl, heteroaryl or -(CH2)_{c}-R¹², and A⁸ is NH, NCH₃ or S, then the ring containing A³, A⁴ and A⁵ is substituted in at least one position by alkyl, alkoxy, F, Cl, Br, CN, NH₂, NO₂, NH(alkyl) or N(alkyl)₂. The ring constituted by A7, A8, A9, A10 and A11 is aromatic, and accordingly the groups must satisfy certain requirements. When A10 is -CH=CH- the ring is a six membered ring. As such, it can only comprise atoms of the type -C(R)= and -N=. Hence A7 and A11 must both be C and A8 and A9 must be either CH or N. When A¹⁰ is not -CH=CH- then the ring is a five-membered ring. In this case one, and only one, of the atoms in the ring must be S or a trigonal nitrogen. In this context, a "trigonal nitrogen" is a nitrogen atom linked covalently to three different atoms. Two of these atoms are the immediate neighbours to the nitrogen atom in the five-membered ring. The third is a hydrogen, carbon or other atom linked to the five-membered ring. Thus it follows that, when A10 is not -CH=CH- then one (and only one) of A7, A8, A9, A10 and A11 must be S or a trigonal nitrogen. Hence the selection of A7, A8, A9, A10 and A11 is subject to the following restrictions.

If A10 is not -CH=CH- then one of A8, A9 and A10 is NH, N-(CH2)b-R11 or S or one of A7 and A11 is N. Not more than one of A8, A9 and A10 may be NH, N-(CH2)b-R11 or S. A7 and A11 may not both simultaneously be N. Neither A7 nor A11 may be N if one of A8, A9 and A10 is NH, N-(CH2)b-R11 or S.

According to aspects of the invention, additional and preferred embodiments of the invention are as set out below, in the description and the claims.

The term "alkyl" includes saturated hydrocarbon residues including:
- linear groups up to 10 atoms (C₁-C₁₀). Examples of such alkyl groups include, but are not limited to, C₁ - methyl, C₂ - ethyl, C₃ - propyl and C₄- n-butyl;
- branched groups of between 3 and 10 atoms (C₃-C₁₀). Examples of such alkyl groups include, but are not limited to, C₃ - iso-propyl, C₄ - sec-butyl, C₄ - iso-butyl, C₄ - tert-butyl and C₅ - neo-pentyl;
- cyclic groups of between 3 and 8 atoms (C₃-C₈). Examples of such groups include but are not limited to, C₃ - cyclopropyl, C₄ - cyclobutyl, C₅-cyclopentyl and C₆ - cyclohexyl;
- combinations of linear, branched and cyclic groups. Examples of such groups include, but are not limited to,

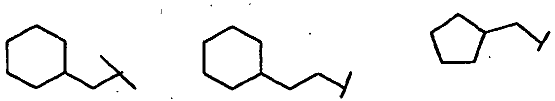

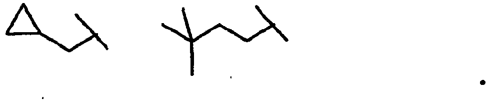

The term "alkoxy" is used to denote O-alkyl groups.

The term "alkenyl" includes monounsaturated hydrocarbon residues including
- linear groups of between two and six atoms (C₂-C₆). Examples of such alkenyl groups include, but are not limited to, C₂ - vinyl, C₃ - 1-propenyl, C₃ - allyl and C₄ - 2-butenyl;
- branched groups of between 3 and 8 atoms (C₃-C₈). Examples of such alkenyl groups include, but are not limited to, C₄ - 2-methyl-2-propenyl and C₆ - 2,3-dimethyl-2-butenyl;
- cyclic groups of between 3 and 8 atoms (C₃-C₈). Examples of such groups include, but are not limited to, C₅ - 3-cyclopentenyl and C₆ - 1-cyclohexenyl.

The term "aryl" includes optionally substituted phenyl and optionally substituted naphthyl. Examples of such aryl groups include, but are not limited to, phenyl, 2-tolyl, 3-chlorophenyl, 4-chlorophenyl, 3-fluorophenyl, 2-methoxyphenyl , 3-methoxyphenyl, 4-methoxyphenyl, 2,5-difluorophenyl, 1-naphthyl and 2-naphthyl.

The term "heteroaryl" includes but is not limited to, pyridyl, 2-chloropyridyl, 4-methylpyridyl, thienyl, 3-chlorothienyl, 2,3-dimethylthiophenyl, furyl, 2-methylfuryl, pyrrole, *N*-methylpyrrole, oxazole, imidazole, pyrazole and triazole.

The term "acyl" denotes a group R-C(=O), where R is H, a saturated or unsaturated hydrocarbon moiety of up to seven carbon atoms or an optionally substituted phenyl, optionally substituted pyridyl or optionally substituted thienyl group. Examples of acyl groups include, but are not limited to: formyl, acetyl, pivaloyl, benzoyl and nicotinoyl.

Certain compounds of the present invention are capable of forming salts with acids or bases. For example, compounds containing one or more basic nitrogen atoms can form addition salts with mineral and organic acids such as hydrochloric acid, sulphuric acid, phosphoric acid, acetic acid, trifluoroacetic acid, methanesulphonic acid, citric acid and benzoic acid. Compounds containing acidic groups can form salts with bases. Examples of such salts include the sodium, potassium, calcium, triethylammonium and tetraethylammonium salts. Furthermore, compounds that have both acidic and basic groups can form internal salts (zwitterions). Insofar as these salts are pharmaceutically acceptable, they are included within the scope of the invention.

Certain compounds within the scope of the present invention may exist as tautomers. For example, where G¹ is general formula **4a (4b, 4c, 4d)** and X² is NH the resulting imidazole can exist as its tautomer which is defined by G¹ as general formula **5a (5b, 5c, 5d)** and X² as NH. All such tautomers are considered to be within the scope of the present invention.

The compounds according to the present invention may have one or more stereogenic centres ("asymmetric carbon atoms") and so may exhibit optical isomerism. The scope of the present invention includes all epimers, enantiomers and diastereomers of compounds according to the present invention, including single isomers, mixtures and racemates.

According to an aspect, it is preferred that G is selected among:

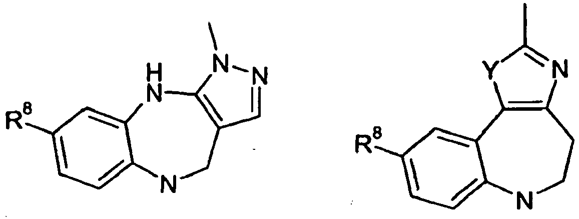

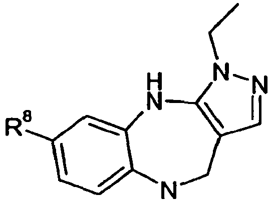

According to an aspect, it is further preferred that G is selected among:

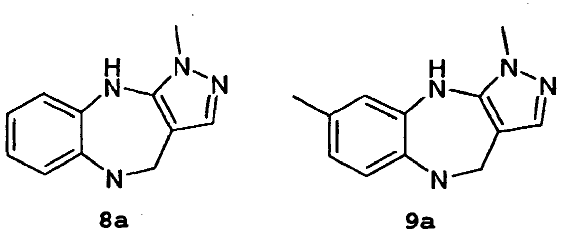

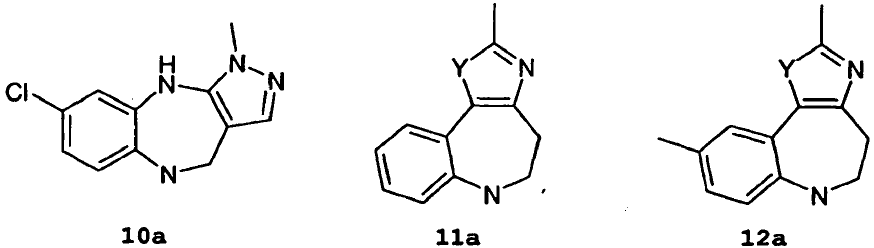

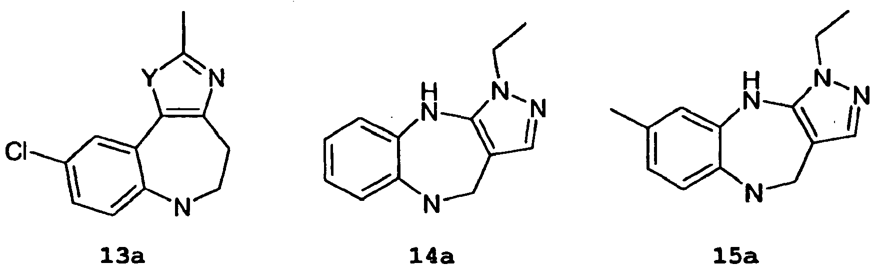

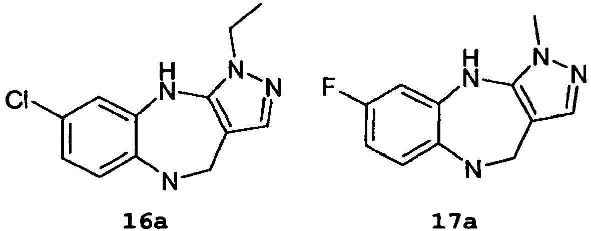

According to an aspect, it is preferred that one of R¹, R² and R³ is selected among methyl, chlorine and fluorine, and the others are hydrogen.

According to different aspects, it is preferred that W is NH; R⁴ is alkyl; d is 2 and e is 2; R¹³ is alkyl; and/or n is 0.

According to an aspect, it is preferred that R² is H, R⁴ is a pipridine where d is 2 and e is 2, W is NH, X is C(=O) and n is 0, as shown in formula **18a:** 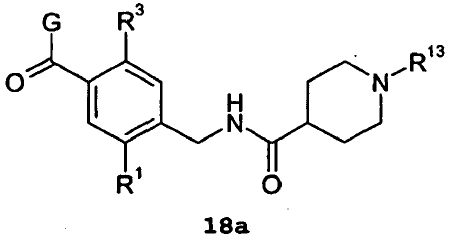 wherein R¹ is selected among methyl, chlorine and fluorine, and R³ is H; or R¹ is H, and R³ is selected among methyl, chlorine and fluorine, and R¹³ is alkyl.

According to an aspect, it is preferred that X is C(=O), n is 0 and R⁴ is a pipridine where d is 2 and e is 2, as shown in formula **19a:** 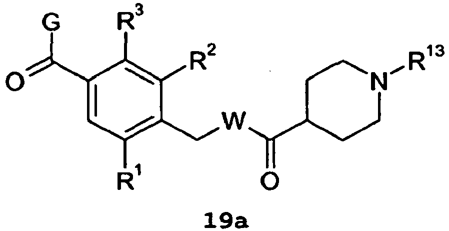 wherein G is selected among general formulae 8a to 17a, and R¹³ is alkyl.

According to an aspect, it is preferred that R² is H and W is NH as shown in formula **20a:** 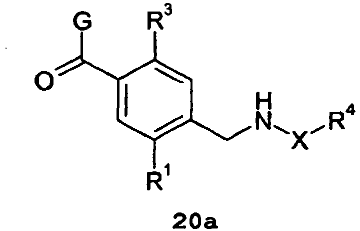 wherein either R¹ is selected among methyl, chlorine and fluorine, and R³ is H; or R¹ is H, and R³ is selected among methyl, chlorine and fluorine; and G is selected from general formulae **8a** to **17a.**

According to an aspect, it is preferred that R² is H, W is NH and X is C(=O) as shown in formula **21a:** 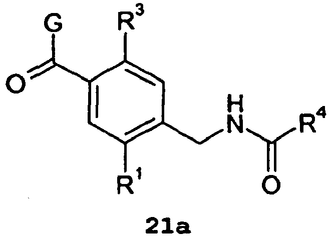 wherein R¹ is selected among methyl, chlorine and fluorine, and R³ is H; or R¹ is H, and R³ is selected among methyl, chlorine and fluorine; and R⁴ is alkyl.

According to an aspect, it is preferred that X is C(=O) as shown in formula **22a:** 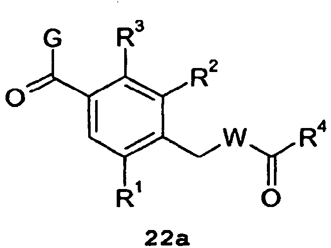 wherein G is selected among general formulae **8a** to **17a,** and R⁴ is alkyl.

According to an aspect, it is preferred that R² and R³ are both H, W is NH and X is C(=O) as shown in formula **23a:** 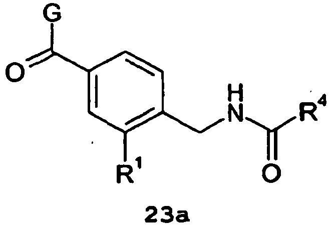 wherein G is selected among general formulae **9a, 10a, 15a, 16a and 17a,** R¹ is selected among methyl, chlorine and fluorine, and R⁴ is alkyl.

According to an aspect, it is preferred that R² and R³ are both H, R⁴ is a pipridine where d is 2 and e is 2, W is NH, X is C(=O) and n is 0 as shown in formula **24a:** 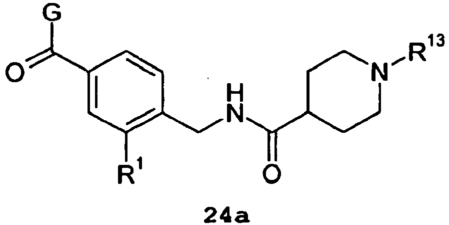 wherein G is selected among general formulae **9a, 10a, 15a, 16a and 17a,** R¹ is selected among methyl, chlorine and fluorine, and R¹³ is alkyl.

According to an aspect, particularly preferred compounds are provided in claim 17.

The particularly preferred compounds of the invention are V₁ₐ antagonists of high activity.

According to an aspect, at least one of the compounds, identified as follows, is not part of the present invetion: claim 1 and ex. 286 (3-Pyridin-3-yl-4,5-dihydro-isoxazole-5-carboxylic acid [4-(4-dimethylamino-benzylcarbamoyl)-phenyl]amide) of US 6 583 141; ex. 5 and 6 of WO 02/04403 (N-[4-(N-Methyl-N-phenylaminocarbonyl)-phenhylmethyl]-3-(4'-trifluoromethylbiphenyl-2-carbonylamino)benzoic acid amide and N-[4-(N-Methyl-N-phenylaminocarbonyl)-phenhylmethyl]-3-(biphenyl-2-carbonylamino)benzoic acid amid); abstract, J. Org. Chem. USSR, vol. 18, no. 6, 1982, p. 1115-1119 (p-(N,N-diethylcarbamoyloxy)-N,N-diethylbenzamide); p. 3, 1. 16 - p. 4, 1. 11, ex. 1-4, 11, and 14-19 of WO 01/29005; p. 3, 1. 9 - p. 5, 1. 23, ex. 11, 28-31, 38, and 71-77 of WO 02/00626; and p. 3, 1. 1 - p. 4, 1. 17, ex. 12-18 of WO 03/016316.

According to an aspect, the invention concerns a pharmaceutical composition comprising a compound according to the invention as an active agent.

The invention is further related to pharmaceutical compositions incorporating a compound according to the invention used as a V₁ₐ antagonist, which compositions are particularly useful for medical indications such as the treatment of dysmenorrhoea (primary and/or secondary dysmenorrhoea), pre-term labour, hypertension, Raynaud's disease, brain oedema, motion sickness, hyperlipemia, small cell lung cancer, depression, anxiety, hyponatremia, liver cirrhosis and congestive heart failure.

Any excipients used will depend on the intended nature of the formulation, which will, in turn, depend on the intended route of administration. Administration may be oral, transmucosal (such as sublingual, buccal, intranasal, vaginal and rectal), transdermal or by injection (such as subcutaneous, intramuscular and intravenous). Oral administration is generally preferred. For oral administration, the formulation may be a tablet, a capsule or a sachet. Other formulations include dry powders, solutions, suspensions, suppositories and the like.

Disclosed herein is the use of a compound of the invention for the manufacture of a medicament for the treatment of a disease selected among dysmenorrhoea (primary and/or secondary dysmenorrhoea), pre-term labour, hypertension, Raynaud's disease, brain oedema, motion sickness, hyperlipemia, small cell lung cancer, depression, anxiety, hyponatremia, liver cirrhosis and congestive heart failure.

According to an aspect, the invention concerns use of a compound of formula lb 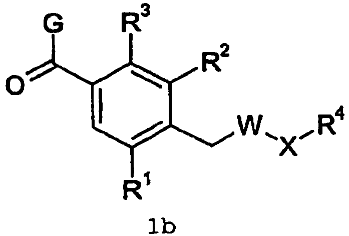 wherein G is a group selected among general formula 2a, 3a, 4a, 5a, and 6a, 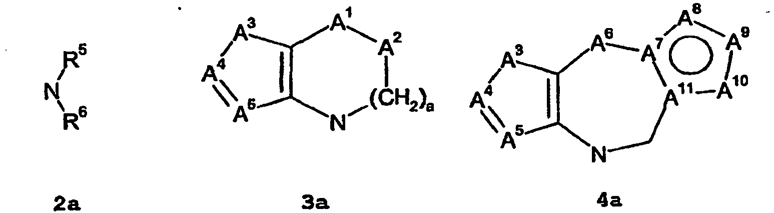 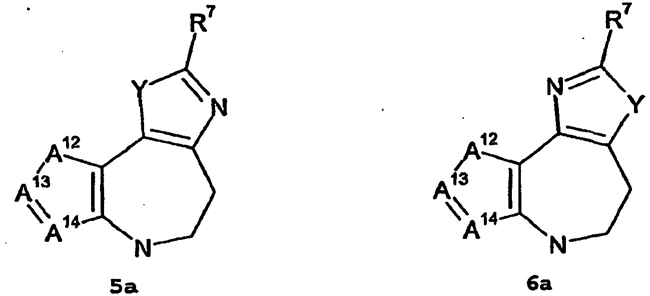 wherein:
A¹ is selected among CH₂, CH(OH), NH, N-alkyl, O and S;
A² is selected among CH₂, CH(OH), C(=O) and NH;
A³ and A¹² are independently selected among S, NH, N-alkyl, - C(R⁸)=CH-, -C(R⁸)=N-, -N=C(R⁸)- and -CH=C(R⁸)-;
A⁴ and A¹³ are independently selected among C(R⁹) and N;
A⁵ and A¹⁴ are independently selected among C(R¹⁰) and N;
A⁶ is selected among CH₂, NH, N-alkyl and O;
A⁷ and A¹¹ are independently selected among C and N;
A⁸ and A⁹ are independently selected among CH, N, NH, N(CH₂)_{b}R¹¹ and S;
A¹⁰ is selected among -CH=CH-, CH, N, NH, N-(CH₂)_{b}-R¹¹ and S;
wherein the ring constituted by A⁷, A⁸, A⁹, A¹⁰ and A¹¹ is aromatic;
R¹, R² and R³ are independently selected among H, alkyl, O-alkyl, NO₂, F, Cl and Br;
R⁴ is selected among H, alkyl, aryl, heteroaryl, -(CH₂)_{c}-R¹², and 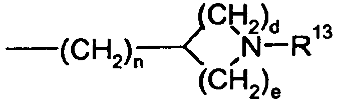
R⁵ and R⁶ are independently selected among alkyl, aryl, -(CH₂)_{f}-aryl, and -(CH₂)_{f}-heteroaryl;
R⁷ is selected among H, alkyl, aryl, heteroaryl, and -(CH₂)_{g}-R¹⁴;
R⁸, R⁹ and R¹⁰ are independently selected among H, alkyl, alkoxy, F, Cl, Br, CN, NH₂, NO₂, NH(alkyl), N(alkyl)₂; with the provisos that when G is **3a,** and R⁴ is H, alkyl, aryl, heteroaryl or -(CH₂)_{c}-R¹², then the ring containing A³, A⁴ and A⁵ is substituted in at least one position by alkyl, alkoxy, F, Cl, Br, CN, NH₂, NO₂, NH(alkyl) or N(alkyl)₂; and
when G is **4a,** and R⁴ is H, alkyl, aryl, heteroaryl or -(CH₂)_{c}-R¹², and A⁸ is NH, NCH₃ or S, then the ring containing A³, A⁴ and A⁵ is substituted in at least one position by alkyl, alkoxy, F, Cl, Br, CN, NH₂, NO₂, NH(alkyl) or N(alkyl)₂;
R¹¹ and R¹² are independently selected among H, alkyl, aryl, heteroaryl, F, OH, O-alkyl, S-alkyl, O-acyl, NH₂, NH-alkyl, N(alkyl)₂, NH-acyl, N(alkyl)-acyl, CO₂H, CO₂-alkyl, CONH₂, CONH-alkyl, CON(alkyl)₂, CN and CF₃;
R¹³ is selected among H, alkyl, aryl, heteroaryl, -(CH₂)ₕ-R¹⁵ and Z-R¹⁶;
R¹⁴ and R¹⁵ are independently selected among H, alkyl, alkenyl, aryl, heteroaryl, F, OH, O-alkyl, S-alkyl, O-acyl, NH₂, NH-alkyl, N(alkyl)₂, NH-acyl, N(alkyl)-acyl, CO₂H, CO₂-alkyl, CO-alkyl, CO-aryl, CONH₂, CONH-alkyl, CON(alkyl)₂, alkenyl-CO₂-alkyl, alkenyl-aryl, CN and CF₃;
R¹⁶ is selected among H, alkyl, alkenyl, aryl, heteroaryl, O-aryl, -(CH₂)ᵢ-R¹⁷, cyclopropyl-aryl and O-(CH₂)**ᵢ**-R¹⁷;
R¹⁷ is selected among H, alkyl, aryl, heteroaryl, F, OH, O-alkyl, S-alkyl, O-acyl, NH₂, NH-alkyl, N(alkyl)₂, NH-acyl, N (alkyl) -acyl, CO₂H, CO₂-alkyl, CONH₂, CONH-alkyl, CON(alkyl)₂, CN and CF₃;
W is selected among O and NH;
X is selected among (CH₂)ₘ, C(=O) and S(=O)ⱼ
Y is selected among O, S, NH and N-alkyl;
Z is selected among -C(=O), -C(=O)-O and -S(=O)ₖ;
a is selected among 1 and 2;
b and c are independently selected among 0, 1, 2 and 3;
d, e and f are independently selected among 1 and 2;
g, h and i are independently selected among 1, 2 and 3;
j and k are independently selected among 1 and 2; and
m and n are independently selected among 0, 1 and 2,
or a compound which is a tautomer or a pharmaceutically acceptable salt thereof, for the manufacture of a medicament for the treatment of a condition selected among dysmenorrhoea, pre-term labour, hypertension, Raynaud's disease, brain oedema, motion sickness, hyperlipemia, small cell lung cancer, depression, anxiety, hyponatremia, liver cirrhosis and congestive heart failure.

The compounds according to the present invention may be useful for treatment of several diseases, disorders or conditions. The term "treatment" used herein relates to both treatment in order to cure or alleviate a disease, disorder or a condition, and to treatment in order to prevent the development of a disease, disorder or a condition. The treatment may either be performed in an acute or in a chronic way. The human or animal to be treated, i.e. the patient, may be any human or non-human mammal in need of treatment according to the invention.

The administration of the compositions of the present invention will generally be under the control of a physician. The physician will determine the amount of composition to be administered and the dosing schedule, taking into account the patient's physical condition and the therapeutic goals.

Methods of treatment of the above mentioned diseases, disorders or conditions are disclosed herein. According to a method disclosured herein, a therapeutically effective amount of the compound, or of the pharmaceutical composition described above, is administered to a patient in need of this treatment. According to different disclosures, it concerns a method of treatment of a disorder selected among dysmenorrhoea (primary and/or secondary dysmenorrhoea), pre-term labour, hypertension, Raynaud's disease, brain oedema, motion sickness, hyperlipemia, small cell lung cancer, depression, anxiety, hyponatremia, liver cirrhosis and congestive heart failure.

The term "therapeutically effective amount" relates to an amount that will lead to the desired therapeutical effect. The therapeutically effective amount will be determined by the attending physician taking into consideration all appropriate factors. Generally a single dose will comprise between 0.1 mg and 1000 mg, preferably between 1 mg and 250 mg, of the active compound according to the invention. The dose may be given on a single occasion or repeatedly. When given repeatedly, it may be given at regular intervals, such as once, twice or three times daily, or on demand, according to the condition being treated.

The pharmaceutical composition according to the present invention may be presented in any form that is known in the art. For example, the formulation may be presented as a tablet, capsule, powder, suppository, cream, solution or suspension, or in a more complex form such as an adhesive patch. The formulation will generally include one or more excipients, such as diluents, bulking agents, binding agents, dispersants, solvents, preservatives, flavouring agents and the like. The formulation may also include one or more additional phar-macologically active species. Preferably the formulation includes no such additional active agents.

When used as therapeutic agents, the compositions of the present invention may be administered by any appropriate route that is known in the art. For example, they may be administered by the oral, buccal, sublingual, rectal, intra-vaginal, nasal, pulmonary or transdermal routes. Alternatively, they may be given by injection, including intravenous, subcutaneous and intramuscular injection.

The compounds of the present invention can be prepared using standard chemical manipulations. These are described in detail in WO 03/016316 A1, pages 12-17. (Hudson, P. J. et al., "Diazacycloalkanes as Oxytocin Agonists").

The following examples are to be considered as enabling and not limiting for the invention, although Examples 4, 8-10, 12-14, 16-24 and 26-87 are reference examples only.

### Examples

The following abbreviations are used:
- AIBN: 2,2'-azobisisobutyronitrile
- Boc: carboxylic acid tert-butyl ester or tert-butoxycarbonylamino
- Bu: butyl - alkyl residues may be further denoted as n (normal, i.e. unbranched), i (iso) and t (tertiary)
- DIEA: *N,N*-diisopropylethylamine
- DMAP: 4-(dimethylamino)pyridine
- DMF: dimethylformamide
- Et: ethyl
- EtOAc: ethyl acetate
- HOBt: 1-hydroxybenzotriazole
- HPLC: High performance liquid chromatography
- H: hour(s)
- Me: methyl
- Min: minute(s)
- MS: mass spectrum
- NMR: nuclear magnetic resonance spectrum - NMR spectra were recorded in CDCl₃ at a frequency of 270MHz unless otherwise indicated
- OVA: ornithine vasotocin analogue
- pet.: petroleum ether boiling in the range 60-80°C
- Ether Ph: phenyl
- Pn: pentyl
- Pr: propyl
- THF: tetrahydrofuran
- . Tos: toluene-4-sulphonyl
- WSCD: water-soluble carbodiimide (*N*-ethyl-*N*'-(3-dimethylaminopropyl)carbodiimide hydrochloride

Examples E1 - E125 describe the synthesis of intermediates and compounds of the present invention. Examples A describes how compounds can be assayed based on their ability to inhibit the cellular consequences of AVP stimulation on intact cells. Example B describes tablets for oral administration comprising a compound according to the invention.

### Example E1

### 6-Chloro-3-methyl-3,4,9,10-tetrahydro-2,3,4,9-tetraaza-benzo[f]azulene

### Example E1.1

### 5-(4-Chloro-2-nitro-phenylamino)-1-methyl-1H-pyrazole-4-carboxylic acid ethyl ester.

Sodium hydride (60% dispersion in oil, 7.28g, 180mmol) was added portionwise to a solution of ethyl 5-amino-1-methylpyrazole-4-carboxylate (21.8g, 148mmol) in anhydrous THF at 0°C. The mixture was allowed to warm to room temperature and stirred for 1h. A solution of 4-chloro-2-fluoronitrobenzene (22.6g, 129mmol) in anhydrous THF (50ml) was added dropwise. The resultant deep purple solution was stirred at room temperature for 18h then poured into ice-cold 1N hydrochloric acid. The resulting mixture was extracted with dichloromethane (200ml x 2), and the combined organic extracts were washed with brine and concentrated *in vacuo*. The residue was purified by flash chromatography on silica gel (eluant; 60% pet ether:40% EtOAc) to yield the title compound (27.5g, 62%).

### Example E1.2

### 5-(2-Amino-4-chloro-phenylamino)-1-methyl-1H-pyrazole-4-carboxylic acid ethyl ester.

Zinc powder (26.17g, 400mmol) was added to a suspension of 5-(4-chloro-2-nitro-phenylamino)-1-methyl-1H-pyrazole-4-carboxylic acid ethyl ester from Example E1.1 (26.0g, 80mmol) in methanol/acetic acid (10:1, 330ml) at room temperature. After the exothermic reaction which followed, the resulting suspension was stirred at room temperature for 18h before being filtered through Celite® filter agent. The filtrate was concentrated *in vacuo.* The residue was dissolved in EtOAc, and the solution was washed with saturated NaHCO₃ and brine, and concentrated *in vacuo.* The residue was purified by flash chromatography on silica gel (eluant; 65% EtOAc:35% pet ether to 80% EtOAc:20% pet ether) to yield the title compound (18.41g, 78.0%).

### Example E1.3

### 6-Chloro-3-methyl-4,9-dihydro-3H-2,3,4,9-tetraaza-benzo[f]azulen-10-one

Sodium (methylsulphinyl)methanide (53.4mmol) was prepared from sodium hydride (60% dispersion in oil, 2.14g, 53.4mmol) and anhydrous dimethyl sulphoxide (35ml) by heating at 65°C until a solution was observed. To this was added a solution of 5-(2-amino-4-chloro-phenylamino)-1-methyl-1H-pyrazole-4-carboxylic acid ethyl ester from Example E1.2 (9.02g, 30.5mmol) in anhydrous dimethyl sulphoxide (20ml), and stirring continued at 65°C for 30min. The mixture was poured into ice (200ml), and the resulting solid collected and purified by recrystallisation from methanol/EtOAc to yield the title compound (5.56g, 73.3%).

### Example E1.4

### 6-Chloro-3-methyl-3,4,9,10-tetrahydro-2,3,4,9-tetraaza-benzo[f]azulene

To a suspension of 6-chloro-3-methyl-4,9-dihydro-3H-2,3,4,9-tetraaza-benzo[f]azulen-10-one from Example E1.3 (5.56g, 22.4 mmol) in anhydrous THF (200ml) at 0°C was added lithium aluminium hydride (4.24g, 112mmol), and the resulting suspension was heated at reflux for 18h, then allowed to cool to room temperature. A further portion of lithium aluminium hydride (4.24g, 112mmol) was added, and the mixture was heated at reflux for 18h. The mixture was cooled to 0°C, 35% ammonia solution (10ml) was added dropwise over 15 min and the mixture was stirred at room temperature for 30min. The resulting suspension was filtered through Celite® filter agent and the filtrate concentrated in *vacuo.* The residue was purified by flash chromatography on silica gel (eluant; 0.5% 35% ammonia:5% metha-nol:dichloromethane) to yield the title compound (3.88g, 74%).

### Example E2

### 3,6-Dimethyl-3,4,9,10-tetrahydro-2,3,4,9-tetraaza-benzo[f]azulene

### Example E2.1

### 1-Methyl-5-(4-methyl-2-nitro-phenylamino)-1H-pyrazole-4-carboxylic acid ethyl ester.

Sodium hydride (60% dispersion in oil, 7.28g, 180mmol) was added portionwise to a solution of ethyl 5-amino-1-methylpyrazole-4-carboxylate (21.8g, 148mmol) in anhydrous THF at 0°C. The mixture was allowed to warm to room temperature and stirred for 1h then a solution of 3-fluoro-4-nitrotoluene (20g, 129mmol) in anhydrous THF (50ml) was added dropwise. The resultant deep purple solution was stirred at room temperature for 18h then poured into ice-cold 1N hydrochloric acid. The resulting mixture was extracted with dichloromethane (200ml x2), and the combined organic extracts were washed with brine and concentrated *in vacuo.* The residue was purified by flash chromatography on silica gel (eluant; 60% pet ether:40% EtOAc) to yield the title compound (28.00g, 61%).

### Example E2.2

### 5-(2-Amino-4-methyl-phenylamino)-1-methyl-1H-pyrazole-4-carboxylic acid ethyl ester

Tin (II) chloride (86.65g, 457.0mmol) was added to a solution of 1-methyl-5-(4-methyl-2-nitro-phenylamino)-1H-pyrazole-4-carboxylic acid ethyl ester from Example E2.1 (28.00g, 91.4mmol) in methanol and the mixture heated at reflux for 3 days. The solvent was removed in vacuo and the residue taken up in EtOAc (4400ml) and cooled to 0°C. 35% Ammonia solution was added to pH 14, and the mixture was stirred for 15min before being filtered through Celite^{®} filter agent. The filtrate was washed with 2M NH₃ and brine and concentrated *in vacuo*. The residue was purified by flash chromatography on silica gel (eluant; 6% methanol:94% dichloromethane rising to 10% methanol:90% dichloromethane) to yield the title compound (12.8g, 52%).

### Example E2.3

### 3,6-Dimethyl-4,9-dihydro-3H-2,3,4,9-tetraaza-benzo[f]azulen-10-one

Sodium (methylsulphinyl)methanide (22.7mmol) was prepared from sodium hydride (60% dispersion in oil, 912mg, 22.7mmol) and anhydrous dimethyl sulphoxide (5.5ml) by heating at 65°C until a solution was observed. To this was added a solution of 5-(2-amino-4-methyl-phenylamino)-1-methyl-1H-pyrazole-4-carboxylic acid ethyl ester from Example E2.2 (3.56g, 13.0mmol) in anhydrous dimethyl sulphoxide (10ml), and stirring continued at 65°C for 30 min. The mixture was then poured into ice (200ml), the resulting solid collected and purified by flash chromatography on silica gel (eluant; 10%methanol:90% dichloromethane) to yield the title compound (1.12g, 38%).

### Example E2.4

### 3,6-Dimethyl-3,4,9,10-tetrahydro-2,3,4,9-tetraaza-benzo[f]azulene

To a suspension of 3,6-dimethyl-4,9-dihydro-3H-2,3,4,9-tetraaza-benzo[f]azulen-10-one from Example E2.3 (2.35g, 10.3mmol) in anhydrous THF (100ml) at 0°C was added lithium aluminium hydride (1.56g, 41.2mmol), and the resulting suspension was heated at reflux for 18h then allowed to cool to room temperature. A further portion of lithium aluminium hydride (781 mg, 20.6mmol) was added, and the mixture was heated at reflux for 3h. The mixture was cooled to 0°C, 35% ammonia solution (10ml) was added dropwise over 15 min and the mixture was stirred at room temperature for 30min. The resulting suspension was filtered through Celite^{®} filter agent and the filtrate concentrated in *vacuo.* The residue was purified by flash chromatography on silica gel (eluant; 10% methanol:90% dichloromethane) to yield the title compound (1.60g, 72%).

Examples E2a-g are described in WO 03/016316 A1, (Hudson, P. et al. "Diazacycloalkanes as Oxytocin Agonists"), pages 26-31.

### Example E2h

### 4-(tert-Butoxycarbonylamino-methyl)-3-methyl-benzoic acid

### Example E2h.1

### 4-Cyano-3-methyl-benzoic acid methyl ester

A mixture of 4-cyano-3-methyl-benzoic acid from Example E2d (1.5g, 9.3mmol) and thionyl chloride (5ml, 68.5mmol) in dichloromethane (20ml) was heated at reflux for 2 h then solvents were removed *in vacuo* and azeotroped with toluene. The residue was redissolved in dichloromethane (25ml). Methanol (10ml) was added and the solution was stirred at room temperature for 2h then concentrated *in vacuo*. The residue was dissolved in EtOAc, washed with saturated NaHCO₃ then brine, dried and concentrated *in vacuo* to yield the title compound (1.6g, 100%).

### Example E2h.2

### 4-Aminomethyl-3-methyl-benzoic acid methyl ester

A solution of 4-cyano-3-methyl-benzoic acid methyl ester
from Example E2h.1 (1.6g, 9.3mmol) in methanol (50ml) to 0°C was treated with cobalt(II) chloride hexahydrate (5.1g, 18.6mmol). The mixture° was stirred for 15min at room temperature then sodium borohydride (3.5g, 93mmol) was added portionwise. The reaction mixture was stirred for 90min then concentrated NH₃ (5ml) was added dropwise. The mixture was warmed up to room temperature over 30min, filtered through Celite^{®} filter agent, washed with methanol and the filtrate was concentrated *in vacuo.* The residue was purified by flash chromatography on silica gel (eluant; 1% 35% ammonia:10% methanol:89% dichloromethane) to yield the title compound (670mg, 37%).

### Example E2h.3

### 4-(tert-Butoxycarbonylamino-methyl)-3-methyl-benzoic acid methyl ester

Di-tert-butyl dicarbonate (900mg, 4.1mmol) was added to a solution of 4-aminomethyl-3-methyl-benzoic acid methyl ester from Example E2h.2 (670mg, 3.7mmol) in dichloromethane (50ml) and triethylamine (to pH9) at room temperature. The mixture was stirred for 1h then concentrated *in vacuo.* The residue was redissolved in EtOAc, washed with 0.3N KHSO₄ then brine, dried and concentrated *in vacuo* to yield the title compound (1.04g, 100%).

### Example E2h.4

### 4-(tert-Butoxycarbonylamino-methyl)-3-methyl-benzoic acid

4-(tert-Butoxycarbonylamino-methyl)-3-methyl-benzoic acid methyl ester from Example E2h.3 (1.04g, 3.7mmol) was dissolved in dioxan (20ml). 1N NaOH solution (5.6ml, 5.6mmol) was added and the mixture was stirred for 18h at room temperature then concentrated *in vacuo.* The residue was redissolved in EtOAc, washed with 1N KHSO₄ then brine, dried and concentrated *in vacuo* to yield the title compound (800mg, 81%).

### Example E3

### 4-(tert-Butoxycarbonylamino-methyl)-3-fluoro-benzoic acid

### Example E3.1

### 3-Fluoro-4-methylbenzoic acid methyl ester

Thionyl chloride (9.62g, 81mmol) was added to a solution of 3-fluoro-4-methylbenzoic acid (5.0 g, 32.4mmol) in toluene (50ml). The mixture was stirred at room temperature for 1.5h and heated at reflux for 3h. The solvent was removed *in vacuo* and the residue was taken up in methanol (30ml) and CH₂Cl₂ (30ml) and stirred for 18h. The mixture was evaporated *in vacuo* and the residue was taken up in EtOAc (50ml), washed with saturated NaHCO₃ solution (3x75ml), dried and evaporated *in vacuo* to yield the title compound (4.5g, 83%).

### Example E3.2

### 4-Bromomethyl-3-fluorobenzoic acid methyl ester

3-Fluoro-4-methylbenzoic acid methyl ester from Example E3.1 (4.5g, 26.6mmol) was dissolved in carbon tetrachloride (150ml). AIBN (457mg, 2.7mmol) and *N-*bromosuccinimide (5.2g, 29.3mmol) were added and the mixture was heated at reflux for 18h. The mixture was allowed to cool and further portions of AIBN (457mg, 2.7mmol) and N-bromosuccinimide (5.2g, 29.3mmol) were added. The mixture was heated at reflux for 56h. The mixture was allowed to cool and evaporated in *vacuo.* The residue was purified by flash chromatography on silica gel (eluant; 10% EtOAc:90% pet ether) to yield the title compound (2.7g, 41%).

### Example E3.3

### 4-Azidomethyl-3-fluorobenzoic acid methyl ester

Sodium azide (609mg) was added to a solution of 4-bromomethyl-3-fluorobenzoic acid methyl ester from Example E3.2 (2.1g, 8.5mmol) in DMF (30ml). The mixture was stirred for 18h, diluted with EtOAc, washed with water and brine and concentrated *in vacuo* to give a colourless oil identified as the title compound (1.8 g, 100%).

### Example E3.4

### 4-Aminomethyl-3-fluorobenzoic acid methyl ester

Hydrogen was passed through a degassed solution of 4-azidomethyl-3-fluorobenzoic acid methyl ester from Example E3.3 (2.11g, 10mmol) in methanol containing 10% palladium on carbon for 2h. The reaction mixture was filtered through Celite® filter agent and the filtrate was concentrated *in vacuo* to give a colourless oil identified as the title compound (1.51 g, 83%).

### Example E3.5

### 9-(tert-Butoxycarbonylamino-methyl)-3-fluorobenzoic acid methyl ester

To a solution of 4-aminomethyl-3-fluorobenzoic acid methyl ester from Example E3.4 (1.5g, 8.2mmol) in dichloromethane (20ml) were added di-tert-butyl dicarbonate (2.3g, 11mmol) and triethylamine (1.4ml, 10mmol). The mixture was stirred for 18h, washed with 0.3M KHSO₄ and brine and concentrated in vacuo. The residue was purified by flash chromatography on silica gel (eluant; 10% EtOAc:90% pet. ether) to give a white solid identified as the title compound (1.4 g, 60%).

### Example E3.6

### 4-(tert-Butoxycarbonylamino-methyl)-3-fluorobenzoic acid

To a solution of 4-(tert-butoxycarbonylamino-methyl)-3-fluorobenzoic acid methyl ester from Example E3.5 (640mg, 2.25mmol) in dioxan (40ml) was added 1N NaOH (4.5ml, 4.5mmol). The mixture was stirred for 18h, diluted with EtOAc, washed with 1N KHSO₄, water and brine and concentrated *in vacuo* to give a white solid identified as the title compound (608 mg, 100%).

### Example E4

### 1-(3,3-Dimethyl-butyl)-piperazine dihydrochloride

### Example E4.1

### 4-(3,3-Dimethyl-butyl)-piperazine-1-carboxylic acid tert-butyl ester

To a solution of 1-boc-piperazine (8.19g, 43.9mmol) in methanol/acetic acid (99:1, v/v, 100ml) was added 3,3-dimethylbutyraldehyde (4.0g, 40.0mmol) and the resulting mixture was stirred at room temperature for 1h. Sodium cyanoborohydride (3.27g, 51.9mmol) was added, and the resulting mixture was stirred at room temperature for 18h then concentrated in vacuo. The residue was dissolved in EtOAc and the resulting solution was washed with saturated NaHCO₃, water and brine, dried and concentrated *in vacuo.* The residue was purified by flash chromatography on silica gel (eluant; 5% methanol: 95% EtOAc) to yield the title compound (9-1g, 84%).

### Example E4.2

### 1-(3,3-Dimethyl-butyl)-piperazine dihydrochloride

A solution of 4-(3,3-dimethyl-butyl)-piperazine-1-carboxylic acid tert-butyl ester from Example E4.1 (9.1g, 31.8mmol) in methanol (100ml) was treated with 4N HCl/dioxan (100ml) and the mixture was stirred at room temperature for 30min then concentrated in *vacuo.* The residue was triturated with diethyl ether and the resulting solid recrystallised from methanol/diethyl ether to yield 1-(3,3-dimethyl-butyl)-piperazine dihydrochloride (7.4g, 90%).

### Example E5

### 2-Methyl-1,4,5,6-tetrahydro-1,3,6-triaza-benzo[e]azulene

### Example E5.1

### 2-(Toluene-4-sulfonylamino)-benzoic acid methyl ester

Methyl anthranilate (110g, 0.73mol) was dissolved in dichloromethane (1 litre) at 0°C and triethylamine (115ml, 0.8mol) was added. Tosyl chloride (133g, 0.745mol) was added portionwise at 0°C. The reaction mixture was stirred for 30min at 0°C and 64h at room temperature. The mixture was reduced *in vacuo.* The residue was dissolved in EtOAc and the organic layer was washed with 5% KHCO₃(aq solution, 1N HCl solution and brine, dried, filtered and concentrated *in vacuo.* The residue was crystallised from EtOAc/hexane to yield the title compound (181g, 81%).

### Example E5.2

### 2-[(3-Ethoxycarbonyl-propyl)-(toluene-4-sulfonyl)-amino]-benzoic acid methyl ester

2-(Toluene-4-sulfonylamino)-benzoic acid methyl ester from Example E5.1 (100g) was dissolved in DMF (250ml). Potassium carbonate (125g) and ethyl 4-bromobutanoate (60g) were added and the mixture was heated at 80°C for 18h. The mixture was cooled to room temperature, filtered and reduced in vacuo. The residue was partitioned between chloroform and 1M HCl solution. The aqueous layer was extracted with chloroform. The organic layers were combined, washed with brine, dried, filtered and concentrated in *vacuo.* the material was crystallised from EtOAc/hexane and dried in a vacuum oven at 60°C for 3 hours to yield the title compound (98.6g, 72%).

### Example E5.3

### 5-Hydroxy-1-(toluene-4-sulfonyl)-2,3-dihydro-1H-benzo[b]azepine-4-carboxylic acid methyl ester and 5-hydroxy-1-(toluene-4-sulfonyl)-2,3-dihydro-1H-benzo[b]azepine-4-carboxylic acid ethyl ester

2-[3-Ethoxycarbonyl-propyl)-(toluene-4-sulfonyl)-amino]-benzoic acid methyl ester from Example E5.2 (98.6g, 0.235mol) was taken up in warm toluene (600ml) and was added dropwise to a mixture of potassium *tert-*butoxide (40g) in toluene (1litre) refluxing under Dean-Stark conditions. The mixture was heated at reflux under Dean-Stark condidtions for 1h further and cooled to room temperature. It was diluted with EtOAc (500ml) and washed with 1M HCl solution, saturated NaHCO₃(aq) and brine. The organic layer was dried, filtered and reduced *in vacuo.* The residue was precipitated from EtOAc/hexane and dried in a vacuum oven to yield a mixture of 5-hydroxy-1-(toluene-4-sulfonyl)-2,3-dihydro-1H-benzo[b]azepine-4-carboxylic acid methyl ester and 5-hydroxy-1-(toluene-4-sulfonyl)-2,3-dihydro-1H-benzo[b]azepine-4-carboxylic acid ethyl ester (53.5g).

### Example E5.4

### 1-(Toluene-4-sulfonyl)-1,2,3,4-tetrahydro-benzo[b]azepin-5-one

A mixture of 5-hydroxy-1-(toluene-4-sulfonyl)-2,3-dihydro-1H-benzo[b]azepine-4-carboxylic acid methyl ester and 5-hydroxy-1-(toluene-4-sulfonyl)-2,3-dihydro-1H-benzo[b]azepine-4-carboxylic acid ethyl ester from Example E5.3 were heated at reflux in a mixture of ethanol (100ml), acetic acid (300ml), concentrated HCl (100ml) and water (50ml) for 18h. The mixture was cooled to room temperature, diluted with water (800ml) and extracted with chloroform. The combined organic extracts were dried, faltered and reduced *in vacuo.* The residue was crystallised twice from methanol to yield the title compound (44g, 60% over two steps).

### Example E5.5

### 1,2,3,4-Tetrahydro-benzo[b]azepin-5-one

Polyphosphoric acid (25g) was heated at 100°C under nitrogen until it could be stirred. 1-(Toluene-4-sulfonyl)-1,2,3,4-tetrahydro-benzo[b]azepin-5-one from Example E5.4 (2.6g, 8.26mmol) was added portionwise and the reaction mixture was heated at 100°C for 1.5h. It was poured into ice and basified with 2M NaOH(aq). The aqueous layer was extracted twice with dichloromethane. The organic extracts were combined, washed with brine, dried and reduced *in vacuo.* The residue was purified by flash chromatography on silica gel (eluant; 40% EtOAc: 60% hexane) to yield the title compound (1.05g, 79%).

### Example E5.6

### 1-Benzoyl-1,2,3,4-tetrahydro-benzo[b]azepin-5-one

1,2,3,4-Tetrahydro-benzo[b]azepin-5-one from Example E5.5 (480mg, 2.98mmol) was dissolved in a mixture of dichloromethane (30ml) and triethylamine (1.3ml). Benzoyl chloride (0.46g, 3.28mmol) was added and the reaction mixture was heated for at reflux for 2h. The mixture was cooled and reduced *in vacuo.* The residue was dissolved in EtOAc and washed with 1M KHSO₄(aq), water and brine. The organic layer was dried, filtered and reduced *in vacuo.* The residue was purified by flash chromatography on silica gel (eluant; 50% EtOAc: 50% hexane) to yield the title compound (440mg, 56%).

### Example E5.7

### 1-Benzoyl-4-bromo-1,2,3,4-tetrahydro-benzo[b]azepin-5-one

1-Benzoyl-1,2,3,4-tetrahydro-benzo[b]azepin-5-one from Example E5.6 (54.2g, 205mmol), *N*-bromosuccinimide (3.6g, 20.4mmol) and bromine (11.1ml, 214.7mmol) were dissolved in dichloromethane (1.0litre). triethylamine (30ml, 215mmol) was added dropwise over 30 minutes then the reaction mixture was heated at reflux for 4h. Additional bromine (1.1ml, 21.5mmol) and triethylamine (3.0ml, 21.5mmol) were added and the reaction mixture was heated at reflux for a further 4h. Additional bromine (1.1ml, 21.5mmol) and triethylamine (3.0ml, 21.5mmol) were added again and the reaction mixture was heated at reflux for a further 4h. On cooling to room temperature, the reaction solution was washed with 5% aqueous sodium metabisulfate solution (150ml) and the aqueous phase was diluted with water (600ml). The organic phase was separated, washed with saturated Na-HCO₃(aq), dried over sodium sulphate and filtered. The *fi*ltrate was diluted with EtOAc (100ml), filtered through a silica pad (eluant; CH₂Cl₂) and reduced *in vacuo* to yield the title compound (73.6g) which was used without further purification.

### Example E5.8

### 6-Benzoyl-2-methyl-1,4,5,6-tetrahydro-1,3,6-triaza-benzo[e]azulene

1-Henzoyl-4-bromo-1,2,3,4-tetrahydro-benzo[b]azepin-5-one (67.5g, 200mmol) from Example E5.7, acetamidine hydrochloride (92.7g, 980mmol) and potassium carbonate (136.0g, 980mmol) were suspended in acetonitrile (2.0litres) and heated at reflux for 17h under nitrogen. Additional acetamidine hydrochloride (18.5g, 200mmol) and potassium carbonate (27.7g, 200mmol) were added and the reaction mixture was heated at reflux for a further 6h. Additional acetamidine hydrochloride (18.5g, 200mmol) and potassium carbonate (27.7g, 200mmol) were added again and the reaction mixture was heated at reflux for a further 6h. On cooling to room temperature, the reaction mixture was filtered and the filtrate was concentrated *in vacuo.* The residue was taken up in dichloromethane (1.4litres), washed with water (500ml), dried over sodium sulfate, filtered and reduced *in vacuo.* The crude products were purified by flash chromatography on silica gel (eluant; 45% EtOAc: 45% acetonitrile: 10% methanol) to yield the title compound (26.7g, 34%) and 6-benzoyl-2-methyl-5,6-dihydro-4H-1-oxa-3,6-diaza-benzo[e]azulene (3.3g, 4%).

### Example E5.9

### 2-Methyl-1,4,5,6-tetrahydro-1,3,6-triaza-benzo[e]azulene

6-Benzoyl-2-methyl-1,4,5,6-tetrahydro-1,3,6-triaza-benzo[e]azulene from Example E5.8 (160mg, 0.53mmol) was dissolved in a 6M HCl/dioxan solution (50ml) and heated at reflux for 18h. The reaction mixture was cooled to room temperature and reduced *in vacuo.* The residue was partitioned between EtOAc and saturated NaHCO₃(aq) and the layers were separated. The organic layer was washed with brine, dried, filtered and reduced *in vacuo* to yield the title compound (69mg, 66%).

### Example E5a

### 1-Benzyl-2-methyl-1,4,5,6-tetrahydro-1,3,6-triaza-benzo[e]azulene

### Example E5a.1

### (1-Benzyl-2-methyl-4,5-dihydro-1H-1,3,6-triaza-benzo[e]azulen-6-yl)-phenyl-methanone

Sodium hydride (60% dispersion in oil, 905mg, 22.5mmol) was placed in anhydrous THF (200ml) and cooled down to 0°C. 6-Benzoyl-2-methyl-1,4,5,6-tetrahydro-1,3,6-triaza-benzo[e]azulene from Example E5.8 (4.9g, 16.1mmol) was added dropwise and the mixture was stirred for 1 h at room temperature. Benzyl bromide (2.31ml, 19.3mmol) and potassium iodide (1.34g, 8.0mmol) were added and the mixture was stirred for 16h. The solution was diluted with EtOAC, washed with saturated NaHCO₃ then brine, dried and concentrated *in vacuo.* The residue was purified by flash chromatography on silica gel (eluant; EtOAc) to yield the title compound (5.73g, 90%).

### Example E5a.2

### 1-Benzyl-2-methyl-1,4,5,6-tetrahydro-1,3,6-triaza-benzo[e]azulene

(1-Benzyl-2-methyl-4,5-dihydro-1H-1,3,6-triaza-benzo[e]azulen-6-yl)-phenyl-methanone from Example E5a.1 (5.73g, 14.6mmol) was placed in methanol (50ml) and a 6M HCl aqueous solution (200ml) was added. The reaction mixture was heated at reflux for 18h then concentrated *in vacuo.* The residue was dissolved in dichloromethane, basified with saturated NaHCO₃ then washed with brine, dried and concentrated *in vacuo* to yield the title compound (3.30g, 78%).

### Example E6

### 2-Methyl-5,6-dihydro-4H-1-oxa-3,6-diaza-benzo[e]azulene

6-Benzoyl-2-methyl-5,6-dihydro-4H-1-oxa-3,6-diaza-benzo[e]azulene from Example E5.8 (1.0g, 3.25mmol) was reacted with a 6M HCl/dioxan solution (100ml) using an analogous procedure to that described for Example E5.9 to yield the title compound (540mg, 82%).

### Example E7

### 2-Methyl-5,6-dihydro-4H-3-thia-1,6-diaza-benzo[e]azulene

### Example E7.1

### (2-Methyl-4,5-dihydro-3-thia-1,6-diaza-benzo[e]azulen-6-yl)-phenyl-methanone

To a solution of 1-benzoyl-4-bromo-1,2,3,4-tetrahydrobenzo[b]azepin-5-one (1.0g, 2.9mmol) from Example E5.7 in ethanol (50 ml) was added thioacetamide (0.75g, 10mmol). The solution was stirred for 16h. The resultant suspension was reduced in volume by evaporation and cooled. The precipitate was collected by filtration and the solid was washed with cold ethanol and dried to yield the title compound as a white solid (0.65g, 70%).

### Example E7.2

### 2-Methyl-5,6-dihydro-4H-3-thia-1,6-diaza-benzo[e]azulene

A suspension of (2-methyl-4,5-dihydro-3-thia-1,6-diaza-benzo[e]azulen-6-yl)-phenyl-methanone from Example E7.1 (0.42g, 1.3mmol) in 6M hydrochloric acid (45ml) was heated at reflux for 16h. The solution was cooled and treated with saturated NaHCO₃(aq) (100ml). Additional solid NaHCO₃ was added until the solution was basic. The mixture was extracted with dichloromethane and the organic extracts were dried and reduced *in vacuo* to yield the title compound as a yellow oil (0.21g, 76%).

### Example E8

### 4-(3,3-Dimethyl-butyl)-piperazine-1-carboxylic acid 4-(3,6-dimethyl-4,10-dihydro-3H-2,3,4,9-tetraaza-benzo[f]azulene-9-carbonyl)-2-fluoro-benzylamide

### Example E8.1

### [4-(3,6-Dimethyl-4,10-dihydro-3H-2,3,4,9-tetraaza-benzo[f]azulene-9-carbonyl)-2-fluoro-benzyl]-carbamic acid tert-butyl ester

A solution of 4-(tert-butoxycarbonylamino-methyl)-3-fluorobenzoic acid from Example E3.1 (538mg, 2.0mmol) and DMAP (220mg, 1.8mmol) in dichloromethane (20ml) at room temperature was treated with DIEA (0.93mal, 5.4mmol) and WSCD (460mg, 2.4mmol), and the resulting solution was stirred at room temperature for 1h. 3,6-Dimethyl-3,4,9,10-tetrahydro-2,3,4,9-tetraaza-benzo[f]azulene from Example E2 (385mg, 1.8mmol) was added and the resulting solution was heated at reflux for 20h and allowed to cool to room temperature. The solution was diluted with dichloromethane, washed with saturated NaHCO₃ and brine and concentrated *in vacuo.* The residue was purified by preparative HPLC (eluant 10% methanol:90% dichloromethane) to yield the title compound (265mg, 32%).

### Example E8.2

### (4-Aminomethyl-3-fluoro-phenyl)-(3,6-dimethyl-4,10-dihydro-3H-2,3,4,9-tetraaza-benzo[f]azulen-9-yl)-methanone hydrochloride

[4-(3,6-dimethyl-9,10-dihydro-3H-2,3,4,9-tetraaza-benzo[f]azulene-9-carbonyl)-2-fluoro-benzyl]-carbamic acid tert-butyl ester from Example E8.1 (237mg, 0.51mmo1) was reacted with 4N HCl/dioxan using an analogous procedure to that described for Example E4.2 to yield the title compound (205mg, 100%).

### Example E8.3

### 4-(3,3-Dimethyl-butyl)-piperazine-1-carboxylic acid 4-(3,6-dimethyl-4,10-dihydro-3H-2,3,4,9-tetraaza-benzo[f]azulene-9-carbonyl)-2-fluoro-benzylamide

DIEA (0.10ml, 0.60mmol) and 1,1'-carbonyldiimidazole (28mg, 0.17mmol) were added to a solution of (4-aminomethyl-3-fluoro-phenyl)-(3,6-dimethyl-4,10-dihydro-3H-2,3,4,9-tetraaza-benzo[f]azulen-9-yl)-methanone hydrochloride from Example E8.2 (57mg, 0.14mmol) in DMF (5.0ml) and the mixture was stirred at room temperature for 4h. 1-(3,3-Dimethyl-butyl)-piperazine dihydrochloride from Example E4 (38mg, 0.16mmol) was added and the mixture was stirred at room temperature for 24h and concentrated *in vacuo.* The residue was dissolved in EtOAc and the resulting solution was washed with brine and concentrated *in vacuo.* The residue was purified by preparative HPLC (eluant; 10% methanol:90% dichloromethane) to give a white solid identified as the title compound (45mg, 56%).

### Example E9

### 4-(tert-Butoxycarboaylamiao-methyl)-cyclohexanecarboxylic acid

To a solution of 4-aminomethyl-cyclohexanecarboxylic acid (20.0g, 127.39mmol) in dioxan (400ml) was added 1N KHCO₃ (300ml, 300mmol) and di-tert-butyl dicarbonate (33.3g, 129.57mmol). The mixture was stirred for 18h and concentrated in vacuo. The aqueous residue was washed with ether, then acidified with 1N KHSO₄ and extracted with EtOAc (x 3). The combined organic extracts were washed with water and brine and concentrated in vacuo to give a white solid identified as the title compound (31.9g, 98%).

### Example E10

### Cyclopropanecarboxylic acid [4-(6-chloro-3-methyl-4,10-dihydro-3H-2,3,4,9-tetraaza-benzo[f]azulene-9-carbonyl)-cyclohexylmethyl]-amide

### Example E10.1

### [4-(6-Chloro-3-methyl-4,10-dihydro-3H-2,3,4,9-tetraaza-benzo[f]azulene-9-carbonyl)-cyclohexylmethyl]-carbamic acid tert-butyl ester

A solution of 4-(tert-butoxycarbonylamino-methyl)-cyclohexanecarboxylic acid from Example E9 (510mg, 2.0mmol) in dichloromethane (25 ml) at room temperature was treated with DIEA (0.70ml, 4.0mmol), PyBrop (2.40g, 5.1mmol), and 5-chloro-3-methyl-3,4,9,10-tetrahydro-2,3,4,9-tetraaza-benzo[f]azulene from Example E1 (422mg, 1.8mmol) and the resulting solution was heated at reflux for 20h and allowed to cool to room temperature. The solution was diluted with dichloromethane, washed with brine and concentrated in vacuo. The residue was purified by flash chromatography on silica gel (eluant EtOAc) to yield the title compound (775mg, 91%).

### Example E10.2

### (4-Aminomethyl-cyclohexyl)-(6-chloro-3-methyl-4,10-dihydro-3H-2,3,4,9-tetraaza-benzo[f]azulen-9-yl)-methanone hydrochloride

A solution of [4-(6-chloro-3-methyl-4,10-dihydro-3H-2,3,4,9-tetraaza-benzo[f]azulene-9-carbonyl)-cyclohexylmethyl]-carbamic acid tert-butyl ester from Example E10.1 (775mg, 1.63mmol) was reacted with 4N HCl/dioxan using an analogous procedure to that described for Example E4.2 to yield the title compound (655mg, 100%).

### Example E10.3

### Cyclopropanecarboxylic acid [4-(6-chloro-3-methyl-4,10-dihydro-3H-2,3,4,9-tetraaza-benzo[f]azulene-9-carbonyl)-cyclohexylmethyl]-amide

DIEA (0.50ml, 2.90mmol) and cyclopropanecarbonyl chloride (0.045ml, 0.50mmol) were added to a solution of (4-aminomethyl-cyclohexyl)-(6-chloro-3-methyl-4,10-dihydro-3H-2,3,4,9-tetraaza-benzo[f]azulen-9-yl)-methanone hydrochloride from Example E10.2 (220mg, 0.53mmol) in dichloromethane (5ml). The mixture was stirred for 2h then diluted with dichloromethane, washed with water and concentrated in vacuo. The residue was purified by preparative HPLC (eluant; 0.5% 35% ammonia/10% methanol/90% dichloromethane) to give a white solid identified as the title compound (132 mg, 60%).

### Example E11

### 3-Methyl-3,4,9,10-tetrahydro-2,3,4,9-tetraaza-benzo[f]azulene

### Example E11.1

### 1-Methyl-5-(2-nitro-phenylamino)-1H-pyrazole-4-carboxylic acid ethyl ester

Sodium hydride (60% dispersion in oil, 7.0g, 170mmol) was added portionwise to a suspension of 5-amino-1-methyl-1H-pyrazole-4-carboxylic acid ethyl ester (21.1g, 125mmol) in anhydrous THF (300ml) at 0°C. The mixture was allowed to warm to room temperature and stirred for 0.75h then cooled to 0°C. 1-fluoro-2-nitrobenzene (17.6g, 125mmol) was added and the resultant suspension was stirred at room temperature for 18h. EtOAc and 0.3M KHS04 were added and separated. The aqueous layer was extracted with EtOAc and the combined organic layers were washed with brine, dried and concentrated in vacuo. The residue was purified by flash chromatography on silica gel (eluant 50% hexanes/50% ethyl acetate) to yield the title compound (20.8g, 58%).

### Example E11.2

### 5-(2-Amino-phenylamino)-1-methyl-1H-pyrazole-4-carboxylic acid ethyl ester

1-Methyl-5-(2-nitro-phenylamino)-1H-pyrazole-4-carboxylic acid ethyl ester (20.8g, 72mmol) from Example E11.1 was dissolved in methanol (330ml) and hydrogenated over 10% Pd/C catalyst for 4h. The mixture was filtered through Celite® filter agent and the filtrate was concentrated in vacuo to give a white solid identified as the title compound (16.2g, 87%).

### Example E11.3

### 3-Methyl-4,9-dihydro-3H-2,3,4,9-tetraaza-benzo[f]azulen-10-one

Sodium (methylsulphinyl)methanide (29.7mmol) was prepared from sodium hydride (60% dispersion in oil, 1.19g, 29.7mmol) and anhydrous dimethyl sulphoxide (7ml) by heating at 65°C until a solution was observed. To this was added a solution of 5-(2-amino-phenylamino)-1-methyl-1H-pyrazole-4-carboxylic acid ethyl ester from Example E11.2 (3.63g, 16.9mmol) in anhydrous dimethyl sulphoxide (10ml), and stirring continued at 65°C for 2.5h. The mixture was poured into ice (100ml), and the resulting solid collected and purified by recrystallisation from methanol/EtOAc/60-80 pet ether to yield the title compound (1.46g, 40%).

### Example E11.4

### 3-Methyl-3,4,9,10-tetrahydro-2.3,4,9-tetraaza-benzo[f]azulene

To a suspension of 3-methyl-4,9-dihydro-3H-2,3,4,9-tetraaza-benzo[f]azulen-10-one from Example E11.3 (3.01g, 14.1 mmol) in anhydrous THF (100ml) at 0°C was added lithium aluminium hydride (2.13g, 56.2mmol), and the resulting suspension was heated at reflux for 18h, then allowed to cool to room temperature. The mixture was cooled to 0°C, 35% ammonia solution (10ml) was added dropwise over 15 min and the mixture was stirred at room temperature for 30min. The resulting suspension was filtered through Celite® filter agent and the filtrate concentrated in vacuo. The residue was purified by flash chromatography on silica gel (eluant; 2% methanol:chloroform rising to 5% methanol:chloroform) to yield the title compound (1.60g, 57%).

### Example E12

### [4-(Isobutylamino-methyl)-cyclohexyl]-(3-methyl-4,10-dihydro-3H-2,3,4,9-tetraaza-benzo[f]azulen-9-yl)-methanone

A solution of isobutyraldehyde (0.36mg, 0.005mmol) in 1,2-dichloroethane (0.05ml) was added to a solution of (4-Aminomethyl-cyclohexyl)-(3-methyl-4,10-dihydro-3H-2,3,4,9-tetraaza-benzo[f]azulen-9-yl)-methanone hydrochloride from Example E10.2 (1.88mg, 0.005mmol) in 1,2-dichloroethane (0.05ml) and DIEA (0.0026ml). The mixture was stirred at room temperature for 1h then a solution of sodium triacetoxyborohydride (1.59mg, 0.0075mmol) in DMF (0.05ml) was added. The mixture was stirred for 20h at room temperature then solvents were removed *in vacuo* to yield the title compound. (ESI)+: [M+H]+= 396.4

### Example E13

### [4-(3,6-Dimethyl-4,10-dihydro-3H-2,3,4,9-tetraaza-benzo[f]azulene-9-carbonyl)-cyclohexylmethyl]-carbamic acid 4-nitro-benzyl ester

A solution of 4-nitrobenzyl chloroformate (1.08mg, 0.005mmol) in dichloromethane (0.05ml) was added to a solution of (4-Aminomethyl-cyclohexyl)-(3,6-dimethyl-4,10-dihydro-3H-2,3,4,9-tetraaza-benzo[f]azulen-9-yl)-methanone hydrochloride (Compound number 149) (1.95mg, 0.005mmol) in dichloromethane (0.05ml) and triethylamine (0.0035ml). The mixture was stirred at room temperature for 1h then solvents were removed *in vacuo* to yield the title compound. (ESI)+: [M+H]+= 533.3

### Example E14

### (6-Chloro-3-methyl-4,10-dihydro-3H-2,3,4,9-tetraaza-benzo[f]azulen-9-yl)-(4-hexylaminomethyl-cyclohexyl)-methanone

A solution of 1-bromohexane (0.83mg, 0.005mmol) in DMF(0.05ml) was added to a solution of (4-Aminomethyl-cyclohexyl)-(6-chloro-3-methyl-4,10-dihydro-3H-2,3,4,9-tetraaza-benzo[f]azulen-9-yl)-methanone hydrochloride from Example E10.2 (2.05mg, 0.005mmol) in DMF (0.05ml) and triethylamine (0.0021ml). The mixture was stirred for 20h at room temperature then solvents were removed *in vacuo* to yield the title compound. (ESI)+: (M+H]+= 458.4

### Example E15

### 1-(3,3-Dimethyl-butyl)-piperidine-4-carboxylic acid

### Example E15.1

### 1-(3,3-Dimethyl-butyl)-piperidine-4-carboxylic acid ethyl ester

3,3-Dimethylbutyraldehyde (5.26ml), 42.0mmol) was added to a solution of ethyl isonipecotate (6.6g, 42.0mmol) in methanol/acetic acid (99:1, v/v, 50ml) and the mixture was stirred at room temperature for 1h. Sodium cyanoborohydride (3.43g, 54.6mmol) was added, and the mixture was stirred at room temperature for 4 days then concentrated *in vacuo.* The residue was purified by flash chromatography on silica gel (eluant; 25% EtOAc:75% cyclohexane) to yield the title compound (7.16g, 71%).

### Example E15.2

### 1-(3,3-Dimethyl-butyl)-piperidine-4-carboxylic acid

Lithium hydroxide monohydrate (1.37g, 32.6mmol) was added to a solution of 1-(3,3-dimethyl-butyl)-piperidine-4-carboxylic acid ethyl ester from Example E15.1 (7.16g, 29.7mmol) in THF (50ml) and water (5mal). The mixture was stirred at room temperature for 18h then concentrated *in vacuo.* The residue was purified by flash chromatography on silica gel through an isolute (eluant; 90% dichloromethane:9% methanol:1% acetic acid) to yield the title compound (5.51g, 87%).

### Example E15a

### 1-Cyclopropylmethyl-piperidine-4-carboxylic acid

The title compound was prepared from cyclopropanecarboxaldehyde and ethyl isonipecotate using similar procedures to those described for Example E15.

### Example E16

### 1-(3,3-Dimethyl-butyl)-piperidine-4-carboxylic acid [4-(3-methyl-4,10-dihydro-3H-2,3,4,9-tetraaza-benzo[f]azulene-9-carbonyl)-cyclohexylmethyl]-amide

### Example E16.1

### 4-(3,3-Dimethyl-butyl)-piperazine-4-carboxylic acid [4-(3-methyl-4,10-dihydro-3H-2,3,4,9-tetraaza-benzo[f]azulene-9-carbonyl)-cyclohexylmethyl]-amide

HBTU (84mg, 0.22mmol) was added to a solution of 1-(3,3-dimethyl-butyl)-piperidine-4-carboxylic acid from Example E15 (42mg, 0.17mmol) in DMF (5ml) and DIEA (to pH9). The mixture was stirred at room temperature for 1 h. (4-Aminomethyl-cyclohexyl)-(3-methyl-4,10-dihydro-3H-2,3,4,9-tetraaza-benzo[f]azulen-9-yl)-methanone hydrochloride (Compound Number 150) (42mg, 0.11mmol) was added and the mixture was stirred at room temperature for 18h. Solvents were removed *in vacuo* and azeotroped with toluene. The residue was redissolved in EtOAc, washed with saturated NaHCO₃ then brine, dried and concentrated *in vacuo.* The residue was purified by preparative HPLC (eluant; 10% methanol:90% dichloromethane) to give a white solid identified as the title compound (34mg, 57%).

### Example E17

### N-[4-(3,6-Dimethyl-4,10-dihydro-3H-2,3,4,9-tetraaza-benzo[f]azulene-9-carbonyl)-cyclohexylmethyl]-2-piperidin-4-yl-acetamide hydrochloride

A solution of 4-({[4-(3,6-dimethyl-4,10-dihydro-3H-2,3,4,9-tetraaza-benzo[f]azulene-9-carbonyl)-cyclohexylmethyl]-carbamoyl}-methyl)-piperidine-1-carboxylic acid tert-butyl ester (Compound Number 228) (259mg, 0.45mmol) was reacted with 4N HCl/dioxan using an analogous procedure to that described for Example E4.2 to yield the title compound (230mg, 93%).

### Example E18

### N-[4-(3,6-Dimethyl-4,10-dihydro-3H-2,3,4,9-tetraaza-benzo[f]azulene-9-carbonyl)-cyclohexylmethyl]-methanesulfonamide

A solution of methanesulfonyl chloride (0.57mg, 0.005mmol) in dichloromethane (0.05ml) was added to a solution of (4-aminomethyl-cyclohexyl)-(3,6-dimethyl-4,10-dihydro-3H-2,3,4,9-tetraaza-benzo[f]azulen-9-yl)-methanone (Compound number 149) (1.95mg, 0.005mmol) in dichloromethane (0.05ml) and triethylamine (0.0035ml). The mixture was stirred at room temperature for 1h then solvents were removed *in vacuo* to yield the title compound. (ESI)+: [M+H]+= 432.1

### Example E19

### N-[4-(3,6-Dimethyl-4,10-dihydro-3H-2,3,4,9-tetraaza-benzo[f]azulene-9-carbonyl)-cyclohexylmethyl]-2-(1-methanesulfonyl-piperidin-4-yl)-acetamide

A solution of methanesulfonyl chloride (0.57mg, 0.005mmol) in dichloromethane (0.05ml) was added to a solution of N-[4-(3,6-dimethyl-4,10-dihydro-3H-2,3,4,9-tetraaza-benzo[f]azulene-9-carbonyl)-cyclohexylmethyl]-2-piperidin-4-yl-acetamide hydrochloride from Example E17 (2.57mg, 0.005mmol) in dichloromethane (0.05ml) and triethylamine (0.0035ml). The mixture was stirred at room temperature for 1h then solvents were removed *in vacuo* to yield the title compound. (ESI)+: [M+H]+= 557.4

### Example E20

### 2-(1-Acetyl-piperidin-4-yl)-N-[4-(3,6-dimethyl-4,10-dihydro-3H-2,3,4,9-tetraaza-benzo[f]azulene-9-carbonyl)-cyclohexylmethyl]-acetamide

A solution of acetyl chloride (0.39mg, 0.005mmol) in dichloromethane (0.05ml) was added to a solution of N-[4-(3,6-dimethyl-4,10-dihydro-3H-2,3,4,9-tetraaza-benzo[f]azulene-9-carbonyl)-cyclohexylmethyl]-2-piperidin-4-yl-acetamide hydrochloride from Example E17 (2.57mg, 0.005mmol) in dichloromethane (0.05ml) and triethylamine (0.0035ml). The mixture was stirred at room temperature for 1h then solvents were removed *in vacuo* to yield the title compound. (ESI)+: [M+H]+= 521.5

### Example E21

### 1-Isobutyl-piperidine-4-carboxylic acid [4-(3-methyl-4,10-dihydro-3H-2,3,4,9-tetraaza-benzo[f]azulene-9-carbonyl)-cyclohexylmethyl]-amide

A solution of isobutyraldehyde (0.36mg, 0.005mmol) in 1,2-dichloroethane (0.05ml) was added to a solution of piperidine-4-carboxylic acid [4-(3-methyl-4,10-dihydro-3H-2,3,4,9-tetraaza-benzo[f]azulene-9-carbonyl)-cyclohexylmethyl]-amide hydrochloride (Compound number 238) (2.43mg, 0.005mmol) in 1,2-dichloroethane (0.05ml) and DIEA (0.0026ml). The mixture was stirred at room temperature for 1h then a solution of sodium triacetoxyborohydride (1.59mg, 0.0075mmol) in DMF (0.05ml) was added. The mixture was stirred for 20h at room temperature then solvents were removed *in vacuo* to yield the title compound. (ESI)+: [M+H]+= 507.4

### Example E22

### N-[4-(6-Chloro-3-methyl-4,10-dihydro-3H-2,3,9,9-tetraaza-benzo[f]azulene-9-carbonyl)-cyclohexylmethyl]-3-(1-hexyl-piperidin-4-yl)-propionamide

A solution of 1-bromohexane (0.83mg, 0.005mmol) in DMF (0.05ml) was added to a solution of N-[4-(6-chloro-3-methyl-4,10-dihydro-3H-2,3,4,9-tetraaza-benzo[f]azulene-9-carbonyl)-cyclohexylmethyl]-3-piperidin-4-yl-propionamide hydrochloride (Compound number 237) (2.57mg, 0.005mmol) in DMF (0.05ml) and triethylamine (0.0021ml). The mixture was stirred for 20h at room temperature then solvents were removed *in vacuo* to yield the title compound. (ESI)+: [M+H]+= 597.6/599.6

### Example E23

### 4-({[4-(3,6-Dimethyl-4,10-dihydro-3H-2,3,4,9-tetraaza-benzo[f]azulene-9-carbonyl)-cyclohexylmethyl]-carbamoyl)-methyl)-piperidine-1-carboxylic acid 4-nitro-benzyl ester

A solution of 4-nitrobenzyl chloroformate (1.08mg, 0.005mmol) in dichloromethane (0.05ml) was added to a solution of N-[4-(3,6-Dimethyl-4,10-dihydro-3H-2,3,4,9-tetraaza-benzo[f]azulene-9-carbonyl)-cyclohexylmethyl]-2-piperidin-4-yl-acetamide hydrochloride from Example E17 (2.58mg, 0.005mmol) in dichloromethane (0.05ml) and triethylamine (0.0035ml). The mixture was stirred at room temperature for 1h then solvents were removed in *vacuo* to yield the title compound. (ESI)+: [M+H]+= 658.5

### Example E24

### 4-(3,3-Dimethyl-butyl)-piperazine-1-carboxylic acid [4-(3,6-dimethyl-4,10-dihydro-3H-2,3,4,9-tetraaza-benzo[f]azulene-9-carbonyl)-cyclohexylmethyl]-amide

DIEA (0.30ml, 1.7mmol) and 1,1'-carbonyldiimidazole (39mg, 0.24mmol) were added to a solution of (4-aminomethyl-cyclohexyl)-(3,6-dimethyl-4,10-dihydro-3H-2,3,4,9-tetraaza-benzo[f]azulen-9-yl)-methanone hydrochloride (Compound number 149) (78mg, 0.20mmol) in DMF (4.0ml) and the mixture was stirred at room temperature for 2h. 1-(3,3-Dimethyl-butyl)-piperazine dihydrochloride from Example E4 (56mg, 0.23mmol) was added and the mixture was stirred at room temperature for 18h. The mixture was diluted with EtOAc, washed with brine, dried and concentrated in vacuo. The residue was purified by preparative HPLC (eluant; 10% methanol:90% dichloromethane) to give a white solid identified as the title compound (32mg, 29%).

### Example E25

### 3-Chloro-6,7,8,9-tetrabydro-5-oxa-9-aza-benzocycloheptene

### Example E25.1

### N-(4-Chloro-2-hydroxy-phenyl)-benzamide

2-Amino-5-chlorophenol (1.45g, 10mmol) was dissolved in EtOAc (30ml) and water (20ml). Sodium dicarbonate (1.25g, 15mmol) then benzoyl chloride (1.42g, 10mmol) were added and the mixture was stirred at room temperature for 1h. The layers were partitioned and the organic layer was washed with brine, dried and concentrated in vacuo. The residue was triturated with diethyl ether to yield the title compound (2.05g, 82%).

### Example E25.2

### (3-Chloro-7,8-dihydro-6H-5-oxa-9-aza-benzocyclohepten-9-yl)-phenyl-methanone

N-(4-Chloro-2-hydroxy-phenyl)-benzamide from Example E25.1 (1.0g, 4mmol) was dissolved in acetonitrile (10ml) and dichloromethane (15ml). 1,3-Dibromopropane (3.26g, 16mmol), aliquat 336 (170mg, 0.4mmol) and sodium hydroxide (750mg, 16mmol) were added and the mixture was heated at 60°C for 3h. The solid was filtered off and the filtrate concentrated *in vacuo.* The residue was purified by flash chromatography on silica gel (eluant; 30% EtOAc:70% hexane) to yield the title compound (783mg, 83%).

### Example E25.3

### 3-Chloro-6,7,8,9-tetrahydro-5-oxa-9-aza-benzocycloheptene

(3-Chloro-7,8-dihydro-6H-5-oxa-9-aza-benzocyclohepten-9-yl)-phenyl-methanone from Example E25.2 (783mg, 2.7mmol) was dissolved in dioxan (10ml) and 6M HCl aqueous solution (50ml) was added. The mixture was heated at reflux for 20h then concentrated *in vacuo* and azeotroped with toluene. The residue was dissolved in diethyl ether and water, basified with NaHCO₃ and the layers were partitioned. The organic layer was washed with brine, dried and concentrated *in vacuo* to yield the title compound (280mg, 47%).

### Example E26

### N-Cyclohexyl-N'-methyl-benzene-1,2-diamine

### Example E26.1

### Cyclohexyl-(2-nitro-phenyl)-amine

A mixture of 2-fluoronitrobenzene (5.0g, 35.4mmol), cyclohexylamine (4.5ml, 39.0mmol) and potassium carbonate (17.1g, 124mmol) in acetonitrile (100ml) was heated at reflux for 2 days. The mixture was diluted with EtOAC, washed with water then brine, dried and partially concentrated *in vacuo.* The solid which precipitated was collected and washed with hexane to yield the title compound (6.7g, 86%).

### Example E26.2

### N-Cyclohexyl-benzene-1,2-diamine

Cyclohexyl-(2-nitro-phenyl)-amine from Example E26.1 (3.0g, 13.6mmol) was dissolved in methanol (100ml) and tin (II) chloride (12.9g, 68.1mmol) was added. The mixture was stirred for 20h at room temperature then heated at reflux for 18h and concentrated *in vacuo.* The residue was placed in EtOAC (100ml), cooled down to 0°C and pH was adjusted to 14 with conc. NH₃. The precipitate was filtered off and washed with EtOAc. The filtrate was washed with 2M NH₃, water then brine, dried and concentrated *in vacuo* to yield the title compound (2.25g, 86%).

### Example E26.3

### N-Cyclohexyl-N'-methyl-benzene-1,2-diamine

Potassium carbonate (2.45g, 17.7mmol) and iodomethane (0.81ml, 13.0mmol) were added to a solution of N-cyclohexyl-benzene-1,2-diamine from Example E26.2 (2.25g, 11.8mmol) in DMF (10ml). The mixture was stirred for 6h at room temperature then poured into water and extracted with EtOAc. The organic layer was washed with water then brine, dried and concentrated *in vacuo*. The residue was purified by flash chromatography on silica gel (eluant; 5% EtOAc:95% hexane) to yield the title compound (950mg, 39%).

### Example E27

### 3-Fluoro-4-hydroxy-benzoic acid ethyl ester

A solution of 3-fluoro-4-hydroxy-benzoic acid (5.16g, 33.1mmol) in ethanol (100ml) was treated with conc. sulphuric acid (5ml), and the mixture heated at reflux for 4 days. The volatiles were removed *in vacuo,* and the aqueous residue was basified with saturated NaHCO₃ and extracted twice with diethyl ether. The combined organic extracts were dried and concentrated *in vacuo* to yield the title compound (5.16g, 85%).

### Example E28

### 2-Chloro-4-hydroxy-benzoic acid ethyl ester

A solution of 2-chloro-4-hydroxy-benzonitrile (5.0g, 32.6mmol) in ethanol (125ml) was treated with conc. sulphuric acid (25ml) and the mixture heated at reflux for 3 days. Conc. sulphuric acid (25ml) was added and the mixture was heated at reflux for another 2 days. The volatiles were removed *in vacuo,* and the aqueous residue was basified with saturated NaHCO₃ and extracted 4 times with diethyl ether. The combined organic extracts were dried and concentrated *in vacuo* to yield the title compound (2.5g, 38%).

### Example E29

### 4-Hydroxy-3-methyl-benzoic acid methyl ester

4-Amino-3-methyl-benzoic acid methyl ester (5.25g, 32.0mmol) was treated with a 35% solution of sulphuric acid (50ml) and the mixture was stirred and heated until dissolution then cooled to 0°C. Sodium nitrite (2.82g, 41.6mmol) in water (50ml) was added dropwise and the mixture was stirred for 5min at 0°C. Urea was added to destroy the excess nitrite. Copper nitrate (121g, 320mmol) in water (11) was added then copper oxide (4.25g, 32.0mmol). The mixture was warmed up to room temperature over 30 min and extracted with EtOAc (x 3). The organics were combined, washed with brine, dried and concentrated *in vacuo.* The residue was purified by flash chromatography on silica gel (eluant; 30% EtOAc:70% hexane) to yield the title compound (2.2g, 42%) .

### Example E30

### 4-(3-Carboxy-propyl)-3-methyl-benzoic acid methyl ester

### Example E30.1

### 4-((E)-2-tert-Butoxycarbonyl-vinyl)-3-methyl-benzoic acid methyl ester

Tetrakis(triphenylphosphine)palladium (0) (5.0g, 4.33mmol) was added to a stirred solution of methyl 4-bromo-3-methylbenzoate (9.93g, 43.3mmol), tert-butyl acrylate (50ml, 341.3mmol) and sodium acetate (35.8g, 436.4mmol) in DMA (350ml). The mixture was heated at 140°C for 5h, filtered through Celite® filter agent and the filtrate was concentrated *in vacuo.* The residue was redissolved in EtOAc, washed with 0.3M KHSO₄ then brine, dried and concentrated *in vacuo.* The residue was purified by flash chromatography on silica gel (eluant; 15% EtOAc:85% hexane to 20% EtOAc:80% hexane) to yield the title compound (4.81g, 40%).

### Example E30.2

### 4-(2-tert-Butoxycarbonyl-ethyl)-3-methyl-benzoic acid methyl ester

4-((E)-2-tert-Butoxycarbonyl-vinyl)-3-methyl-benzoic acid methyl ester from Example E30.1 (4.00g, 14.5mmol) was dissolved in methanol (100ml) and hydrogenated over 10% Pd/C catalyst (480mg) for 5h. The mixture was filtered through Celite® filter agent and the filtrate was concentrated *in vacuo* to yield the title compound (3.41g, 84%).

### Example E30.3

### 4-(2-Carboxy-ethyl)-3-methyl-benzoic acid methyl ester

Trifluoroacetic acid (40ml) was added slowly to a stirred solution of 4-(2-tert-butoxycarbonyl-ethyl)-3-methyl-benzoic acid methyl ester from Example E30.2 (4.9g, 17.6mmol) in dichloromethane (80ml) at room temperature. The mixture was stirred for 2h then concentrated *in vacuo* and azeotroped with dichloromethane. The residue was recrystallised in EtOAc to yield the title compound (2.77g, 71%).

### Example E30.4

### 4-(3-Carboxy-propyl)-3-methyl-benzoic acid methyl ester

4-(2-Carboxy-ethyl)-3-methyl-benzoic acid methyl ester from Example E30.3 (500mg, 2.25mmol) was dissolved in dry dichloromethane (10ml) and a few drops of DMF. Oxalyl chloride (0.393ml, 4.5mmol) was added dropwise and the mixture was stirred for 1h at room temperature. Solvents were removed *in vacuo* and azeotroped with toluene. The residue was redissolved in acetonitrile (20ml) and cooled to 0°C. A solution of 2M (trimethylsilyl)diazomethane in hexanes (2.25ml, 4.5mmol) was added dropwise and the reaction mixture was stirred for 5h at 0°C then 20h at room temperature. Ethyl acetate then 10% citric acid solution were added and the layers were partitioned. The organic layer was washed with saturated NaHCO₃ then brine, dried and concentrated *in vacuo*. The residue was purified by flash chromatography on silica gel (eluant; 50% ethyl acetate:50% cyclohexane). The product obtained was dissolved in a mixture of acetonitrile and water. A solution of silver benzoate (103mg, 0.45mmol) in triethylamine (1.25ml, 9mmol) was added gradually while the mixture was sonicated in an ultrasound bath. After 30min at room temperature, solvents were removed *in vacuo.* Ethyl acetate and 10% aqueous citric acid solution were added and the layers were partitioned. The organic layer was washed with brine, dried and concentrated *in vacuo.* The residue was purified by flash chromatography on silica gel (eluant; 50% EtOAc:50% cyclohexane) to yield the title compound (378mg, 71%).

### Example E31

### 4-(4-Methoxycarbonyl-butyl)-3-methyl-benzoic acid

### Example E31.1

### 4-Bromo-3-methyl-benzoic acid tert-butyl ester

4-Bromo-3-methyl-benzoic acid (2.06g, 9.6mmol) and thionyl chloride (2.2ml, 30.2mmol) in toluene (50ml) were heated at reflux for 2h then concentrated *in vacuo* and azeotroped with toluene. The residue was dissolved in THF (100ml) and triethylamine (2.8ml, 20.1mmol), cooled down to 0°C and lithium tert-butoxide (1.24g, 15.5mmol) was added portionwise. The mixture was stirred for 18h at room temperature then concentrated in *vacuo.* The residue was dissolved in EtOAc, washed with 1M HCl, saturated NaHCO₃ then brine, dried and concentrated in *vacuo*. The residue was purified by flash chromatography on silica gel (eluant; 5% EtOAc:95% pet. ether) to yield the title compound (1.86g, 71%).

### Example E31.2

### 4-((1E,3E)-4-Methoxycarbonyl-buta-1,3-dienyl)-3-methyl-benzoic acid tert-butyl ester

Tetrakis(triphenylphosphine)palladium (0) (852mg, 0.7mol) was added to a stirred solution of 4-bromo-3-methyl-benzoic acid tert-butyl ester from Example E31.1 (1.86g, 6.8mmol), 1-acetoxy-1,3-butadiene (7.5ml, 64.5mmol) and sodium acetate (5.69g, 69.4mmol) in DMA (75ml). The mixture was heated at 140°C for 3h, filtered through Celite® filter agent and the filtrate was concentrated *in vacuo*. The residue was purified by flash chromatography on silica gel (eluant; 20% EtOAc:80% pet. ether) to yield the title compound (1.54g, 74%).

### Example E31.3

### 4-(4-Methoxycarbonyl-butyl)-3-methyl-benzoic acid tert-butyl ester

4-((1E,3E)-4-Methoxycarbonyl-buta-1,3-dienyl)-3-methyl-benzoic acid tert-butyl ester from Example E31.2 (1.54g, 5.1mmol) was dissolved in methanol (40ml) and hydrogenated over 10% Pd/C catalyst (200mg) for 5h. The mixture was filtered through Celite® filter agent and the filtrate was concentrated *in vacuo* to yield the title compound (1.43g, 92%).

### Example E31.4

### 4-(4-Methoxycarbonyl-butyl)-3-methyl-benzoic acid

Trifluoroacetic acid (20ml) was added to a solution of 4-(4-methoxycarbonyl-butyl)-3-methyl-benzoic acid tert-butyl ester from Example E31.3 (1.43g, 4.7mmol) in dichloromethane (40ml) and the mixture was stirred for 2h at room temperature. Solvents were concentrated *in vacuo* and azeotroped with dichloromethane to yield the title compound (1.07g, 92%).

### Example E32

### 4-Carboxymethyl-3-methyl-benzoic acid methyl ester

### Example E32.1

### 4-tert-Butoxycarbonylmethyl-3-methyl-benzoic acid methyl ester

Copper (II) fluoride (4.08g, 40.1mmol) and bis-[tri-(o-tolyl)phosphine]palladium dichloride (473mg, 0.6mmol) were added to a solution of methyl 4-bromo-3-methyl benzoate (4.60g, 20.1mmol) in THF (30ml). The mixture was heated at reflux before silyl ketene acetal (18.5g, 80.3mmol) was added. The mixture was heated at reflux for 2 days then diluted with Et₂O. Aqueous NH₄Cl solution (250ml) was added and the mixture was stirred for 30min at room temperature. The layers were partitioned and the aqueous layer extracted twice with Et₂O. The organics were combined, dried and concentrated *in vacuo.* The residue was purified by flash chromatography on silica gel (eluant; 5% EtOAc:95% hexane) to yield the title compound (2.84g, 53%).

### Example E32.2

### 4-Carboxymethyl-3-methyl-benzoic acid methyl ester

Trifluoroacetic acid (15ml) was added to a solution of 4-tert-butoxycarbonylmethyl-3-methyl-benzoic acid methyl ester from Example E32.1 (2.84g, 10.7mmol) in dichloromethane (15ml). The mixture was stirred for 90min at room temperature, concentrated *in vacuo* and azeotroped with toluene. The residue was recrystallised from EtOAc and hexane to yield the title compound (1.81g, 81%).

### Example E33

### 4-[1,3]Dioxolan-2-yl-piperidine

### Example E33.1

### 4-Hydroxymethyl-piperidine-1-carboxylic acid benzyl ester

4-Piperidinemethanol (5.0g, 43mmol) was dissolved in dichloromethane (100ml) and triethylamine (12ml, 86mmol) at 0°C. Benzylchloroformate (6.8ml, 47.3mmol) was added and the mixture was stirred for 20h at room temperature then solvents were removed *in vacuo.* The residue was redissolved in EtOAc, washed with 1M KHSO₄, water then brine, dried and concentrated *in vacuo* to yield the title compound (8.33g, 77%).

### Example E33.2

### 4-Formyl-piperidine-1-carboxylic acid benzyl ester

Dess-Martin reagent (17g, 39.6mmol) was added portionwise to a solution of 4-hydroxymethyl-piperidine-1-carboxylic acid benzyl ester from Example E33.1 (8.33g, 33mmol) in dichloromethane (100ml) at room temperature. The mixture was stirred for 3h under an inert atmosphere then diluted with chloroform and water and the layers were partitioned. The organic layer was washed with brine, dried and concentrated *in vacuo.* The residue was purified by flash chromatography on silica gel (eluant; 40% EtOAc:60% hexane) to yield the title compound (5.5g, 66%).

### Example E33.3

### 4-[1,3]Dioxolan-2-yl-piperidine-1-carboxylic acid benzyl ester

Ethylene glycol (5ml) and a catalytic amount of p-toluenesulfonic acid were added to 4-formyl-piperidine-1-carboxylic acid benzyl ester from Example E33.2 (5.6g, 22.6mmol) in toluene (100ml) and the mixture was heated at reflux under Dean and Stark conditions for 2.5h. Solvents were removed *in vacuo* and the residue was redissolved in EtOAc, washed with saturated NaHCO₃ then brine, dried and concentrated *in vacuo.* The residue was purified by flash chromatography on silica gel (eluant; 50% EtOAc:50% pet. ether) to yield the title compound (5.14g, 78%).

### Example E33.4

### 4-[1,3]Dioxolan-2-yl-piperidine

4-[1,3]Dioxolan-2-yl-piperidine-1-carboxylic acid benzyl ester from Example E33.3 (5.14g, 17.7mmol) was dissolved in methanol (100ml) and hydrogenated over 10% Pd/C catalyst (551mg) for 4h. The mixture was filtered through Celite® filter agent and the filtrate was concentrated *in vacuo* to yield the title compound (2.84g, 100%).

### Example E34

### 4-(3-Hydroxy-propyl)-piperazine-1-carboxylic acid tert-butyl ester

A solution of piperazine-1-carboxylic acid tert-butyl ester (26.5g, 142mmol) in acetone (300ml) at room temperature was treated with 3-bromo-propan-1-ol (14.5ml), 156.2mmol), potassium carbonate (50g, 361.8mmol) and potassium iodide (2.4g, 14.2mmol), and the mixture was heated at reflux for 18h then allowed to cool to room temperature. The suspension was filtered and the filtrate concentrated *in vacuo.* The residue was purified by flash chromatography on silica gel (eluant; 5% methanol:95% dichloromethane) to yield the title compound (27.7g, 80%).

### Example E35

### 1-Cyclopropylmethyl-imidazolidine ditrifluoroacetate

### Example E35.1

### [2-(Cyclopropylmethyl-amino)-ethyl]-carbamic acid tert-butyl ester

Potassium hydrogen carbonate (220mg, 2.2mmol) and (bromoethyl)cyclopropane (270mg, 2.0mmol) were added to a solution of tert-butyl-N-(2-aminoethyl)carbamate (320mg, 2.0mmol) in THF (10ml). The mixture was heated at 66°C for 20h and solvents were concentrated in *vacuo*. The residue was dissolved in chloroform, washed with water, dried and concentrated *in vacuo*. The residue was purified by flash chromatography on silica gel (eluant; 1% triethylamine:4% methanol:95% chloroform) to yield the title compound (130mg, 30%).

### Example E35.2

### 1-Cyclopropylmethyl-imidazolidine ditrifluoroacetate

Trifluoroacetic acid (5ml) was added to a solution of [2-(cyclopropylmethyl-amino)-ethyl]-carbamic acid tert-butyl ester from Example E35.1 (180mg, 0.84mmol) in dichloromethane (3ml) and the mixture was stirred for 75min at room temperature. Solvents were removed in *vacuo* and azeotroped with toluene. The residue was placed in water (10ml) and formaldehyde (37% w/w solution, 0.10ml, 1.36mmol) was added. The mixture was stirred for 6 days at room temperature, concentrated *in vacuo,* azeotroped with toluene then pet. ether to yield the title compound (255mg, 86%).

### Example E36

### 2-Piperazin-1-yl-1-p-tolyl-ethanone dihydrochloride

### Example E36.1

### 4-(2-Oxo-2-p-tolyl-ethyl)-piperazine-1-carboxylic acid tert-butyl ester

2-Bromo-4-methylacetophenone (572mg, 2.68mmol) was added to a solution of piperazine-1-carboxylic acid tert-butyl ester (500mg, 2.68mmol) in dichloromethane (5ml) and triethylamine (0.45ml, 3.22mmol). The mixture was stirred for 3 days at room temperature and solvents were removed *in vacuo.* The residue was purified by flash chromatography on silica gel (eluant; 40% hexane:60% EtOAc) to yield the title compound (661mg, 77%).

### Example E36.2

### 2-Piperazin-1-yl-1-p-tolyl-ethanone dihydrochloride

4-(2-Oxo-2-p-tolyl-ethyl)-piperazine-1-carboxylic acid tert-butyl ester from Example E36.1 (661mg, 2.08mmol) was dissolved in 4M HCl solution in dioxan (25ml) at 0°C and the mixture was stirred for 45min at room temperature. Solvents were concentrated *in vacuo* and azeotroped with diethyl ether to yield the title compound (536mg, 88%).

### Example E37

### 2-Bromo-1-[4-(3,3-dimethyl-butyl)-piperazin-1-yl]-ethanone

Bromoacetyl bromide (0.52ml, 6.0mmol) was added to a solution of 1-(3,3-dimethyl-butyl)-piperazine hydrochloride from Example E4 (1.5g, 5.7mmol) in dichloromethane (20ml) and triethylamine (3.57ml, 25.6mmol) at 0°C. The mixture was stirred for 20h at room temperature, washed with saturated NaHCO₃ dried and concentrated *in vacuo.* The residue was purified by flash chromatography on silica gel (eluant; 5% methanol:95% dichloromethane) to yield the title compound (220mg, 13%).

### Example E38

### Cyclopropyl-piperidin-4-ylmethyl-carbamic acid tert-butyl ester

### Example E38.1

### 4-Cyclopropylaminomethyl-piperidine-1-carboxylic acid benzyl ester

Cyclopropylamine (1.4g, 24.4mmol) and acetic acid (0.5ml) were added to a solution of 4-formyl-piperidine-1-carboxylic acid benzyl ester from Example E33.2 (5.5g, 22.2mmol) in methanol (49.5ml) and the mixture was stirred for 1h at room temperature. Sodium cyanoborohydride (1.84g, 28.9mmol) was added and the mixture was stirred for 20h at room temperature under an inert atmosphere. Solvents were removed *in vacuo* and azeotroped with toluene. The residue was dissolved in EtOAC, washed with saturated NaHCO₃ then brine, dried and concentrated *in vacuo.* The residue was purified by flash chromatography on silica gel (eluant; 10% methanol:90% dichloromethane) to yield the title compound (3.0g, 47%).

### Example E38.2

### 4-[(tert-Butoxycarbonyl-cyclopropyl-amino)-methyl]-piperidine-1-carboxylic acid benzyl ester

DMAP (127mg, 1.04mmol) and di-tert-butyl dicarbonate (3.4g, 12.5mmol) were added to a solution of 4-cyclopropylaminomethyl-piperidine-1-carboxylic acid benzyl ester from Example E38.1 (3.0g, 10.4mmol) in dichloromethane (50ml) and triethylamine (to pH9). The mixture was stirred for 20h at room temperature then concentrated *in vacuo.* The residue was dissolved in EtOAC, washed with saturated NaHCO₃ then brine, dried and concentrated *in vacuo.* The residue was purified by flash chromatography on silica gel (eluant; 40% EtOAc:60% hexane) to yield the title compound (3.67g, 92%).

### Example E38.3

### Cyclopropyl-piperidin-4-ylmethyl-carbamic acid tert-butyl ester

4-[(tert-Butoxycarbonyl-cyclopropyl-amino)-methyl]-piperidine-1-carboxylic acid benzyl ester from Example E38.2 (3.67g, 9.4mmol) was dissolved in methanol (100ml) and hydrogenated over 10% Pd/C catalyst (3g) for 1h. The mixture was filtered through Celite® filter agent and the filtrate was concentrated *in vacuo* to yield the title compound (2.07g, 87%).

### Example E39

### 4-[3-(4-Carboxy-2-fluoro-phenoxy)-propyl]-piperazine-1-carboxylic acid tert-butyl ester

### Example E39.1

### 4-[3-(4-Ethoxycarbonyl-2-fluoro-phenoxy)-propyl]-piperazine-1-carboxylic acid tert-butyl ester

Triphenylphosphine polystyrene (loading 1.0 mmol/g, 11g, 11.0mmol) and DEAD (2.45g, 11.0mmol) were added to a solution of 3-fluoro-4-hydroxy-benzoic acid ethyl ester from Example E27 (1.3g, 7.4mmol) and 4-(3-hydroxy-propyl)-piperazine-1-carboxylic acid tert-butyl ester from Example E34 (1.8g, 7.4mmol) in tetrahydrofuran (100ml) at 0°C. The suspension was allowed to warm to room temperature and stirred for 18h. The mixture was filtered and the filtrate concentrated *in vacuo.* The residue was purified by flash chromatography on silica gel (eluant; EtOAC) to yield the title compound (1.7g, 57%).

### Example E39.2

### 4-[3-(4-Carboxy-2-fluoro-phenoxy)-propyl]-piperazine-1-carboxylic acid tert-butyl ester

A solution of 4-[3-(4-ethoxycarbonyl-2-fluoro-phenoxy)-propyl]-piperazine-1-carboxylic acid tert-butyl ester from Example E39.1 (1.7g, 4.1 mmol) in dioxan (25ml) was treated with 2N NaOH (3ml) and the mixture stirred at 50°C for 18h. A further aliquot of 2N NaOH was added (2ml), and stirring continued at 50°C for 3h. Solvents were removed *in vacuo* and azeotroped with toluene. The residue was purified by flash chromatography on silica gel (eluant; 1% acetic acid:9% methanol:90% chloroform) to yield the title compound (1.45g, 92%).

### Example E40

### 4-{2-[4-(3,3-Dimethyl-butyl)-piperazin-1-yl]-ethoxy}-3-methyl-benzoic acid

### Example E40.1

### 4-[2-(4-Methoxycarbonyl-2-methyl-phenoxy)-ethyl]-piperazine-1-carboxylic acid tert-butyl ester

4-(2-Hydroxy-ethyl)-piperazine-1-carboxylic acid tert-butyl ester (500mg, 2.2mmol) was dissolved in THF (30ml) and cooled down to 0°C. Polymer-supported triphenylphosphine (2.2g, 2.2mmol), DEAD (378mg, 2.2mmol) and 4-hydroxy-3-methyl-benzoic acid methyl ester from Example E29 (361mg, 2.2mmol) were added and the mixture was stirred for 20h at room temperature. The resin was filtered off and the filtrate concentrated *in vacuo.* The residue was purified by flash chromatography on silica gel (eluant; EtOAc) to yield the title compound (495mg, 60%).

### Example E40.2

### 3-Methyl-4-(2-piperazin-1-yl-ethoxy)-benzoic acid methyl ester dihydrochloride

4-[2-(4-Methoxycarbonyl-2-methyl-phenoxy)-ethyl]-piperazine-1-carboxylic acid tert-butyl ester from Example E40.1 (200mg, 0.53mmol) was dissolved in 4M HCl solution in dioxan (5ml). The mixture was stirred for 30min at room temperature, concentrated *in vacuo* and azeotroped with toluene to yield the title compound (185mg, 100%).

### Example E40.3

### 4-{2-[4-(3,3-Dimethyl-butyl)-piperazin-1-yl]-ethoxy)-3-methyl-benzoic acid methyl ester

3,3-Dimethylbutyraldehyde (54mg, 0.53mmol) was added to a solution of 3-methyl-4-(2-piperazin-1-yl-ethoxy)-benzoic acid methyl ester dihydrochloride from Example E40.2 (146mg, 0.42mmol) in methanol/acetic acid (99:1, v/v, 10ml) and the mixture was stirred at room temperature for 1h. Sodium cyanoborohydride (42mg, 0.69mmol) was added, and the mixture was stirred at room temperature for 18h then concentrated *in vacuo.* The residue was dissolved in EtOAc, washed with saturated NaHCO₃, water then brine, dried and concentrated *in vacuo.* The residue was purified by flash chromatography on silica gel through an isolute (eluant; EtOAc) to yield the title compound (64mg, 34%).

### Example E40.4

### 4-{2-[4-(3,3-Dimethyl-butyl)-piperazin-1-yl]-ethoxy}-3-methyl-benzoic acid

4-{2-[4-(3,3-Dimethyl-butyl)-piperazin-1-yl]-ethoxy}-3-methyl-benzoic acid methyl ester from Example E40.3 (42mg, 0.12mmol) was dissolved in dioxan (5ml) and 1M NaOH (1ml) was added. The mixture was heated at reflux for 2h then solvents were concentrated *in vacuo.* The residue was dissolved in EtOAc, washed with saturated NaHCO₃, water then brine, dried and concentrated *in vacuo* to yield the title compound (40mg, 100%).

### Example E41

### 4-{2-[4-(3,3-Dimethyl-butyl)-piperazin-1-yl]-2-oxo-ethoxy}-3-methyl-benzoic acid

### Example E41.1

### 4-tert-Butoxycarbonylmethoxy-3-methyl-benzoic acid methyl ester

Potassium carbonate (4.6g, 33mmol), potassium iodide (0.25g, 1.5mmol) and tert-butylbromoacetate (2.5ml, 16.5mmol) were added to a solution of 4-hydroxy-3-methyl-benzoic acid methyl ester from Example E29 (2.5g, 15mmol) in acetone (150ml) and the mixture was heated at reflux for 20h. The solid was filtered off and the filtrate concentrated *in vacuo.* The residue was dissolved in EtOAc, washed with 1N KHSO₄, water then brine, dried and concentrated *in vacuo.* The residue was purified by flash chromatography on silica gel (eluant; 15% EtOAc:85% hexane) to yield the title compound (3.8g, 90%).

### Example E41.2

### 4-Carboxymethoxy-3-methyl-benzoic acid methyl ester

Trifluoroacetic acid (20ml) was added to a solution of 4-tert-butoxycarbonylmethoxy-3-methyl-benzoic acid methyl ester from Example E41.1 (3.8g, 13.6mmol) in dichloromethane (40ml) and the mixture was stirred for 1h at room temperature. Volatiles were removed *in vacuo* and azeotroped with dichloromethane to yield the title compound (3.04g, 100%).

### Example E41.3

### 4-{2-[4-(3,3-Dimethyl-butyl)-piperazin-1-yl]-2-oxo-ethoxy}-3-methyl-benzoic acid methyl ester

WSCD (5.14g, 27.2mmol) and DMAP (1.64g, 13.6mmol) were added to a solution of 4-carboxymethoxy-3-methyl-benzoic acid methyl ester from Example E41.2 (3.04g, 13.6mmol) in dichloromethane (100ml) and triethylamine (5ml). The mixture was stirred for 1h at room temperature then 1-(3,3-dimethyl-butyl)-piperazine dihydrochloride from Example E4(3.67g, 14.9mmol) was added. The mixture was stirred for 20h at room temperature then solvents were removed *in vacuo.* The residue was dissolved in EtOAc, washed with saturated NaHCO₃ then brine, dried and concentrated *in vacuo.* The residue was purified by flash chromatography on silica gel (eluant; EtOAc) to yield the title compound (2.8g, 55%).

### Example E41.4

### 4-{2-[4-(3,3-Dimethyl-butyl)-piperazin-1-yl]-2-oxo-ethoxy}-3-methyl-benzoic acid

1M Boron tribromide solution (2.66ml, 2.66mmol) was added dropwise to a solution of 4-{2-[4-(3,3-dimethylbutyl)-piperazin-1-yl]-2-oxo-ethoxy}-3-methyl-benzoic acid methyl ester from Example E41.3 (500mg, 1.33mmol) in dichloromethane (20ml) at 0°C under an inert atmosphere. The mixture was stirred for 20h at room temperature then cooled down to 0°C. 1M Boron tribromide solution (1.33ml, 1.33mmol) was added and the mixture was stirred for 2h at room temperature. Solvents were removed in *vacuo* and azeotroped with toluene. The residue was purified by flash chromatography on silica gel (eluant; 1% acetic acid:5% methanol:94% chloroform) to yield the title compound (350mg, 72%).

### Example E41a

### 4-(2-Bromo-acetyl)-piperazine-1-carboxylic acid tert-butyl ester

A solution of bromoacetyl bromide (8.5ml, 97mmol) in DCM (250ml) at 0°C was treated dropwise with a solution of 1-Boc-piperazine (15.9, 85.3mmol) and triethylamine (18.0ml, 130mmol) in DCM (150ml). After addition was complete, the mixture was stirred at room temperature for 4 h. The solution was washed with ice-cold 1M HCl, sat. aq. NaHCO3, dried and concentrated in vacuo. The residue was purified by flash chromatography on silica gel (eluant; 20% EtOAc:80% cyclohexane to 50% EtOAc:50% cyclohexane) to yield the title compound (15.7g, 60.0%).

### Example E41b

### 2-Chloro-4-hydroxy-benzoic acid methyl ester

### Example E41b.1

### 4-Amino-2-chloro-benzoic acid methyl ester

A solution of 4-Amino-2-chloro-benzoic acid (5.3g, 31mmol) in methanol (100 ml) was treated with acetyl chloride (5ml) and then heated at reflux for 16 hours. The solvents were evaporated in vacuo. The residue was dissolved in EtOAc, washed with saturated sodium hydrogen carbonate, dried and concentrated in vacuo to yield the title compound as a purple solid, 5.45g, 95%.

### Example E41b.2

### 2-Chloro-4-hydroxy-benzoic acid

4-Amino-2-chloro-benzoic acid methyl ester from Example E41b.1 (5.45g, 29.4mmol) was treated with a 35% solution of sulphuric acid (120ml) and the mixture was stirred and heated until dissolution then cooled to 0°C. Sodium nitrite (4.30g, 62.5mmol) in water (25ml) was added dropwise and the mixture was stirred for 15min at 0°C. Urea was added to destroy the excess nitrite. Copper nitrate (200mg, 0.83mmol) was added and heated to 90 °C. The reaction mixture was cooled to room temperature, extracted into ethyl acetate ×3, dried and concentrated in vacuo. The residue was purified by flash chromatography on silica gel (eluant; 30% EtOAc:70%. hexane to 50% EtOAc:49% hexane:1% AcOH) to yield the title compound (3.7g, 73%).

### Example E41b.3

### 2-Chloro-4-hydroxy-benzoic acid methyl ester

A solution of 2-chloro-4-hydroxy-benzoic acid (3.70 g, 21.4 mmol)) in methanol (50 ml) was treated with thionyl chloride (2.4 ml, 32 mmol) dropwise and stirred for 24 hours and the solvents were removed in vacuo. The residue was dissolved in ethyl acetate and washed with sat. sodium hydrogen carbonate, dried and solvents were removed in vacuo. The residue was purified by column chromatography on silica gel (eluant with 30% EtOAc:70% cyclohexane) to yield the desired product as a yellow solid, 3.68 g, 92%.

### Example E41c

### 4-[2-(4-Carboxy-3-chloro-phenoxy)-acetyl]-piperazine-1-carboxylic acid tert-butyl ester

### Example E41c.1

### 4-[2-(3-Chloro-4-methoxycarbonyl-phenoxy)-acetyl]-piperazine-1-carboxylic acid tert-butyl ester

2-Chloro-4-hydroxy-benzoic acid methyl ester from example E41b (2.0g, 10.9mmol) and 4-(2-Bromo-acetyl)-piperazine-1-carboxylic acid tert-butyl ester from Example E41a (3.68g, 12.0mmol) in acetonitrile (30ml) were treated with potassium carbonate (1.6g, 11.5mmol), and the mixrure heated at reflux 18 h. before the solvents were removed in vacuo. The residue was adsorbed onto silica gel and purified by flash chromatography on silica gel (eluant; 20% EtOAc:80% cyclohexane to 50% EtOAc:50% cyclohexane to 70% EtOAc:30% cyclohexane) to yield the title compound (4.5g, 100%).

### Example E41c.2

### 4-[2-(4-Carboxy-3-chloro-phenoxy)-acetyl]-piperazine-1-carboxylic acid tert-butyl ester

A solution of 4-[2-(3-Chloro-4-methoxycarbonyl-phenoxy)-acetyl]-piperazine-1-carboxylic acid tert-butyl ester from Example E41c.1 (4.5g, 10.8mmol) in THF at 0°C was treated with potassium trimethylsilanolate (1.6g, 10.9mmol), and the mixture stirred at room temperature for 18 h. A further 1.6g (10.9mmol) potassium silanolate was added, and stirring continued at room temperatuire for 2h. A further 1.6g (10.9mmol) potassium silanolate was added, and the mixture was stirred at 45°C for 2h. The mixture was then diluted with water and THF was removed in vacuo. The residue was washed with ether, cooled in an ice-bath and adjusted to pH5 with solid KHSO₄ whereupon it was extracted with CHCl₃ and CHCl₃/isopropanol (90:10 v/v). The combined organics were dried and solvents were removed in vacuo to yield the title compound (3.7g, 85%).

### Example E42

### 4-(3-tert-Butoxycarbonyl-propoxy)-3-methyl-benzoic acid

### Example E42.1

### 4-Bromo-butyric acid tert-butyl ester

1M Boron tribromide (39ml, 39mmol) was added to a solution of butyrolactone (3ml, 39mmol) in dichloromethane (30ml). The mixture was stirred for 20h at room temperature then quenched with an excess of tert-butylalcohol. The mixture was stirred for 2h at room temperature, diluted with dichloromethane, washed with saturated NaHCO₃, saturated Na₂S₂O₃ then brine, dried and concentrated *in vacuo.* The residue was purified by flash chromatography on silica gel through an isolute (eluant; 5% EtOAc:95% hexane) to yield the title compound (3g, 34%).

### Example E42.2

### 4-(3-tert-Butoxycarbonyl-propoxy)-3-methyl-benzoic acid methyl ester

Potassium carbonate (580mg, 4.20mmol) and potassium iodide (72mg, 0.43mmol) were added to a solution of 4-hydroxy-3-methyl-benzoic acid methyl ester from Example E29 (293mg, 1.76mmol) and 4-bromo-butyric acid tert-butyl ester from Example E42.1 (397mg, 1.78mmol) in acetone (50ml). The mixture was heated at reflux for 18h then the solid was filtered off and the filtrate concentrated in *vacuo.* The residue was dissolved in EtOAc, washed with water then brine, dried and concentrated *in vacuo.* The residue was purified by flash chromatography on silica gel (eluant; 20% EtOAc:80% pet. ether) to yield the title compound (414mg, 76%).

### Example E42.3

### 4-(3-tert-Butoxycarbonyl-propoxy)-3-methyl-benzoic acid

Lithium hydroxide monohydrate (137mg, 3.26mmol) was added to a solution of 4-(3-tert-butoxycarbonyl-propoxy)-3-methyl-benzoic acid methyl ester from Example E42.2 (414mg, 1.34mmol) in THF (10ml) and water (5ml). The mixture was stirred for 48h at room temperature and concentrated *in vacuo.* The residue was dissolved in chloroform, acidified with 1M HCl then washed with brine, dried and concentrated *in vacuo* to yield the title compound (275mg, 70%).

### Example E43

### 4-{4-[4-(3,3-Dimethyl-butyl)-piperazin-1-yl]-4-oxo-butyl}-3-methyl-benzoic acid

### Example E43.1

### 4-{4-[4-(3,3-Dimethyl-butyl)-piperazin-1-yl]-4-oxo-butyl}-3-methyl-benzoic acid methyl ester

HBTU (910mg, 2.4mmol) was added to a solution of 4-(3-carboxy-propyl)-3-methyl-benzoic acid methyl ester from Example E30 (378mg, 1.6mmol) and 1-(3,3-dimethyl-butyl)-piperazine hydrochloride from Example E4 (467mg, 1.9mmol) in dichloromethane (15ml) and DIEA (0.836ml, 4.8mmol). The mixture was stirred for 20h at room temperature, washed with saturated NaHCO₃ then brine, dried and concentrated in vacuo. The residue was purified by flash chromatography on silica gel through an isolute (eluant; 5% methanol:95% dichloromethane) to yield the title compound (582mg, 94%).

### Example E43.2

### 4-{4-[4-(3,3-Dimethyl-butyl)-piperazin-1-yl]-4-oxo-butyl}-3-methyl-benzoic acid

Lithium hydroxide monohydrate (108mg, 2.6mmol) was added to a solution of 4-{4-[4-(3,3-dimethyl-butyl)-piperazin-1-yl]-4-oxo-butyl}-3-methyl-benzoic acid methyl ester from Example E43.1 (500mg, 1.3mmol) in THF (12ml) and water (6ml). The mixture was stirred for 20h at room temperature then solvents were removed in vacuo. The residue was purified by flash chromatography on silica gel (eluant; 2% acetic acid:4% methanol:94% dichloromethane) then recrystallised from chloroform and pet. ether to yield the title compound (439mg, 91%).

### Example E44

### 4-{3-[4-(3,3-Dimethyl-butyl)-piperazin-1-yl]-3-oxo-propyl}-3-methyl-benzoic acid

### Example E44.1

### 4-{3-[4-(3,3-Dimethyl-butyl)-piperazin-1-yl]-3-oxo-propyl}-3-methyl-benzoic acid methyl ester

Oxalyl chloride (2.6ml, 30mmol) was added slowly to a solution of 4-(2-carboxy-ethyl)-3-methyl-benzoic acid methyl ester from Example E30.3 (5.33g, 24mmol) in dichloromethane (60ml) and few drops of DMF at 0°C. The mixture was stirred for 2h at room temperature then concentrated *in vacuo* and azeotroped with toluene. The residue was dissolved in dichloromethane (30ml) and added to a solution of 1-(3,3-dimethyl-butyl)-piperazine hydrochloride from Example E4 (6.3g, 26mmol) in dichloromethane (45ml) and DIEA (17ml, 96mmol) at 0°C. The mixture was stirred for 1h at room temperature then concentrated *in vacuo.* The residue was purified by flash chromatography on silica gel (eluant; 5% metanol:95% dichloromethane) to yield the title compound (8.5g, 96%).

### Example E44.2

### 4-{3-[4-(3,3-Dimethyl-butyl)-piperazin-1-yl]-3-oxopropyl}-3-methyl-benzoic acid

Lithium hydroxide monohydrate (2.4g, 56.7mmol) was added to a solution of 4-{3-[4-(3,3-dimethyl-butyl)-piperazin-1-yl]-3-oxo-propyl}-3-methyl-benzoic acid methyl ester from Example E44.1 (8.5g, 22.7mmol) in THF (200ml) and water (100ml). The mixture was stirred for 24h at room temperature then solvents were removed in *vacuo.* The residue was purified by flash chromatography on silica gel (eluant; 2% acetic acid:4% methanol:94% dichloromethane) to yield the title compound (8.1g, 99%).

### Example E45

### 3-[2-Methyl-4-(3-methyl-4,10-dihydro-3H-2,3,4,9-tetraaza-benzo[f]azulene-9-carbonyl)-phenyl]-propionic acid

### Example E45.1

### 4-((E)-2-tert-Butoxycarbonyl-vinyl)-3-methyl-benzoic acid

Lithium hydroxide monohydrate (384mg, 9.2mmol) was added to a solution of 4-((E)-2-tert-butoxycarbonylvinyl)-3-methyl-benzoic acid methyl ester from Example E30.1 (1.27g, 4.6mmol) in THF (50ml) and water (20ml). The mixture was stirred for 20h at room temperature then solvents were removed *in vacuo.* The residue was dissolved in EtOAc, washed with 1M KHSO₄, water then brine, dried and concentrated *in vacuo* to yield the title compound (1.1g, 92%).

### Example E45.2

### (E)-3-[2-Methyl-4-(3-methyl-4,10-dihydro-3H-2,3,4,9-tetraaza-benzo[f]azulene-9-carbonyl)-phenyl]-acrylic acid tert-butyl ester

4-((E)-2-tert-Butoxycarbonyl-vinyl)-3-methyl-benzoic acid from Example E45.1 (1.1g, 4.2mmol) and 3-methyl-3,4,9,10-tetrahydro-2,3,4,9-tetraaza-benzo[f]azulene from Example E11 (720mg, 3.6mmol) were dissolved in dichloromethane (50ml) and triethylamine (0.75ml). DMAP (4.45g, 3.6mmol) and WSCD (1.36g, 7.1mmol) were added and the mixture was heated at reflux for 2 days. The solution was diluted with dichloromethane, washed with 0.3M KHSO₄, saturated NaHCO₃ then brine, dried and concentrated *in vacuo.* The residue was purified by flash chromatography on silica gel (eluant; EtOAc) to yield the title compound (1.35g, 82%).

### Example E45.3

### 3-[2-Methyl-4-(3-methyl-4,10-dihydro-3H-2,3,4,9-tetraaza-benzo[f]azulene-9-carbonyl)-phenyl]-propionic acid tert-butyl ester

(E)-3-[2-Methyl-4-(3-methyl-4,10-dihydro-3H-2,3,4,9-tetraaza-benzo[f]azulene-9-carbonyl)-phenyl]-acrylic acid tert-butyl ester from Example E45.2 (436mg, 0.99mmol) was dissolved in methanol (40ml) and hydrogenated over 10% Pd/C catalyst (91mg) for 5h. The mixture was filtered through Celite® filter agent and the filtrate was concentrated in *vacuo* and azeotroped with dichloromethane to yield the title compound (426mg, 96%).

### Example E45.4

### 3-[2-Methyl-4-(3-methyl-4,10-dihydro-3H-2,3,4,9-tetraaza-benzo[f]azulene-9-carbonyl)-phenyl]-propionic acid

Trifluoroacetic acid (5ml) was added to a solution of 3-[2-methyl-4-(3-methyl-4,10-dihydro-3H-2,3,4,9-tetraaza-benzo[f]azulene-9-carbonyl)-phenyl]-propionic acid tert-butyl ester from Example E45.3 (413mg, 0.92mmol) in dichloromethane (10ml) and the mixture was stirred for 45min at room temperature. Solvents were removed *in vacuo* and azeotroped with dichloromethane. The residue was crystallised with MeOH/Et₂O to yield the title compound (322mg, 90%).

### Example E46

### (E)-3-[2-Methyl-4-(3-methyl-4,10-dihydro-3H-2,3,4,9-tetraaza-benzo[f]azulene-9-carbonyl)-phenyl]-acrylic acid

Trifluoroacetic acid (5ml) was added to a solution of (E)-3-[2-methyl-4-(3-methyl-4,10-dihydro-3H-2,3,4, 9-tetraaza-benzo[f]azulene-9-carbonyl)-phenyl]-acrylic acid tert-butyl ester from Example E45.2 (338mg, 0.76mmol) in dichloromethane (10ml) and the mixture was stirred for 45min at room temperature. Solvents were removed *in* vacuo and azeotroped with dichloromethane to yield the title compound (337mg, 80%).

### Example E47

### 3-[2-Methyl-4-(3-methyl-4,10-dihydro-3H-2,3,4,9-tetraaza-benzo[f]azulene-9-carbonyl)-phenyl]-1-piperazin-1-yl-propan-1-one

### Example E47.1

### 4-{3-[2-Methyl-4-(3-methyl-4,10-dihydro-3H-2,3,4,9-tetraaza-benzo[f]azulene-9-carbonyl)-phenyl]-propionyl}-piperazine-1-carboxylic acid tert-butyl ester

PyBroP (1.54g, 3.3mmol) was added to a solution of 3-[2-methyl-4-(3-methyl-4,10-dihydro-3H-2,3,4,9-tetraaza-benzo[f]azulene-9-carbonyl)-phenyl]-propionic acid from Example E45 (850mg, 2.25mmol) and DMAP (275mg, 2.25mmol) in dichloromethane (20ml) and DIEA (0.90ml, 5.2mmol). The mixture was stirred for 15 mins at room temperature then 3-methyl-3,4,9,10-tetrahydro-2,3,4,9-tetraaza-benzo[f]azulene from Example E11 (450mg, 2.25mmol) was added. The mixture was heated at reflux for 18h, diluted with dichloromethane, washed with 0.3M KHSO₄ solution then saturated NaHCO₃, dried and concentrated *in vacuo.* The residue was purified by flash chromatography on silica gel (eluant; 5% methanol:95% EtOAc) to yield the title compound (1.09g, 87%).

### Example E47.2

### 3-[2-Methyl-4-(3-methyl-4,10-dihydro-3H-2,3,4,9-tetraaza-benzo[f]azulene-9-carbonyl)-phenyl]-1-piperazin-1-yl-propan-1-one

4-{3-[2-Methyl-4-(3-methyl-4,10-dihydro-3H-2,3,4,9-tetraaza-benzo[f]azulene-9-carbonyl)-phenyl]-propionyl}-piperazine-1-carboxylic acid tert-butyl ester from Example E47.1 (1.09g, 1.95mmol) was dissolved in methanol (20ml) and 4M HCl/dioxan solution (20ml) was added. The mixture was stirred for 3h at room temperature, concentrated in *vacuo* and azeotroped with toluene. The residue was dissolved in methanol (30ml) and ammonia (5ml) then concentrated in *vacuo.* The residue was purified by flash chromatography on silica gel (eluant; 0.5% 35% ammonia:4.5% methanol:95% dichloromethane to 1% 35% ammonia:9% methanol:90% dichloromethane) to yield the title compound (759mg, 73%).

### Example E48

### {4-[3-(4-Cyclopropyl-piperazin-1-yl)-propoxy]-3-fluorophenyl}-(3-methyl-4,10-dihydro-3H-2,3,4,9-tetraaza-benzo[f]azulen-9-yl)-methanone

### Example E48.1

### 4-(3-Bromo-propoxy)-3-fluoro-benzoic acid ethyl ester

1,3-Dibromopropane (2.2g, 11.0mmol), potassium carbonate (1.86g, 13.6mmol) and potassium iodide (90mg, 0.5mmol) were added to 3-fluoro-4-hydroxy-benzoic acid ethyl ester from Example E27 (1.0g, 5.4mmol) in acetone (25ml) and the mixture was heated at reflux for 20h. The solid was filtered off and washed with EtOAc. The filtrate was concentrated *in vacuo* and azeotroped with toluene. The residue was purified by flash chromatography on silica gel (eluant; 20% EtOAc:80% hexane) to yield the title compound (1.18g, 71%).

### Example E48.2

### 4-[3-(4-Cyclopropyl-piperazin-1-yl)-propoxy]-3-fluoro-benzoic acid ethyl ester

1-Cyclopropyl-piperazine dihydrochloride (642mg, 3.2mmol, according to the procedure described by G.S. Poindexter, M.A. Bruce, K.L. Le Boulluec, I. Monkovic, Tet. Lett., 35(44), 7331-7334, 1994), potassium carbonate (2.1g, 15.2mmol) and potassium iodide (50mg, 0.3mmol) were added to 4-(3-bromo-propoxy)-3-fluoro-benzoic acid ethyl ester from Example E48.1 (1.2g, 3.9mmol) in acetone (25ml). The mixture was heated at reflux for 20h. The solid was filtered off, washed with EtOAc and the filtrate was concentrated *in vacuo.* The residue was purified by flash chromatography on silica gel (eluant; EtOAc then 1% 35% ammonia:9% methanol:90% dichloromethane) to yield the title compound (1.0g, 89%).

### Example E48.3

### 4-[3-(4-Cyclopropyl-piperazin-1-yl)-propoxy]-3-fluoro-benzoic acid

4-[3-(4-Cyclopropyl-piperazin-1-yl)-propoxy]-3-fluoro-benzoic acid ethyl ester from Example E48.2 (1.0g, 2.9mmol) was dissolved in dioxan (10ml) and 2N NaOH solution (3ml, 6.0mmol) was added. The mixture was heated at 60°C for 18h then concentrated *in vacuo.* The residue was purified by flash chromatography on silica gel (eluant; 1% 35% ammonia:19% methanol:80% dichloromethane) to yield the title compound (920mg, 100%).

### Example E48.4

### {4-[3-(4-Cyclopropyl-piperazin-1-yl)-propoxy]-3-fluoro-phenyl}-(3-methyl-4,10-dihydro-3H-2,3,4,9-tetraaza-benzo[f]azulen-9-yl)-methanone

HBTU (325mg, 0.9mmol) and 4M HCl in dioxan (0.45ml, 1.8mmol) were added to a solution of 4-[3-(4-cyclopropyl-piperazin-1-yl)-propoxy]-3-fluoro-benzoic acid from Example E48.3 (230mg, 0.7mmol) in DMF (5ml). The mixture was stirred at room temperature for 30min. 3-Methyl-3,4,9,10-tetrahydro-2,3,4,9-tetraaza-benzo[f]azulene from Example E65 (186mg, 0.9mmol) and DIEA (pH=9) were added and the mixture was stirred at room temperature for 18h. The mixture was heated at 60°C for 3h then HBTU (300mg, 0.8mmol) was added. The mixture was heated at 60°C for 2 days then solvents were removed *in vacuo* and azeotroped with toluene. The residue was redissolved in EtOAc, washed with saturated NaHCO₃ then brine, dried and concentrated in *vacuo.* The residue was purified by preparative HPLC (eluant; 0.5% 35% ammonia:9.5% methanol:90% dichloromethane) to give a brown solid identified as the title compound (55mg, 15%).

### Example E49

### [3-Fluoro-4-(3-piperazin-1-yl-propoxy)-phenyl]-(3-methyl-4,10-dihydro-3H-2,3,4,9-tetraaza-benzo[f]azulen-9-yl)-methanone dihydrochloride

### Example E49.1

### 4-{3-[2-Fluoro-4-(3-methyl-4,10-dihydro-3H-2,3,4,9-tetraaza-benzo[f]azulene-9-carbonyl)-phenoxy]-propyl}-piperazine-1-carboxylic acid tert-butyl ester

A solution of 4-[3-(4-carboxy-2-fluoro-phenoxy)-propyl]-piperazine-1-carboxylic acid tert-butyl ester from Example E39 (865mg, 2.3mmol) in dichloromethane (50ml) was treated with triethylamine (to pH9), WSCD (865mg, 4.5mmol) and DMAP (276mg, 2.3mmol), and the mixture was stirred at room temperature for 10min. 3-Methyl-3,4,9,10-tetrahydro-2,3,4,9-tetraaza-benzo[f]azulene from Example E11 (541mg, 2.7mmol) was added. The mixture was heated at reflux for 18h then concentrated *in vacuo.* The residue was dissolved in EtOAc, washed with saturated NaHCO₃ then brine and concentrated *in vacuo.* The residue was purified by flash chromatography on silica gel (eluant; 10% methanol:90% dichloromethane) to yield the title compound (615mg, 48%).

### Example E49.2

### [3-Fluoro-4-(3-piperazin-1-yl-propoxy)-phenyl]-(3-methyl-4,10-dihydro-3H-2,3,4,9-tetraaza-benzo[f]azulen-9-yl)-methanone dihydrochloride

A solution of 4-{3-[2-fluoro-4-(3-methyl-4,10-dihydro-3H-2,3,4,9-tetraaza-benzo[f]azulene-9-carbonyl)-phenoxy]-propyl}-piperazine-1-carboxylic acid tert-butyl ester from Example E49.1 (615 mg, 1.09mmol) in methanol (2ml) at 0°C was treated with 4N HCl in dioxan (5ml). The solution was allowed to warm to room temperature and stirred for 1h. Solvents were removed in vacuo to give a white foam identified as the title compound (585 mg, 100%).

### Example E50

### (4-{3-[4-(3,3-Dimethyl-butyl)-piperazin-1-yl]-propoxy}-3-fluoro-phenyl)-(3-methyl-4,10-dihydro-3H-2,3,4,9-tetraaza-benzo[f]azulen-9-yl)-methanone bis(trifluoroacetate)

3,3-Dimethylbutyraldehyde (120mg, 1.20mmol) and triethylamine (to pH9) were added to a solution of [3-fluoro-4-(3-piperazin-1-yl-propoxy)-phenyl]-(3-methyl-4,10-dihydro-3H-2,3,4,9-tetraaza-benzo[f]azulen-9-yl)-methanone dihydrochloride from Example E49 (585mg, 1.09mmol) in methanol/acetic acid (99:1, v/v, 20ml) and the mixture was stirred at room temperature for 1h. Sodium cyanoborohydride (103mg, 1.30mmol) was added and the mixture was stirred at room temperature for 18h then concentrated *in vacuo.* The residue was purified by flash chromatography on silica gel (eluant; 10% methanol:90% dichloromethane) to yield (4-{3-[4-(3,3-dimethyl-butyl)-piperazin-1-yl]-propoxy}-3-fluoro-phenyl)-(3-methyl-4,10-dihydro-3H-2,3,4,9-tetraaza-benzo[f]azulen-9-yl)-methanone, which was lyophilised from aqueous trifluoroacetic acid to give a white solid identified as the title compound (700mg, 83%).

### Example E51

### (3,6-Dimethyl-4,10-dihydro-3H-2,3,4,9-tetraaza-benzo[f]azulen-9-yl)-{4-[3-(1-isobutyl-piperidin-4-yl)-propoxy]-3-methyl-phenyl}-methanone

A solution of isobutyraldehyde (0.36mg, 0.005mmol) in 1,2-dichloroethane (0.05ml) was added to a solution of (3,6-dimethyl-4,10-dihydro-3H-2,3,4,9-tetraaza-benzo[f]azulen-9-yl)-[3-methyl-4-(3-piperidin-4-yl-propoxy)-phenyl]-methanone hydrochloride(2.6mg, 0.005mmol) in 1,2-dichloroethane (0.05ml) and DIEA (0.0026ml). The mixture was stirred at room temperature for 1h then a solution of sodium triacetoxyborohydride (1.59mg, 0.0075mmol) in DMF (0.05ml) was added. The mixture was stirred for 20h at room temperature then solvents were removed *in vacuo* to yield the title compound. (ESI)+: [M+H]+= 530.5

### Example E52

### (6-Chloro-3-methyl-4,10-dihydro-3H-2,3,4,9-tetraaza-benzo[f]azulen-9-yl)-{2-fluoro-4-[3-(4-hexyl-piperazin-1-yl)-propoxy]-phenyl}-methanone

A solution of 1-bromohexane (0.83mg, 0.005mmol) in DMF(0.05ml) was added to a solution of (6-chloro-3-methyl-4,10-dihydro-3H-2,3,4,9-tetraaza-benzo[f]azulen-9-yl)-[2-fluoro-4-(3-piperazin-1-yl-propoxy)-phenyl]-methanone dihydrochloride from Example E5a (2.85mg, 0.005mmol) in DMF (0.05ml) and triethylamine (0.0021ml). The mixture was stirred for 20h at room temperature then solvents were removed in *vacuo* to yield the title compound. (ESI)+: [M+H]+= 583.6

### Example E53

### 1-(4-{3-[2-Methyl-4-(3-methyl-4,10-dihydro-3H-2,3,4,9-tetraaza-benzo[f]azulene-9-carbonyl)-phenoxy]-propyl}-piperazin-1-yl)-ethanone

A solution of acetyl chloride (0.39mg, 0.005mmol) in dichloromethane (0.05ml) was added to a solution of (3-methyl-4,10-dihydro-3H-2,3,4,9-tetraaza-benzo[f]azulen-9-yl)-[3-methyl-4-(3-piperazin-1-yl-propoxy)-phenyl]-methanone dihydrochloride (Compound number 408) (2.67mg, 0.005mmol) in dichloromethane (0.05ml) and triethylamine (0.0035ml). The mixture was stirred at room temperature for 1h then solvents were removed in *vacuo* to yield the title compound. (ESI)+: [M+H]+= 503.2

### Example E54

### {4-[3-(4-Methanesulfonyl-piperazin-1-yl)-propoxy]-3-methyl-phenyl}-(3-methyl-4,10-dihydro-3H-2,3,4,9-tetraaza-benzo[f]azulen-9-yl)-methanone

A solution of methanesulfonyl chloride (0.57mg, 0.005mmol) in dichloromethane (0.05ml) was added to a solution of (3-methyl-4,10-dihydro-3H-2,3,4,9-tetraaza-benzo[f]azulen-9-yl)-[3-methyl-4-(3-piperazin-1-yl-propoxy)-phenyl]-methanone dihydrochloride (Compound number 408) (2.67mg, 0.005mmol) in dichloromethane (0.05ml) and triethylamine (0.0035ml). The mixture was stirred at room temperature for 1h then solvents were removed *in vacuo* to yield the title compound. (ESI)+: [M+H]+= 539.2

### Example E55

### 1-(4-[1,3]Dioxolan-2-yl-piperidin-1-yl)-3-[2-methyl-4-(3-methyl-4,10-dihydro-3H-2,3,4,9-tetraaza-benzo[f]azulene-9-carbonyl)-phenyl]-propan-1-one

HBTU (327mg, 0.86mmol) was added to a solution of 4-[1,3]dioxolan-2-yl-piperidine from Example E33 (143mg, 0.91mmol) and 3-[2-methyl-4-(3-methyl-4,10-dihydro-3H-2,3,4,9-tetraaza-benzo[f]azulene-9-carbonyl)-phenyl]-propionic acid from Example E45 (320mg, 0.82mmol) in dichloromethane (20ml) and DIEA (0.7ml, 4.02mmol) and the mixture was stirred for 20h at room temperature. Dichloromethane was added and the solution was washed with saturated NaHCO₃ then brine, dried and concentrated *in vacuo.* The residue was purified by flash chromatography on silica gel (eluant; 4% methanol:96% dichloromethane to 8% methanol:92% dichloromethane) to give a white solid identified as the title compound (359mg, 83%).

### Example E56

### 1-[4-(Furan-2-carbonyl)-piperazin-1-yl]-3-[2-methyl-4-(3-methyl-4,10-dihydro-3H-2,3,4,9-tetraaza-benzo[f]azulene-9-carbonyl)-phenyl]-propan-1-one

A solution of HBTU (1.90mg, 0.005mmol) in DMF (0.05ml) was added to a solution of 3-[2-methyl-4-(3-methyl-4,10-dihydro-3H-2,3,4,9-tetraaza-benzo[f]azulene-9-carbonyl)-phenyl]-propionic acid from Example E45 (1.95mg, 0.005mmol) in DMF (0.05ml) and DIEA (0.0022ml). The mixture was stirred at room temperature for 30min then a solution of 1-(2-furoyl)piperazine (0.90mg, 0.005mmol) in DMF (0.05ml) was added. The mixture was stirred for 18h at room temperature then solvents were removed *in vacuo* to yield the title compound. (ESI)+: [M+H]+= 553.4

### Example E57

### (E)-3-[2-Methyl-4-(3-methyl-4,10-dihydro-3H-2,3,4,9-tetraaza-benzo[f]azulene-9-carbonyl)-phenyl]-1-[4-(2-piperidin-1-yl-ethyl)-piperazin-1-yl]-propenone

A solution of HBTU (1.90mg, 0.005mmol) in DMF (0.05ml) was added to a solution of (E)-3-[2-Methyl-4-(3-methyl-4,10-dihydro-3H-2,3,4,9-tetraaza-benzo[f]azulene-9-carbonyl)-phenyl]-acrylic acid from Example E46 (1.95mg, 0.005mmol) in DMF (0.05ml) and DIEA (0.0022ml). The mixture was stirred at room temperature for 30min then a solution of 1-(2-Piperidin-1-yl-ethyl)-piperazi
ne (0.99mg, 0.005mmol) in DMF (0.05ml) was added. The mixture was stirred for 18h at room temperature then solvents were removed *in vacuo* to yield the title compound.

### Example E58

### 1-{4-[(Butyl-methyl-amino)-methyl]-piperidin-1-yl}-3-[2-methyl-4-(3-methyl-4,10-dihydro-3H-2,3,4,9-tetraaza-benzo[f]azulene-9-carbonyl)-phenyl]-propan-1-one

### Example E58.1

### 1-{3-[2-Methyl-4-(3-methyl-4,10-dihydro-3H-2,3,4,9-tetraaza-benzo[f]azulene-9-carbonyl)-phenyl]-propionyl}-piperidine-4-carbaldehyde

Pyridinium p-toluenesulfonate (85mg, 0.34mmol) was added to a solution of 1-(4-[1,3]dioxolan-2-yl-piperidin-1-yl)-3-[2-methyl-4-(3-methyl-4,10-dihydro-3H-2,3,4,9-tetraaza-benzo[f]azulene-9-carbonyl)-phenyl]-propan-1-one from Example E55 (614mg, 1.16mmol) in acetone (20ml) and water (20ml). The mixture was heated at reflux for 7 days then solvents were removed *in vacuo.* The residue was redissolved in EtOAc, washed with 0.3M KHSO₄ then brine, dried, concentrated *in vacuo* then crystallised with chloroform and pet. ether to yield the title compound (451mg, 80%).

### Example E58.2

### 1-{4-[(Butyl-methyl-amino)-methyl]-piperidin-1-yl}-3-[2-methyl-4-(3-methyl-4,10-dihydro-3H-2,3,4,9-tetraaza-benzo[f]azulene-9-carbonyl)-phenyl]-propan-1-one

A solution of 1-{3-[2-methyl-4-(3-methyl-4,10-dihydro-3H-2,3,4,9-tetraaza-benzo[f]azulene-9-carbonyl)-phenyl]-propionyl}-piperidine-4-carbaldehyde from Example E58.1 (2.43mg, 0.005mmol) in 1,2-dichloroethane (0.05ml) was added to a solution of butyl-methyl-amine (0.43mg, 0.005mmol) in 1,2-dichloroethane (0.05ml) and the mixture was heated at 50°C for 3h then stirred at room temperature for 2 days. A solution of sodium triacetoxyborohydride (1.59mg, 0.0075mmol) in DMF (0.05ml) was added and the mixture was stirred for 20h at room temperature. Solvents were removed *in vacuo* to yield the title compound. MS: (ESI)⁺:[M+H]⁺= 557.6

### Example E59

### 3-[2-Methyl-4-(3-methyl-4,10-dihydro-3H-2,3,4,9-tetraaza-benzo[f]azulene-9-carbonyl)-phenyl]-1-[4-(2-morpholin-4-yl-ethylamino)-piperidin-1-yl]-propan-1-one

A solution of 1-{3-[2-methyl-4-(3-methyl-4,10-dihydro-3H-2,3,4,9-tetraaza-benzo[f]azulene-9-carbonyl)-phenyl]-propionyl}-piperidin-4-one (Compound number 701) (2.36mg, 0.005mmol) in 1,2-dichloroethane (0.05ml) was added to a solution of 2-morpholin-4-yl-ethylamine (0.65mg, 0.005mmol) in 1,2-dichloroethane (0.05ml) and the mixture was heated at 50°C for 3h then stirred at room temperature for 2 days. A solution of sodium triacetoxyborohydride (1.59mg, 0.0075mmol) in DMF (0.05ml) was added and the mixture was stirred for 20h at room temperature. Solvents were removed *in vacuo* to yield the title compound. MS: (ESI)⁺:[M+H]⁺= 586.6

### Example E60

### 1-(4-Hexyl-piperazin-1-yl)-3-[2-methyl-4-(3-methyl-4,10-dihydro-3H-2,3,4,9-tetraaza-benzo[f]azulene-9-carbonyl)-phenyl]-propan-1-one

A solution of 1-bromohexane (0.83mg, 0.005mmol) in DMF(0.05ml) was added to a solution of 3-[2-methyl-4-(3-methyl-4,10-dihydro-3H-2,3,4,9-tetraaza-benzo[f]azulene-9-carbonyl)-phenyl]-1-piperazin-1-yl-propan-1-one from Example E47 (2.29mg, 0.005mmol) in DMF (0.05ml) and triethylamine (0.0021ml). The mixture was stirred for 20h at room temperature then solvents were removed *in vacuo* to yield the title compound. (ESI)+: [M+H]+= 543.4

### Example E61

### 3-[4-(3,6-Dimethyl-4,10-dihydro-3H-2,3,4,9-tetraaza-benzo[f]azulene-9-carbonyl)-2-methyl-phenyl]-1-(4-propyl-piperazin-1-yl)-propan-1-one

A solution of propionaldehyde (0.29mg, 0.005mmol) in 1,2-dichloroethane (0.05ml) was added to a solution of 3-[4-(3,6-dimethyl-4,10-dihydro-3H-2,3,4,9-tetraaza-benzo[f]azulene-9-carbonyl)-2-methyl-phenyl]-1-piperazin-1-yl-propan-1-one (2.36mg, 0.005mmol) in 1,2-dichloroethane (0.05ml) and DIEA (0.0026ml). The mixture was stirred at room temperature for 1h then a solution of sodium triacetoxyborohydride (1.59mg, 0.0075mmol) in DMF (0.05ml) was added. The mixture was stirred for 20h at room temperature then solvents were removed *in vacuo* to yield the title compound. (ESI)+: [M+H]+= 515.5

### Example E62

### 1-(4-Acetyl-piperazin-1-yl)-3-[4-(6-chloro-3-methyl-4,10-dihydro-3H-2,3,4,9-tetraaza-benzo[f]azulene-9-carbonyl)-2-methyl-phenyl]-propan-1-one

A solution of acetyl chloride (0.39mg, 0.005mmol) in dichloromethane (0.05ml) was added to a solution of 3-[4-(6-chloro-3-methyl-4,10-dihydro-3H-2,3,4,9-tetraaza-benzo[f]azulene-9-carbonyl)-2-methyl-phenyl]-1-piperazin-1-yl-propan-1-one (2.47mg, 0.005mmol) in dichloromethane (0.05ml) and triethylamine (0.0035ml). The mixture was stirred at room temperature for 20h then solvents were removed in *vacuo* to yield the title compound. (ESI)+: [M+H]+= 535.5

### Example E63

### 3-[4-(3,6-Dimethyl-4,10-dihydro-3H-2,3,4,9-tetraaza-benzo[f]azulene-9-carbonyl)-2-methyl-phenyl]-1-(4-methanesulfonyl-piperazin-1-yl)-propan-1-one

A solution of methanesulfonyl chloride (0.57mg, 0.005mmol) in dichloromethane (0.05ml) was added to a solution of 3-[4-(3,6-dimethyl-4,10-dihydro-3H-2,3,4,9-tetraaza-benzo[f]azulene-9-carbonyl)-2-methyl-phenyl]-1-piperazin-1-yl-propan-1-one (2.36mg, 0.005mmol) in dichloromethane (0.05ml) and triethylamine (0.0035ml). The mixture was stirred at room temperature for 20h then solvents were removed in *vacuo* to yield the title compound. (ESI)+: [M+H]+= 551.3

### Example E64

### 1-[4-(3,3-Dimethyl-butyl)-piperazin-1-yl]-4-[2-methyl-4-(3-methyl-4,10-dihydro-3H-2,3,4,9-tetraaza-benzo[f]azulene-9-carbonyl)-phenyl]-butan-1-one

4-{4-[4-(3,3-Dimethyl-butyl)-piperazin-1-yl]-4-oxobutyl}-3-methyl-benzoic acid from Example E43 (139mg, 0.37mmol) and 3-methyl-3,4,9,10-tetrahydro-2,3,4,9-tetraaza-benzo[f]azulene from Example E11 (75mg, 0.37mmol) were dissolved in dichloromethane (5ml) and DIEA (0.195ml, 1.11mmol). WSCD (93mg, 0.48mmol) and DMAP (9mg, 0.07mmol) were added and the mixture was heated at reflux for 3 days, then washed with saturated NaHCO₃ then brine, dried and concentrated in *vacuo.* The residue was purified by preparative HPLC (eluant; 0.5% 35% ammonia:9.5% methanol:90% dichloromethane) to give a white powder identified as the title compound (11mg, 5%).

### Example E65

### N-Benzyl-3-[4-(2,3,4,5-tetrahydro-benzo[b]azepine-1-carbonyl)-phenyl]-propionamide

### Example E65.1

### 4-((E)-2-Methoxycarbonyl-vinyl)-benzoic acid

Sodium hydride (60% dispersion in oil, 1.46g, 36.6mmol) was added to a solution of 4-formylbenzoic acid (5.0g, 33.3mmol) in toluene (200ml) and the mixture was stirred for 2h at room temperature. Methyl (triphenyl-phosphoranylidene)acetate (11.69g, 35.0mmol) was added and the mixture was heated at reflux for 20h. Solvents were removed *in vacuo* and the residue was redissolved in dichloromethane and 1M NaHCO₃. The layers were partitioned and the aqueous layer was acidified with 1M HCl solution then extracted with chloroform. The organic layer was dried and concentrated *in vacuo* to yield the title compound (3.65g, 53%).

### Example E65.2

### (E)-3-[4-(2,3,4,5-Tetrahydro-benzo[b]azepine-1-carbonyl)-phenyl]-acrylic acid methyl ester

WSCD (1.17g, 6.16mmol) was added to a solution of 4-((E)-2-methoxycarbonyl-vinyl)-benzoic acid from Example E65.1 (740mg, 3.59mmol), 2,3,4,5-tetrahydro-1H-benzo[b]azepine (445mg, 3.02mmol) and DMAP (370mg, 3.02mmol) in dichloromethane (40ml) and triethylamine (0.7ml, 5.02mmol). The mixture was heated at reflux for 42h then solvents were concentrated *in vacuo.* The residue was dissolved in EtOAc, washed with 1M KHSO₄ then brine, dried and concentrated *in vacuo.* The residue was purified by flash chromatography on silica gel (eluant; 30% EtOAc:70% pet. ether) to yield the title compound (484mg, 48%).

### Example E65.3

### 3-[4-(2,3,4,5-Tetrahydro-benzo[b]azepine-1-carbonyl)-phenyl]-propionic acid methyl ester

(E)-3-[4-(2,3,4,5-Tetrahydro-benzo[b]azepine-1-carbonyl)-phenyl]-acrylic acid methyl ester from Example E65.2 (485mg, 1.44mmol) was dissolved in methanol (60ml) and hydrogenated over 10% Pd/C catalyst (214mg) for 2h. The mixture was filtered through Celite® filter agent and the filtrate was concentrated *in vacuo* to yield the title compound (415mg, 85%).

### Example E65.4

### 3-[4-(2,3,4,5-Tetrahydro-benzo[b]azepine-1-carbonyl)-phenyl]-propionic acid

Lithium hydroxide monohydrate (114mg, 2.72mmol) was added to a solution of 3-[4-(2,3,4,5-tetrahydro-benzo[b]azepine-1-carbonyl)-phenyl]-propionic acid methyl ester from Example E65.3 (415mg, 1.23mmol) in water (5ml) and dioxan (20ml). The mixture was stirred for 18h at room temperature then concentrated *in vacuo.* The residue was dissolved in chloroform, washed with 1M HCl solution then brine, dried and concentrated in *vacuo* to yield the title compound (241mg, 61%).

### Example E65.5

### N-Benzyl-3-[4-(2,3,4,5-tetrahydro-benzo[b]azepine-1-carbonyl)-phenyl]-propionamide

A mixture of 3-[4-(2,3,4,5-tetrahydro-benzo[b]azepine-1-carbonyl)-phenyl]-propionic acid from Example E65.4 (57mg, 0.18mmol) and thionyl chloride (0.2ml, 2.74mmol) in dichloromethane (6ml) was heated at reflux for 2h then volatiles were removed *in vacuo.* The residue was dissolved in dichloromethane (8ml) then benzylamine (0.022ml, 0.20mmol) and triethylamine (0.05ml, 0.36mmol) were added. The mixture was stirred for 3h at room temperature then concentrated in vacuo. The residue was purified by flash chromatography on silica gel (eluant; 30% pet. ether:70% EtOAc) to give a white fluffy solid identified as the title compound (36.5mg, 50%).

### Example E66

### 1-[4-(3,3-Dimethyl-butyl)-piperazin-1-yl]-3-[2-methyl-4-(2-methyl-4,5-dihydxo-1H-1,3,6-triaza-benzo[e]azulene-6-carbonyl)-phenyl]-propan-1-one

Oxalyl chloride (0.049ml, 0.55mmol) was added slowly to a solution of 4-{3-[4-(3,3-dimethyl-butyl)-piperazin-1-yl]-3-oxo-propyl}-3-methyl-benzoic acid from Example E44 (100mg, 0.28mmol) in dichloromethane (10ml) and few drops of DMF. The mixture was stirred for 1h at room temperature then concentrated *in vacuo* and azeotroped with toluene. The residue was redissolved in dichloromethane (10ml) and DIEA (0.144ml, 0.84mmol) then 2-methyl-1,4,5,6-tetrahydro-1,3,6-triaza-benzo[e]azulene from Example E5 (55mg, 0.28mmol) was added. The mixture was stirred for 20h at room temperature then washed with saturated NaHCO₃ then brine, dried and concentrated *in vacuo.* The residue was purified by preparative HPLC (eluant; 0.5% 35% ammonia:9.5% methanol:90% dichloro-methane) to give a white fluffy solid identified as the title compound (42mg, 28%).

### Example E67

### 1-[4-(3,3-Dimethyl-butyl)-piperazin-1-yl]-3-[2-methyl-4-(6-methyl-3,4-dihydro-2H-quinoline-1-carbonyl)-phenyl]-propan-1-one

DMAP (51mg, 0.42mmol) and WSCD (161mg, 0.84mmol) were added to a solution of 4-{3-[4-(3,3-dimethyl-butyl)-piperazin-1-yl]-3-oxo-propyl}-3-methyl-benzoic acid from Example E44 (150mg, 0.42mmol) in dichloromethane (10ml) and triethylamine (0.176ml, 1.26mmol) and the mixture was stirred for 30min at room temperature. 6-Methyl-1,2,3,4-tetrahydro-quinoline (67mg, 0.46mmol) was added and the mixture was stirred for 20h at room temperature then solvents were removed *in vacuo.* The residue was redissolved in EtOAC, washed with saturated NaHCO₃ then brine, dried and concentrated *in vacuo.* The residue was purified by preparative HPLC (eluant; 0.5% 35% ammonia:9.5% methanol:90% dichloromethane) to give a white solid identified as the title compound (12mg, 6%).

### Example E68

### (4-{3-[1-(3,3-Dimethyl-butyl)-piperidin-4-yl]-propoxy}-3-methyl-phenyl)-(3-methyl-4,10-dihydro-3H-2,3,4,9-tetraaza-benzo[f]azulen-9-yl)-methanone

### Example E68.1

### 4-(3-Hydroxy-propyl)-piperidine-1-carboxylic acid tert-butyl ester

4-(2-Carboxy-ethyl)-piperidine-1-carboxylic acid tert-butyl ester (1.0g, 3.9mmol, according to the procedure described in J. Med. Chem., 41(14), 2492, 1998) was dissolved in THF (50ml) and cooled down to 0°C. 2M Borane solution in THF (3.9ml, 7.8mmol) was added slowly and the mixture was stirred for 20h at room temperature. Water was added and solvents were concentrated in vacuo. The residue was dissolved in EtOAC, basified with NaHCO₃, washed with brine, dried and concentrated *in vacuo* to yield the title compound (938mg, 99%).

### Example E68.2

### 4-[3-(4-Methoxycarbonyl-2-methyl-phenoxy)-propyl]-piperidine-1-carboxylic acid tert-butyl ester

4-(3-Hydroxy-propyl)-piperidine-1-carboxylic acid tert-butyl ester from Example E68.1 (400mg, 1.64mmol) and 4-hydroxy-3-methyl-benzoic acid methyl ester from Example E29 (274mg, 1.64mmol) were dissolved in THF (30ml) and cooled down to 0°C. Polymer-supported triphenylphosphine (1.7g, 2.46mmol) then DEAD (0.387ml), 2.46mmol) were added and the mixture was heated at reflux for 20h. The resin was filtered off, washed with EtOAc and the filtrate was concentrated *in vacuo.* The residue was purified by flash chromatography on silica gel through an isolute (eluant; 20% EtOAC:80% cyclohexane) to yield the title compound (340mg, 53%).

### Example E68.3

### 4-[3-(4-Carboxy-2-methyl-phenoxy)-propyl]-piperidine-1-carboxylic acid tert-butyl ester

2M NaOH solution (1.25ml, 2.5mmol) was added to 4-[3-(4-methoxycarbonyl-2-methyl-phenoxy)-propyl]-piperidine-1-carboxylic acid tert-butyl ester from Example E68.2 (340mg, 0.87mmol) in dioxan (10ml) and the mixture was heated at 60°C for 20h. 2M NaOH solution (5ml, 10mmol) was added and the mixture was heated at 60°C for 20h then concentrated *in vacuo.* The residue was purified by flash chromatography on silica gel through an isolute (eluant; 1% acetic acid:9% methanol:90% dichloromethane) to yield the title compound (303mg, 92%).

### Example E68.4

### 4-{3-[2-Methyl-4-(3-methyl-4,10-dihydro-3H-2,3,4,9-tetraaza-benzo[f]azulene-9-carbonyl)-phenoxy]-propyl}-piperidine-1-carboxylic acid tert-butyl ester

PyBroP (224mg, 0.48mmol) and DMAP (39mg, 0.32mmol) were added to a solution of 4-[3-(4-carboxy-2-methyl-phenoxy)-propyl]-piperidine-1-carboxylic acid tert-butyl ester from Example E68.3 (120mg, 0.32mmol) and 3-methyl-3,4,9,10-tetrahydro-2,3,4,9-tetraaza-benzo[f]azulene from Example E11 (70mg, 0.35mmol) in dichloromethane (5ml) and DIEA (0.111ml, 0.64mmol). The mixture was heated at reflux for 2 days, washed with saturated NaHCO₃ then brine, dried and concentrated *in* vacuo. The residue was purified by preparative HPLC (eluant; 0.5% 35% ammonia:9.5% methanol:90% dichloromethane) to yield the title compound (71mg, 40%).

### Example E68.5

### (3-Methyl-4,10-dihydro-3H-2,3,4,9-tetraaza-benzo[f]azulen-9-yl)-[3-methyl-4-(3-piperidin-4-yl-propoxy)-phenyl]-methanone hydrochloride

4M HCl solution in dioxan (1ml) was added to a solution of 4-{3-[2-methyl-4-(3-methyl-4,10-dihydro-3H-2,3,4,9-tetraaza-benzo[f]azulene-9-carbonyl)-phenoxy]-propyl}-piperidine-1-carboxylic acid tert-butyl ester from Example E68.4 (71mg, 0.13mmol) in methanol (3ml). The mixture was stirred for 20h at room temperature then solvents were concentrated *in vacuo.* The residue was triturated with diethyl ether to yield the title compound (61mg, 97%).

### Example E68.6

### (4-{3-[1-(3,3-Dimethyl-butyl)-piperidin-4-yl]-propoxy}-3-methyl-phenyl)-(3-methyl-4,10-dihydro-3H-2,3,4,9-tetraaza-benzo[f]azulen-9-yl)-methanone

Acetic acid (0.05ml) and 3,3-dimethylbutyraldehyde (0.024ml, 0.19mmol) were added to a solution of (3-methyl-4,10-dihydro-3H-2,3,4,9-tetraaza-benzo[f]azulen-9-yl)-[3-methyl-4-(3-piperidin-4-yl-propoxy)-phenyl]-methanone hydrochloride from Example E68.5 (60mg, 0.12mmol) in methanol (2.45ml) and triethylamine (0.026ml, 0.19mmol). The mixture was stirred at room temperature for 1h then sodium triacetoxyborohydride (40mg, 0.19mmol) was added. The mixture was stirred at room temperature for 18h, diluted with dichloromethane, washed with saturated NaHCO₃ then brine, dried and concentrated *in vacuo.* The residue was purified by preparative HPLC (eluant; 0.5% 35% ammonia:9.5% methanol:90% dichloromethane) to give a white fluffy solid identified as the title compound (33mg, 48%).

### Example E69

### (4-{3-[4-(3,3-Dimethyl-butyl)-piperazin-1-yl]-propoxy}-3-fluoro-phenyl)-(2-methyl-4,5-dihydro-1H-1,3,6-triaza-benzo[e]azulen-6-yl)-methanone

### Example E69.1

### 4-{3-[4-(1-Benzyl-2-methyl-4,5-dihydro-1H-1,3,6-triaza-benzo[e]azulene-6-carbonyl)-2-fluoro-phenoxy]-propyl}-piperazine-1-carboxylic acid tert-butyl ester

4-[3-(4-Carboxy-2-fluoro-phenoxy)-propyl]-piperazine-1-carboxylic acid tert-butyl ester from Example E39 (130mg, 0.34mmol) was dissolved in dichloromethane (5ml) and a few drops of DMF. Oxalyl chloride (0.059ml, 0.68mmol) was added slowly and the mixture was stirred for 30min at room temperature. Solvents were concentrated *in vacuo* and azeotroped with toluene. The residue was dissolved in dichloromethane (5ml) and 1-benzyl-2-methyl-1,4,5,6-tetrahydro-1,3,6-triaza-benzo[e]azulene from Example E5a (100mg, 0.34mmol) then DIEA (0.176ml, 1.02mmol) were added. The mixture was stirred for 2h at room temperature, washed with saturated NaHCO₃ then brine, dried and concentrated in *vacuo.* The residue was purified by preparative HPLC (eluant; 0.5% 35% ammonia:9.5% methanol:90% dichloromethane) to yield the title compound (110mg, 50%).

### Example E69.2

### (1-Benzyl-2-methyl-4,5-dihydro-1H-1,3,6-triaza-benzo[e]azulen-6-yl)-[3-fluoro-4-(3-piperazin-1-yl-propoxy)-phenyl]-methanone dihydrochloride

4-{3-[4-(1-Benzyl-2-methyl-4,5-dihydro-1H-1,3,6-triaza-benzo[e]azulene-6-carbonyl)-2-fluoro-phenoxy]-propyl}-piperazine-1-carboxylic acid tert-butyl ester from Example E69.1 (110mg, 0.17mmol) was dissolved in methanol (5ml) and 4M HCl in dioxan (2ml) was added. The mixture was stirred for 20h at room temperature then concentrated in *vacuo* to yield the title compound (105mg, 100%).

### Example E69.3

### (1-Benzyl-2-methyl-4,5-dihydro-1H-1,3,6-triazabenzo[e]azulen-6-yl)-(4-{3-[4-(3,3-dimethyl-butyl)-piperazin-1-yl]-propoxy}-3-fluoro-phenyl)-methanone

(1-Benzyl-2-methyl-4,5-dihydro-1H-1,3,6-triazabenzo[e]azulen-6-yl)-[3-fluoro-4-(3-piperazin-1-yl-propoxy)-phenyl]-methanone dihydrochloride from Example E69.2 (105mg, 0.17mmol) was dissolved in methanol (2.45ml) and triethylamine (0.07ml, 0.50mmol). Acetic acid (0.05ml) then 3,3-dimethylbutyraldehyde (0.032ml), 0.25mmol) were added and the mixture was stirred for 1h at room temperature. Sodium cyanoborohydride (16mg, 0.25mmol) was added and the mixture was stirred for 3h at room temperature then concentrated *in vacuo*. The residue was dissolved in dichloromethane, washed with saturated NaHCO₃ then brine, dried and concentrated *in vacuo.* The residue was purified by flash chromatography on silica gel (eluant; 5% methanol : 95% dichloromethane) to yield the title compound (102mg, 95%).

### Example E69.4

### (4-{3-[4-(3,3-Dimethyl-butyl)-piperazin-1-yl]-propoxy}-3-fluoro-phenyl)-(2-methyl-4,5-dihydro-1H-1,3,6-triazabenzo[e]azulen-6-yl)-methanone

(1-Benzyl-2-methyl-4,5-dihydro-1H-1,3,6-triazabenzo[e]azulen-6-yl)-(4-{3-[4-(3,3-dimethyl-butyl)-piperazin-1-yl]-propoxy}-3-fluoro-phenyl)-methanone from Example E69.3 (90mg, 0.14mmol) was dissolved in methanol (5ml) and acetic acid (1ml) and hydrogenated over 20 wt.% palladium hydroxide catalyst (90mg) for 8h. The mixture was filtered through Celite® filter agent and the filtrate concentrated *in vacuo.* The residue was dissolved in dichloromethane, washed with saturated NaHCO₃ then brine, dried and concentrated *in vacuo.* The residue was purified by preparative HPLC (eluant; 0.5% 35% ammonia:9.5% methanol : 90% dichloromethane) to give a white powder identified as the title compound (39mg, 51%).

### Example E70

### (4-{3-[4-(3,3-Dimethyl-butyl)-piperazin-1-yl]-propoxy}-3-methyl-phenyl)-(2-methyl-4,5-dihydro-1H-1,3,6-triazabenzo[e]azulen-6-yl)-methanone

### Example E70.1

### 4-{3-[4-(1-Benzyl-2-methyl-4,5-dihydro-1H-1,3,6-triazabenzo[e]azulene-6-carbonyl)-2-methyl-phenoxy]-propyl}-piperazine-1-carboxylic acid tert-butyl ester

4-[3-(4-Carboxy-2-methyl-phenoxy)-propyl]-piperazine-1-carboxylic acid tert-butyl ester (350mg, 0.92mmol) was dissolved in dichloromethane (5ml) and a few drops of DMF. Oxalyl chloride (0.162ml, 1.84mmol) was added slowly and the mixture was stirred for 30min at room temperature. Solvents were concentrated *in vacuo* and azeotroped with toluene. The residue was dissolved in dichloromethane (10ml) and 1-benzyl-2-methyl-1,4,5,6-tetrahydro-1,3,6-triaza-benzo[e]azulene from Example E5a (268mg, 0.92mmol) then DIEA (0.48ml, 2.77mmol) were added. The mixture was stirred for 2 days at room temperature, washed with saturated NaHCO₃ then brine, dried and concentrated *in vacuo.* The residue was purified by preparative HPLC (eluant; 0.5% 35% ammonia:9.5% methanol:90% dichloromethane) to yield the title compound (230mg, 38%).

### Example E70.2

### (1-Benzyl-2-methyl-4,5-dihydro-1H-1,3,6-triazabenzo[e]azulen-6-yl)-[3-methyl-4-(3-piperazin-1-yl-propoxy)-phenyl]-methanone dihydrochloride

4-{3-[4-(1-Benzyl-2-methyl-4,5-dihydro-1H-1,3,6-triazabenzo[e]azulene-6-carbonyl)-2-methyl-phenoxy]-propyl}-piperazine-1-carboxylic acid tert-butyl ester from Example E70.1 (230mg, 0.35mmol) was dissolved in methanol (5ml) and 4M HCl in dioxan (2ml) was added. The mixture was stirred for 2h at room temperature then concentrated *in vacuo* and triturated with diethyl ether to yield the title compound (215mg, 98%).

### Example E70.3

### (1-Benzyl-2-methyl-4,5-dihydro-1H-1,3,6-triazabenzo[e]azulen-6-yl)-4-{3-[4-(3,3-dimethyl-butyl)-piperazin-1-yl]-propoxy}-3-methyl-phenyl)-methanone

(1-Benzyl-2-methyl-4,5-dihydro-1H-1,3,6-triazabenzo[e]azulen-6-yl)-[3-methyl-4-(3-piperazin-1-yl-propoxy)-phenyl]-methanone dihydrochloride from Example E70.2 (100mg, 0.16mmol) was dissolved in methanol (2.45ml) and triethylamine (0.068ml, 0.48mmol). Acetic acid (0.05ml) then 3,3-dimethylbutyraldehyde (0.031ml, 0.24mmol) were added and the mixture was stirred for 1h at room temperature. Sodium cyanoborohydride (16mg, 0.24mmol) was added and the mixture was stirred for 20h at room temperature then concentrated *in vacuo.* The residue was dissolved in dichloromethane, washed with saturated NaHCO₃ then brine, dried and concentrated *in vacuo* to yield the title compound (98mg, 96%).

### Example E70.4

### (4-{3-[4-(3,3-Dimethyl-butyl)-piperazin-1-yl]-propoxy}-3-methyl-phenyl)-(2-methyl-4,5-dihydro-1H-1,3,6-triazabenzo[e]azulen-6-yl)-methanone

(1-Benzyl-2-methyl-4,5-dihydro-1H-1,3,6-triazabenzo[e]azulen-6-yl)-(4-{3-[4-(3,3-dimethyl-butyl)-piperazin-1-yl]-propoxy}-3-methyl-phenyl)-methanone from Example E70.3 (98mg, 0.15mmol) was dissolved in methanol (5ml) and acetic acid (1ml) and hydrogenated over 20 wt.% palladium hydroxide catalyst (98mg) for 8h. The mixture was filtered through glass-fibre paper and the filtrate was concentrated *in vacuo.* The residue was dissolved in dichloromethane, washed with saturated NaHCO₃ then brine, dried and concentrated *in vacuo*. The residue was purified by preparative HPLC (eluant; 0.5% 35% ammonia:9.5% methanol:90% dichloromethane) to give a white powder identified as the title compound (17mg, 20%).

### Example E71

### N-(4-Chloro-phenyl)-4-{3-[4-(3,3-dimethyl-butyl)-piperazin-1-yl]-propoxy}-3-fluoro-N-methyl-benzamide

### Example E71.1

### 4-(3-{4-[(4-Chloro-phenyl)-methyl-carbamoyl]-2-fluorophenoxy}-propyl)-piperazine-1-carboxylic acid tert-butyl ester

PyBroP (606mg, 1.3mmol) was added to a solution of 4-[3-(4-carboxy-2-fluoro-phenoxy)-propyl]-piperazine-1-carboxylic acid tert-butyl ester from Example E39 (382mg, 1.0mmol) in dichloromethane (20ml) and DIEA (to pH9). The mixture was stirred for 1h at room temperature then (4-chloro-phenyl)-methyl amine (156mg, 1.1mmol) was added. The mixture was stirred for 2 days at room temperature then solvents were concentrated *in vacuo.* The residue was dissolved in EtOAC, washed with saturated NaHCO₃ then brine, dried and concentrated *in vacuo.* The residue was purified by flash chromatography on silica gel (eluant; EtOAc) to yield the title compound (210mg, 41%).

### Example E71.2

### N-(4-Chloro-phenyl)-3-fluoro-N-methyl-4-(3-piperazin-1-yl-propoxy)-benzamide dihydrochloride

4-(3-{4-[(4-Chloro-phenyl)-methyl-carbamoyl]-2-fluorophenoxy}-propyl)-piperazine-1-carboxylic acid tert-butyl ester from Example E71.1 (210mg, 0.4mmol) was dissolved in 4M HCl in dioxan (5ml) and stirred for 1h at room temperature. Solvents were concentrated *in vacuo* and azeotroped with toluene to yield the title compound (184mg, 100%).

### Example E71.3

### N-(4-Chloro-phenyl)-4-{3-[4-(3,3-dimethyl-butyl)-piperazin-1-yl]-propoxy}-3-fluoro-N-methyl-benzamide

N-(4-Chloro-phenyl)-3-fluoro-N-methyl-4-(3-piperazin-1-yl-propoxy)-benzamide dihydrochloride from Example E71.2 (184mg, 0.4mmol) was dissolved in methanol (4.95ml) and triethylamine (0.167ml, 1.2mmol). Acetic acid (0.05ml) then 3,3-dimethylbutyraldehyde (46mg, 0.44mmol) were added and the mixture was stirred for 1h at room temperature. Sodium cyanoborohydride (31mg, 0.5mmol) was added and the mixture was stirred for 20h at room temperature then concentrated *in vacuo*. The residue was purified by flash chromatography on silica gel (eluant; 10% methanol:90% dichloromethane) to give a white foam identified as the title compound (77mg, 37%).

### Example E72

### (4-{4-[4-(3,3-Dimethyl-butyl)-piperazin-1-yl]-butoxy}-3-fluoro-phenyl)-(3-methyl-4,10-dihydro-3H-2,3,4,9-tetraaza-benzo[f]azulen-9-yl)-methanone

### Example E72.1

### 4-(4-Bromo-butoxy)-3-fluoro-benzoic acid ethyl ester

1,4-Dibromobutane (2.34g, 10.8mmol), potassium carbonate (1.86g, 13.5mmol) and potassium iodide (90mg, 0.5mmol) were added to a solution of 3-fluoro-4-hydroxy-benzoic acid ethyl ester from Example E27 (1.0g 5.4mmol) in acetone (25ml) and the mixture was heated at reflux for 20h. The solid was filtered off, washed with EtOAc and the filtrate was concentrated *in vacuo*. The residue was purified by flash chromatography on silica gel (eluant; 15% EtOAc:85% hexane) to yield the title compound (1.38g, 80%).

### Example E72.2

### 9-{4-[4-(3,3-Dimethyl-butyl)-piperazin-1-yl]-butoxy}-3-fluoro-benzoic acid ethyl ester

1-(3,3-Dimethyl-butyl)-piperazine hydrochloride from Example E4 -(-874mg, 3.7mmol), potassium carbonate (2.5g, 18.0mmol) and potassium iodide (60mag, 0.4mmol) were added to a solution of 4-(4-bromo-butoxy)-3-fluorobenzoic acid ethyl ester from Example E72.1 (1.4g, 4.4mmol) in acetone (35ml) and the mixture was heated at reflux for 20h. The solid was filtered off, washed with EtOAc and the filtrate was concentrated in vacuo. The residue was purified by flash chromatography on silica gel (eluant; EtOAc to 1% 35% ammonia:10% methanol:89% EtOAc) to yield the title compound (1.35g, 90%) .

### Example E72.3

### 4-{4-[4-(3,3-Dimethyl-butyl)-piperazin-1-yl]-butoxy}-3-fluoro-benzoic acid

Sodium hydroxide (0.5g, 13.2mmol) and water (5ml) were added to a solution of 4-{4-[4-(3,3-dimethyl-butyl)-piperazin-1-yl]-butoxy}-3-fluoro-benzoic acid ethyl ester from Example E72.2 (1.35g, 3.3mmol) in dioxan (20ml). The mixture was stirred for 1h at room temperature then heated at 60°C for 20h. Solvents were concentrated *in vacuo* and azeotroped with toluene. The residue was purified by flash chromatography on silica gel (eluant; 1% 35% ammonia:19% methanol:80% chloroform) to yield the title compound (0.85g, 67%).

### Example E72.4

### (4-{4-[4-(3,3-Dimethyl-butyl)-piperazin-1-yl]-butoxy}-3-fluoro-phenyl)-(3-methyl-4,10-dihydro-3H-2,3,4,9-tetraaza-benzo[f]azulen-9-yl)-methanone

A mixture of 4-{4-[4-(3,3-dimethyl-butyl)-piperazin-1-yl]-butoxy}-3-fluoro-benzoic acid from Example E72.3 (190mg, 0.5mmol) and thionyl chloride (2ml) in dichloromethane (5ml) was heated at reflux for 1h. Solvents were concentrated *in vacuo* and the residue was dissolved in dichloromethane (10ml). 3-Methyl-3,4,9,10-tetrahydro-2,3,4,9-tetraaza-benzo[f]azulene from Example E11 (110mg, 0.55mmol) and DIEA (to pH9) were added. The mixture was stirred at room temperature for 2 days and concentrated *in vacuo.* The residue was dissolved in EtOAC, washed with saturated NaHCO₃ then brine, dried and concentrated *in vacuo.* The residue was purified by preparative HPLC (eluant; 0.5% 35% ammonia:9.5% methanol:90% dichloromethane) to give a colourless oil identified as the title compound (48mg, 17%).

### Example E73

### (4-{2-[4-(3,3-Dimethyl-butyl)-piperazin-1-yl]-ethoxy}-3-methyl-phenyl)-(3-methyl-4,10-dihydro-3H-2,3,4,9-tetraaza-benzo[f]azulen-9-yl)-methanone

WSCD (58mg, 0.30mmol) and DMAP (20mg, 0.15mmol) were added to a solution of 3-methyl-3,4,9,10-tetrahydro-2,3,4,9-tetraaza-benzo[f]azulene from Example E11 (37mg, 0.20mmol) and 4-{2-[4-(3,3-dimethyl-butyl)-piperazin-1-yl]-ethoxy}-3-methyl-benzoic acid from Example E40 (53mg, 0.15mmol) in dichloromethane) (5ml) and triethylamine (to pH9). The mixture was heated at reflux for 2 days then concentrated *in vacuo.* The residue was dissolved in EtOAC, washed with saturated NaHCO₃ then brine, dried and concentrated *in vacuo.* The residue was purified by flash chromatography on silica gel (eluant; 10% methanol:90% chloroform) to give an off-white solid identified as the title compound (3.5mg, 4%).

### Example E74

### 1-[4-(3,3-Dimethyl-butyl)-piperazin-1-yl]-2-[2-methyl-4-(3-methyl-4,10-dihydro-3H-2,3,4,9-tetraazabenzo[f]azulene-9-carbonyl)-phenoxy]-ethanone

Oxalyl chloride (42mg, 0.34mmol) and 2 drops of DMF were added to a solution of 4-{2-[4-(3,3-dimethylbutyl)-piperazin-1-yl]-2-oxo-ethoxy}-3-methyl-benzoic acid from Example E41 (100mg, 0.28mmol) in dichloromethane (5ml) at 0°C. The mixture was stirred for 1h at room temperature, concentrated *in vacuo* and azeotroped with toluene. The residue was dissolved in dichloromethane (5ml) and a solution of 3-methyl-3,4,9,10-tetrahydro-2,3,4,9-tetraaza-benzo[f]azulene from Example E11 (61mg, 0.31mmol) in dichloromethane (1ml) and triethylamine (to pH9) was added. The mixture was stirred for 2 days at room temperature then concentrated *in vacuo.* The residue was dissolved in EtOAC, washed with saturated NaHCO₃ then brine, dried and concentrated in vacuo. The residue was purified twice by flash chromatography on silica gel (eluant; 5% methanol:95% chloroform to 10% methanol:90% chloroform then 5% methanol:95% EtOAc to 10% methanol:90% EtOAc) to give a white solid identified as the title compound (25mg, 17%).

### Example E75

### 1-[4-(3,3-Dimethyl-butyl)-piperazin-1-yl]-2-[4-(3-methyl-4,10-dihydro-3H-2,3,4,9,9-tetraazabenzo[f]azulene-9-carbonyl)-phenoxy]-ethanone

### Example E75.1

### (4-Benzyloxy-phenyl)-(3-methyl-4,10-dihydro-3H-2,3,4,9-tetraaza-benzo[f]azulen-9-yl)-methanone

Oxalyl chloride (0.41ml, 4.7mmol) and a few drops of DMF were added to a solution of 4-(benzyloxy)benzoic acid (855mg, 3.7mmol) in dichloromethane (15ml). The mixture was stirred for 2h at room temperature, concentrated *in vacuo* and azeotroped with toluene. The residue was dissolved in dichloromethane (10ml) and a solution of 3-methyl-3,4,9,10-tetrahydro-2,3,4,9-tetraazabenzo[f]azulene from Example E11 (500mg, 2.5mmol) in dichloromethane (10ml) and triethylamine (0.87ml, 6.2mmol) was added. The mixture was stirred for 20h at room temperature and concentrated *in vacuo.* The residue was purified by flash chromatography on silica gel (eluant; EtOAc to 3% methanol:97% EtOAc) to yield the title compound (820mg, 80%).

### Example E75.2

### (4-Hydroxy-phenyl)-(3-methyl-4,10-dihydro-3H-2,3,4,9-tetraaza-benzo[f]azulen-9-yl)-methanone

(4-Benzyloxy-phenyl)-(3-methyl-4,10-dihydro-3H-2,3,4,9-tetraaza-benzo[f]azulen-9-yl)-methanone from Example E75.1 (800mg, 1.95mmol) was dissolved in methanol (100ml) and hydrogenated over 10% Pd/C catalyst (400mg) for 7h. The mixture was filtered through Celite® filter agent, washed with chloroform and methanol and the filtrate was concentrated *in vacuo* to yield the title compound (140mg, 22%).

### Example E75.3

### 1-[4-(3,3-Dimethyl-butyl)-piperazin-1-yl]-2-[4-(3-methyl-4,10-dihydro-3H-2,3,4,9-tetraazabenzo[f]azulene-9-carbonyl)-phenoxy]-ethanone

Potassium carbonate (104mg, 0.76mmol) was added to a mixture of 2-bromo-1-[4-(3,3-dimethyl-butyl)-piperazin-1-yl]-ethanone from Example E37 (110mg, 0.38mmol) and (4-hydroxy-phenyl)-(3-methyl-4,10-dihydro-3H-2,3,4,9-tetraaza-benzo[f]azulen-9-yl)-methanone from Example E75.2 (90mg, 0.28mmol) in acetonitrile (5ml). The mixture was heated at reflux for 20h then concentrated *in vacuo*. The residue was dissolved in dichloromethane, washed with 5% KHCO₃ solution, dried and concentrated *in vacuo.* The residue was purified by preparative HPLC (eluant; 0.5% 35% ammonia:9.5% methanol:90% dichloromethane) to give a white powder identified as the title compound (21mg, 11%).

### Example E76

### 1-[4-(3,3-Dimethyl-butyl)-piperazin-1-yl]-4-[2-methyl-4-(3-methyl-4,10-dihydro-3H-2,3,4,9-tetraazabenzo[f]azulene-9-carbonyl)-phenoxy]-butan-1-one

### Example E76.1

### 4-[2-Methyl-4-(3-methyl-4,10-dihydro-3H-2,3,4,9-tetraaza-benzo[f]azulene-9-carbonyl)-phenoxy]-butyric acid tert-butyl ester

WSCD (391mg, 2.06mmol) and DMAP (133mg, 1.09mmol) were added to a solution of 4-(3-tert-butoxycarbonyl-propoxy)-3-methyl-benzoic acid from Example E42 (275mg, 0.94mmol) and 3-methyl-3,4,9,10-tetrahydro-2,3,4,9-tetraaza-benzo[f]azulene from Example E11 (191mg, 0.95mmol) in dichloromethane (30ml) and triethylamine (0.28ml, 2.01mmol). The mixture was heated at reflux for 72h, washed with 0.3M KHSO₄, saturated NaHCO₃ then brine, dried and concentrated *in vacuo.* The residue was purified by flash chromatography on silica gel (eluant; EtOAc) to yield the title compound (80mg, 18%).

### Example E76.2

### 4-[2-Methyl-4-(3-methyl-4,10-dihydro-3H-2,3,4,9-tetraaza-benzo[f]azulene-9-carbonyl)-phenoxy]-butyric acid

Trifluoroacetic acid (5ml) was added to a solution of 4-[2-methyl-4-(3-methyl-4,10-dihydro-3H-2,3,4,9-tetraaza-benzo[f]azulene-9-carbonyl)-phenoxy]-butyric acid tert-butyl ester from Example E76.1 (80mg, 0.17mmol) in dichloromethane (10ml). The mixture was stirred for 2h at room temperature, concentrated *in vacuo* and azeotroped with dichloromethane to yield the title compound (71mg, 100%).

### Example E76.3

### 1-[4-(3,3-Dimethyl-butyl)-piperazin-1-yl]-4-[2-methyl-4-(3-methyl-4,10-dihydro-3H-2,3,4,9-tetraazabenzo[f]azulene-9-carbonyl)-phenoxy]-butan-1-one

HBTU (101mg, 0.27mmol) was added to a solution of 4-[2-methyl-4-(3-methyl-4,10-dihydro-3H-2,3,4,9-tetraazabenzo[f]azulene-9-carbonyl)-phenoxy]-butyric acid from E76.2 (71mg, 0.17mmol) and 1-(3,3-dimethyl-butyl)-piperazine dihydrochloride from Example E4 (54mg, 0.22mmol) in dichloromethane (10ml) and DIEA (0.15ml, 0.86mmol). The mixture was stirred for 20h at room temperature, washed with saturated NaHCO₃ then brine, dried and concentrated *in vacuo.* The residue was purified by flash chromatography on silica gel (eluant; 6% methanol:94% dichloromethane) to give a white powder identified as the title compound (52mg, 53%).

### Example E77

### 3-[2-Methyl-4-(3-methyl-4,10-dihydro-3H-2,3,4,9-tetraaza-benzo[f]azulene-9-carbonyl)-phenyl]-propionic acid piperidin-4-ylmethyl ester

### Example E77.1

### 4-{3-[2-Methyl-4-(3-methyl-4,10-dihydro-3H-2,3,4,9-tetraaza-benzo[f]azulene-9-carbonyl)-phenyl]-propionyloxymethyl}-piperidine-1-carboxylic acid benzyl ester

WSCD (107mg, 0.54mmol) and DMAP (33mg, 0.27mmol) were added to a solution of 3-[2-methyl-4-(3-methyl-4,10-dihydro-3H-2,3,4,9-tetraaza-benzo[f]azulene-9-carbonyl)-phenyl]-propionic acid from Example E45 (106mg, 0.27mmol) and 4-hydroxymethyl-piperidine-1-carboxylic acid benzyl ester (81mg, 0.32mmol) in dichloromethane (5ml) and DIEA (0.095ml, 0.54mmol). The mixture was stirred for 3h at room temperature, washed with saturated NaHCO₃ then brine, dried and concentrated *in vacuo.* The residue was purified by flash chromatography on silica gel through an isolute (eluant; EtOAc) to yield the title compound (44mg, 26%).

### Example E77.2

### 3-[2-Methyl-4-(3-methyl-9,10-dihydro-3H-2,3,4,9-tetraaza-benzo[f]azulene-9-carbonyl)-phenyl]-propionic acid piperidin-4-ylmethyl ester

4-{3-[2-Methyl-4-(3-methyl-4,10-dihydro-3H-2,3,4,9-tetraaza-benzo[f]azulene-9-carbonyl)-phenyl]-propionyloxymethyl}-piperidine-1-carboxylic acid benzyl ester from Example E77.1 (44mg, 0.07mmol) was dissolved in methanol (5ml) and hydrogenated over 10% Pd/C catalyst (5mg) for 2h. The mixture was filtered through Celite® filter agent and the filtrate concentrated *in vacuo* to give a white powder identified as the title compound (34mg, 98%).

### Example E78

### Methyl (3-chloro-4-(3,6-dimethyl-4,10-dihydro-3H-2,3,4,9-tetraaza-benzo[f]azulene-9-carbonyl)-phenoxy)acetate

Diisopropylethylamine (2.1ml, 12.0mmol), dimethylaminopyridine (0.74g, 6.0mmol), PyBroP® (4.20g, 9.0mmol) and 3,6-dimethyl-9,10-dihydro-3H-2,3,4,9-tetraaza-benzo[f]azulene from Example E2 (1.28g, 6.0mmol) were added to a solution of tert-butyl 4-(2-(4-carboxy-3-chloro-phenoxy)-acetyl)-piperazine-1-carboxylate from Example E41c (2.39g, 6.0mmol) in dichloromethane (50ml) and the mixture was heated at reflux for 20h. The reaction mixture was cooled, diluted with dichloromethane (50ml), washed with saturated aqueous sodium hydrogen carbonate, dried and concentrated in vacuo. The residue was dissolved in methanol (150ml) and treated with a 4M solution of hydrogen chloride in dioxan (50ml), with cooling in an ice bath. The solution was stirred at room temperature for 16h. The reaction mixture was evaporated and the residue taken up in water and washed with chloroform. The aqueous was treated with solid sodium hydrogen carbonate until basic pH, extracted into chloroform, dried and evaporated. The residue was purified by flash chromatography on silica gel (eluant; EtOAc followed by a 2% to 10% methanol:98% to 90% EtOAc gradient) to yield a white solid identified as the title compound (0.53g, 20%).

### Example E79

### 1-(4-Hydroxymethyl-piperidin-1-yl)-3-[2-methyl-4-(3-methyl-4,10-dihydro-3H-2,3,4,9-tetraaza-benzo[f]azulene-9-carbonyl)-phenyl]-propan-1-one

Sodium borohydride (12mg, 0.32mmol) was added to a solution of 1-{3-[2-methyl-4-(3-methyl-4,10-dihydro-3H-2,3,4,9-tetraaza-benzo[f]azulene-9-carbonyl)-phenyl]-propionyl}-piperidine-4-carbaldehyde from Example E58.1 (74mg, 0.15mmol) in methanol (6ml). The mixture was stirred for 1h at room temperature, acidified with 1M HCl and concentrated *in vacuo.* The residue was dissolved in EtOAc, washed with saturated NaHCO₃ then brine, dried and concentrated *in vacuo.* The residue was crystallised from chloroform and pet. ether to give a white powder identified as the title compound (28mg, 38%).

### Example E80

### 1-[4-(3,3-Dimethyl-butyl)-piperazin-1-yl]-3-[4-(3,4-dimethyl-4,10-dihydro-3H-2,3,4,9-tetraaza-benzo[f]azulene-9-carbonyl)-2-methyl-phenyl]-propan-1-one

### Example E80.1

### 3-[4-(3,4-Dimethyl-4,10-dihydro-3H-2,3,4,9-tetraaza-benzo[f]azulene-9-carbonyl)-2-methyl-phenyl]-propionic acid tert-butyl ester

Sodium hydride (14mg, 0.34mmol) was added to a solution of 3-[2-methyl-4-(3-methyl-4,10-dihydro-3H-2,3,4,9-tetraaza-benzo[f]azulene-9-carbonyl)-phenyl]-propionic acid tert-butyl ester from Example E45 (125mg, 0.28mmol) in DMF (2ml) at 0°C. The mixture was stirred for 20min at room temperature then cooled down to 0°C. Methyl iodide (0.09ml, 1.4mmol) was added and the mixture was stirred for 20h at room temperature. EtOAc was added and the mixture was washed with 0.3M KHSO₄ then brine, dried, concentrated *in vacuo* and azeotroped with toluene. The residue was purified by flash chromatography on silica gel through an isolute (eluant; EtOAc) to yield the title compound (46mg, 36%).

### Example E80.2

### 3-[4-(3,4-Dimethyl-4,10-dihydro-3H-2,3,4,9-tetraaza-benzo[f]azulene-9-carbonyl)-2-methyl-phenyl]-propionic acid

Trifluoroacetic acid (2ml) was added to 3-[4-(3,4-dimethyl-4,10-dihydro-3H-2,3,4,9-tetraaza-benzo[f]azulene-9-carbonyl)-2-methyl-phenyl]-propionic acid tert-butyl ester from Example E80.1 (46mg, 0.10mmol) in dichloromethane (5ml). The mixture was stirred for 2h at room temperature then concentrated *in vacuo* to yield the title compound (40mg, 100%).

### Example E80.3

### 1-[4-(3,3-Dimethyl-butyl)-piperazin-1-yl]-3-[4-(3,4-dimethyl-4,10-dihydro-3H-2,3,4,9-tetraaza-benzo[f]azulene-9-carbonyl)-2-methyl-phenyl]-propan-1-one

Oxalyl chloride (0.018ml, 0.20mmol) was added to a solution of 3-[4-(3,4-dimethyl-4,10-dihydro-3H-2,3,4,9-tetraaza-benzo[f]azulene-9-carbonyl)-2-methyl-phenyl]-propionic acid from Example E80.2 (40mg, 0.10mmol) in dichloromethane (5ml) and a few drops of DMF. The mixture was stirred for 1h at room temperature, concentrated *in vacuo* and azeotroped with toluene. The residue was dissolved in dichloromethane (5ml) and 1-(3,3-dimethyl-butyl)-piperazine dihydrochloride from Example E4 (30mg, 0.12mmol) and DIEA (0.052ml, 0.30mmol) were added. The mixture was stirred for 20h at room temperature, washed with saturated NaHCO₃ then brine, dried and concentrated *in vacuo.* The residue was purified by preparative HPLC (eluant; 0.5% 35% ammonia:9.5% methanol:90% dichloromethane) to give a white powder identified as the title compound (36mg, 65%).

### Example E81

### 1-[4-(3,3-Dimethyl-butyl)-piperazin-1-yl]-4-[2-methyl-9-(2,3,4,5-tetrahydro-benzo[e][1,4]diazepine-1-carbonyl)-phenyl]-butan-1-one

### Example E81.1

### 1-(4-{4-[4-(3,3-Dimethyl-butyl)-piperazin-1-yl]-4-oxobutyl}-3-methyl-benzoyl)-1,2,3,5-tetrahydro-benzo[e][1,4]diazepine-4-carboxylic acid tert-butyl ester

WSCD (58mg, 0.30mmol) and DMAP (18mg, 0.15mmol) were added to a solution of 4-{4-[4-(3,3-dimethyl-butyl)-piperazin-1-yl]-4-oxo-butyl}-3-methyl-benzoic acid from Example E43 (55mg, 0.15mmol) and 1,2,3,5-tetrahydro-benzo[e][1,4]diazepine-4-carboxylic acid tert-butyl ester (73mg, 0.30mmol) in dichloromethane (3ml) and DIEA (0.052ml, 0.30mmol). The mixture was heated at reflux for 5 days, washed with saturated NaHCO₃ then brine, dried and concentrated *in vacuo.* The residue was purified by preparative HPLC (eluant; 0.5% 35% ammonia:9.5% methanol:90% dichloromethane) to yield the title compound (25mg, 28%).

### Example E81.2

### 1-[4-(3,3-Dimethyl-butyl)-piperazin-1-yl]-4-[2-methyl-4-(2,3,4,5-tetrahydro-benzo[e][1,4]diazepine-1-carbonyl)-phenyl]-butan-1-one

1-(4-{4-[4-(3,3-Dimethyl-butyl)-piperazin-1-yl]-4-oxobutyl}-3-methyl-benzoyl)-1,2,3,5-tetrahydro-benzo[e][1,4]diazepine-4-carboxylic acid tert-butyl ester from Example E81.1 (25mg, 0.04mmol) was dissolved in methanol (2ml) and 4M HCl in dioxan (2ml) was added. The mixture was stirred for 1h at room temperature then concentrated *in vacuo* to give a white powder identified as the title compound (6mg, 28%). (ESI)+: [M+H]+= 505.5

### Example E82

### 4-[9-(7,8-Dihydro-6H-5-oxa-9-aza-benzocycloheptene-9-carbonyl)-2-methyl-phenyl]-1-[4-(3,3-dimethyl-butyl)-piperazin-1-yl]-butan-1-one

WSCD (58mg, 0.30mmol) and DMAP (18mg, 0.15mmol) were added to a solution of 4-{4-[4-(3,3-dimethyl-butyl)-piperazin-1-yl]-4-oxo-butyl}-3-methyl-benzoic acid from Example E43 (55mg, 0.15mmol) and 6,7,8,9-tetrahydro-5-oxa-9-aza-benzocycloheptene (44mg, 0.30mmol) in dichloromethane (3ml) and DIEA (0.052ml, 0.30mmol). The mixture was heated at reflux for 5 days, washed with saturated NaHCO₃ then brine, dried and concentrated *in vacuo.* The residue was purified by preparative HPLC (eluant; 0.5% 35% ammonia:9.5% methanol:90% dichloromethane) to give a white solid identified as the title compound (6mg, 10%).

### Example E83

### 1-[4-(3,3-Dimethyl-butyl)-piperazin-1-yl]-5-[2-methyl-4-(3-methyl-4,10-dihydro-3H-2,3,4,9-tetraaza-benzo[f]azulene-9-carbonyl)-phenyl]-pentan-1-one

### Example E83.1

### 5-[2-Methyl-4-(3-methyl-4,10-dihydro-3H-2,3,4,9-tetraaza-benzo[f]azulene-9-carbonyl)-phenyl]-pentanoic acid methyl ester

WSCD (822mg, 4.3mmol) and DMAP (273mg, 2.2mmol) were added to a solution of 4-(9-methoxycarbonyl-butyl)-3-methyl-benzoic acid from Example E31 (571mg, 2.3mmol) and 3-methyl-3,4,9,10-tetrahydro-2,3,4,9-tetraaza-benzo[f]azulene from Example E11 (424mg, 2.1mmol) in dichloromethane (50ml) and triethylamine (0.6ml, 4.3mmol). The mixture was heated at reflux for 72h, diluted with dichloromethane, washed with 0.3M KHSO₄, saturated NaHCO₃ then brine, dried and concentrated *in vacuo.* The residue was purified by flash chromatography on silica gel (eluant; EtOAc to 5% triethyamine:95% EtOAc) to yield the title compound (424mg, 46%).

### Example E83.2

### 5-[2-Methyl-4-(3-methyl-4,10-dihydro-3H-2,3,4,9-tetraaza-benzo[f]azulene-9-carbonyl)-phenyl]-pentanoic acid

Lithium hydroxide monohydrate (120mg, 2.86mmol) was added to a solution of 5-[2-methyl-4-(3-methyl-4,10-dihydro-3H-2,3,4,9-tetraaza-benzo[f]azulene-9-carbonyl)-phenyl]-pentanoic acid methyl ester from Example E83.1 (410mg, 0.95mmol) in THF (10ml) and water (5ml). The mixture was stirred for 24h at room temperature and concentrated *in vacuo.* The residue was acidified with 1M HCl and extracted with chloroform. The organic layer was washed with brine, dried and concentrated *in vacuo*. The residue was purified by flash chromatography on silica gel (eluant; 3% acetic acid:7% methanol:90% chloroform) to yield the title compound (105mg, 26%).

### Example E83.3

### 1-[4-(3,3-Dimethyl-butyl)-piperazin-1-yl]-5-[2-methyl-4-(3-methyl-4,10-dihydro-3H-2,3,4,9-tetraaza-benzo[f]azulene-9-carbonyl)-phenyl]-pentan-1-one

HBTU (104mg, 0.27mmol) was added to a solution of 5-[2-methyl-4-(3-methyl-4,10-dihydro-3H-2,3,4,9-tetraaza-benzo[f]azulene-9-carbonyl)-phenyl]-pentanoic acid from E83.2 (105mg, 0.25mmol) and 1-(3,3-dimethyl-butyl)-piperazine dihydrochloride from Example E4 (69mg, 0.28mmol) in DMF (10ml) and DIEA (0.2ml, 1.15mmol). The mixture was stirred for 24h at room temperature then concentrated *in vacuo*. The residue was dissolved in dichloromethane, washed with saturated NaHCO₃ then brine, dried and concentrated *in vacuo.* The residue was purified by flash chromatography on silica gel (eluant; 4% methanol:96% dichloromethane to 8% methanol:92% dichloromethane) to give a white solid identified as the title compound (88mg, 62%).

### Example E84

### 1-(4-Hydroxy-piperidin-1-yl)-3-[2-methyl-4-(3-methyl-4,10-dihydro-3H-2,3,4,9-tetraaza-benzo[f]azulene-9-carbonyl)-phenyl]-propan-1-one

Sodium borohydride (4mg, 0.1mmol) was added to a solution of 1-{3-[2-methyl-4-(3-methyl-4,10-dihydro-3H-2,3,4,9-tetraaza-benzo[f]azulene-9-carbonyl)-phenyl]-propionyl}-piperidin-4-one (Compound number 701) (50mg, 0.11mmol) in methanol/acetic acid (99:1, v/v, 100ml) and the mixture was stirred at room temperature for 1h. Sodium borohydride (4mg, 0.11mmol) was added again and the mixture was stirred for 1h at room temperature then concentrated *in vacuo.* The residue was dissolved in dichloromethane, washed with saturated NaHCO₃ then brine, dried and concentrated *in vacuo* to give a white powder identified as the title compound (28mg, 56%).

### Example E85

### 1-(4-Cyclobutylmethyl-piperazin-1-yl)-2-[2-methyl-4-(3-methyl-4,10-dihydro-3H-2,3,4,9-tetraaza-benzo[f]azulene-9-carbonyl)-phenyl]-ethanone

### Example E85.1

### 4-[2-(4-Cyclobutylmethyl-piperazin-1-yl)-2-oxo-ethyl]-3-methyl-benzoic acid methyl ester

Oxalyl chloride (0.36ml, 3.3mmol) was added to a solution of 4-carboxymethyl-3-methyl-benzoic acid methyl ester from Example E32 (687mg, 3.3mmol) in dichloromethane (20ml) and few drops of DMF. The mixture was stirred for 3h at room temperature, concentrated *in vacuo* and azeotroped with toluene. The residue was dissolved in dichloromethane (10ml) and added to a solution of 1-cyclobutylmethyl-piperazine dihydrochloride (750mg, 3.3mmol) in dichloromethane (10ml) and DIEA (1.73ml, 9.9mmol) at 0°C. The mixture was stirred for 20h at room temperature and concentrated *in vacuo.* The residue was purified by flash chromatography on silica gel (eluant; 3% methanol:97% dichloromethane) to yield the title compound (528mg, 46%).

### Example E85.2

### 4-[2-(4-Cyclobutylmethyl-piperazin-1-yl)-2-oxo-ethyl]-3-methyl-benzoic acid

Lithium hydroxide monohydrate (122mg, 2.9mmol) was added to a solution of 4-[2-(4-cyclobutylmethyl-piperazin-1-yl)-2-oxo-ethyl]-3-methyl-benzoic acid methyl ester from Example E85.1 (500mg, 1.4mmol) in THF (10ml) and water (5ml). The mixture was stirred for 20h at room temperature and concentrated *in vacuo.* The residue was purified by flash chromatography on silica gel (eluant; 3% acetic acid:7% methanol:90% chloroform) to yield the title compound (300mg, 63%).

### Example E85.3

### 1-(4-Cyclobutylmethyl-piperazin-1-yl)-2-[2-methyl-4-(3-methyl-4,10-dihydro-3H-2,3,4,9-tetraaza-benzo[f]azulene-9-carbonyl)-phenyl]-ethanone

WSCD (76mg, 0.39mmol) and DMAP (7mg, 0.06mmol) were added to a solution of 4-[2-(4-cyclobutylmethyl-piperazin-1-yl)-2-oxo-ethyl]-3-methyl-benzoic acid from Example E85.2 (100mg, 0.30mmol) and 3-methyl-3,4,9,10-tetrahydro-2,3,4,9-tetraaza-benzo[f]azulene from Example E11 (61mg, 0.30mmol) in dichloromethane) (20ml) and DIEA (0.16ml, 0.91mmol). The mixture was heated at reflux for 20h and concentrated *in vacuo*. The residue was purified by flash chromatography on silica gel (eluant; 4% methanol:96% dichloromethane to 6% methanol:94% dichloromethane to 1% 35% ammonia:7% methanol:92% dichloromethane) to give an off-white solid identified as the title compound (50mg, 32%).

### Example E86

### 1-(4-Cyclopropylaminomethyl-piperidin-1-yl)-3-[2-methyl-4-(3-methyl-4,10-dihydro-3H-2,3,4,9-tetraaza-benzo[f]azulene-9-carbonyl)-phenyl]-propan-1-one

### Example E86.1

### 4-(3-{4-[(tert-Butoxycarbonyl-cyclopropyl-amino)-methyl]-piperidin-1-yl}-3-oxo-propyl)-3-methyl-benzoic acid methyl ester

Oxalyl chloride (1.26ml, 14.4mmol) was added to a solution of 4-(2-carboxy-ethyl)-3-methyl-benzoic acid methyl ester from Example E30.3 (1.60g, 7.2mmol) in dichloromethane (50ml) and few drops of DMF. The mixture was stirred for 1h at room temperature, concentrated *in vacuo* and azeotroped with toluene. The residue was dissolved in dichloromethane (50ml) and DIEA (2.5ml, 14.4mmol) and cyclopropyl-piperidin-4-ylmethyl-carbamic acid tert-butyl ester from Example E38 (1.83g, 7.2mmol) in dichloromethane (5ml) was added. The mixture was stirred for 2h at room temperature, diluted with dichloromethane, washed with saturated NaHCO₃ then brine, dried and concentrated *in vacuo*. The residue was purified by flash chromatography on silica gel (eluant; 50% EtOAc:50% cyclohexane) to yield the title compound (1.42g, 43%).

### Example E86.2

### 4-(3-{4-[(tert-Butoxycarbonyl-cyclopropyl-amino)-methyl]-piperidin-1-yl}-3-oxo-propyl)-3-methyl-benzoic acid

Lithium hydroxide monohydrate (142mg, 3.4mmol) was added to a solution of 4-(3-{4-[(tert-butoxycarbonyl-cyclopropyl-amino)-methyl]-piperidin-1-yl}-3-oxo-propyl)-3-methyl-benzoic acid methyl ester from Example E86.1 (1.41g, 3.1mmol) in THF (20ml) and water (2ml). The mixture was stirred for 16h at room temperature then 1M NaOH solution (5ml) was added. The mixture was heated at 60°C for 3h then concentrated *in vacuo.* The residue was dissolved in EtOAc, washed with 1M HCl then brine, dried and concentrated *in vacuo* to yield the title compound (1.25g, 91%).

### Example E86.3

### Cyclopropyl-(1-{3-[2-methyl-4-(3-methyl-4,10-dihydro-3H-2,3,4,9-tetraaza-benzo[f]azulene-9-carbonyl)-phenyl]-propionyl}-piperidin-4-ylmethyl)-carbamic acid tert-butyl ester

WSCD (1.08g, 5.6mmol) and DMAP (343mg, 2.8mmol) were added to a solution of 4-(3-{4-[(tert-butoxycarbonyl-cyclopropyl-amino)-methyl]-piperidin-1-yl}-3-oxopropyl)-3-methyl-benzoic acid from Example E86.2 (1.25g, 2.8mmol) in dichloromethane (100ml) and triethylamine (0.783ml, 5.6mmol). The mixture was stirred for 1h at room temperature then 3-methyl-3,4,9,10-tetrahydro-2,3,4,9-tetraaza-benzo[f]azulene from Example E11 (676mg, 3.4mmol) was added. The mixture was heated at reflux for 2 days, washed with saturated NaHCO₃ then brine, dried and concentrated *in vacuo*. The residue was purified by flash chromatography on silica gel (eluant; 2% methanol:98% EtOAc to 5% methanol:95% EtOAc) to yield the title compound (1.1g, 62%).

### Example E86.4

### 1-(4-Cyclopropylaminomethyl-piperidin-1-yl)-3-[2-methyl-4-(3-methyl-4,10-dihydro-3H-2,3,4,9-tetraaza-benzo[f]azulene-9-carbonyl)-phenyl]-propan-1-one

Cyclopropyl-(1-{3-[2-methyl-4-(3-methyl-4,10-dihydro-3H-2,3,4,9-tetraaza-benzo[f]azulene-9-carbonyl)-phenyl]-propionyl}-piperidin-4-ylmethyl)-carbamic acid tert-butyl ester from Example E86.3 (220mg, 0.35mmol) was dissolved in methanol (5ml) and 4M HCl solution in dioxan (5ml) was added. The mixture was stirred for 2h at room temperature then concentrated *in vacuo* to give an off-white solid identified as the title compound (189mg, 95%).

### Example E87

### N-Cyclopropyl-3-[2-methyl-4-(3-methyl-4,10-dihydro-3H-2,3,4,9-tetraaza-benzo[f]azulene-9-carbonyl)-phenyl]-N-piperidin-4-ylmethyl-propionamide hydrochloride

### Example E87.1

### 4-[(Cyclopropyl-{3-[2-methyl-4-(3-methyl-4,10-dihydro-3H-2,3,4,9-tetraaza-benzo[f]azulene-9-carbonyl)-phenyl]-propionyl}-amino)-methyl]-piperidine-1-carboxylic acid tert-butyl ester

PyBroP (238mg, 0.51mmol) was added to a solution of 3-[2-methyl-4-(3-methyl-4,10-dihydro-3H-2,3,4,9-tetraaza-benzo[f]azulene-9-carbonyl)-phenyl]-propionic acid from Example E45 (200mg, 0.51mmol) and 4-cyclopropylaminomethyl-piperidine-1-carboxylic acid tert-butyl ester (130mg, 0.51mmol) in dichloromethane (10ml) and DIEA (0.18ml, 1.02mmol). The mixture was stirred for 4 days at room temperature, diluted with EtOAc, washed with 5% KHCO₃ then brine, dried and concentrated *in vacuo.* The residue was purified by flash chromatography on silica gel (eluant; 5% methanol:95% dichloromethane) to yield the title compound (235mg, 73%).

### Example E87.2

### N-Cyclopropyl-3-[2-methyl-4-(3-methyl-9,10-dihydro-3H-2,3,4,9-tetraaza-benzo[f]azulene-9-carbonyl)-phenyl]-N-piperidin-4-ylmethyl-propionamide hydrochloride

4-[(Cyclopropyl-{3-[2-methyl-4-(3-methyl-4,10-dihydro-3H-2,3,4,9-tetraaza-benzo[f]azulene-9-carbonyl)-phenyl]-propionyl}-amino)-methyl]-piperidine-1-carboxylic acid tert-butyl ester from Example E87.1 (235mg, 0.37mmol) was dissolved in 4M HCl solution in dioxan (5ml). The mixture was stirred for 1h at room temperature, concentrated *in vacuo* and azeotroped with toluene. The residue was dissolved in 1M HCl, washed twice with dichloromethane and freeze-dried to give an off-white solid identified as the title compound (82mg, 39%).

### Example E88

### 1-Methyl-5-(3-methyl-2-nitro-phenylamino)-1H-pyrazole-4-carboxylic acid ethyl ester

Cesium carbonate (35.2g, 108mmol), 4,5-bis(diphenylphosphino)-9,9-dimethylxanthene (0.4g, 0.7mmol) and tris(dibenzylideneacetone)dipalladium(0) (0.2g, 0.2mmol) were added to a solution of ethyl 5-amino-1-methylpyrazole-4-carboxylate (15.3g, 90mmol) in dioxan (50ml) under an inert atmosphere. A solution of 3-bromo-2-nitrotoluene (16.2g, 75mmol) in dioxane (10ml) was added and the mixture was stirred for 30min at room temperature then heated at reflux for 72h. The suspension was filtered through Celite® filter agent, washed with EtOAc and the filtrate was concentrated *in vacuo.* The residue was purified by flash chromatography on silica gel (eluant; 50% EtOAc:50% pet. ether) then recrystallised from EtOAc/pet. ether to yield the title compound (12.5g, 45%).

### Example E89

### 5-Amino-1-ethyl-1H-pyrazole-4-carboxylic acid ethyl ester

Triethylamine (19.6ml, 144mmol) was added cautiously to a solution of ethyl (ethoxymethylene)cyanoacetate (10.1g, 60mmol) and ethyl hydrazine oxalate (9.9g, 66mmol) in ethanol (200ml). The mixture was heated at reflux for 24h then concentrated *in vacuo.* The residue was redissolved in EtOAc, washed with 5% KHCO₃ solution then brine, dried and concentrated *in vacuo.* The residue was purified by flash chromatography on silica gel (eluant; 25% pet. ether:75% EtOAc) to yield the title compound (8.8g, 80%).

### Example E90

### 1-Cyclopropylmethyl-piperidine-4-carboxylic acid 3-fluoro-4-(3-methyl-4,10-dihydro-3H-2,3,4,9-tetraaza-benzo[f]azulene-9-carbonyl)-benzylamide

### Example E90.1

### [3-Fluoro-4-(3-methyl-4,10-dihydro-3H-2,3,4,9-tetraaza-benzo[f]azulene-9-carbonyl)-benzyl]-carbamic acid tert-butyl ester

A mixture of 4-(tert-butoxycarbonylamino-methyl)-2-fluoro-benzoic acid from Example E2 (1.35g, 5.0mmol), 3-methyl-3,4,9,10-tetrahydro-2,3,4,9-tetraaza-benzo[f]azulene from Example E11 (1.0g, 5.0mmol) and PyBroP (3.03g, 6.5mmol) in dichloromethane (25ml) and DIEA (1.31ml, 7.5mmol) was heated at reflux for 18 h. After cooling to room temperature, the mixture was washed with 5% KHCO₃, 1M HCl then brine, dried and concentrated *in vacuo.* The residue was purified by flash chromatography on silica gel (eluant; EtOAc) to yield the title compound (1.25g, 56%).

### Example E90.2

### (4-Aminomethyl-2-fluoro-phenyl)-(3-methyl-4,10-dihydro-3H-2,3,4,9-tetraaza-benzo[f]azulen-9-yl)-methanone hydrochloride

[3-Fluoro-4-(3-methyl-4,10-dihydro-3H-2,3,4,9-tetraaza-benzo[f]azulene-9-carbonyl)-benzyl]-carbamic acid tert-butyl ester from Example E90.1 (1.25g, 2.8mmol) was reacted with 4N HCl/dioxan using an analogous procedure to that described for Example E4.2 to yield the title compound (1.0g, 97%).

### Example E90.3

### 1-Cyclopropylmethyl-piperidine-4-carboxylic acid 3-fluoro-4-(3-methyl-4,10-dihydro-3H-2,3,4,9-tetraaza-benzo[f]azulene-9-carbonyl)-benzylamide

HBTU (227mg, 0.6mmol) was added to a solution of 1-cyclopropylmethyl-piperidine-4-carboxylic acid from Example E15a (99mg, 0.45mmol) in DMF (5ml) and DIEA (to pH9). The mixture was stirred at room temperature for 1 h. (4-Aminomethyl-2-fluoro-phenyl)-(3-methyl-4,10-dihydro-3H-2,3,4,9-tetraaza-benzo[f]azulen-9-yl)-methanone hydrochloride from Example E90.2 (116mg, 0.3mmol) was added and the mixture was stirred at room temperature for 18h. Solvents were removed *in vacuo* and azeotroped with toluene. The residue was redissolved in EtOAc, washed with saturated NaHCO₃ then brine, dried and concentrated *in vacuo.* The residue was purified by flash chromatography on silica gel (eluant; 1% 35% ammonia:19% methanol:80% dichloromethane) to give a white solid identified as the title compound (71.5mg, 46%).

### Example E91

### N-[2-Methyl-4-(3-methyl-4,10-dihydro-3H-2,3,4,9-tetraaza-benzo[f]azulene-9-carbonyl)-benzyl]-2-piperidin-4-yl-acetamide hydrochloride

A solution of 4-{[2-methyl-4-(3-methyl-4,10-dihydro-3H-2,3,4,9-tetraaza-benzo[f]azulene-9-carbonyl)-benzylcarbamoyl]-methyl}-piperidine-1-carboxylic acid tert-butyl ester (Compound number 1067) (156mg, 0.27mmol) was reacted with 4N HCl/dioxan using an analogous procedure to that described for Example E4.2 to yield the title compound (135mg, 97%).

### Example E92

### 2-[1-(3,3-Dimethyl-butyl)-piperidin-9-yl]-N-[2-methyl-4-(3-methyl-4,10-dihydro-3H-2,3,4,9-tetraaza-benzo[f]azulene-9-carbonyl)-benzyl]-acetamide

3,3-Dimethylbutyraldehyde (0.048ml, 0.38mmol) was added to a solution of N-[2-methyl-4-(3-methyl-4,10-dihydro-3H-2,3,4,9-tetraaza-benzo[f]azulene-9-carbonyl)-benzyl]-2-piperidin-4-yl-acetamide from Example E91 (130mg, 0.25mmol) in methanol/acetic acid (49:1, v/v, 2.5ml) and the mixture was stirred at room temperature for 1h. Sodium cyanoborohydride (24mg, 0.38mmol) was added, and the mixture was stirred at room temperature for 18h then concentrated *in vacuo.* The residue was dissolved in dichloromethane, washed with saturated Na-HCO₃ then brine, dried and concentrated *in vacuo.* The residue was purified by flash chromatography on silica gel through an isolute (eluant; 1.5% 35% ammonia:15% methanol:83.5% dichloromethane) to give a white solid identified as the title compound (107mg, 75%).

### Example E93

### 1-Propyl-piperidine-4-carboxylic acid 2-chloro-4-(3-methyl-4,10-dihydro-3H-2,3,4,9-tetraaza-benzo[f]azulene-9-carbonyl)-benzylamide

A solution of propionaldehyde (0.29mg, 0.005mmol) in 1,2-dichloroethane (0.05ml) was added to a solution of piperidine-4-carboxylic acid 2-chloro-4-(3-methyl-4,10-dihydro-3H-2,3,4,9-tetraaza-benzo[f]azulene-9-carbonyl)-benzylamide hydrochloride (Compound number 1109) (2.8mg, 0.005mmol) in 1,2-dichloroethane (0.05ml) and DIEA (0.0026ml). The mixture was stirred at room temperature for 1h then a solution of sodium triacetoxyborohydride (1.59mg, 0.0075mmol) in DMF (0.05ml) was added. The mixture was stirred for 20h at room temperature then solvents were removed *in vacuo* to yield the title compound. (ESI)+: [M+H]+= 521.4

### Example E94

### N-[4-(3,6-Dimethyl-4,10-dihydro-3H-2,3,4,9-tetraaza-benzo[f]azulene-9-carbonyl)-2-fluoro-benzyl]-2-(1-hexyl-piperidin-4-yl)-acetamide

A solution of 1-bromohexane (0.83mg, 0.005mmol) in DMF (0.05ml) was added to a solution of N-[4-(3,6-dimethyl-4,10-dihydro-3H-2,3,4,9-tetraaza-benzo[f]azulene-9-carbonyl)-2-fluoro-benzyl]-2-piperidin-4-yl-acetamide hydrochloride (Compound number 1110) (2.82mg, 0.005mmol) in DMF (0.05ml) and triethylamine (0.0021ml). The mixture was stirred for 20h at room temperature then solvents were removed *in vacuo* to yield the title compound. (ESI)+: [M+H]+= 575.7

### Example E95

### N-[4-(6-Chloro-3-methyl-4,10-dihydro-3H-2,3,4,9-tetraaza-benzo[f]azulene-9-carbonyl)-3-methyl-benzyl]-3-(1-propionyl-piperidin-4-yl)-propionamide

A solution of propionyl chloride (0.46mg, 0.005mmol) in dichloromethane (0.05ml) was added to a solution of N-[4-(6-chloro-3-methyl-4,10-dihydro-3H-2,3,4,9-tetraaza-benzo[f]azulene-9-carbonyl)-3-methyl-benzyl]-3-piperidin-4-yl-propionamide hydrochloride (Compound number 1108) (2.97mg, 0.005mmol) in dichloromethane (0.05ml) and triethylamine (0.0035ml). The mixture was stirred at room temperature for 1h then solvents were removed *in vacuo* to yield the title compound. (ESI)+: [M+H]+= 577.4

### Example E96

### N-[4-(6-Chloro-3-methyl-4,10-dihydro-3H-2,3,4,9-tetraaza-benzo[f]azulene-9-carbonyl)-3-methyl-benzyl]-3-(1-ethanesulfonyl-piperidin-4-yl)-propionamide

A solution of ethanesulfonyl chloride (0.64mg, 0.005mmol) in dichloromethane (0.05ml) was added to a solution of N-[4-(6-chloro-3-methyl-4,10-dihydro-3H-2,3,4,9-tetraaza-benzo[f]azulene-9-carbonyl)-3-methylbenzyl]-3-piperidin-4-yl-propionamide hydrochloride (Compound number 1108) (2.97mg, 0.005mmol) in dichloromethane (0.05ml) and triethylamine (0.0035ml). The mixture was stirred at room temperature for 1h then solvents were removed *in vacuo* to yield the title compound. (ESI)+: [M+H]+= 613.4

### Example E97

### 4-{2-[4-(6-Chloro-3-methyl-4-10-dihydro-3H-2,3,4,9-tetraaza-benzo[f]azulene-9-carbonyl)-3-methylbenzylcarbamoyl]-ethyl}-piperidine-1-carboxylic acid 4-nitro-benzyl ester

A solution of 4-nitrobenzyl chloroformate (1.08mg, 0.005mmol) in dichloromethane (0.05ml) was added to a solution of N-[4-(6-chloro-3-methyl-4,10-dihydro-3H-2,3,4,9-tetraaza-benzo[f]azulene-9-carbonyl)-3-methylbenzyl]-3-piperidin-4-yl-propionamide hydrochloride (Compound number 1108) (2.97mg, 0.005mmol) in dichloromethane (0.05ml) and triethylamine (0.0035ml). The mixture was stirred at room temperature for 1h then solvents were removed *in vacuo* to yield the title compound. (ESI)+: [M+H]+= 700.5, 702.5

### Example E98

### Propane-1-sulfonic acid 4-(3,6-dimethyl-4,10-dihydro-3H-2,3,4,9-tetraaza-benzo[f]azulene-9-carbonyl)-2-fluoro-benzylamide

### Example E98.1

### [4-(3,6-Dimethyl-4,10-dihydro-3H-2,3,4,9-tetraaza-benzo[f]azulene-9-carbonyl)-2-fluoro-benzyl]-carbamic acid tert-butyl ester

A mixture of 4-(tert-butoxycarbonylamino-methyl)-3-fluoro-benzoic acid from Example E3 (1.38g, 5.1mmol) and 3,6-dimethyl-3,4,9,10-tetrahydro-2,3,4,9-tetraaza-benzo[f]azulene from Example E2 (1.0g, 4.7mmol) in dichloromethane (20ml) at room temperature was treated with DIEA (2.44ml, 14.0mmol), DMAP (627mg, 5.1mmol) and WSCD (1.16g, 6.1mmol). The solution was heated at reflux for 2 days then washed with saturated NaHCO₃ then brine, dried and concentrated *in vacuo.* The residue was purified by flash chromatography on silica gel through an isolute (eluant; EtOAc) to yield the title compound (1.29g, 59%).

### Example E98.2

### (4-Aminomethyl-3-fluoro-phenyl)-(3,6-dimethyl-4,10-dihydro-3H-2,3,4,9-tetraaza-benzo[f]azulen-9-yl)-methanone hydrochloride

[4-(3,6-Dimethyl-4,10-dihydro-3H-2,3,4,9-tetraaza-benzo[f]azulene-9-carbonyl)-2-fluoro-benzyl]-carbamic acid tert-butyl ester from Example E98.1 (1.29g, 2.8mmol) was reacted with 4N HCl/dioxan using an analogous procedure to that described for Example E4.2 to yield the title compound (1.1g, 99%).

### Example E98.3

### Propane-1-sulfonic acid 4-(3,6-dimethyl-4,10-dihydro-3H-2,3,4,9-tetraaza-benzo[f]azulene-9-carbonyl)-2-fluoro-benzylamide

1-Propanesulfonyl chloride (0.016ml, 0.14mmol) was added to a solution of (4-aminomethyl-3-fluoro-phenyl)-(3,6-dimethyl-4,10-dihydro-3H-2,3,4,9-tetraaza-benzo[f]azulen-9-yl)-methanone hydrochloride from Example E98.2 (50mg, 0.12mmol) in dichloromethane (2ml) and triethylamine (0.038ml, 0.27mmol) at room temperature. The mixture was stirred for 1h then concentrated *in vacuo.* The residue was purified by preparative HPLC (eluant; 0.5% 35% ammonia:9.5% methanol:90% dichloromethane) to give a white solid identified as the title compound (30mg, 51%).

### Example E99

### N-[4-(3,6-Dimethyl-4,10-dihydro-3H-2,3,4,9-tetraaza-benzo[f]azulene-9-carbonyl)-2-fluoro-benzyl]-formamide

Formic acid (0.85ml, 22.5mmol) and acetic anhydride (1.4ml, 13.5mmol) were combined and stirred for 1 h at room temperature. (4-Aminomethyl-3-fluoro-phenyl)-(3,6-dimethyl-4,10-dihydro-3H-2,3,4,9-tetraaza-benzo[f]azulen-9-yl)-methanone hydrochloride from Example E98.2 (113mg, 0.3mmol) was added and the mixture was stirred for 1 h. Water (20ml) was added and the mixture was stirred for 1 h. It was diluted with chloroform, washed with saturated NaHCO₃ then brine, dried and concentrated *in vacuo* to yield a white solid identified as the title compound (48mg, 40%).

### Example E100

### Cyclopropanecarboxylic acid 4-(3,6-dimethyl-4,10-dihydro-3H-2,3,4,9-tetraaza-benzo[f]azulene-9-carbonyl)-2-fluoro-benzylamide

DIEA (0.08ml, 0.46mmol) and cyclopropanecarbonyl chloride (0.013ml, 0.14mmol) were added to a solution of (4-aminomethyl-3-fluoro-phenyl)-(3,6-dimethyl-4,10-dihydro-3H-2,3,4,9-tetraaza-benzo[f]azulen-9-yl)-methanone hydrochloride from Example E98.2 (57mg, 0.14mmol) in dichloromethane (10ml) at 0°C. The mixture was stirred for 90min at room temperature then diluted with dichloromethane, washed with saturated NaHCO₃ then brine, dried and concentrated *in vacuo*. The residue was purified by preparative HPLC (eluant; 0.5% 35% ammonia:9.5% methanol:90% dichloromethane) to give a white solid identified as the title compound (33mg, 54%).

### Example E101

### Cyclopropanecarboxylic acid 4-(3,6-dimethyl-4,10-dihydro-3H-2,3,4,9-tetraaza-benzo[f]azulene-9-carbonyl)-2-methyl-benzylamide

### Example E101.1

### 4-(3,6-Dimethyl-4,10-dihydro-3H-2,3,4,9-tetraaza-benzo[f]azulene-9-carbonyl)-2-methyl-benzonitrile

A mixture of 4-cyano-3-methyl-benzoic acid from Example E6 (753mg, 4.7mmol) and thionyl chloride (1.02ml, 14.0mmol) in toluene (50ml) was heated at reflux for 2 h. After cooling at room temperature, solvents were removed *in vacuo* and azeotroped with toluene. The residue was redissolved in dichloromethane (45ml) and a solution of 3,6-dimethyl-3,4,9,10-tetrahydro-2,3,4,9-tetraaza-benzo[f]azulene from Example E2 (1.0g, 4.7mmol) in dichloromethane (5ml) and triethylamine (1.3ml, 9.3mmol) was added slowly. The mixture was stirred at room temperature for 20h, washed with brine, dried and concentrated *in vacuo.* The residue was purified by flash chromatography on silica gel through an isolute (eluant; EtOAc) to yield the title compound (1.42g, 85%).

### Example E101.2

### (4-Aminomethyl-3-methyl-phenyl)-(3,6-dimethyl-4,10-dihydro-3H-2,3,4,9-tetraaza-benzo[f]azulen-9-yl)-methanone

A solution of 4-(3,6-dimethyl-4,10-dihydro-3H-2,3,4,9-tetraaza-benzo[f]azulene-9-carbonyl)-2-methyl-benzonitrile from Example E101.1 (1.42g, 4.0mmol) in methanol (50ml) at 0°C was treated with cobalt(II) chloride hexahydrate (1.90g, 8.0mmol). The mixture was stirred for 15min at room temperature then sodium borohydride (1.51g, 40.0mmol) was added portionwise. The reaction mixture was stirred for 2h then saturated NH₄Cl was added. The mixture was stirred for 30min then the solid was filtered off through Celite^{®} filter agent and the filtrate concentrated *in vacuo.* The residue was redissolved in EtOAc and saturated NaHCO₃. The layers were partitioned and the aqueous layer was extracted further with chloroform. Organics were combined, dried and concentrated *in vacuo.* The residue was purified by flash chromatography on silica gel through an isolute (eluant; 1% 35% ammonia:9% methanol:90% dichloromethane) to yield the title compound (511mg, 35%).

### Example E101.3

### Cyclopropanecarboxylic acid 4-(3,6-dimethyl-4,10-dihydro-3H-2,3,4,9-tetraaza-benzo[f]azulene-9-carbonyl)-2-methyl-benzylamide

Triethylamine (0.145ml, 1.04mmol) and cyclopropanecarbonyl chloride (0.041ml, 0.45mmol) were added to a solution of (4-aminomethyl-3-methyl-phenyl)-(3,6-dimethyl-4,10-dihydro-3H-2,3,4,9-tetraazabenzo[f]azulen-9-yl)-methanone from Example E101.2 (150mg, 0.41mmol) in dichloromethane) (10ml) at room temperature. The mixture was stirred for 20h then concentrated *in vacuo*. The residue was purified by preparative HPLC (eluant; 0.5% 35% ammonia:9.5% methanol:90% dichloromethane) to give an off-white solid identified as the title compound (91mg, 51%).

### Example E102

### Cyclopropanecarboxylic acid 4-(6-chloro-3-methyl-4,10-dihydro-3H-2,3,4,9-tetraazabenzo[f]azulene-9-carbonyl)-2-fluoro-benzylamide

### Example E102.1

### [4-(6-Chloro-3-methyl-4,10-dihydro-3H-2,3,4,9-tetraaza-benzo[f]azulene-9-carbonyl)-2-fluoro-benzyl]-carbamic acid tert-butyl ester

A solution of 4-(tert-butoxycarbonylamino-methyl)-3-fluoro-benzoic acid from Example E3 (568mg, 2.1mmol) in dichloromethane (25ml) at room temperature was treated with DMAP (256mg, 2.1mmol), DIEA (1.1ml, 6.3mmol) then WSCD (520mg, 2.7mmol). The mixture was stirred for 3 h then 6-chloro-3-methyl-3,4,9,10-tetrahydro-2,3,4,9-tetraaza-benzo[f]azulene from Example E1 (450mg, 1.9mmol) was added. The mixture was stirred for 4 days then washed with saturated NaHCO₃ then brine, dried and concentrated *in vacuo.* The residue was purified by flash chromatography on silica gel (eluant; EtOAc) to yield the title compound (503mg, 54%).

### Example E102.2

### (4-Aminomethyl-3-fluoro-phenyl)-(6-chloro-3-methyl-4,10-dihydro-3H-2,3,4,9-tetraaza-benzo[f]azulen-9-yl)-methanone hydrochloride

[4-(6-Chloro-3-methyl-4,10-dihydro-3H-2,3,4,9-tetraaza-benzo[f]azulene-9-carbonyl)-2-fluoro-benzyl]-carbamic acid tert-butyl ester from Example E102.1 (503mg, 1.03mmol) was reacted with 4N HCl/dioxan using an analogous procedure to that described for Example E4.2 to yield the title compound (440mg, 100%).

### Example E102.3

### Cyclopropanecarboxylic acid 4-(6-chloro-3-methyl-4,10-dihydro-3H-2,3,4,9-tetraazabenzo[f]azulene-9-carbonyl)-2-fluoro-benzylamide

Cyclopropanecarbonyl chloride (0.045ml, 0.5mmol) was added to a solution of (4-aminomethyl-3-fluoro-phenyl)-(6-chloro-3-methyl-4,10-dihydro-3H-2,3,4,9-tetraazabenzo[f]azulen-9-yl)-methanone hydrochloride from Example E102.2 (210mg, 0.5mmol) in dichloromethane (40ml) and DIEA (0.30ml, 1.7mmol) at 0°C. The mixture was stirred for 1h at room temperature then washed with saturated NaHCO₃, brine, dried and concentrated *in vacuo.* The residue was purified by preparative HPLC (eluant; 0.5% 35% ammonia:9.5% methanol:90% dichloromethane) to give a white powder identified as the title compound (134mg, 59%).

### Example E103

### N-[4-(6-Chloro-3-methyl-9,10-dihydro-3H-2,3,4,9-tetraaza-benzo[f]azulene-9-carbonyl)-2-fluoro-benzyl]-propionamide

DIEA (0.27ml, 1.56mmol) and propionyl chloride (0.048ml, 0.55mmol) were added to a solution of (4-aminomethyl-3-fluoro-phenyl)-(6-chloro-3-methyl-4,10-dihydro-3H-2,3,4,9-tetraaza-benzo[f]azulen-9-yl)-methanone hydrochloride from Example E102.2 (220mg, 0.52mmol) in dichloromethane (6ml) at room temperature. The mixture was stirred for 2h, washed with saturated NaHCO₃ then brine, dried and concentrated *in vacuo.* The residue was purified by preparative HPLC (eluant; 0.5% 35% ammonia:9.5% methanol:90% dichloromethane) to give a white powder identified as the title compound (129mg, 56%) .

### Example E104

### N-[4-(6-Chloro-3-methyl-4,10-dihydro-3H-2,3,4,9-tetraaza-benzo[f]azulene-9-carbonyl)-2-fluoro-benzyl]-butyramide

DIEA (0.135ml, 0.78mmol) and butyryl chloride (0.027ml, 0.27mml) were added to a solution of (4-aminomethyl-3-fluoro-phenyl)-(6-chloro-3-methyl-4,10-dihydro-3H-2,3,4,9-tetraaza-benzo[f]azulen-9-yl)-methanone hydrochloride from Example E102.2 (110mg, 0.26mmol) in dichloromethane (3ml) at room temperature. The mixture was stirred for 1h, washed with saturated NaHCO₃ then brine, dried and concentrated *in vacuo.* The residue was purified by preparative HPLC (eluant; 0.5% 35% ammonia:9.5% methanol:90% dichloromethane) to give a white powder identified as the title compound (77mg, 65%).

### Example E105

### N-[4-(6-Chloro-3-methyl-4,10-dihydro-3H-2,3,4,9-tetraaza-benzo[f]azulene-9-carbonyl)-2-fluoro-benzyl]-isobutyramide

DIEA (0.135ml, 0.78mmol) and isobutyryl chloride (0.029ml, 0.27mmol) were added to a solution of (4-aminomethyl-3-fluoro-phenyl)-(6-chloro-3-methyl-4,10-dihydro-3H-2,3,4,9-tetraaza-benzo[f]azulen-9-yl)-methanone hydrochloride from Example E102.2 (110mg, 0.26mmol) in dichloromethane (3ml) at room temperature. The mixture was stirred for 1h, washed with saturated NaHCO₃ then brine, dried and concentrated *in vacuo.* The residue was purified by preparative HPLC (eluant; 0.5% 35% ammonia:9.5% methanol:90% dichloromethane) to give a white powder identified as the title compound (85mg, 71%).

### Example E106

### Cyclopropanecarboxylic acid 2-chloro-9-(6-chloro-3-methyl-4,10-dihydro-3H-2,3,4,9-tetraazabenzo[f]azulene-9-carbonyl)-benzylamide

### Example E106.1

### [2-Chloro-4-(6-chloro-3-methyl-4,10-dihydro-3H-2,3,4,9-tetraaza-benzo[f]azulene-9-carbonyl)-benzyl]-carbamic acid tert-butyl ester

A solution of 4-(tert-butoxycarbonylamino-methyl)-3-chloro-benzoic acid from Example E2b (568mg, 2.0mmol) in dichloromethane (25ml) at room temperature was treated with DMAP (244mg, 2.0mmol), DIEA (1.0ml, 6.0mmol) then WSCD (500mg, 2.6mmol). The mixture was stirred for 3 h then 6-chloro-3-methyl-3,4,9,10-tetrahydro-2,3,4,9-tetraaza-benzo[f]azulene from Example E1 (430mg, 1.8mmol) was added. The mixture was stirred for 4 days then washed with saturated NaHCO₃, brine, dried and concentrated *in* vacuo. The residue was purified by flash chromatography on silica gel (eluant; EtOAc) to yield the title compound (570mg, 63%).

### Example E106.2

### (4-Aminomethyl-3-chloro-phenyl)-(6-chloro-3-methyl-4,10-dihydro-3H-2,3,4,9-tetraaza-benzo[f]azulen-9-yl)-methanone hydrochloride

[2-Chloro-4-(6-chloro-3-methyl-4,10-dihydro-3H-2,3,4,9-tetraaza-benzo[f]azulene-9-carbonyl)-benzyl]-carbamic acid tert-butyl ester from Example E106.1 (570mg, 1.13mmol) was reacted with 4N HCl/dioxan using an analogous procedure to that described for Example E4.2 to yield the title compound (490mg, 100%).

### Example E106.3

### Cyclopropanecarboxylic acid 2-chloro-4-(6-chloro-3-methyl-4,10-dihydro-3H-2,3,4,9-tetraazabenzo[f]azulene-9-carbonyl)-benzylamide

Cyclopropanecarbonyl chloride (0.045ml, 0.5mmol) was added to a solution of (4-aminomethyl-3-chloro-phenyl)-(6-chloro-3-methyl-4,10-dihydro-3H-2,3,4,9-tetraazabenzo[f]azulen-9-yl)-methanone hydrochloride from Example E106.2 (220mg, 0.5mmol) in dichloromethane) (40ml) and DIEA (0.30ml, 1.7mmol) at 0°C. The mixture was stirred for 1h at room temperature then washed with saturated NaHCO₃, brine, dried and concentrated *in vacuo*. The residue was purified by preparative HPLC (eluant; 0.5% 35% ammonia:9.5% methanol:90% dichloromethane) to give a white powder identified as the title compound (135mg, 57%).

### Example E107

### N-[2-Chloro-4-(6-chloro-3-methyl-4,10-dihydro-3H-2,3,4,9-tetraaza-benzo[f]azulene-9-carbonyl)-benzyl]-butyr amide

Butyryl chloride (46mg, 0.4mmol) was added to a solution of (4-aminomethyl-3-chloro-phenyl)-(6-chloro-3-methyl-9,10-dihydro-3H-2,3,4,9-tetraaza-benzo[f]azulen-9-yl)-methanone hydrochloride from Example E106.2 (161mg, 0.4mmol) in dichloromethane (10ml) and triethylamine (to pH9) at room temperature. The mixture was stirred for 20h, diluted with EtOAc, washed with saturated NaHCO₃ then brine, dried and concentrated *in vacuo.* The residue was purified by preparative HPLC (eluant; 0.5% 35% ammonia:9.5% methanol:90% dichloromethane) to give a white solid identified as the title compound (110mg, 58%).

### Example E108

### N-[2-Chloro-4-(6-chloro-3-methyl-4,10-dihydro-3H-2,3,4,9-tetraaza-benzo[f]azulene-9-carbonyl)-benzyl]-isobutyramide

Isobutyryl chloride (46mg, 0.4mmol) was added to a solution of (4-aminomethyl-3-chloro-phenyl)-(6-chloro-3-methyl-4,10-dihydro-3H-2,3,4,9-tetraaza-benzo[f]azulen-9-yl)-methanone hydrochloride from Example E106.2 (161mg, 0.4mmol) in dichloromethane (10ml) and triethylamine (to pH9) at room temperature. The mixture was stirred for 18h, diluted with EtOAc, washed with saturated NaHCO₃ then brine, dried and concentrated *in vacuo.* The residue was purified by preparative HPLC (eluant; 0.5% 35% ammonia:9.5% methanol:90% dichloromethane) to give a white solid identified as the title compound (75mg, 40%).

### Example E109

### {4-[(Cyclopropylmethyl-amino)-methyl]-3-methylphenyl}(3-methyl-4,10-dihydro-3H-2,3,4,9-tetraaza-benzo[f]azulen-9-yl)-methanone

Cyclopropanecarboxaldehyde (26mg, 0.34 mmol) was added to a solution of (4-Aminomethyl-3-methyl-phenyl)-(3-methyl-4,10-dihydro-3H-2,3,4,9-tetraaza-benzo[f]azulen-9-yl)-methanone (131mg, 0.38 mmol) in MeOH/AcOH (99:1, 5ml) and the mixture was stirred at room temperature for 30 min. Sodium cyanoborohydride (31mg, 0.49 mmol) was added and the mixture was stirred at room temperature for 18 h. The solvents were removed in vacuo and the residue was dissolved in EtOAc. The solution was washed with sat. aq. NaHCO₃ and brine, then dried and concentrated *in vacuo.* The residue was purified by preparative HPLC (eluant; 0.5% 35% ammonia:9.5% methanol:90% dichloromethane to give a white solid identified as the title compound (70mg, 51%).

### Example E110

### [4-(6-Chloro-3-methyl-4,10-dihydro-3H-2,3,4,9-tetraaza-benzo[f]azulene-9-carbonyl)-2-fluoro-benzylamino]-acetic acid methyl ester and

### Example E111

### {[4-(6-Chloro-3-methyl-4,10-dihydro-3H-2,3,4,9-tetraaza-benzo[f]azulene-9-carbonyl)-2-fluoro-benzyl]-methoxycarbonylmethyl-amino}-acetic acid methyl ester

Methyl bromoacetate (0.054ml, 0.58mmol) was added to a solution of (4-aminomethyl-3-fluoro-phenyl)-(6-chloro-3-methyl-4,10-dihydro-3H-2,3,4,9-tetraaza-benzo[f]azulen-9-yl)-methanone hydrochloride from example E102.2 (107mg, 0.28mmol) and DIEA (0.26ml, 1.53mmol) in THF (25ml) and the mixture was stirred at room temperature for 2h. A further amount of methyl bromoacetate (0.10ml, 1.11mmol) was added, and the mixture was stirred overnight at room temperature. The solution was quenched with water and diluted with EtOAc. The organic layer was washed with brine (x 3), dried and concentrated *in vacuo*. The residue was purified by preparative HPLC (eluant; 0.5% 35% ammonia:9.5% methanol:90% dichloromethane) to yield two products. The faster eluting product was isolated as a white solid and identified as E111 (68mg, 46%).

The slower eluting product was isolated as a white solid and identified as E110 (13mg, 10%).

### Example E112

### (6-Chloro-3-methyl-4,10-dihydro-3H-2,3,9,9-tetraaza-benzo[f]azulen-9-yl)-{3-fluoro-4-[(2-hydroxy-ethylamino)-methyl]-phenyl}-methanone

Lithium aluminium hydride (14.5mg, 0.38mmol) was added to a solution of [4-(6-chloro-3-methyl-4,10-dihydro-3H-2,3,4,9-tetraaza-benzo[f]azulene-9-carbonyl)-2-fluoro-benzylamino]-acetic acid methyl ester from example E110 (58.5mg, 0.13mmol) in THF (5ml), and the mixture was stirred at room temperature for 30 min. Before being quenched with MeOH and extracted with EtOAc. The organic extract was washed with brine, dried and concentrated *in vacuo.* The residue was purified by flash chromatography on silica gel (eluant; 10% methanol:90% dichloromethane then 20% methanol:80% dichloromethane) to give a pale yellow solid identified as the title compound (15mg, 28%).

### Example E113

### {[4-(6-Chloro-3-methyl-4,10-dihydro-3H-2,3,4,9-tetraaza-benzo[f]azulene-9-carbonyl)-2-fluoro-benzylcarbamoyl]-methyl}-carbamic acid tert-butyl ester

HBTU (165mg, 0.44mmol) was added to a solution of N-(tert-butoxycarbonyl)glycine (65mg, 0.37mmol) in DMF (10ml) and the mixture was stirred for 30min at room temperature. (4-Aminomethyl-3-fluoro-phenyl)-(6-chloro-3-methyl-4,10-dihydro-3H-2,3,4,9-tetraaza-benzo[f]azulen-9-yl)-methanone hydrochloride from Example E102.2 (100mg, 0.22mmol) and DIEA (0.228ml, 1.31mmol) were added and the mixture was stirred for 24h at room temperature. The solution was diluted with EtOAc, washed with water then brine, dried and concentrated *in vacuo*. The residue was purified by flash chromatography on silica gel (eluant; 5% methanol:95% dichloromethane then 10% methanol:90% dichloromethane) to give a white solid identified as the title compound (123mg, 100%).

### Example E114

### 2-Amino-N-[4-(6-chloro-3-methyl-4,10-dihydro-3H-2,3,4,9-tetraaza-benzo[f]azulene-9-carbonyl)-2-fluorobenzyl]-acetamide hydrochloride

{[4-(6-Chloro-3-methyl-4,10-dihydro-3H-2,3,4,9-tetraaza-benzo[f]azulene-9-carbonyl)-2-fluoro-benzylcarbamoyl]-methyl}-carbamic acid tert-butyl ester from Example E113 (120mg, 0.22mmol) was reacted with 4N HCl/dioxan using an analogous procedure to that described for Example E4.2 to yield the title compound (95mg, 83%).

### Example E115

### 4-[(3-Chloro-benzoylamino)-methyl]-N-(4-chloro-phenyl)-3,N-dimethyl-benzamide

### Example E115.1

### N-(4-Chloro-phenyl)-4-cyano-3,N-dimethyl-benzamide

A mixture of 4-cyano-3-methyl-benzoic acid from Example E6 (3.4g, 21mmol) and thionyl chloride (10ml, 137mmol) in dichloromethane (50ml) was heated at reflux for 2 h. After cooling at room temperature, solvents were removed *in vacuo* and azeotroped with toluene. The residue was redissolved in dichloromethane (50ml). (4-Chlorophenyl)-methyl amine (3.0g, 21mmol) and triethylamine (to pH9) were added and the mixture was stirred at room temperature for 20h then concentrated *in vacuo*. The residue was redissolved in EtOAc, washed with 1M KHSO₄, water then brine, dried and concentrated *in vacuo.* The residue was purified by flash chromatography on silica gel (eluant; 35% EtOAc:65% cyclohexane) to yield the title compound (5.1g, 85%).

### Example E115.2

### 4-Aminomethyl-N-(4-chloro-phenyl)-3,N-dimethyl-benzamide

A solution of N-(4-chloro-phenyl)-4-cyano-3,N-dimethyl-benzamide from Example E115.1 (5.1g, 17.9mmol) in methanol (250ml) was treated with cobalt(II) chloride hexahydrate (8.4g, 35.8mmol). The mixture was stirred for 15min at room temperature then cooled down to 0°C and sodium borohydride (6.7g, 179mmol) was added portionwise. The mixture was stirred for 1h at room temperature, filtered through Celite^{®} filter agent, washed with methanol and the filtrate was concentrated in *vacuo.* The residue was purified by flash chromatography on silica gel (eluant; 1% 35% Ammonia:10% methanol:89% dichloromethane) to yield the title compound (3.6g, 70%).

### Example E115.3

### 4-[(3-Chloro-benzoylamino)-methyl]-N-(4-chloro-phenyl)-3,N-dimethyl-benzamide

3-Chlorobenzoyl chloride (61mg, 0.35mmol) and triethylamine (to pH9) were added to a solution of 4-aminomethyl-N-(4-chloro-phenyl)-3,N-dimethyl-benzamide from Example E115.2 (100mg, 0.35mmol) in dichloromethane (5ml) at room temperature. The mixture was stirred for 1h then concentrated *in vacuo.* The residue was purified by preparative HPLC (eluant; 0.5% 35% ammonia:9.5% methanol:90% dichloromethane) to give a white solid identified as the title compound (101mg, 69%).

### Example E116

### 1-Cyclopropylmethyl-piperidine-4-carboxylic acid 4-[(4-chloro-phenyl)-methyl-carbamoyl]-2-methyl-benzylamide

PyBroP (212mg, 0.45mmol) and 1-cyclopropylmethyl-piperidine-4-carboxylic acid from Example E15a (76mg, 0.42mmol) were added to a solution of 4-aminomethyl-N-(4-chloro-phenyl)-3,N-dimethyl-benzamide from Example E115.2 (100mg, 0.35mmol) in dichloromethane (5ml) and DMF (1ml). The mixture was stirred at room temperature for 18h then solvents were removed *in vacuo* and azeotroped with toluene. The residue was purified by preparative HPLC (eluant; 0.5% 35% ammonia:9.5% methanol:90% dichloromethane) to give a white powder identified as the title compound (34mg, 22%).

### Example E117

### 1-(3,3-Dimethyl-butyl)-piperidine-4-carboxylic acid 4-[(4-chloro-phenyl)-methyl-carbamoyl]-2-methyl-benzylamide

HBTU (296mg, 0.78mmol) was added to a solution of 1-(3,3-dimethyl-butyl)-piperidine-4-carboxylic acid from Example E15 (182mg, 0.73mmol) in DMF (5ml) and DIEA (0.272ml, 1.56mmol). The mixture was stirred at room temperature for 2 h then 4-aminomethyl-N-(4-chloro-phenyl)-3,N-dimethyl-benzamide from Example E115.2 (150mg, 0.52mmol) was added. The mixture was stirred for 2 h at room temperature then diluted with EtOAc, washed with saturated NaHCO₃ then brine, dried and concentrated *in vacuo.* The residue was purified by preparative HPLC (eluant; 0.5% 35% ammonia:9.5% methanol:90% dichloromethane) to give a white powder identified as the title compound (209mg, 83%).

### Example E118

### 1-(3,3-Dimethyl-butyl)-piperidine-4-carboxylic acid 2-methyl-4-[methyl-(4-trifluoromethyl-phenyl)-carbamoyl]-benzylamide

### Example E118.1

### 4-Cyano-3-methyl-N-(4-trifluoromethyl-phenyl)-benzamide

A mixture of 4-cyano-3-methyl-benzoic acid from Example E6 (750mg, 4.6mmol) and thionyl chloride (1.02ml, 14.0mmol) in toluene (40ml) was heated at reflux for 2 h. After cooling at room temperature, solvents were removed *in vacuo* and azeotroped with toluene. The residue was redissolved in dichloromethane (25ml) then 4-trifluoromethylaniline hydrochloride (1.11g, 5.6mmol) in dichloromethane (5ml) and triethylamine (1.95ml, 14.0mmol) was added. The mixture was stirred at room temperature for 2 days, washed with brine, dried and concentrated *in vacuo.* The residue was purified by flash chromatography on silica gel through an isolute (eluant; 20% EtOAc:80% cyclohexane) to yield the title compound (1.13g, 80%).

### Example E118.2

### 4-Cyano-3,N-dimethyl-N-(4-trifluoromethyl-phenyl)-benzamide

4-Cyano-3-methyl-N-(4-trifluoromethyl-phenyl)-benzamide from Example E118.1 (1.10g, 3.6mmol) was dissolved in DMF (10ml) and cooled down to 0°C. Sodium hydride (60% dispersion in oil, 174mg, 4.3mmol) was added and the mixture was stirred for 30min at room temperature. Methyl iodide (0.27ml, 4.3mmol) was added and the mixture was stirred for 20h. Solvents were removed *in vacuo* and azeotroped with toluene. The residue was redissolved in EtOAc, washed with brine, dried and concentrated *in vacuo* to yield the title compound (1.08g, 94%).

### Example E118.3

### 4-Aminomethyl-3,N-dimethyl-N-(4-trifluoromethyl-phenyl)-benzamide

A solution of 4-cyano-3,N-dimethyl-N-(4-trifluoromethyl-phenyl)-benzamide from Example E118.2 (1.08g, 3.4mmol) in methanol (40ml) at room temperature was treated with cobalt(II) chloride hexahydrate (1.61g, 6.8mmol). The mixture was stirred for 10min then sodium borohydride (1.28g, 34mmol) was added portionwise. The mixture was stirred for 20h then saturated NH₄Cl solution (10ml) was added. The mixture was stirred for 30min, filtered through glass-fibre paper and the filtrate was concentrated *in vacuo*. The residue was purified by flash chromatography on silica gel (eluant; 1% 35% ammonia:9% methanol:90% dichloromethane) to yield the title compound (863mg, 79%).

### Example E118.4

### 1-(3,3-Dimethyl-butyl)-piperidine-4-carboxylic acid 2-methyl-4-[methyl-(4-trifluoromethyl-phenyl)-carbamoyl]-benzylamide

PyBroP (188mg, 0.40mmol) and 1-(3,3-dimethyl-butyl)-piperidine-4-carboxylic acid (82mg, 0.38mmol) were added to a solution of 4-aminomethyl-3,N-dimethyl-N-(4-trifluoromethyl-phenyl)-benzamide from Example E118.3 (100mg, 0.31mmol) in dichloromethane (5ml) and DIEA (0.081ml, 0.46mmol). The mixture was stirred at room temperature for 2 days, washed with saturated NaHCO₃ then brine, dried and concentrated *in vacuo*. The residue was purified by preparative HPLC (eluant; 0.5% 35% ammonia:9.5% methanol:90% dichloromethane) to give a off-white powder identified as the title compound (22mg, 14%).

### Example E119

### Cyclopropanecarboxylic acid 2-methyl-4-(2-methyl-4,5-dihydro-1-oxa-3,6-diaza-benzo[e]azulene-6-carbonyl)-benzylamide

### Example E119.1

### [2-Methyl-4-(2-methyl-4,5-dihydro-1-oxa-3,6-diaza-benzo[e]azulene-6-carbonyl)-benzyl]-carbamic acid tert-butyl ester

A solution of 4-(tert-butoxycarbonylamino-methyl)-3-methyl-benzoic acid from Example E2h (400mg, 1.5mmol) in dichloromethane (20ml) and triethylamine (to pH9) was treated with WSCD (573mg, 3.0mmol) and DMAP (183mg, 1.5mmol). The solution was stirred for 30min at room temperature then 2-methyl-5,6-dihydro-4H-1-oxa-3,6-diaza-benzo[e]azulene from Example E6 (300mg, 1.5mmol) was added and the mixture was heated at reflux for 3 days. WSCD (287mg, 1.5mmol) was added and the mixture was heated at reflux for another 24h then concentrated *in vacuo*. The residue was dissolved in EtOAc, washed with 1N KHSO₄, water then brine, dried and concentrated *in vacuo.* The residue was purified by flash chromatography on silica gel (eluant; EtOAc) to yield the title compound (62mg, 9%).

### Example E119.2

### (4-Aminomethyl-3-methyl-phenyl)-(2-methyl-4,5-dihydro-1-oxa-3,6-diaza-benzo[e]azulen-6-yl)-methanone hydrochloride

[2-Methyl-4-(2-methyl-4,5-dihydro-1-oxa-3,6-diaza-benzo[e]azulene-6-carbonyl)-benzyl]-carbamic acid tert-butyl ester from Example E119.1 (62mg, 0.14mmol) in methanol (0.5ml) was reacted with 4N HCl/dioxan using an analogous procedure to that described for Example E4.2 to yield the title compound (53mg, 100%).

### Example E119.3

### Cyclopropanecarboxylic acid 2-methyl-4-(2-methyl-4,5-dihydro-1-oxa-3,6-diaza-benzo[e]azulene-6-carbonyl)-benzylamide

Cyclopropanecarbonyl chloride (26mg, 0.25mmol) was added to a solution of (4-aminomethyl-3-methyl-phenyl)-(2-methyl-4,5-dihydro-1-oxa-3,6-diaza-benzo[e]azulen-6-yl)-methanone hydrochloride from Example E119.2 (85mg, 0.22mmol) in dichloromethane (5ml) and triethylamine (to pH9) at room temperature. The mixture was stirred for 1h then purified by flash chromatography on silica gel (eluant; 10% methanol:90% dichloromethane) to give a white solid identified as the title compound (62mg, 67%).

### Example E120

### 1-(3,3-Dimethyl-butyl)-piperidine-4-carboxylic acid 2-methyl-4-(2-methyl-4,5-dihydro-1-oxa-3,6-diaza-benzo[e]azulene-6-carbonyl)-benzylamide

(4-Aminomethyl-3-methyl-phenyl)-(2-methyl-4,5-dihydro-1-oxa-3,6-diaza-benzo[e]azulen-6-yl)-methanone hydrochloride from Example E119.2 (89mg, 0.225mmol) was dissolved in dichloromethane (10ml) and triethylamine (to pH9) at room temperature. 1-(3,3-Dimethyl-butyl)-piperidine-4-carboxylic acid (53mg, 0.25mmol), DMAP (27mg, 0.225mmol) and PyBroP (165mg, 0.36mmol) were added and the mixture was heated at reflux for 18h. The solution was diluted with EtOAc, washed with saturated NaHCO₃ then brine, dried and concentrated *in vacuo.* The residue was purified by flash chromatography on silica gel (eluant; 1% 35% ammonia: 9%, methanol:90% dichloromethane) to give a white solid identified as the title compound (84mg, 69%).

### Example E121

### 1-(3,3-Dimethyl-butyl)-piperidine-9-carboxylic acid 4-(1-benzyl-2-methyl-4,5-dihydro-1H-1,3,6-triaza-benzo[e]azulene-6-carbonyl)-2-methyl-benzylamide

### Example E121.1

### 4-(1-Benzyl-2-methyl-4,5-dihydro-1H-1,3,6-triaza-benzo[e]azulene-6-carbonyl)-2-methyl-benzonitrile

A mixture of 4-cyano-3-methyl-benzoic acid from Example E6 (557mg, 3.4mmol) and thionyl chloride (0.755ml, 10.3mmol) in toluene (100ml) was heated at reflux for 2 h. After cooling to room temperature, solvents were removed *in vacuo* and azeotroped with toluene. The residue was redissolved in dichloromethane (100ml) and triethylamine (0.962ml, 6.9mmol) then 1-benzyl-2-methyl-1,4,5,6-tetrahydro-1,3,6-triaza-benzo[e]azulene from Example E5a (1.0g, 3.4mmol) was added. The mixture was stirred at room temperature for 2h, washed with saturated NaHCO₃ then brine, dried and concentrated *in vacuo*. The residue was purified by flash chromatography on silica gel (eluant; 50% EtOAc:50% pet. ether then EtOAc) to yield the title compound (844mg, 56%).

### Example E121.2

### (4-Aminomethyl-3-methyl-phenyl)-(1-benzyl-2-methyl-4,5-dihydro-1H-1,3,6-triaza-benzo[e]azulen-6-yl)-methanone

A solution of 4-(1-benzyl-2-methyl-4,5-dihydro-1H-1,3,6-triaza-benzo[e]azulene-6-carbonyl)-2-methyl-benzonitrile from Example E121.1 (830mg, 1.9mmol) in methanol (10ml) at room temperature was treated with cobalt(II) chloride hexahydrate (913mg, 3.8mmol). The mixture was stirred for 15min then cooled down to 0°C. Sodium borohydride (726mg, 19mmol) was added portionwise and the reaction mixture was stirred for 2h at room temperature. 35% Ammonia (5ml) was added dropwise and the mixture was stirred for 30min then concentrated *in vacuo.* The residue was purified by flash chromatography on silica gel (eluant; 1% 35% ammonia:9% methanol:90% dichloromethane) to yield the title compound (680mg, 81%).

### Example E121.3

### 1-(3,3-Dimethyl-butyl)-piperidine-4-carboxylic acid 4-(1-benzyl-2-methyl-4,5-dihydro-1H-1,3,6-triaza-benzo[e]azulene-6-carbonyl)-2-methyl-benzylamide

HBTU (175mg, 0.46mmol) was added to a solution of 1-(3,3-dimethyl-butyl)-piperidine-4-carboxylic acid (86mg, 0.34mmol) in DMF (5ml). The mixture was stirred at room temperature for 2 h then (4-aminomethyl-3-methyl-phenyl)-(1-benzyl-2-methyl-4,5-dihydro-1H-1,3,6-triaza-benzo[e]azulen-6-yl)-methanone from Example E121.2 (100mg, 0.23mmol) and DIEA (0.12ml, 0.69mmol) were added. The mixture was stirred at room temperature for 18h then solvents were removed *in vacuo* and azeotroped with toluene. The residue was purified by flash chromatography on silica gel through an isolute (eluant; 5% methanol:95% dichloromethane then 10% methanol:90% dichloromethane) to give a yellow foam identified as the title compound (114mg, 79%).

### Example E122

### 1-(3,3-Dimethyl-butyl)-piperidine-4-carboxylic acid 2-methyl-4-(2-methyl-4,5-dihydro-1H-1,3,6-triaza-benzo[e]azulene-6-carbonyl)-benzylamide.

1-(3,3-Dimethyl-butyl)-piperidine-4-carboxylic acid 4-(1-benzyl-2-methyl-4,5-dihydro-1H-1,3,6-triaza-benzo[e]azulene-6-carbonyl)-2-methyl-benzylamide from Example E121 (110mg, 0.17mmol) was dissolved in absolute ethanol (5ml) and placed under an inert atmosphere. 10% Palladium on carbon (110mg) and cyclohexene (0.176ml, 1.7mmol) were added and the mixture was heated at 60°C for 3 h. The catalyst was filtered off through Celite® filter agent and the filtrate was concentrated *in vacuo.* The residue was purified by preparative HPLC (eluant; 0.5% 35% ammonia:9.5% methanol:90% dichloromethane) to give a white powder identified as the title compound (45mg, 48%).

### Example E123

### Cyclopropanecarboxylic acid 2-methyl-4-(2-methyl-4,5-dihydro-1H-1,3,6-triaza-benzo[e]azulene-6-carbonyl)-benzylamide

### Example E123.1

### Cyclopropanecarboxylic acid 4-(1-benzyl-2-methyl-4,5-dihydro-1H-1,3,6-triaza-benzo[e]azulene-6-carbonyl)-2-methyl-benzylamide

DIEA (0.176ml, 1.02mmol) and cyclopropanecarbonyl chloride (0.033ml, 0.36mmol) were added to a solution of (4-aminomethyl-3-methyl-phenyl)-(1-benzyl-2-methyl-4,5-dihydro-1H-1,3,6-triaza-benzo[e]azulen-6-yl)-methanone from Example E121.2 (150mg, 0.34mmol) in dichloromethane (5ml) at room temperature. The mixture was stirred for 20h, washed with saturated NaHCO₃ then brine, dried and concentrated *in vacuo.* The residue was purified by preparative HPLC (eluant; 0.5% 35% ammonia:9.5% methanol:90% dichloromethane) to give the title compound (155mg, 90%).

### Example E123.2

### Cyclopropanecarboxylic acid 2-methyl-4-(2-methyl-4,5-dihydro-1H-1,3,6-triaza-benzo[e]azulene-6-carbonyl)-benzylamide

Cyclopropanecarboxylic acid 4-(1-benzyl-2-methyl-4,5-dihydro-1H-1,3,6-triaza-benzo[e]azulene-6-carbonyl)-2-methyl-benzylamide from Example E123.1 (155mg, 0.31mmol) was dissolved in absolute ethanol (10ml) and placed under an inert atmosphere. 10% Palladium on carbon (155mg) and cyclohexene (0.311ml, 3.1mmol) were added and the mixture was heated at 60°C for 18 h. The catalyst was filtered off through Celite® filter agent and the filtrate was concentrated *in vacuo.* The residue was purified by preparative HPLC (eluant; 0.5% 35% ammonia:9.5% methanol:90% dichloromethane) to give a white solid identified as the title compound (67mg, 53%).

### Example E124

### Cyclopropanecarboxylic acid 2-methyl-4-(2-methyl-4,5-dihydro-3-thia-1,6-diaza-benzo[e]azulene-6-carbonyl)-benzylamide

### Example E124.1

### 2-Methyl-4-(2-methyl-4,5-dihydro-3-thia-1,6-diaza-benzo[e]azulene-6-carbonyl)-benzonitrile

A mixture of 4-cyano-3-methyl-benzoic acid from Example E6 (147mg, 0.91mmol) was dissolved in dichloromethane (25ml) and 2 drops of DMF and cooled down to 0°C. Oxalyl chloride (0.1ml) was added slowly and the mixture was stirred for 2 h at room temperature. Solvents were removed *in vacuo* and azeotroped with toluene. The residue was redissolved in dichloromethane (10ml) and cooled down to 0°C. Triethylamine (0.5ml) and 2-methyl-5,6-dihydro-4H-3-thia-1,6-diaza-benzo[e]azulene from Example E7 (165mg, 0.76mmol) in dichloromethane (10ml) were added and the mixture was stirred for 2 days at room temperature. Another 0.91mmol of acid chloride was formed as previously and added to the mixture. The solution was stirred for 20h at room temperature, washed with saturated NaHCO₃ then brine, dried and concentrated *in vacuo.* The residue was purified by flash chromatography on silica gel (eluant; 10% EtOAc:90% cyclohexane then 20% EtOAc:80% cyclohexane) to yield the title compound (220mg, 80%).

### Example E124.2

### (4-Aminomethyl-3-methyl-phenyl)-(2-methyl-4,5-dihydro-3-thia-1,6-diaza-benzo[e]azulen-6-yl)-methanone

A solution of 2-methyl-4-(2-methyl-4,5-dihydro-3-thia-1,6-diaza-benzo[e]azulene-6-carbonyl)-benzonitrile from Example E124.1 (190mg, 0.53mmol) in methanol (15ml) at room temperature was treated with cobalt(II) chloride hexahydrate (275mg, 1.15mmol). The mixture was stirred for 20min then cooled down to 0°C. Sodium borohydride (200mg, 5.3mmol) was added portionwise and the mixture was stirred for 20h at room temperature. Saturated NH₄Cl (2ml) was added dropwise and the mixture was stirred for 30min then concentrated *in vacuo.* The residue was purified by flash chromatography on silica gel (eluant; 1% 35% ammonia:9% methanol:90% dichloromethane) to yield the title compound (100mg, 52%).

### Example E124.3

### Cyclopropanecarboxylic acid 2-methyl-4-(2-methyl-4,5-dihydro-3-thia-1,6-diaza-benzo[e]azulene-6-carbonyl)-benzylamide

DIEA (0.02ml, 0.115mmol) and cyclopropanecarbonyl chloride (0.008ml, 0.088mmol) were added to a solution of (4-aminomethyl-3-methyl-phenyl)-(2-methyl-4,5-dihydro-3-thia-1,6-diaza-benzo[e]azulen-6-yl)-methanone from E124.2 (20mg, 0.055mmol) in dichloromethane (5ml) at 0°C. The mixture was stirred for 20h at room temperature then concentrated *in vacuo* and purified by preparative HPLC (eluant; 0.5% 35% ammonia:9.5% methanol:90% dichloromethane) to give a white powder identified as the title compound (11mg, 46%).

### Example E125

### N-[4-(9-Chloro-2-methyl-4,5-dihydro-1H-1,3,6-triaza-benzo[e]azulene-6-carbonyl)-2-methyl-benzyl] -isobutyramide

### Example E125.1

### 4-[1-(4-Cyano-3-methyl-benzoyl)-9-chloro-2-methyl-4,5-dihydro-1H-1,3,6-triaza-benzo[e]azulene-6-carbonyl]-2-methyl-benzonitrile

A mixture of 4-cyano-3-methyl-benzoic acid from Example E6 (262mg, 1.6mmol) was dissolved in dichloromethane (20ml) and 2 drops of DMF and cooled down to 0°C. Oxalyl chloride (0.6ml) was added and the mixture was stirred for 0.5 h at room temperature: Solvents were removed *in vacuo* and azeotroped with toluene. The residue was redissolved in dichloromethane (10ml) and added to a solution of 9-chloro-2-methyl-1,4,5,6-tetrahydro-1,3,6-triaza-benzo[e]azulene from Example E7 (190mg, 0.8mmol) and DIEA (0.5ml) in dichloromethane (10ml) and the mixture was stirred for 20h. at room temperature. The solvents were removed *in vacuo* and the residue was taken up in EtOAc, washed with saturated NaHCO₃ then brine, dried and concentrated *in vacuo.* The residue was purified by flash chromatography on silica gel (eluant; 40% EtOAc:60% cyclohexane) to yield the title compound (124mg, 30%).

### Example E125.2

### 4-(9-Chloro-2-methyl-4,5-dihydro-1H-1,3,6-triaza-benzo[e]azulene-6-carbonyl)-2-methyl-benzonitrile

4-[1-(4-Cyano-3-methyl-benzoyl)-9-chloro-2-methyl-4,5-dihydro-1H-1,3,6-triaza-benzo[e]azulene-6-carbonyl)-2-methyl-benzonitrile from E125.1 (124mg, 0.24mmol) was dissolved in MeOH 5ml). 2M NaOH (2ml) was added, and the mixture was stirred for 1 h. at room temperature. The solvents were removed *in vacuo*, and azeotroped with toluene. The residue was taken up in EtOAc, was with saturated NaHCO₃ then brine, dried and concentrated *in vacuo* to yield the title compound (80mg, 89%)

### Example E125.3

### (4-Aminomethyl-3-methyl-phenyl)-(9-chloro-2-methyl-4,5-dihydro-1H-1,3,6-triaza-benzo[e]azulen-6-yl)-methanone

A solution of 4-(9-chloro-2-methyl-4,5-dihydro-1H-1,3,6-triaza-benzo[e]azulene-6-carbonyl)-2-methyl-benzonitrile from E125.2 (80mg, 0.2 mmol) in methanol (5ml) at room temperature was treated with cobalt(II) chloride hexahydrate (101mg, 0.4mmol). The mixture was cooled to 0°C then sodium borohydride (80mg, 2.1mmol) was added portionwise. The reaction mixture was stirred at 0°C for 30 min. then for 2h at room temperature then saturated NH₄Cl was added. The mixture was stirred for 10min then the solid was filtered off through Celite^{®} filter agent and the filtrate concentrated in *vacuo.* The residue was purified by flash chromatography on silica gel (eluant; 1% 35% ammonia:9% methanol:90% dichloromethane) to yield the title compound (24mg, 30%).

### Example E125.4

### N-[4-(9-Chloro-2-methyl-4,5-dihydro-1H-1,3,6-triaza-benzo[e]azulene-6-carbonyl)-2-methyl-benzyl]-isobutyramide

Isobutyryl chloride (0.07ml, 0.07mmol) was added to a solution of (4-aminomethyl-3-methyl-phenyl)-(9-chloro-2-methyl-4,5-dihydro-1H-1,3,6-triaza-benzo[e]azulen-6-yl)-methanone from Example E125.3 (24mg, 0.07mmol) in dichloromethane (3ml) and DIEA (0.10ml, 0.57mmol) at 0°C. The mixture was stirred for 20h at room temperature then solvents were removed *in vacuo.* The residue was purified by flash chromatography on silica gel (eluant; 5% methanol:95% dichloromethane to 10% methanol:90% dichloromethane) to give a colourless oil identified as the title compound 24mg, 85%).

### Example A

### In vitro Testing

Compounds were assayed to determine their ability to inhibit the cellular consequences of AVP stimulation on intact cells. In the assay, the compounds of the invention cause significant inhibition of cellular activation at concentrations of 30 µM or less. Preferred compounds cause significant inhibition at concentrations of 300 nM.

### Reference Example B

### Tablet for Oral Administration

Tablets containing 100 mg of the compound of Example E8 as the active agent may be prepared from the following:

| | |
|---|---|
| Compound of Example E8 | 200.0 g |
| Corn starch | 71.0 g |
| Hydroxypropylcellulose | 18.0 g |
| Carboxymethylcellulose calcium | 13.0 g |
| Magnesium stearate | 3.0 g |
| Lactose | 195.0 g |
| *Total* | *500.0 g* |

The materials are blended and then pressed to give 2000 tablets of 250 mg, each containing 100 mg of the compound of Example E8.

### SCHEMES AND STRUCTURES

### Example E1

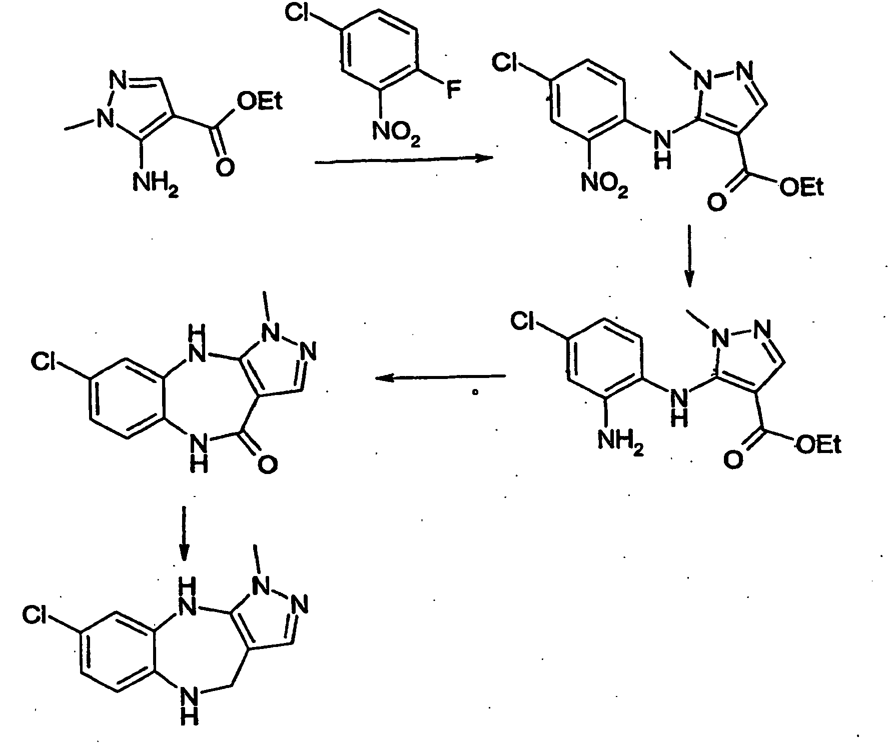

### Example E2

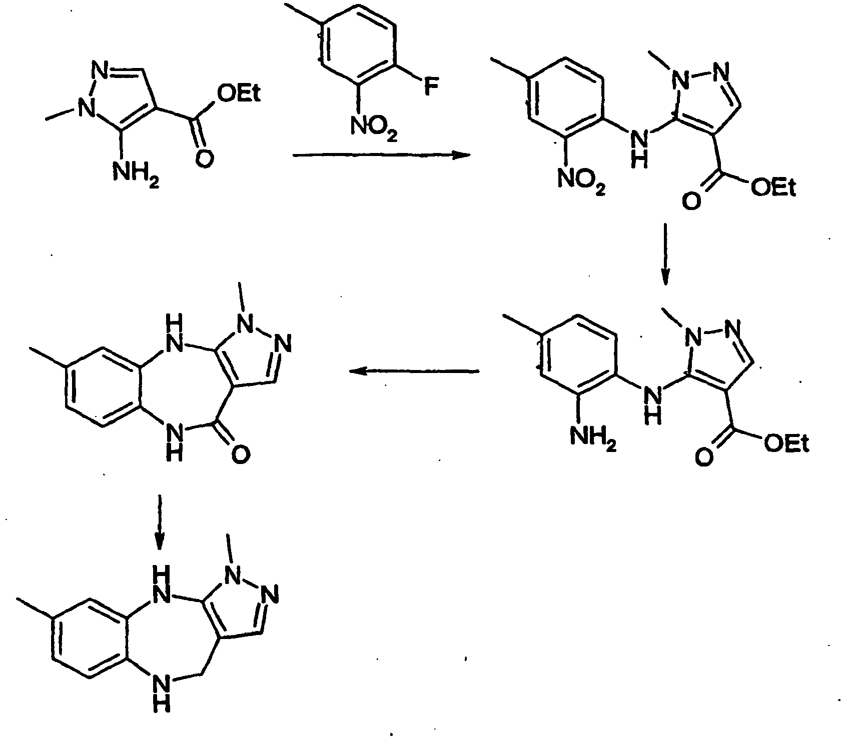

### Examples E2a-g

| | |
|---|---|
| 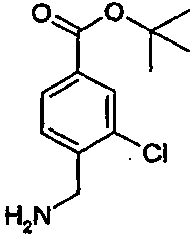 | 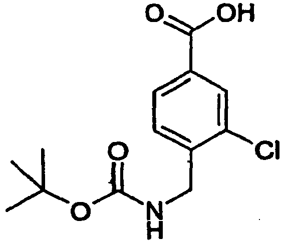 |
| Example E2a 4-Aminomethyl-3-chlorobenzoic acid tert-butyl ester | Example E2b 4-(tert-Butoxycarbonylaminomethyl)-3-chloro-benzoic acid |
| 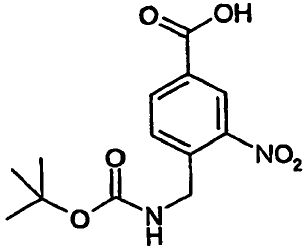 | 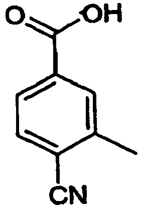 |
| Example E2c 4-(tert-Butoxycarbonylaminomethyl)-3-nitro-benzoic acid | Example E2d 4-Cyano-3-methyl-benzoic acid |
| 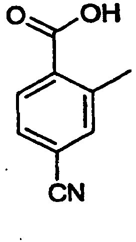 | 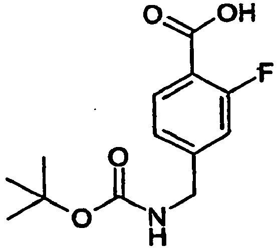 |
| Example E2e 4-Cyano-2-methyl-benzoic acid | Example E2f 4-(tert-Butoxycarbonylamino-methyl)-2-fluoro-benzoic acid |
| 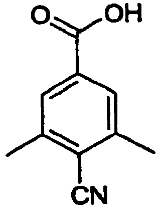 | |
| Example E2g 4-Cyano-3,5-dimethyl-benzoic acid | |

### Example E2h

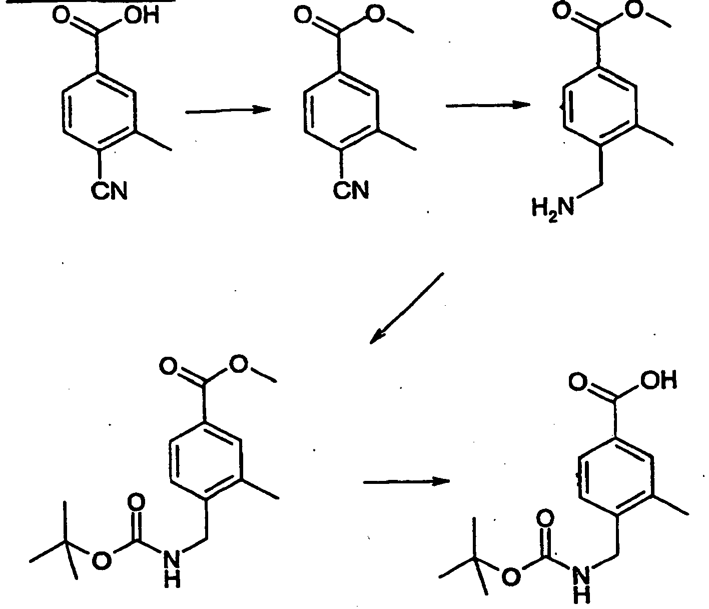

### Example E3

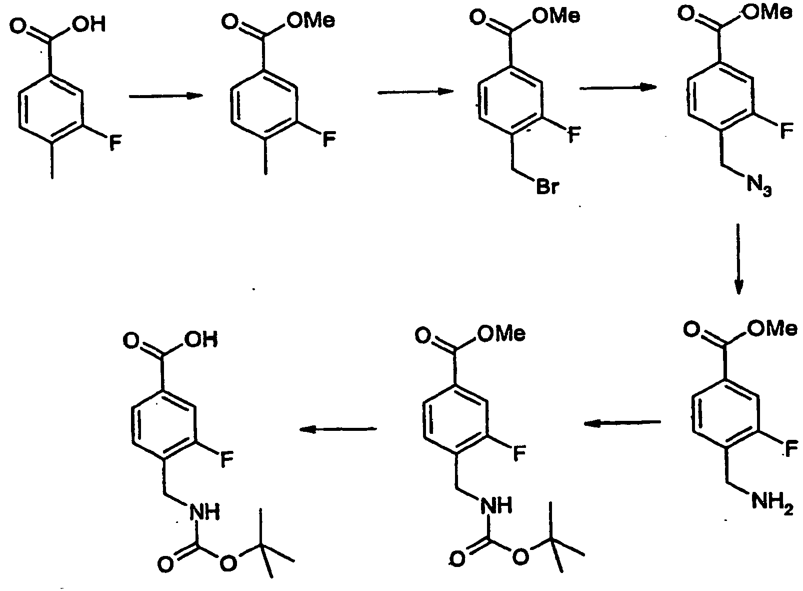

### Example E4

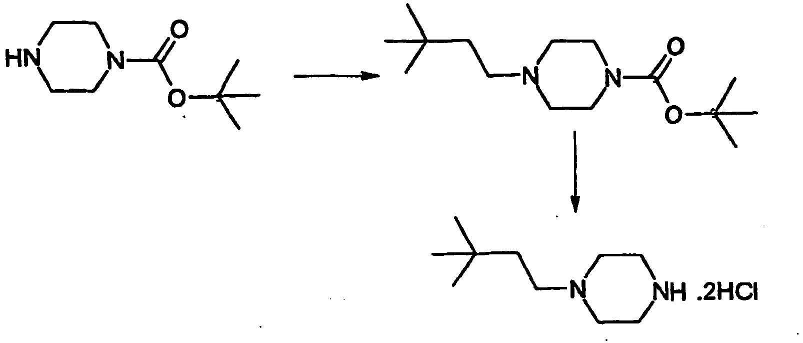

### Example E5

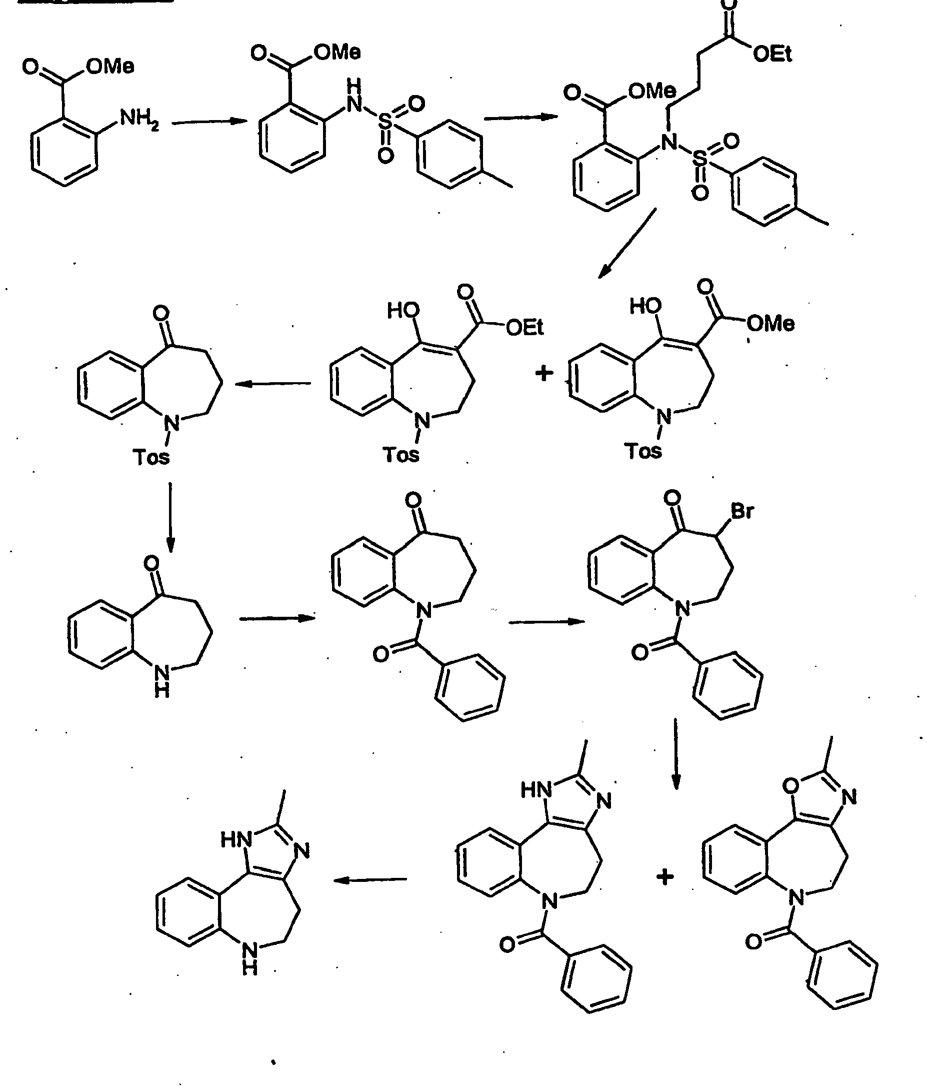

### Example E5a

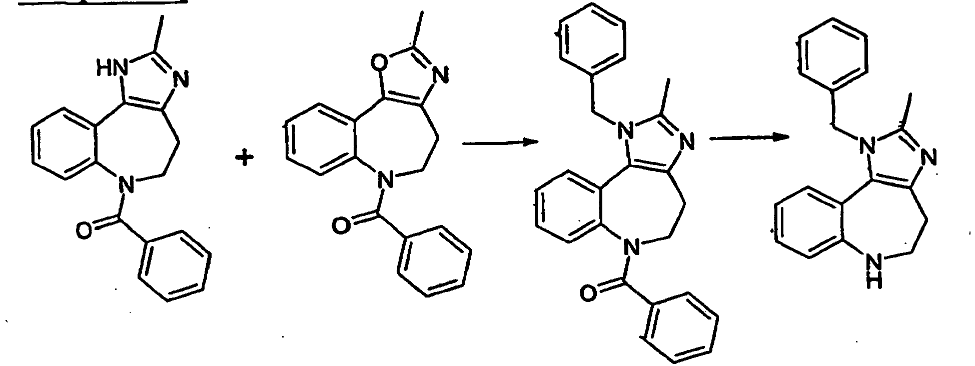

### Example E6

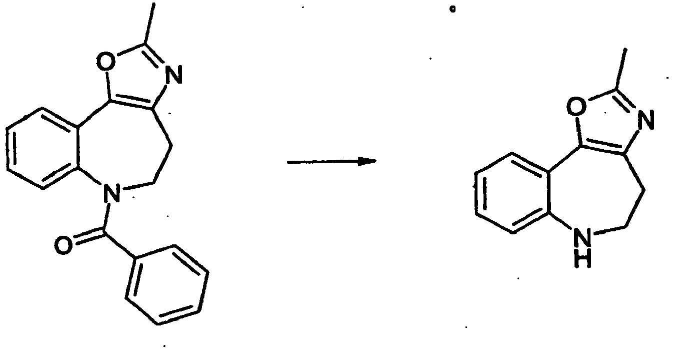

### Example E7

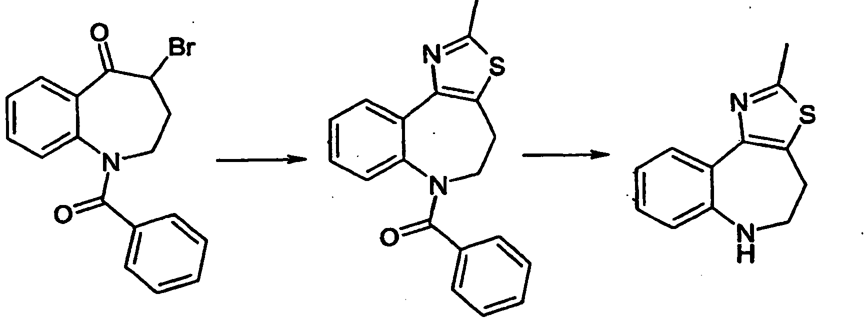

### Example E8

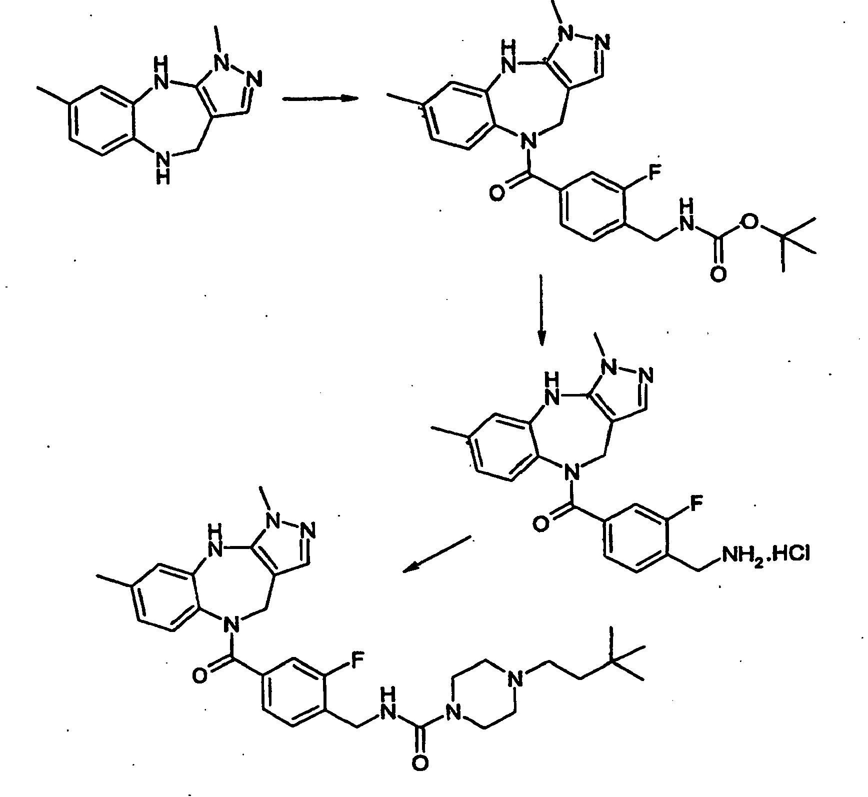

### Example E9

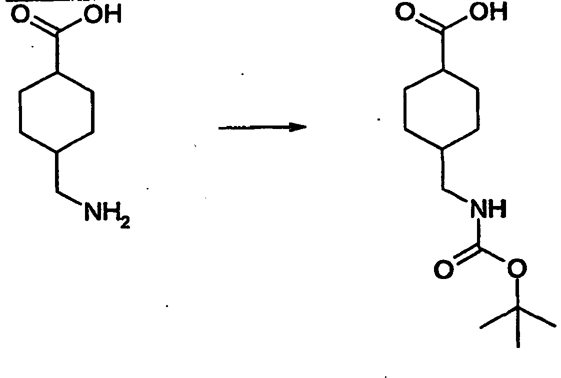

### Example E10

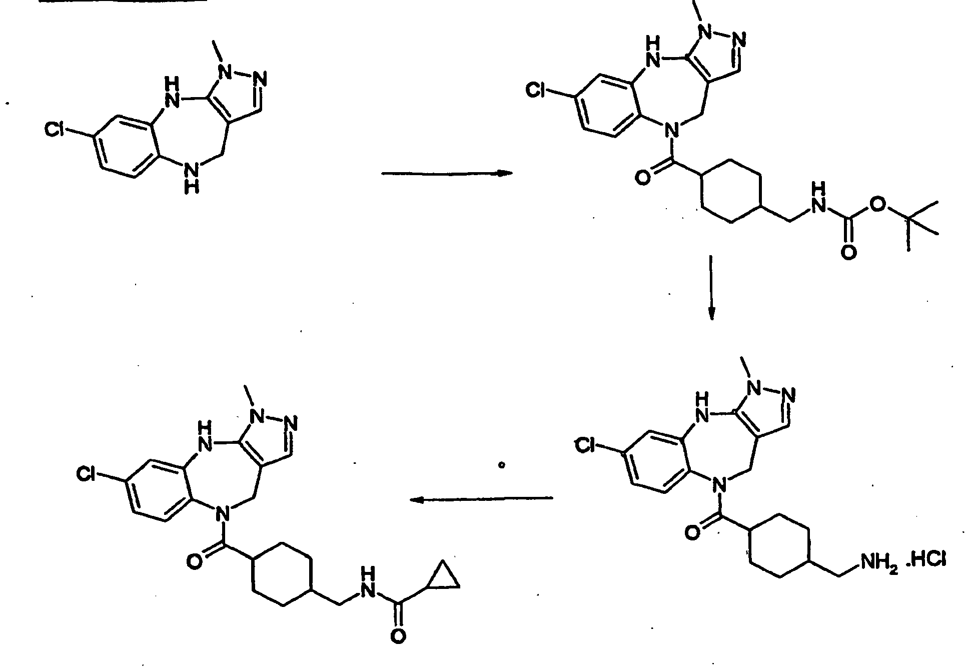

### Example E11

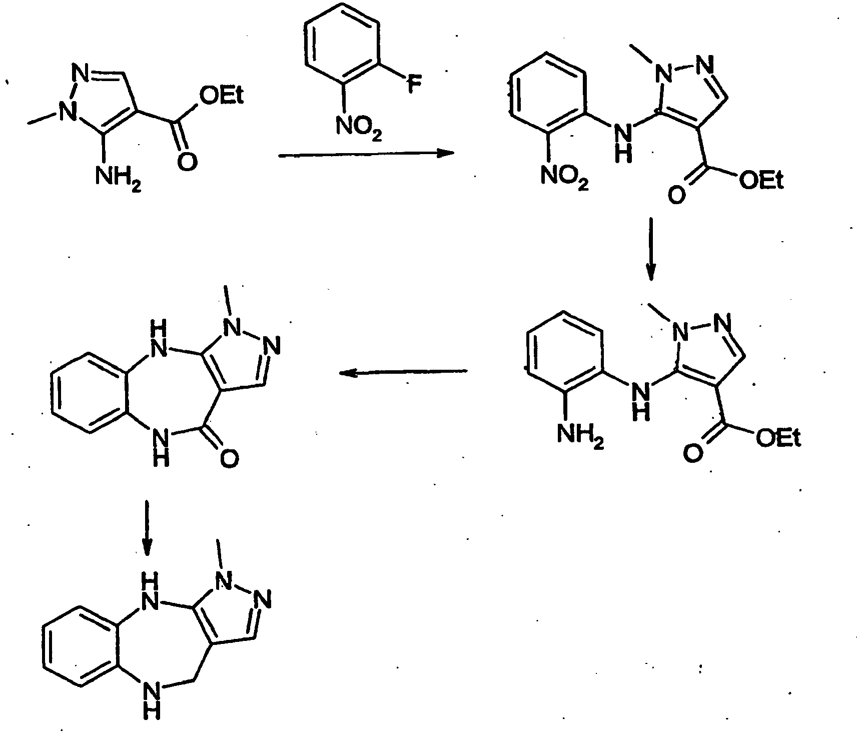

### Example E12

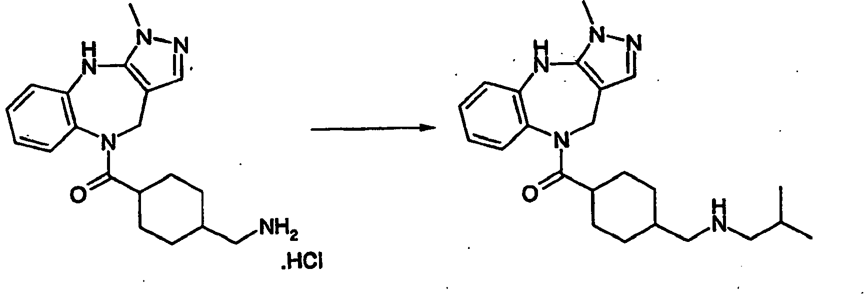

### Example E13

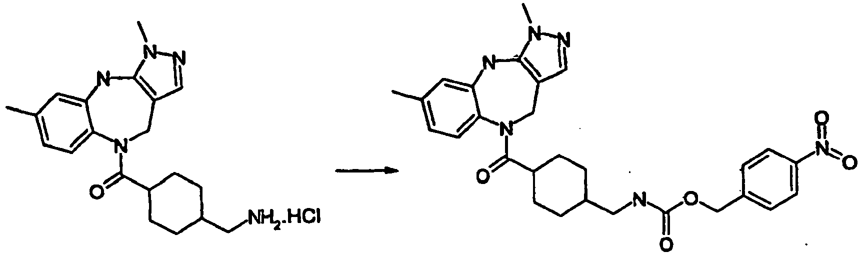

### Example E14

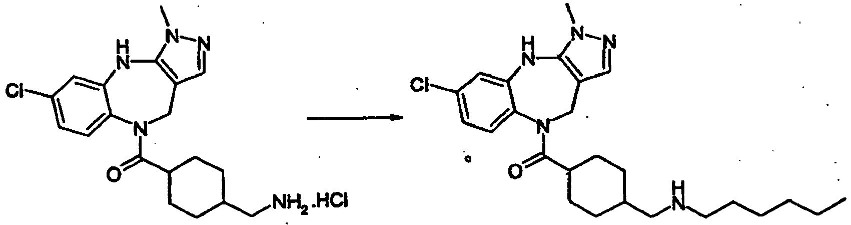

### Example E15

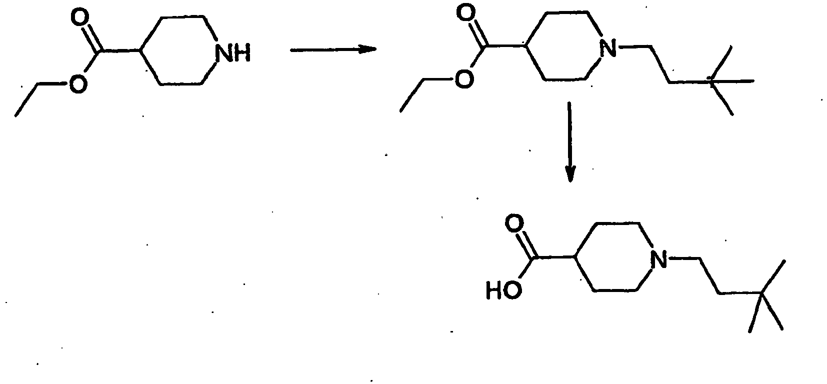

### Example E15a

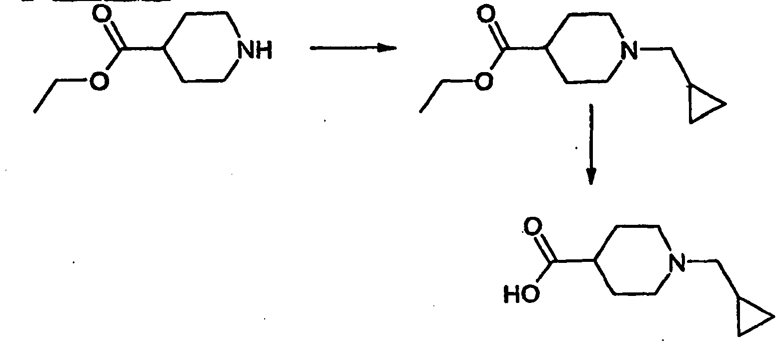

### Example E16

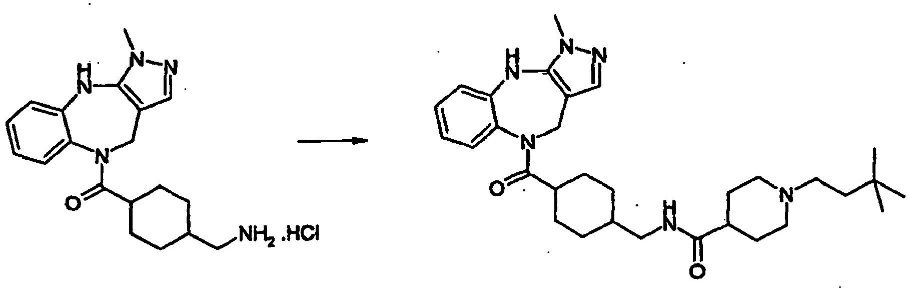

### Example E17

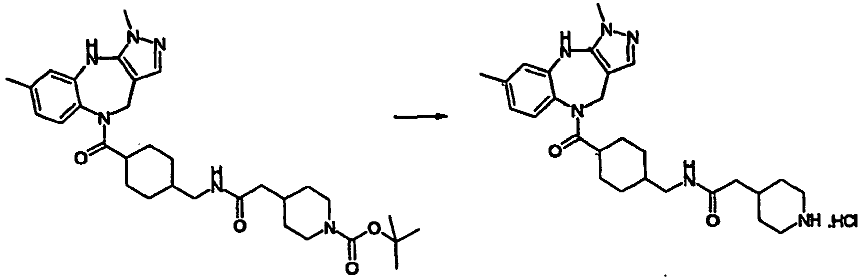

### Example E18

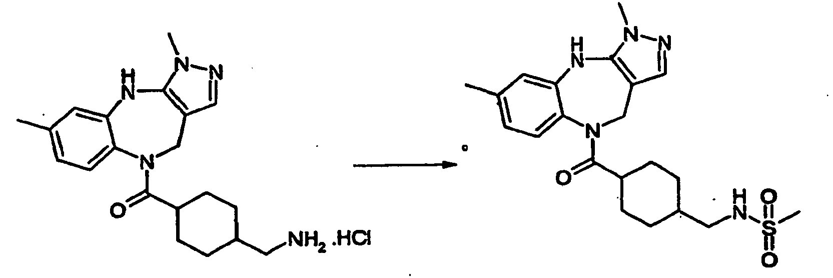

### Example E19

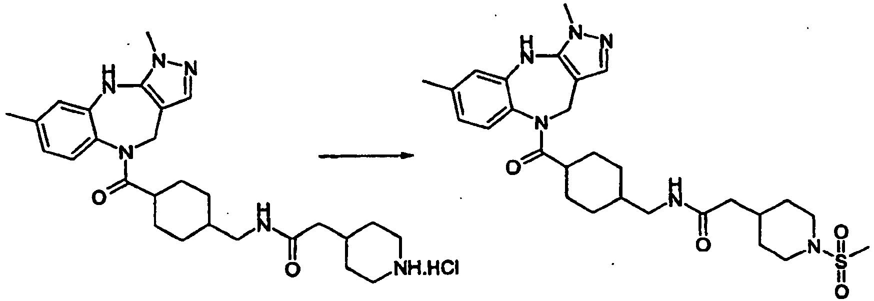

### Example E20

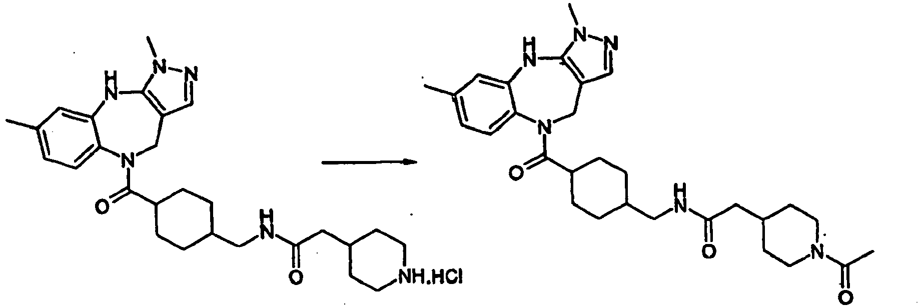

### Example E21

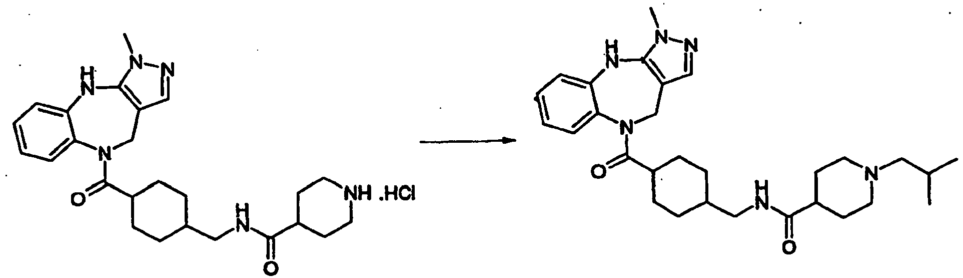

### Example E22

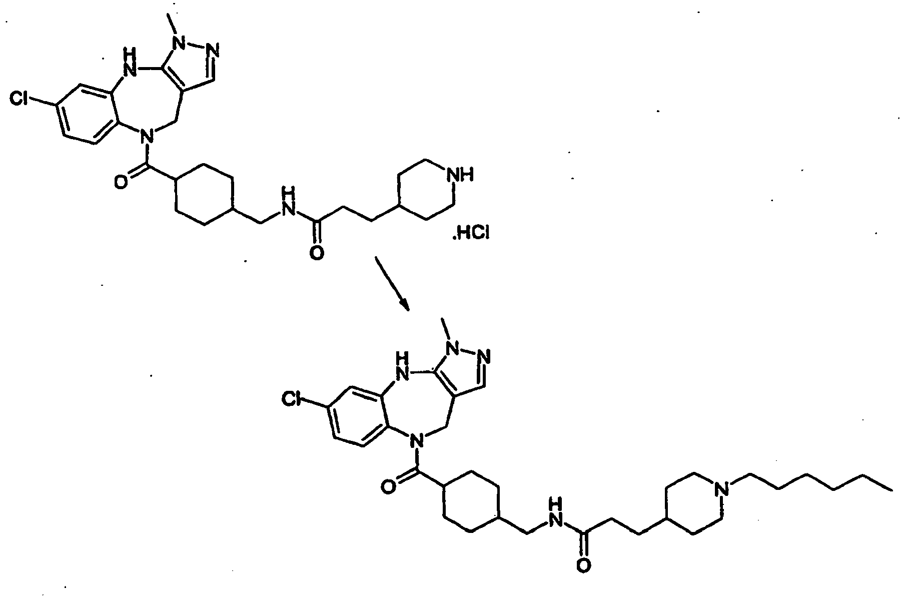

### Example E23

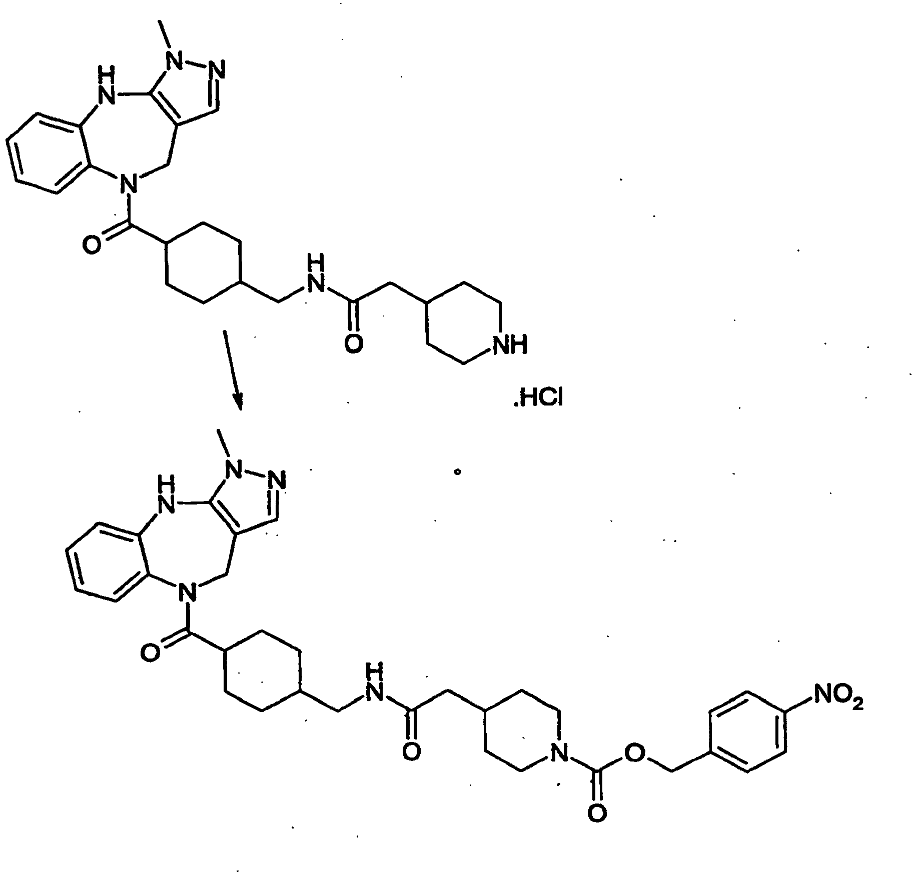

### Example E24

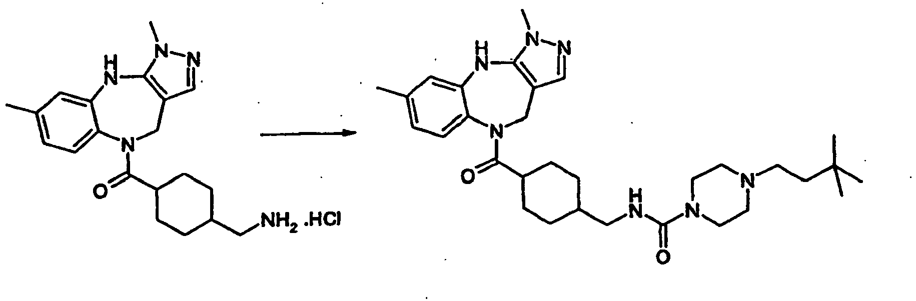

### Example E25

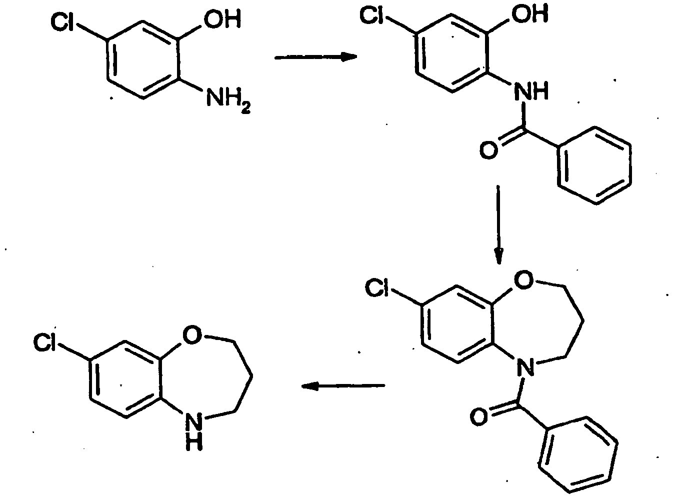

### Example E26

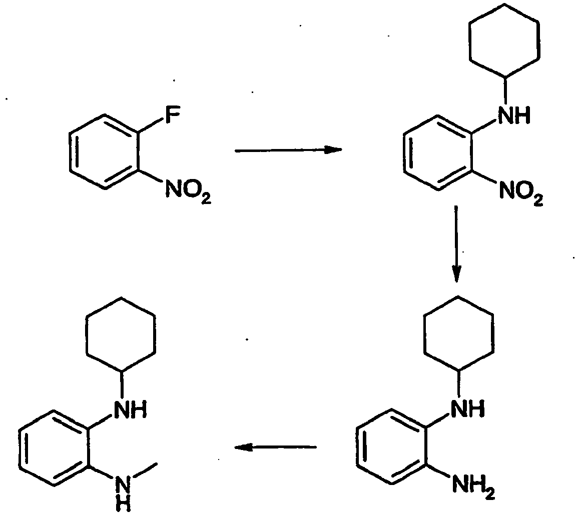

### Example E27

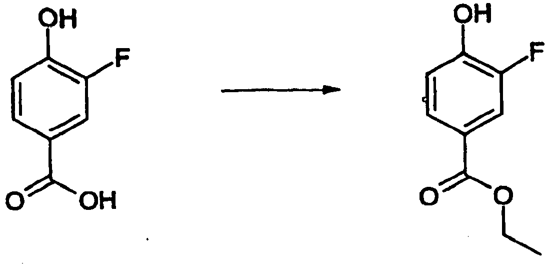

### Example E28

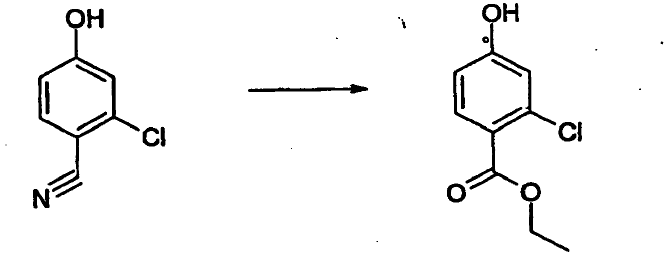

### Example E29

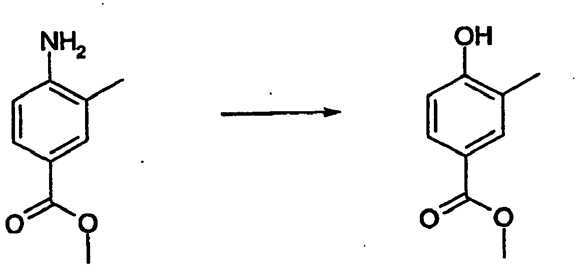

### Example E30

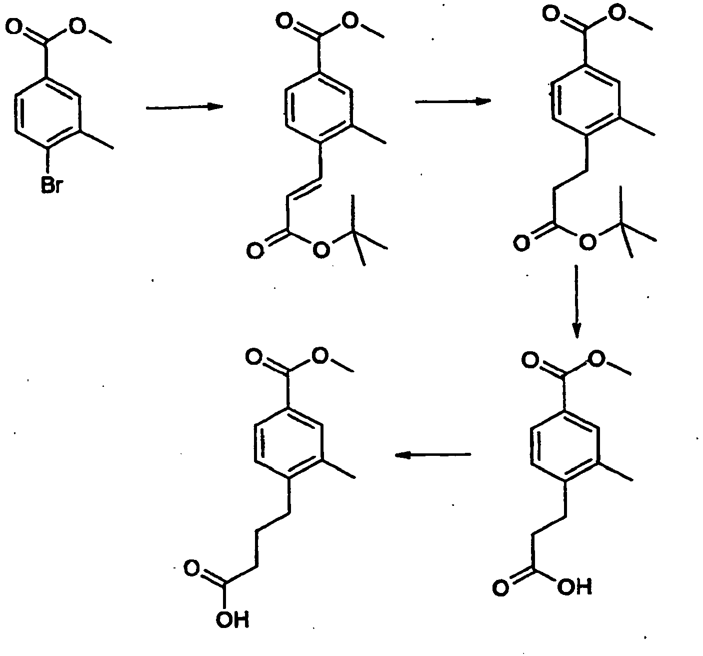

### Example E31

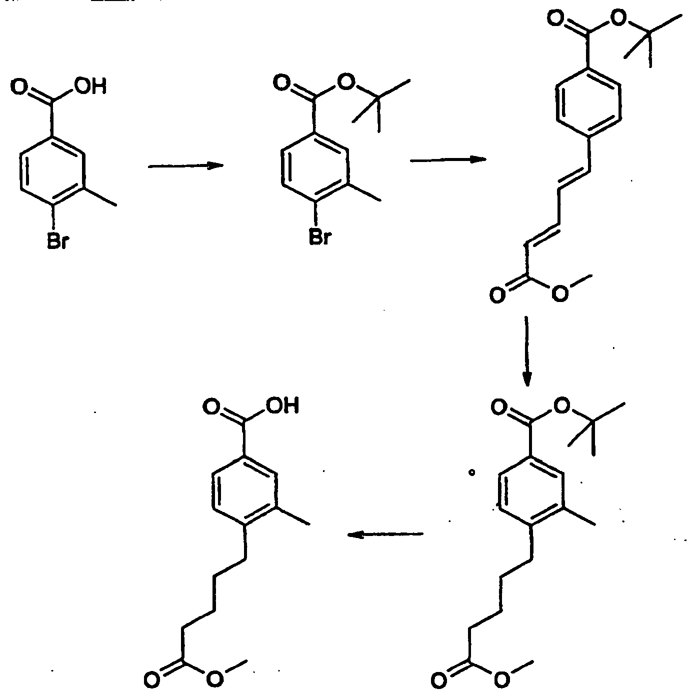

### Example E32

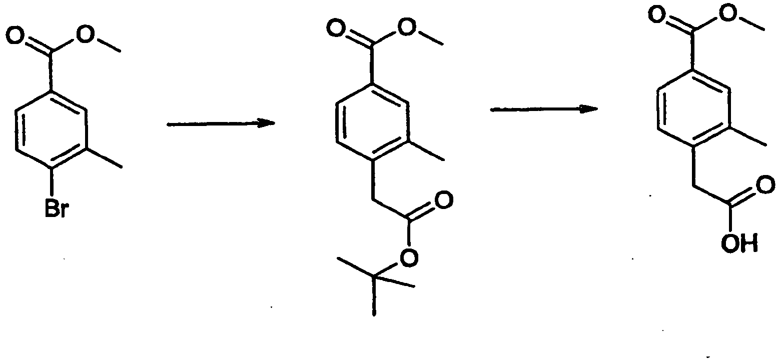

### Example E33

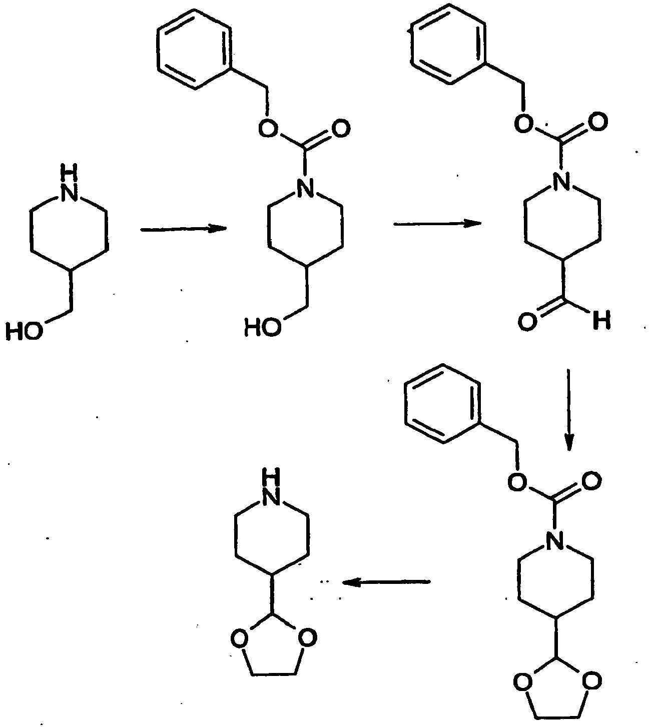

### Example E34

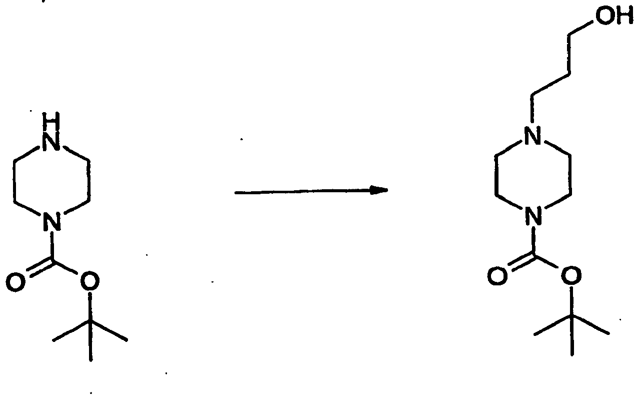

### Example E35

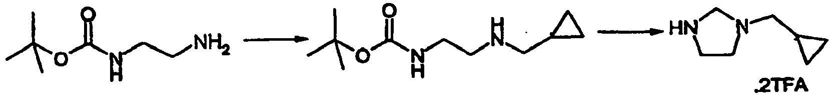

### Example E36

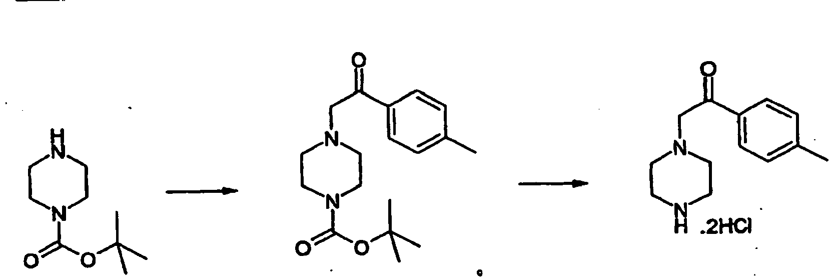

### Example E37

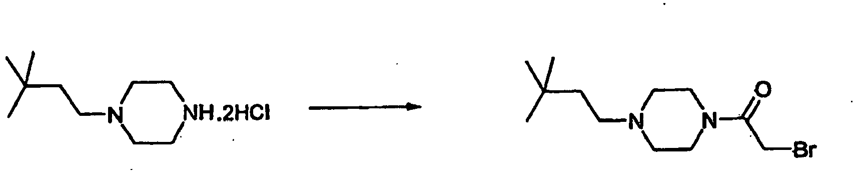

### Example E38

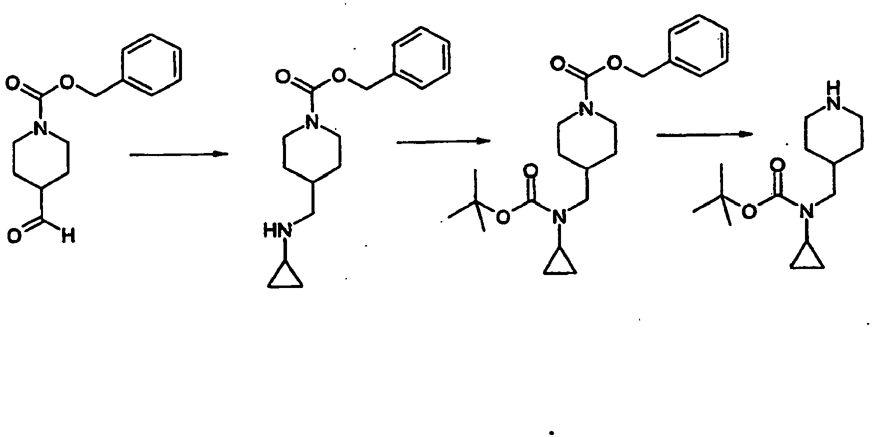

### Example E39

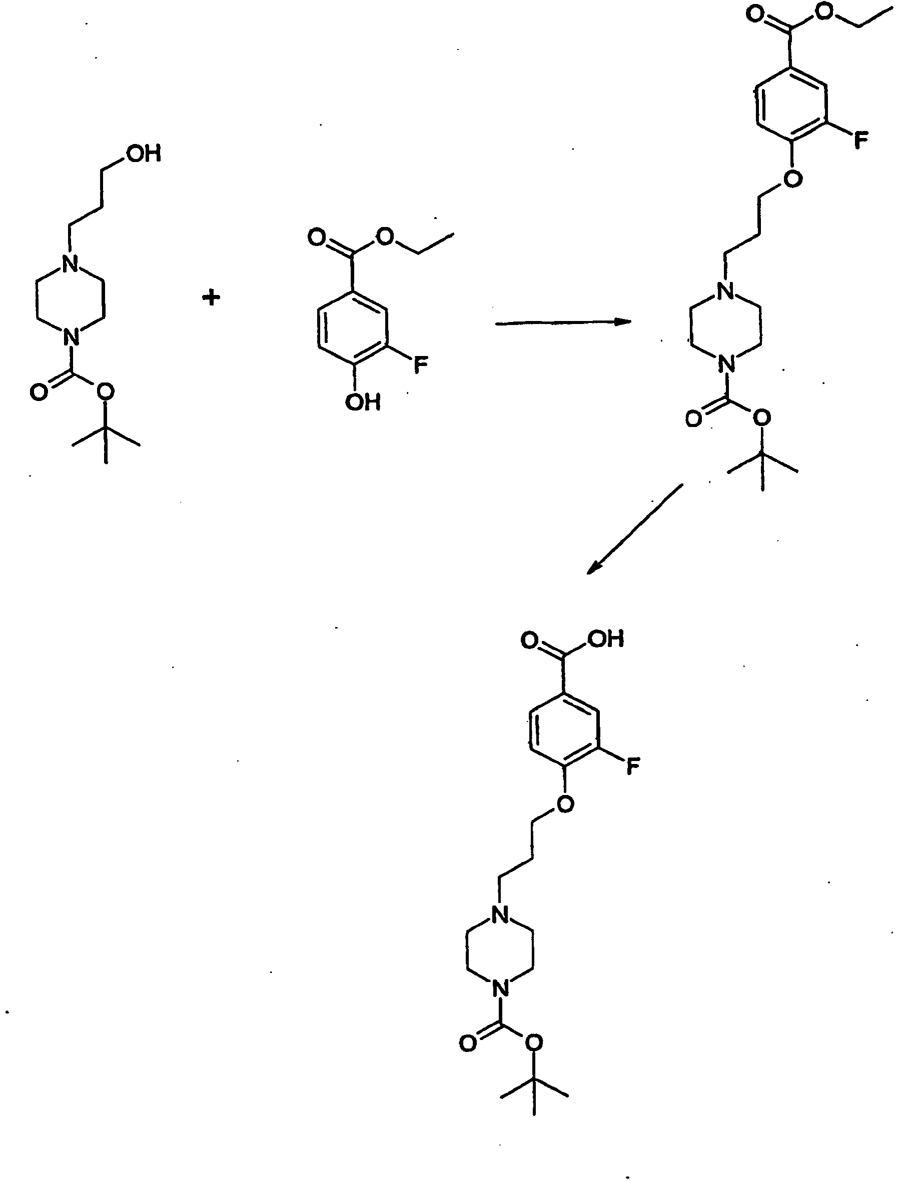

### Example E40

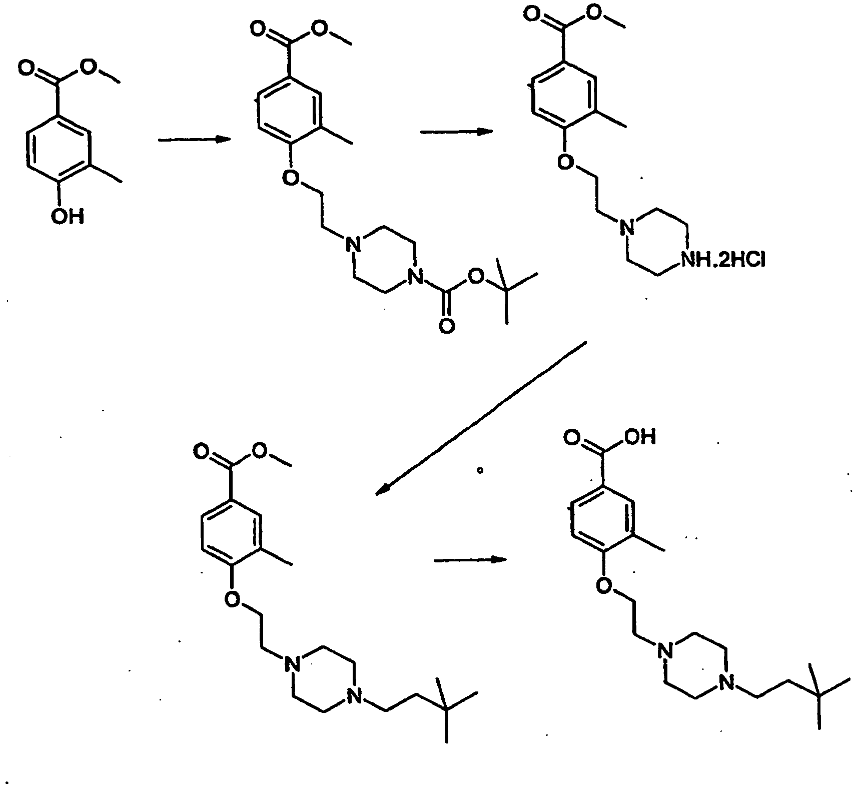

### Example E41

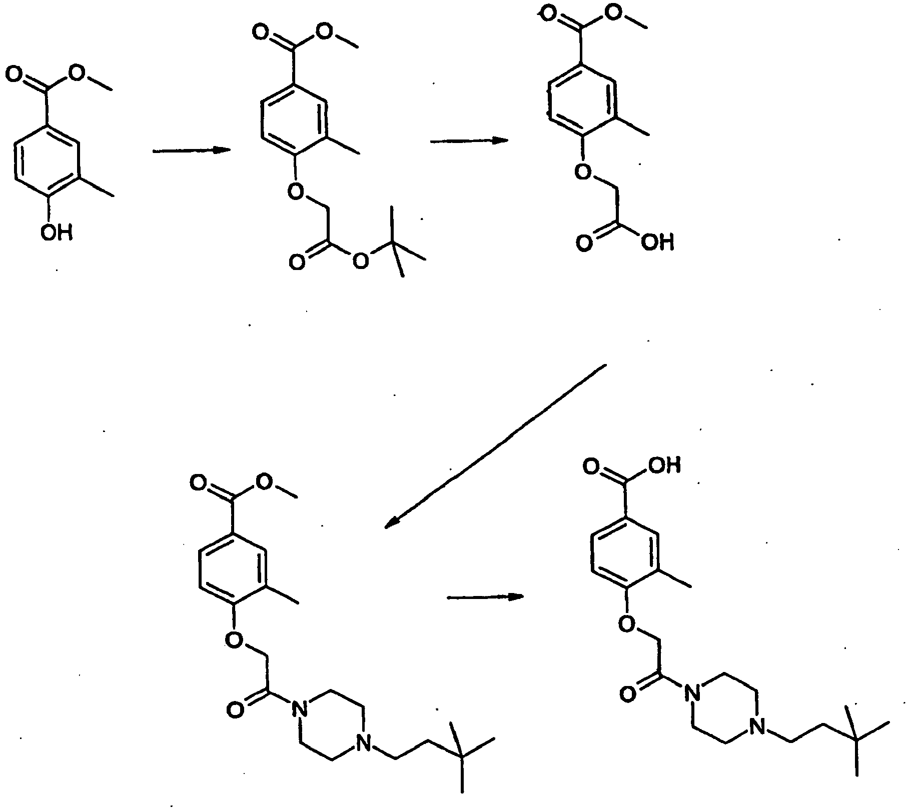

### Example E41a

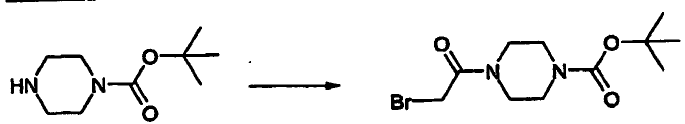

### Example E41b

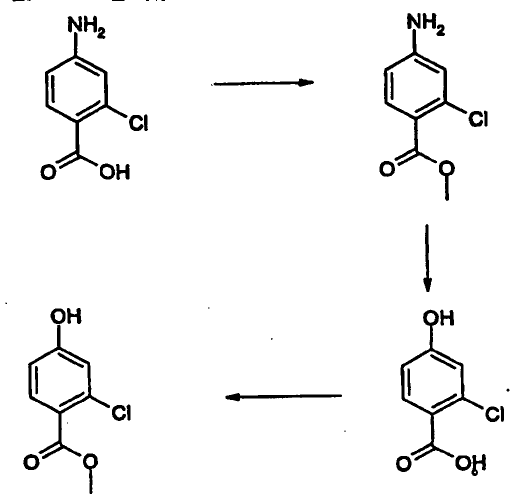

### Example E41c

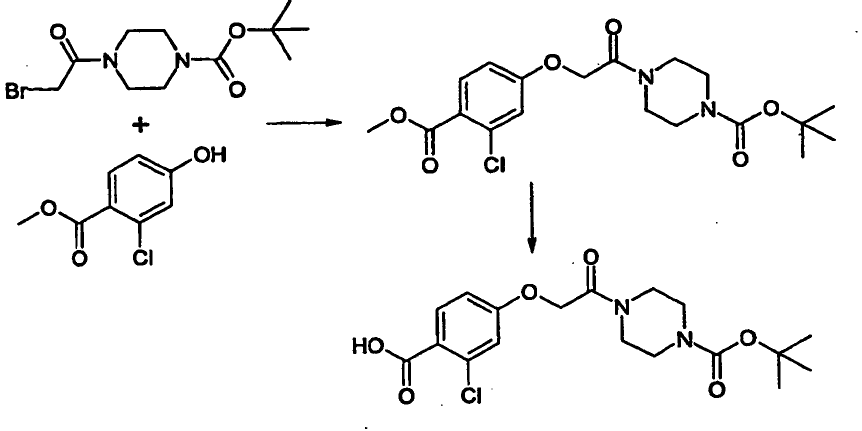

### Example E42

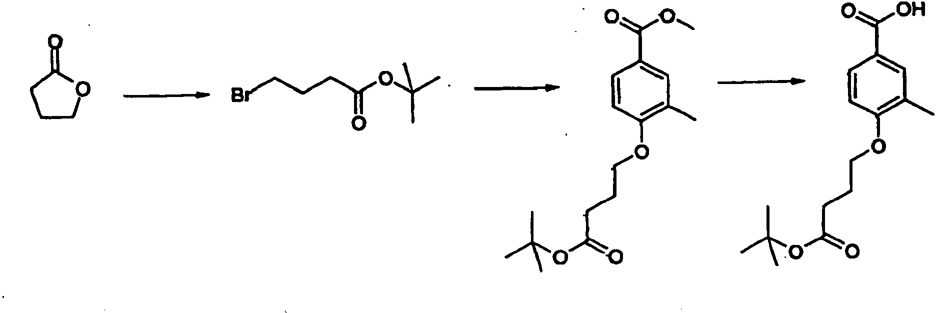

### Example E43

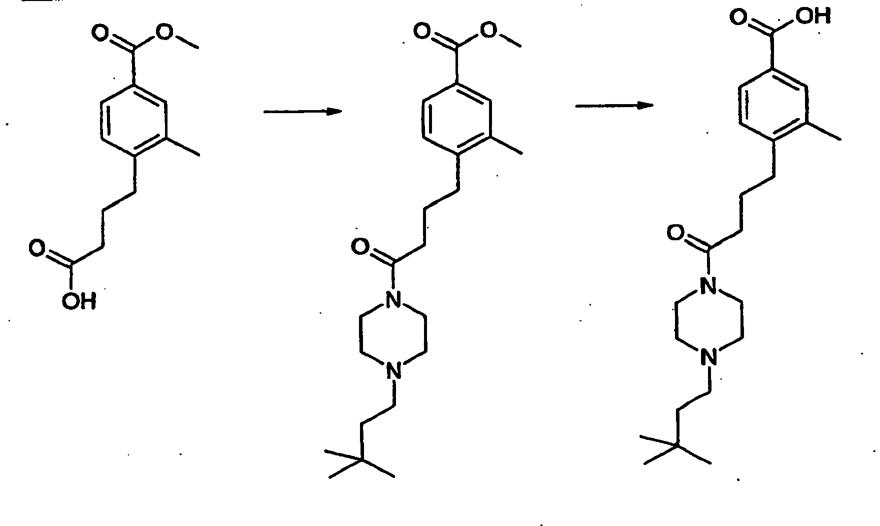

### Example E44

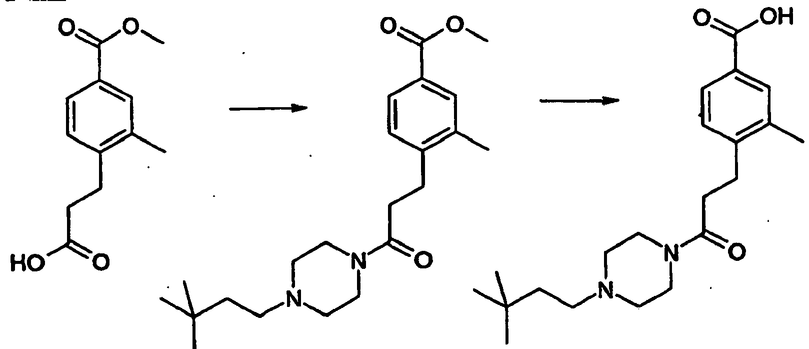

### Example E45

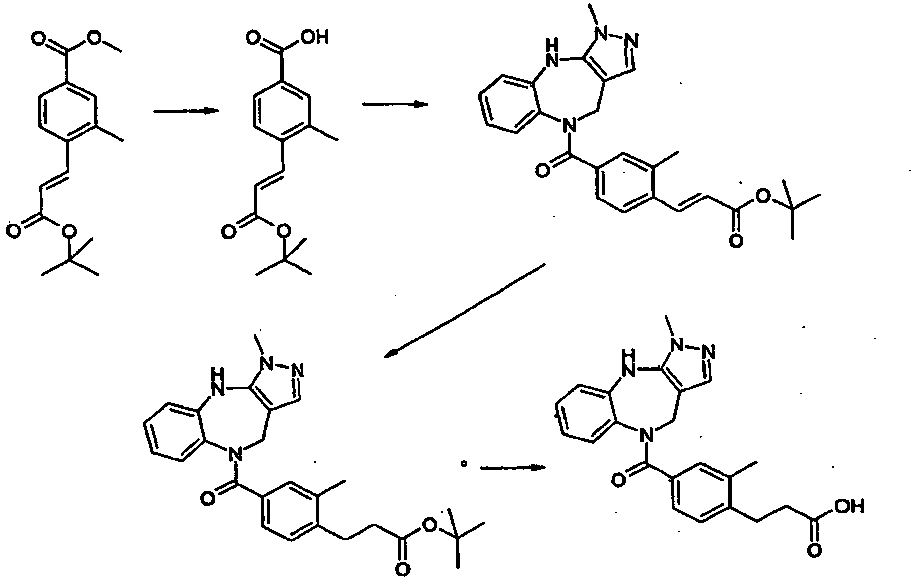

### Example E46

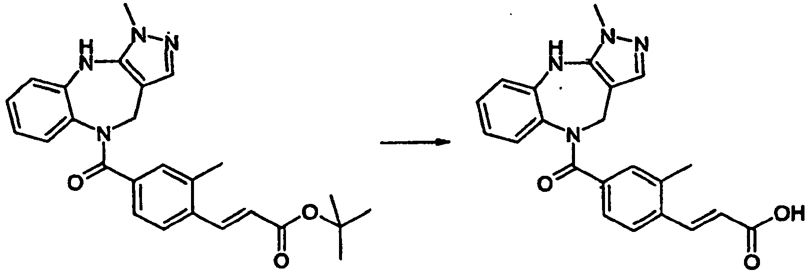

### Example E47

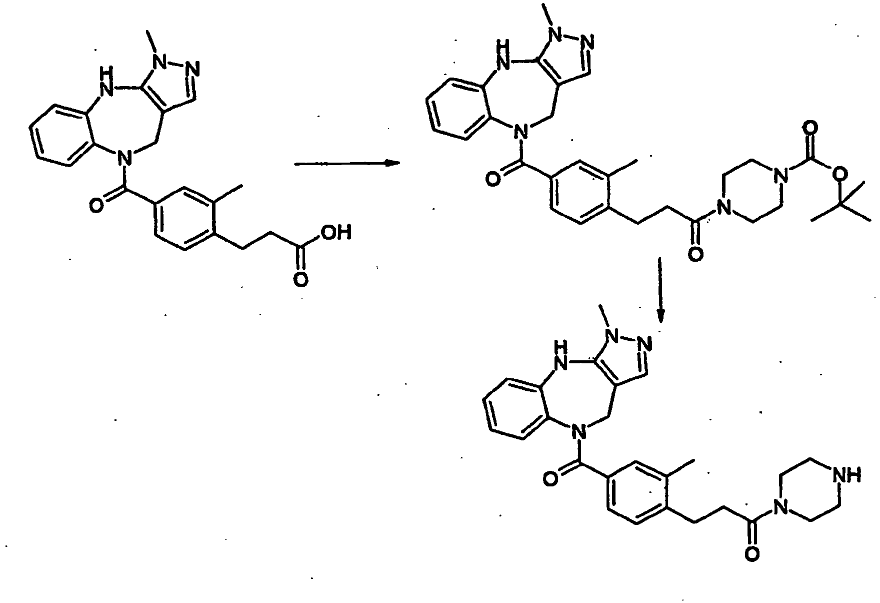

### Example E48

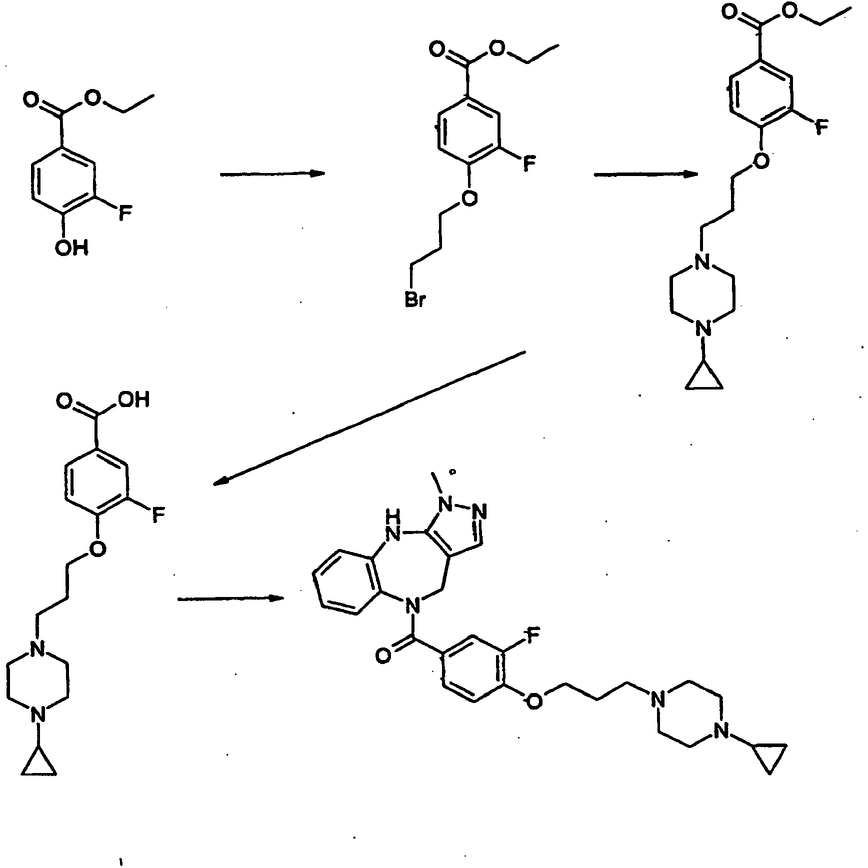

### Example E49

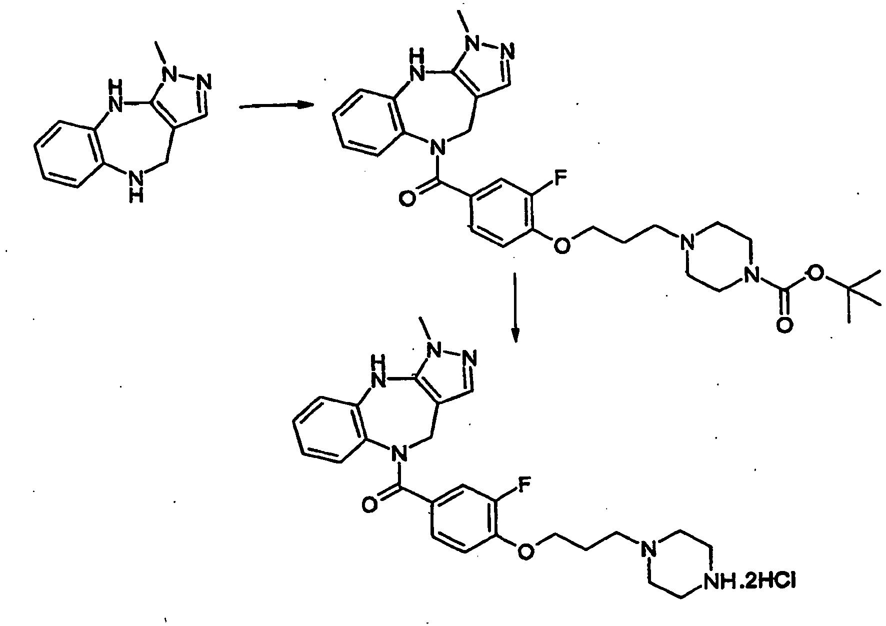

### Example E50

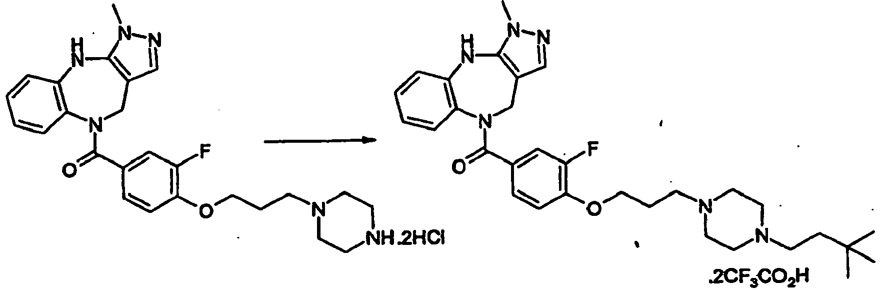

### Example E51

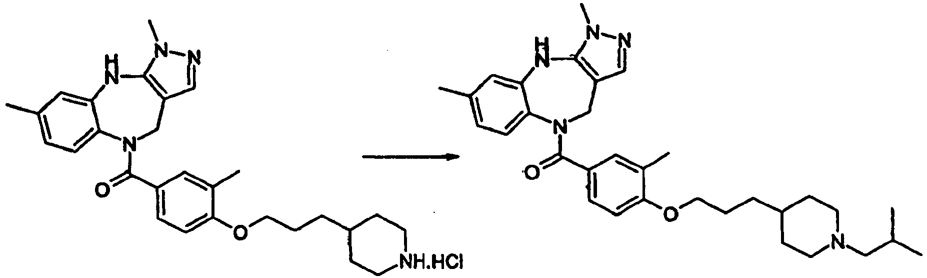

### Example E52

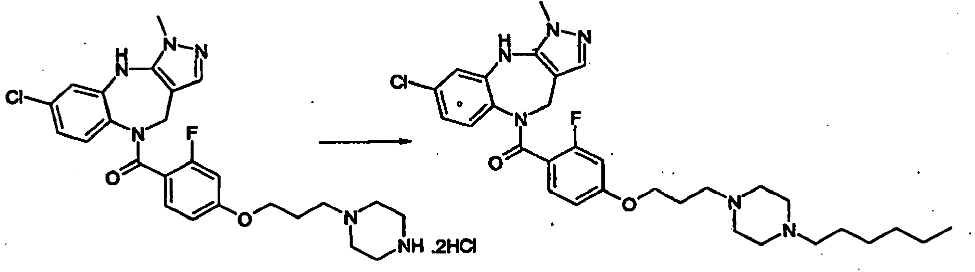

### Example E53

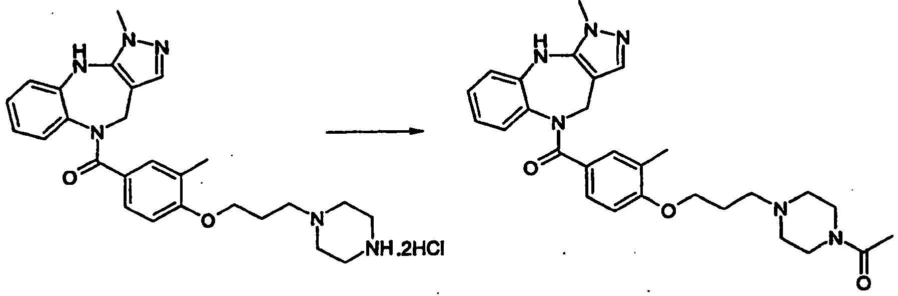

### Example E54

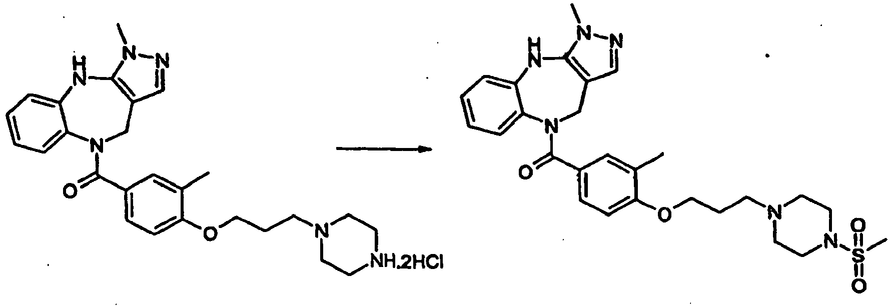

### Example E55

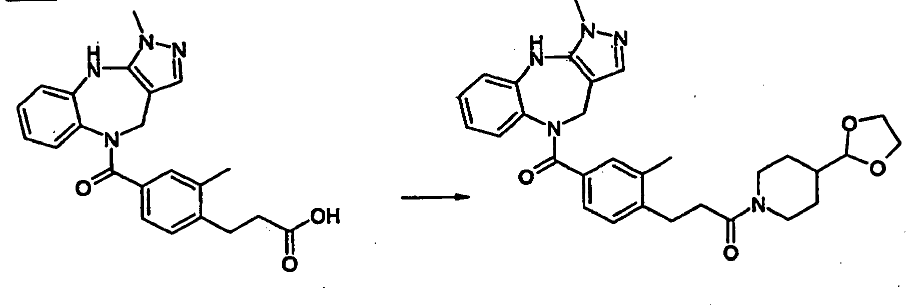

### Example E56

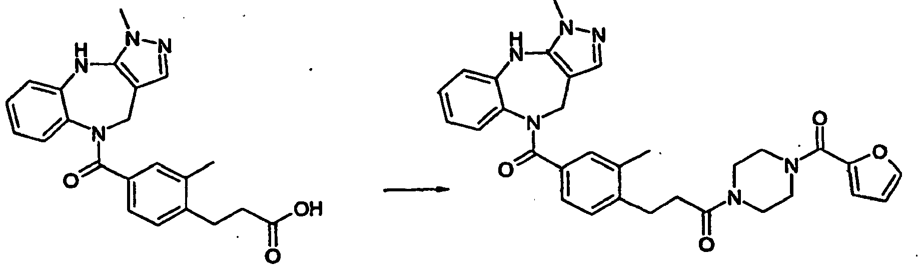

### Example E57

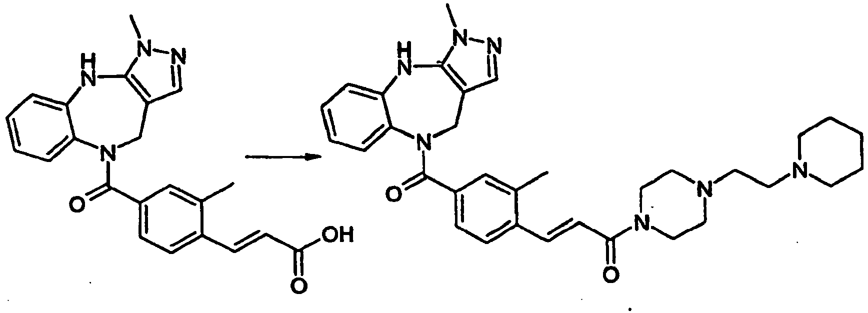

### Example E58

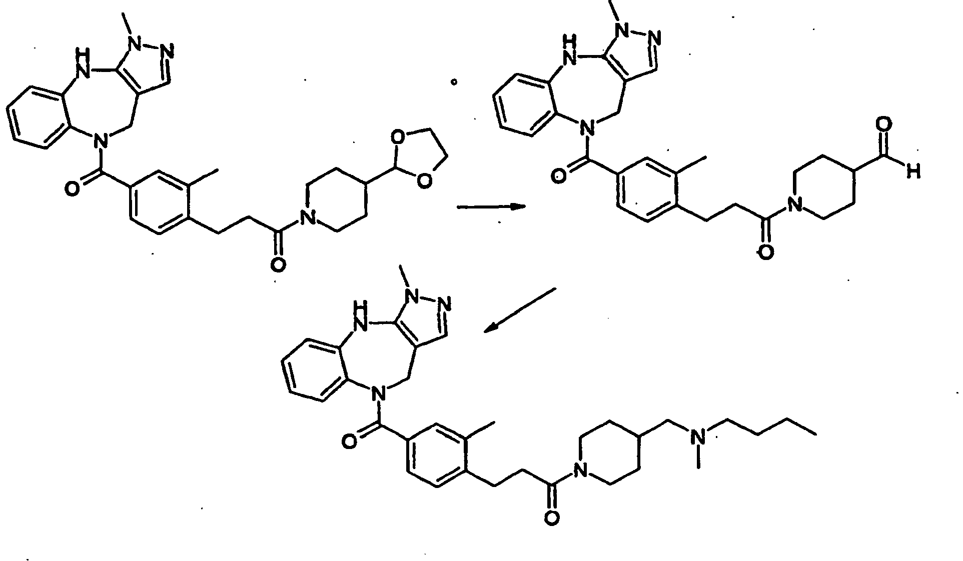

### Example E59

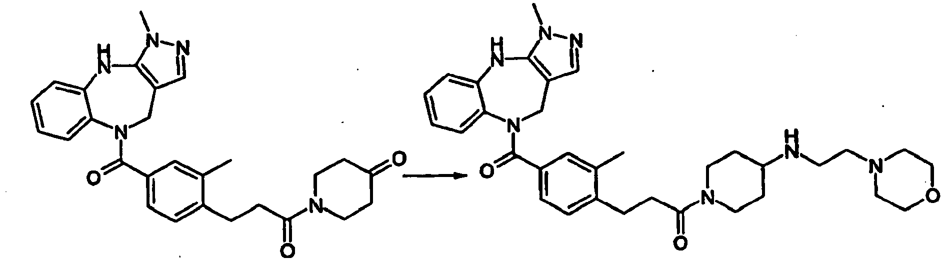

### Example E60

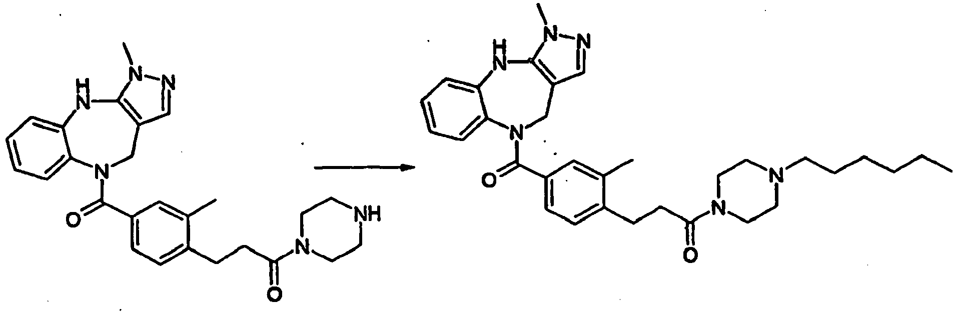

### Example E61

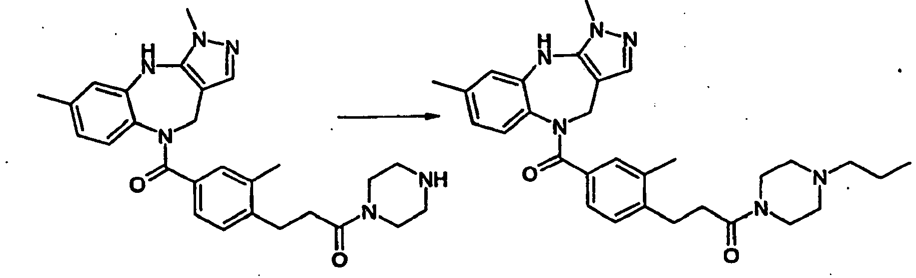

### Example E62

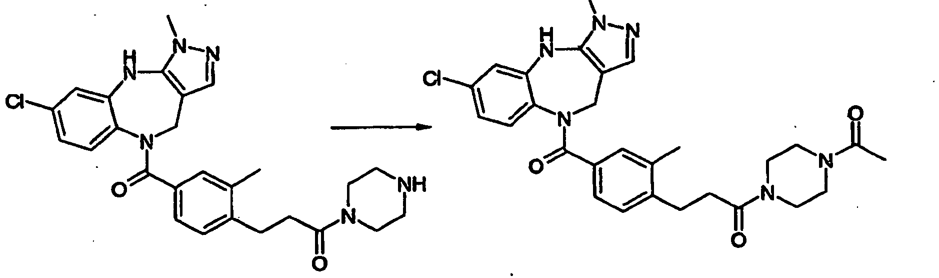

### Example E63

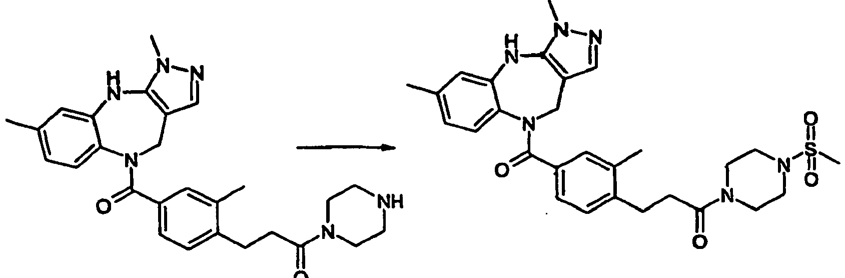

### Example E64

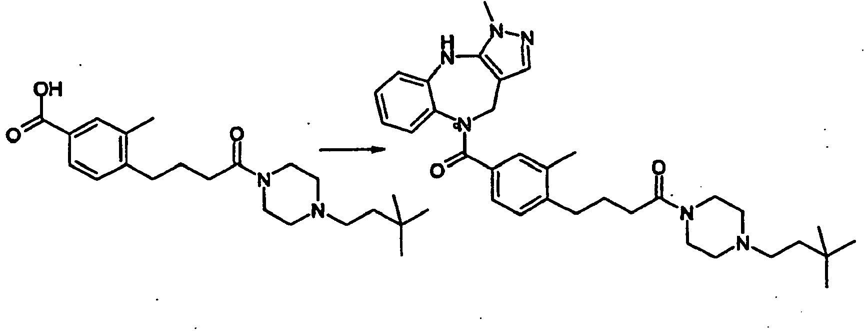

### Example E65

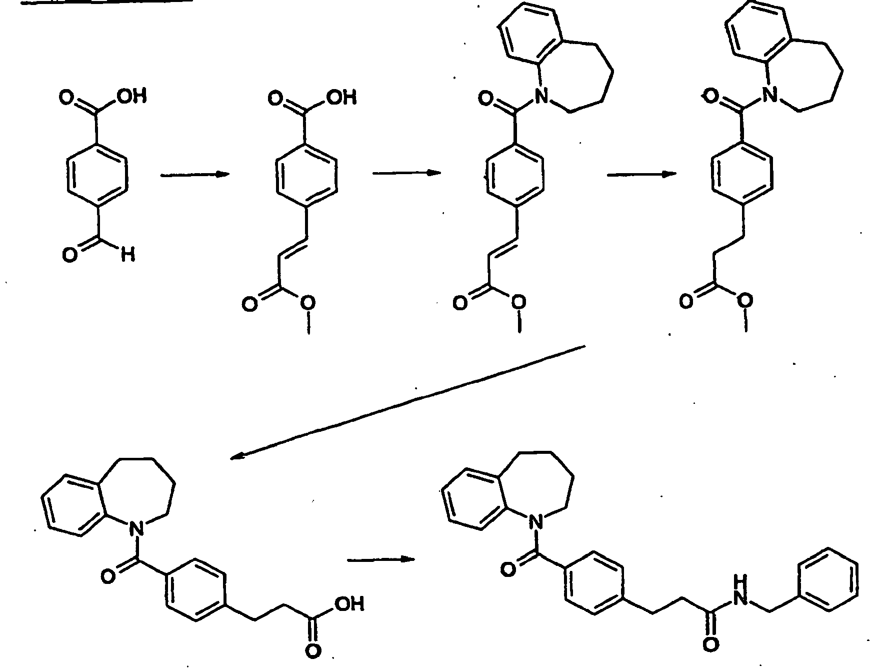

### Example E66

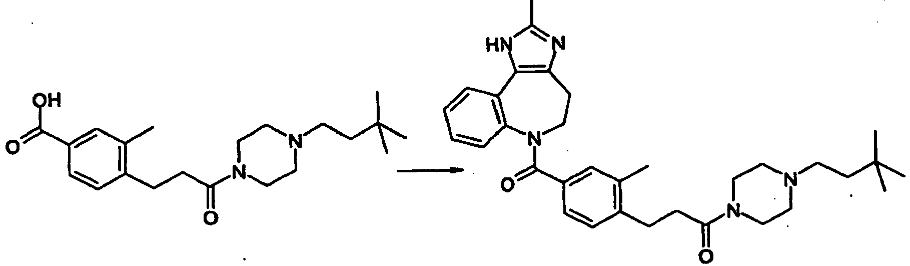

### Example E67

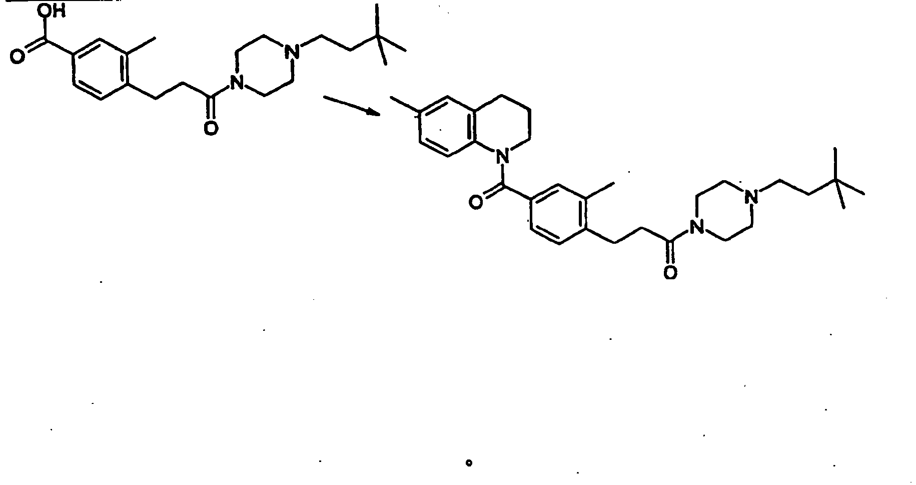

### Example E68

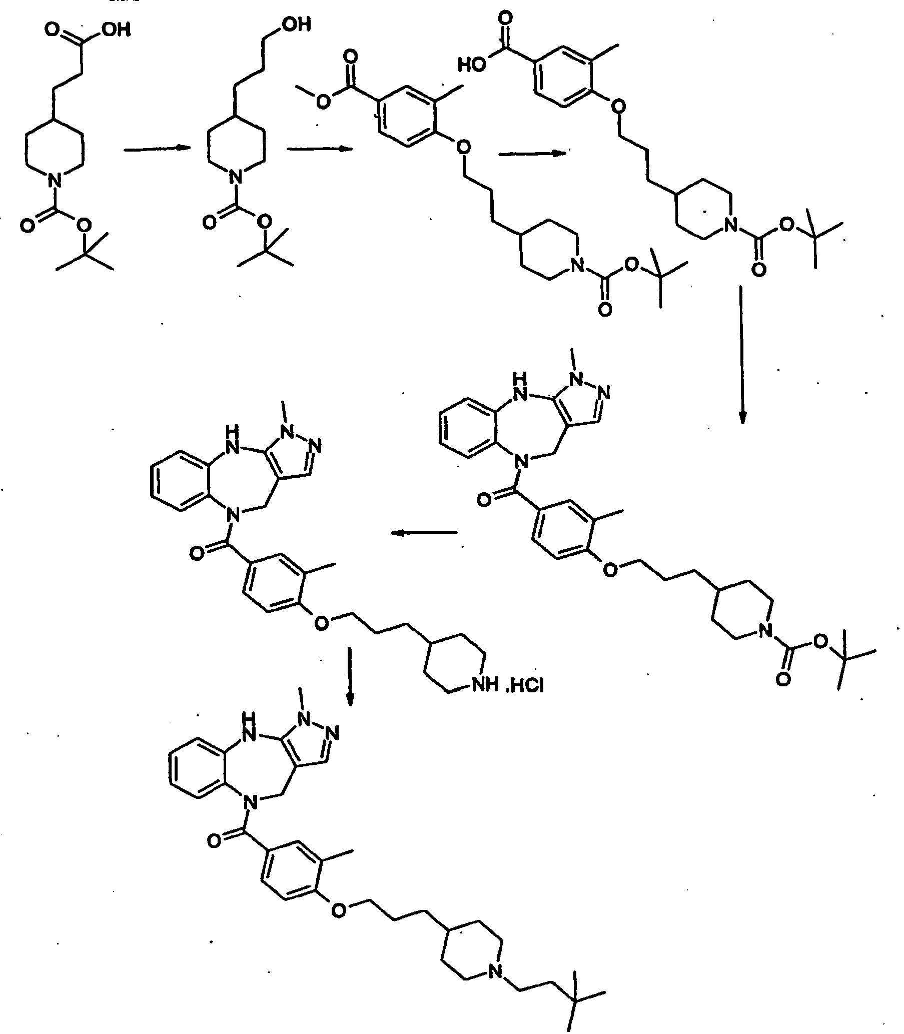

### Example E69

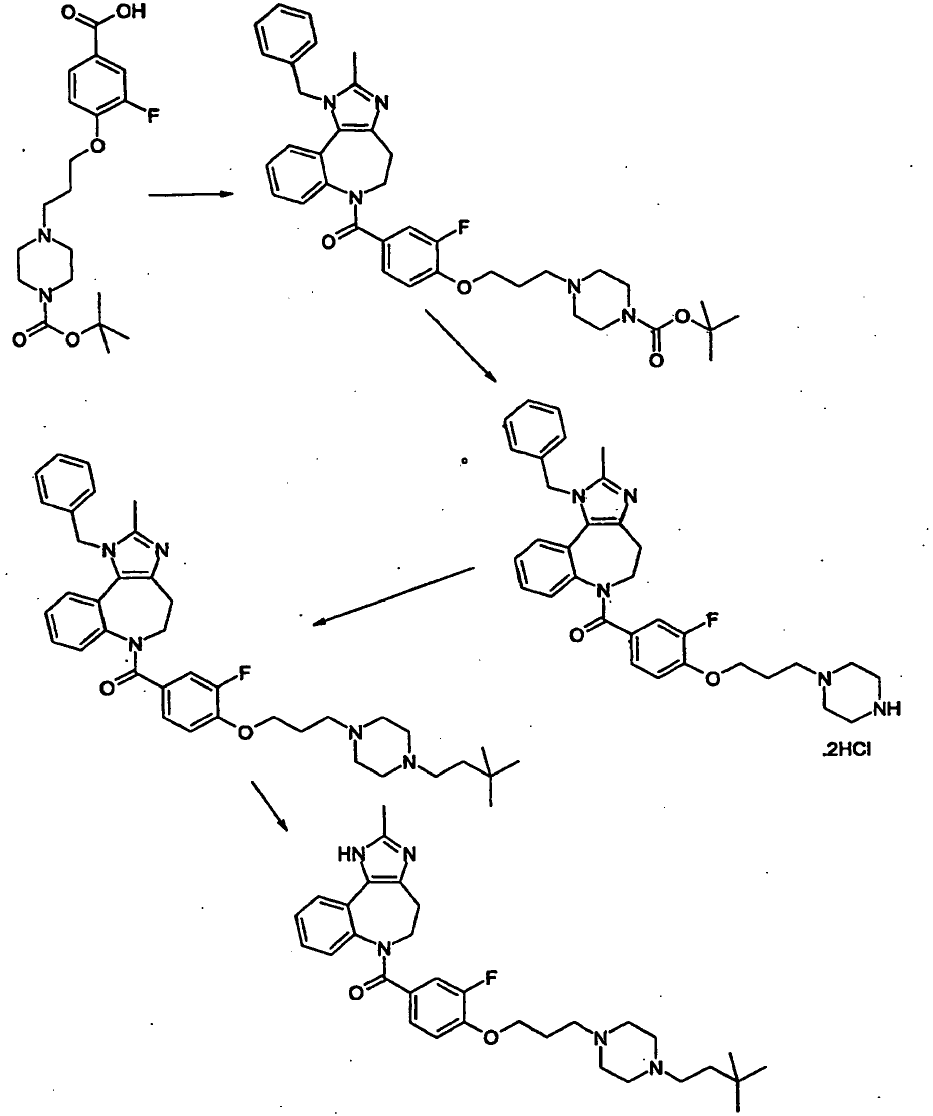

### Example E70

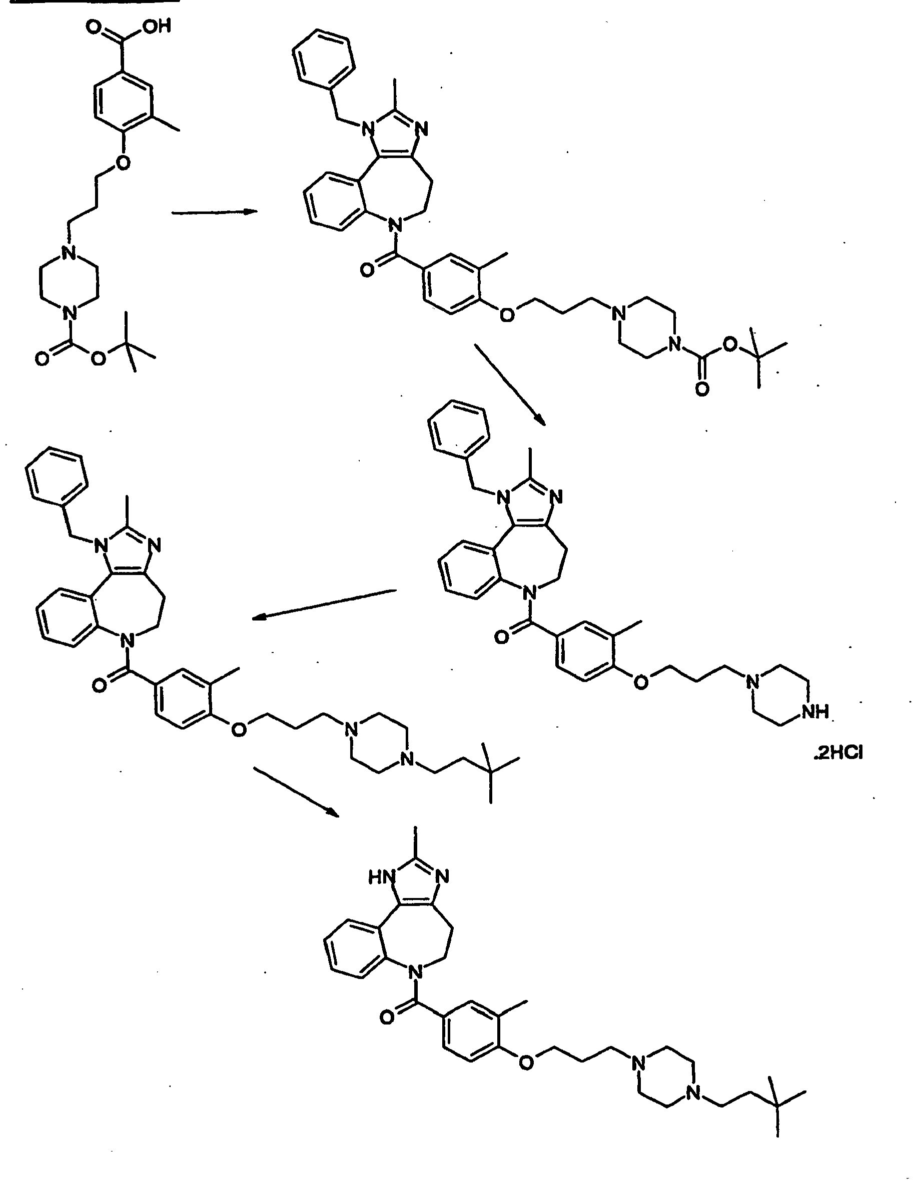

### Example E71

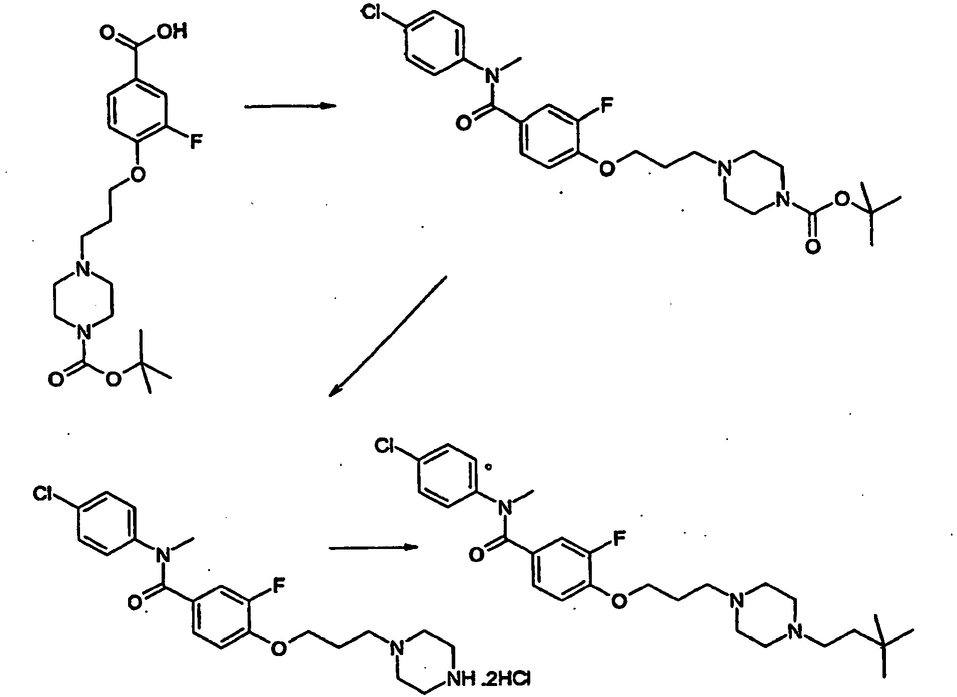

### Example E72

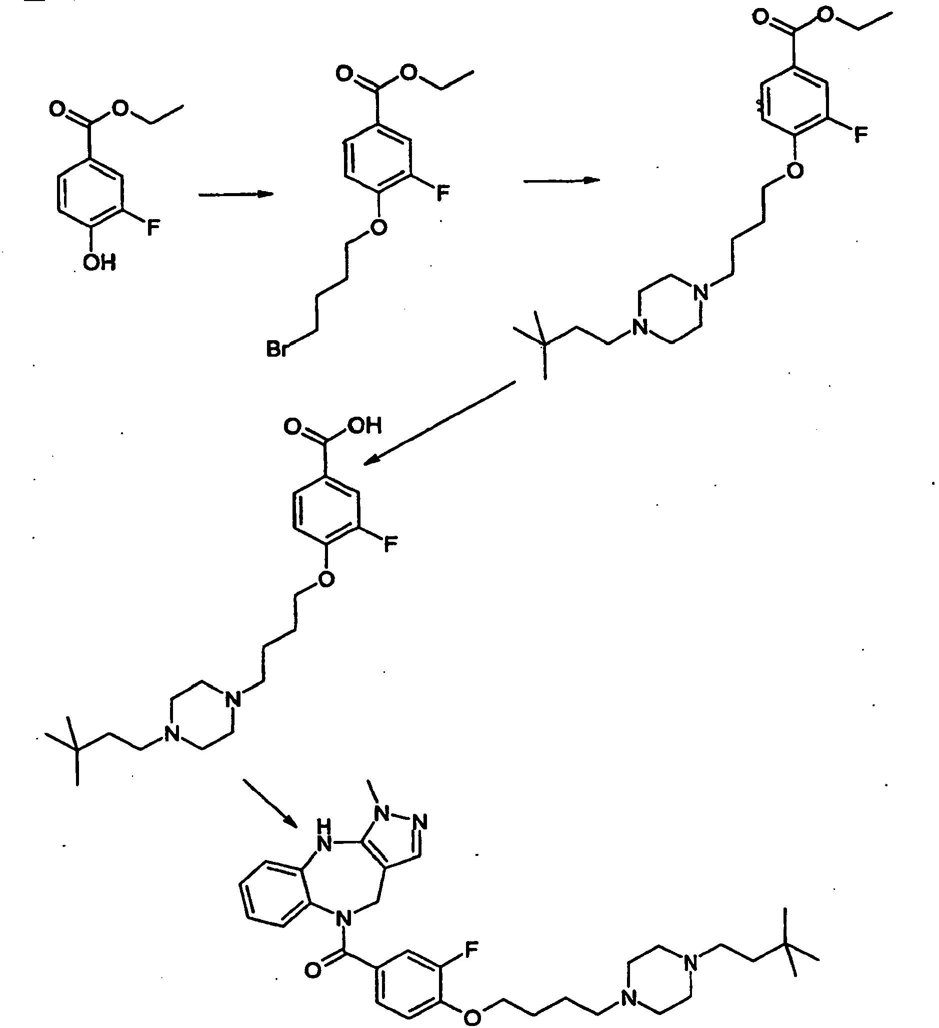

### Example E73

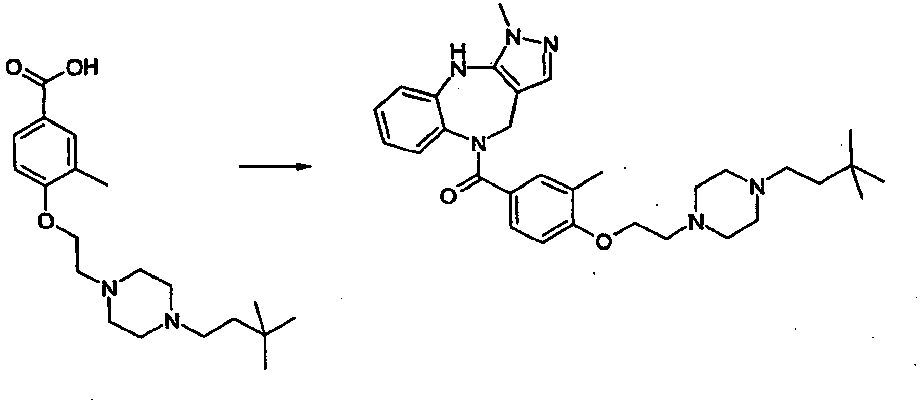

### Example E74

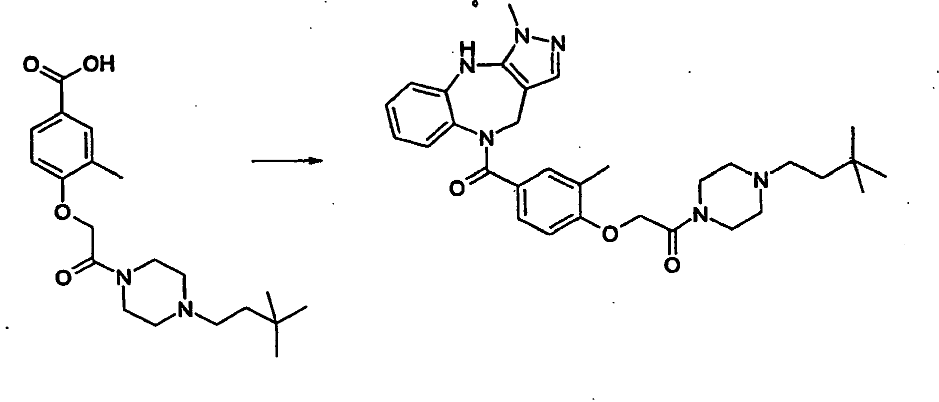

### Example E75

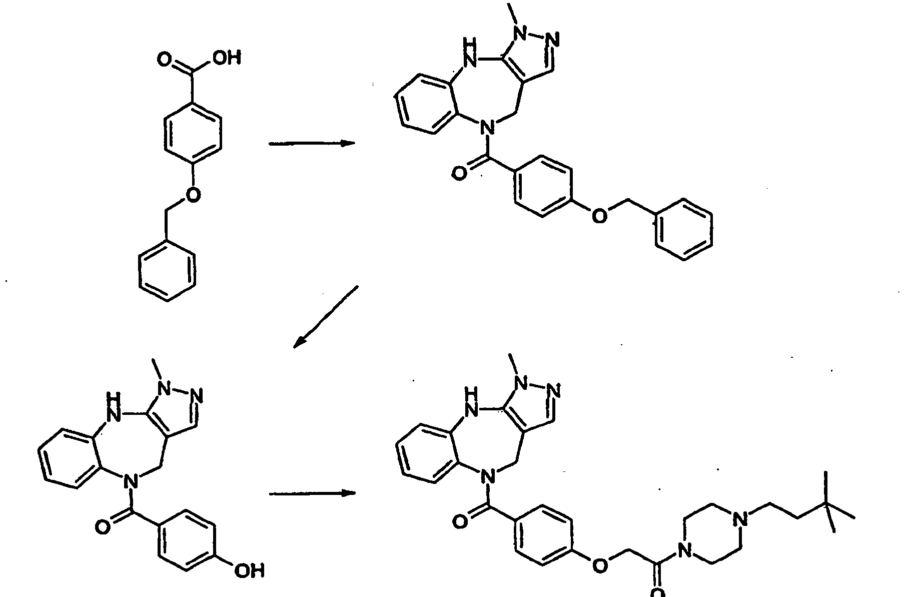

### Example E76

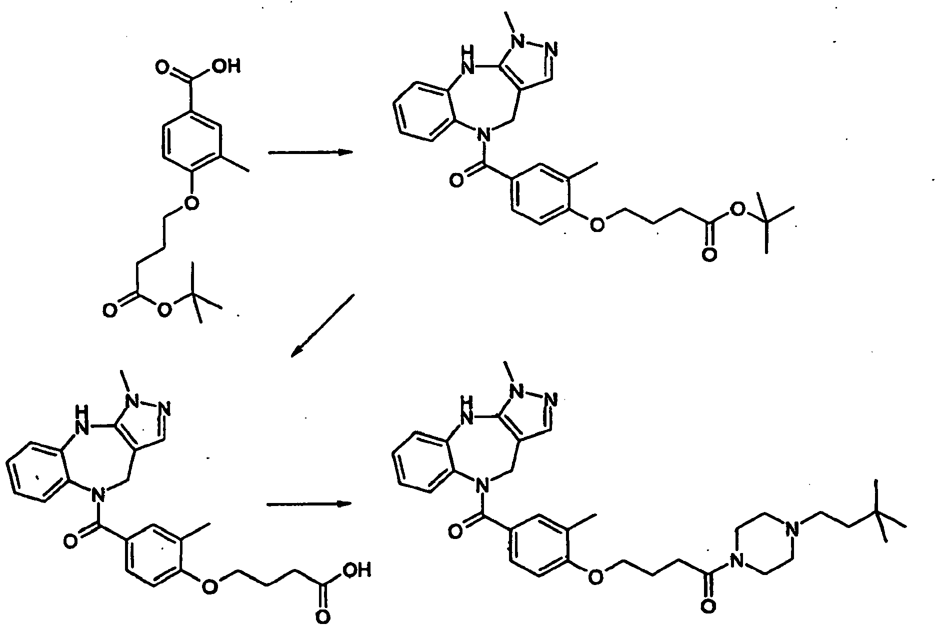

### Example E77

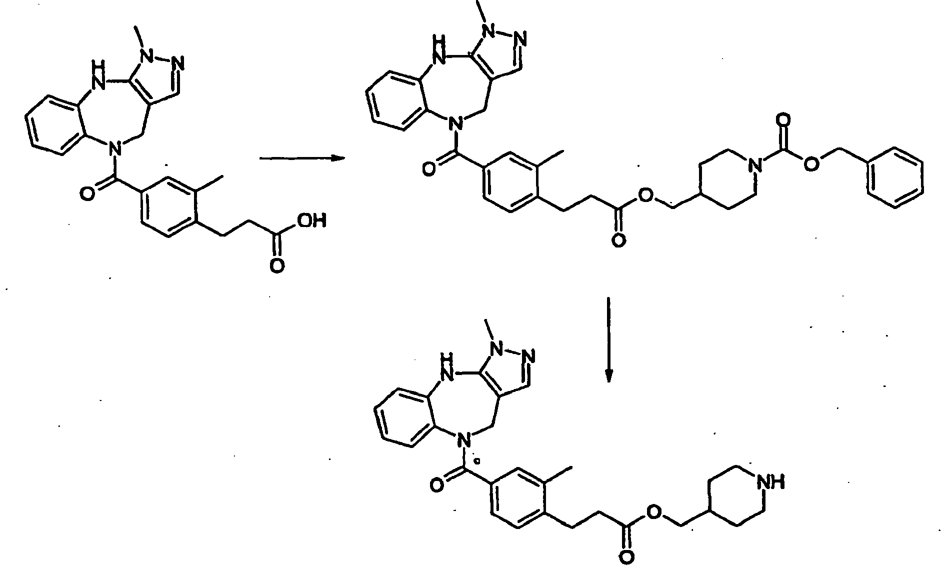

### Example E78

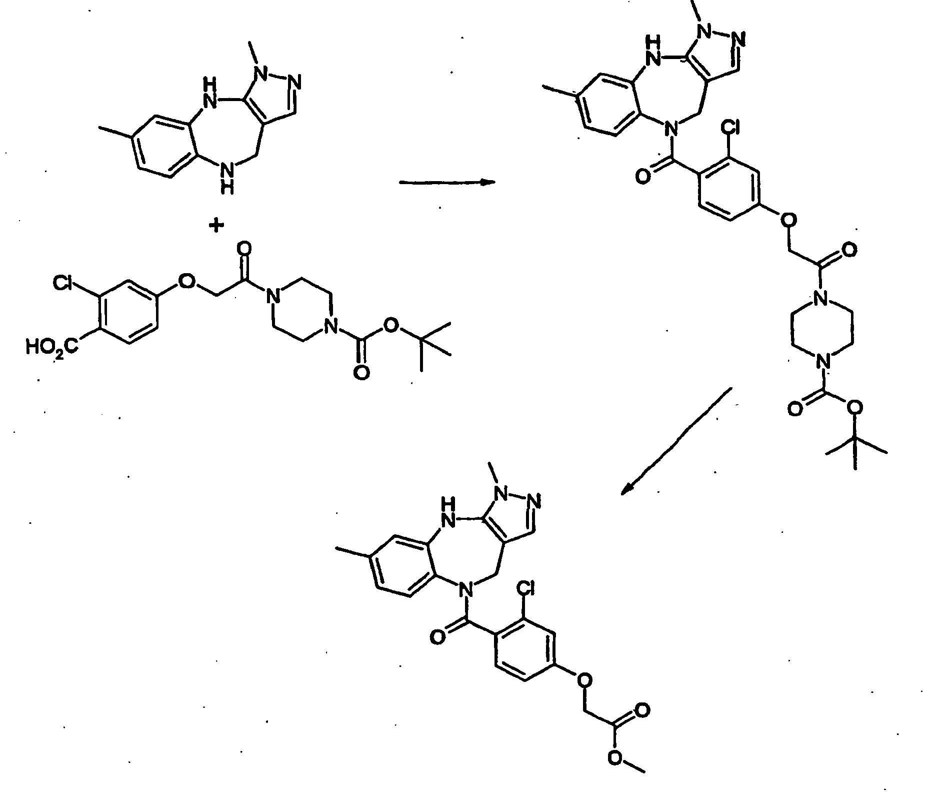

### Example E79

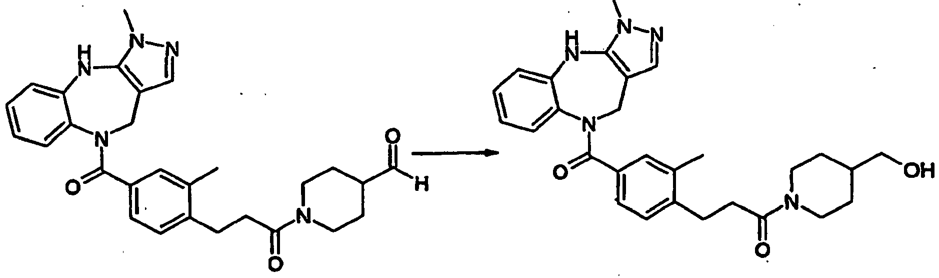

Example E80

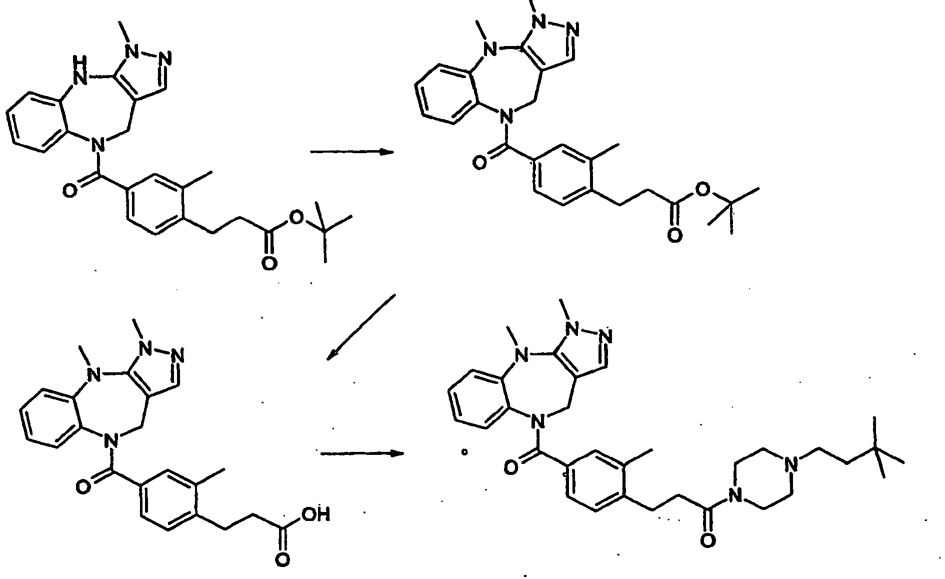

### Example E81

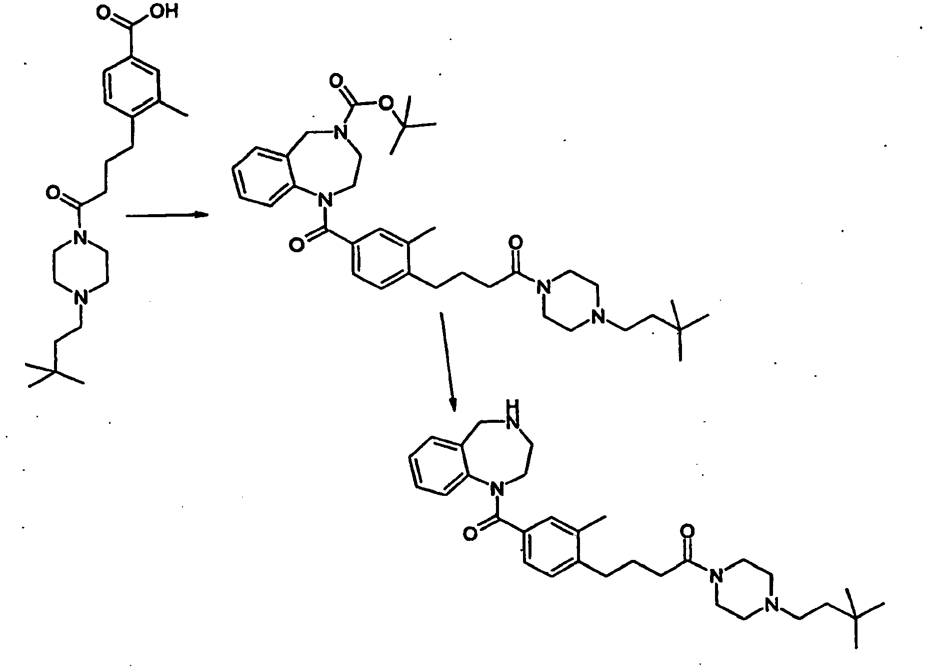

### Example E82

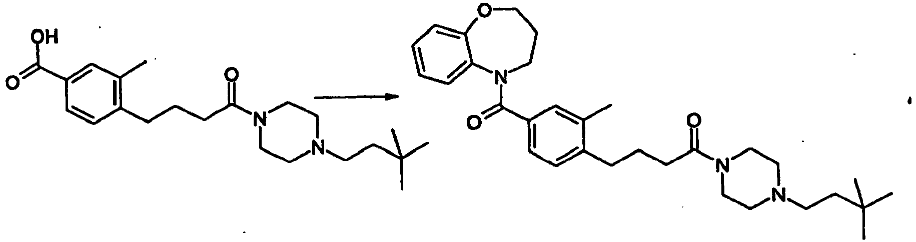

### Example E83

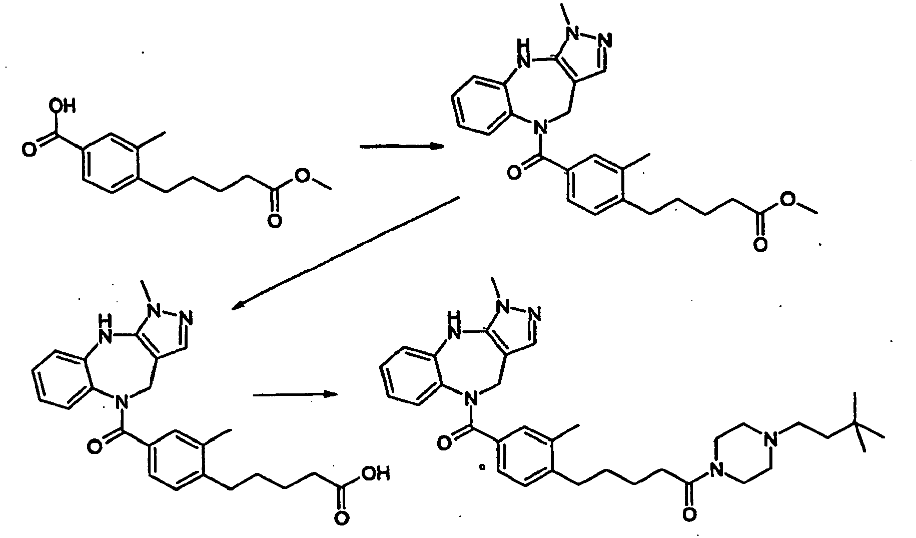

### Example E84

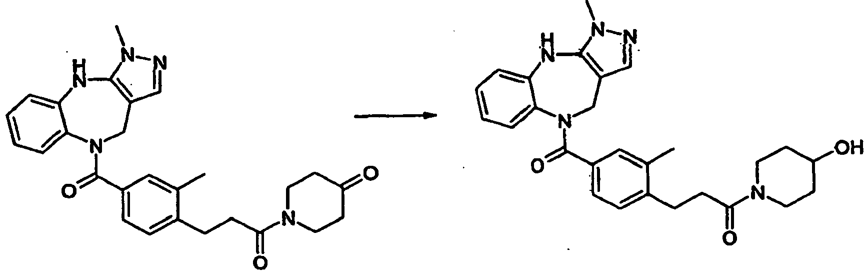

### Example E85

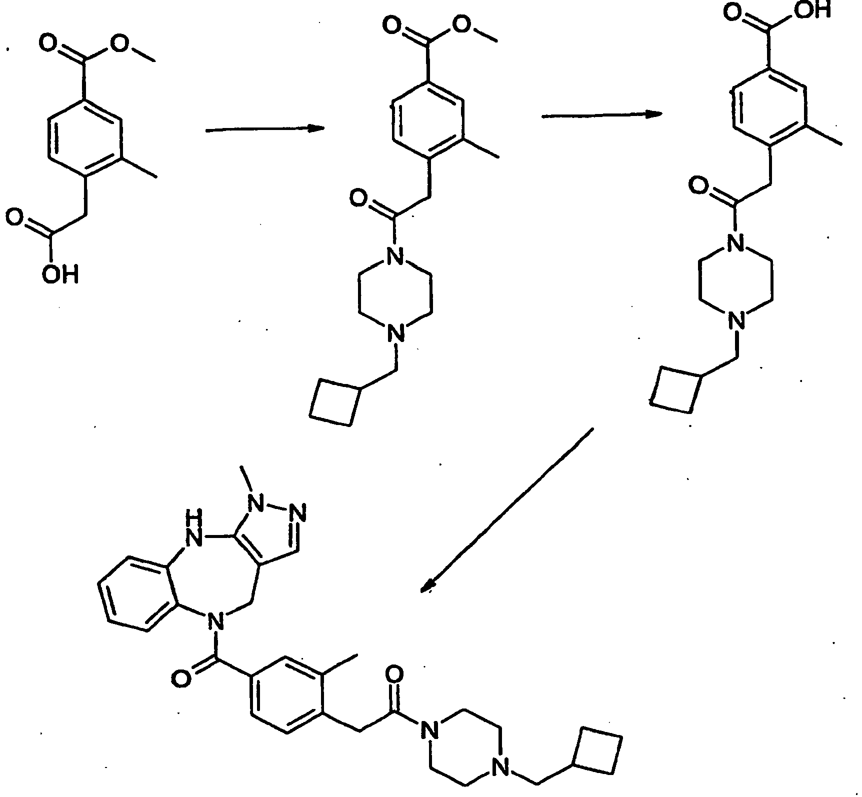

### Example E86

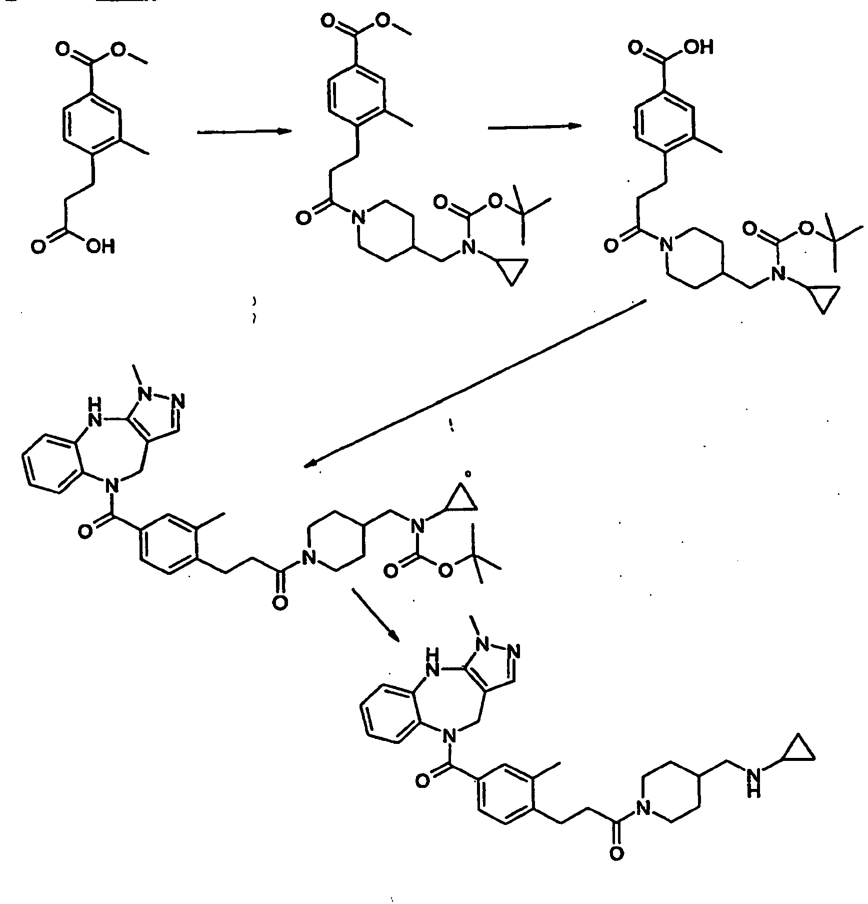

### Example E87

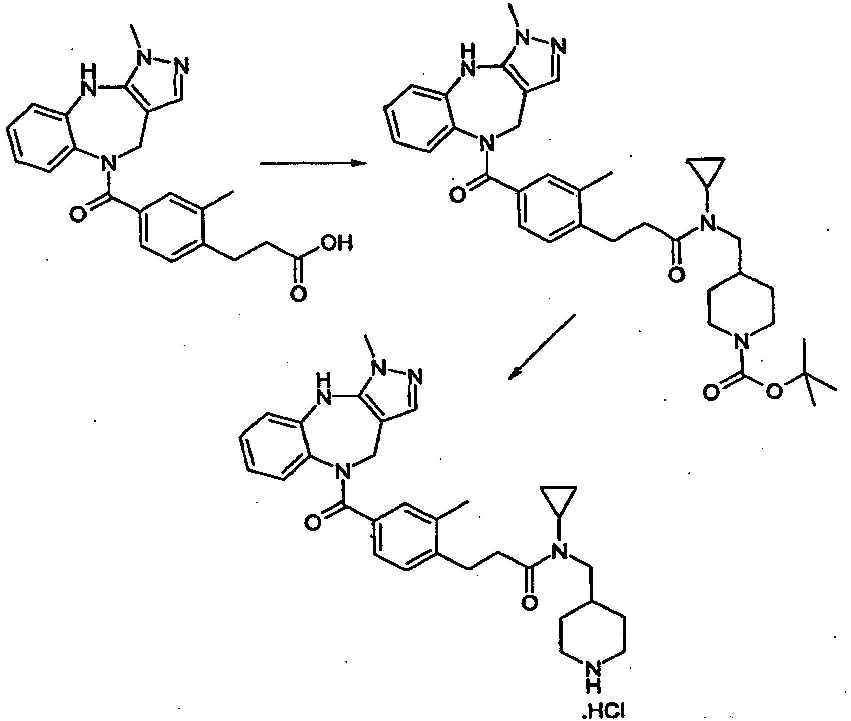

### Example E88

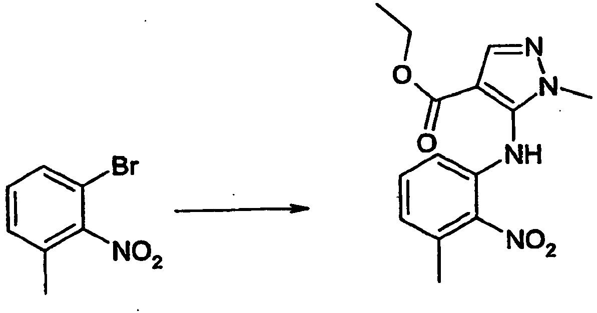

### Example E89

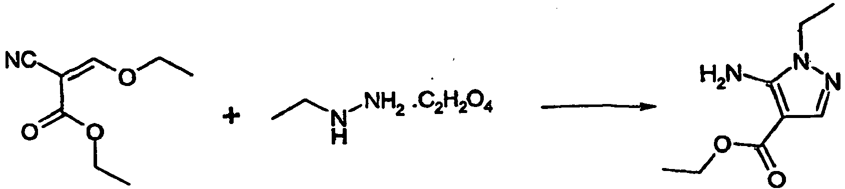

### Example E90

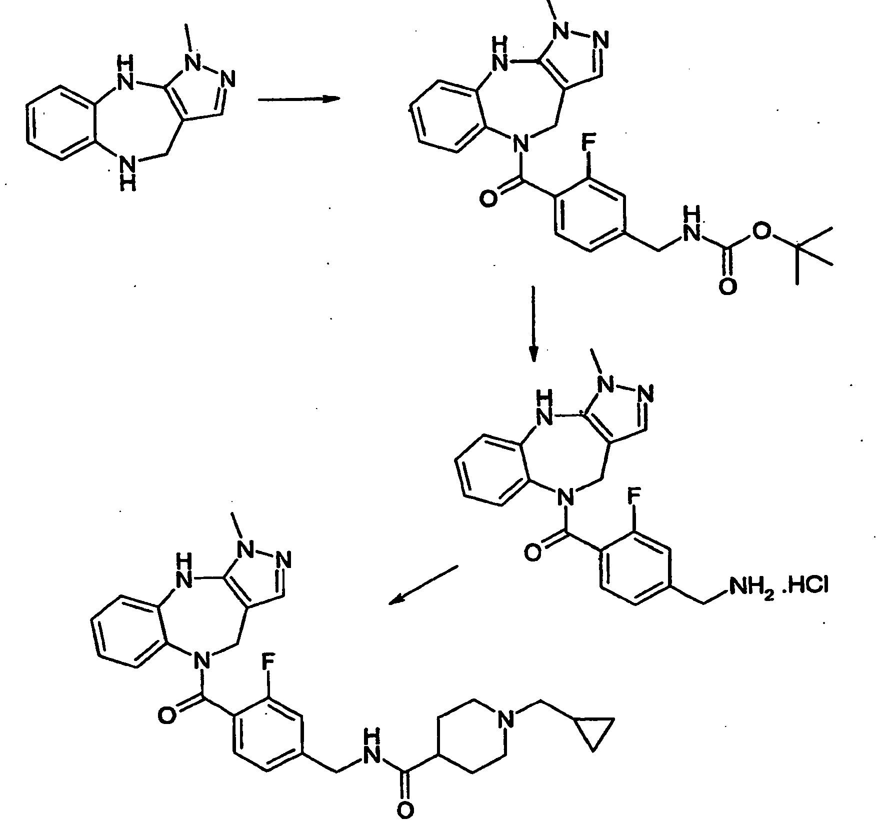

### Example E91

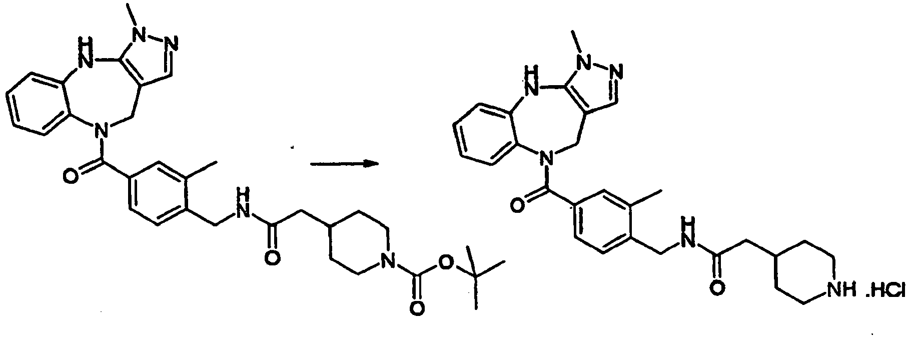

### Example E92

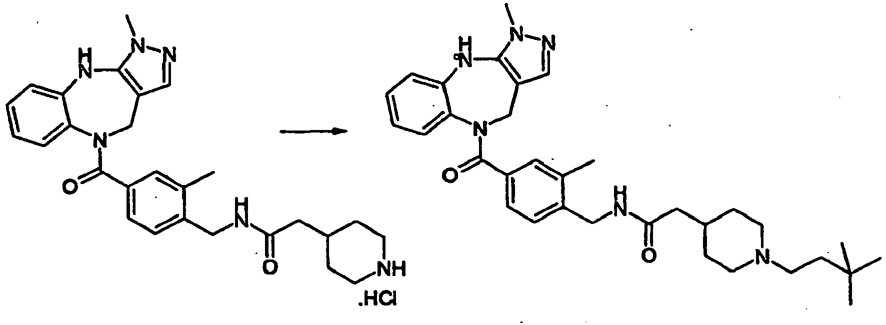

### Example E93

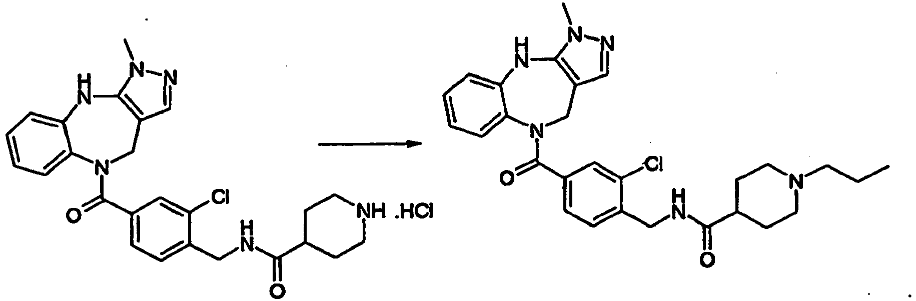

### Example E94

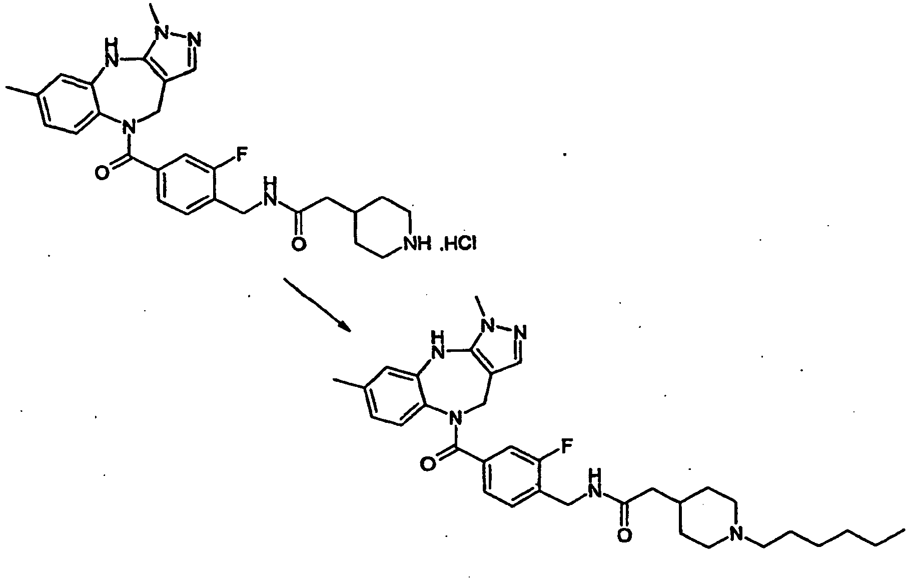

### Example E95

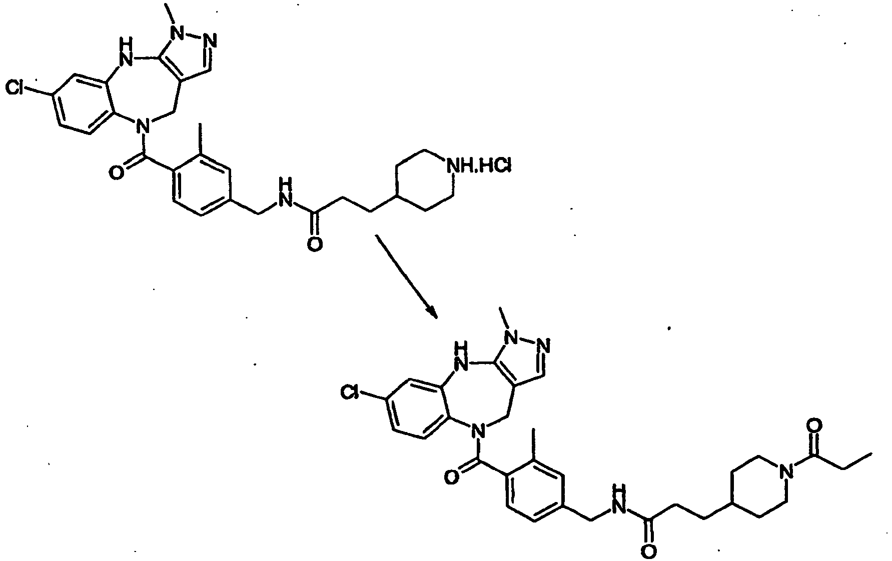

### Example E96

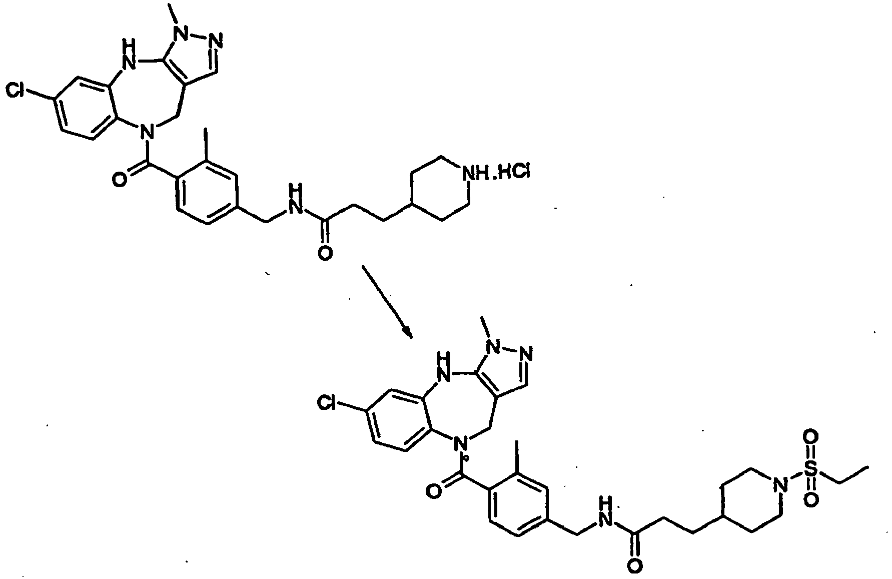

### Example E97

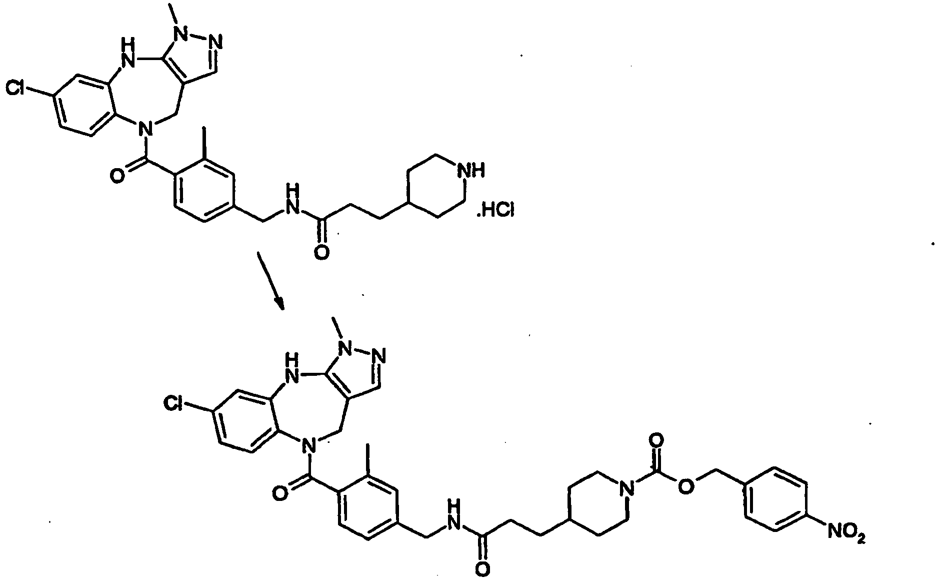

### Example E98

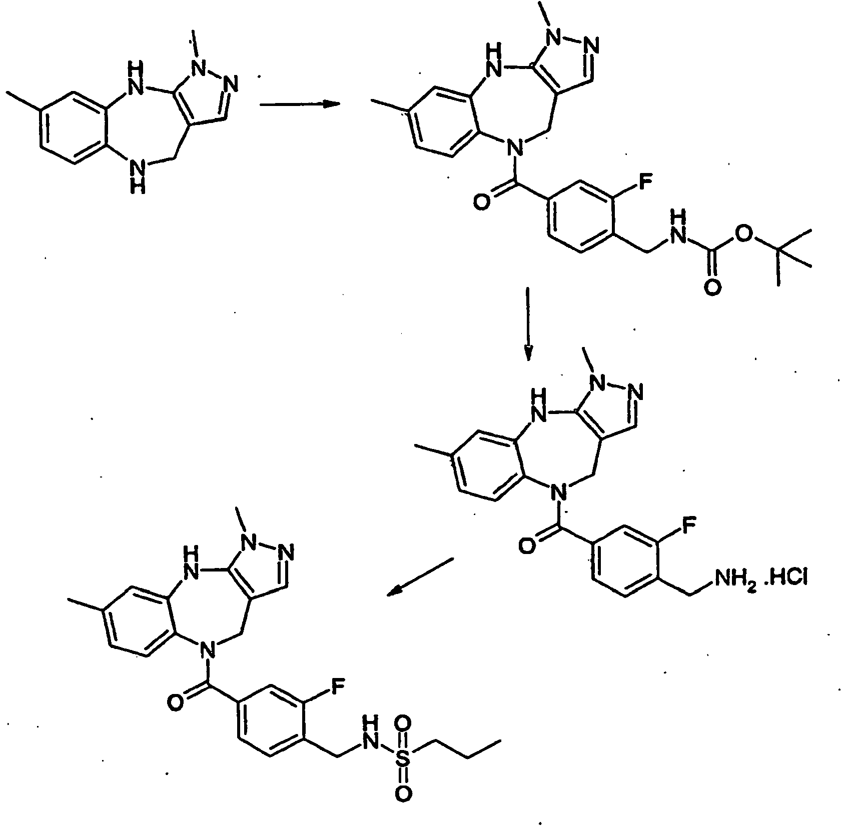

### Example E99

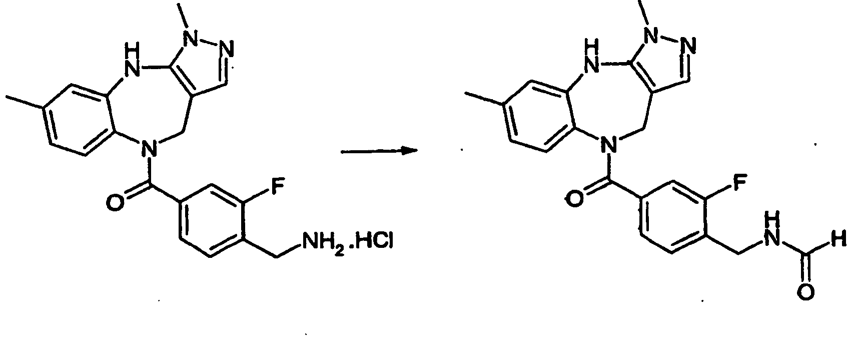

### Example E100

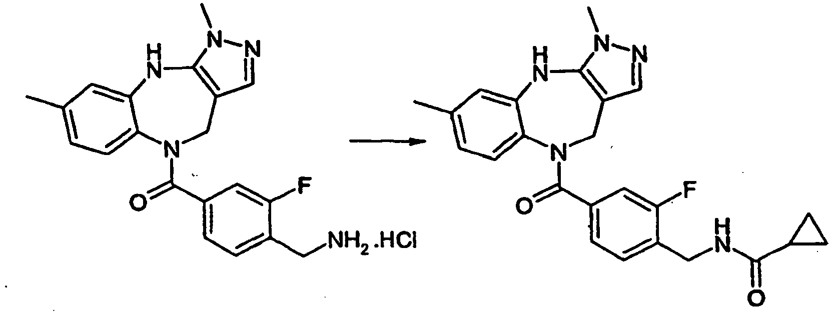

### Example E101

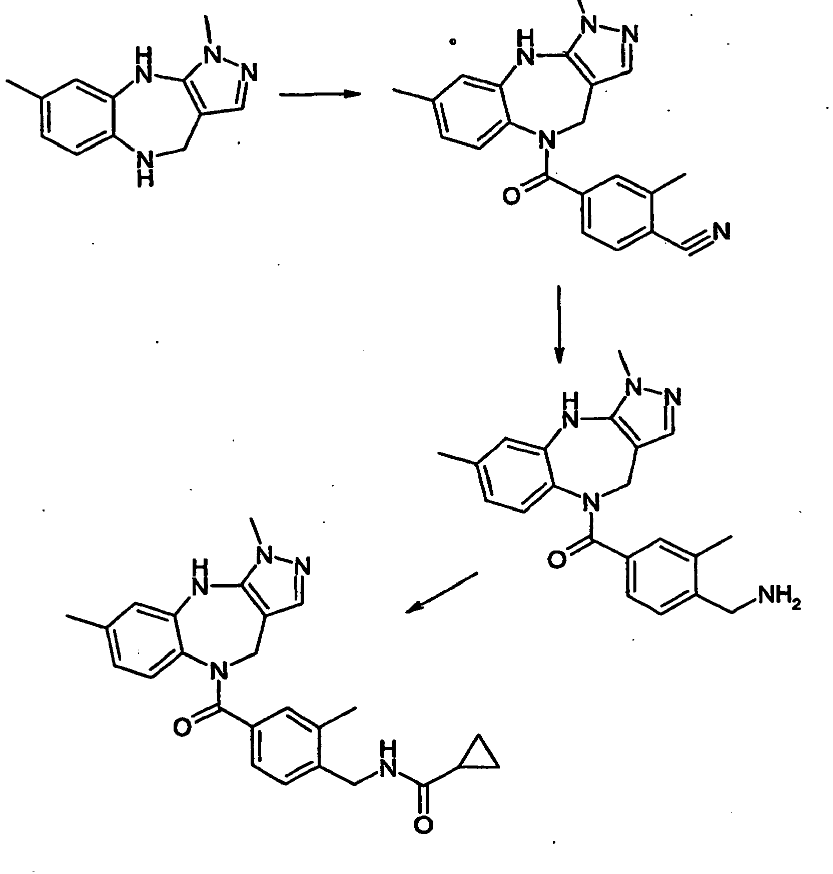

### Example E102

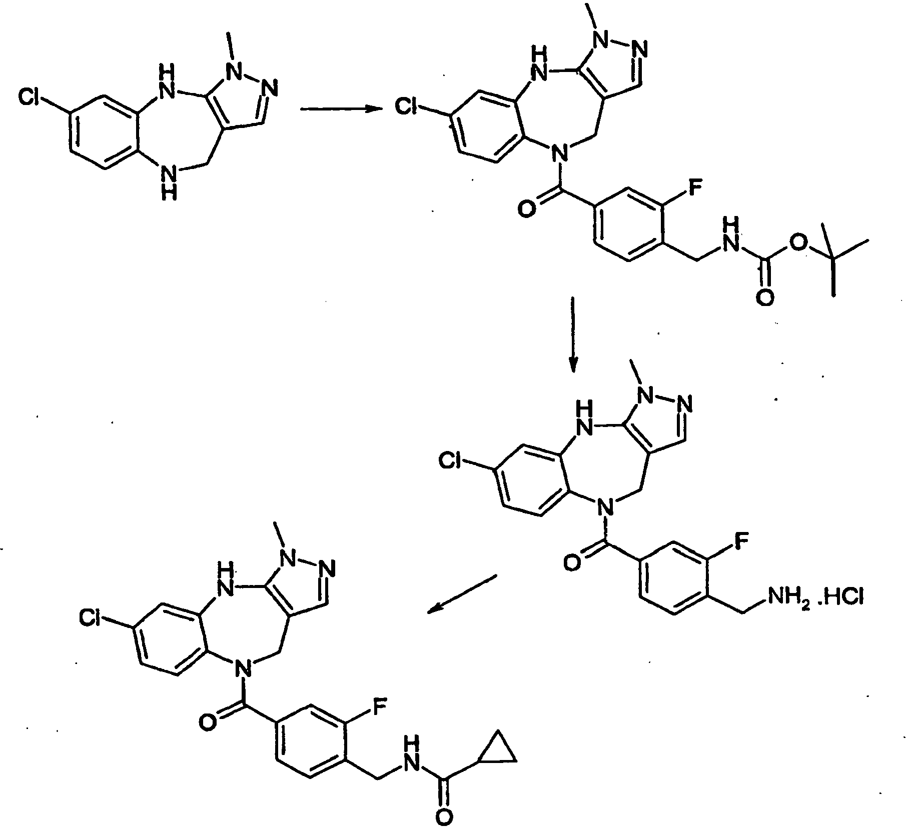

### Example E103

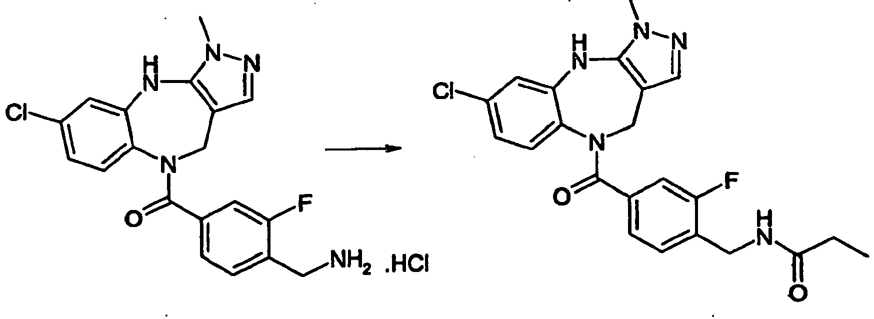

### Example E104

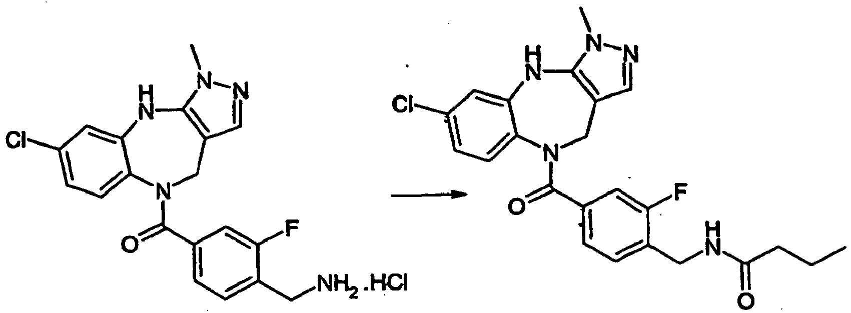

### Example E105

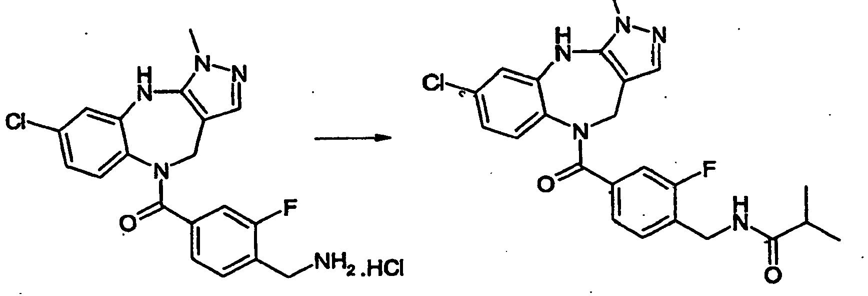

### Example E106

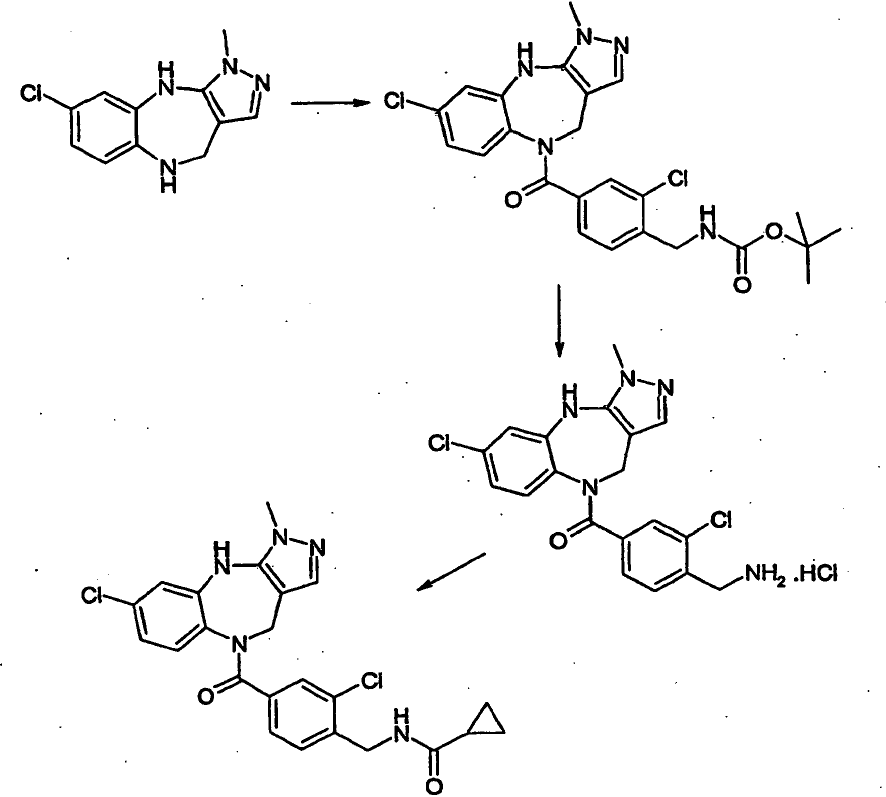

### Example E107

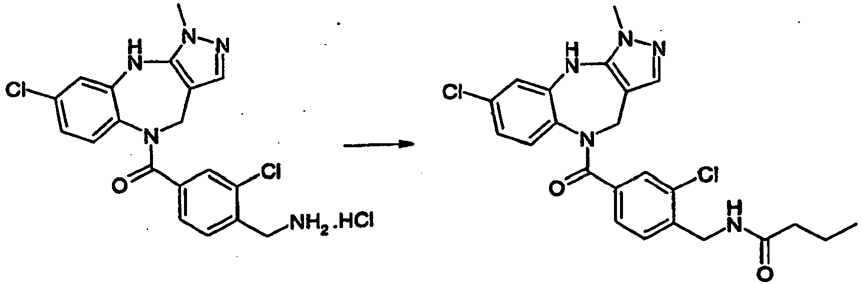

### Example E108

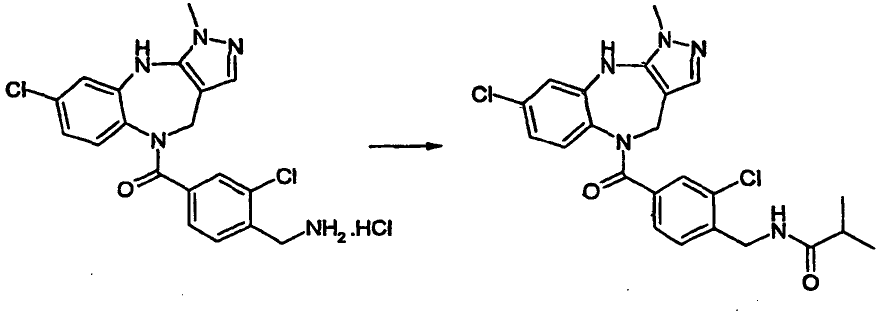

### Example E109

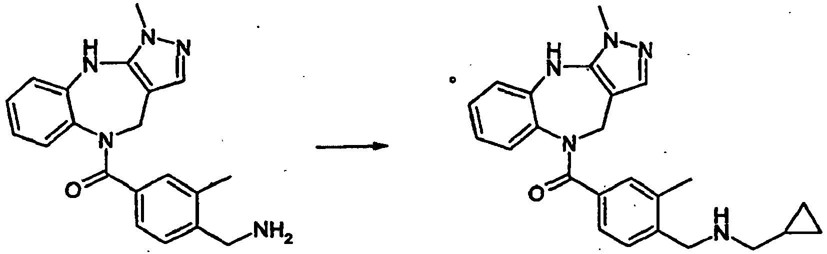

### Example E110 and Example E111

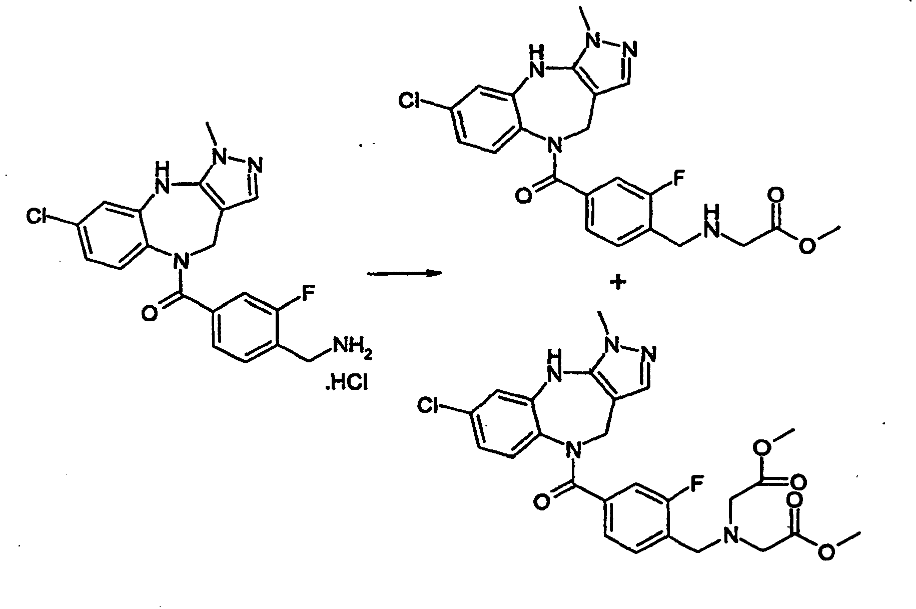

### Example E112

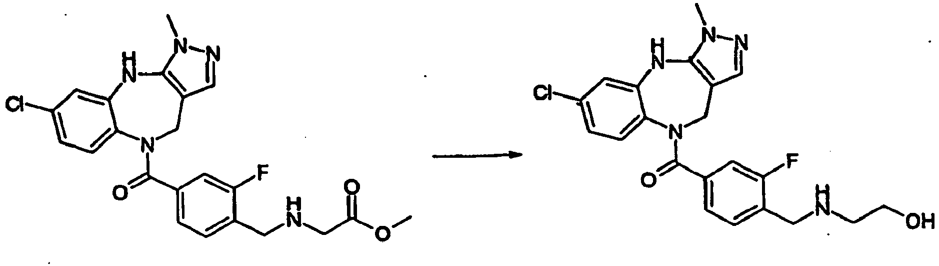

### Example E113

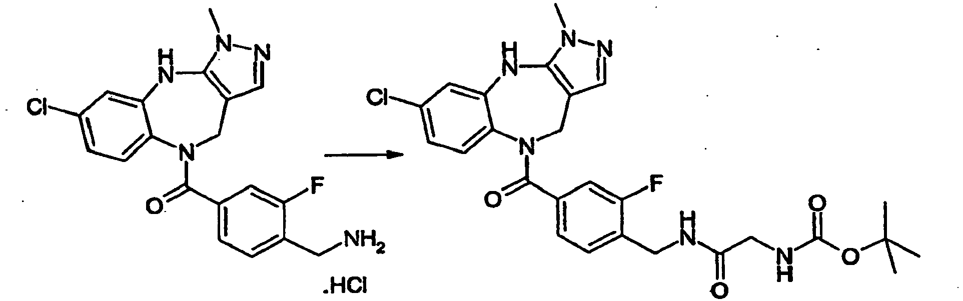

### Example E114

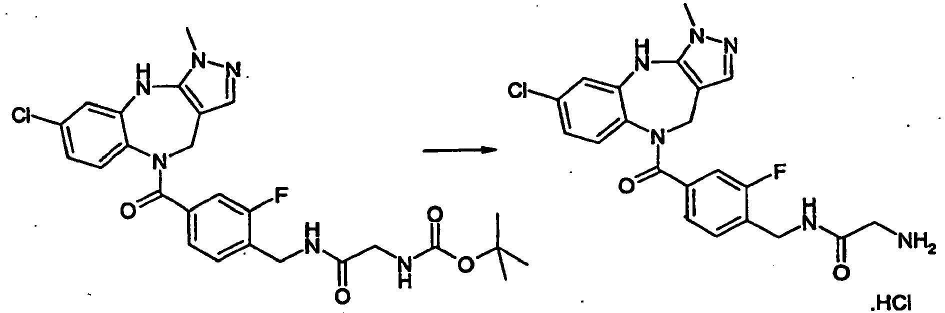

### Example E115

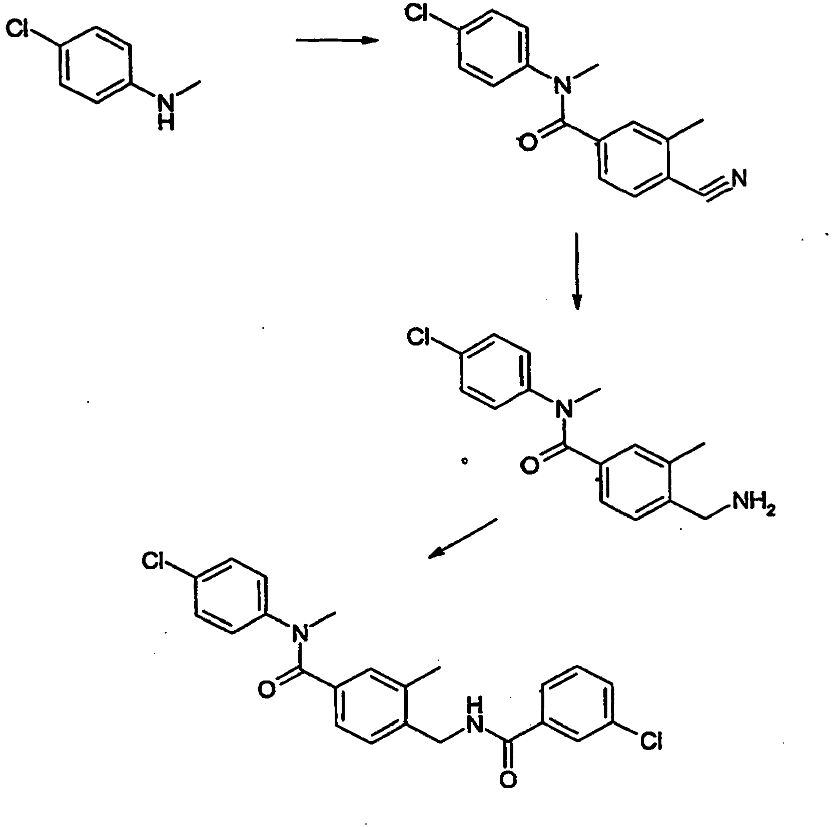

### Example E116

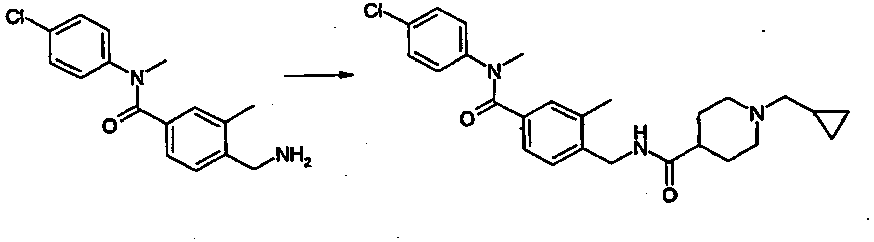

### Example E117

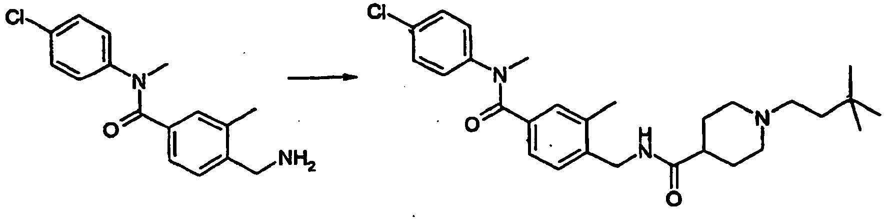

### Example E118

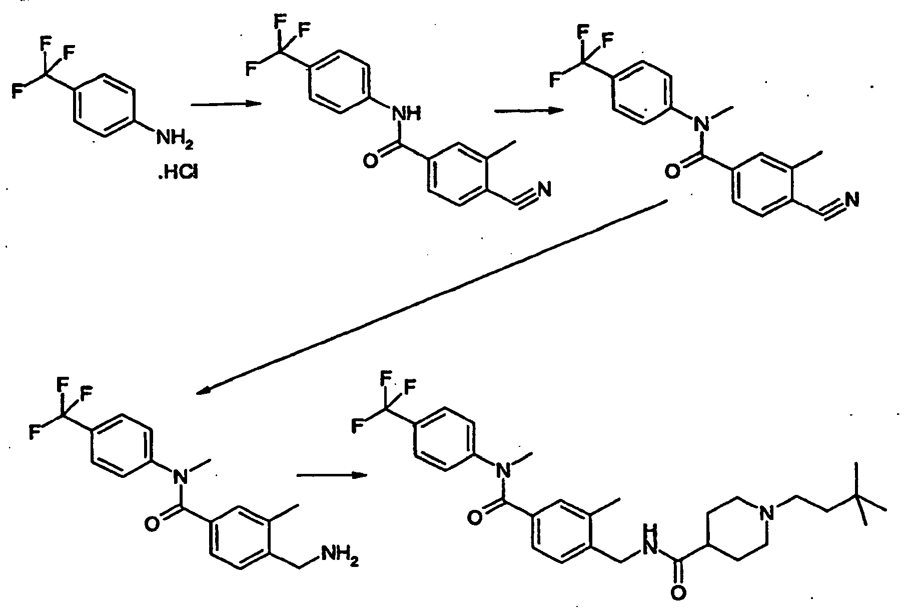

### Example E119

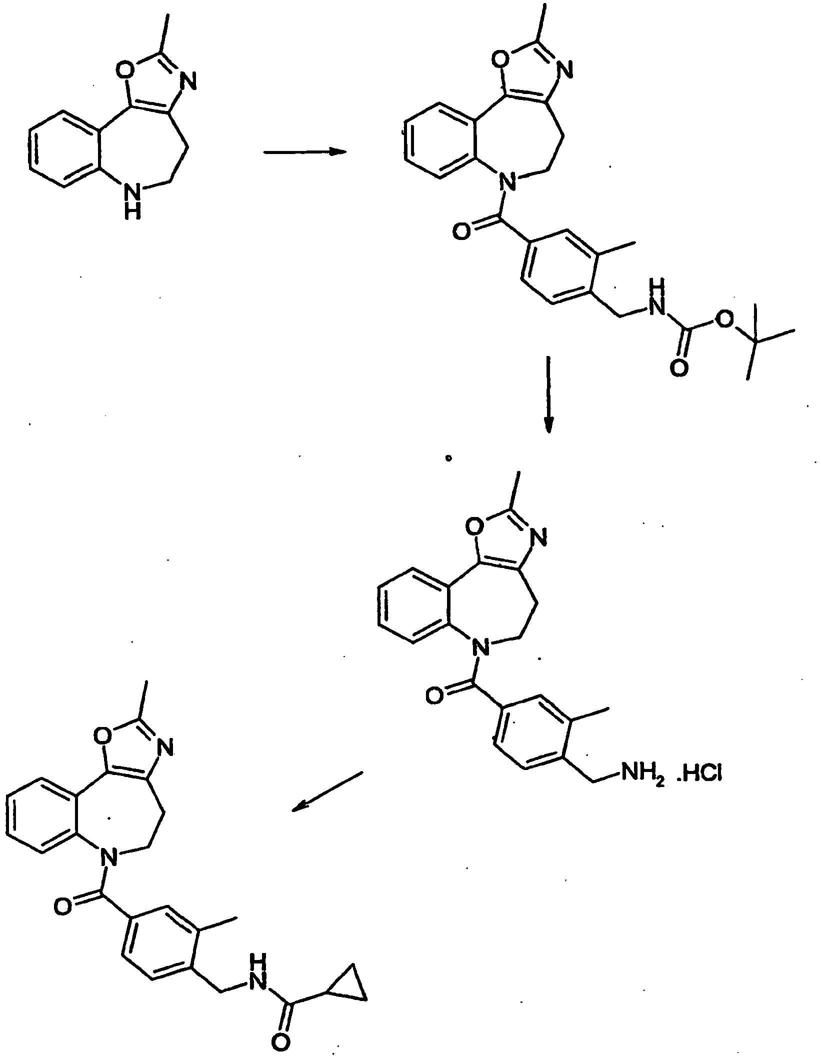

### Example E120

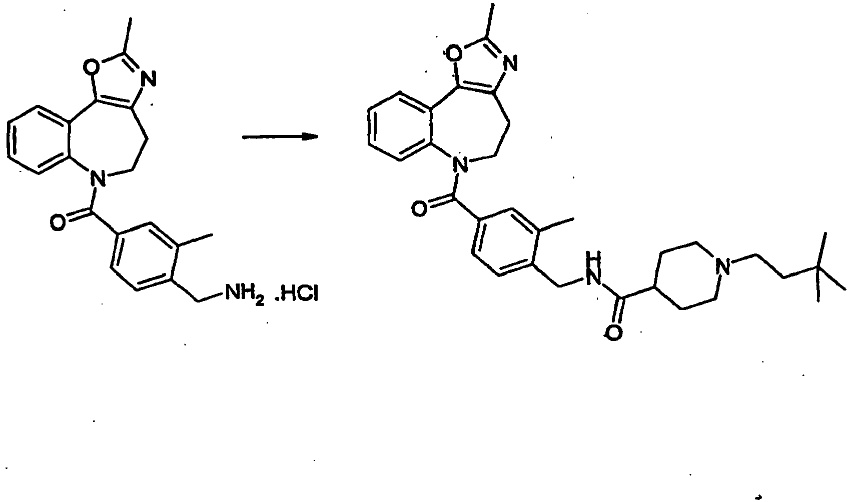

### Example E121

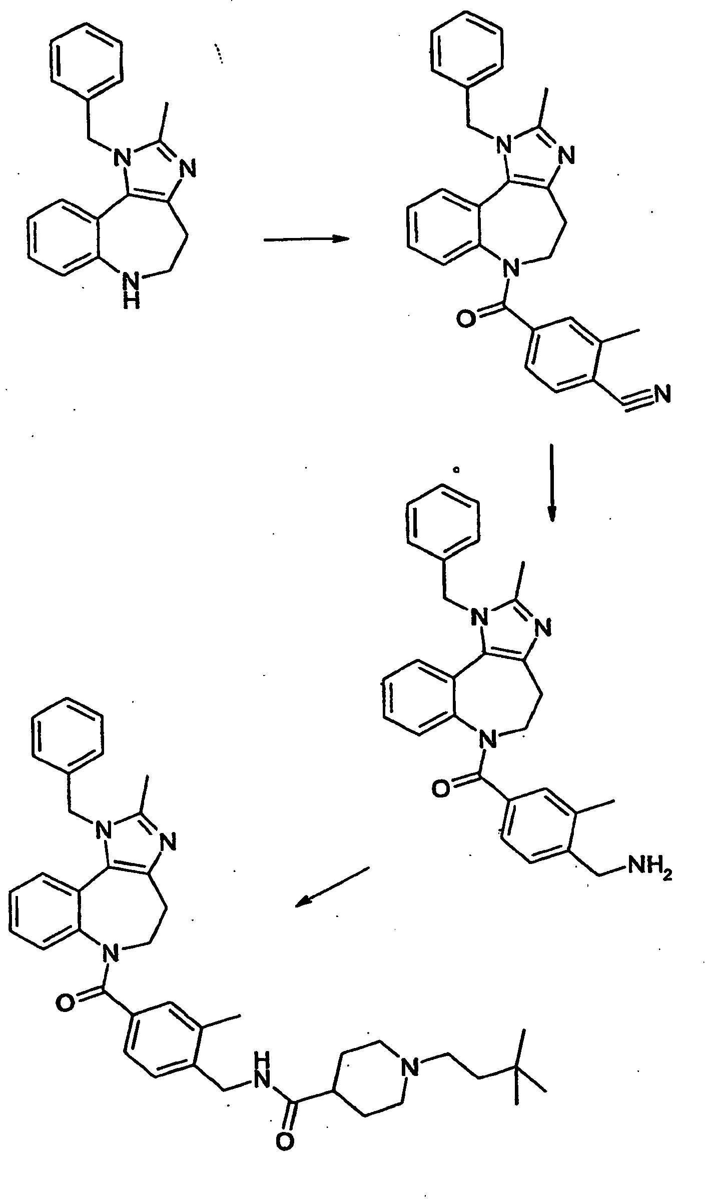

### Example E122

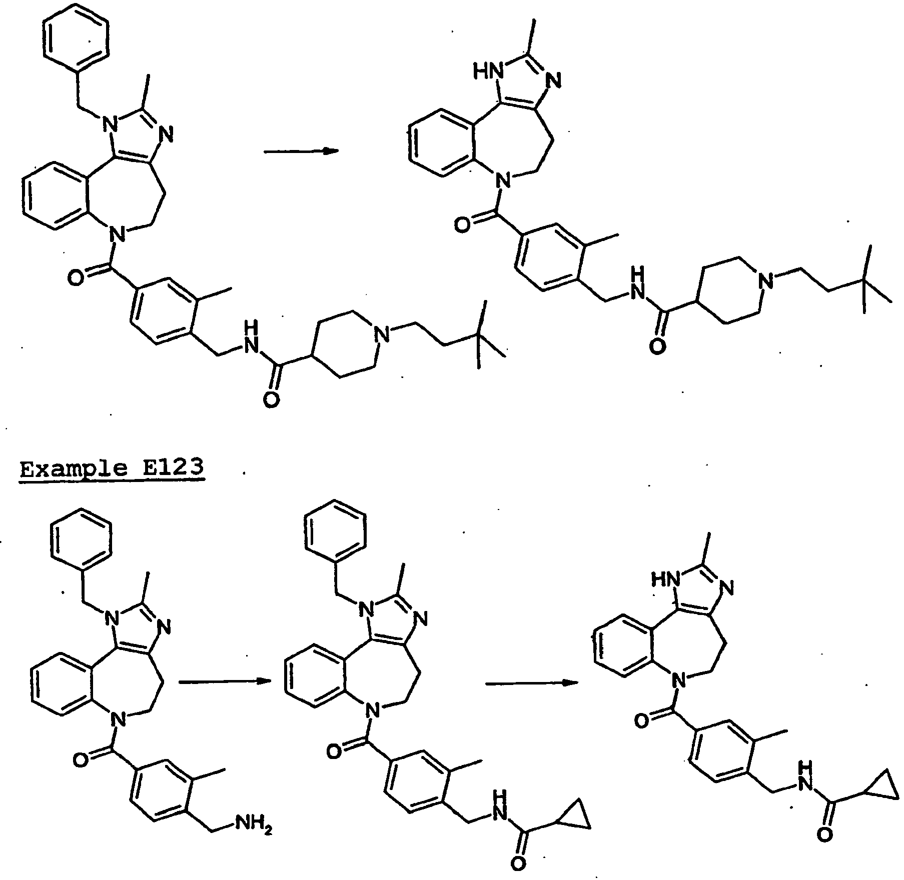

### Example E124

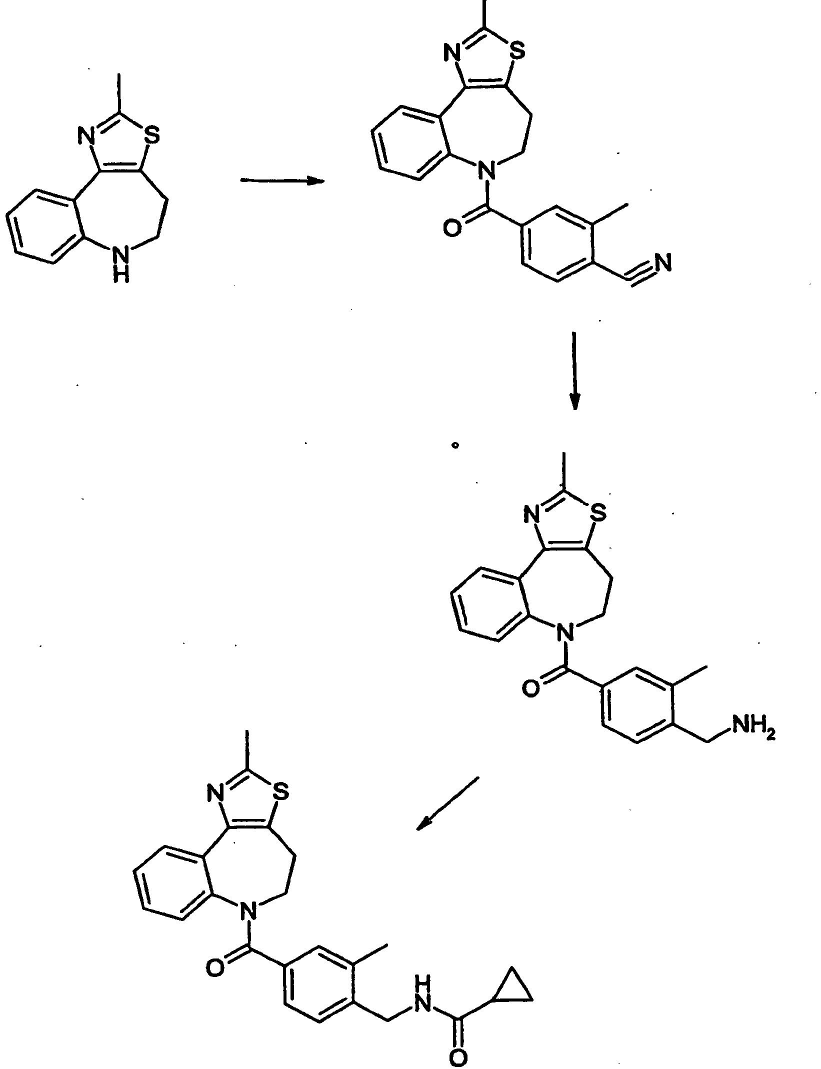

### Example E125

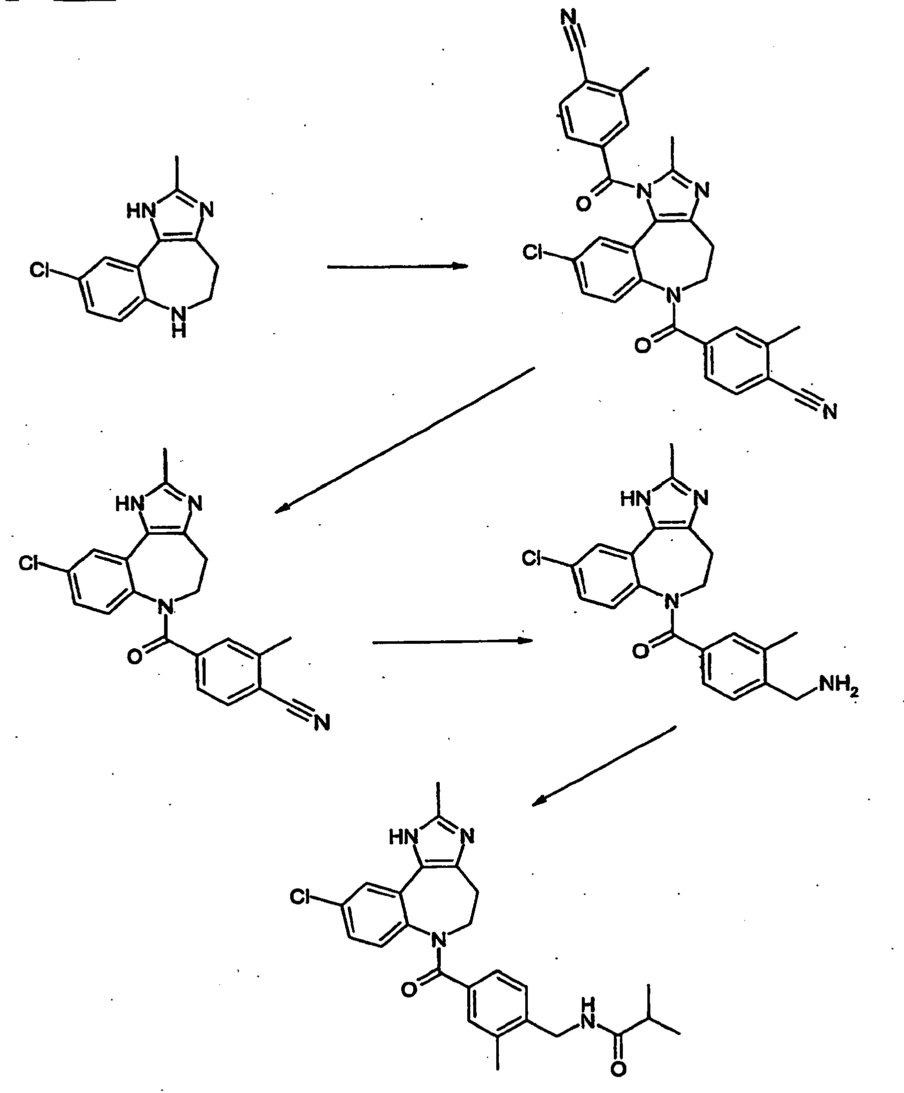

### EXAMPLE AND REFERENCE EXAMPLE TABLES

The following compounds were prepared using methods analogous to those described above.

Examples E5 and Compound number 2 have particular utility as intermediates.

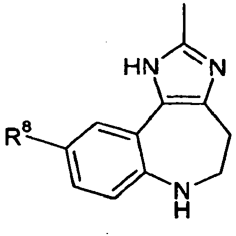

| **Compound number** | **R⁸** | **MS (ESI)+:[M+H]+=** | **¹H NMR: δ (ppm)** |
|---|---|---|---|
| 1 E5 | H | | |
| 2 | Cl | 234.0, 236.0 | 2.43 (3H, s), 2.98 (2H, d, J=5.2Hz), 3.34 (2H, t, J=5.2 Hz), 6.59 (2H, d, J=8.4Hz), 6.90-6.95 (1H, m), 7.80-8.10 (1H, m) |

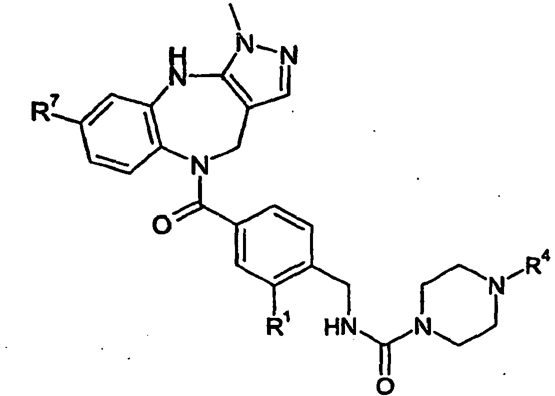

| **Compound number** | **R¹** | **R⁴** | **R⁷** | **MS: [M+H]⁺** |
|---|---|---|---|---|
| 3 E8 | F | 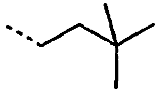 | Me | 562.4 |
| 4 | Me | 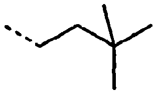 | Me | 558.4 |
| 5 | Me | 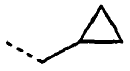 | Me | 528.4 |
| 6 | Me | 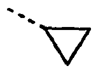 | Me | 514.3 |
| 7 | Me | 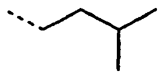 | Me | 544.4 |
| 8 | Me | 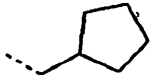 | Me | 556.6 |
| 9 | Me | 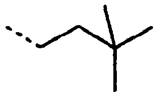 | Cl | 578.4, 580.4 |
| 10 | Me | 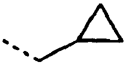 | Cl | 548.3 550.3 |
| 11 | F | 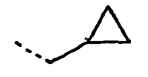 | Me | 532.3 |
| 12 | F | 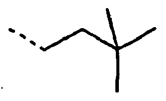 | Cl | 582.4, 584.4 |
| 13 | F | 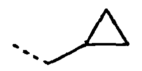 | Cl | 552.3, 554.3 |
| 14 | F | 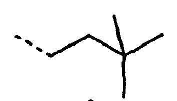 | F | 566.2 |
| 15 | Cl | 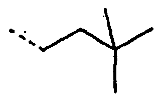 | Cl | 598.3, 600.4 |
| 16 | Cl | 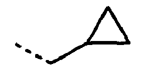 | Cl | 568.1 |
| 17 | Cl | 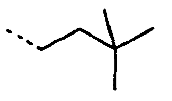 | Me | 578.4, 580.4 |
| 18 | Cl | 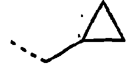 | Me | 548.3, 550.3 |
| 19 | Me | 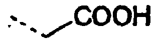 | Me | 532.5 |
| 20 | Me | 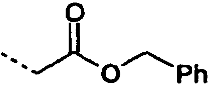 | Me | 622.4 |
| 21 | Me | 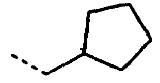 | Cl | 576.4, 578.3 |
| 22 | Me | 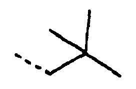 | Cl | 564.4, 566.4 |
| 23 | Me | 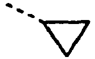 | Cl | 534.3, 536.3 |
| 24 | Me | 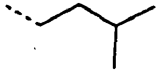 | Cl | 564.4, 566.4 |
| 25 | Me | 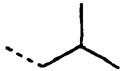 | Cl | 550.4, 552.4 |
| 26 | Me | 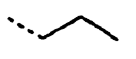 | Cl | 536.4, 538.1 |
| 27 | Me | 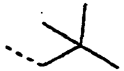 | Me | 544.4 |
| 28 | Me | 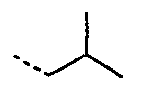 | Me | 530.8 |
| 29 | Me | 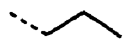 | Me | 516.5 |

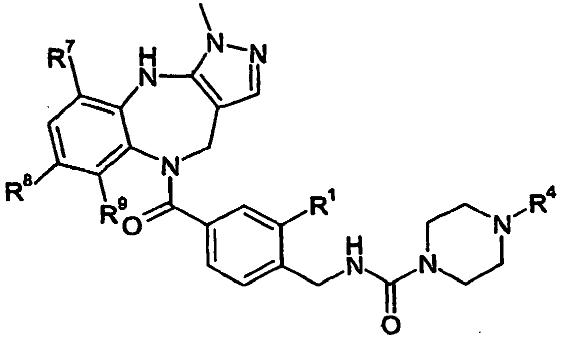

| **Compound number** | **R¹** | **R⁴** | **R⁷** | **R⁸** | **R⁹** | **MS: [M+H]⁺** |
|---|---|---|---|---|---|---|
| 30 | Me | 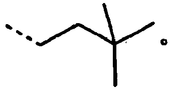 | H | Me | H | 558.4 |
| 31 | F | 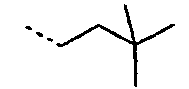 | Me | H | H | 562.4 |

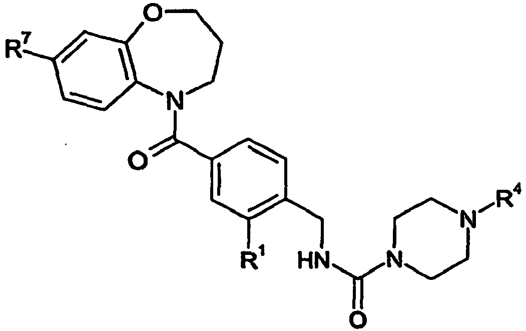

| Compound number | **R¹** | **R⁴** | **R⁷** | **MS: [M+H]⁺** |
|---|---|---|---|---|
| 32 | Me | 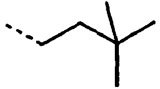 | Cl | 527.1, 529.1 |
| 33 | Me | 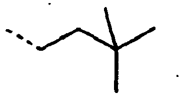 | Me | 507.4 |

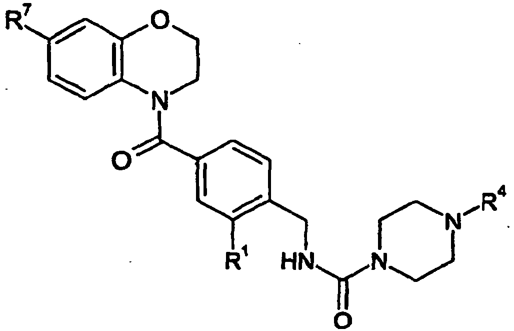

| **Compound number** | **R¹** | **R⁴** | **R⁷** | **MS: [M+H]⁺** |
|---|---|---|---|---|
| 34 | Me | 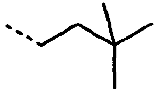 | Me | 493.4 |
| 35 | Me | 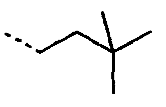 | Cl | 513.1 |

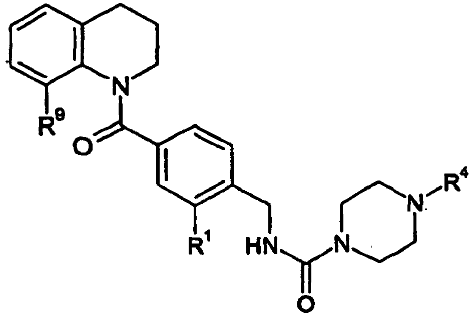

| **Compound number** | **R¹** | **R⁴** | **R⁹** | **MS: [M+H]⁺** |
|---|---|---|---|---|
| 36 | Me | 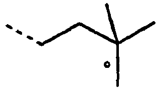 | Me | 491.8 |

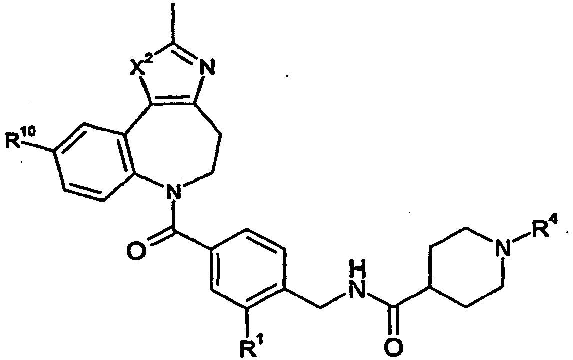

| **Compound number** | **X²** | **R¹** | **R⁴** | **R¹⁰** | **MS: (ESI)⁺: [M+H]⁺** |
|---|---|---|---|---|---|
| 37 | NH | Me | 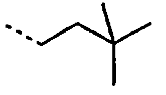 | CI | |
| 38 | NH | Me | 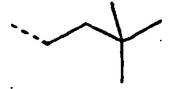 | Me | 557.0 |
| 39 | O | Me | 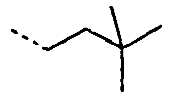 | H | 544.5 |
| 40 | NH | Me | 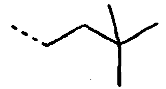 | H | 543.4 |

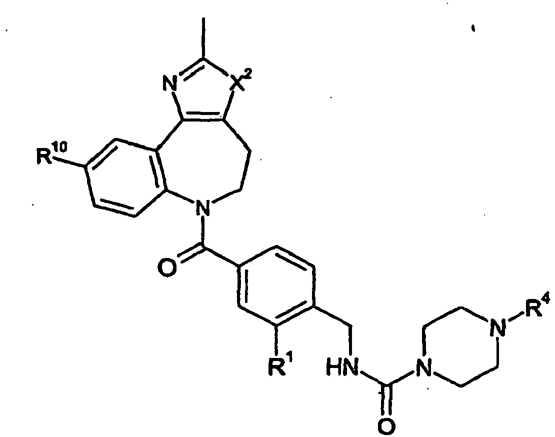

| **Compound** | **X²** | **R¹** | **R⁴** | **R¹⁰** | **MS: [M+H]⁺** |
|---|---|---|---|---|---|
| 41 | S | Me | 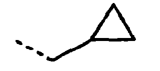 | Me | 544.2 |
| 42 | S | Me | 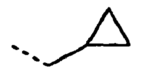 | H | 530.4 |
| 43 | S | Me | 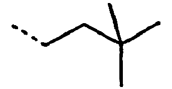 | H | 560.4 |

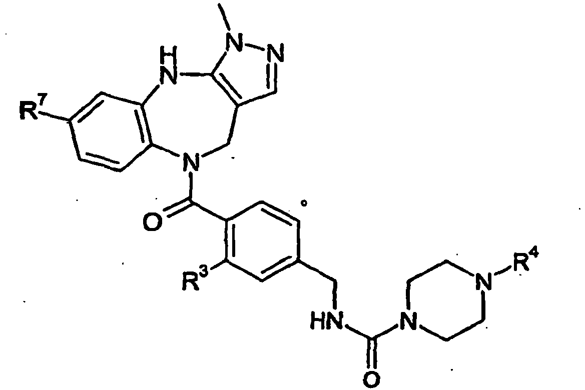

| **Compound number** | **R³** | **R⁴** | **R⁷** | **MS: [M+H]⁺** |
|---|---|---|---|---|
| 44 | Me | 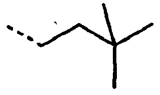 | Me | 558.4 |
| 45 | Me | 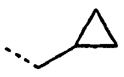 | Me | 528.4 |
| 46 | Me | 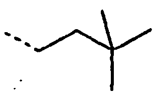 | Cl | 578.3, 580.3 |
| 47 | Me | 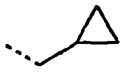 | C! | 548.3, 550.3 |
| 48 | F | 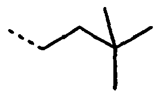 | Me | 562.4 |
| 49 | F | 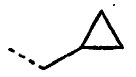 | Me | 532.4 |
| 50 | F | 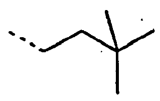 | Cl | 582.4, 584.3 |
| 51 | F | 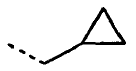 | Cl | 552.3, 554.3 |
| 52 | Cl | 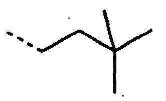 | Cl | 598.3, 600.3 |
| 53 | Cl | 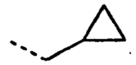 | Cl | 568.3 |
| 54 | Cl | 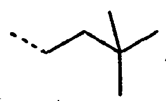 | Me | 578.3, 580.3 |
| 55 | Cl | 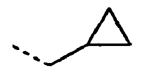 | Me | 548.3, 550.3 |

| **Compound number** | **¹H NMR: δ(ppm)** |
|---|---|
| 3E8 | 0.86 (9H, s), 1.33-1.39 (2H, m), 2.12 (3H, s), 2.27-2.38 (6H, m), 3.33-3.35 (4H, m), 3.68 (3H, s), 3.92, (1H, d, J=14.3Hz), 4.25 (2H, d, ! J=5.4Hz), 5.45 (1H, t, J=5.4Hz), 5.79 (1H, d, J=14.3Hz), 6.43 (1H, d, J=8.0Hz), 6.53 (1H, d, J=8.0Hz), 6.79-7.00 (4H, m). 7.17 (1H, s), 7.31 (1H, s) |
| 4 | 0.88 (9H, s), 1.34-1.41 (2H, m), 2.09 (3H, s), 2.18 (3H, s), 2.29-2.36 (2H, m), 2.40 (4H, t, J=4.7Hz), 3.37 (4H, t, J=4.7Hz), 3.75 (3H, s), 3.94 (1H, d, J=14.4Hz), 4.24 (2H, d, J=5.2Hz), 4.95 (1 H, t), 5.85 (1H, d, J=14.4Hz), 6.53 (1H, q, J=7.9Hz), 6.76 (1H, d, J=5.7Hz), 6.90 (1H, s), 7.02-7.11 (3H, m), 7.58 (1H, s) |
| 5 | 0.05-0.09 (2H, m), 0.47-0.54 (2H, m), 0.81-0.86 (1H, m), 2.08 (3H, s), 2.16 (3H, s), 2.24 (2H, d, J=6.4Hz), 2.47-2.49 (4H, m), 3.38-3.44(4H, m), 3.67 (3H, s), 3.92 (1H, d, J=14.3Hz), 4.22 (2H, m), 4.92 (1H, m), 5.85 (1H, d, J=14.3Hz), 6.43-6.56(2H, m), 6.66-6.85-(3H, m), 7.07-7.24-(3H, m) |
| 6 | 0.45-0.47 (4H, m), 1.64 (1H, m), 2.05 (3H, s), 2.13 (3H, s), 2.56 (4H, m), 3.32 (4H, m), 3.65 (3H, s), 3.90 (1H, d, J=14.6Hz), 4.18 (2H, m), 5.21 (1H, m), 5.82 (1H, d, J=14.6Hz), 6.43 (1H, d, J=8.0Hz), 6.53 (1H, d, J=8.0Hz), 6.80-6.85 (3H, m), 6.93 (1H, s), 7.04 (1H, s), 7.16 (1H, s) |
| 7 | 0.84 (3H, s), 0.86 (3H, s), 1.28-1.37 (2H, m). 1.48-1.60 (1H, m), 2.01(3H, s), 2.10 (3H, s). 2.22-2.35 (2H, m), 2.32 (4H, br t), 3.34 (4H, br t), 3.59 (3H, s), 3.90 (1H, d, J=14.6Hz), 4.16 (2H, d, J=4.9Hz), 5.15 (1H, t, J=5.2Hz), 5.81 (1H, d, J=14.6Hz), 6.40 (1H, d, J=7.9Hz), 6.51 (1H, d, J=7.9Hz), 6.69-6.83 (3H, m), 7.01 (1H, s), 7.11 (1H, s), 7.14 (1H, s) |
| 8 | 1.06-1.23 (2H, m), 1.38-1.59 (4H, m), 1.63-1.78 (2H, m), 1.98-2.05 (1H, m), 2.07 (3H, s), 2.15 (3H, s), 2.22 (2H, d, J=7.4Hz), 2.35 (4H, t, J=4.7Hz), 3.33 (4H, t, J=4.7Hz), 3.67 (3H, s), 3.91 (1H, d, J=14.6Hz), 4.22 (2H, d, J=5.2Hz), 4.84 (1H, br t), 5.84 (1H, d, J=14.6Hz), 6.43 (1H, d, J=8.2Hz), 6.54 (1H, d, J=8.2Hz), 6.67 (1H, s), 6.76 (1H, s), 6.77-6.88 (2H, m), 7.07(1Hs), 7.18(1H, s) |
| 9 | 0.88 (9H, s), 1.34-1.41 (2H, m), 2.09 (3H, s), 2.25-2.36 (2H, m), 2.36-2.41 (4H, m), 3.35-3.42 (4H, m), 3.72 (3H, s), 3.93 (1H, d, J=14.6Hz), 4.24 (2H, d, J=4.45Hz), 5.02 (1H, br t), 5.83 (1H, d, J=14.6Hz), 6.59 (1H, s), 6.88 (1H, s), 7.01-7.09 (3H, m), 7.56-7.63 (2H, m) |
| 10 | 0.11 (2H, q, J=4.7Hz), 0.52 (2H, q, J=4.7Hz), 0.76-0.93 (1H, m), 2.10 (3H, s), 2.31 (2H, d, J=6.4Hz), 2.45-2.62 (4H, m), 3.39-3.53 (4H, m), 3.79 (3H, s), 3.93 (1H, d, J=14.6Hz), 4.23-4.25 (2H, m), 5.27 (1H, s), 5.81 (1H, d, J=14.6Hz), 6.58 (2H, s), 6.80-6.90 (2H, m), 7.07 (3H, s), 7.19 (1H, s) |
| 11 | 0.05-0.11 (2H, m), 0.47-0.55 (2H, m), 0.75-0.89 (1H, m), 2.18 (3H, s), 2.24 (2H, d, J=6.7Hz), 2.47 (4H, m), 3.38 (4H, m), 3.75 (3H, s), 3.93 (1 H, d, J=14.6Hz), 4.31 (2H, d, J=5.7Hz), 5.13 (1H, t, J=5.4Hz), 5.82 (1H, d, J=14.6Hz), 6.47-6.58 (2H, m), 6.72-6.80 (2H, m), 6.87-6.95 (2H, m), 7.00-7.04 (1H, m), 7.08-7.17 (1H, m) |
| 12 | 0.86 (9H, s), 1.33-1.39 (2H, m), 2.28-2.38 (6H, m), 3.37 (4H, m), 3.66 (3H, m), 3.94 (1H, d, J=14.6Hz), 4.28 (2H, d, J=5.5Hz), 5.41 (1H, t, J=5.5Hz), 5.78 (1H, d, J=14.6Hz), 6.56 (2H, s), 6.84-6.88 (2H, m), 7.00-7.06 (2H, m), 7.18 (1H, s), 7.98 (1H, s) |
| 13 | 0.27-0.29 (2H, m), 0.65-0.67 (2H, m), 0.94-1.10 (1H, m), 1.36-1.56 (2H, m), 2.59 (1 H, d, J=6.4Hz), 2.76-2.89 (4H, m), 3.54-3.70 (4H, m), 3.83 (3H, s), 3.96 (1H, d, J=14.6), 4.31-4.35 (2H, m), 5.81 (1H, d, J=14.6Hz), 6.63 (1H, s), 6.83-7.00 (1H, m), 7.11 (4H, s), 7.23-7.24 (1H, m), 7.70 (1H, s) |
| 14 | 0.89 (9H, s), 1.37-1.43 (2H, m), 2.33-2.43 (6H, m), 3.35-3.39 (4H, m), 3.74 (3H, s), 3.96 (1H, d, J=14.6Hz), 4.34 (2H, d, J=5.0Hz), 5.83 (1H, d, J=14.6Hz), 6.35-6.41 (1H, m), 6.62-6.73 (2H, m), 6.80 (1H, s), 6.89-6.93 (2H, m), 7.06-7.12 (1H, m) |
| 15 | 0.87 (9H, s), 1.33-1.39 (2H, m), 2.28-2.38 (6H, m), 3.37 (4H, m), 3.66 (3H, m), 3.94 (1H, d, J=14.6Hz), 4.31 (2H, d, J=5.5Hz), 5.45 (1H, t, J=5.5Hz), 5.78 (1H, d, J=14.6Hz), 6.91 (1H, m), 7.03 (2H, m), 7.19 (2H, m), 7.84 (1H, s) |
| 16 | (CD3OD): 0.14-0.18 (2H, m), 0.52-0.59 (2H, m), 0.89 (1H, m), 2.28-2.30 (2H, m), 2.52-2.56 (4H, m), 3.43-3.47 (4H, m), 3.80 (3H, s), 3.97 (1H, d, J=14.6Hz), 4.34 (2H, d, J=4.5Hz), 5.73 (1H, d, J=14.6Hz), 6.64-6.77 (2H, m), 7.08-7.17 (2H, m), 7.23-7.28 (3H, m) |
| 17 | 0.87 (9H, s), 1.36 (2H, m), 2.12 (3H, s), 2.32 (6H, m), 3.39 (4H, m), 3.66 (3H, s), 3.92 (1H, d, J=14.3Hz), 4.28 (2H, m), 5.43 (1H, m), 5.78 (1H, d, J=14.3Hz), 6.48 (1H, d, J=7.9Hz), 6.52 (1H, d, J=7.9Hz), 6.78 (1H, s), 6.88-6.98 (2H, m), 7.05 (1H, s), 7.17 (1H, m) |
| 18 | 0.06-0.08 (2H, m), 0.48-0.51 (2H, m), 0.76-0.89 (1H, m), 2.15 (3H, s), 2.21-2.29 (2H, m), 2.44-2.48 (4H, m), 3.36-3.38 (4H, m), 3.68 (3H, s), 3.93 (1H, d, J=14.6Hz), 4.30 (2H, d, J=6.0Hz), 5.33 (1H, m), 5.80 (1H, d, J=14.6Hz), 6.48-6.55 (2H, m), 6.77-7.00 (4H, m), 7.19-7.21 (2H, m) |
| 19 | CD3OD: 2.18 (3H, s), 2.22 (3H, s), 3.05-3.09 (4H, m), 3.47 (2H, s), 3.58-3.62 (4H, m), 3.79 (3H, s), 3.93 (1H, d, J=14.6Hz), 4.25 (2H, s), 5.75 (1H, d, J=14.6Hz), 6.47 (1H, d, J=8.0Hz), 6.59 (1H, d, J=8.0Hz), 6.97-7.06 (4H, m), 7.21 (1 H, s) |
| 20 | 2.02 (3H, s), 2.11 (3H, s), 2.39-2.50 (4H, m), 3.22 (2H, s), 3.32-3.43 (4H, m), 3.60 (3H, s), 3.90 (1H, d, J=14.6Hz), 4.16 (2H, d, J=5.0Hz), 5.12 (2H, s), 5.25 (1 H, t, J=5.0Hz), 5.80 (1H, d, J=14.6Hz), 6.41 (1H, d, J=7.9Hz), 6.51 (1 H, d, J=7.9Hz), 6.76-6.83 (3H, m), 7.02 (1H, s), 7.12-7.14 (2H, m), 7.28-7.31 (5H, m) |
| 21 | 1.11-1.18 (2H, m), 1.45-1.56 (4H, m), 1.67-1.72 (2H, m), 1.98-2.01, (1 H, m), 2.04 (3H, s), 2.22 (2H, d, J=7.2Hz), 2.29-2.50 (4H, m), 3.30-3.41 (4H, m), 3.62 (3H, s), 3.91 (1H, d, J=14.6Hz), 4.19-4.21 (2H, m), 5.04-5.06 (1H, m), 5.80 (1H, d, J=14.6Hz), 6.53-6.54 (2H, m), 6.80-6.84 (2H, m), 7.01-7.03 (2H, m), 7.16 (1H, s), 7.72 (1H, s) |
| 22 | 0.84 (9H, s), 2.05 (2H, s), 2.12 (3H, s), 2.41-2.54 (4H, m), 3.27-3.39 (4H, m), 3.72 (3H, s), 3.92 (1H, d, J=14.6Hz), 4.26-4.27 (2H, m), 4.66-4.68 (1H, m), 5.85 (1H, d, J=14.6Hz), 6.58-6.59 (2H, m), 6.88-6.92 (2H, m), 6.98-7.00 (2H, m), 7.09 (1H, s), 7.21 (1H, s) |
| 23 | 0.38-0.44 (4H, m), 1.57-1.58 (1 H, m), 2.04 (3H, s), 2.53 (4H, m), 3.31 m), 3.62 (3H, s), 3.91 (1H, d, J=14.6Hz), 4.20 (2H, m), 5.08 (1H, m), 5.80 (1H, d, J=14.6Hz), 6.54-6.58 (2H, m), 6.80-6.86(2H, m), 7.01-7.03 (2H, m), 7.16 (1H, s), 7.71 (1H, s) |
| 24 | 0.85 (6H, d, J=6.4Hz), 1.23-1.36 (2H, m), 1.49-1.56 (1H, m), 2.01 (3H, s), 2.23-2.35 (6H, m), 3.36 (4H, m), 3.62 (3H, s), 3.91 (1H, d, J=14.6Hz), 4.18 (2H, m), 5.29 (1 H, m), 5.78 (1H, d, J=14.6Hz), 6.48-6.56 (2H, m), 6.80-6.86 (2H, m), 6.98-7.02 (2H, m), 7.15 (1H, s), 8.12 (1H, s) |
| 25 | 0.85 (6H, d, J=6.4Hz) 1.73 (1 H, m), 2.05 (5H, m), 2.26-2.31 (4H, m), 3.34 (4H, m), 3.62 (3H, s), 3.91 (1H, d, J=14.6Hz), 4.21 (2H, m), 5.02 (1 H, m), 5.80 (1H, d, J=14.6Hz), 6.54 (2H, m), 6.84-6.87 (2H, m), 7.02 (2H, m), 7.16 (1H, s), 7.72 (1H, s) |
| 26 | 0.85 (3H, t, J=7.2Hz), 1.40-1.49 (2H, m), 2.01 (3H, s), 2.22-2.28 (2H, m), 2.34 (4H, m) 3.35 (4H, m), 3.58 (3H, s), 3.90 (1H, d, J=14.6Hz), 4.18 (2H, m), 5.21 (1H, m), 5.77 (1H, d, J=14.6Hz), 6.51-6.55 (2H, m), 6.81-6.85 (2H, m), 7.00 (2H, m), 7.13 (1H, s), 7.88 (1H. s) |
| 27 | 0.84 (9H, s), 2.04 (2H, s), 2.11 (3H, s), 2.18 (3H, s). 2.45 (4H, t, J=4.7Hz), 3.30 (4H, t, J=4.7Hz), 3.71 (3H, s), 3.92 (1H, d, J=14.6Hz), 4.24 (2H, d, J=5.2Hz), 4.66 (1H, br t), 5.86 (1H, d, J=14.6Hz), 6.41 (1H, s), 6.46 (1H, d, J=7.9Hz), 6.56 (1H, d, J=7.9Hz), 6.75 (1H, s), 6.88 (2H, s), 7.10 (1H, s), 7.20 (1H, s) |
| 28 | 0.86 (6H, d, J=6.7Hz), 1.68-1.81 (1H, m), 2.05 (2H, d, J=7.4Hz), 2.09 (3H, s), 2.17 (3H, s), 2.32 (4H, t, J=4.7Hz), 3.33 (4H, t, J=4.7Hz), 3.69 (3H, s), 3.92 (1H, d, J=14.6Hz), 4.23 (2H, d, J=4.9Hz), 4.78 (1H, t, J=4.9Hz), 5.85 (1H, d, J=14.6Hz), 6.46 (1H, d, J=7.9Hz), 6.55 (1H, d, J=7.9Hz), 6.57 (1H, s), 6.76 (1H, s), 6.86 (2H, s), 7.08 (1H, s), 7.19 (1H, s) |
| 29 | 0.88 (3H, t, J=7.4Hz), 1.43-1.54 (2H, m), 2.12 (3H, s), 2.19 (3H, s), 2.28 (2H, t, J=7.4Hz), 2.38 (4H, t, J=4.9Hz), 3.35 (4H, t, J=4.9Hz), 3.73 (3H, s), 3.93 (1H, d, J=14.6Hz), 4.25 (2H, d, J=5.2Hz), 4.66 (1H, br t), 5.87 (1H, d, J=14.6Hz), 6.29 (1H, s), 6.47 (1H, d, J=7.7Hz), 6.56 (1H, d, J=7.7.Hz), 6.74 (1H, s), 6.89 (2H, s), 7.11 (1 H, s), 7.21 (1H, s) |
| 30 | 0.88 (9H, s), 1.32-1.42 (2H, m), 2.02 (3H, s), 2.14 (3H, s), 2.28-2.46 (6H, m), 3.30-3.42 (4H, m), 3.75 (3H, s), 3.94 (1H, d, J=14.4Hz), 4.26-4.32 (2H, m), 4.48-4.56 (1H, m), 5.87 (1H, d, J=14.4Hz), 6.07 (1H, s), 6.52 (1H, s), 6.78-6.97 (4H, m), 7.13 (1H, s) |
| 31 | 0.88 (9H, s), 1.35-1.41 (2H, m), 2.28-2.42 (8H, m), 3.27-3.40 (4H, m), 3.82 (3H, s), 3.96 (1H, d, J=14.4Hz), 4.33 (2H, d, J=5.4Hz), 4.68-4.79 (1H, m), 5.80 (1H, s), 5.89 (1H, d, J=14.4Hz), 6.58-6.70 (2H, m), 6.88-6.95 (2H, m), 6.96-7.12 (3H, m), 7.65 (1H, s) |
| 32 | 0.88 (9H, s), 1.35-1.41 (2H, m), 2.00-2.18 (2H, m), 2.27-2.35 (2H, m), 2.36-2.43 (4H, m), 3.34-3.41 (4H, m), 3.44 (2H, s), 4.29 (2H, d, J=5.2Hz), 4.58-4.63 (1H, m), 6.48-6.61 (1H, m), 6.62-6.75 (1H, m), 6.88-7.03 (2H, m), 7.07-7.11 (2H, m) |
| 33 | 0.87 (9H, s), 1.35-1.41 (2H, m), 2.16 (3H, s), 2.22 (3H, s), 2.23-2.36 (2H, m), 2.40 (4H, t, J=4.7Hz), 3.35 (4H, t, J=4.7Hz), 4.28-4.30 (2H, m), 4.50 (1H, br s), 6.52 (2H, br s), 6.88 (1H, s), 6.95 (2H, s), 7.11 (1H, s) |
| 34 | 0.89 (9H, s), 1.36-1.43 (2H, m), 2.23 (3H, s), 2.31 (3H, s), 2.31-2.41 (2H, m), 2.43 (4H, t, J= 4.9Hz), 3.40 (4H, t, J=4.9Hz), 3.95 (2H, t, J=4.7Hz), 4.31 (2H, t, J=4.7Hz), 4.41-4.44 (2H, m), 4.55-4.60 (1H, m), 6.47-6.51 (1H, m), 6.70 (1H, s), 6.81-7.00 (1H, m), 7.21-7.27 (2H, m), 7.34 (1 H. s) |
| 35 | 0.89 (9H, s), 1.36-1.43 (2H, m), 2.31 (3H, s), 2.32-2.39 (2H, m), 2.44 (4H, t, J=4.9Hz), 3.40 (4H, t, J=4.9Hz), 3.94 (2H, t, J=4.4Hz), 4.31 (2H, t, J=4.4Hz), 4.42 (2H, d, J=5.4Hz), 4.62 (1H, t, J=5.2Hz), 6.64-6.73 (1H, m), 6.91 (1H, d, J=2.2Hz), 7.00-7.15 (1H, m), 7.24 (3H, d, J=3.0Hz) |
| 36 | 0.88 (9H, s), 0.89 (3H, s), 1.32-1.42 (2H, m), 1.65-2.20 (4H, m), 2.30-2.45 (7H, m), 2.49 (2H, t, J=5.2Hz), 2.80-2.90 (1H, m), 3.36 (4H, br s), 3.60 (2H, t, J=4.9Hz), 4.25-4.40 (1H, m), 6.80-7.40 (6H, m), 7.84 (1H, s) |
| 37 | 0.92 (9H, s), 1.42-1.49 (2H, m), 2.09 (3H, s), 2.48 (3H, s)2.50-2.58 (4H, m), 2.80-3.03 (2H, m), 3.13-3.33 (1H, m), 3.40-3.53 (4H, m), 4.17-4.33 (2H, m), 4.90-5.08 (1H, m), 5.67-5.78 (1H, m), 6.54-6.58 (2H, m), 6.79-6.90 (2H, m), 7.10 (2H, s), 7.68 (2H, s), 8.13 (1H, d, J=2.0Hz) |
| 38 | 0.89 (9H, s), 1.37-1.43 (2H, m), 2.13 (3H, s), 2.29 (3H, s), 2.30-2.47 (8H, m), 2.49 (3H, s), 2.76-2.88 (1H, m), 2.90-3.07 (1H, m), 3.31-3.42 (4H, m), 4.26 (2H, d, J=4.2Hz), 4.70-4.80 (1H, m), 4.96-5.08 (1H, m), 6.51 (1H, d, J=7.9Hz), 6.65 (2H, d, J=7.4Hz), 6.85 (1H, d, J=7.7Hz), 7.06 (1H, s). 7.86-7.97 (1H, m) |
| 39 | 0.87 (9H, s), 1.33-1.39 (2H, m), 2.12 (3H, s), 2.27-2.36 (2H, m), 2.36-2.43 (4H, m), 2.54 (3H, s), 2.82-2.91 (1H, m), 2.91-2.98 (1 H, m), 3.20-3.27 (1H, m), 3.31-3.35 (4H, m), 4.27 (2H, t, J=4.0Hz), 4.54 (1H, t, J=4.9Hz), 5.11-5.16 (1H, m), 6.62-6.72 (2H, m), 6.88-6.98 (2H, m), 6.99 (1H, s), 7.15-7.23 (1H, m), 7.75-7.80 (1H, m) |
| 40 | 0.82 (9H, s), 1.31-1.37 (2H, m), 2.20 (3H, s), 2.25-2.42 (6H, m), 3.32-3.42 (4H, m), 3.55 (3H, s), 3.94 (1H, d, J=14.6Hz), 4.06-4.11 (2H, m), 5.53 (1H, m), 5.77 (1H, d, J=14.6Hz), 6.49-6.57 (2H, m), 6.64-6.73 ( 2H, m), 6.80-6.94, (3H, m), 7.16 (1H, s), 7.67 (1H, s) |
| 41 | 0.07-0.13 (2H, m), 0.49-0.55 (2H, m), 0.83-0.88 (1H, m), 2.17 (3H, s), 2.28 (2H, d, J=6.4Hz), 2.33 (3H, s), 2.49-2.53 (4H, m), 2.73 (3H, s), 3.02-3.17 (2H, m), 3.36-3.41 (4H, m), 4.27-4.31 (2H, m), 4.51 (1H, s), 5.11-5.18 (1H, m), 6.55 (1H, d, J=7.9Hz), 6.67 (1H, d, J=7.0Hz), 6.77 (1H, d, J=7,0Hz), 6.86 (1H, d, J=7.9Hz), 7.15 (1H, s), 8.15 (1H, s) |
| 42 | 0.07-0.09 (2H, m), 0.46-0.54 (2H, m), 0.80-0.85, (1H, m), 2.15 (3H, s), 2.23-2.27 (2H, m), 2.45-2.49 (4H, m), 2.73 (3H, s), 3.10-3.18 (2H, m), 3.35-3.37 (4H, m), 3.48-3.68 (1H, m), 4.25-4.29 (2H, m), 4.48 (1H, m), 5.16 (1H, m), 6.67 (2H, m), 6.86 (1H, d, J=7.9Hz), 6.92-6.98 (1 H, m), 7.12 (1H, s) 7.20 (1H, m) 8.36 (1H, d, J=7.9Hz) |
| 43 | 0.88 (9H, s), 1.34-1.40 (2H, m), 2.14 (3H, s), 2.29-2.49 (6H, m), 2.72 (3H, s), 3.03-3.18 (2H, m), 3.31-3.35 (4H, m), 3.47-3.67 (1H, m), . 4.21-4.31 (2H, m), 4.51-4.54 (1H, m), 5.12-5.19 (1H, m), 6.67 (2H, m), 6.85 (1H, d, J=7.9Hz), 6.92-6.97 (1H, m), 7.06-7.22 (2H, m), 8.36 (1H, dd, J=1.2, 7.9Hz) |
| 44 | 0.85 (9H, s), 1.31-1.39 (2H, m), 1.99 (3H, s), 2.19 (3H, s), 2.24-2.40 (6H, m), 3.33-3.38 (4H, m), 3.53 (3H, s), 3.90 (1H, d, J=14.6Hz), 4.10-4.12 (2H, m), 5.56 (1H, m), 5.75 (1H, d, J=14.6Hz), 6.33-6.35 (1H, m), 6.52-6.56 (2H, m), 6.66-7.11 (3H, m), 7.14 (1H, s), 7.48 (1H, s) |
| 45 | 0.01-0.06 (2H, m), 0.42-0.49 (2H, m), 0.75-0.81 (1H, m), 1.98 (3H, s), 2.17-2.25 (5H, m), 2.38-2.48 (4H, m), 3.32-3.41 (4H, m), 3.50 (3H, s), 3.89 (1H, d, J=14.6Hz), 4.11 (2H, m), 5.54 (1H, m), 5.75 (1H, d, J=14.6Hz), 6.34 (1H, dd, J=1.0, 8.0Hz), 6.50-6.54 (2H, m), 6.66-7.07 (3H, m), 7.13 (1H, s) 7.37 (1H, s) |
| 46 | 0.88 (9H, s), 1.33-1.42 (2H, m), 2.24 (3H, s), 2.27-2.45 (6H, m), 3.35-3.43 (4H, m), 3.68 (3H, s), 3.94 (1H, d, J=14.6Hz), 4.20(2H, d, J=5.4Hz), 5.01-5.07 (1H, m), 5.79 (1H, d, J=14.6Hz), 6.54 (1H, dd, J=2.2, 8.4Hz), 6.61-7.02 (5Hm), 7.05 (1H, d, J=1.0Hz), 7.53 (1H, d, J=4.7Hz) |
| 47 | 0.01-0.06 (2H, m), 0.42-0.47 (2H, m), 0.76-0.80 (1H, m), 2.15-2.23 (5H, m), 2.41-2.46 (4H, m), 3.35-3.48 (4H, m), 3.54 (3H, s), 3.90 (1H, d, J=14.6Hz), 4.11 (2H, m), 5.46 (1H, m), 5.72 (1H, d, J=14.6Hz), 6.46 (1H, dd, J=1.5, 8.5Hz), 6.54-6.61 (2H, m), 6.67-6.73 (1H, m), 6.81-7.09 (2H, m), 7.13 (1H, s), 7.99 (1H, s) |
| 48 | 0.84 (9H, s), 1.31-1.37 (2H, m), 1.99 (3H, s), 2.24-2.33 (6H, m), 3.34 (4H, m), 3.57 (3H, s), 3.90 (1H, d, J=14.6Hz), 4.08 (2H, m), 5.70 (1H, d, J=14.6Hz), 6.01 (1Hm), 6.34 (1H, m), 6.53-6.60 (2H, m), 6.70 (1H, s), 6.82-6.91 (2H, m), 7.10 (1H, s), 7.49 (1H, s) |
| 49 | 0.02-0.08 (2H, m), 0.44-0.51 (2H, m), 0.77-0.82 (1H, m), 2.02 (3H, s), 2.16-2.24 (2H, m), 2.42-2.44 (4H, m), 3.36-3.42 (4H, m), 3.57 (3H, s), 3.92 (1H, d, J=14.6Hz), 4.11 (2H, m), 5.72 (1H, d, J=14.6Hz), 5.86 (1H, m), 6.36 (1H, dd, J=1.0, 8.0Hz), 6.54-6.89 (5H, m), 7.12 (1H.,s), 7.27 (1H, s) |
| 50 | 0.87 (9H, s), 1.33-1.40 (2H, m), 2.27-2.40 (6H, m), 3.34-3.42 (4H, m), 3.60 (3H, s), 3.94 (1H, d, J=14.6Hz), 4.16 (2H, d, J=5.2Hz), 5.72 (1H, d, J=14.6Hz), 5.81 (1H, t, J=5.4, 11.1 Hz), 6.50 (1H, dd, J=2.2, 8.4Hz). 6.60-6.73 (3H, m), 6.91-7.02 (3H, m), 8.00 (1H, s) |
| 51 | 0.02-0.08 (2H, m), 0.44-0.48 (2H, m), 0.77-0.81 (1H, m), 2.20-2.24, (2H, m), 2.43 (4H, m), 3.37-3.47 (4H, m), 3.57 (3H, s), 3.93 (1H, d, J=14.6Hz), 4.14 (2H, m), 5.70 (1H, d, J=14.6Hz), 5.82 (1H, m), 6.48 (1 H, dd, J=2.0, 8.5Hz), 6.58-6.72 (3H, m), 6.89-7.00 (2H, m), 7.14 (1H, s), 7.92 (1H, s) |
| 52 | 0.89 (9H, s), 1.34-1.42 (2H, m), 2.29-2.46 (6H, m), 3.35-3.44 (4H, m), 3.66 (3H, s), 3.98 (1H, d, J=14.6Hz), 4.21 (2H, d, J=5.4Hz), 5.14-5.24 (1H, m), 5.76 (1H, d, J=14.6Hz), 6.58 (1H, dd, J=2.2, 8.4Hz), 6.74-7.00 (4H, m), 7.07 (1H, s), 7.22 (1H, s) |
| 53 | 0.02-0.07 (2H, m), 0.43-0.48 (2H, m), 0.77-0.81 (1H, m), 2.19-2.24 (2H, m), 2.42-2.47 (4H, m), 3.37-3.41 (4H, m), 3.56 (3H, s), 3.95 (1H, d, J=14.6Hz), 4.10 (2H, m), 5.68 (1H, d, J=14.6Hz), 5.89 (1H, m), 6.49 (1H, dd, J=2.0, 8.5Hz), 6.68-6.96 (5H, m), 7.14 (1H, s), 7.93 (1H, s) |
| 54 | 0.82 (9H, s), 1.33-1.41 (2H, m), 1.99 (3H, s), 2.28-2.38 (6H, m), 3.36-3.37 (4H, m), 3.60 (3H, s), 3.95 (1H, d, J=14.6Hz), 4.07 (2H, m), 5.69 (1H, d, J=14.6Hz), 6.06 (1H, m), 6.37 (1H, m), 6.61-6.82 (3H, m), 6.94 (2H, m), 7.14 (1H, s), 7.46 (1H, s) |
| 55 | 0.02-0.07 (2H, m), 0.43-0.50 (2H, m), 0.76-0.81 (1H, m), 1.97 (3H, s), 2.16-2.23 (2H, m), 2.41-2.45 (4H, m), 3.36-3.44, (4H, m), 3.54 (3H, s), 3.93 (1H, d, J=14.6Hz), 4.05 (2H, m), 5.68 (1H, d, J=14.6Hz), 6.00 (1H, m), 6.34 (1H, m), 6.58-6.79 (4H, m), 6.90 (1H, s), 7.11(1H, s), 7.35(1H, s) |

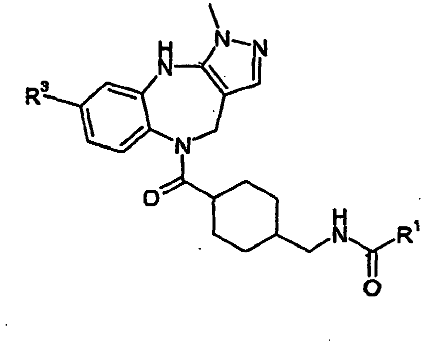

| **Compound number** | **R³** | **R¹** | **¹H NMR: δ (ppm)** | **MS** |
|---|---|---|---|---|
| 109 E10 | Cl | 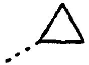 | 0.42-0.90 (6H, m), 1.28 (6H, m), 1.78-1.80 (2H, m), 2.08 (1H, m), 2.95-3.01 (2H, m), 3.66 (3H, s), 3.76 (1H,d, J=14.6Hz), 5.55 (1H, d, J=14.6Hz), 5.95 (1H, m), 6.97 (1H, dd, J=8.4, 2.1 Hz), 7.03 (1H, s), 7.07 (1 H, d, J=8.4Hz), 7.09 (1H, d, J=2.1Hz), 7.14 (1H, s) | (ESI)+:[M+H]+=4 42.3, 444.3 |
| 110 | Me | 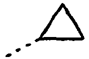 | 0.58-0.90 (6H, m), 1.23-2.13 (9H, m), 2.33 (3H, s), 2.95-3.03 (2H, m), 3.68 (3H, s), 3.76 (1H, d, J=14.6Hz), 5.59 (1H, d, J=14.6Hz), 5.80 (1H, m), 6.15 (1H, s), 6.81-6.83 (2H, m), 6.97-7.00 (1H, m), 7.14 (1H, s) | (ESI)+:[M+H]+=4 22.4 |
| 111 | H | 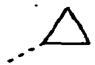 | 0.58-0.81 (6H, m), 1.31-1.74 (6H, m), 1.69-1.80 (2H, m), 2.09-2.16 (1H, m), 2.89-2.98 (2H, m), 3.64 (3H, s), 3.80 (1H, d, J=14.6Hz), 5.58 (1H, d, J=14.6Hz), 6.49 (1 H, m), 6.95-7.23 (5H, m), 7.39 (1H, s) | (APCI)+: [M+H]+= 408.3 [M+H]+= 408.3 |
| 112 | Me | CH₃ | | |
| 113 | Me | 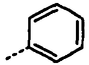 | | (ESI)+:[M+H]+=4 58.7 |
| 114 | Me | 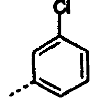 | | (ESI)+:[M+H]+=4 92.2, 494.2 |
| 115 | Me | 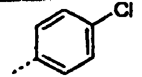 | | (ESI)+:[M+H]+=4 92.6, 494.2 |
| 116 | Me | 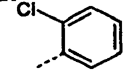 | | (ESI)+:[M+H]+=4 92.2. 494.2 |
| 117 | Me | 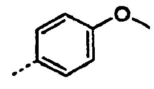 | | (ESI)+:[M+H]+=4 88.7 |
| 118 | Me | 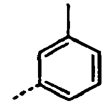 | | (ESI)+:[M+H]+=4 72.7 |
| 119 | Me | 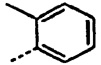 | | (ESI)+:[M+H]+=4 72.7 |
| 120 | Me | 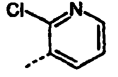 | | (ESI)+:[M+H]+=4 93.2. |
| 121 | Me | 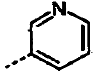 | | (ESI)+:[M+H]+=4 59.2 |
| 122 | Me | 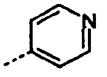 | | (ESI)+:[M+H]+=4 59.2 |
| 123 | Me | 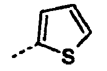 | | (ESI)+:[M+H]+=4 64.6 |
| 124 | Me | 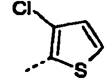 | | (ESI)+:[M+H]+=4 98.1 |
| 125 | Me | 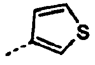 | | (ESI)+:[M+H)+=4 64.2 |
| 126 | Me | 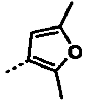 | | (ESI)+:[M+H]+=4 76.6 |
| 127 | Me | 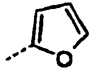 | | (ESI)+:[M+H]+=4 48.6 |
| 128 | Me | 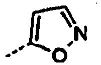 | | (ESI+:[M+H]+=4 49.6 |
| 129 | Me | 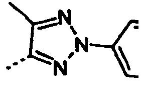 | | (ESI)+:[M+H]+=5 39.3 |
| 130 | Me | CH₂CH₃ | | (ESI)+:[M+H]+=4 10.7 |
| 131 | Me | CH₂CH₂CH₃ | | (ESI)+:[M+H]+=4 24.7 |
| 132 | Me | C(CH₃)₃ | | (ESI)+:[M+H]+=4 38.3 |
| 133 | Me | 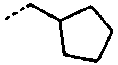 | | (ESI)+:[M+H]+=4 64.7 |
| 134 | Me | CH₂(CH₂)₃C H₃ | | (ESI)+:[M+H]+=4 52.8 |
| 135 | Me | CH(CH₂CH₃) CH₂CH₂CH₂ CH₃ | | (ESI)+:[M+H]+= (ESI)+:[M+H]+=4 80.8 |
| 136 | Me | 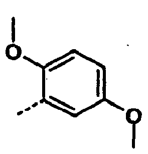 | | (ESI)+:[M+H]+=6 32.3 |
| 137 | Me | 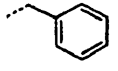 | | (ESI)+:[M+H]+=4 72.7 |
| 138 | Me | 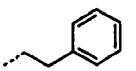 | | (ESI)+:[M+H]+=4 86.7 |
| 139 | Me | 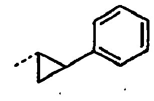 | | (ESI)+:[M+H]+=4 98.7 |
| 140 | Me | 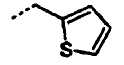 | | (ESI)+:[M+H]+=4 78.6 |
| 141 | Me | 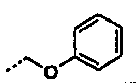 | | (ESI)+:[M+H]+=4 88.7 |
| 142 | Me | 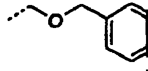 | | (ESI)+:[M+H]+=5 02.7 |
| 143 | Me | CH₂CO₂CH₂ CH₃ | | (ESI)+:[M+H]+=4 68.2 |
| 144 | Me | CH₂CH₂CO₂ CH₃ | | (ESI)+:[M+H]+=4 68.2 |
| 145 | Me | CH₂(CH₂)₂C O₂CH₂CH₃ | | (ESI)+:[M+H]+=4 96.7 |

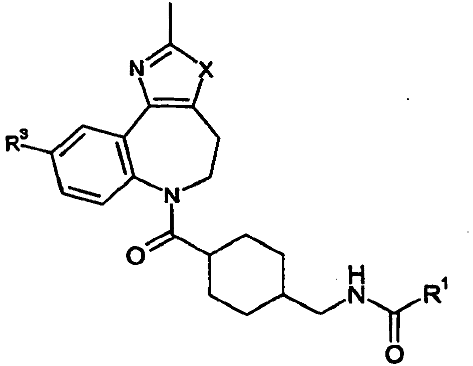

| **Compound number** | **R¹** | **R³** | **X** | **MS (ESI)+: [M+H] +=** | **¹H NMR: δ (ppm)** |
|---|---|---|---|---|---|
| 146 | 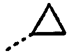 | Cl | S | 458.4, 460.4 | 0.51-0.98 (6H, m), 1.11-1.99 (7H, m), 2.30-2.41 (1H, m), 2.67 (3H, s), 2.83-3.08 (4H, m), 3.42-3.62 (1H, m), 5.55 (1H, s), 7.10 (1H, d, J=8.4Hz), 7.25 (1 H, d d, J=2.5, 8.4Hz), 8.38 (1H, d, J=2.5Hz) |
| 147 | 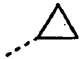 | Me | S | 438.2 | 0.55-0.62 (1H, m), 0.62-0.72 (2H, m), 0.72-0.85 (1H, m), 0.85-1.00 (2H, m), 1.10-1.45 (4H, m), 1.45-1.95 (4H, m), 2.35-2.50 (1H, m), 2.43 (3H, s), 2.68 (3H, s), 2.85-3.10 (4H, m), 3.45-3.65 (1H, m), 4.75-4.85 (1H, m), 5.45-5.65 (1H, m), 7.00-7.15 (2H, m), 8.14 (1H, s) |

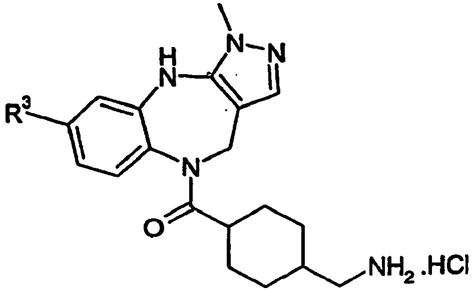

| **Compound number** | **R³** | **MS (ESI)+:[M+H]+=** |
|---|---|---|
| 148 E10.2 | Cl | |
| 149 | Me | 354.2 |
| 150 | H | 340.2 |

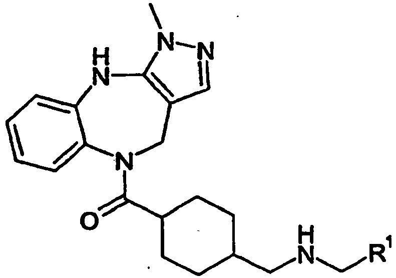

| **Compound number** | **R⁸** | **MS (ESI)+:[M+H]+=** |
|---|---|---|
| 151 E12 | CH (CH₃)₂ | 396.4 |
| 152 | CH₂CH₃ | 396.4 |
| 153 | 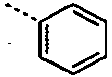 | 430.4 |
| 154 | 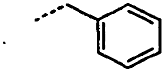 | 444.4 |
| 155 | 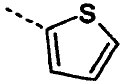 | 436.1 |
| 156 | 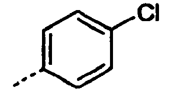 | 464.2, 466.2 |
| 157 | 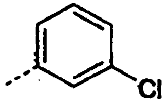 | 464.3, 66.3 |
| 158 | CH₂CH₂CH₂CH₃ | 410.4 |
| 159 | 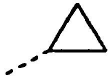 | 394.4 |
| 160 | 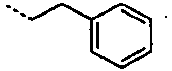 | 458.4 |
| 161 | CH₂CH(CH₃)₂ | 410.4 |
| 162 | 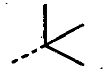 | 410.3 |
| 163 | 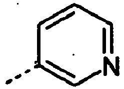 | 431.1 |
| 164 | 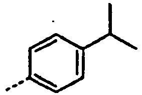 | 472.4 |
| 165 | 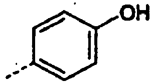 | 446.4 |
| 166 | 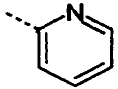 | 431.4 |
| 167 | 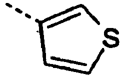 | 436.3 |
| 168 | CH₂CF₃ | 436.3 |
| 169 | 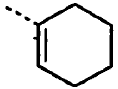 | 434.4 |
| 170 | 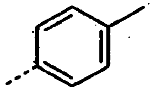 | 444.4 |
| 171 | 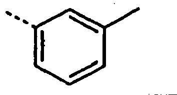 | 444.3 |
| 172 | 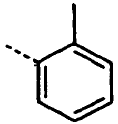 | 444.2 |
| 173 | 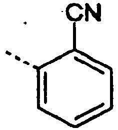 | 454.2 |
| 174 | 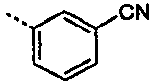 | 454.4 |
| 175 | 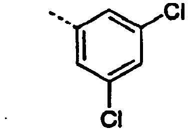 | 498.3 |
| 176 | 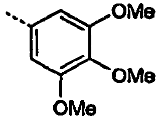 | 520.4 |
| 177 | 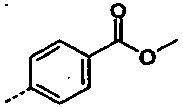 | 488.4 |

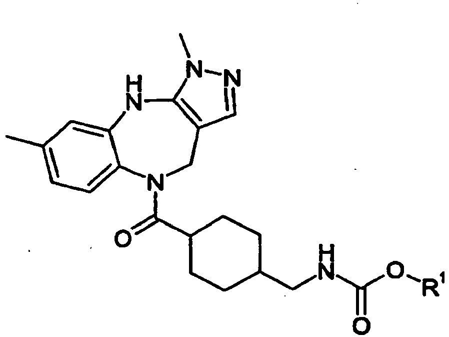

| **Compound number** | **R¹** | **MS (ESI)+: [M+H]+=** |
|---|---|---|
| 178 E13 | 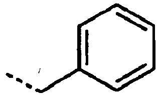 | 533.3 |
| 179 | 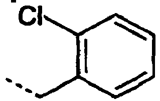 | 522.2, 524.2 |
| 180 | 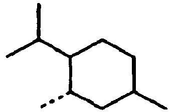 | 536.4 |
| 181 | 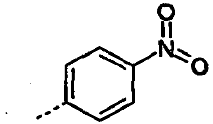 | 519.7 |
| 182 | 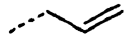 | 438.2 |
| 183 | 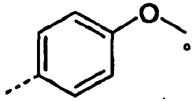 | 504.7 |
| 184 | 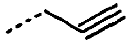 | 436.7 |
| 185 | 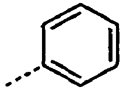 | 474.7 |
| 186 | 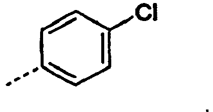 | 508.6 |
| 187 | 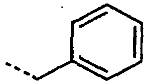 | 488.7 |
| 188 | 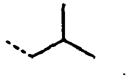 | 454.7 |
| 189 | 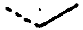 | 426.2 |
| 190 | 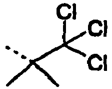 | 556.2, 558.2 |
| 191 | CH₃ | 412.2 |
| 192 | 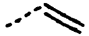 | 424.2 |
| 193 | 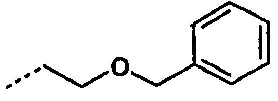 | 532.3 |

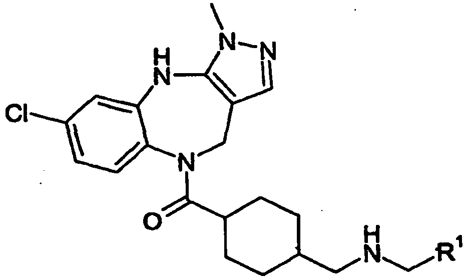

| **Compound number** | **R¹** | **MS (ESI)+: [M+H)+=** |
|---|---|---|
| 194 E14 | CH₂CH₂CH₂CH₂CH₃ | 458.4 |
| 195 | CH₂CH₃ | 416.4, 418.4 |
| 196 | (CH₂)₂CN | 441.5, 443.5 |
| 197 | 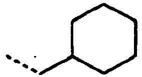 | 484.4, 486.4 |
| 198 | 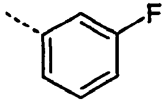 | 482.4,484.4 |
| 199 | 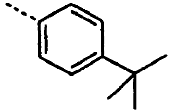 | 520.6, 522.6 |
| 200 | 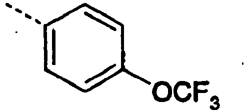 | 548.5, 550.5 |
| 201 | 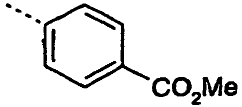 | 522.6, 524.6 |
| 202 | 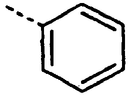 | 464.5, 466.5 |
| 203 | 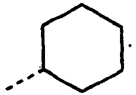 | 470.5, 472.5 |
| 204 | CH₂OH | 418.3, 420.3 |
| 205 | CO₂CH(CH₃)₂ | 474.4, 476.4 |
| 206 | 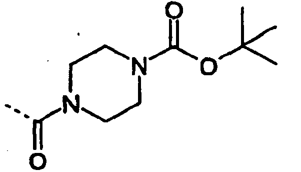 | 600.6, 602.6 |
| 207 | CH₂CH(CH₃)₂ | 444.5, 446.5 |
| 208 | CH₂CH₂OH | 432.5 |
| 209 | CN | 413.4, 415.5 |
| 210 | 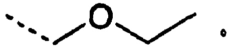 | 446.5, 448.5 |
| 211 | 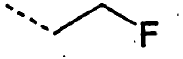 | 434.3, 436.3 |
| 212 | 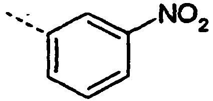 | 509.5, 511.5 |
| 213 | 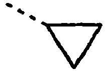 | 428.4, 430.4 |
| 214 | 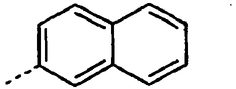 | 514.6, 516.6 |
| 215 | 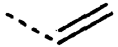 | 414.4, 416.5 |
| 216 | 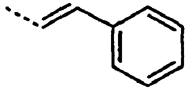 | 490.6, 492.6 |
| 217 | 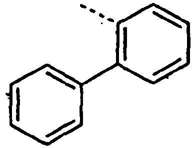 | 540.6, 542.6 |
| 218 | 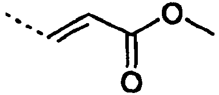 | 472.3, 474.4 |
| 219 | 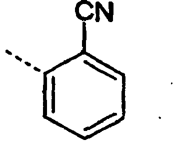 | 489.4, 491.4 |
| 220 | 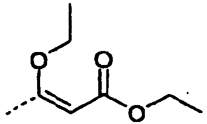 | 530.5, 532.5 |
| 221 | COC(CH₃)₃ | 472.5, 474.5 |
| 222 | CONH₂ | 431.5, 433.5 |
| 223 | 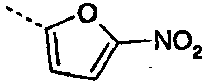 | 499.5, 501.5 |
| 224 | 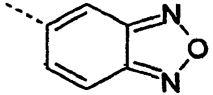 | 506.6, 508.6 |

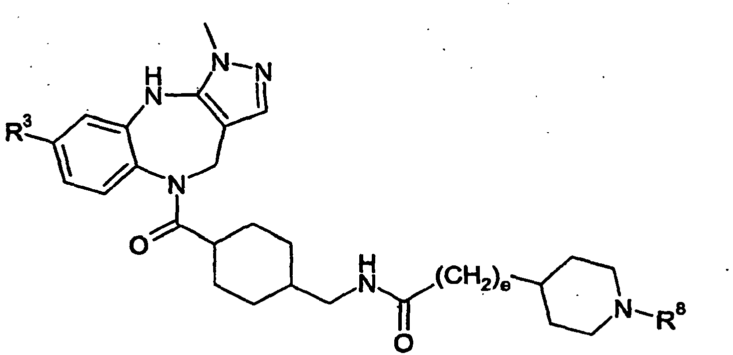

| **Compound number** | **R³** | **e** | **R⁸** | **MS** | **¹H NMR: δ (ppm)** |
|---|---|---|---|---|---|
| 225 E16 | H | 0 | CH₂CH₂C(CH₃)₃ | (ESI)+:[ M+H]+= 535.5 | 0.45-0.80 (2H, m), 0.88 (9H, s), 1.20-1.62 (7H, m), 1.62-2.19 (10H, m), 2.33-2.46 (2H, m), 3.72 (3H, s), 3.79 (1 H, d, J=14.6Hz), 5.61 (1H, d, J=14.6Hz), 5.63-5.70 (1H, m), 6.10 (1H, s), 7.02(2H, t, J=8.4Hz), 7.10-7.19 (3H, m), 7.26-7.28 (2H, m) |
| 226 | Cl | 2 | CO₂C(CH₃)₃ | (ESI)+:[ [M+H]+= 613.4, 615.4 | 0.43-0.62 (1H, m), 0.64-0.83 (1H, m), 0.91-1.14 (2H, m). 1.22-1.81 (11H, m), 1.44 (9H, s), 1.98-2.07 (2H, m), 2.16 (2H, t, J=7.7Hz), 2.61 (2H, brs), 2.98 (2H, d, J=5.4Hz), 3.70 (3H, s), 3.75 (1H, d, J=14.6Hz), 4.02 (2H, d, J=12.4Hz), 5.55 (1H, d, J=14.6Hz), 5.95 (1 H, t, J=5.2Hz), 6.96 (1H, dd, J=2.0, 8.4Hz), 7.04 (1 H, d, J=8.4Hz), 7.09-7.13 (2H, m), 7.35 (1H, s) |
| 227 | H | 0 | CO₂C(CH₃)₃ | - | d4-MeOH δ 0.46-0.80 (2H, m), 1.13-1.84 (11H, m), 1.44 (9H, s), 2.13-2.37 (2H, m), 2.74 (2H, brs), 2.89 (2H, t, J=6.2Hz), 3.72 (3H, s), 3.80 (1H, d, J=14.6Hz), 4.07 (2H, d, J=13.4Hz), 5.47 (1H, d, J=14.6Hz), 7.01-7.09 (1H, m), 7.14 (1H, s), 7.16-7.22 (1H, m), 7.26-7.37 (2H, m), 7.82-7.92 (2H, m) |
| 228 | M e | 1 | CO₂C(CH₃)₃ | | 0.48-0.61 (1H, m), 0.63-1.81 (1H, m), 0.92-1.14 (2H, m), 1.16-1.30 (2H, m), 1.42 (9H, s), 1.50-2.21 (10H, m), 2.31 (3H, s), 2.53-2.72 (2H, m), 2.95 (2H, t, J=5.9Hz), 3.31-3.53 (1H, m), 3.67 (3H, s), 3.75 (1H, d, J=14.6Hz), 3.98-4.03 (2H, m), 5.53 (1H, d, J=14.6Hz), 6.10 (1 H, brt), 6.71 (1H, s), 6.80 (1H, d, J=7.9Hz), 6.86 (1H, s), 6.97 (1H, d, J=7.9Hz), 7.10 (1H, s) |
| 229 | H | 0 | 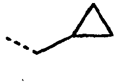 | (ESI)+:[ M+H]+= 505.5 | 0.19 (2H, d, J=4.7Hz), 0.41-0.80 (4H, m), 0.80-0.99 (2H, m), 1.06-1.60 (6H, m), 1.61-1.93 (4H, m), 2.18-2.30 (2H, m), 2.30-2.45 (2H, m), 2.48 (2H, d, J=6.7Hz), 2.91 (2H, t, J=6.4Hz), 3.16-3.30 (2H, m). 3.68 (3H, s), 3.78 (1H, d, J=14.6Hz), 5.54 (1H, d, J=14.6Hz), 6.42-6.74 (1H, m), 6.95-7.18 (4H, m), 7.20-7.30 (2H, m) |
| 230 | Cl | 0 | CH₂CH₂C(CH₃)₃ | (APCI)+ [M+H]+ =569.5, 571.5 | 0.42-0.82 (2H, m), 0.86 (9H, s), 1.19-2.14 (17H, m), 2.28-2.34 (2H, m), 2.86-3.00 (4H, m), 3.65 (3H, s), 3.76 (1 H, d, J=14.6Hz), 5.52 (1H, d, J=14.6Hz), 6.01-6.03 (1H, m), 6.94 (1H, dd, J=2.2, 8.4Hz), 7.03 (1H, d, J=8.4Hz), 7.10-7.11 (2H, m), 7.72 (1H, s) |
| 231 | Cl | 0 | 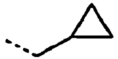 | (APCI)+ :[M+H]+ =539.4, 541.4 | d₄-MeOH: 0.37-0.43 (2H, m), 0.57-0.80 (4H, m), 1.05-2.01 (12H, m), 2.15-2.24 (1H, m), 2.44-2.52 (1 H, m), 2.91-3.04 (6H, m), 3.81-3.69 (2H, m), 3.71 (3H, s), 3.80 (1H, d, J=14.6Hz), 5.43 (1H, d, J=14.6Hz), 7.03 (1H. dd, J=2.2, 8.4Hz), 7.15 (1H, s), 7.20 (1 H, d, J=8.4Hz), 7.33 (1H, d, J=2.2Hz) |
| 232 | M e | 0 | CH₂CH₂C(CH₃)₃ | (APCI)+ :[M+H]+ =549.5 | 0.51-0.82 (2H, m), 0.86 (9H, s), 1.12-1.84 (13H, m), 1.91-2.11 (4H, m), 2.20-2.36 (5H, m), 2.94-3.02 (4H, m), 3.66 (3H, s), 3.75 (1H, d, J=14.6Hz), 5.55 (1H, d, J=14.6Hz), 6.53 (1H, s), 6.80 (1H, d, J=7.9Hz), 6.85 (1H, s), 6.97 (1H, d, J=7.9Hz), 7.12 (1H, s) |
| 233 | M e | 0 | 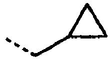 | (APCI)+ :[M+H]+ =519.4 | d₄-MeOH: 0.39-0.76 (6H, m), 1.05-2.02 (16H, m), 2.20-2.26 (1H, m), 2.34 (3H, s), 2.44-2.50 (1H, m), 2.88-3.00 (4H, m), 3.69 (3H, s), 3.75 (1H, d, J=14.6Hz), 5.43 (1H, d, J=14.6Hz), 6.86-6.89 (1H, m), 7.03-7.13 (3H, m), 7.79 (1H, t, J=5.9Hz), 7.85 (1H, s) |

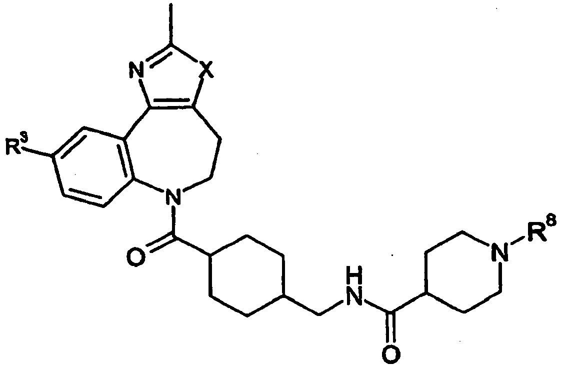

| **Compound number** | **R³** | **R⁸** | **X** | **MS** | **¹H NMR: δ (ppm)** |
|---|---|---|---|---|---|
| 234 | Cl | CH₂CH₂C(CH₃)₃ | S | 585.5, 587.6 | 0.50-0.56 (1H, m), 0.88 (9H, s), 1.11-1.19 (2H, m), 1.35-1.49 (4H, m), 1.56-2.10 (9H, m), 2.28-2.34 (3H, m), 2.67 (3H, s), 2.83-3.12 (6H, m), 3.42-3.60 (2H, m), 4.76-4.83 (1H, m), 5.39-5.44 (1H, m), 7.10 (1H, d, J=8.4Hz), 7.26 (1H, dd, J=2.5, 8.4Hz), 8.38 (1H, d, J=2.5Hz) |
| 235 | Me | CH₂cH₂C(CH₃)₃ | S | 565.4 | 0.42-0.62 (1H, m), 0.72-0.84 (1H, m), 0.87 (9H. s), 1.08-1.20 (2H, m), 1.30-1.50 (4H, m), 1.56-1.75 (6H, m), 1.75-1.95 (5H, m), 1.96-2.10 (1 H, m), 2.25-2.35 (1 H, m), 2.43 (3H, s), 2.69 (3H, s), 2.85-3.5 (6H, m), 3.40-3.65 (1H, m), 4.75-4.85 (1H, m), 5.35-5.45 (1H, m), 7.00-7.15 (2H, m), 8.15 (1H, s), |

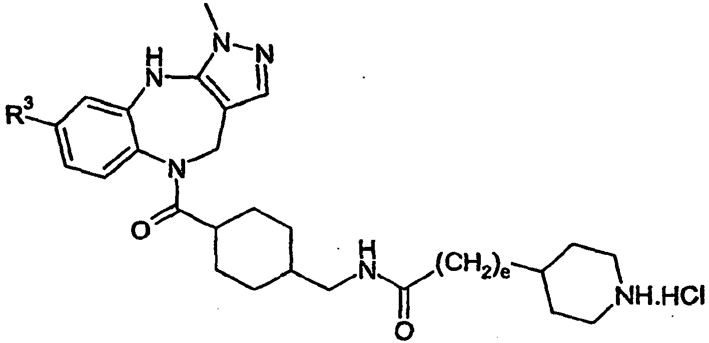

| **Compound number** | **R³** | **e** | **MS** | **¹H NMR: δ (ppm)** |
|---|---|---|---|---|
| 236 E17 | Me | 1 | (APCI) +:[M+ H]+= 579.5 | 0.44-0.68 (1H, m), 0.69-0.87 (1H, m), 1.19-2.32 (15H, m), 2.40 (3H, s), 2.88-3.03 (4H, m), 3.28-3.43 (4H, m), 3.52-3.75 (1H, m), 3.84 (1H, d, J=14.6Hz), 3.96 (3H, s), 5.50 (1H, d, J=14.6Hz), 7.07 (1H, d, J=7.9Hz), 7.22 (1H, d, J=7.9Hz), 7.28 (1H, s), 7.96 (1H, s) |
| 237 | Cl | 2 | (ESI)+ [M+H] +=513 .4, 515.4 | CD₃OD δ 0.54-0.70 (1H, m), 0.72-0.89 (1H, m), 1.12-1.98 (14H, m), 2.20-2.29 (3H, m), 2.89-2.98 (4H, m), 3.30-3.39 (2H, m), 3.87 (1H, d, J=14.6Hz), 3.98 (3H, s), 5.51 (1H, d, J=14.6Hz), 7.24 (1H, dd, J=2.2, 8.4Hz), 7.37 (1H, d, J=8.4Hz), 7.56 (1H, d, J=2.2Hz), 7.91 (1H, s), 8.00 (1H, s) |
| 238 | H | 0 | (ESI)+ [M+H]+= 451.3 | CD₃OD δ 0.44-0.63 (1H, m), 0.66-0.83 (1H, m), 1.10-2.03 (11H, m), 2.21-2.31 (1H, m), 2.47-2.61 (1H, m), 2.89-2.92 (2H, m), 2.98-3.14 (2H, m), 3.34-3.42 (2H, m), 3.87 (1H, d, J=14.6Hz), 3.99 (3H, s), 5.53 (1H, d, J=14.6Hz), 7.24 (1H, dd, J=2.0, 8.4Hz), 7.32 (1H, d, J=8.4Hz), 7.38-7.53 (2H, m), 7.98 (1H, s) |

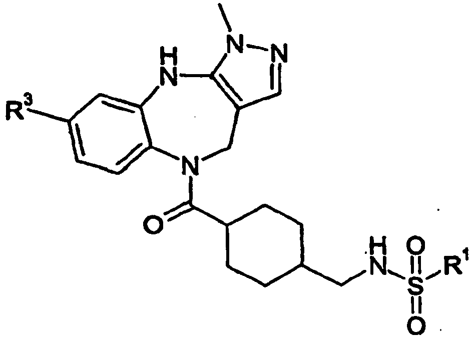

| **Compound number** | **R¹** | **R³** | **MS (ESI)+: [M+H]+=** | **¹H NMR: δ (ppm)** |
|---|---|---|---|---|
| 239 E18 | Me | Me | 432.1 | |
| 240 | CH(CH₃)₂ | Me | 460.2 | |
| 241 | 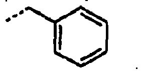 | Me | 508.2 | |
| 242 | 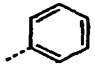 | Me | (494.2 | |
| 243 | 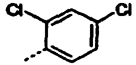 | Me | 562.2 | |
| 244 | 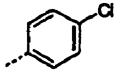 | Me | 528.2, 530.2 | |
| 245 | 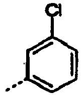 | Me | 528.2, 530.2 | |
| 246 | 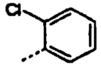 | Me | 528.2, 530.2 | |
| 247 | 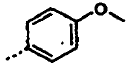 | Me | 524.2 | |
| 248 | 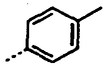 | Me | 508.7 | 0.50-0.80 (2H, m), 1.20-1.80 (7H, m), 2.05-2.20 (1H, m), 2.35 (3H, s), 2.40 (3H, s), 2.67 (2H, t, J=6.4Hz). 3.71 (3H, s), 3.75 (1H, d, J=14.5Hz), 4.29 (1H, t, J=6.5Hz), 6.59 (1H, d, J=14.5Hz), 5.71 (1H, s), 6.79 (1H, s), 6.79-6.89 (1 H, m), 6.99 (1H, d, J=7.9Hz), 7.23-7.31 (2H, m), 7.66 (2H, d, J=8.2Hz) |
| 249 | 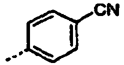 | Me | 519.7 | |
| 250 | 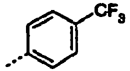 | Me | 562.2 | |
| 251 | 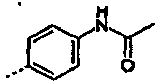 | Me | 551.3 | |
| 252 | 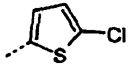 | Me | 534.1, 536.1 | |
| 253 | 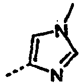 | Me | 498.2 | |
| 254 | CH₂CF₃ | Me | 500.2 | |
| 255 | 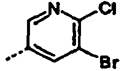 | Me | 607.1, 609.1 | |
| 256 | 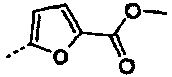 | Me | 542.2 | |
| 257 | CH₂CH₃ | Me | 446.1 | 0.55-0.90 (2H, m), 1.20-1.90 (10H, m) 2.08-2.20 (1H, m), 2.36 (3H, s), 2.86 (2H, tJ=6.0Hz)2.95-3.15 (2H, m), 3.71 (3H, s), 3.75 (1H, d, J=14.6Hz), 4.12-4.16 (1H, m), 5.60 (1H, d, J=14.6Hz), 5.77 (1H, s), 6.80 (1H, s), 6.83 (1H, d, J=7.9Hz), 7.01 (1H, d, J=7.9Hz), 7.17 (1H, s) |
| 258 | CH₂CH₂CH₃ | Me | 460.6 | |
| 259 | CH₂CH₂CH₂CH₃ | Me | 474.2 | |
| 260 | CH₂CH₂CH₂CH₃ | Cl | 494.2, 496.2 | 0.55-0.85 (2H, m), 0.92 (3H, t J=7.3Hz), 1.25-1.85 (11H, m), 2.05-2.20 (1H, m), 2.80-3.00 (4H, m), 3.72 (3H, s), 3.76 (1H, d, J=14.6Hz), 4.10-4.20 (1H, m), 5.59 (1H, d, J=14.6Hz), 5.91 (1H, s), 7.00-7.10 (3H, m), 7.19(1H, s) |
| 261 | 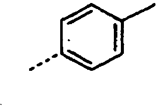 | Cl | 528.1, 530.1 | 0.50-0.80 (2H, m), 1.20-1.80 (7H, m), 2.00-2.15 (1H, m), 2.40 (3H, s), 2.60-2.75 (2H, m), 3.71 (3H, s), 3.75 (1H, d, J=14.6Hz), 4.64 (1H, t, J=6.5Hz), 5.57 (1H, d, J=14.6Hz), 6.29 (1 H, s), 6.95-7.10 (3H, m), 7.16 (1H, s), 7.25-7.35 (2H, m), 7.71 (2H, d, J=8.2Hz) |
| 262 | 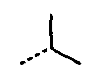 | H | 446.2 | 0.50-0.80 (1 H, m), 1.30 (6H, d, J=6.7Hz), 1.32-1.48 (4H, m), 1.50-1.90 (5H, m), 2.10-2.20 (1H, m)2.75-3.00 (2H, m), 3.72 (3H, s), 3.81 (1H, d, J=14.6Hz), 4.25-4.45 (1H, m), 5.62 (1H, d, J=14.6Hz), 6.21 (1H, s), 6.95-7.10 (2H, m), 7.10-7.20 (2H, m), 7.25-7.30 (1H, m) |
| 263 | 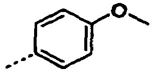 | H | 510.1 | 0.45-0.75 (1H, m), 1.20-1.85 (8H, m), 2.00-2.15 (1H,m), 2.60-2.70 (2H, m), 3.73 (3H, s), 3.79 (1H, d, J=14.6Hz), 3.84 (3H, s), 4.30-4.40 (1H, m), 5.60 (1H, d, J=14.6Hz), 5.90 (1H, s), 6.90-7.10 (4H, m), 7.10-7.20 (2H, m), 7.25-7.30 (1H, m), 7.70-7.80 (2H, m) |

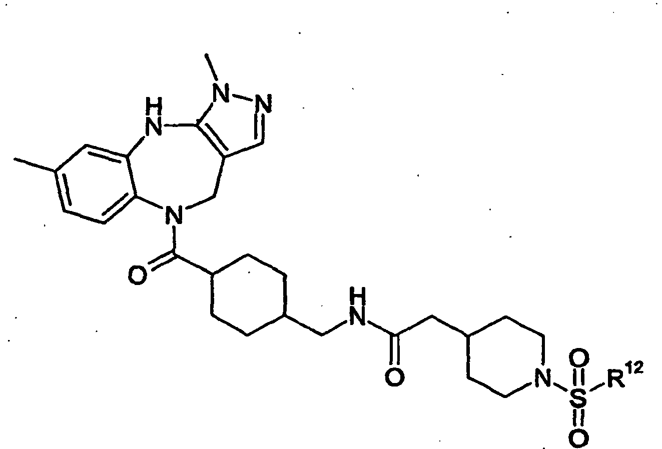

| **Compound number** | **R¹²** | **MS** |
|---|---|---|
| 264 E19 | Me | (ESI)+: [M+H]+= 557.4 |
| 265 | CH(CH₃)₂ | (ESI)+: [M+H]+= 585.4 |
| 266 | 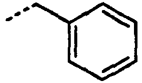 | (ESI)+: [M+H]+= 633.6 |
| 267 | 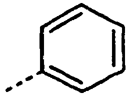 | (ESI)+: [M+H]+= 619.5 |
| 268 | 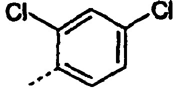 | (ESI)+: [M+H]+= 687.4, 689.4 |
| 269 | 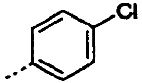 | (ESI)+: [M+H]+= 653.4, 655.4 |
| 270 | 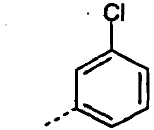 | (ESI)+: [M+H]+= 653.5, 655.5 |
| 271 | 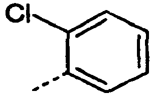 | (ESI)+: [M+H]+= 653.5, 655.5 |
| 272 | 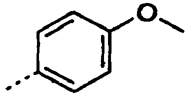 | (ESI)+: [M+H]+= 649.6 |
| 273 | 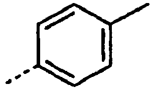 | (ESI)+: [M+H]+= 633.5 |
| 274 | 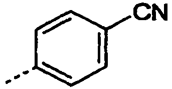 | (ESI)+: [M+H]+= 644.5 |
| 275 | 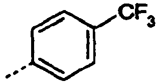 | (ESI)+: [M+H]+= 687.5 |
| 276 | 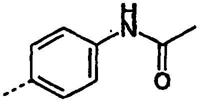 | (ESI)+: [M+H]+= 676.5 |
| 277 | 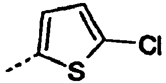 | (ESI)+: [M+H]+= 659.4, 661.4 |
| 278 | 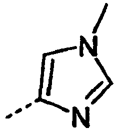 | (ESI)+: [M+H]+= 623.5 |
| 279 | CH₂CF₃ | (ESI)+: [M+H]+= 625.5 |
| 280 | 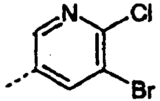 | (ESI)+: [M+H]+= 732.4, 734.4 |
| 281 | 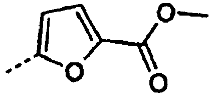 | (ESI)+: [M+H]+= 667.5 |
| 282 | CH₂CH₃ | (ESI)+: [M+H]+= 571.4 |
| 283 | CH₂CH₂CH₃ | (ESI)+: [M+H]+= 585.5 |
| 284 | CH₂CH₂CH₂CH₃ | (ESI)+: [M+H]+= 599.5 |

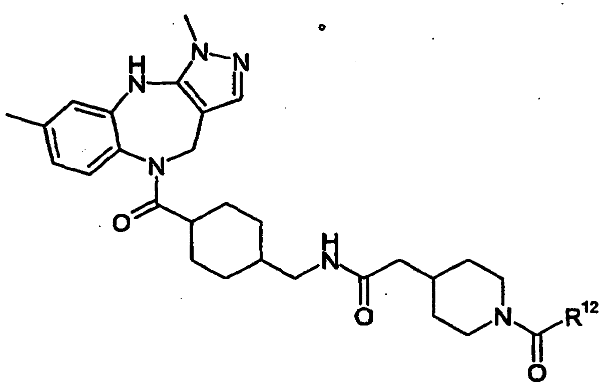

| **Compound number** | **R¹²** | **MS** |
|---|---|---|
| 285 E20 | Me | (ESI)+: [M+H]+= 521.5 |
| 286 | 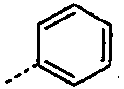 | (ESI)+: [M+H]+= 583.4 |
| 287 | 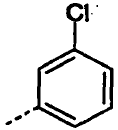 | (ESI)+: [M+H]+= 617.5, 619.5 |
| 288 | 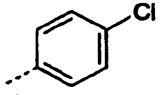 | (ESI)+: [M+H]+= 617.5, 619.5 |
| 289 | 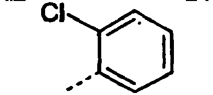 | (ESI)+: [M+H]+= 617.3, 619.3 |
| 290 | 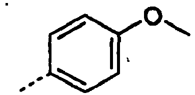 | (ESI)+: [M+H]+= 613.5 |
| 291 | 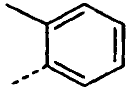 | (ESI)+: [M+H]+= 597.5 |
| 292 | 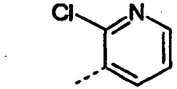 | (ESI)+: [M+H]+= 618.5, 620.5 |
| 293 | 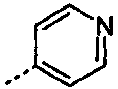 | (ESI)+: [M+H]+= 584.4 |
| 294 | 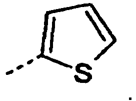 | (ESI)+: [M+H]+= 589.3 |
| 295 | 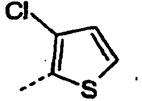 | (ESI)+: [M+H]+= 623.5, 625.5 |
| 296 | 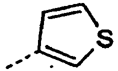 | (ESI)+: [M+H]+= 589.2 |
| 297 | 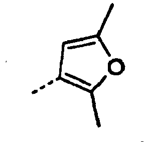 | (ESI)+: [M+H]+= 601.5 |
| 298 | 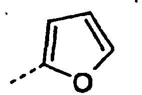 | (ESI)+: [M+H]+= 573.4 |
| 299 | 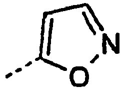 | (ESI)+: [M+H]+= 574.4 |
| 300 | 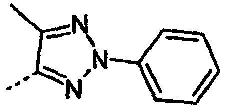 | (ESI)+: [M+H]+= 664.6 |
| 301 | CH₂CH₃ | (ESI)+: [M+H]+= 535.3 |
| 302 | CH₂CH₂CH₃ | (ESI)+: [M+H]+= 549.4 |
| 303 | 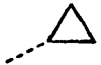 | (ESI)+: [M+H]+= 547.4 |
| 304 | C(CH₃)₃ | (ESI)+: [M+H]+= 563.5 |
| 305 | 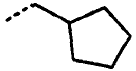 | (ESI)+: [M+H]+= 589.5 |
| 306 | CH₂(CH₂)₃CH₃ | (ESI)+: [M+H]+= 577.5 |
| 307 | CH(CH₂CH₃)CH₂CH₂C H₂CH₃ | (ESI)+: [M+H]+= 605.6 |
| 308 | 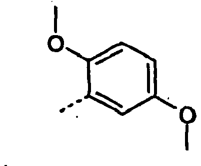 | (ESI)+: [M+H]+= 657.6 |
| 309 | 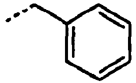 | (ESI)+: [M+H]+= 597.4 |
| 310 | 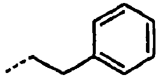 | (ESI)+: [M+H]+= 611.5 |
| 311 | 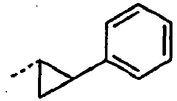 | (ESI)+: [M+H]+= 623.6 |
| 312 | 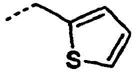 | (ESI)+: [M+H]+= 603.5 |
| 313 | 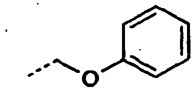 | (ESI)+: [M+H]+= 613.5 |
| 314 | 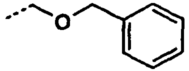 | (ESI)+: [M+H]+= 627.6 |
| 315 | CH₂CH₂CO₂CH₃ | (ESI)+: [M+H]+= 593.4 |
| 316 | CH₂(CH₂)₂CO₂CH₂CH₃ | (ESI)+: [M+H]+= 621.6 |

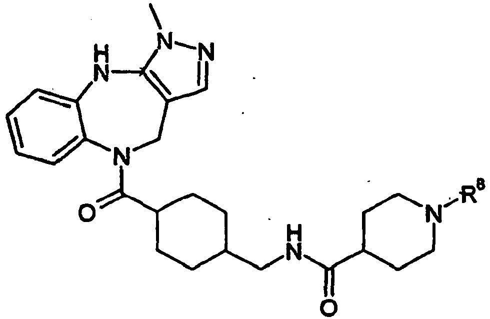

| **Compound number** | **R⁸** | **MS** |
|---|---|---|
| 317 E21 | | (ESI)+: [M+H]+= 507.4 |
| 318 | CH₂CH₂CH₃ | (ESI)+: [M+H]+= 493.5 |
| 319 | 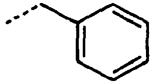 | (ESI)+: [M+H]+= 541.5 |
| 320 | 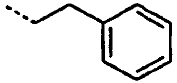 | (ESI)+: [M+H]+= 555.5 |
| 321 | 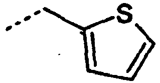 | (ESI)+: [M+H]+= 547.4 |
| 322 | CH₂CH₂CH₂CH₂CH₃ | (ESI)+: [M+H]+= 521.5 |
| 323 | 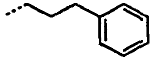 | (ESI)+: [M+H]+= 569.4 |
| 324 | CH₂CH₂CH(CH₃)₂ | (ESI)+: [M+H]+= 521.5 |
| 325 | 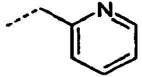 | (ESI)+: [M+H]+= 542.4 |
| 326 | 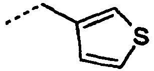 | (ESI)+: [M+H]+= 547.4 |
| 327 | CH₂CH₂CF₃ | (ESI)+: [M+H]+= 547.3 |
| 328 | 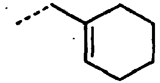 | (ESI)+: [M+H]+= 545.5 |
| 329 | CH₂CH₂OH | (ESI)+: [M+H]+= 495.6 |
| 330 | 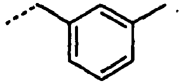 | (ESI)+: [M+H]+= 555.5 |
| 331 | 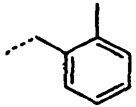 | (ESI)+: [M+H]+= 555.3 |
| 332 | 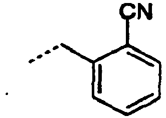 | (ESI)+: [M+H]+= 566.4 |
| 333 | 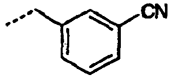 | (ESI)+: [M+H]+= 566.4 |
| 334 | 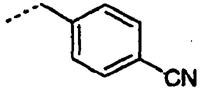 | (ESI)+: [M+H]+= 566.5 |
| 335 | 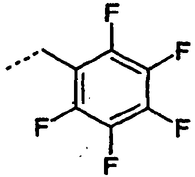 | (ESI)+: [M+H]+= 631.5 |
| 336 | 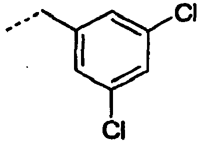 | (ESI)+: [M+H]+= 609.5 |
| 337 | 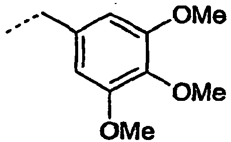 | (ESI)+: [M+H]+= 631.6 |

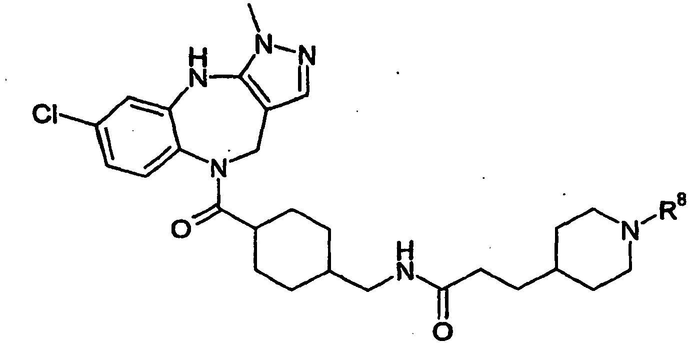

| **Compound number** | **R⁸** | **MS** |
|---|---|---|
| 338 E22 | | (ESI)+: [M+H]+= 597.6, 599.6 |
| 339 | CH₂CH₂CH₃ | (ESI)+: [M+H]+= 555.6, 557.6 |
| 340 | CH₂(CH₂)₂CN | (ESI)+: [M+H]+= 580.7, 582.7 |
| 341 | 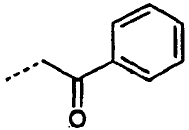 | (ESI)+: [M+H]+= 631.8, 633.8 |
| 342 | 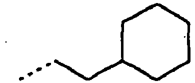 | (ESI)+: [M+H]+= 623.6, 625.6 |
| 343 | 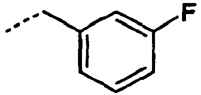 | (ESI)+: [M+H]+= 621.6, 623.6 |
| 344 | 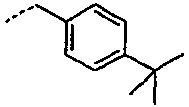 | (ESI)+: [M+H]+= 659.8, 661.8 |
| 345 | 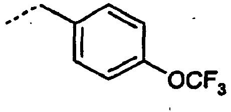 | (ESI)+: [M+H]+= 687.7, 689.7 |
| 346 | 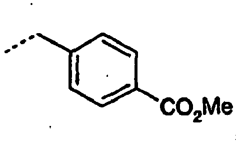 | (ESI)+: [M+H]+= 661.8, 663.8 |
| 347 | 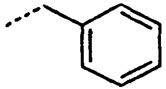 | (ESI)+: [M+H]+= 603.7, 605.7 |
| 348 | CH₂CH₂OH | (ESI)+: [M+H]+= 557.5, 559.5 |
| 349 | CH₂CO₂CH(CH₃)₂ | (ESI)+: [M+H]+= 613.6, 615.6 |
| 350 | CH₂CH₂CH(CH₃)₂ | (ESI)+: [M+H]+= 583.6, 585.6 |
| 351 | CH₂CH₂CH₂OH | (ESI)+: [M+H]+= 571.6, 573.6 |
| 352 | CH₂CN | (ESI)+: [M+H]+= 552.6, 554.6 |
| 353 | 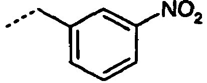 | (ESI)+: [M+H]+= 648.7, 650.7 |
| 354 | 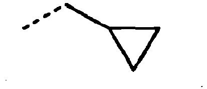 | (ESI)+: [M+H]+= 567.7, 569.6 |
| 355 | 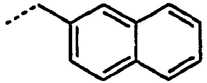 | (ESI)+: [M+H]+= 653.8, 655.8 |
| 356 | 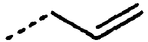 | (ESI)+: [M+H]+= 553.6. 555.6 |
| 357 | 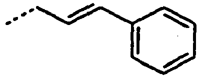 | (ESI)+: [M+H]+= 629.8, 631.8 |
| 358 | 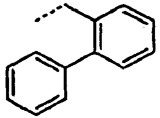 | (ESI)+: [M+H]+= 679.8, 681.8 |
| 359 | 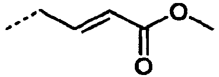 | (ESI)+: [M+H]+= 611.6, 613.6 |
| 360 | 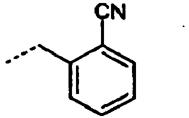 | (ESI)+: [M+H]+= 628.7, 630.7 |
| 361 | CH₂COC(CH₃)₃ | (ESI)+: [M+H]+= 611.7, 613.7 |
| 362 | CH₂CONH₂ | (ESI)+: [M+H]+= 570.6, 572.6 |
| 363 | 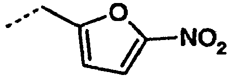 | (ESI)+: [M+H]+= 638.8,640.8 |
| 364 | 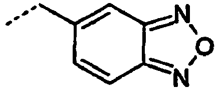 | (ESI)+: [M+H]+= 645.8, 647.8 |

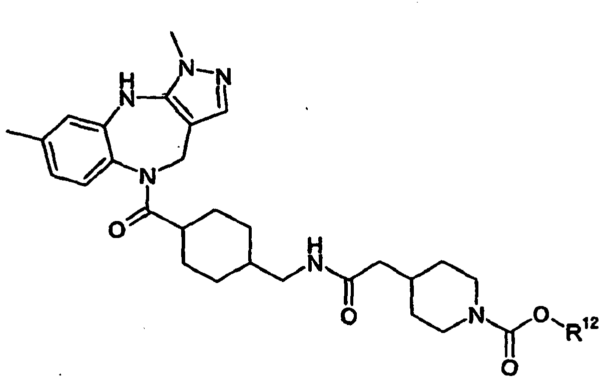

| **Compound number** | **R¹²** | **MS** |
|---|---|---|
| 365 E23 | 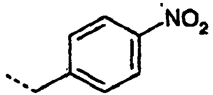 | (ESI)+: [M+H]+= 658.5 |
| 366 | 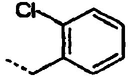 | (ESI)+: [M+H]+ = 647.5 |
| 367 | 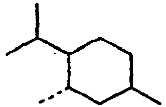 | (ESI)+: [M+H]+ = 661.7 |
| 368 | 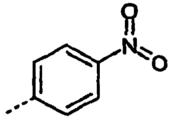 | (ESI)+: [M+H]+ = 644.5 |
| 369 | 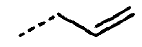 | (ESI)+: [M+H]+ = 563.5 |
| 370 | 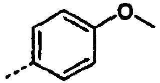 | (ESI)+: [M+H]+ = 629.6 |
| 371 | 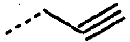 | (ESI)+: [M+H]+ = 561.4 |
| 372 | 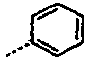 | (ESI)+: [M+H]+ = 599.4 |
| 373 | 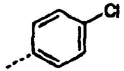 | (ESI)+: [M+H]+ = 633.5 |
| 374 | 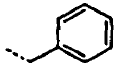 | (ESI)+: [M+H]+ = 613.5 |
| 375 | 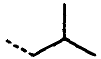 | (ESI)+: [M+H]+ = 579.5 |
| 376 | 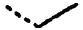 | (ESI)+: [M+H]+ = 551.5 |
| 377 | 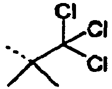 | (ESI)+: [M+H]+ = 683.5 |
| 378 | CH₃ | (ESI)+: [M+H]+= 537.4 |
| 379 | 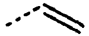 | (ESI)+: [M+H]+ = 549.4 |
| 380 | 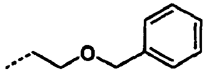 | (ESI)+: [M+H]+ = 657.6 |

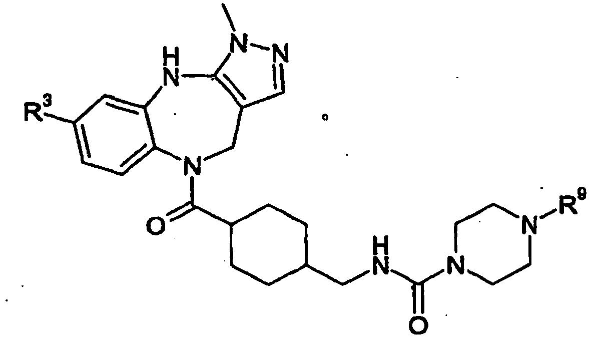

| **Compound number** | **R³** | **R⁹** | **MS** | **¹H NMR: δ (ppm)** |
|---|---|---|---|---|
| 381 E24 | Me | CH₂CH₂C(CH₃)₃ | (APCl)+:[ M+H]+= 550.4 | 0.41-0.82 (2H, m), 0.86 (9H, s), 1.33-1.50 (7H, m), 1.73 (2H, m), 2.12 (1H, m), 2.27-2.39 (6H, m), 2.29 (3H, s), 2.90-2.93 (2H, m), 3.31-3.35 (4H, m), 3.64 (3H, s), 3.74 (1H, d, J=14.6Hz), 4.78 (1H, m), 5.54 (1H, d, J=14.6Hz), 6.77 (1H, dd, J=1.0, 8.0Hz), 6.88 (1H, s), 6.95 (1H, d, J=8.0Hz), 7.03 (1H, d, J=1.0Hz), 7.11 (1H, s) |
| 382 | Me | CH₂CH₂CH₂SCH₃ | (ESI)+: [M+H]+= 554.6 | - |
| 383 | Me | CH₂CH₂OCH₃ | (ESI)+: [M+H]+= 524.7 | - |
| 384 | Me | CH₂CH₂CH₂CF₃ | (ESI)+: [M+H]+= 576.6 | - |
| 385 | Me | CH₂CH₂N(CH₂CH₃)₂ | (ESI)+: [M+H]+= 565.6 | - |
| 386 | Me | CH₂CH₂CH₂F | (ESI)+: [M+H]+= 526.6 | - |
| 387 | Me | CH₂CH₂CH₂OH | (ESI)+: [M+H]+= 524.6 | - |
| 388 | Me | CH₂C(CH₃)₃ | (ESI)+: [M+H]+= 536.5 | 0.55-0.84 (2H, m), 0.84 (9H, s), 1.18-1.75 (7H, m), 2.04-2.16 (3H, m), 2.34 (3H, s), 2.44-2.48 (4H, m), 2.93-2.97 (2H, m), 3.26-3.29 (4H, m), 3.70 (3H, s), 3.75 (1H, d, J=14.6Hz), 4.35-4.39 (1 H, bs), 5.58 (1H, d, J=14.6Hz), 5.84 (1H, s), 6.79-6.83 (2H, m), 6.98 (1H, m), 7.16 (1H, s) |
| 389 | Me | 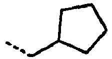 | (APCl)+: [M+H]+= 548.5 | 0.46-0.71 (2H, m), 1.11-1.27 (4H, m), 1.37-1.59 (7H, m), 1.69-1.78 (4H, m), 1.95-2.17 (2H, m), 2.21 (2H, d, J=7.4Hz), 2.30 (3H, s), 2.32-2.36 (4H, m), 2.85-2.98 (2H, m), 3.29-3.32 (4H, m), 3.64 (3H, s), 3.74 (1H, d, J=14.6Hz), 4.67-4.71 (1H, m), 5.55 (1H, d, J=14.6Hz), 6.78 (1H, d, J=8.0Hz), 6.87-6.88 (2H, m), 6.95 (1H, d, J=8.0Hz), 7.11 (1H, s) |
| 390 | Me | CH₂CH₂CH(CH₃)₂ | (APCl)+: [M+H]+= 536.5 | 0.48-0.72 (2H, m), 0.86 (6H, t, J=6.4Hz), 1.18-1.38 (5H, m), 1.46-1.60 (3H, m), 1.68-1.79 (2H, m), 2.07-2.18 (1H, m), 2.28-2.38 (9H, m), 2.88-2.96 (2H, m), 3.31-3.34 (4H, m), 3.65 (3H, s), 3.75 (1H, d, J=14.6Hz), 4.71-4.75 (1H, m), 5.55 (1H, d, J=14.6Hz), 6.78 (1H, d, J=7.9Hz), 6.88 (1H, s), 6.96 (1H, d, J=7.9Hz), 7.04 (1H, dd, J=1.2, 7.9Hz), 7.12 (1H, s) |
| 391 | Me | CH₂CH(CH₃)₂ | (APCl)+: [M+H]+= 522.4 | 0.46-0.71 (2H, m), 0.85 (6H, t, J=6.4Hz), 1.19-1.27 (2H, m), 1.37-1.54 (3H, m), 1.68-1.78 (3H, m), 2.04 (2H, d, J=7.4Hz), 2.05-2.17 (1H, m) 2.29 (3H, s), 2.29-2.33 (4H, m), 2.87-2.98 (2H, m), 3.28-3.32 (4H, m), 3.64 (3H, s), 3.74 (1H, d, J=14.6Hz), 4.68-4.72 (1H, m), 5.55 (1H, d, J=14.6Hz), 6.78 (1H, d, J=8.0Hz), 6.88 (1H, s), 6.93 (1H, s), 6.95 (1H, d, J=8.0Hz), 7.11 (1H, s) |
| 392 | Me | CH₂CH₂CH₃ | (APCl)+: [M+H]+= 508.4 | 0.47-0.89 (2H, m), 0.87 (3H, t, J=7.2Hz), 1.18-1.27 (2H, m), 1.37-1.54 (5H, m), 1.68-1.78 (2H, m), 2.07-2.17 (1 H, m), 2.23-2.26 (2H, m), 2.29 (3H, s), 2.34-2.38 (4H, m), 2.87-2.97 (2H, m), 3.30-3.34 (4H, m), 3.64 (3H, s), 3.74 (1H, d, J=14.6Hz), 4.72-4.76 (1H, m), 5.54 (1H, d, J=14.6Hz), 6.78 (1H, d, J=8.0Hz) 6.87-6.88, (2H, m), 6.95 (1H, d, J=8.0Hz), 7.11 (1H, s) |
| 393 | Cl | 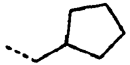 | (APCl)+: [M+H]+= 568.4, 570.4 | 0.41-0.56 (1H, m), 0.60-0.75 (1H, m), 0.78 (1H, d, J=6.4Hz), 0.94-1.14 (2H, m), 1.14-1.55 (9H, m), 1.57-1.72 (4H, m), 1.83-2.00 (2H, m), 2.18 (2H, d, J=6.9Hz), 2.29 (4H, t, J=4.9Hz), 2.70-2.90 (2H, m), 3.22 (4H, t, J=4.9Hz), 3.58 (3H, s), 3.66 (1H, d, J=14.6Hz), 5.17 (1H, t, J=5.4Hz), 5.38 (1H, d, J=14.6Hz), 6.86 (1H, d, J=2.2Hz), 6.91 (1H, s), 7.03 (1H, s), 7.12 (1H, d, J=2.2Hz) |
| 394 | Cl | CH₂CH₂CH(CH₃)₂ | (APCl)+: [M+H]+= 556.4, 558.4 | 0.40-0.59 (1H, m), 0.60-0.80 (1H, m), 0.86 (6H, d, J=6.7Hz), 1.09-1.65 (9H, m), 1.65-1.81 (2H, m), 2.01-2.17 (1H, m), 2.30-2.41 (2H, m), 2.41 (4H, t, J=4.9Hz) 2.92 (2H, t, J=5.9Hz), 3.38 (4H, t, J=4.9Hz), 3.70 (3H, s), 3.77 (1H, d, J=14.6Hz), 5.07 (1H, t, J=5.4Hz) 5.52 (1H, d, J=14.6Hz), 6.94 (1H, d, J=2.2Hz), 7.02 (1H, s), 7.12 (1H, s), 7.32 (1H, d, J=2.2Hz) |
| 395 | Cl | CH₂CH(CH₃)₂ | (APCl)+: [M+H]+= 542.4, 544.4 | 0.38-0.60 (1H, m), 0.60-0.78 (1H, m), 0.82 (6H, d, J=6.7Hz), 1.10-1.40 (2H, m), 1.40-1.56 (2H, m), 1.63-1.80 (4H, m), 2.03 (2H, d, J=7.4Hz), 2.12 (2H, s), 2.31 (4H, t, J=4.9Hz), 2.85-2.94 (2H, m), 3.30 (4H, t, J=4.9Hz), 3.64 (3H, s), 3.72 (1H, d, J=14.6Hz), 4.75 (1 H, t, J=5.7Hz), 5.49 (1H, d, J=14.6Hz), 6.93 (1H, d, J=2.2Hz), 6.98 (1H, s), 7.11 (1H, s), 7.15 (1H, d, J=2.2Hz) |
| 396 | Cl | CH₂CH₂CH₃ | (APCl)+: [M+H]+= 528.3, 530.3 | 0.39-0.60 (1H, m), 0.60-0.83 (1H, m), 0.88 (3H, t, J=7.4Hz), 1.17-1.63 (9H, m), 1.64-1.83 (2H, m), 2.00-2.15 (1H, m), 2.25-2.35 (2H, m), 2.40 (4H, t, J=4.9Hz), 2.90-3.00 (2H, m), 3.35 (4H, t, J=4.9Hz), 3.72 (3H, s), 4.59 (1H, t, J=5.7Hz), 5.54 (1H, d, J=14.6Hz), 6.90-7.04 (2H, m), 7.10-7.19 (2H, m) |
| 397 | Cl | 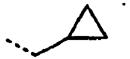 | (APCl)+: [M+H]+= 540.3, 542.3 | d₆-DMSO: 0.02-0.04 (2H, m), 0.41-0.47 (2H, m), 0.80-1.75 (10H, m), 2.03 (1H, m), 2.14 (2H, d, J=6.5Hz), 2.30-2.34 (4H, m), 2.74 (2H, m), 3.20-3.28 (4H, m), 3.67 (3H, s), 3.70 (1H, d, J=14.6Hz), 5.34 (1H, d, J=14.6Hz), 6.29 (1H, m), 7.02 (1H, dd, J=2.5, 8.0Hz), 7.08 (1H, s), 7.25 (1H,d, J=8.0Hz), 7.42 (1H, d, J=2.5Hz) |
| 398 | Me | 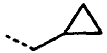 | (APCl)+: [M+H]+= 520.4 | 0.06-0.08 (2H, m), 0.46-0.53 (2H, m), 0.81 (1H, m), 1.13-1.79 (9H, m), 2.09 (1H, m), 2.23 (2H, m), 2.30 (3H, s), 2.45-2.49 (4H, m), 2.92 (2H, m), 3.33-3.37 (4H, m), 3.65 (3H, s), 3.74 (1H, d, J=14.6Hz), 4.69 (1H, m), 5.54 (1H, d, J=14.8Hz), 6.67 (1H, s), 6.78 (1H, dd, J=1.0, 8.0Hz), 6.86 (1H, d, J=1.0Hz), 6.96 (1 H, d, J=8.0Hz), 7.11 (1H, s) |
| 399 | Cl | CH₂CH₂C(CH₃)₃ | (APCl)+: [M+H]+= 570.3, 572.3 | 0.42-0.81 (2H, m), 0.87 (9H, s), 1.22-1.40 (7H, m), 1.74-1.78 (2H, m), 2.08 (1H, m), 2.28-2.40 (6H, m), 2.87-2.92 (2H, m), 3.33-3.37 (4H, m), 3.63 (3H, s), 3.74 (1H, d, J=14.6Hz), 4.89 (1H, m), 5.51 (1H, d, J=14.6Hz), 6.93 (1H, dd, J=2.2, 8.4Hz), 7.03 (1 H, d, J=8.4Hz), 7.10 (1H, s), 7.20 (1H, d, J=2.2Hz), 8.10 (1H, s) |
| 400 | H | CH₂CH₂C(CH₃)₃ | (APCl)+: [M+H]+= 536.4 | 0.40-0.46 (1H, m), 0.63-0.68 (1H, m), 0.82 (9H, s), 1.26-1.67 (7H, m), 1.73-1.79 (2H, m), 2.03-2.08 (1H, m), 2.34-2.37 (6H, m), 2.82-2.94 (2H, m), 3.32 (4H, m), 3.64 (3H, s), 3.77 (1H, d, J=14.6Hz), 4.80 (1H, t, J=5.4Hz), 5.56 (1H, d, J=14.6Hz), 6.95 (1H, m), 7.05-7.12 (3H, m), 7.19 (1H, m), 7.32 (1H, s) |
| 401 | H | 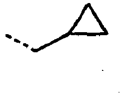 | (ESI)+: [M+H]+= 506.3 | 0.04-0.11 (2H, m), 0.46-0.53 (2H, m), 0.61-0.72 (1H, m), 0.72-0.89 (1H, m), 1.20-1.59 (6H, m), 1.69-1.81 (2H, m), 2.04-2.17 (1H, m), 2.23 (2H, d, J=6.4Hz), 2.46 (4H, t, J=4.9Hz), 2.81-3.05 (2H, m), 3.35 (4H, t, J=4.9Hz), 3.69 (3H, s), 3.79 (1H, d, J=14.6Hz), 4.56 (1H, t, J=5.6Hz), 5.60 (1H, d, J=14.6Hz), 6.67 (1H, s), 6.96-7.26 (4H, m), 7.15 (1H, s) |
| 402 | H | 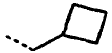 | (ESI)+: [M+H]+= 520.3 | - |

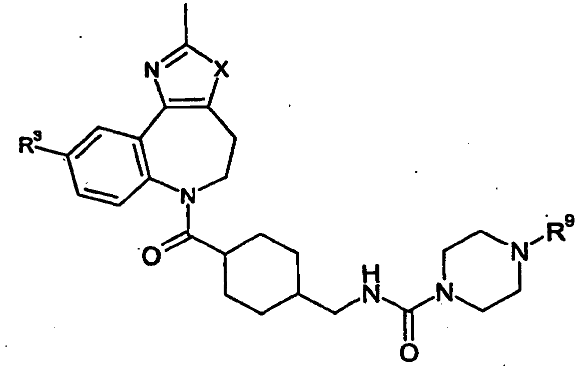

| **Compound number** | **R³** | **R⁹** | **X** | **MS** | **¹H NMR: δ (ppm)** |
|---|---|---|---|---|---|
| 403 | Cl | CH₂CH₂C(CH₃)₃ | S | (ESI)⁺: [M+H]⁺ = 586.6, 588.6 | 0.49-0.60 (1H, m), 0.88 (9H, s), 1.11-1.24 (2H, m), 1.34-1.41 (4H, m), 1.47-1.87 (5H, m), 2.28-2.41 (5H, m), 2.67 (3H, s), 2.82-3.18 (4H, m), 3.29-3.33 (4H, m), 3.37-3.59 (1H, m), 4.34-4.38 (1H, m), 4.76-4.83 (1H, m), 7.10 (1H, dJ, =8.4Hz), 7.25 (1H, dd, J=2.5, 8.4Hz), 8.38 (1H, d, J=2.5Hz) |
| 404 | Me | 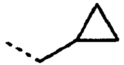 | S | (ESI)+: [M+H]+ = 536.3 | 0.05-0.15 (2H, m), 0.40-0.60 (3H, m), 0.70-0.90 (2H, m), 1.10-1.20 (2H, m), 1.45-1.95 (5H, m), 2.24 (2H, d, J=6.7Hz), 2.35-2.55 (8H, m), 2.69 (3H, s), 2.85-3.05 (4H, m), 3.30-3.40 (4H, m), 3.46-3.65 (1H, m), 4.32-4.38 (1H, m), 4.74-4.86 (1H, m), 7.00-7.15 (2H, m), 8.14(1H, s) |

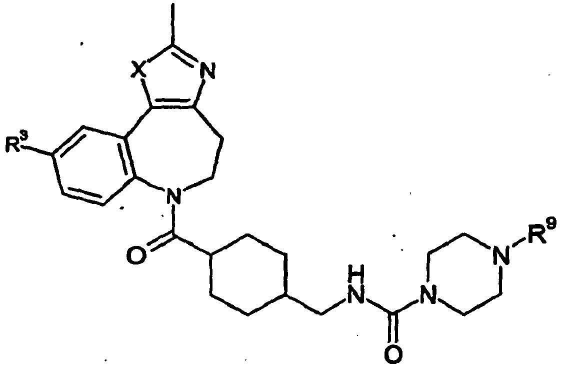

| **Compound number** | **R³** | **R⁹** | **X** | | **¹H NMR: δ (ppm)** |
|---|---|---|---|---|---|
| 405 | Me | CH₂CH₂C(CH₃)₃ | NH | | 0.45-0.55 (1H, m), 0.75-0.90 (10H, m), 1.14-1.20 (1H, m), 1.20-1.90 (8H, m), 2.30-2.45 (13H, m), 2.65-3.00 (4H, m), 3.30-3.40 (5H, m), 4.35-4.45 (1H, m), 4.70-4.80 (1H, m), 6.95-7.15 (2H, m), 7.95-8.05 (1H, m) |

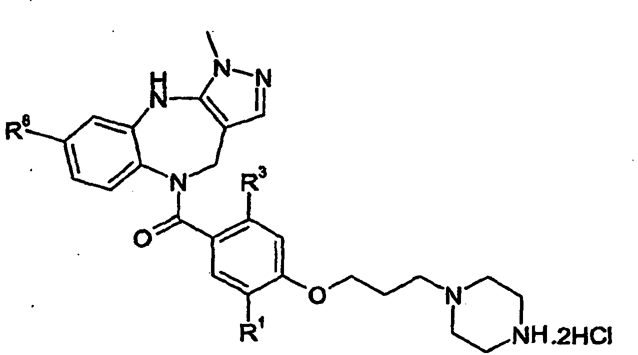

| **Compound number** | **R¹** | **R³** | **R⁸** | **MS** | **¹H NMR: δ (ppm)** |
|---|---|---|---|---|---|
| 406 E49 | F | H | H | (APCl)+: [M+H]+= 465.3 | d4 MeOH 2.29-2.36 (2H, m), 3.47 (2H, t, J=7.7Hz), 3.50-3.78 (8H, m), 4.02 (3H, s), 4.03 (1H, d), 4.16 (2H, t, J=5.4Hz), 5.87 (1H, d, J=15.0Hz), 6.88-7.05 (4H, m), 7.24-7.31 (1H, m), 7.39-7.43 (1H, m), 8.02 (1H, s) |
| 407 | H | F | Cl | (APCl)+: [M+H]+= 499.4, 501.4 | - |
| 408 | Me | H | H | (APCI)+: [M+H]+= 461.4 | d4-MeOH 2.07 (3H, s), 2.25-2.35 (2H, m), 3.43-3.49 (2H, m), 3.52-3.73 (8H, m), 4.03 (3H, s), 3.98-4.07 (3H, m), 5.90 (1 H, d, J=14.6Hz), 6.71 (1H, d, J=8.9Hz), 6.81-6.88 (2H, m), 7.04-7.07 (2H, m), 7.22-7.29 (1 H, m), 7.40 (1H, d, J=7.7Hz), 8.02 (1H, s) |

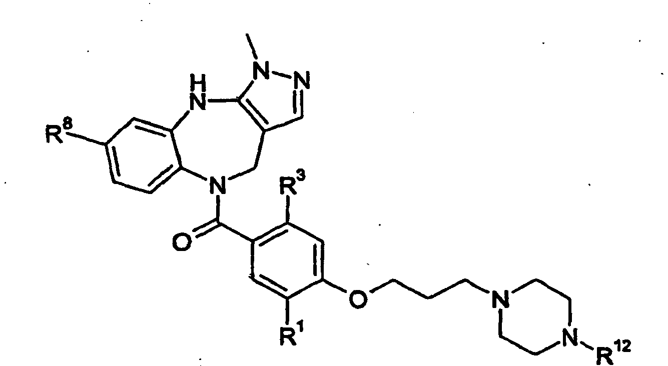

| **Compound number** | **R¹** | **R³** | **R⁸** | **R¹²** | **MS** | **¹H NMR: δ (ppm)** |
|---|---|---|---|---|---|---|
| 409 E50 | F | H | H | CH₂CH₂C(CH₃)₃ | (APCI) ⁺:[M+H ]⁺= 549.4 | (9H, s), 1.35-1.46 (2H, m),1.90-1.95 (2H, m), 2.42-2.70 (11H, m), 2.95-3.08 (1H, m), 3.47-3.59 (1H, m), 3.79 (3H, s), 3.90-4.03 (2H, m), 5.87 (1H, d, J=14.8Hz), 6.31 (1H, s), 6.70 (3H, s), 6.89-7.04 (3H, m), 7.04-7.13 (1H, m), 7.21 (1H, s) |
| 410 | H | Cl | H | CH₂CH(CH₃)₂ | (APCI) +: [M+H] += 537.4, 539.3 | 0.84-0.88 (6H, m), 1.69-2.08 (5H, m), 2.37-2.53 (10H, m), 3.68(3H, s), 3.83-4.03 (3H, m), 4.33-4.42 (1H, brs), 5.81 (1H, d, J=14.6Hz), 6.40-6.44 (2H, m), 6.54-7.34 (5H, m), 7.21 (1H, s) |
| 411 | H | Cl | H | CH₂CH₂CH₃ | (APCI) +: [M+H] += 523.3, 525.4 | 0.84-0.90 (3H, m), 1.41-1.54 (2H, m), 1.80-2.00 (2H, m), 2.24-2.54 (12H, m), 3.68 (3H, s), 3.81-4.01 (3H, m), 4.33-4.42 (1H, brs), 5.80 (1H, d, J=14.6Hz), 6.40-7.34 (7H, m), 7.21 (1H, s) |
| 412 | Me | H | Me | 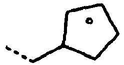 | (APCI) +: [M+H] += 557.5 | 1.06-1.26 (3H, m), 1.40-1.63 (6H, m), 1.63-1.78 (2H, m), 1.78-1.96 (1H, m), 1.96-2.10 (4H, m), 2.10-2.29 (6H, m), 2.30-2.60 (8H, m), 3.76 (3H, s), 3.81-4.00 (3H, m), 5.89 (1H, d, J=14.6Hz), 6.00 (1H, s), 6.40-6.62 (2H, m), 6.73 (1H, s), 6.98 (1H, d. J=7.9Hz), 7.12 (1 H, brs), 7.20 (1H, brs) |
| 413 | Me | H | Me | CH₂C(CH₃)₃ | (ESI)⁺: [M+H] += 545.4 | 0.85 (9H, s), 1.95-2.01 (3H, m), 2.01-2.11 (1 H, m), 2.16 (2H, s), 2.19 (3H, s), 2.70-2.82 (6H, m), 2.85-3.07 (6H, m), 3.73-3.85 (5H, m), 3.90 (1H, d, J=14.6Hz), 5.83 (1H, d, J=14.6Hz), 6.39 (1H, d, J=8.7Hz), 6.43-6.56 (2H, m), 6.88 (1H, s), 6.92 (1H, s), 6.95-7.10 (1H, m), 7.12 (1H, s), 7.18 (1H, s) |
| 414 | Me | H | Me | CH₂CH(CH₂CH₃)₂ | (ESI)+: [M+H] += 559.5 | 0.83 (6H, t, J=7.4Hz), 1.20-1.40 (7H, m), 1.40-1.52 (1H, m), 1.90-2.10 (8H, m), 2.19 13H, s), 2.41 (2H, d, J=6.7Hz), 2.70-2.85 (3H, m), 2.85-2.92 (3H, m), 3.79 (3H, s), 3.80-3.90 (2H, m), 5.83 (1H, d, J=14.6Hz), 6.37-6.60 (2H, m), 6.80 (1H, s), 6.87-6.97 (2H, m), 7.13 (2H, d, J=16.1Hz) |
| 415 | Me | H | Me | CH₂CH₂CH(CH₃)₂ | (APCI) +: [M+H] += 545.5 | 0.86 (6H, d, J=6.7Hz), 1.33-1.45 (2H, m), 1.46-1.62 (1H, m), 1.82-1.93 (2H, m), 2.01 (3H, s), 2.10-2.25 (2H, m), 2.29-2.40 (2H, m), 2.40-2.59 (8H, m), 2.79-2.91 (1H, m), 3.48-3.60 (1H, m), 3.60-3.80 (3H, m), 3.80-3.96 (3H, m), 3.98-4.10 (1H, m), 5.88 (1H, d, J=14.6Hz), 6.19 (1H, s), 6.41-6.52 (2H, m), 6.52-6.60 (1H, m), 6.75 (1H, s), 6.97 (1H, d, J=7.7Hz) 7.12 (1 H, s), 7.19 (1H, s) |
| 416 | Me | H | Me | CH₂CH(CH₃)₂ | (APCI) +: [M+H] += 531.2 | 0.90 (6H, d, J=6.7Hz), 1.28 (2H, t, J=7.4Hz), 1.72-1.88 (1H, m), 1.90-2.07 (7H, m), 2.19 (3H, s), 2.26 (2H, d, J=7.2Hz), 2.61-2.88 (8H, m), 3.45 (1H, s), 3.78 (2H, s), 3.81-3.91 (2H, m), 5.86 (1H, d, J=14.6Hz), 6.40-6.60 (3H, m), 6.83 (1H, s), 6.95 (1H, d, J=8.2Hz), 7.13-7.19 (2H, m) |
| 417 | Me | H | Me | CH₂CH₂CH₃ | (APCI) +: [M+H] += 517.4 | 0.88 (3H, t, J=7.4Hz), 1.39-1.60 (2H, m), 1.80-1.95 (2H, m), 1.96-2.06 (2H, M), 2.19 (3H, s), 2.29-2.38 (2H, m), 2.39-2.64 (12H, m). 3.75 (3H, s), 3.80-3.96 (3H, m), 5.88 (1H, d, J=14.6Hz), 6.23 (1H, s), 6.40-6.60 (2H, m), 6.76 (1H, s), 6.98 (1H, d, J=7.7 Hz), 7.04-7.20 (2H, m) |
| 418 | F | H | H | CH₂CH(CH₃)₂ | (APCI) +: [M+H] += 521.4 | 0.85 (6H, d, J=6.4Hz), 1.71-1.76 (1H, m), 1.89-1.95 (2H, m), 2.04 (2H, d, J=7.2Hz), 2.43 (10H, m), 3.74 (3H, s), 3.93-3.98 (3H, m), 5.86 (1H, d, J=14.6Hz), 6.67-6.72 (4H, m), 6.91-7.08 (4H, m), 7.20 (1H, s) |
| 419 | F | H | H | 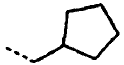 | (APCI) +: [M+H] += 547.5 | 1.10-1.17 (2H, m), 1.44-1.58 (4H, m), 1.64-1.73 (2H, m), 1.87-2.03 (3H, m), 2.22 (2H, d, J=7.2Hz), 2.42 (10H, m), 3.72 (3H, s), 3.92-3.97 (3H, m), 5.86 (1H, d, J=14.6Hz), 6.66-6.72 (3H, m), 6.76 (1H, s), 6.87-7.07 (4H, m), 7.19 (1H, s) |
| 420 | F | H | H | CH₂CH(CH₂CH₃)₂ | (APCI) +: [M+H] += 549.5 | 0.81 (6H, t, J=7.3Hz), 1.21-1.37 (5H, m), 1.88-1.93 (2H, m), 2.10 (2H, d, J=6.7Hz), 2.41 (10H, m), 3.73 (3H, s), 3.93-3.98 (3H, m), 5.87 (1H, d, J=14.6Hz), 6.67-6.72 (4H, m), 6.87-7.07 (4H, m), 7.20 (1H, s) |
| 421 | F | H | H | CH₂CH₂CH(CH₃)₂ | (APCI) +: [M+H] += 535.4 | 0.83 (6H, d, J=6.4Hz), 1.27-1.35 (2H, m), 1.50 (1H, m), 1.85-1.94 (2H, m), 2.24-2.40 (12H, m), 3.69 (3H, s), 3.90-3.95 (3H, m), 5.84 (1H, d, J=14.6Hz), 6.63-6.69 (3H, m), 6.86 (1H, d, J=11.9Hz), 6.97-7.02 (4H, m), 7.16 (1H, s) |
| 422 | F | H | H | CH₂C(CH₃)₃ | (ESI)+: [M+H] += 535.4 | 0.83 (9H, s), 1.92-2.00 (2H, m), 2.03 (2H, s), 2.47-2.54 (10H, m), 3.79 (3H, s), 3.93-3.98 (3H, m), 5.88 (1H, d, J=14.6Hz), 6.20 (1H, s), 6.67-6.70 (3H, m), 6.91-6.96 (2H, m), 7.03-7.13 (2H, m), 7.21-7.22 (1H, m) |
| 423 | F | H | H | CH₂CH₂CH₃ | (APCI) +: [M+H] += 507.4 | 0.85 (3H, t, J=7.4Hz), 1.47 (2H, m), 1.90 (2H, m), 2.25 (2H, m), 2.43 (10H, m), 3.73 (3H, s), 3.95 (3H, m), 5.86 (1H, d, J=14.6Hz), 6.67-6.72 (4H, m), 6.87-7.08 (4H, m), 7.20 (1H, s) |
| 424 | Me | H | H | 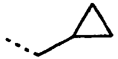 | (ESI)+: [M+H] += 515.3 | 0.07-0.13 (2H, m), 0.47-0.54 (2H, m), 0.81-0.92 (1H, m), 1.87-1.96 (3H, brt), 2.01 (3H, s). 2.27 (2H, d, J=6.7Hz), 2.42-2.64 (10H, m), 3.78 (3H, s), 3.86-4.01 (3H, m), 5.92 (1 H, d, J=14.8Hz), 6.06 (1H, s), 6.48 (1H, brd, J=9.6Hz), 6.70 (2H, s), 6.92 (1 H, d, J=7.4Hz), 6.94-7.11 (3H, m), 7.22 (1H, s) |
| 425 | F | H | H | 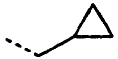 | (ESI)+: [M+H] += 519.3 | 0.05-.0.12 (2H, m), 0.46-0.53 (2H, m), 0.81-0.87 (1H, m), 1.87-1.98 (2H, m), 2.25 (2H, d, J=6.7Hz), 2.38-2.62 (10H, m), 3.78 (3H, s), 3.98 (2H, t, J=5.7Hz), 3.96 (1H, d, J=14.6Hz), 5.88 (1H, d, J=14.6Hz), 6.21 (1H, s), 6.68-6.74 (3H, m), 6.89-6.96 (2H, m), 7.03-7.13 (2H, m), 7.22 (1H, s) |
| 426 | H | Cl | Me | CH₂cH₂C(CH₃)₃ | (APCI) +: [M+H]+= 579.4, 581.4 | 0.89 (9H, s), 1.38-1.50 (2H, m), 1.79-1.91 (2H, m), 2.16 (3H, s), 2.43-2.60 (8H, m), 2.60-2.79 (4H, m), 3.46 (3H, s), 3.74 (2H, s), 3.86 (2H, t, J=5.9Hz), 5.78 (1H, d, J=14.6Hz), 6.26 (1H, s), 6.47 (1H, d, J=6.7Hz), 6.67-6.84 (4H, m), 7.21 (1H, s) |
| 427 | H | Cl | Cl | CH₂CH₂C(CH₃)₃ | (APCI) +: [M+H] += 599.3 | 0.88 (9H, s), 1.35 (1H, d, J=6.7Hz), 1.37-1.47 (2H, m), 1.83-1.90 (1H, m), 2.42-2.78 (10H, m), 3.46 (3H, s), 3.74 (2H, s), 3.88 (2H, t, J=6.2Hz), 3.94-4.00 (1 H, m), 5.77 (1H, d, J=14.6Hz), 6.50 (1H, dd, J=2.2, 8.7Hz), 6.61 (1H, dd, J=2.2, 8.7Hz), 6.73 (1H, d, J=2.2Hz), 6.81 (1H, s), 6.83 (1 H, s), 6.84-7.00 (2H, m), 7.22 (1H, s) |
| 428 | H | F | Cl | CH₂CH₂C(CH₃)₃ | (APCI) +: [M+H] += 583.4, 585.4 | 0.87 (9H, s), 1.26 (1H, d, J=5.2Hz), 1.32-1.41 (2H, m), 1.88-1.91 (1H, m), 2.30-2.41 (2H, m), 2.42-2.63 (8H, m), 3.44 (3H, s), 3.69 (2H, s), 3.82-3.93 (2H, m), 5.78 (1H, d, J=14.8Hz), 6.33 (1H, dd, J=2.2, 14.1 Hz), 6.43-6.51 (1H, m), 6.54-6.70 (2H, m), 6.86 (1H, s), 6.97 (1H, d, J=2.2Hz), 7.02-7.10 (2H, m), 7.19 (1H, s) |
| 429 | Cl | H | Cl | CH₂CH₂C(CH₃)₃ | (ESI)+: [M+H] += 599.4 | 0.88 (9H, s), 1.35-1.41 (2H, m), 1.88-, 2.12 (4H, m), 2.29-2.35 (2H, m), 2.38-2.57 (8H, m), 3.46 (3H, s), 3.72-3.76 (2H, m), 3.92-4.08 (2H, m), 5.85 (1H, d, J=14.6Hz), 6.43 (1H, s), 6.56-6.74 (2H, m), 6.98 (1H, s), 7.08 (1H, d, J=8.7Hz), 7.21 (1H, s). 7.27 (1H, s) |
| 430 | F | H | Cl | CH₂CH₂C(CH₃)₃ | (APCI) +: [M+H] += 583.4, 585.4 | 0.88 (9H, s), 1.36-1.42 (2H, m), 1.84-2.03 (4H, m), 2.31-2.39 (2H, m), 2.40-2.62 (8H, m), 3.46 (3H, s), 3.76 (2H, s), 3.92-4.04 (2H, m), 5.85 (1H, d, J=14.1Hz), 6.52 (1H, s), 6.57-6.80 (3H, m), 6.95-7.06 (2H, m), 7.21 (1H, s) |
| 431 | H | F | Me | CH₂CH₂C(CH₃)₃ | (APCI) +: [M+H] += 563.5 | 0.89 (9H, s), 1.35-1.41 (2H, m), 1.88-1.97 (2H, m), 2.05-2.13 (1H, m), 2.21 (3H, s), 2.28-2.34 (2H, m), 2.34-2.54 (9H, m), 3.37 (3H, s), 3.68 (3H, s), 3.91 (2H, t, J=6.4Hz), 5.96-6.01 (1H, m), 6.24-6.28 (1H, m), 6.33-6.42 (1H, m), 6.42-6.48 (1H, m), 6.54-6.60 (1H, m), 6.72 (1H, d, J=7.9Hz), 7.48-7.51 (1H, m) |
| 432 | Me | H | Me | CH₂CH₂C(CH₃)₃ | (ESI)+: [M+H] += 559.5 | 0.88 (9H, t, J=12.9Hz), 1.35-1.42 (2H, m), 1.72-2.00 (2H, m), 2.02 (3H, s), 2.22 (3H, s), 2.28-2.35 (2H, m), 2.37-2.62 (10H, m), 3.77 (3H, s), 3.88-3.96 (3H, m), 5.89 (1H, d, J=11.6Hz), 6.45-6.62 (3H, m), 6.73 (1H, s), 6.94-7.01 (1H, m), 7.13 (1H, s), 7.19-7.22 (1H, m) |
| 433 | Me | H | Cl | CH₂cH₂C(CH₃)₃ | (ESI)+: [M+H] += 579.4. 581.5 | 0.88 (9H, t, J=12.9Hz), 1.36-1.43 (2H, m), 1.91-2.02 (2H, m), 2.05 (3H, s), 2.30-2.37 (2H, m), 2.39-2.63 (10H, m), 3.75 (3H, s), 3.82-3.98 (3H, m), 5.87 (1H, d, J=14.6Hz), 6.25 (1H, s), 6.53-6.70 (2H, m), 6.96 (2H, s), 7.13-7.23 (2H, m) |
| 434 | Cl | H | Me | CH₂CH₂C(CH₃)₃ | (APCI) +: [M+H] += 579.4, 581.4 | 0.88 (9H, s), 1.32-1.43 (2H, m), 1.61-1.82 (1H, m), 1.89-2.02 (2H, m), 2.23 (3H, s), 2.28-2.37 (2H, m), 2.37-2.68 (9H, m), 3.78 (3H, s), 3.96-4.01 (3H, m), 5.86 (1H, d, J=14.6Hz), 6.00 (1H, s), 6.48-6.61 (2H, m), 6.61-6.70 (2H, m), 6.75 (1H, s), 7.14 (1H, d, J=8.2Hz), 6.22 (1H, s) |
| 435 | F | H | Me | CH₂CH₂C(CH₃)₃ | (APCI) +: [M+H] += 563.4 | 0.87 (9H, s), 1.35-1.41 (2H, m), 1.88-1.97 (2H, m), 2.05-2.13 (1H, m), 2.21 (3H, s), 2.28-2.34 (2H, m), 2.34-2.54 (9H, m), 3.76 (3H, s), 3.90-4.04 (3H, m), 5.85 (1H, d, J=12.1Hz), 6.18 (1H, s), 6.47-6.60 (2H, m), 6.67-6.79 (2H, m), 6.91 (1H, d, J=9.3Hz), 7.05 (1H, d, J=9.3Hz), 7.20 (1H, s) |
| 436 | Cl | H | H | CH₂CH₂C(CH₃)3 | (APCI) +: [M+H] += 565.4, 567.4 | 0.88 (9H, s), 1.29 (2H, d. J=6.7Hz), 1.31-1.42 (2H, m), 1.59-1.82 (2H, m), 1.92-1.97 (2H, m), 2.29-2.35 (2H, m), 2.35.2.67 (9H, m), 3.80 (3H, s), 3.91-4.04 (2H. m), 5.89 (1H, d, J=15Hz), 6.03 (1H, s), 6.60-6.80 (2H, m), 6.94 (1H, d, J=7.9Hz), 7.07-7.19 (2H, m), 7.23 (1H, s) |
| 437 d | H | Cl | H | CH₂CH₂C(CH₃)₃ | (APCI) +: [M+H] += 565.5, 567.4 | 0.88 (9H, s), 1.32-1.43 (2H, m), 1.80-1.93 (2H, m), 2.27-2.38 (2H, m), 2.38-2.58 (9H, m), 3.61 (1H, s), 3.77 (3H, s), 3.86 (1H, t, J=6.2Hz), 4.02 (1H, s), 5.85 (1H, d, J=14.8Hz), 6.02 (1H, s), 6.45 (1H, dd, J=2.2, 8.4Hz), 6.67-6.69 (1H, m), 6.69-6.91 (2H, m), 6.91-7.12 (2H, m) |
| 438 | H | F | H | CH₂CH₂C(CH₃)₃ | (APCI) +: [M+H] += 549.4 | 0.88 (9H, s), 1.35-1.46 (2H, m), 1.80-1.94 (2H, m), 2.30-2.41 (2H, m), 2.41-2.62 (9H, m), 3.57 (1H, s), 3.76 (3H, s), 3.87 (2H, t, J=6.4Hz), 3.99 (1H, d, J=14.6Hz), 5.85 (1H, d, J=14.6Hz), 6.16 (1H, s), 6.39 (1H, dd, J=2.2, 21.8Hz), 6.62-6.75 (1H, m), 6.77 (1H, d, J=6.7Hz), 6.87 (1H, d, J=6.7Hz), 6.97-7.12 (2H, m), 7.23 (1H, s) |
| 439 | Me | H | H | CH₂CH₂C(CH₃)₃ | (ESI)⁺: [M+H] += 545.4 | 0.88 (9H, s), 1.38-1.53 (2H, m), 1.82-1.96 (2H, m), 1.96-2.06 (3H, s), 2.53-2.69 (4H, m), 2.69-2.80 (2H, m), 2.80-2.96 (2H, m), 3.73 (3H, s), 3.82-3.93 (2H, m), 5.90 (1H, d, J=14.3Hz), 6.37 (1H, s), 6.46 (1H, d, J=7.7Hz), 6.69 (2H, s), 6.90-7.01 (2H, m), 7.02-7.13 (2H, m), 7.14-7.27 (2H, m), 7.83-8.13 (3H, m) |

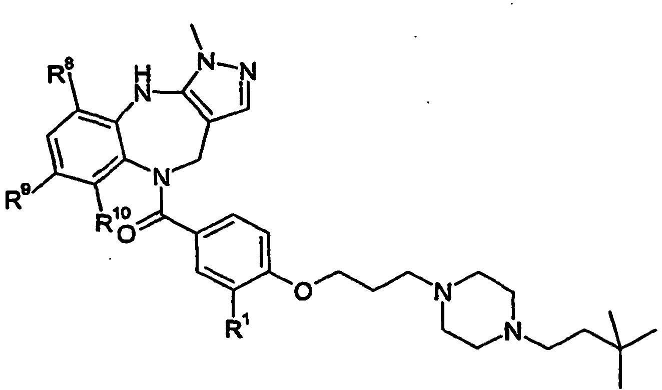

| **Compound number** | **R¹** | **R⁸** | **R⁹** | **R¹⁰** | **MS** | **¹H NMR: δ (ppm)** |
|---|---|---|---|---|---|---|
| 440 | Me | H | H | Me | (APCI)+: [M+H]+= 559.6 | 0.88 (9H, s), 1.35-1.50 (2H, m), 1.60-1.85 (2H, m), 1.90-2.05 (8H, m), 2.35-2.40 (2H, m), 2.40-2.70 (8H, m), 3.79 (3H, s), 3.85-3.95 (2H, m), 4.00-4.10 (2H, m), 5.85-5.96 (1H, m), 6.41-7-28 (7H, m) |
| 441 | Me | H | Me | H | (APCI)+: [M+H]+= 559.6 | 0.88(9H, s), 1.36-1.42 (2H, m), 1.60-1.70 (2H, m), 1.85-2.15 (10H, m), 2.25-2.60 (8H, m), 3.77 (3H, s), 3.90-3.92 (3H, m), 5.80-6.00 (2H, m), 6.45-6.55 (2H, m), 6.75-7.22 (5H, m) |
| 442 | F | Me | H | H | (ESI)+: [M+H]+= 563.4 | 0.92 (9H, s), 1.49-1.56 (2H, m), 1.85-2.03 (2H, m), 2.41 (3H, s), 2.50-2.97 (13H, m), 3.83 (3H, s), 3.88-4.03 (3H, m), 5.78-5.99 (2H, m), 6.57-6.75 (3H, m), 6.92-7.08 (3H, m) |

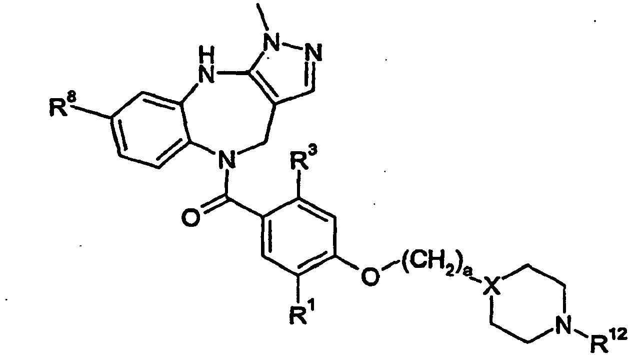

| **Compound number** | **R¹** | **R³** | **R⁸** | **R¹²** | **X** | **a** | **MS** |
|---|---|---|---|---|---|---|---|
| 443 E51 | Me | H | Me | CH₂CH(CH₃)₂ | CH | 3 | (ESI)+: [M+H]+= 530.5 |
| 444 | Me | H | Me | CH₂CH₂CH₃ | CH | 3 | (ESI)+: [M+H]+= 516.5 |
| 445 | Me | H | Me | 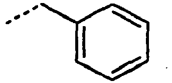 | CH | 3 | (ESI)+: [M+H]+= 564.5 |
| 446 | Me | H | Me | 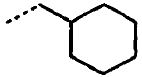 | CH | 3 | (ESI)+: [M+H]+= 570.5 |
| 447 | Me | H | Me | 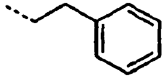 | CH | 3 | (ESI)+: [M+H]+= 578.5 |
| 448 | Me | H | Me | 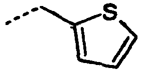 | CH | 3 | (ESI)+: [M+H]+= 570.5 |
| 449 | Me | H | Me | 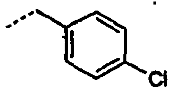 | CH | 3 | (ESI)+: [M+H]+= 598.4 |
| 450 | Me | H | Me | 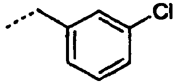 | CH | 3 | (ESI)+: [M+H]+= 598.5 |
| 451 | Me | H | Me | 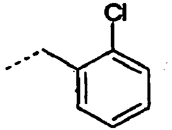 | CH | 3 | (ESI)+: [M+H]+= 598.5 |
| 452 | Me | H | Me | CH₂CH₂CH₂CH₂CH₃ | CH | 3 | (ESI)+: [M+H]+= 544.5 |
| 453 | Me | H | Me | 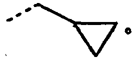 | CH | 3 | (ESI)+: [M+H]+= 528.5 |
| 454 | Me | H | Me | 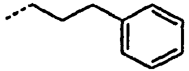 | CH | 3 | (ESI)+: [M+H]+= 592.5 |
| 455 | Me | H | Me | CH₂CH₂CH(CH₃)₂ | CH | 3 | (ESI)+: [M+H]+= 544.5 |
| 456 | Me | H | Me | 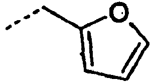 | CH | 3 | (ESI)+: [M+H]+= 554.4 |
| 457 | Me | H | Me | 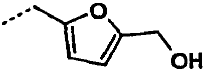 | CH | 3 | (ESI)+: [M+H]+= 584.5 |
| 458 | Me | H | Me | 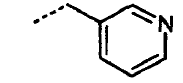 | CH | 3 | (ESI)+: [M+H]+= 565.5 |
| 459 | Me | H | Me | 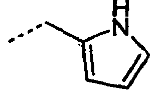 | CH | 3 | (ESI)+: [M+H]+= 553.4 |
| 460 | Me | H | Me | 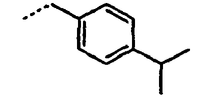 | CH | 3 | (ESI)+: [M+H]+= 606.6 |
| 461 | Me | H | Me | 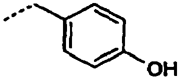 | CH | 3 | (ESI)+: [M+H]+= 580.5 |
| 462 | Me | H | Me | 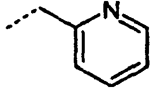 | CH | 3 | (ESI)+: [M+H]+= 565.4 |
| 463 | Me | H | Me | 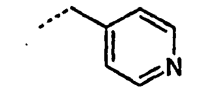 | CH | 3 | (ESI)+: [M+H]+= 565.4 |
| 464 | Me | H | Me | 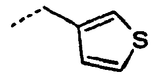 | CH | 3 | (ESI)+: [M+H]+= 570.4 |
| 465 | Me | H | Me | CH₂CH₂CF₃ | CH | 3 | (ESI)+: [M+H]+= 570.4 |
| 466 | Me | H | Me | 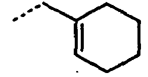 | CH | 3 | (ESI)+: [M+H]+= 568.5 |
| 467 | Me | H | Me | 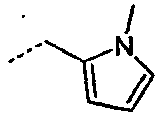 | CH | 3 | (ESI)+: [M+H]+= 567.5 |
| 468 | Me | H | Me | 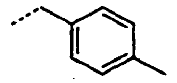 | CH | 3 | (ESI)+: [M+H]+= 578.5 |
| 469 | Me | H | Me | 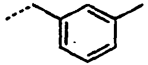 | CH | 3 | (ESI)+: [M+H]+= 578.5 |
| 470 | Me | H | Me | 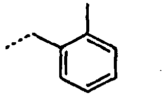 | CH | 3 | (ESI)+: [M+H]+= 578.5 |
| 471 | Me | H | Me | 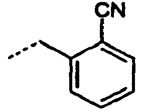 | CH | 3 | (ESI)+: [M+H]+= 589.5 |
| 472 | Me | H | Me | 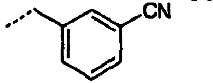 | CH | 3 | (ESI)+: [M+H]+= 589.5 |
| 473 | Me | H | Me | 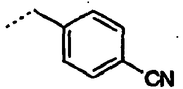 | CH | 3 | (ESI)+: [M+H]+= 589.5 |
| 474 | Me | H | Me | 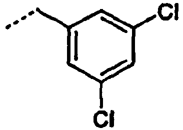 | CH | 3 | (ESI)+: [M+H]+= 632.5 |
| 475 | Me | H | Me | 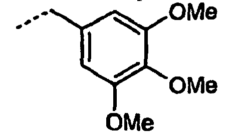 | CH | 3 | (ESI)+: [M+H]+= 654.6 |
| 476 | Me | H | Me | 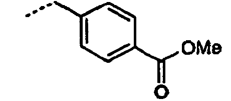 | CH | 3 | (ESI)+: [M+H]+= 622.6 |
| 477 | Me | H | Me | CH₂CH(CH₃)₂ | N | 2 | (ESI)+: [M+H]+= 517.5 |
| 478 | Me | H | Me | CH₂CH₂CH₃ | N | 2 | (ESI)+: [M+H]+= 503.5 |
| 479 | Me | H | Me | 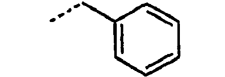 | N | 2 | (ESI)+: [M+H]+= 551.4 |
| 480 | Me | H | Me | 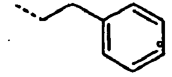 | N | 2 | (ESI)+: [M+H]+= 565.5 |
| 481 | Me | H | Me | 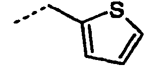 | N | 2 | (ESI)+: [M+H]+= 557.3 |
| 482 | Me | H | Me | 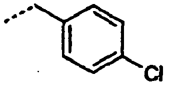 | N | 2 | (ESI)+: [M+H]+= 585.4 |
| 483 | Me | H | Me | 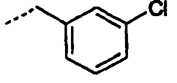 | N | 2 | (ESI)+: [M+H]+= 585.4 |
| 484 | Me | H | Me | 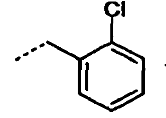 | N | 2 | (ESI)+: [M+H]+= 585.4 |
| 485 | Me | H | Me | CH₂CH₂CH₂CH₂CH₃ | N | 2 | (ESI)+: [M+H]+= 531.4 |
| 486 | Me | H | Me | 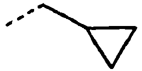 | N | 2 | (ESI)+: [M+H] = 515.5 |
| 487 | Me | H | Me | 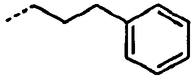 | N | 2 | (ESI)+: [M+H]+= 579.5 |
| 488 | Me | H | Me | CH₂CH₂CH(CH₃)₂ | N | 2 | (ESI)+: [M+H]+= 531.5 |
| 489 | Me | H | Me | CH₂C(CH₃)₃ | N | 2 | (ESI)+: [M+H]+= 531.4 |
| 490 | Me | H | Me | 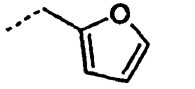 | N | 2 | (ESI)+: [M+H]+= 541.4 |
| 491 | Me | H | Me | 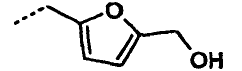 | N | 2 | (ESI)+: [M+H]+= 571.5 |
| 492 | Me | H | Me | 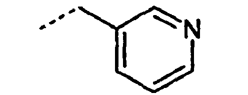 | N | 2 | (ESI)+: [M+H]+= 552.4 |
| 493 | Me | H | Me | 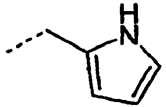 | N | 2 | (ESI)+: [M+H]+= 540.4 |
| 494 | Me | H | Me | 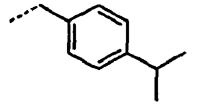 | N | 2 | (ESI)+: [M+H]+= 593.5 |
| 495 | Me | H | Me | 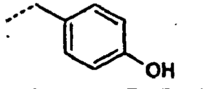 | N | 2 | (ESI)+: [M+H]+= 567.4 |
| 496 | Me | H | Me | 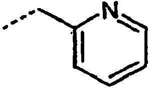 | N | 2 | (ESI)+: [M+H]+= 552.4 |
| 497 | Me | H | Me | 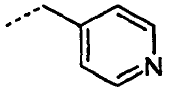 | N | 2 | (ESI)+: [M+H]+= 552.5 |
| 498 | Me | H | Me | 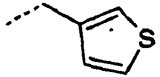 | N | 2 | (ESI)+: [M+H]+= 557.4 |
| 499 | Me | H | Me | CH₂CH₂CF₃ | N | 2 | (ESI)+: [M+H]+= 557.4 |
| 500 | Me | H | Me | 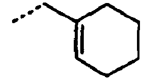 | N | 2 | (ESI)+: [M+H]+= 555.5 |
| 501 | Me | H | Me | 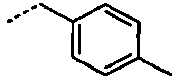 | N | 2 | (ESI)+: [M+H]+= |
| 502 | Me | H | Me | 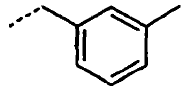 | N | 2 | (ESI)+: [M+H]+= 565.4 |
| 503 | Me | H | Me | 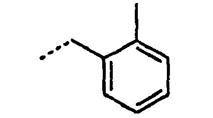 | N | 2 | (ESI)+: [M+H]+= 565.4 |
| 504 | Me | H | Me | 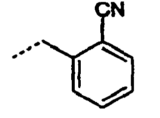 | N | 2 | (ESI)+: [M+H]+= 576.5 |
| 505 | Me | H | Me | 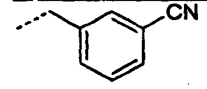 | N | 2 | (ESI)+: [M+H]+= 576.4 |
| 506 | Me | H | Me | 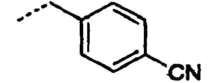 | N | 2 | (ESI)+: [M+H]+= 576.4 |
| .. 507 | Me | H | Me | 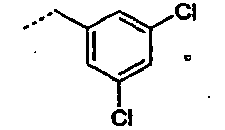 | N | 2 | (ESI)+: [M+H]+= 619.4 |
| 508 | Me | H | Me | 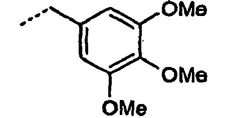 | N | 2 | (ESt)+: [M+H]+= 641.5 |
| 509 | Me | H | Me | 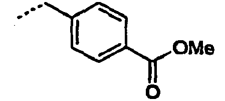 | N | 2 | (ESI)+: [M+H]+= 609.5 |
| 510 | H | F | H | CH₂CH(CH₃)₂ | N | 2 | (ESI)+: [M+H]+= 507.4 |
| 511 | H | F | H | CH₂CH₂CH₃ | N | 2 | (ESI)+: [M+H]+= 493.4 |
| 512 | H | F | H | 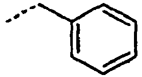 | N | 2 | (ESI)+: [M+H]+= 541.4 |
| 513 | H | F | H | 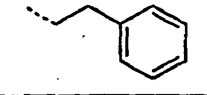 | N | 2 | (ESI)+: [M+H]+= 555.4 |
| 514 | H | F | H | 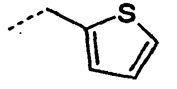 | N | 2 | (ESI)+: [M+H]+= 547.3 |
| 515 | H | F | H | 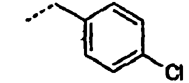 | N | 2 | (ESI)+: [M+H]+= 575.3 |
| 516 | H | F | H | 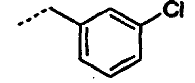 | N | 2 | (ESI)+: [M+H]+= 575.3 |
| 517 | H | F | H | 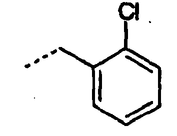 | N | 2 | (ESI)+: [M+H]+= 575.3 |
| 518 | H | F | H | CH₂CH₂CH₂CH₂CH₃ | N | 2 | (ESI)+: [M+H]+= 521.4 |
| 519 | H | F | H | 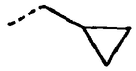 | N | 2 | (ESI)+: [M+H]+= 505.4 |
| 520 | H | F | H | 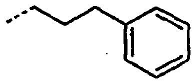 | N | 2 | (ESI)+: [M+H]+= 569.4 |
| 521 | H | F | H | 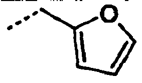 | N | 2 | (ESI)+: [M+H]+= 531.3 |
| 522 | H | F | H | 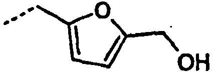 | N | 2 | (ESI)+: [M+H]+= 561.4 |
| 523 | H | F | H | 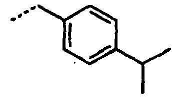 | N . | 2 | (ESI)+: [M+H]+= 583.4 |
| 524 | H | F | H | 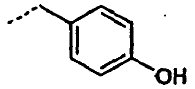 | N | 2 | (ESI)+: [M+H]+= 557.3 |
| 525 | H | F | H | 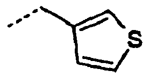 | N | 2 | (ESI)+: [M+H]+= 547.3 |
| 526 | H | F | H | CH₂CH₂O | N | 2 | (ESI)+: [M+H]+= 495.3 |
| 527 | H | F | H | 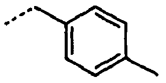 | N | 2 | (ESI)+: [M+H]+= 555.3 |
| 528 | H | F | H | 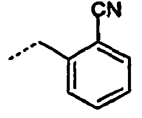 | N | 2 | (ESI)+: [M+H]+= 566.3 |
| 529 | H | F | H | 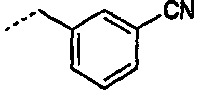 | N | 2 | (ESI)+: [M+H]+= 566.4 |
| 530 | H | F | H | 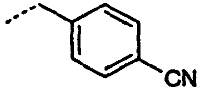 | N | 2 | (ESI)+: [M+H]+= 566.3 |
| 531 | H | F | H | 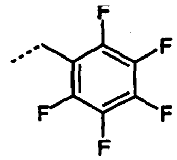 | N | 2 | (ESI)+: [M+H]+= 629.4 |
| 532 | H | F | H | 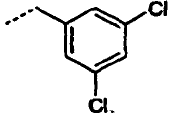 | N | 2 | (ESI)+: [M+H]+= 609.3 |
| 533 | H | F | H | 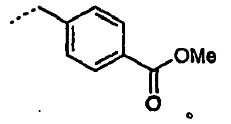 | N | 2 | (ESI)+: [M+H]+= 599.4 |

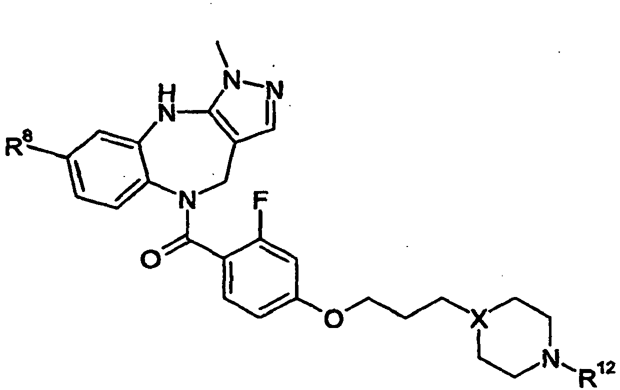

| **Compound number** | **R⁸** | **R¹²** | **X** | **MS** |
|---|---|---|---|---|
| 534 E52 | Cl | CH₂CH₂CH₂CH₂CH₂CH₃ | N | (ESI)+: [M+H]+= 583.6 |
| 535 | Cl | CH₂(CH₂)₂CH₃ | N | (ESI)+: [M+H]+= 555.6 |
| 536 | Cl | CH₂(CH₂)₂CN | N | (ESI)+: [M+H]+= 566.5 |
| 537 | Cl | 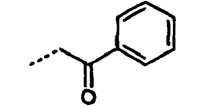 | N | (ESI)+: [M+H]+= 617.7 |
| 538 | Cl | 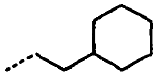 | N | (ESI)+: [M+H]+= 609.8 |
| 539 | Cl | 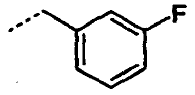 | N | (ESI)+: [M+H]+= 607.7 |
| 540 | Cl | 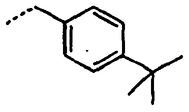 | N | (ESI)+: [M+H]+= 645.8 |
| 541 | Cl | 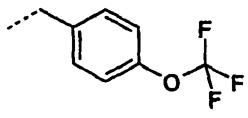 | N | (ESI)+: [M+H]+= 673.7 |
| 542 | Cl | 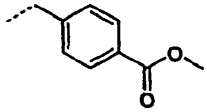 | N | (ESI)+: [M+H]+=647.7 |
| 543 | Cl | 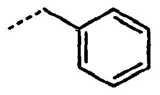 | N | (ESI)+: [M+H]+= 589.6 |
| 544 | Cl | 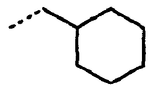 | N | (ESI)+: [M+H]+= 595.6 |
| 545 | Cl | CH₂CO₂CH(CH₃)₂ | N | (ESI)+: [M+H]+= 599.7 |
| 546 | Cl | CH₂CH₂CH(CH₃)₂ | N | (ESI)+: [M+H]+= 569.6 |
| 547 | Cl | CH₂CH₂CN | N | (ESI)+: [M+H]+= 552.5 |
| 548 | Cl | CH₂CH₂CH₂OH | N | (ESI)+: [M+H]+= 557.6 |
| 549 | Cl | CH₂CN | N | (ESI)+: [M+H]+= 538.5 |
| 550 | Cl | CH₂CH₂OCH₂CH₃ | N | (ESI)+: [M+H]+= 571.5 .. |
| 551 | Cl | CH₂CH₂CH₂F | N | (ESI)+: [M+H]+= 559.6 |
| 552 | Cl | 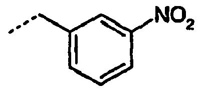 | N | (ESI)+: [M+H]+= 634.8 |
| 553 | Cl | 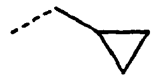 | N | (ESI)+: [M+H]+= 553.5 |
| 554 | Cl | 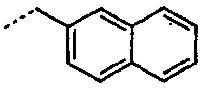 | N | (ESI)+: [M+H]+= 639.8 |
| 555 | Cl | 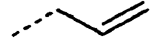 | N | (ESI)+: [M+H]+= 539.6 |
| 556 | Cl | 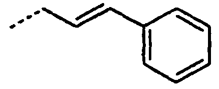 | N | (ESI)+: [M+H]+= 615.7 |
| 557 | Cl | 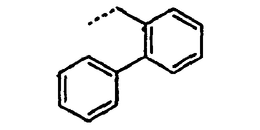 | N | (ESI)+: [M+H]+= 665.6 |
| 558 | Cl | 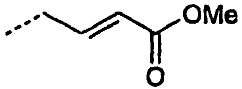 | N | (ESI)+: [M+H]+= 597.6 |
| 559 | CI | 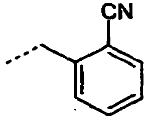 | N | (ESI)+: [M+H]+= 614.7 |
| 560 | Cl | CH₂COC(CH₃)₃ | N | (ESI)+: [M+H]+= 597.6 |
| 561 | Cl | CH₂CONH₂ | N | (ESI)+: [M+H]+= 556.5 |
| 562 | Cl | 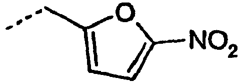 | N | (ESI)+: [M+H]+= 624.6 |
| 563 | Cl | 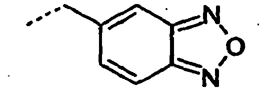 | N | (ESI)+: [M+H]+= 631.6 |
| 564 | H | CH₂(CH₂)₄CH₃ | CH | (ESI)+: [M+H]+= 548.6 |
| 565 | H | CH₂(CH₂)₂CH₃ | CH | (ESI)+: [M+H]+= 520.6 |
| 566 | H | CH₂(CH₂)₃OH | CH | (ESI)+: [M+H]+= 536.6 |
| 567 | H | 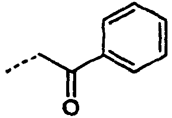 | CH | (ESI)+: [M+H]+= 582.5 |
| 568 | H | 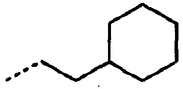 | CH | (ESI)+: [M+H]+= 574.6 |
| 569 | H | 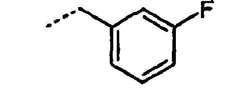 | CH | (ESI)+: [M+H]+= 672.5 |
| 570 | H | 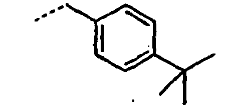 | CH | (ESI)+: [M+H]+= 610.7 |
| 571 | H | 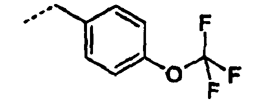 | CH | (ESI)+: [M+H]+= 638.7 |
| 572 | H | 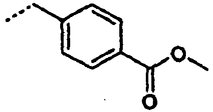 | CH | (ESI)+: [M+H]+= 612.6 |
| 573 | H | 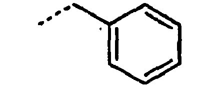 | CH | (ESI)+: [M+H]+= 554.5 |
| 574 | H | 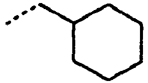 | CH | (ESI)+: [M+H]+= 560.1 |
| 575 | H | CH₂CO₂CH(CH₃)₂ | CH | (ESI)+: [M+H]+= 564.6 |
| 576 | H | CH_{z}CH₂CH(CH₃)₂ | CH | (ESI)+: [M+H]+= 534.6 |
| 577 | H | CH₂CH₂CN | CH | (ESI)+: [M+H]+= 517.5 |
| 578 | H | CH₂CN | CH | (ESI)+: [M+H]+= 503.5 |
| 579 | H | CH₂CH₂OCH₂CH | CH | (ESI)+: [M+H]+= 536.6 |
| 580 | H | CH₂CH₂CH₂F | CH | (ESI)+: [M+H]+= 524.6 |
| 581 | H | 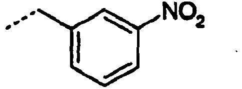 | CH | (ESI)+: [M+H]+= 599.6 |
| 582 | H | 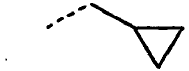 | CH | (ESI)+: [M+H]+= 518.6 |
| 583 | H | 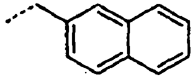 | CH | (ESI)+: [M+H]+= 604.6 |
| 584 | H | 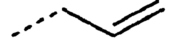 | CH | (ESI)+: [M+H]+= 504.5 |
| 585 | H | 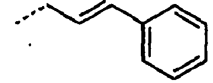 | CH | (ESI)+: [M+H]+= 580.5 |
| 586 | H | 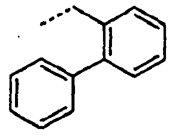 | CH | (ESI)+: [M+H]+=630.7 |
| 587 | H | 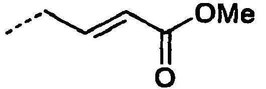 | CH | (ESI)+: [M+H]+= 562.5 |
| 588 | H | 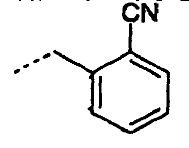 | CH | (ESI)+: [M+H]+= 579.5 |
| 589 | H | CH₂COC(CH₃)₃ | CH | (ESI)+: [M+H]+= 562.6 |
| 590 | H | CH₂CONH₂ | CH | (ESI)+: [M+H]+= 521:5 |
| 591 | H | 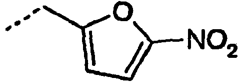 | CH | (ESI)+: [M+H]+= 589.5 |
| 592 | H | 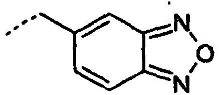 | CH | (ESI)+: [M+H]+= 596.6 |

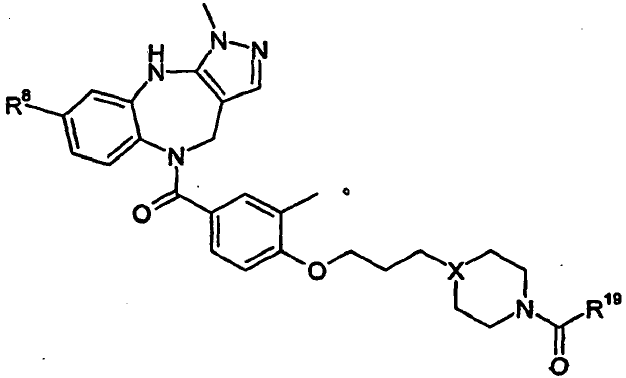

| **Compound number** | **X** | **R⁸** | **R¹⁹** | **MS** |
|---|---|---|---|---|
| 593 E53 | N | H | Me | (ESI)+: [M+H]+= 503.2 |
| 594 | N | H | 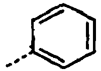 | (ESI)+: [M+H]+= 565.3 |
| 595 | N | H | 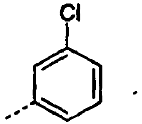 | (ESI)+: [M+H]+= 599.3 - |
| 596 | N | I H | 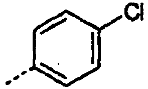 | (ESI)+: [M+H]+= 599.3 |
| 597 | N | H | 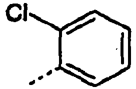 | (ESI)+: [M+H]+= 599.3 |
| 598 | N | H | 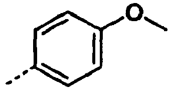 | (ESI)+: [M+H]+= 595.3 |
| 599 | N | H | 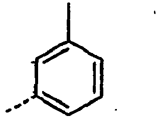 | (ESI)+: [M+H]+= 579.3 |
| 600 | N | H | 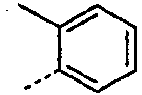 | (ESI)+: [M+H]+= 579.3 |
| 601 | N | H | 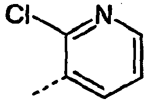 | (ESI)+: [M+H]+= 600.3 |
| 602 | N | H | 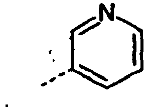 | (ESI)+: [M+H]+= 566.3 |
| 603 | N | H | 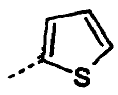 | (ESI)+: [M+H]+= 571.3 |
| 604 | N | H | 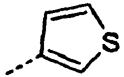 | (ESI)+: [M+H]+= 571.3 |
| 605 | N | H | 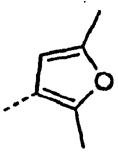 | 5ESI)+:[M+H]+= 583.3 |
| 606 | N | H | 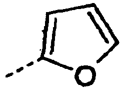 | (ESI)+: [M+H]+= 555.3 |
| 607 | N | H | 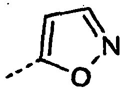 | (ESI)+: [M+H]+= 556.3 |
| 608 | N | H | 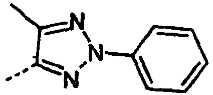 | (ESI)+: [M+H]+= 646.4 |
| 609 | N | H | CH₂CH₃ | (ESI)+: [M+H]+= 517.3 |
| 610 | N | H | CH₂CH₂CH₃ | (ESI)+:[M+H]+=531.3 |
| 611 | N | H | 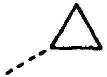 | (ESI)+: [M+H]+= 529.3 |
| 612 | N | H | C(CH₃)₃ | (ESI)+: [M+H]+= 545.3 |
| 613 | N | H | 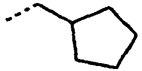 | (ESI)+: [M+H]+= 571.3 |
| 614 | N | H | CH₂(CH₂)₃CH₃ | (ESI)+: [M+H]+= 559.3 |
| 615 | N | H | CH(CH₂CH₃)CH₂CH₂C H₂CH₃ | (ESI)+: [M+H]+= 587.4 |
| 616 | N | H | 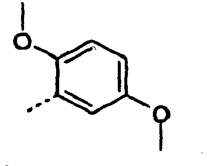 | (ESI)+: [M+H]+= 639.4 |
| 617 | N | H | 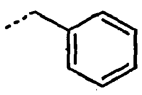 | (ESI)+: [M+H]+= 579.3 |
| 618 | N | H | 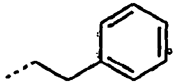 | (ESI)+: [M+H]+= 593.3 |
| 619 | N | H | 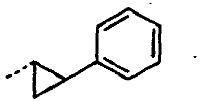 | (ESI)+: [M+H]+= 605.4 |
| 620 | N | H | 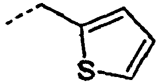 | (ESI)+: [M+H]+= 585.3 |
| 621 | N | H | 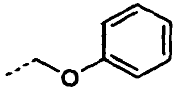 | (ESI)+: [M+H]+= 595.3 |
| 622 | N | H | 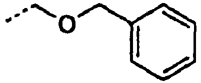 | (ESI)+: [M+H]+= 609.4 |
| 623 | N | H | CH₂CH₂CO₂CH₃ | (ESI)+: [M+H]+= 575.3 |
| 624 | N | H | CH₂CH₂CH₂CO₂CH₂C | (ESI)+: [M+H]+=603.4 |
| 625 | CH | Me | CH₃ | (ESI)+:[M+H]+= 516.4 |
| 626 | CH | Me | 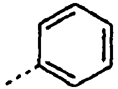 | (ESI)+: [M+H]+= 578.4 |
| 627 | CH | Me | 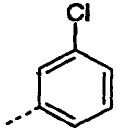 | (ESI)+: [M+H]+= 612.4 |
| 628 | CH | Me . | 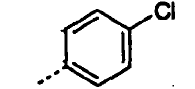 | (ESI)+: [M+H]+= 612.4 |
| 629 | CH | Me | 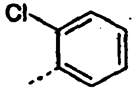 | (ESI)+: [M+H]+= 612.4 |
| 630 | CH | Me | 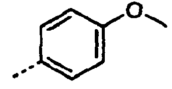 | (ESI)+: [M+H]+= 608.5 |
| 631 | CH | Me | 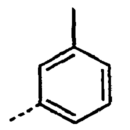 | (ESI)+: [M+H]+= 592.4 |
| 632 | CH | Me | 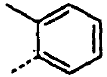 | (ESI)+: [M+H]+= 592.4 |
| 633 | CH | Me | 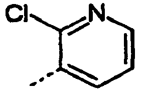 | (ESI)+: [M+H]+= 613.4 |
| 634 | CH | Me | 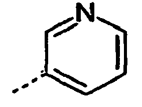 | (ESI)+: [M+H]+= 579.4 |
| 635 | CH | Me | 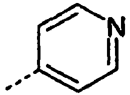 | (ESI)+: [M+H]+= 579.4 |
| 636 | CH | Me | 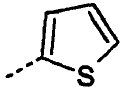 | (ESI)+: [M+H]+= 584.4 |
| 637 | CH | Me | 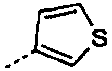 | (ESI)+: [M+H]+= 584.4 |
| 638 | CH | Me | 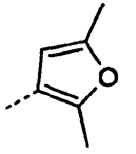 | (ESI)+: [M+H]+= 596.4 |
| 639 | CH | Me | 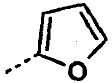 | (ESI)+: [M+H]+= 568.4 |
| 640 | CH | Me | 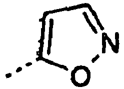 | (ESI)+: [M+H]+= 569.4 |
| 641 | CH | Me | 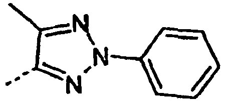 | (ESI)+: [M+H]+= 659.5 |
| 642 | CH | Me | CH₂CH₂CH₃ | (ESI)+: [M+H]+= 544.4 |
| 643 | CH | Me | 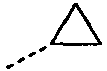 | (ESI)+: [M+H]+= 542.4 |
| 644 | CH | Me | C(CH₃)₃ | (ESI)+: [M+H]+= 558.4 |
| 645 | CH | Me | 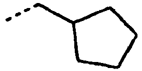 | (ESI)+: [M+H]+= 584.4 |
| 646 | CH | Me | CH₂(CH₂)₃CH₃ | (ESI)+: [M+H]+= 572.4 |
| 647 | CH | Me | CH(CH₂CH₃)CH₂CH₂C H₂CH₃ | (ESI)+: [M+H]+= 600.5 |
| 648 | CH | Me | 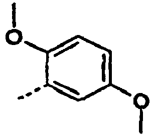 | (ESI)+: [M+H]+= 652.5 |
| 649 | CH | Me | 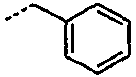 | (ESI)+: [M+H]+= 592.5 |
| 650 | CH | Me | 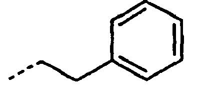 | (ESI)+: [M+H]+= 606.5 .. |
| 651 | CH | Me | 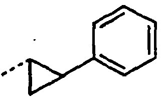 | (ESI)+: [M+H]+= 618.5 |
| 652 | CH | Me | 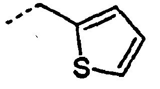 | (ESI)+: [M+H]+= 598.4 |
| 653 | CH | Me | 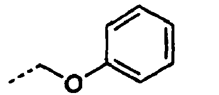 | (ESI)+: [M+H]+= 608.5 |
| 654 | CH | Me | 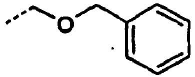 | (ESI)+: [M+H]+= 622.5 |
| 655 | CH | Me | CH₂CH₂CH₂CO₂CH₂C H₃ | (ESI)+: [M+H]+= 616.5 |

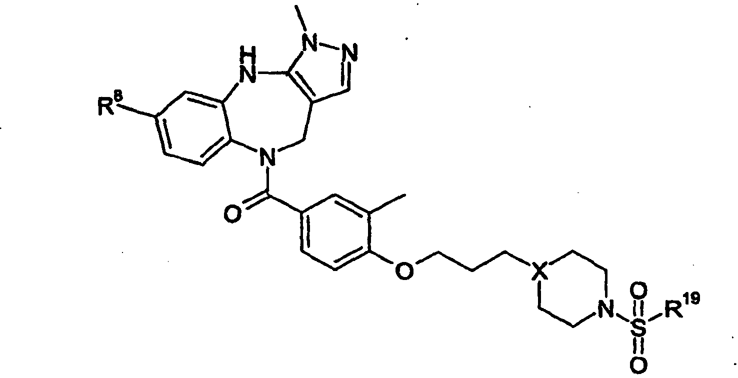

| **Compound number** | **X** | **R⁸** | **R¹⁹** | **MS** |
|---|---|---|---|---|
| 656 E54 | N | H | Me | (ESI)+: [M+H]+= 539.2 |
| 657 | N | H | CH(CH₃)₂ | (ESI)+: [M+H]+= 567.3 |
| 658 | N | H | 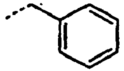 | (ESI)+: [M+H]+= 615.3 |
| 659 | N | H | 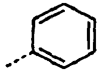 | (ESI)+: [M+H]+= 601.3 |
| 660 | N | H | 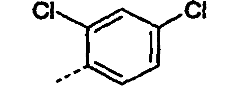 | (ESI)+: [M+H]+= 669.3 |
| 661 | N | H | 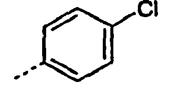 | (ESI)+: [M+H]+= 635.3 |
| 662 | N | H | 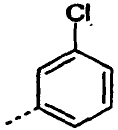 | (ESI)+: [M+H]+= 635.3 |
| 663 | N | H | 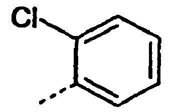 | (ESI)+: [M+H]+= 635.3 |
| 664 | N | H | 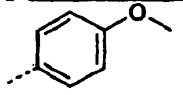 | (ESI)+: [M+H]+= 631.3 |
| 665 | N | H | 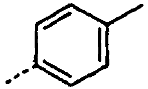 | (ESI)+: [M+H]+= 615.3 |
| 666 | N | H | 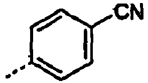 | (ESI)+: [M+H]+= 626.3 |
| 667 | N | H | 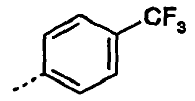 | (ESI)+: [M+H]+= 669.3 |
| 668 | N | H | 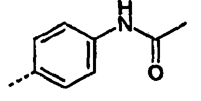 | (ESI)+: [M+H]+= 658.4 |
| 669 | N | H | 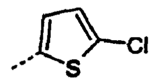 | (ESI)+: [M+H]+= 641.3 |
| 670 | N | H | 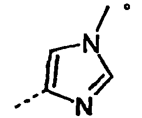 | (ESI)+: [M+H]+= 605.3 |
| 671 | N | H | CH₂CF₃ | (ESI)+: [M+H]+= 607.3 |
| 672 | N | H | 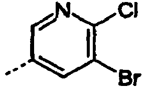 | (ESI)+: [M+H]+= 716.2 |
| 673 | N | H | 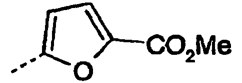 | (ESI)+: [M+H]+= 649.3 |
| 674 | N | H | CH₂CH₃ | (ESI)+: [M+H]+= 553.3 |
| 675 | N | H | CH₂CH₂CH₃ | (ESI)+: [M+H]+= 567.3 |
| 676 | N | H | CH₂CH₂CH₂CH₃ | (ESI)+: [M+H]+= 581.3 |
| 677 | CH | Me | CH₃ | (ESI)+: [M+H]+= 552.3 |
| 678 | CH | Me | CH(CH₃)₂ | (ESI)+: [M+H]+= 580.4 |
| 679 | CH | Me | 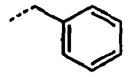 | (ESI)+: [M+H]+= 628.5 |
| 680 | CH | Me | 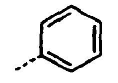 | (ESI)+: [M+H]+= 614.4 |
| 681 | CH | Me | 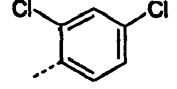 | (ESI)+: [M+H]+= 682.4 |
| 682 | CH | Me | 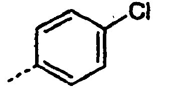 | (ESI)+: [M+H]+= 648.4 |
| 683 | CH | Me | 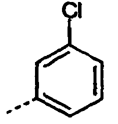 | (ESI)+: [M+H]+= 648.4 |
| 684 | CH | Me | 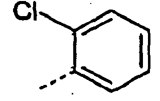 | (ESI)+: [M+H]+= 648.4 |
| 685 | CH | Me | 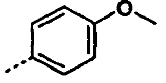 | (ESI)+: [M+H]+= 644.5 |
| 686 | CH | Me | 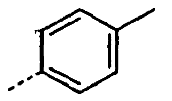 | (ESI)+: [M+H]+= 628.5 |
| 687 | CH | Me | 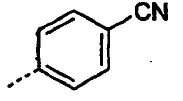 | (ESI)+: [M+H]+= 639.5 |
| 688 | CH | Me | 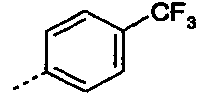 | (ESI)+: [M+H]+= 682.4 |
| 689 | CH | Me | 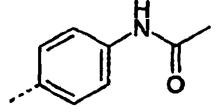 | (ESI)+: [M+H]+= 671.5 |
| 690 | CH | Me | 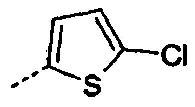 | (ESI)+: [M+H]+= 654.4 |
| 691 | CH | Me | 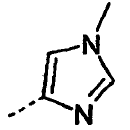 | (ESI)+: [M+H]+= 618.5 |
| 692 | CH | Me | 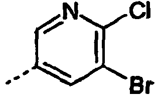 | (ESI)+: [M+H]+= 729.2 |
| 693 | CH | Me | 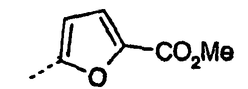 | (ESI)+: (M+H]+= 662.4 |
| 694 | CH | Me | CH₂CH₃ | (ESI)+: [M+H]+= 566.3 |
| 695 | CH | Me | CH₂CH₂CH₃ | (ESI)+: [M+H]+= 580.4 |
| 696 | CH | Me | CH₂CH₂CH₂CH₃ | (ESI)+: [M+H]+= 594.4 |

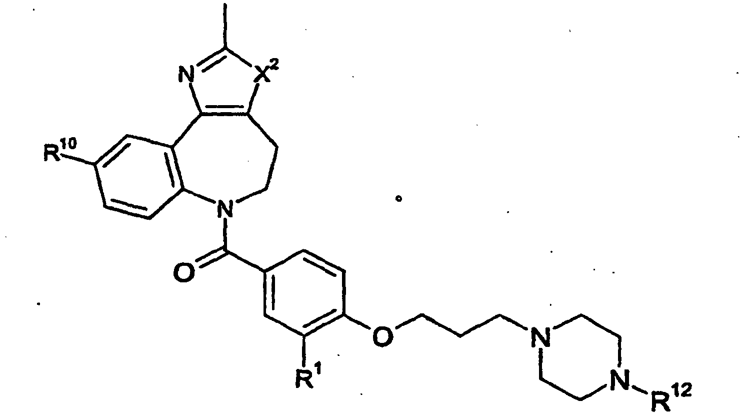

| **Compound number** | **R¹** | **R¹⁰** | **R¹²** | **X²** | **MS** | **1H NMR** |
|---|---|---|---|---|---|---|
| 697 | F | Cl | 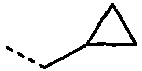 | S | (ESI)+: [M+H]+ = 569.5, 571.3 | 0.05-0.15(2H, m), 0.45-0.55 (2H, m), 0.80-0.90 (1H, m), 1.90-2.00 (2H, m), 2.24 (2H, d, J=6.4Hz), 2.35-2.70 (10H, m), 2.73 (3H, s)2.95-3.25 (2H, m), 3.45-3.55 (1 H, m), 3.99 (2H, t, J=6,4Hz), 5.15-5.25 (1H, m), 6.60-6.75 (3H, m), 6.90-7.05 (2H, m), 8.41 (1H, d, J=2.5Hz) |
| 698 | Cl | Me | 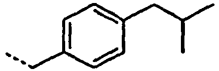 | S | (ESI)+: [M+H]+ = 643.3, 645.4 | 1.22 (6H, d, 1.22 (6H, d, J=6.9Hz), 1.87-1.99 (2H, m), 2.34-(3H, s), 2.45-2.52 (10H, m), 2.74(3H, s), 2.87 (1H, septet, J=6.9Hz), 3.03-3.18 (2H, m), 3.45 (2H, s), 3.45-3.54 (1H, m), 3.95 (2H, t, J=6.2Hz), 5.13-5.19 (1H, m), 6.52 (1H, d, J=8.6Hz), 6.57 (1H, d, J=8.2Hz), 6.75 (1H, dd, J=2.2, 8.6Hz), 6.81 (1 H, d, J=8.2Hz), 7.15 (2H, d, J=8.2Hz), 7.21 (2H, d, J=8.2Hz), 7.38 (1H, d, J=2.2Hz), 8.18 (1H, s) |
| 699 | Cl | Me | 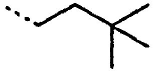 | S | (ESI)+: [M+H]+ = 595.4, 597.4 | 0.89(9H, s), 1.38-1.44 (2H, m), 1.89-1.99 (2H, m), 2.34, (3H, s), 2.35-2.41 (2H, m), 2.47-256 (10H, m), 2.73 (3H, s), 3.01-3.18 (2H, m), 3.45-3.57 (1H, m), 3.95 (2H, t, J=6.4Hz), 5.12-5.19, (1 H, m), 6.52 (1H, d, J=8.6Hz), 6.57 (1H, d, J=8.4Hz), 6.75 (1 H, dd, J=2.2, 8.4Hz), 6.80 (1 H, d, J=8.6Hz), 7.38 (1H, d, J=2.2Hz), 8.17(1H, s) |

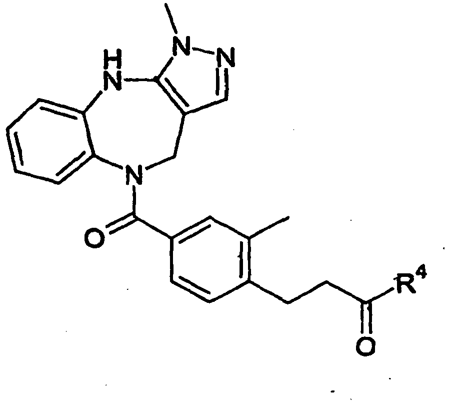

| **Compound number** | **R⁴** | | **¹H NMR: δ (ppm)** |
|---|---|---|---|
| 700 | 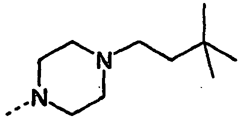 | (ESI)+: [M+H]+= 543.6 | 0.85 (9H, s), 1.30-1.41 (4H, m), 2.10 (3H, s), 2.28-2.45 (6H, m), 2.71-2.82 (2H, m), 3.25-3.34 (2H, m), 3.52-3.65 (2H, m), 3.71 (3H, s), 3.93 (1H, d, J=14.6Hz), 5.85 (1H, d, J=14.6Hz), 6.65 (1H, s), 6.75-7.01 (5H, m), 7.07-7.17(3H, m) |
| 701 | 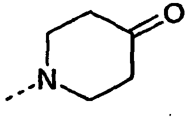 | (ESI)+: [M+H]+= 472.7 | 2.17 (3H, s), 2.29 (2H, t, J=5.7Hz), 2.40 (2H, t, J=5.7Hz), 2.53 (2H, t, J=7.2Hz), 2.87 (2H, t, J=7.2Hz), 3.52 (2H, brs), 3.79 (3H, s), 3.77-3.83 (2H, m), 3.95 (1H, d, J=14.6Hz), 5.89 (1H, d, J=14.6Hz), 6.19 (1H, s), 6.62-6.74 (2H, m), 6.81-6.94 (3H, m), 7.02-7.08 (1H, m), 7.16 (1H, s) |
| 702 | 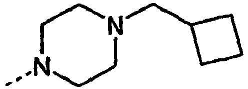 | (ESI)+: [M+H]+= 527.7 | 1.61-1.68 (2H, m), 1.68-1.91 (3H, m), 1.92-2.08 (2H, m), 2.14 (3H, s), 2.28-2.51 (4H, m), 2.72-2.89 (2H, m), 2.93-3.08 (2H, m), 3.21-3.34 (2H, m), 3.46-3.69 (4H, m), 3.80 (3H, s), 3.98 (1H, d, J=14.6Hz), 5.87 (1H, d, J=14.6Hz), 6.51 (1H, s), 6.68 (2H, s), 6.74-6.89 (2H, m), 6.94-7.08 (2H, m), 7.13 (1H, s) |
| 703 | 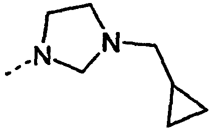 | (ESI)+: [M+H]+= 499.7 | - |
| 704 | 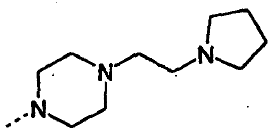 | (ESI)+: [M+H]+= 556.7 | - |
| 705 | 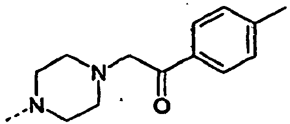 | (ESI)+: [M+H]+= 632.0 (MeCN adduct) | - |
| 706 | 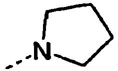 | (ESI)+: [M+H]+= 444.3 | - |
| 707 | 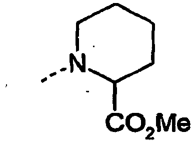 | (ESI)+: [M+H]+= 516.4 | - |
| 708 | 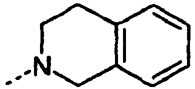 | (ESI)+: [M+H]+= 506.3 | - |
| 709 | 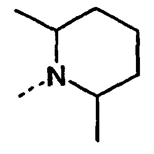 | (ESI)+: [M+H]+= 486.4 | - |
| 710 E56 | 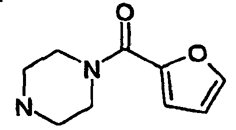 | (ESI)+: [M+H]+= 553.4 | |
| 711 | 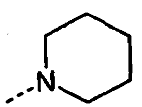 | (ESI)+: [M+H]+= 458.3 | - |
| 712 | 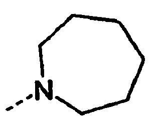 | (ESI)+: [M+H]+= 472.4 | - |
| 713 | 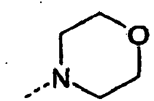 | (ESI)+: [M+H]+= 460.4 | - |
| 714 | 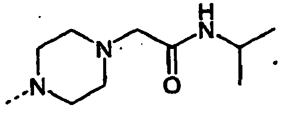 | (ESI)+: [M+H]+= 558.4 | - |
| 715 | 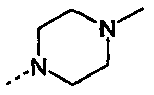 | (ESI)+: [M+H]+= 473.3 | - |
| 716 | 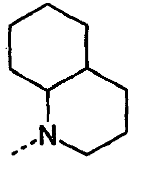 | (ESI)+: [M+H]+= 512.4 | - |
| 717 | 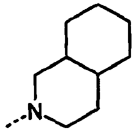 | (ESI)+: [M+H]+= 512.4 | - |
| 718 | 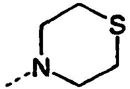 | (ESI)+: [M+H]+= 476.3 | - |
| 719 | 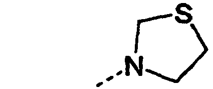 | (ESI)+: [M+H]+= 462.3 | - |
| 720 | 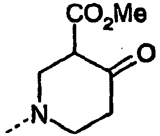 | (ESI)+: [M+H]+= 530.4 | - |
| 721 | 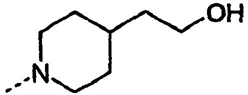 | (ESI)+: [M+H]+= 502.4 | - |
| 722 | 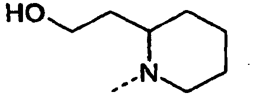 | (ESI)+: [M+H]+= 502.4 | - |
| 723 | 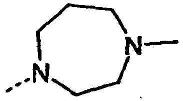 | (ESI)+: [M+H]+= 487.3 | - |
| 724 | 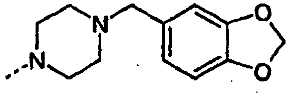 | (ESI)+: [M+H]+= 593.4 | - |
| 725 | 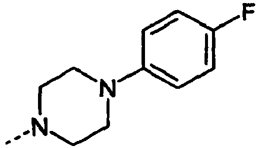 | (ESI)+: [M+H]+= 553.4 | - |
| 726 | 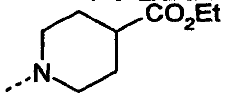 | (ESI)+: [M+H]+= 530.4 | - |
| 727 | 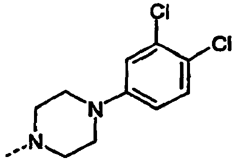 | (ESI)+: [M+H]+= 603.4 | - |
| 728 | 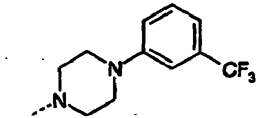 | (ESI)+: [M+H]+= 603.4 | - |
| 729 | 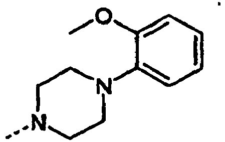 | (ESI)+: [M+H]+= 565.4 | - |
| 730 | 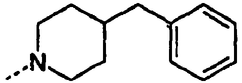 | (ESI)+: [M+H)+= 548.4 | - |
| 731 | 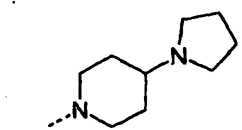 | (ESI)+: [M+H]+= 527.5 | - |
| 732 | 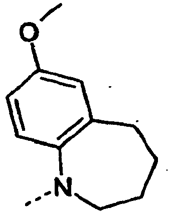 | (ESI)+: [M+H]+= 550.3 | - |
| 733 | 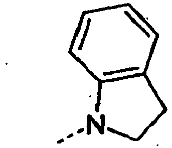 | (ESI)+: [M+H]+= 492.4 | - |
| 734 | 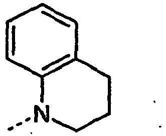 | (ESI)+: [M+H]+= 506.4 | - |
| 735 | 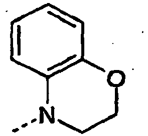 | (ESI)+: [M+H]+= 508.4 | - |
| 736 | 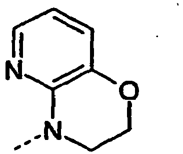 | (ESI)+: [M+H]+= 509.3 | - |
| 737 | 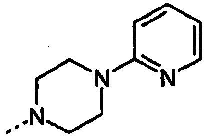 | (ESI)+: [M+H]+= 536.4 | - |
| 738 | 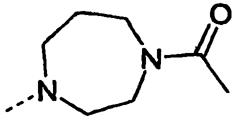 | (ESI)+: [M+H]+= 515.4 | - |
| 739 | 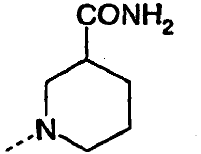 | (ESI)+: [M+H]+= 501.4 | - |
| 740 | 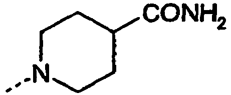 | (ESI)+: [M+H]+= 501.4 | - |
| 741 | 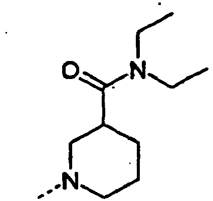 | (ESI)+: [M+H]+= 557.5 | - |
| 742 | 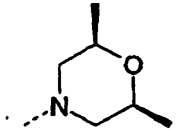 | (ESI)+: [M+H]+= 488.4 | - |

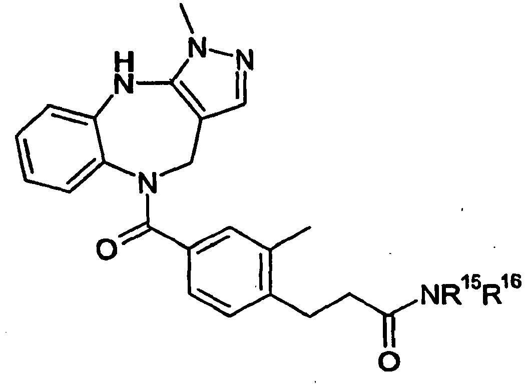

| Compound number | R¹⁵ | R¹⁶ | |
|---|---|---|---|
| 743 | H | 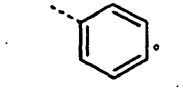 | (ESI)+: [M+H]+= 466.1 |
| 744 | H | 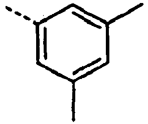 | (ESI)+: [M+H]+= 494.2 |
| 745 | H | 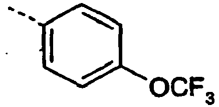 | (ESI)+: [M+H]+= 550.3 |
| 746 | H | 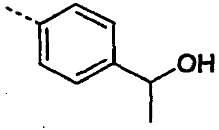 | (ESI)+: [M+H]+= 510.3 |
| 747 | H | 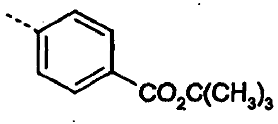 | (ESI)+: [M+H]+= 566.3 |
| 748 | H | 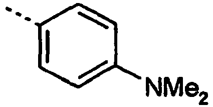 | (ESI)+: [M+H]+= 509.3 |
| 749 | H | 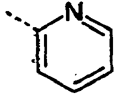 | (ESI)+: [M+H]+= 467.2 |
| 750 | H | 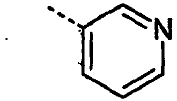 | (ESI)+: [M+H]+= 467.2 |
| 751 | H | 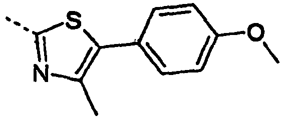 | (ESI)+: [M+H)+= 593.3 |
| 752 | H | 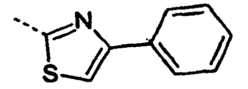 | (ESI)+: [M+H]+= 549.3 |
| 753 | H | 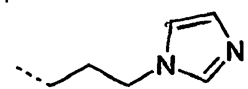 | (ESI)+: [M+H]+= 498.3 |
| 754 | H | CH₃ | (ESI)+: [M+H]+= 404.2 |
| 755 | H | CH₂CH₃ | (ESI)+: [M+H]+= 418.2 |
| 756 | H | CH₂CH(CH₃)₃ | (ESI)+: [M+H]+= 446.2 |
| 757 | H | CH₂CH₂CH₃ | (ESI)+: [M+H]+= 432.2 |
| 758 | H | 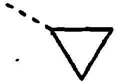 | (ESI)+: [M+H]+= 430.2 |
| 759 | H | CH₂(CH₂)₄CH₃ | (ESI)+: [M+H]+= 474.3 |
| 760 | H | 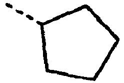 | (ESI)+: [M+H]+= 458.2 |
| 761 | H | 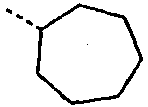 | (ESI)+: [M+H]+= 486.3 |
| 762 | H | C(CH₃)₂CH₂CH₃ | (ESI)+: [M+H]+= 460.2 |
| 763 | H | CH(CH₂CH₃)₂ | (ESI)+: [M+H]+= 460.3 |
| 764 | H | CH₂CH₂CH(CH₃)₂ | (ESI)+: [M+H]+= 460.2 |
| 765 | H | 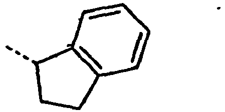 | (ESI)+: [M+H]+= 506.3 |
| 766 | H | 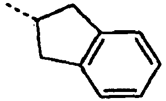 | (ESI)+: [M+H]+= 506.3 |
| 767 | H | 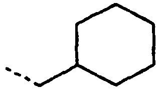 | (ESI)+: [M+H]+= 486.3 |
| 76B | H | 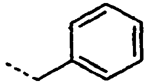 | (ESI)+: [M+H]+= 480.2 |
| 769 | H | 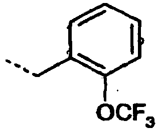 | (ESI)+: [M+H]+= 564.2 |
| 770 | H | 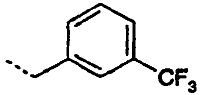 | (ESI)+: [M+H]+= 548.2 |
| 771 | H | 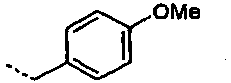 | (ESI)+: [M+H]+= 510.3 |
| 772 | H | 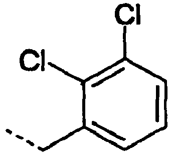 | (ESI)+: [M+H)+= 548.2 |
| 773 | H | 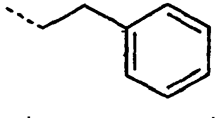 | (ESI)+: [M+H]+= 494.3 |
| 774 | H | 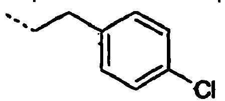 | (ESI)+: [M+H]+= 528.2 |
| 775 | H | 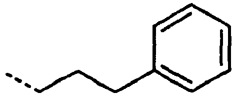 | (ESI)+: [M+H]+= 508.2 |
| 776 | H | 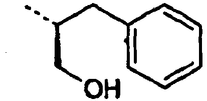 | (ESI)+: [M+H]+= 524.3 |
| 777 | H | 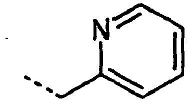 | (ESI)+: [M+H]+= 481.2 |
| 778 | H | 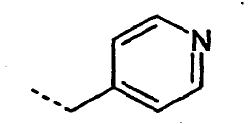 | (ESI)+: [M+H]+= 481.2 |
| 779 | H | 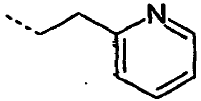 | (ESI)+: [M+H]+= 495.3 |
| 780 | H | 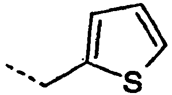 | (ESI)+: [M+H]+= 486.2 |
| 781 | H | 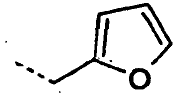 | (ESI)+: [M+H]+= 470.2 |
| 782 | H | CH₂CH₂F | (ESI)+: [M+H]+= 436.2 |
| 783 | H | CH₂CH₂OH | (ESI)+: [M+H]+= 434.2 |
| 784 | H | CH₂CH₂CH₂OH | (ESI)+: [M+H]+= 448.2 |
| 785 | H | CH₂(CH₂)₃OH | (ESI)+: [M+H]+= 462.2 |
| 786 | H | CH₂(CH₂)₄OH | (ESI)+: [M+H]+= 476.2 |
| 787 | H | 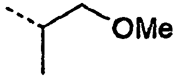 | (ESI)+: [M+H]+= 462.2 |
| 788 | H | 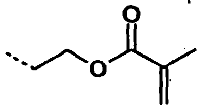 | (ESI)+: [M+H]+= 502.2 |
| 789 | H | CH₂(CH₂)₂SCH₃ | (ESI)+: [M+H]+= 478.2 |
| 790 | H | 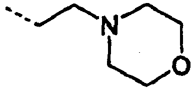 | (ESI)+: [M+H]+= 503.2 |
| 791 | H | CH₂CH₂N(CH₃)₂ | (ESI)+: [M+H]+= 461.2 |
| 792 | H | 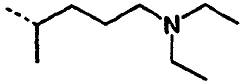 | (ESI)+: [M+H]+= 531.4 |
| 793 | H | CH₂(CH₂)₂N(CH₃)₂ | (ESI)+: [M+H]+= 475.2 |
| 794 | H | 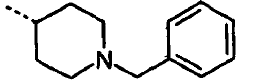 | (ESI)+: [M+H]+= 583.3 |
| 795 | H | CH₂CH₂NHCOCH₃ | (ESI)+: [M+H]+= 475.2 |
| 796 | H | 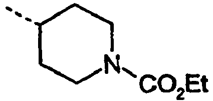 | (ESI)+: [M+H]+= 545.3 |
| 797 | H | CH₂CO₂CH₃ | (ESI)+: [M+H]+= 462.2 |
| 798 | H | 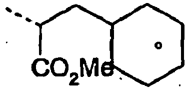 | (ESI)+: [M+H]+= 558.4 |
| 799 | H | CH₂CO₂CH(CH₃)₂ | (ESI)+: [M+H]+= 490.3 |
| 800 | H | 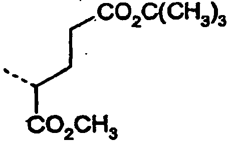 | (ESI)+: [M+H]+= 590.3 |
| 801 | H | CH₂CONH₂ | (ESI)+: [M+H]+= 447.1 |
| 802 | H | - CH₂CN | (ESI)+: [M+H]+= 429.2 |
| 803 | H | CH₂CH₂CN | (ESI)+: [M+H]+= 443.2 |
| 804 | H | CH₂(CH₂)₄CN | (ESI)+: [M+H]+= 485.3 |
| 805 | H | CH₂CF₃ | (ESI)+: [M+H]+= 472.2 |
| 806 | CH₃ | 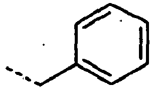 | (ESI)+: [M+H]+= 494.4 |
| 807 | CH₂CH₂ CH₃ | 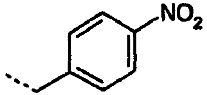 | (ESI)+: [M+H]+= 567.4 |
| 808 | CH₃ | CH₂CH₃ | (ESI)+: [M+H]+= 432.3 |
| 809 | CH₂CH₃ | 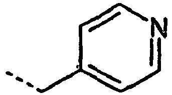 | (ESI)+: [M+H]+= 509.4 |
| 810 | CH₂CH₂ CN | 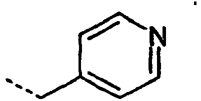 | (ESI)+: [M+H]+= 534.3 |
| 811 | CH₂CO₂ CH₂CH₃ | 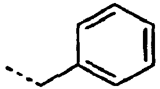 | (ESI)+: [M+H]+= 566.4 |
| 812 | CH₃ | 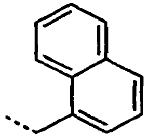 | (ESI)+: [M+H]+= 544.4 |
| 813 | CH₃ | CH₂CH₂CH₂CH₃ | (ESI)+: [M+H]+= 460.3 |
| 814 | CH₃ | CH₂(CH₂)₄CH₃ | (ESI)+: [M+H]+= 488.4 |
| 815 | CH(CH₃) 2 | 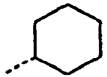 | (ESI)+: [M+H]+= 514.5 |
| 816 | CH₂CH₃ | CH₂CH₃ | (ESI)+: [M+H]+= 446.2 |
| 817 | CH(CH₃) 2 | CH(CH₃)₂ | (ESI)+: [M+H]+= 474.4 |
| 818 | CH₃ | 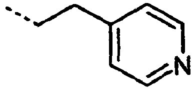 | (ESI)+: [M+H]+= 509.4 |
| 819 | CH₂CH₃ | 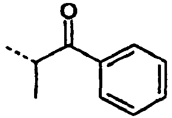 | (ESI)+: [M+H]+= 550.4 |
| 820 | CH(CH₃) 2 | CH₂CH₂OCH₃ | (ESI)+: [M+H]+= 490.4 |
| 821 | CH₃ | 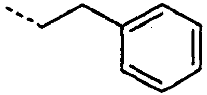 | (ESI)+: [M+H]+= 508.4 |
| 822 | CH₂CH₂ OCH₃ | CH₂CH₂OCH₃ | (ESI)+: [M+H]+= 506.3 |
| 823 | CH₃ | C₆H₅ | (ESI)+: [M+H]+= 480.3 |
| 824 | CH₂CH₂ OH | C₆H₅ | (ESI)+: [M+H]+= 510.4 |
| 825 | CH₃ | 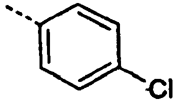 | (ESI)+: [M+H]+= 514.4 |
| 826 | CH₃ | 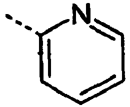 | (ESI)+: [M+H]+= 481.4 |
| 827 | CH₃ | 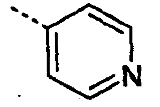 | (ESI)+: [M+H]+= 481.3 |
| 828 | CH₂CH₃ | 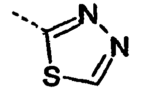 | (ESI)+: [M+H]+= 502.4 |
| 829 | CH₃ | 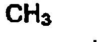 | (ESI)+: [M+H]+= 418.3 |
| 830 | CH₃ | 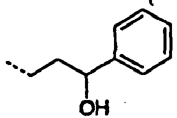 | (ESI)+: [M+H]+= 538.3 |
| 831 | CH₃ | 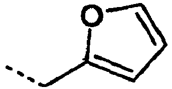 | (ESI)+: [M+H]+= 484.3 |
| 832 | CH₃ | CH₂CO₂H | (ESI)+: [M-H]+= 460.4 |
| 833 | CH₃ | CH₂CH₂CH₂CO₂H | [M+H]+= 490.4 |
| 834 | CH₃ | CH₂CN | (ESI)+: [M+H]+= 443.3 |
| 835 | CH₃ | CH₂CH₂CN | (ESI)+: [M+H]+= 457.3 |
| 836 | CH₂CH₂ N(CH₃)₂ | 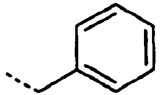 | (ESI)+: [M+H]+= 551.5 |
| 837 | CH₂CH₂ OH | 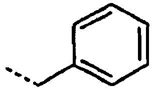 | (ESI)+: [M+H]+= 524.4 |
| 838 | CH₂CN | 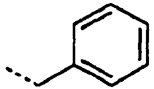 | (ESI)+: [M+H]+= 519.4 |

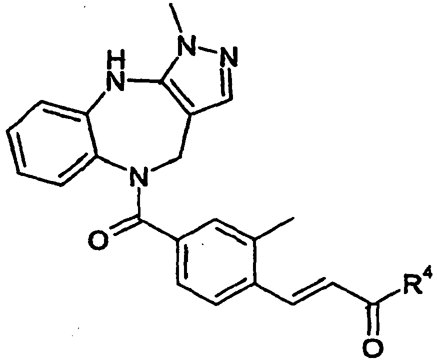

| **Compound number** | **R4** | **MS** |
|---|---|---|
| 839 E57 | 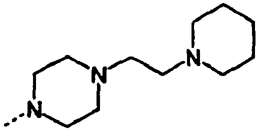 | |
| 840 | 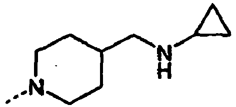 | |
| 841 | 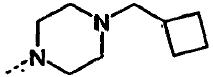 | |

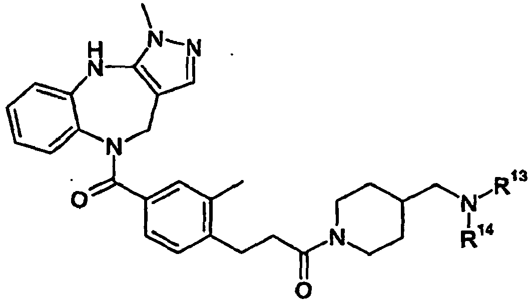

| **Compound number** | **R¹³** | **R¹⁴** | **MS** | **¹H NMR** |
|---|---|---|---|---|
| 842 E58 | Me | CH₂CH₂CH₂CH₃ | (ESI)+:[M +H]+= 557.6 | |
| 843 | H | 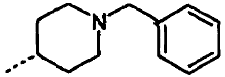 | (ESI)+: [M+H]+= 660.7 | - |
| 844 | H | CH₂C₈H₅ | (ESI)+: [M+H]+= 577.6 | - |
| 845 | H | CH₂CH₂C₈H₅ | (ESI)+: [M+H]+= 591.7 | - |
| 846 | H | 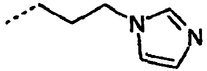 | (ESI)+: [M+H]+= 595.7 | - |
| 847 | H | 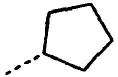 | (ESI)+: [M+H]+= 555.6 | - |
| 848 | H | CH₂CH(CH₃)₂ | (ESI)+: [M+H]+= 543.6 | - |

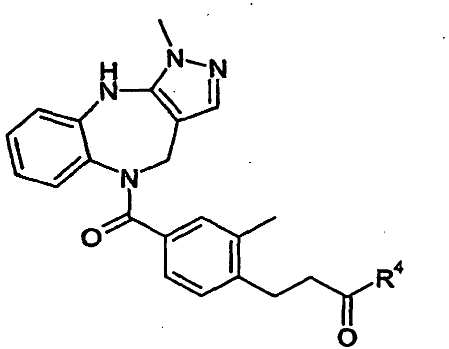

| **Compound number** | **R⁴** | **MS** | **¹H NMR** |
|---|---|---|---|
| 849 | 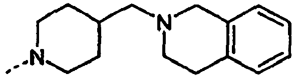 | (ESI)+: [M+H]+= 603.7 | - |
| 850 | 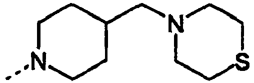 | (ESI)+: [M+H]+= 573.6 | - |

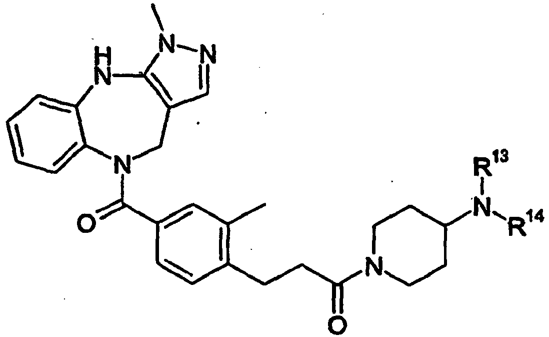

| **Compound number** | **R¹³** | **R¹⁴** | **MS** | **¹H NMR** |
|---|---|---|---|---|
| 851 E59 | H | 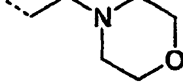 | (ESI)+:[M+ H]+= 586.6 | |
| 852 | H | CH₂C₈H₅ | (ESI)+: [M+H]+= 563.6 | - |
| 853 | H | 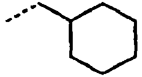 | (ESI)+: [M+H]+= 569.6 | - |
| 854 | H | 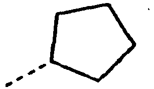 | (ESI)+: [M+H]+= 541.5 | - |
| 855 | H | CH₂CH(CH₃)₂ | (ESI)+: [M+H]+= 529.8 | - |
| 856 | CH₂C₆ H₅ | CH₂CH₂CH₂CH₃ | (ESI)+: [M+H]+= 619.6 | - |
| 857 | H | 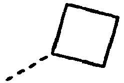 | (ESI)+: [M+H]+= 527.6 | - |
| 858 | CH₂C₆ H₅ | CH₂CH₂N(CH₃)₂ | (ESI)+: [M+H]+= 634.6 | - |
| 859 | H | 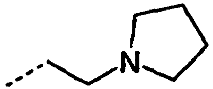 | (ESI)+: [M+H]+= 570.6 | - |
| 860 | H | CH₂CH₂OH | (ESI)+: [M+H]+= 517.6 | - |
| 861 | H | 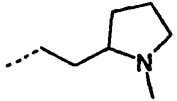 | (ESI)+: [M+H]+= 584.6 | - |
| 862 | H | CH₂CH₂CH₂OH | (ESI)+: [M+H]+= 531.6 | - |
| 863 | H | 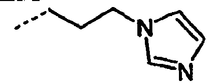 | (ESI)+: [M+H]+= 581.6 | - |
| 864 | H | CH₂CH₂C₆H₅ | (ESI)+: [M+H]+= 577.6 | - |
| 865 | H | 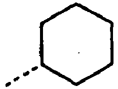 | (ESI)+: [M+H]+= 555.6 | - |
| 866 | H | CH₂(CH₂)₄CH₃ | (ESI)+: [M+H]+= 557.6 | - |
| 867 | H | 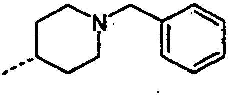 | (ESI)+: [M+H]+= 646.6 | - |

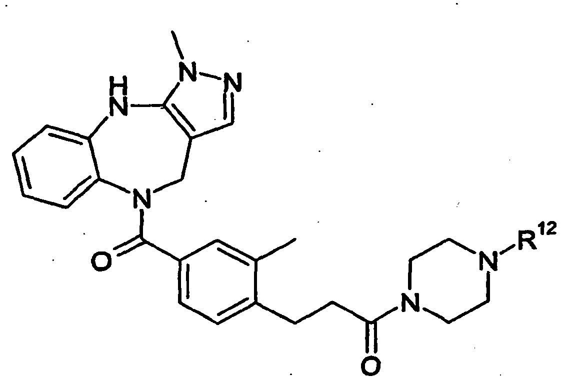

| **Compound number** | **R¹²** | **MS** |
|---|---|---|
| 868 E60 | CH₂CH₂CH₂CH₂CH₂CH₃ | (ESI)+: [M+H]+= 543.4 |
| 869 | CH₂CH₂CH₃ | (ESI)+: [M+H]+= 501.4 |
| 870 | CH₂(CH₂)₃OH | (ESI)+: [M+H]+= 531.3 |
| 871 | CH₂(CH₂)₂CN | (ESI)+: [M+H)+= 526.4 |
| 872 | 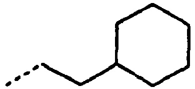 | (ESI)+: [M+H]+= 569.4 |
| 873 | 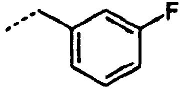 | (ESI)+: [M+H]+= 587.3 |
| 874 | 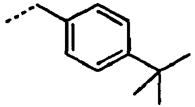 | (ESI)+: [M+H]+= 605.5 |
| 875 | 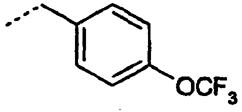 | (ESI)+: [M+H]+= 633.5 |
| 876 | 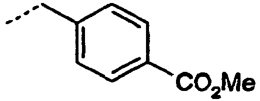 | (ESI)+: [M+H]+= 607.4 |
| 877 | 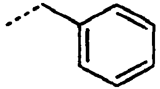 | (ESI)+: [M+H]+= 549.4 |
| 878 | CH₂CH₂OH | (ESI)+: [M+H]+= 503.3 |
| 879 | CH₂CO₂CH(CH₃)₂ | (ESI)+: [M+H]+= 559.3 |
| 880 | CH₂CH₂CH(CH₃)₂ | (ESI)+: [M+H]+= 529.4 |
| 881 | CH₂CH₂CH₂OH | (ESI)+: [M+H]+= 517.4 |
| 882 | CH₂CN | (ESI)+: [M+H]+= 498.4 |
| 883 | CH₂CH₂OCH₂CH₃ | (ESI)+: [M+H]+= 531.4 |
| 884 | 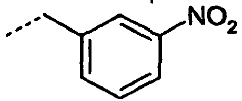 | (ESI)+: [M+H]+= 594.4 |
| 885 | 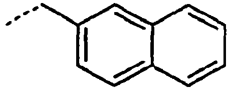 | (ESI)+: [M+H]+= 599.4 |
| 886 | 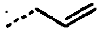 | (ESI)+: [M+H]+= 499.4 |
| 887 | 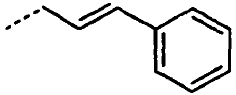 | (ESI)+: [M+H]+= 575.4 |
| 888 | 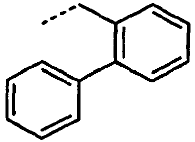 | (ESI)+: [M+H]+= 625.5 |
| 889 | CH₂COC(CH₃)₃ | (ESI)+: [M+H]+= 557.4 |
| 890 | CH₂CONH₂ | (ESI)+: [M+H]+= 516.4 |
| 891 | 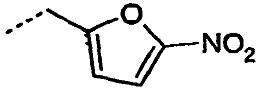 | (ESI)+: [M+H]+= 584.4 |
| 892 | 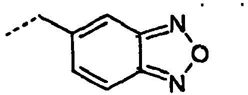 | (ESI)+: [M+H]+=591.4 |

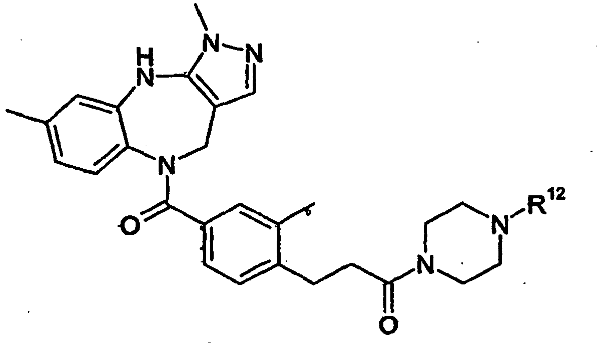

| **Compound number** | **R¹²** | **MS** |
|---|---|---|
| 893 | CH₂CH₂CH₃ | (ESI)+: [M+H]+= 515.5 |
| 894 | CH₂CH(CH₃)₂ | (ESI)+: [M+H]+= 529.5 |
| 895 | 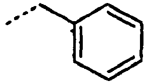 | (ESI)+: [M+H]+= 563.4 |
| 896 | 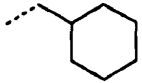 | (ESI)+: [M+H]+= 569.4 |
| 897 | 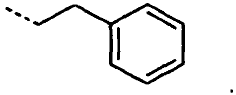 | (ESI)+: [M+H]+= 577.5 |
| 898 | 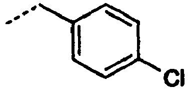 | (ESI)+: [M+H]+= 597.4 |
| 899 | 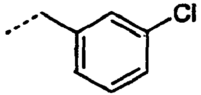 | (ESI)+: [M+H]+= 597.4 |
| 900 | 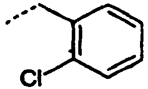 | (ESI)+: [M+H]+= 597.5 |
| 901 | CH₂CH₂CH₂CH₂CH₃ | (ESI)+: [M+H]+= 543.4 |
| 902 | 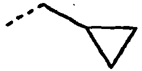 | (ESI)+: [M+H]+= 527.4 |
| 903 | 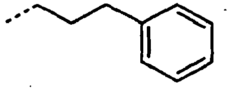 | (ESI)+: [M+H]+= 591.5 |
| 904 | CH₂CH₂CH(CH₃)₂ | (ESI)+: [M+H]+= 543.5 |
| 905 | 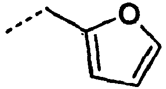 | (ESI)+: [M+H]+= 553.4 |
| 906 | 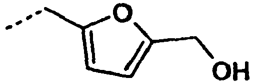 | (ESI)+: [M+H]+= 583.5 |
| 907 | 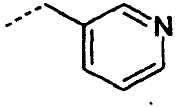 | (ESI)+: [M+H]+= 564.4 |
| 908 | 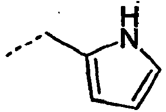 | (ESI)+: [M+H]+= 552.4 |
| 909 | 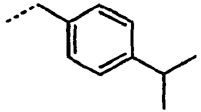 | (ESI)+: [M+H]+= 605.5 |
| 910 | 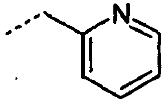 | (ESI)+: [M+H]+= 564.4 |
| 911 | 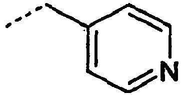 | (ESI)+: [M+H]+= 564.4 |
| 912 | 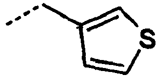 | (ESI)+: [M+H]+= 569.3 |
| 913 | 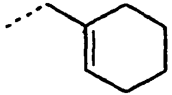 | (ESI)+: [M+H]+= 567.5 |
| 914 | CH₂CH₂OH | (ESI)+: [M+H]+= 517.4 |
| 915 | 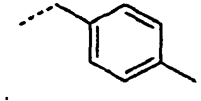 | (ESI)+: [M+H]+= 577.5 |
| 916 | 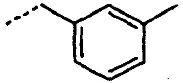 | (ESI)+: [M+H]+= 577.5 |
| 917 | 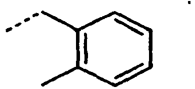 | (ESI)+: [M+H]+= 577.4 |
| 918 | 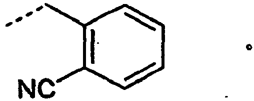 | (ESI)+: [M+H]+= 588.5 |
| 919 | 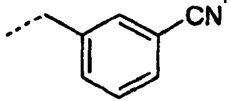 | (ESI)+: [M+H]+= 588.5 |
| 920 | 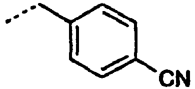 | (ESI)+: [M+H]+= 588.5 |
| 921 | 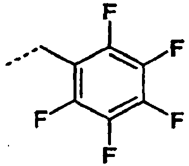 | (ESI)+: [M+H]+= 653.5 |
| 922 | 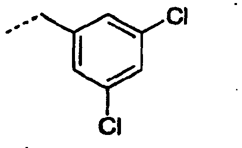 | (ESI)+: [M+H]+= 631.5 |
| 923 | 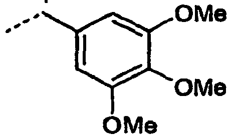 | (ESI)+: [M+H]+= 653.6 |
| 924 | 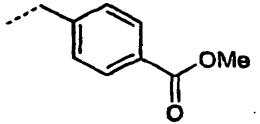 | (ESI)+: [M+H]+= 621.6 |

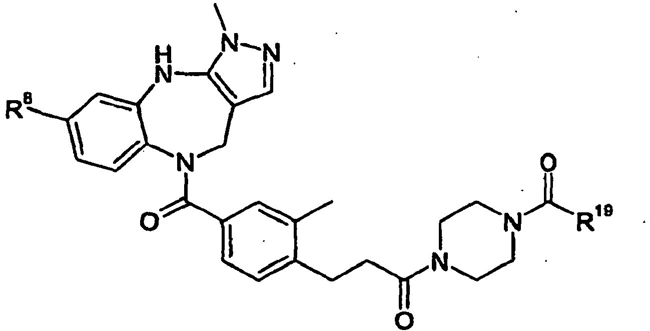

| **Compound number** | **R⁸** | **R¹⁹** | **MS** |
|---|---|---|---|
| 925 E62 | Cl | Me | (ESI)+: [M+H]+= 535.5 |
| 926 | Cl | 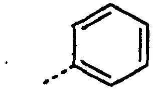 | (ESI)+: [M+H]+= 597.5 |
| 927 | Cl | 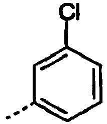 | (ESI)+: [M+H]+= 631.5 |
| 928 | Cl | 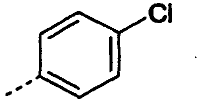 | (ESI)+: [M+H]+= 631.5 |
| 929 | Cl | 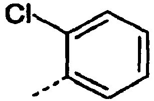 | (ESI)+: [M+H]+= 631.5 |
| 930 | Cl | 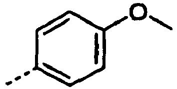 | (ESI)+: [M+H]+= 627.6 |
| 931 | Cl | 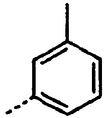 | (ESI)+: [M+H]+= 611.5 |
| 932 | Cl | 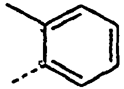 | (ESI)+: [M+H]+= 611.6 |
| 933 | Cl | 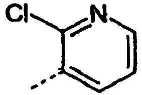 | (ESI)+: [M+H]+= 632.5 |
| 934 | Cl | 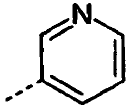 | (ESI)+: [M+H]+= 598.5 |
| 935 | Cl | 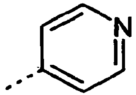 | (ESI)+: [M+H]+= 598.5 |
| 936 | Cl | 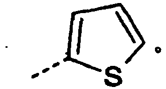 | (ESI)+: [M+H]+= 603.5 |
| 937 | Cl | 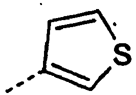 | (ESI)+: [M+H]+= 603.4 |
| 938 | Cl | 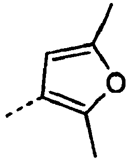 | (ESI)+: [M+H]+= 615.6 |
| 939 | Cl | 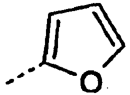 | (ESI)+: [M+H]+= 587.5 |
| 940 | Cl | 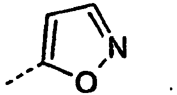 | (ESI)+: [M+H]+= 588.5 |
| 941 | Cl | 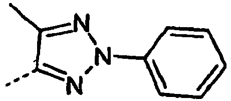 | (ESI)+: [M+H]+= 678.6 |
| 942 | Cl | CH₂CH₃ | (ESI)+: [M+H]+= 549.4 |
| 943 | Cl | CH₂CH₂CH₃ | (ESI)+: [M+H]+= 563.5 |
| 944 | Cl | 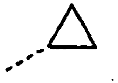 | (ESI)+: [M+H]+= 561.5 |
| 945 | Cl | C(CH₃)₃ | (ESI)+: [M+H]+= 577.5 |
| 946 | Cl | 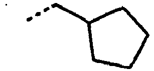 | (ESI)+: [M+H]+= 603.6 |
| 947 | Cl | CH₂(CH₂)₃CH₃ | (ESI)+: [M+H]+= 591.5 |
| 948 | Cl | CH(CH₂CH₃)CH₂CH₂CH₂CH₃ | (ESI)+: [M+H]+= 619.6 |
| 949 | Cl | 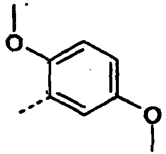 | (ESI)+: [M+H]+= 671.6 |
| 950 | Cl | 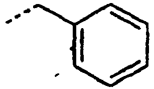 | (ESI)+: [M+H]+= 611.6 |
| 951 | Cl | 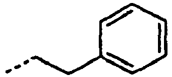 | (ESI)+: [M+H]+= 625.6 |
| 952 | Cl | 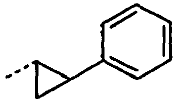 | (ESI)+: [M+H]+= 637.6 |
| 953 | Ci | 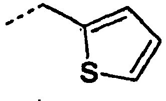 | (ESI)+: [M+H]+= 617.5 |
| 954 | Cl | 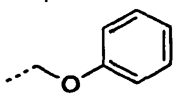 | (ESI)+: [M+H]+= 627.6 |
| 955 | Cl | 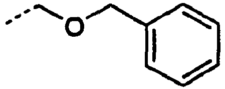 | (ESI)+: [M+H]+= 641.6 |
| 956 | Cl | CH₂CH₂CH₂CO₂CH₂CH₃ | (ESI)+: [M+H]+= 635.6 |
| 957 | Me | 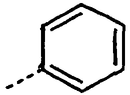 | (ESI)+: [M+H]+= 577.4 |
| 958 | Me | 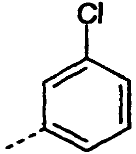 | (ESI)+: [M+H]+= 611.3 |
| 959 | Me | 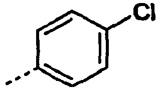 | (ESI)+: [M+H]+= 611.3 |
| 960 | Me | 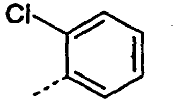 | (ESI)+: [M+H]+= 611.3 |
| 961 | Me | 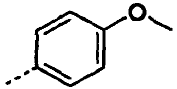 | (ESI)+: [M+H]+= 607.4 |
| 962 | Me | 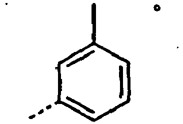 | (ESI)+: [M+H]+= 591.4 |
| 963 | Me | 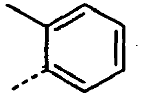 | (ESI)+: [M+H]+= 591.3 |
| 964 | Me | 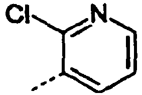 | (ESI)+: [M+H]+= 612.3 |
| 965 | Me | 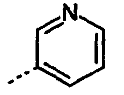 | (ESI)+: [M+H]+= 578.3 |
| 966 | Me | 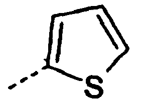 | (ESI)+: [M+H]+= 583.3 |
| 967 | Me | 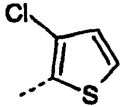 | (ESI)+: [M+H]+= 617.3 |
| 968 | Me | 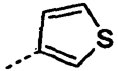 | (ESI)+: [M+H]+= 583.3 |
| 969 | Me | 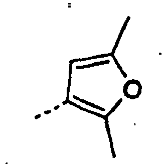 | (ESI)+: [M+H]+= 595.3 |
| 970 | Me | 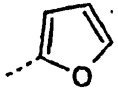 | (ESI)+: [M+H]+= 567.3 |
| 971 | Me | 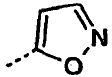 | (ESI)+: [M+H]+= 568.3 |
| 972 | Me | 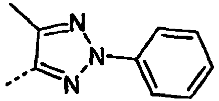 | (ESI)+: [M+H]+= 658.4 |
| 973 | Me | CH₂CH₃ | (ESI)+: [M+H]+= 529.3 |
| 974 | Me | CH₂CH₂CH₃ | (ESI)+: [M+H]+= 543.4 |
| 975 | Me | 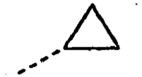 | (ESI)+: [M+H]+= 541.3 |
| 976 | Me | C(CH₃)₃ | (ESI)+: [M+H]+= 557.3 |
| 977 | Me | 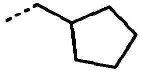 | (ESI)+: [M+H]+= 583.4 |
| 978 | Me | CH₂(CH₂)₃CH₃ | (ESI)+: [M+H]+= 571.4 |
| 979 | Me | CH(CH₂CH₃)CH₂CH₂CH₂CH₃ | (ESI)+: [M+H]+= 599.5 |
| 980 | Me | 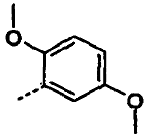 | (ESI)+: [M+H]+= 651.4 |
| 981 | Me | 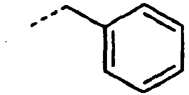 | (ESI)+: [M+H]+= 591.3 |
| 982 | Me | 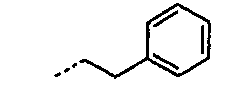 | (ESI)+: [M+H]+= 605.4 |
| 983 | Me | 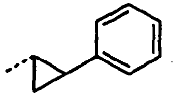 | (ESI)+: [M+H]+= 617.4 |
| 984 | Me | 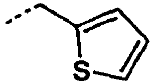 | (ESI)+: [M+H]+= 597.3 |
| 985 | Me | 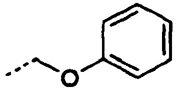 | (ESI)+: [M+H]+= 607.4 |
| 986 | Me | 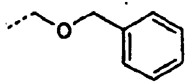 | (ESI)+: [M+H]+= 621.4 |
| 987 | Me | CH₂N(CH₃)₂ | (ESI)+: [M+H]+= 558.3 |
| 988 | Me | CH₂CH₂CH₂CO₂CH₂CH₃ | (ESI)+: [M+H]+= 615.4 |

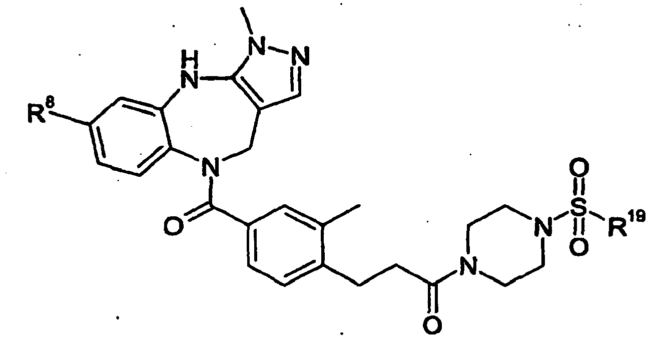

| **Compound number** | **R⁸** | **R¹⁹** | **MS** |
|---|---|---|---|
| 989 E63 | Me | Me | (ESI)+: [M+H]+= 551.3 |
| 990 | Me | CH(CH₃)₂ | (ESI)+: [M+H]+= 579.4 |
| 991 | Me | 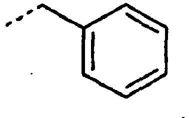 | (ESI)+: [M+H]+= 627.4 |
| 992 | Me | 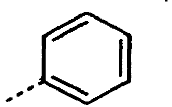 | (ESI)+: [M+H]+= 613.3 |
| 993 | Me | 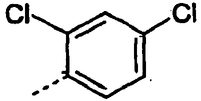 | (ESI)+: [M+H]+= 681.3 |
| 994 | Me | 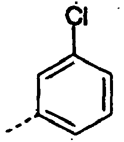 | (ESI)+: [M+H]+= 647.3 |
| 995 | Me | 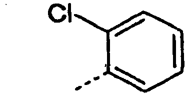 | (ESI)+: [M+H]+= 647.3 |
| 996 | Me | 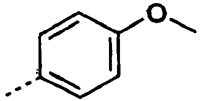 | (ESI)+: [M+H]+= 643:3 .. |
| 997 | Me | 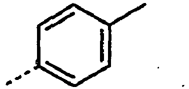 | (ESI)+: [M+H]+= 627.4 |
| 998 | Me | 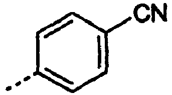 | (ESI)+: [M+H]+= 638.4 |
| 999 | Me | 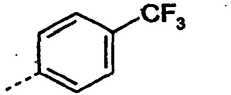 | (ESI)+: [M+H]+= 681.3 |
| 1000 | Me | 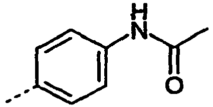 | (ESI)+: [M+H]+= 670.4 |
| 1001 | Me | 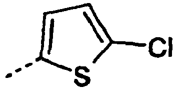 | (ESI)+: [M+H]+= 653.3 |
| 1002 | Me | CH₂CF₃ | (ESI)+: [M+H]+= 619.3 |
| 1003 | Me | 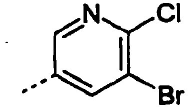 | (ESI)+: [M+H]+= 728.3 |
| 1004 | Me | 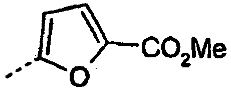 | (ESI)+: [M+H]+= 661.3 |
| 1005 | Me | CH₂CH₃ | (ESI)+: [M+H]+= 565.3 |
| 1006 | Me | CH₂CH₂CH₃ | (ESI)+: [M+H]+= 579.3 |
| 1007 | Me | CH₂CH₂CH₂CH₃ | (ESI)+: [M+H]+= 593.3 |
| 1008 | Cl | CH₃ | (ESI)+: [M+H]+= 571.4 |
| 1009 | Cl | CH(CH₃)₂ | (ESI)+: [M+H]+= 599.5 |
| 1010 | Cl | 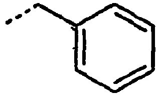 | (ESI)+: [M+H]+= 647.6 |
| 1011 | Cl | 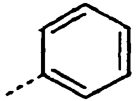 | (ESI)+: [M+H]+= 633.6 |
| 1012 | Cl | 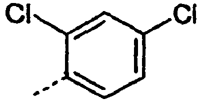 | (ESI)+: [M+H]+= 701.4 |
| 1013 | Cl | 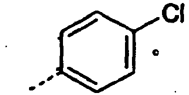 | (ESI)+: [M+H]+= 667.5 |
| 1014 | Cl | 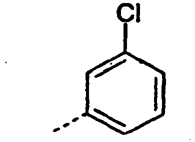 | (ESI)+: [M+H]+= 667.5 |
| 1015 | Cl | 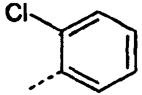 | (ESI)+: [M+H]+= 667.5 |
| 1016 | Cl | 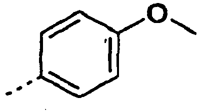 | (ESI)+: [M+H]+= 663.6 |
| 1017 | Cl | 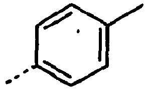 | (ESI)+: [M+H]+= 647.6 |
| 1018 | Cl | 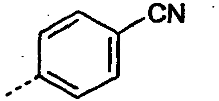 | (ESI)+: [M+H]+= 658.5 |
| 1019 | Cl | 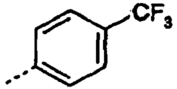 | (ESI)+: [M+H]+= 701.5 |
| 1020 | Cl | 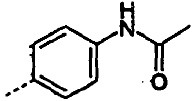 | (ESI)+: [M+H]+= 690.5 |
| 1021 | Cl | 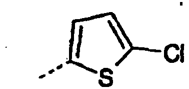 | (ESI)+: [M+H]+= 673.4 |
| 1022 | Cl | 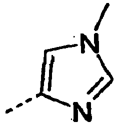 | (ESI)+: [M+H]+= 637.5 |
| 1023 | Cl | CH₂CF₃ | (ESI)+: [M+H]+= 639.5 |
| 1024 | Cl | 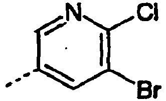 | (ESI)+: [M+H]+= 748.5 |
| 1025 | Cl | 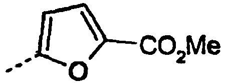 | (ESI)+: [M+H]+= 687.5 |
| 1026 | Cl | CH₂CH₃ | (ESI)+: [M+H]+= 585.4 |
| 1027 | Cl | CH₂CH₂CH₃ | (ESI)+: [M+H]+= 599.5 |
| 1028 | Cl | CH₂CH₂CH₂CH₃ | (ESI)+: [M+H]+= 613.6 |

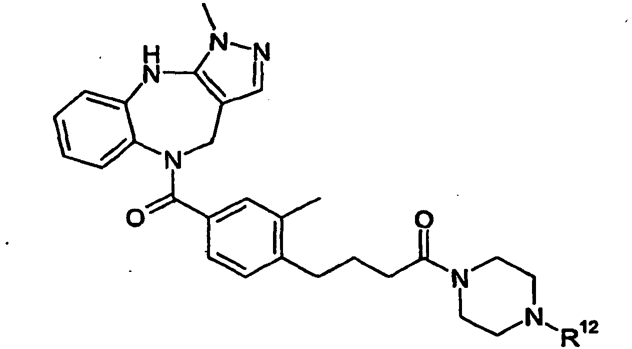

| **Compound number** | **R¹²** | | **¹H NMR: δ (ppm)** |
|---|---|---|---|
| 1029 E64 | CH₂CH₂C(CH₃)₃ | (ESI)+:[ M+H]+ = 557.6 | 0.88 (9H, s). 1.33-1.41 (2H, m), 1.74-1.82 (2H, m), 2.14 (3H, s), 2.15-2.28 (2H, m), 2.29-2.38 (6H, m), 2.42-2.54 (2H, m), 3.31-3.39(2H, m), 3:54-3.63(2H,m), 3.79 (3H, s), 3.95 (1H, d, J=14.6Hz), 5.91 (1H, d, J=14.6Hz), 6.16 (1H, s), 6.62-6.71 (2H, m), 6.79-6.91 (3H, m), 7.01-7.13 (2H, m) |
| 1030 | 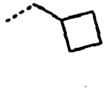 | (ESI)+: [M+H]+= 541.5 | 1.61-2.11 (8H, m), 2.12 (3H, s), 2.14-2.26 (1H, m), 2.27-2.41 (8H, m), 2.50 (2H. t, J=7.OHz), 3.32 (2H, brs), 3.56 (2H, brs), 3.76 (3H, s), 3.94 (1H, d, J=14.6Hz), 5.90 (1H, d, J=14.6Hz), 6.42 (1H, s), 6.63-6.67 (2H, m), 6.74-7.23 (5H, m) |

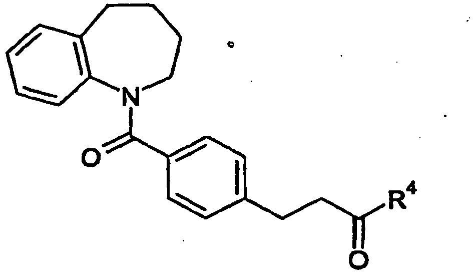

| **Compound number** | **R⁴** | **MS** | **¹H NMR** |
|---|---|---|---|
| 1031 E65 | 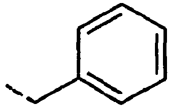 | (APCI)+:[ M+H]+= 413.4 | 1.38-1.60 (1 H, m), 1.84-2.00 (2H, m), 2.00-2.14 (1H, m), 2.38 (2H, t, J=7.7Hz), 2.64-2.77 (1H, m), 2.77-2.91 (1H, m), 2.87 (2H, t, J=7.7Hz), 2.95-3.08 (1H, m), 4.32-4.36(2H, m), 4.99(1H, d, J=13.6Hz), 5.61 (1H, s), 6.58 (1H, d, J=7.7Hz), 6.80-6.87 (1H, m), 6.91-6.95 (2H, m), 7.01-7.12 (3H, m), 7.14-7.21 (3H, m), 7.25-7.35 (3H, m) |
| 1032 | NHCH₂(CH₂)₃OH | - | - |
| 1033 | 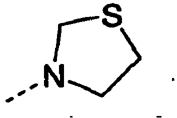 | - | - |
| 1034 | 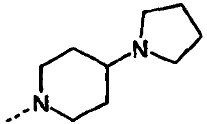 | - | - |

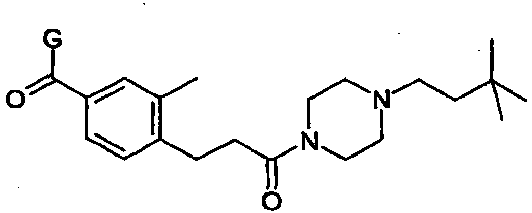

| **Compound number** | **G** | | **¹H NMR: δ (ppm)** |
|---|---|---|---|
| 1035 E66 | 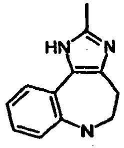 | (ESI)+:[ M+H]+ = 542.5 | 0.88 (9H, s), 1.34-1.40 (2H, m), 2.13 (3H, s), 2.28-2.40 (8H, m), 2.44(3H, s), 2.77-2.87 (3H, m), 2.87-3.05 (2H, m), 3.28-3.41 (3H, m), 3.52-3.67 (2H, m), 5.08 (1H, dd, J=4.2, 12.9Hz), 6.64 (2H, d, J=7.9Hz), 6.75 (1H, d, J=7.9Hz), 6.85 (1H, t, J=7.4Hz), 7.06 (1H, s), 7.17 (1H, t, J=7.6Hz). 8.00-8.17 (1H, m) |
| 1036 | 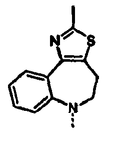 | (APCI) +: [M+H]+ = 559.4 | 0.88 (9H, s), 1.23-1.40 (4H, m), 2.16 (3H, s), 2.34-2.37 (6H, m), 2.73 (3H, s), 2.78-2.84 (2H, m), 3.09-3.57 (7H, m), 5.15-5.20 (1H, m), 6.67 (2H, m), 6.77 (1H, d, J=7.9Hz), 6.96 (1H, m), 7.12 (1H, s), 7.23 (1H. m), 8.37 (1H, dd, J=1.5, 8.2Hz) |
| 1037 | 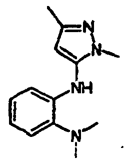 | (ESI)+: [M+H]+ = 559.4 | 0.88 (9H, s), 1.36-1.43 (2H, m), 2.15 (3H, s), 2.23 (3H, s), 2.35-2.46 (8H, m), 2.83 (2H, t, J=7.4Hz), 3.31-3.39 (2H, m), 3.37 (3H, s), 3.48 (3H, s), 3.63 (2H, t, J=4.4Hz), 5.70 (1H, s), 5.74 (1H, s), 6.49-6.56 (1H, m), 6.65-6.72 (1 H, m), 6.83-6.94 (2H, m), 7.01-7.08 (2H, m), 7.19 (1H, s) |
| 1038 | 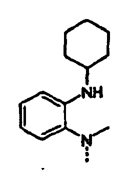 | (ESI)+: [M+H]+ = 547.5 | 0.88 (9H, s), 1.03-1.40 (8H, m), 1.58-1.83 (3H, m), 1.94-2.08 (2H, m), 2.15 (3H, s), 2.26-2.45 (8H, m), 2.81 (2H, t, J=7.4 Hz), 3.24 (3H, s), 3.29 (2H, t, J=4.4Hz), 3.58 (2H, t, J=4.4Hz), 3.91 (1H, d, J=8.2Hz), 6.37-6.43 (1H, m). 6.61-6.70 (2H, m), 6.85 (1H, d, J=7.9Hz), 7.01-7.06 (2H, m), 7.20 1H, s) |
| 1039 | 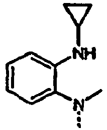 | (APCI) +: [M+H]+ = 505.2 | 0.28-0.38 (2H, m), 0.49-0.59 (2H, m), 0.73-0.81 (1H, m), 0.88 (9H, s), 1.34-1.40 (2H, m), 2.15 (3H, s), 2.25-2.45 (8H, m), 2.82 (2H, t, J=7.7Hz), 3.24 (3H, s), 3.31 (2H, t, J=5.2Hz), 3.59 (2H, t, J=5.2 Hz), 4.44 (1H, s), 6.49-6.55 (1H, m), 6.73 (1H, d, J=6.7 Hz), 6.84 (1H, d, J=7.9Hz), 6.94-7.02 (2H, m), 7.07-7.15 (2H, m) |
| 1040 | 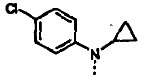 | (ESI)+: [M+H]+ 510.3, 512.4 | 0.51-0.54 (2H, m), 0.80-0.89 (2H, m), 0.88 (9H, s), 1.35-1.41 (2H, m), 1.53-1.62 (1H, m), 2.23 (3H, s), 2.29-2.43 (5H, m), 2.44-2.50 (2H, m), 2.85-2.91 (2H, m), 3.14-3.24 (1H, m), 3.31-3.38 (2H, m), 3.56-3.63 (2H, m), 6.93-7.02 (4H, m), 7.18-7.25 (3H, m) |
| 1041 | 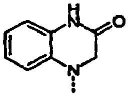 | (ESI)+: [M+H]+ = 491.5 | 0.88 (9H, s), 1.99 (1H, d, J=6.4Hz), 1.34-1.40 (2H, m), 1.80 (1H, s), 2.26 (3H, s), 2.28-2.40 (4H, m), 2.49-2.55 (2H, m), 2.90-2.97 (2H, m), 3.39 (2H, t, J=4.7Hz), 3.62 (2H, t, J=4.7Hz), 4.55 (2H, s), 6.72-6.83 (2H, m), 6.90-6.96 (1H, m), 7.01-7.12 (3H, m), 7.25 (1H, s), 9.20 (1H, s) |
| 1042 | 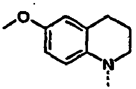 | (ESI)+: [M+H]+ = 506.8 | 0.89 (9H, s), 1.34-1.40 (2H, m), 1.93-. 2.08 (2H, m), 2.26 (3H, s), 2.26-2.43 (6H, m), 2.46-2.53 (2H, m), 2.79 (2H, t, J=6.7Hz), 2.88-2.94 (2H, m), 3.37 (2H, t, J=4.9Hz), 3.62 (2H, t, J=4.9Hz), 3.73 (3H, s), 3.83 (2H, t, J=6.4Hz), 6.40-6.48 (1H, m), 6.66 (1H, d, J=3.0Hz), 6.67-6.81 (1H, m), 6.99 (2H, s), 7.23 (1H, s) |
| 1043 | 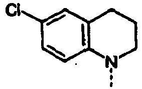 | (ESI)+: [M+H]+ = 510.8 | 0.89 (9H, m), 1.36-1.42 (2H, m), 1.97-2.07 (2H, m), 2.28 (3H, s), 2.28-2.44 (6H, m), 2.46-2.54 (2H, m), 2.80 (2H, t, J=6.7Hz), 3.33-3.42 (2H, m), 3.58-3.70 (2H, m), 3.84 (2H, t, J=6.7Hz), 6.76 (1H, d, J=8.9Hz), 6.87 (1H, dd, J=2.2, 8.7Hz), 7.02 (2H, s), 7.12 (1H, d, J=2.2Hz), 7.25 (1H, s) |
| 1044 | 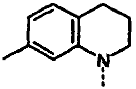 | (ESI)+: [M+H]+ = 490.8 | 0.89 (9H, s), 1.34-1.41 (2H, m), 1.92-1.99 (2H, m), 2.06 (3H, s), 2.25 (3H, s), 2.28-2.43 (6H, m), 2.43-2.52 (2H, m), 2.70-2.81 (2H, m), 2.88-2.96 (2H, m), 3.35-3.43 (2H, m), 3.56-3.68 (2H, m), 3.80-3.90 (2H, m), 6.64 (1H, s), 6.77-6.89 (2H, m), 6.92-7.06 (3H, m) |
| 1045 | 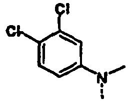 | (ESI)+: [M+H]+ = 518.4 | 0.88 (9H, s), 1.34-1.41 (2H, m), 2.23 (3H, s), 2.28-2.42 (6H, m), 2.43-2.51 (2H, m), 2.85-2.91 (2H, m), 3.36 (2H, t, J=4.9Hz), 3.43 (3H, s), 3.61 (2H, t, J=4.9Hz), 6.83 (1H, dd, J=2.5, 8.7Hz), 6.94 (2H, s), 7.20-7.27 (3H, m) |
| 1046 | 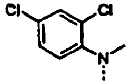 | (ESI)+: [M+H]+ = 518.4 | 0.88 (9H, s), 1.34-1.40 (2H, m), 2.20 (3H, s), 2.26-2.39 (6H, m), 2.45 (2H, t, J=7.9Hz), 2.85 (2H, t, J=7.9Hz), 3.31 (3H, s), 3.24-3.33 (2H, m), 3.59 (2H, d, J=3.2Hz), 6.82-7.03 (3H, m), 7.06-7.14 (1H, m), 7.19 (1H, s), 7.38 (1H, s) |
| 1047 | 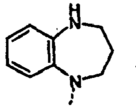 (ESI)+: | (ESI)+: [M+H]+ = 491.6 | 0.88 (9H, s), 1.34-1.40 (2H, m), 2.15 (3H, s), 2.28-2.46 (8H, m), 2.82 (2H, t, J=7.9Hz), 3.31 (2H, t, J=4.7Hz), 3.59 (2H, t, J=4.7Hz), 6.48-6.62 (2H, m), 6.75 (1H, d, J=7.2H_{Z}), 6.83 (1H, d, J=7.2Hz), 6.90-6.98 (2H, m), 7.11 (1H, s) |
| 1048 | 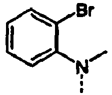 | (APCI) +: [M+H]+ 528.1, 530.3 | 0.87 (9H, s), 1.32-1.39 (2H, m), 2.16 (3H, s), 2.24-2.46 (8H, m), 2.81 (2H, t, J=7.2Hz), 3.28 (2H, t, J=4.4Hz), 3.33 (3H, s), 3.57 (2H, t, J=4.4Hz), 6.85 (1H, d). 6.94-7.07 (3H, m), 7.11-7.19 (2H, m), 7.52 (1H, d, J=7.7Hz) |
| 1049 | 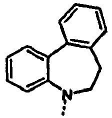 | (ESI)+: [M+H]+ = 538.7 | 0.87 (9H, s), 1.33-1.39 (2H, m), 2.11 (3H, s), 2.25-2.43 (8H, m), 2.75-2.88 (4H, m), 3.28 (2H, t, J=4.9Hz), 3.58 (2H, t, J=4.9Hz), 3.83-3.91 (1H, m), 4.60-4.71 (1H, m), 6.69-6.82 (3H, m), 6.90 (1H, s), 7.06 (1H, t, J=7.2Hz), 7.25-7.52 (6H, m) |
| 1050 | 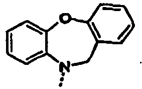 | (ESI)+: [M+H]+ = 540.7 | 0.87 (9H, s), 1.33-1.40 (2H, m), 2.19 (3H, s), 2.26-2.49 (8H, m), 2.85 (2H, t, J=7.7Hz), 3.32 (2H, t, J=4.7Hz), 3.59 (2H, t, J=4.7Hz), 5.07-5.23 (2H, m), 6.71-6.81 (2H, m), 6.83-6.93 (2H, m), 6.98-7.09 (2H, m), 7.11-7.28(5H, m) |
| 1051 | 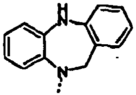 | (ESI)+: [M+H]+ = 539.7 | 0.87 (9H, s), 1.33-1.40 (2H, m), 2.15 (3H, s), 2.24-2.46 (8H, m), 2.81 (2H, t, J=8.2Hz), 3.30 (2H, t, J=4.4Hz), 3.59 (2H, t, J=4.4Hz), 4.08-4.24 (2H, m), 5.68-5.89 (1H, m), 6.48-6.59 (3H, m), 6.72-6.93 (4H, m), 6.95-7.23 (4H, m) |
| 1052 | 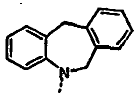 | (ESI)+: [M+H]+ = 538.7 | 0.87 (9H, s), 1.33-1.40 (2H, m), 2.20 (3H, s), 2.27-2.39 (6H, m), 2.42-2.47 (2H, m), 2.85 (2H, t, J=7.4Hz), 3.32 (2H, t, J=4.7Hz), 3.59 (2H, t, J=4.7Hz), 4.02-4.48 (4H, m), 6.78-7.34 (11H, m) |
| 1053 E67 | 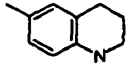 | (ESI)+: [M+H]+ = 490.7 | 0.89 (9H, s), 1.34-1.41 (2H, m), 1.94-2.03 (2H, m), 2.23 (3H, s), 2.26 (3H, s), 2.28-2.40 (6H, m), 2.47-2.53 (2H, m), 2.78 (2H, t, J=6.7Hz), 2.88-2.94 (2H, m), 3.36 (2H, t, J=4.7Hz), 3.61 (2H, t, J=4.7Hz), 3.84 (2H, t, J=6.4Hz), 6.68 (2H, s), 6.93 (1H, s), 6.99 (3H, s) |
| 1054 | 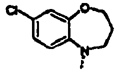 | (APCI) +: [M+H]+ = 526.1, 528.2 | 0.92 (9H, s), 1.34-1.45 (5H, m), 1.47-1.54 (5H, m), 1.55-1.78 (2H, m), 2.00-2.13 (1H, m), 2.20 (3H, s), 2.42-2.53 (2H, m), 2.70-2.81 (1H, m), 2.82-2.93 (2H, m), 3.03-3.13 (2H, m), 3.58-3.72 (2H, m), 6.51-6.61 (1H, m), 6.68-6.77 (1H, m), 6.90 (2H, s), 7.09 (2H, d, J=2.2Hz) ppm. |
| 1055 | 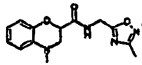 | (ESI)+: [M+H]+ = 617.5 | 0.88 (9H, s), 1.29-1.42 (2H, m), 2.24-2.43 (12H, m), 2.47-2.60 (2H, m), 2.88-3.02 (2H, m), 3.37-3.58 (2H, m), 3.59-3.71 (2H, m), 3.93 (1H, dd, J=7.2, 13.4Hz), 4.31 (1H, dd, J=3.2, 13.4Hz), 4.51-4.78 (2H, m), 4.79-4.82 (1H, m), 6.80-6.90 (1H, m), 7.01-7,06 (2H, m), 7.12-7.16 (1H, m), 7.17-7.23 (1H, m), 7.28-7.39 (2H, m) ppm. |
| 1056 | 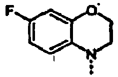 | (ESI)+: [M+H]+ = 496.5 | 0.89 (9H, s), 1.35-1.41 (2H, m), 2.32 (3H, s), 2.36-2.42 (6H, m), 2.51-2.57 (2H, m), 2.93-3.00 (2H, m), 3.40 (2H, t, J=4.7Hz), 3.63 (2H, t, J=4.4Hz), 3.95 (2H, t, J=4.4Hz), 4.31 (2H, t, J=4.4Hz), 6.42-6.48 (1H, m), 5.82 (1H, dd, J=3.0, 9.6Hz), 7.11-7.19 (1H, m), 7.22-7.24 (1H, m), 7.31 (1H, s) ppm. |

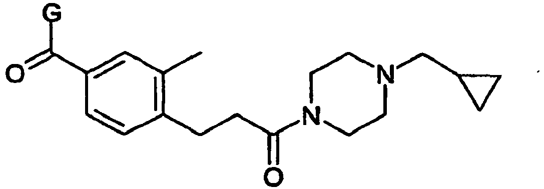

| **Compound number** | **G** | | **¹H NMR** |
|---|---|---|---|
| 1057 | 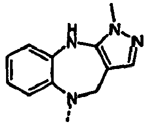 | (ESI)+ [M+H] += 513.6 | CDCl₃ δ 0.05-0.09 (2H, m), 0.42-0.52 (2H, m), 0.71-0.89 (1H, m), 2.13 (3H, s), 2.17-2.22 (2H, m), 2.28-2.52 (6H, m), 2.79-2.86 (2H, m), 3.22-3.35 (2H, m), 3.56-3.72 (2H_{,} m), 3.74 (3H, s).3.94 (1H, d, J=14.6Hz), 5.89 (1H, d, J=14.6Hz), 6.58-6.71 (2H, m), 6.75-6.90 (2H, m), 6.91 -7.06 (2H, m), 7.08-7.22 (2H, m) ppm. |
| 1058 | 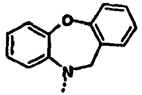 | (ESI)+ : [M+H] += 510.7 | CDCl₃ δ 0.03-0.08 (2H, m), 0.43-0.50 (2H, m), 0.77-0.86 (1H, m), 2.19 (3H, s), 2.18-2.21 (2H, m), 2.36-2.47 (6H, m), 2.84 (2H, t, J=7.4Hz), 3.32 (2H, t, J=4.9Hz), 3.60 (2H, t, J=4.9Hz), 5.03-5.26 (2H, m), 6.74-6.78 (1H, m), 6.82-6.91 (2H, m), 6.98-7.07 (2H, m), 7.11-7.18 (2H, m), 7.19-7.24 (4H, m) ppm. |
| 1059 | 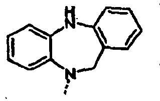 | (ESI)+ : [M+H] += 509.7 | CDCl₃ δ 0.03-0.08 (2H, m), 0.45-0.50 (2H, m), 0.71-0.89 (1H, m), 2.15 (3H, s), 2.19-2.22 (2H, m), 2.34-2.45 (6H, m), 2.83 (2H, t, J=7.2Hz), 3.31 (2H, brs), 3.60 (2H, brs), 4.03-4.34 (2H, m), 5.62-5.97 (1H, m), 6.48-6.59 (3H, m), 6.72-6.93 (5H, m), 6.97-7.03 (1H, m), 7.07-7.16 (2H, m) ppm. |
| 1060 | 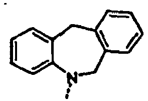 | (ESI)+ : [M+H] += 508.7 | CDCl₃ δ 0.06-0.10 (2H, m), 0.47-0.54 (2H, m), 0.72-0.89 (1H, m), 2.21 (3H, s), 2.19-2.24 (2H, m), 2.38-2.49 (6H, m), 2.85 (2H, t, J=7.4Hz)_{,} 3.34 (2H, t, J=4.9Hz), 3.62 (2H, t, J=4.9Hz), 4.00-4.50 (4H, m), 6.73-7.31 (11H, m) ppm. |

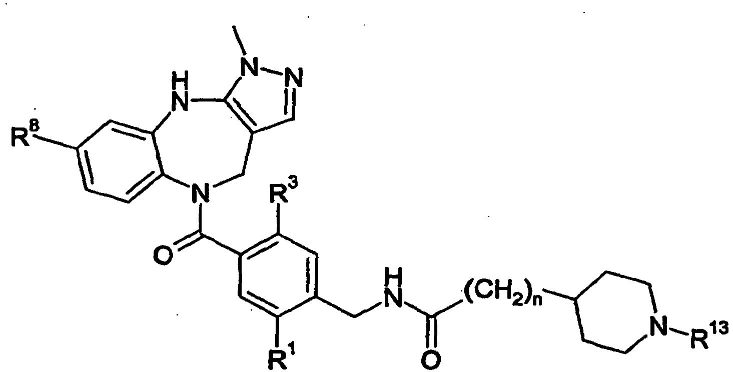

| **Compound number** | **R¹** | **R³** | **R⁸** | **R¹³** | **n** | **MS** | **¹H NMR: δ (ppm)** |
|---|---|---|---|---|---|---|---|
| 1061 E90 | H | F | H | 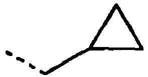 | 0 | (APCI)+: [M+H]+= 517.4 | 0.03-0.12 (3H, m), 0.45-0.54 (3H, m), 0.77-0.91 (2H, m), 1.70-1.82 (3H, m), 1.88-2.03 (2H, m), 2.03-2.17 (1H, m), 2.22 (2H, d, J=4.9Hz), 2.98-3.17 (2H, m), 3.65 (3H, s), 3.98 (1H, d, J=14.6Hz), 4.10-4.30 (2H, m), 5.80 (1H, d, J=14.6Hz), 6.53-6.79 (4H, m), 6.88-7.00 (3H, m), 7.21 (1H, s) |
| 1062 | F | H | F | CH₂CH₂CH₃ | 0 | (ESI)+: [M+H]+= 565.3 | d4-MeOH 0.95 (9H, s), 1.50-1.56 (2H, m), 1.80-1.91 (4H, m), 2.35-2.41 (1H, m), 2.52-2.58 (1H, m), 2.74-2.81 (2H, m), 3.30-3.33 (3H, m), 3.81 (3H, s), 4.00 (1H, d, J=14.6Hz), 4.32 (2H, s), 5.73 (1H, d, J=14.6Hz), 6.39-6.46 (1H, m), 6.77-6.83 (1H, m), 6.93-7.01 (3H, m), 7.12-7.18 (1H, m), 7.24 (1H, s) |
| 1063 | H | Me | Cl | CO₂C(CH₃)₃ | 2 | (APCl)+: [M+H]+= 621.5, 623.5 | 1.44 (9H, s), 1.34-1.71 (5H, m), 2.12-2.30 (2H, m), 2.32 (3H, s), 2.53-2.72 (4H, m), 3.80 (3H, s), 3.94 (1H, d. J=14.6Hz), 3.93-4.04 (2H, m), 4.19-4.42 (2H, m). 5.81 (1H, d, J=14.6Hz), 6.17 (1H, s), 6.57-7.20 (7H, m), 7.38 (1H, s) |
| 1064 | Cl | H | H | CO₂C(CH₃)₃ | 0 | (APCl)+: [M-Boc+H]+ = 479.4, 481.4 | 1.43 (9H, s), 1.47-1.62 (2H, m), 1.65-1.78 (2H, m), 2.16-2.31 (1H, m), 2.57-2.72 (2H, m), 3.77 (3H, s), 3.98 (1H, d, J=14.6Hz), 4.00-4.13 (2H, m), 4.33-4.36 (2H, m), 5.86 (1H, d. J=14.6Hz), 6.18-6.24(1H, m), 6.51 (1H, s), 6.67-6.70 (2H, m). 6.89-6.98 (3H, m), 7.04-7.11 (1H, m), 7.21-7.27 (2H, m) |
| 1065 | F | H | Me | CO₂C(CH₃)₃ | 1 | (APCl)+: [M-Boc+H]+ = 492.2 | 1.43 (9H, s), 1.50-1.63 (2H, m), 1.82-2.00 (2H, m), 2.01-2.11 (2H, m), 2.13-2.23 (1H, m), : 2.20 (3H, s), 2.53-2.71 (3H, m), 3.79 (3H, s), 3.94 (1H, d, J=14.6Hz), 3.96-4.04 (3H, m), 4.19-4.42 (1H, m), 5.83 (1H, d, J=14.6Hz), 6.31-6.42 (1H, m), 6.43-6.58 (2H, m), 6.78-6.83 (2H, m), 6.85-6.96 (2H, m), 7.24 (1H, s) |
| 1066 | Me | H | H | CO₂C(CH₃)₃ | 2 | (APCl)+: [M-Boc+H]+ = 487.5 | 0.91-1.12 (2H, m), 1.30-1.64 (6H, m), 1.43 (9H, s), 2.11 (3H, s), 2.22 (2H, t, J=7.2Hz), 2.48-2.64 (2H, m), 3.79 (3H, s), 3.93-4.09 (3H, m), 4.29-4.44 (1H, m), 5.87 (1H, d, J=14.6Hz), 6.11 (1H, brs), 6.65-6.68 (2H, m), 6.70-6.84 (3H, m), 6.85-6.92 (1H, m), 6.99-7.05 (1H, m) 7.15 (1H, s), 7.21 (1H, s) |
| 1067 | Me | H | H | CO₂C(CH₃)₃ | 1 | (APCl)+: [M-Boc+H]+ = 473.5 | 0.91-1.11 (2H, m), 1.44 (9H. s), 1.49-1.72 (4H, m), 1.84-2.06 (2H, m), 2.09 (3H, s), 2.58-2.74 (2H, m), 3.76 (3H, s), 3.93-4.06 (3H, m), 4.17-4.31 (1H. m), 5.87 (1H, d, J=14.6Hz), 6.25 (1H, brs), 6.64-6.67 (2H, m), 6.74-6.76 (3H, m), 8.94-6.98 (1H, m). 7.01-7.08 (1H, m), 7.12 (1H, s), 7.21 (1H, s) |
| 1068 | Cl | H | H | CH₂CH₂C(CH₃)₃ | 0 | (APCl)+: [M+H]+= 563.4, 565.4 | 0.87 (9H, s), 1.34-1.40 (2H, m), 1.65-1.95 (5H, m), 2.08-2.14 (2H, m), 2.27-2.33 (2H, m). 2.93-2.97 (2H, m), 3.73 (3H, s), 3.97 (1 H, d, J=14.6Hz), 4.33 (1H, d, J=5.9Hz), 5.85 (1H, d, J=14.6Hz), 6.20-6.22 (1H, m), 6.66-6.71 (3H, m), 6.91-6.98 (4H, m), 7.03-7.07 (1H, m), 7.22 (1H, s) |
| 1069 | Cl | H | H | 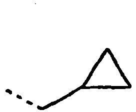 | 0 | (APCl)+: [M+H]+= 533.4, 535.2 | 0.04-0.10 (2H, m), " 0.46-0.53 (4H, m), 0.78-0.85. (1H, m), 1.64-1.77 (4H, m), 1.93-2.16 (2H, m). 2.23 (2H, d, J=6.7Hz), 2.33-2.40 (1H, m), 3.05-3.10 (2H, m), 3.73 (3H, s), 3.98 (1H, d, J=14.6Hz), 4.33 (2H, d. J=5.9Hz), 5.85 (1H, d. J=14.6Hz), 6.30-6.32 (1H, m), 6.66-6.67 (2H, m), 6.83 (1H, s), 6.90-7.08 (3H, m), 7.22 (1H, s) |
| 1070 | H | F | Cl | CH₂CH₂C(CH₃)₃ | 0 | (APCl)+: [M+H]+= 581.4, 583.4 | 0.86 (9H, s), 1.34-1.40 (2H, m), 1.72-1.76 (4H, m), 1.88-1.95 (2H, m), 2.10-2.14 (1H, m), 2.28-2.34 (2H, m), 2.94-2.98 (2H, m), 3.64 (3H, s), 3.97 (1H, d, J=14.6Hz), 4.22 (2H, d, J=5.7Hz), 5.76 (1H, d, J=14.6Hz), 6.54 (1H, dd, J=2.2, 8.4Hz), 6.58-6.76 (4H, m). 6.96-7.03 (2H, m), 7.20 (1H, s) |
| 1071 | H | F | Cl | 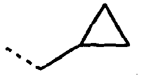 | 0 | (APCl)+: [M+H]+= 551.3, 553.3 | 0.07-0.09 (2H, m), 0.46-0.51 (2H, m). 0.80-0.84 (1H, m), 1.73-1.78 (4H, m), 1.93-1.99 (2H, m), 2.10-2.15 (1H, m), 2.24 (2H, d, J=6.4Hz), 2.51-2.53 (1H, brs), 3.07-3.11 (2H, m), 3.66 (3H, s), 3.97 (1H, d, J=14.6Hz), 4.24 (2H, d, J=5.9Hz), 5.77 (1H, d, J=14.6Hz), 6.55-6.77 (5H, m), 6.96-7.04 (2H, m), 7.21 (1H, s) |
| 1072 | H | F | Me | CH₂CH₂C(CH₃)₃ | 0 | (APCl)+: [M+H]+= 561.4 | 0.87 (9H, s), 1.35-1.41 (2H, m), 1.73-1.81 (4H, m), 1.89-2.02 (2H, m), 2.10-2.14 (4H, m), 2.29-2:35 (2H, m), 2.94-2.98 (2H, m), 3.68 (3H, s), 3.95 (1H, d, J=14.6Hz), 4.20-4.25 (2H, m), 5.79 (1H, d, J=14.6Hz), 6.42 (1H, dd, J=1.2, 8.2Hz), 6.52-7.00 (6H, m), 7.20 (1H, s) |
| 1073 | H | F | Me | 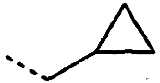 | 0 | (APCl)+: [M+H]+= 531.4 | 0.05-0.10 (2H, m), 0.46-0.53 (2H, m). 0.79-0.83 (1H, m), 1.72-1.79 (4H, m), 1.95-2.04 (2H, m), 2.08-2.14 (4H, m), 2.22-2.26 (2H, m), 2.90-2.92 (1H, brs), 3.06-3.10 (2H, m), 3.66 (3H, s), 3.95 (1H, d, J=4.6Hz), 4.18-4.23 (2H, m), 5.76 (1H, d, J=14.6Hz), 6.43 (1H, dd, J=1.2, 8.2Hz), 6.57-6.97 (6H, m), 7.18 (1H, s) |
| 1074 | F | H | Me | 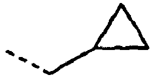 | 0 | (APCl)+: [M+H]+= 531.4 | 0.09-0.15 (2H, m), 0.49-0.57 (2H, m), 0.79-0.92 (1H, m), 1.72-1.84 (4H, m), 1.99-2.19 (3H, m), 2.21 (3H, s), 2.26-2.33 (2H, m), 3.10-3.16 (2H, m), 3.77 (3H, s), 3.95 (1H, d, J=14.6Hz), 4.33 (2H, d, J=5.9Hz), 5.83 (1H, d, J=14.6Hz), 6.24 (1H, brs), 6.36 (1H, s), 6.48-6.58 (2H, m), 6.79 (1H, s), 6.87-7.01 (3H, m), 7.21 (1H, s) |
| 1075 | F | H | Me | CH₂CH₂C(CH₃)₃ | 0 | (APCl)+: [M+H]+= 561.4 | 0.87 (9H, s), 1.36-1.43 (2H, m), 1.62-1.81 (4H, m), 1.96-1.17 (3H, m), 220 (3H, s), 2.33-2.40 (2H, m), 2.97-3.02 (2H, m), 3.76 (3H, s), 3.94 (1H, d, J=14.8Hz), 4.32 (2H, d, J=5.7Hz), 5.83 (1H, d, J=14.6Hz), 6.19 (1H, brs), 6.39 (1H, s), 6.47-6.57 (2H, m), 6.78 (1H, s), 6.86-7.00 (3H, m), 7.21 (1H, s) |
| 1076 | H | F | H | CH₂CH₂C(CH₃)₃ | 0 | (APCl)+: [M+H]+= 547.4 | 0.87 (9H, s), 1.34-1.40 (2H, m), 1.68-1.77 (4H, m), 1.97-2.03 (2H, m), 2.03-2.19 (1H, m), 2.28-2.38 (2H, m), 2.52 (2H, s), 2.90-3.00 (2H, m), 3.65 (3H, s), 3.98 (1H, d, J=14.6Hz), 4.18 (1H, t, J=6.2Hz), 5.79 (1H, d, J=14.6Hz), 6.57-6.72 (4H, m), 6.75 (1H, s), 6.90-7.02 (4H, m) |
| 1077 | H | Cl | H | 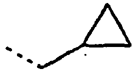 | 0 | (ESI)+: [M+H]+= 533.5, 535.5 | 0.35-0.45 (3H, m), 0.68-0.80 (3H, m), 0.98-1.15 (2H, m), 1.81-2.10 (4H, m), 2.46-2.55 (1H, m), 2.91-3.00 (4H, m), 3.60-3.75 (3H, m), 3.78 (3H, s), 4.02 (1H, d, J=14.6Hz), 5.69 (1H, d, J=14.6Hz), 6.59-6.70 (1H, m), 6.91-7.20 (6H, m), 7.25 (1H, s) |
| 1078 | H | Cl | H | CH₂CH₂C(CH₃)₃ | 0 | (ESI)+: [M+H]+= 563.5, 565.7 | 0.87 (9H, s), 1.31-1.48 (3H, m), 167-1.82 (4H, m), 1.93-2.22 (2H, m), 2.28-2.47 (3H, m), 2.90-3.10 (3H, m), 3.67 (3H, s), 4.03 (1H, d, J=14.6Hz), 4.10-4.21 (2H, m). 5.78 (1H, d, J=14.6Hz), 6.56-6.70 (2H, m), 6.70-6.85 (1H, m), 6.85-6.95 (4H, m), 7.22 (1H, s) |
| 1079 | H | Me | H | 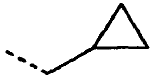 | 0 | (ESI)+: [M+H]+= 513.5 | 0.05-0.07 (3H, m), 0.48-0.51 (3H, m), 0.83-0.85 (2H, m), 1.61-2.00 (7H, m), 2.22 (2H, d, J=6.4Hz), 2.31 (3H, s), 3.00-3.15 (2H, m), 3.71 (3H, s), 4.03 (1H, d, J=14.6Hz), 4.22 (2H, d, J=5.7Hz), 5.85 (1H, d, J=14.6Hz), 6.42 (1H, s), 6.60-6.87 (4H, m), 6.90-7.10 (2H, m) ppm. |
| 1080 | H | Me | H | CH₂CH₂C(CH₃)₃ | 0 | (ESI)+: [M+H]+= 543.5 | 5 0.93 (9H, s), 1.28-1.37 (5H, m), 1.43-1.52 (2H. m), 1.71-1.91 (3H, m), 2.29 (3H, s), 2.30-2.40 (2H, m), 2.60-2.68 (2H, m), 3.12-3.23 (2H, m), 3.62-3.73 (1H, m), 3.78 (3H, s), 4.00 (1H, d, J=14.6Hz), 5.73 (1H, d, J=14.6Hz), 6.59-6.66 (1 H, m), 6.78-6.85 (2H, m), 6.90-7.00 (2H, m), 7.01-7.15 (1H, m), 7.24 (1H, s), 7.25-7.30 (1H, m) |
| 1081 | H | Cl | Cl | 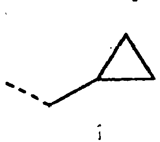 | 0 | (APCl)+: [M+H]+= 567.3 | 0.04-0.09 (2H, m), 0.46-0.51 (2H, m), 0.74-0.89 (1H, m), 1.63-1.84 (2H, m), 1.89-2.04 (2H, m), 2.05-2.21 (1H, m), 2.23 (2H, d, J=6.4Hz), 3.04-3.15 (4H, m), 3.61 (3H, s), 3.98 (1H, d, J=14.6Hz), 4.10-4.19 (2H, m), 4.31-4.38 (1H, m), 5.71 (1H, d, J=14.6Hz), 6.53 (1H, dd, J=2.0, 8.4Hz). 6.70-6.84 (3H, m), 6.96-7.11 (2H, m), 7.17 (1H, s), 7.73 (1H, s) |
| 1082 | H | Me | Cl | 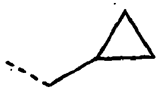 | 0 | (APCl)+: [M+H]+= 547.4, 549.4 | 0.03-0.09 (2H, m), 0.44-0.49 (2H, m), 0.78-0.84 (1H, m), 1.68-1.85 (4H, m), 1.94-2.10 (2H, m), 2.10-2.24 (1H, m), 2.22 (3H, s), 2.23 (2H, d, J=8.2Hz), 3.04-3.13 (2H, m), 3.61 (3H, s), 3.94 (1H, d, J=14.6Hz), 4.17 (2H, d, J=5.7Hz), 4.38 (1H, d, J=5.7Hz), 5.76 (1H, d, J=14.6Hz), 6.49-6.65 (3H, m), 6.71-6.83 (2H, m), 6.87-7.02 (1H, m), 7.19 (1H, s), 7.57 (1H, s) |
| 1083 | H | Cl | Me | 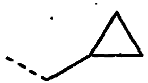 | 0 | (APCl)+: [M+H]+= 547.3, 549.3 | d4-MeOH:0.30-0.36 (2H, m), 0.65-0.71 (2H, m), 0.96-1.10 (1H, m), 1.82-2.04 (4H, m), 2.15 (3H, s), 2.38-2.49 (1H, m), 2.68-2.84 (4H, m), 3.49 (2H, brd, J=12.6Hz), 3.76 (3H, s), 3.98 (1H, d, J=14.6Hz), 4.22 (2H, s), 5.67 (1H, d, J=14.6Hz), 6.46 (1H, dd, J=1.5, 8.2Hz), 6.83 (1H, d, J=8.2Hz), 6.94-7.11 (3H, m), 7.16 (1H, s), 7.22 (1H, s) |
| 1084 | H | Me | Me | 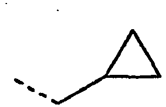 | 0 | (APCl)+: [M+H]+= 527.4 | 0.05-0.09 (2H, m), 0.45-0.52 (2H, m), 0.72-0.86 (1H, m), 1.62-1.84 (4H, m), 1.97-2.11 (2H, m), 2.05 (3H, s), 2.11-2.24 (1H, m), 2.25 (2H, d, J=5.7Hz), 2.26 (3H, s), 3.06-3.17 (2H, m), 3.66 (3H, s), 3.94 (1 H, d, J=14.6Hz), 4.17 (2H, d, J=5.7Hz), 4.37 (1H, d, J=5.7Hz), 5.78 (1H, d, J=14.6Hz), 6.31-6.83 (7H, m), 7.19 (1H, s) |
| 1085 | H | Cl | Cl | CH₂CH₂C(CH₃)₃ | 0 | (APCl)+: [M+H]+= 597.3 | 0.87 (9H, s), 1.33-1.41 (2H, m), 1.63-1.98 (6H, m), 2.07-2.17 (1H, m), 2.26-2.32 (2H, m), 2.92-2.96 (2H, m), 3.61 (3H, s), 3.98 (1H, d, J=14.6Hz), 4.08-4.23 (2H, m), 4.34-4.37 (1H, m), 5.71 (1H, d, J=14.6Hz), 6.52 (1H, dd, J=2.0, 8.4Hz), 6.70-6.98 (5H, m), 7.17 (1H, s), 7.73 (1H, s) |
| 1086 n | H | Me | Cl | CH₂CH₂C(CH₃)₃ | 0 | (APCl)+: [M+H]+= 577.4, 579.3 | 0.87 (9H, s), 1.33-1.41 (2H, m), 1.68-1.98 (6H, m), 2.03-2.17 (1H, m), 2.22 (3H, s), 2.27-2.33 (2H, m), 2.93-2.97 (2H, m), 3.61 (3H, s), 3.95 (1H, d, J=14.6Hz), 4.17-4.19 (2H, m), 4.37-4.39 (1H, m), 5.77 (1H, d, J=14.6Hz), 6.40-7.17 (6H, m), 7.19 (1H, s), 7.47 (1H, s) ppm. |
| 1087 | H | Cl | Me | CH₂CH₂C(CH₃)₃ | 0 | (APCl)+: [M+H]+= 577.4, 579.4 | δ 0.88 (9H, s), 1.34-1.42 (2H, m), 1.67-2.22 (7H, m), 2.07 (3H, s), 2.27-2.33 (2H, m), 2.93-2.97 (2H, m), 3.65 (3H, s), 3.98 (1H, d, J=14.6Hz), 4.13-4.20 (2H, m), 4.34-4.37 (1H, m), 5.75 (1H, d, J=14.6Hz), 6.43 (1H, dd, J=1.2, 8.2Hz), 6.67-6.80 (5H, m), 6.92-6.93 (1H, m), 7.19 (1H, s) |
| 1088 | H | Me | Me | CH₂CH₂C(CH₃)₃ | 0 | (APCl)+: [M+H]+= 557.4 | 0.87 (9H, s), 1.33-1.41 (2H, m), 1.65-2.04 (7H, m), 2.07 (3H, s), 2.22-2.34 (5H, m), 2.92-3.00 (2H, m), 3.62 (3H, s), 3.94 (1H, d, J=14.6Hz), 4.16-4.19 (2H, m), 4.36-4.39 (1H, m), 5.80 (1H, d, J=14.6Hz), 6.34-7.14 (7H, m), 7.19 (1H, s) |
| 1089 | F | H | H | 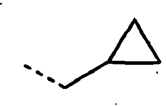 | 0 | (ESI)+: [M+H]+= 517.5 | d4-MeOH: 0.14-0.19 (2H. m), 0.53-0.59 (2H, m), 0.87-0.90 (1H, m), 1.73-1.81 (4H, m), 2.14-2.24 (3H, m), 2.35-2.37 (2H, m), 3.16-3.21 (2H, m), 3.79 (3H, s), 3.97 (1H, d, J=14.6Hz), 4.29 (2H, s), 5.75 (1H, d, J=14.6Hz), 6.65-6.77 (2H, m), 6.90-7.00 (2H, m), 7.07-7.15 (2H, m), 7.20-7.24 (2H, m) |
| 1090 | F | H | H | CH₂CH₂C(CH₃)₃ | 0 | (ESI)+: [M+H]+= 547.5 | 0.86 (9H, s), 1.32-1.39 (2H, m), 1.69-1.92 (3H, m), 2.04-2.10 (2H, m), 2.25-2.31 (1H, m), 2.58 (1H, m), 2.90-2.94 (2H, m), 3.67 (3H, s), 3.97 (1H, d, J=14.6Hz), 4.25-4.28 (2H, m), 5.83 (1H, d, J=14.6Hz), 6.37-6.39 (1H, m), 6.65 (2H, m), 6.83-7.03 (5H, m), 7.14 (1H, s), 7.20 (1H, s) |
| 1091 | Cl | H | Me | 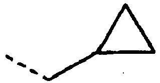 | 0 | (ESI)+: [M+H]+= 547.4, 549.4 | d4-MeOH: 0.14-d4-MeOH:0.14-0.20 (2H, m), 0.52-0.60 (2H, m), 0.90 (1H, m), 1.76-1.84 (4H, m), 2.16-2.32 (6H, m), 2.36-2.39 (2H, m), 3.18-3.22 (2H, m), 3.78 (3H, s), 3.93 (1H, d, J=14.6Hz), 4.32 (2H, s), 5.73 (1H, d, J=14.6Hz), 6.50 (1H, dd, J=1.0, 8.0Hz), 6.62 (1H, d, J=8.0Hz), 7.04 (1H, d, J=1.0Hz), 7.11 (2H, s), 7.20-7.23 (2H, m |
| 1092 | Cl | H | Me | CH₂CH₂C(CH₃)₃ | 0 | (APCl)+: [M+H]+= 577.3, 579.3 | 0.86 (9H, s), 1.33-1.40 (2H, m), 1.70-1.92 (6H, m), 2.12-2.14 (4H, m), 2.28-2.34 (2H, m), 2.93-2.96 (2H, m), 3.70 (3H, s), 3.93 (1H, d, J=14.6Hz), 4.28-4.32 (2H, m), 5.79 (1H, d, J=14.6Hz), 6.45-6.54 (2H, m), 6.79-6.89 (4H, m), 7.17 (1H, s), 7.22 (1H, s) |
| 1093 | Cl | H | Cl | CH₂CH₂C(CH₃)₃ | 0 | (APCI)+: [M+H]+ = 597.2 | 0.86 (9H, s), 1.33-1.39 (2H, m), 1.68-1.90 (6H, m), 2.13 (1H, m), 2.26-2.32 (2H, m), 2.93-2.97 (2H, m), 3.67 (3H, s), 3.94 (1H, d, J=14.6Hz), 4.32 (2H, m), 5.78 (1H, d, J=14.6Hz), 6.56 (2H, s), 6.87-6.97 (2H, m), 7.02 (1H, s), 7.18 (1H, s), 7.21 (1H, d, J=1.2Hz), 7.64 (1H, s) |
| 1094 | F | H | Cl | CH₂CH₂C(CH₃)₃ | 0 | (APCI)+: [M+H]+ = 581.3, 583.3 | 0.85 (9H, s), 1.35-1.41 (2H, m), 1.76 (4H, m), 2.01-2.14 (3H, m), 2.34-2.40 (2H, m), 2.97-3.01 (2H, m), 3.68 (3H, s), 3.94 (1 H, d, J=14.6Hz), 4.27 (2H, m), 5.76 (1H, d, J=14.6Hz), 6.54 (2H, s), 6.81-6.97 (3H, m), 7.05 (1H, s), 7.17 (1 H, s), 8.08 (1H, s) |
| 1095 | Me | H | Cl | 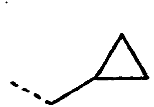 | 0 | (APCI)+: [M+H]+ = 547.4, 549.4 | d6-DMSO: 0.15-0.24 (2H, m), 0.48-0.59 (2H, m), 0.84-1.03 (1 H, m), 1.61-1.88 (4H, m), 2.14 (3H, s), 2.20-2.80 (4H, m), 3.16-3.46 (3H, m), 3.77 (3H, s), 3.90 (1H, d, J=14.6Hz), 4.06-4.23 (2H, m), 5.63 (1H, d, J=14.6Hz), 6.67 (1H, dd, J=2.0, 8.4Hz), 6.74 (1H, d, J=8.4Hz), 6.84 (1H, d, J=7.8Hz), 6.94 (1H, d, J=7.8Hz), 7.05 (1H, s), 7.18 (1H, s), 7.40 (1H, d, J=2.0Hz), 8.26 (1H, s), 8.82 (1H, s) |
| 1096 | Cl | H | Cl | 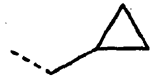 | 0 | (ESI)+: [M+H]+ = 567.2 | d6-DMSO: 0.15-0.22 (2H, m), 0.50-0.52 (2H, m), 0.89 (1H, m), 1.60-1.82 (6H, m), 2.13-2.34 (3H, m), 3.09-3.18 (2H, m), 3.76 (3H, s), 3.93 (1H, d, J=14.6Hz), 4.25 (2H, m), 5.62 (1H, d, J=14.6Hz), 6.71 (1H, dd, J=2.2, 8.4Hz), 6.84 (1H, d, J=8.4Hz), 7.03-7.11 (2H, m), 7.20 (2H, s), 7.38 (1H, d, J=2.2Hz), 8.34 (1H, m), 8.82 (1H, s) |
| 1097 | F | H | Cl | 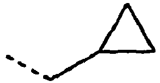 | 0 | (ESI)+: [M+H]+ = 551.3, 553.3 | d6-DMSO: 0.04-0.06 (2H, m), 0.43-0.45 (2H, m), 0.78-0.81 (1H, m), 1.53-1.66 (5H, m), 1.90 (1H, m), 2.07-2.16 (3H, m), 2.98 (2H, m), 3.76 (3H, s), 3.93 (1H, d, J=14.6Hz), 4.19 (2H, m), 5.61 (1 H, d, J=14.6Hz), 6.69 (1H, dd, J=2.2, 8.4Hz), 6.82 (1 H, d, J=8.4Hz), 6.90-6.94 (2H, m), 7.05-7.11 (1H, m), 7.19 (1H, s), 7.37 (1H, d, J=2.2Hz), 8.25 (1H, m), 8.80 (1H, s) |
| 1098 | Me | H | Me | 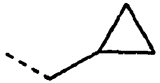 | 0 | (APCI)+: [M+H]+= 527.4 | d4-MeOH: 0.32-0.45 (2H, m), 0.68-0.80 (2H, m), 1.01-1.19 (1H, m), 1.78-2.15 (4H, m), 2.17 (3H, s), 2.24 (3H, s), 2.81-3.20 (4H, m), 3.51-3.78 (3H, m), 3.93 (1H, d, J=14.6Hz), 3.96 (3H, s), 4.26 (2H, s), 5.78 (1H, d, J=14.6Hz), 6.52-6.71 (2H, m), 6.99-7.13 (4H, m), 7.61 (1H, s) |
| 1099 | Me | H | Cl | CH₂CH₂C(CH₃)₃ | 0 | (APCI)+: [M+H]+ = 577.4, 579.4 | 0.88 (9H, s), 1.38-1.45 (2H, m), 1.72-1.94 (4H, m), 2.01-2.18 (2H, m), 2.11 (3H, s), 2.39-2.45 (2H, m), 2.96-3.05 (2H, m), 3.09-3.22 (1H, m), 3.76 (3H, s), 3 .93 (1H, d, J=14.6Hz), 4.26 (2H, d, J=4.5Hz), 5.83 (1H, d, J=14.6Hz), 6.13 (1H, brs), 6.57-6.61 (2H, m), 6.80-6.84 (3H, m), 7.03 (1H, s), 7.08 (1H, s), 7.21 (1H, s) |
| 1100 | Me | H | Me | CH₂CH₂C(CH₃)₃ | 0 | (APCI)+: [M+H]+= 557.5 | 0.88 (9H, s), 1.38-1.45 (2H, m), 1.67-1.83 (4H, m), 1.94-2.23 (2H, m), 2.07 (3H, s), 2.19 (3H, s), 2.35-2.44 (2H, m), 2.93-3.15 (3H, m), 3.74 (3H, s), 3.93 (1H, d, J=14.6Hz), 4.19-4.25 (2H, m), 5.83 (1H, d, J=14.6Hz) 6.19 (1H, brs), 6.45-6.55 (3H, m), 6.77-6.83 (3H, m), 7.06 (1H, s), 7.19 (1H, s) |

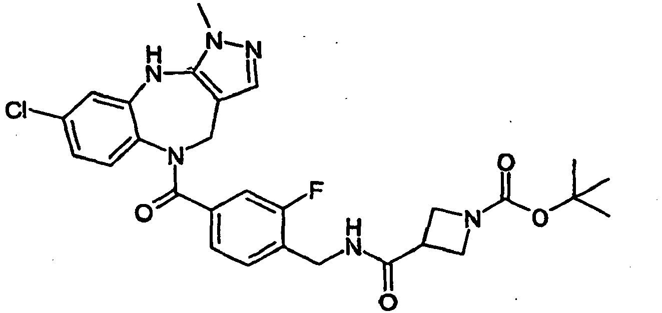

| **Compound number** | **MS** | **¹H NMR: δ (ppm)** |
|---|---|---|
| 1101 | (ESI)+: [M+H]+ =569.2 | 1.41(9H, s), 3.13-3.30(2H, m), 3.73 (2H, s), 3.80-4.15 (6H, m), 4.18-4.34 (2H, m), 5.77 (1H, m), 6.58 (2H, s), 6.73-6.87 (2H, m), 6.87-6.99 (1H, m), 7.08 (1H, s), 7.19 (1H, s), 7.82 (1H, s) |

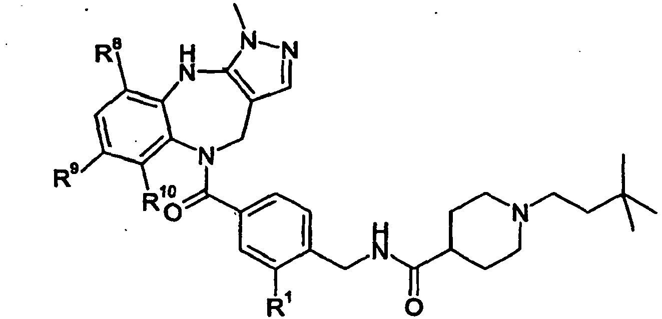

| **Compound number** | **R¹** | **R⁸** | **R⁹** | **R¹⁰** | **MS** | **¹H NMR: δ (ppm)** |
|---|---|---|---|---|---|---|
| 1102 | Me | Me | H | H | (ESI)+ **:** [M+H] += 557.6 | 0.89 (9H, s), 1.46-1.56 (2H, m), 1.78-2.06 (7H, m), 2.20-2.62 (8H, m), 3.10-3.20 (2H, m), 3.88 (3H, s), 3.95 (1H, d, J=14.6Hz), 4.22 (2H, m), 5.90 (1H, d, J=14.6Hz), 6.28-6.40 (1H, m), 6.55-6.68 (2H, m), 6.79**-**6**.**88 (2H, m), 6.90-7.04 (2H, m), 7.25 (1H, s) |
| 1103 | Me | H | Me | H | (ESI)+ : [M+H] += 557.4 | 0.88 (9H, s), 1.36-1.46 (2H, m), 1.68-1.90 (4H, m), 2.03 (3H, s), 2.12 (3H, s), 2.26-2.43 (4H, m), 2.95-3.05 (2H, m), 3.65-3.75 (1 H, m), 3.78 (3H, s), 3.95 (1H, d, J=14.7Hz), 4.25-4.35 (2H, m), 5.86 (1 H, d, J=14.7Hz), 6.02-6.12 (1H, m), 6.51 (1H, s), 6.82 (1H, s), 6.82-6.94 (4H, m), 7.12 (1H, s) |
| 1104 | F | Me | H | H | (ESI)+ : [M+H] += 561.4 | 0.91 (9H, s), 1.79-2.00 (2H, m), 2.10-2.34 (1H, m), 2.42 ' (3H, s), 2.50-2.73 (1H, m), 2.83-2.97 (2H, m), 2.98-3.12 (3H, m), 3.13-3.33 (1H, m), 3.35-3.52 (1H, m), 3.53-3.70 (2H, m), 3.85 (3H, s), 3.95 (1H, d, J=14.6Hz), 4.14-4.42 (2H, m), 5.81 (1H, d, J=14.6Hz), 6.08 (1 H, s), 6.40-6.70 (2H, m), 6.72-7.06 (4H, m), 7.43 (1H, s) |

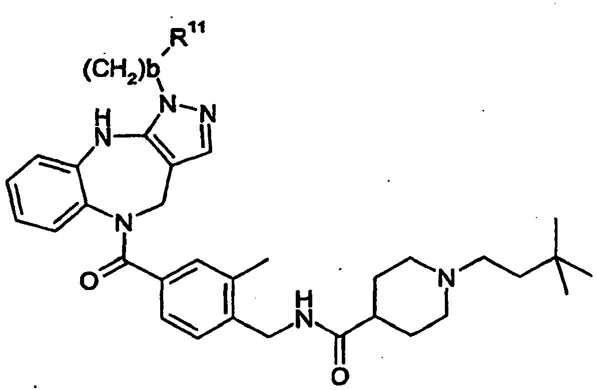

| **Compound number** | **b** | **R¹¹** | **MS** | **¹H NMR: δ (ppm)** |
|---|---|---|---|---|
| 1105 | 3 | Me | 585.4 | 0.88 (9H, s), 0.97 (3H, t, J=7.3Hz), 1.03-1.05 (4H, m), 1.07-1.95 (6H, m), 2.00-2.25 (5H, m), 2.35-2.50 (2H, m), 3.00-3.12 (3H, m), 3.97 (1 H, d, J=14.6Hz), 4.06 (2H, t, J=6.8Hz), 4.26 (2H, d, J=4.7Hz) 5.90 (1H, d, J=14.6Hz) 5.95-6.05 (1H, m) 6.24, (1H, s), 6.67-6.73 (2H, m), 6.80-6.98 (3H, m), 7.05-7.15 (2H, m) |
| 1106 | 1 | Me | 557.4 | 0.87 (9H, s), 1.36-1.52 (5H, m), 1.68-1.90 (4H, m), 1.92-2.16 (6H, m), 2.32-2.42 (2H, m), 2.96-3.06 (2H, m), 3.97 (1H, d, J=14.5Hz), 4.09 (2H, q, J=7.0Hz), 4.46 (2H, d, J=4.9Hz), 5.80-5.90 (1H, m), 5.90 (1H, d, J=14.5Hz), 6.63-6.74 (2H, m), 6.79-7.00 (3H, m), 7.02-7.12 (2H, m), 7.13 (1H, s), 7.24 (1H, s) |

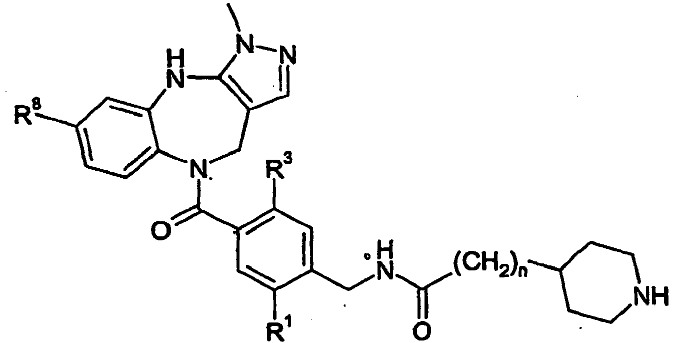

| **Compound number** | **R¹** | **R³** | **R⁸** | **n** | **MS** | **¹H NMR: δ (ppm)** |
|---|---|---|---|---|---|---|
| 1107 E91 | Me | H | H | 1 | (APCI )+:[M +H]+= 473.5 | d4-MeOH 1.35-1.54 (3H, m), 1.89 (2H, d, J=13.6Hz), 1.92-2.11 (1H, m), 2.18 (3H, s), 2.18-2.24 (1H, m), 2.92-3.02 (2H, m), 3.29-3.39 (2H, m), 3.99-4.04 (1H, m), 4.04 (3H, s), 4.27 (2H, s), 5.88 (1 H, d, J=14.6Hz), 6.84-6.86 (2H, m), 6.98-7.07 (3H, m), 7.20-7.27 (1H, m), 7.40 (1H, d, J=8.2Hz), 8.03 (1H, s) |
| 1108 | H | Me | Cl | 2 | (APCI )+:[M +H]+= 521.6, 523.5 | d4-MeOH 1.28-1.41 (1H, m), 1.42-1.67 (2H, m), 1.85-1.91 (2H, m), 2.20-2.33 (2H, m), 2.27 (3H, s), 2.78-2.94 (1H, m), 3.27-3.39 (5H, m), 3.79 (3H, s), 3.99 (1H, d, J=14.6Hz), 4.22 (2H, s), 5.71 (1H, d, J=14.6Hz), 6.53-6.61 (1H, m), 6.74-6.81 (1H, m), 6.83-6.94 (2H, m), 7.00-7.04 (1H, m), 7.22-7.25 (2H, m) |
| 1109 | Cl | H | H | 0 | (APCI )+:[M +H]+= 479.4, 481.4 | d4-MeOH 1.78-2.07 (2H, m), 2.52-2.71 (1H, m), 2.96-3.12 (2H, m), 3.19-3.32 (2H, m), 3.35-3.43 (2H, m), 4.04 (1H, d, J=14.6Hz), 4.05 (3H, s), 4.35 (2H, s), 5.87 (1H, d, 6.86-6.90 (2H, m), 7.11-7.19 (2H, m), 7.22-7.28 (2H, m), 7.42-7.45 (1H, m), 8.05 (1H, s) |
| 1110 | F | H | Me | 1 | (ESI)+: [M+H]+= 491.6 | d4-MeOH 1.34-1.53 (2H, m), 1.85-1.91 (2H, m), 1.93-2.17 (1H, m), 2.20-2.23 (2H, m), 2.28 (3H, s), 2.91-3.02 (2H, m), 3.30-3.38 (2H, m), 3.99 (1H, d, J=14.6Hz), 4.02 (3H, s), 4.32 (2H, s), 5.84 (1H, d, J=14.6Hz), 6.67-6.78 (2H, m), 6.95-7.03 (2H, m), 7.12-7.23 (2H, m), 8.01 (1H, s) |
| 1111 | Me | H | H | 2 | (APCI )+:[M +H]+= 487.4 | d4-MeOH: 1.29-1.48 (2H, m), 1.50-1.64 (3H, m), 1.82-1.98 (2H, m), 2.18 (3H, s), 2.18-2.31 (2H, m), 2.85-2.99 (2H, m), 3.30-3.38 (2H, m), 3.94-4.05 (1H, m), 4.05 (3H, s), 4.26 (2H, s), 5.88 (1H, d, J=14.6Hz), 6.85 (2H, s), 7.00-7.09 (3H, m), 7.19-7.28 (1H, m), 7.40-7.43 (1H, m), 8.04 (1H, s) |

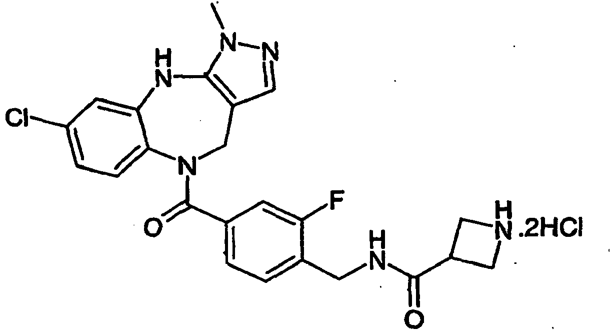

| **Compound number** | **MS** | **¹H NMR: δ (ppm)** |
|---|---|---|
| 1112 | (ESI)+:[M+H]+ = 469.2, 471.2 | d4-MeOH 3.57-3.72 (1H, m), 3.98 (3H, s), 4.04 (1H, d, J=15.3Hz), 4.17 (4H, d, J=8.2Hz), 4.36-4.42 (2H, m), 5.83 (1H, d, J=15.3Hz), 6.82-6.92 (2H, m), 6.96-7.08 (2H, m), 7.18-7.32 (1H, m), 7.41 (1 H, s), 7.92 (1H, s), 8.56-8.64 |

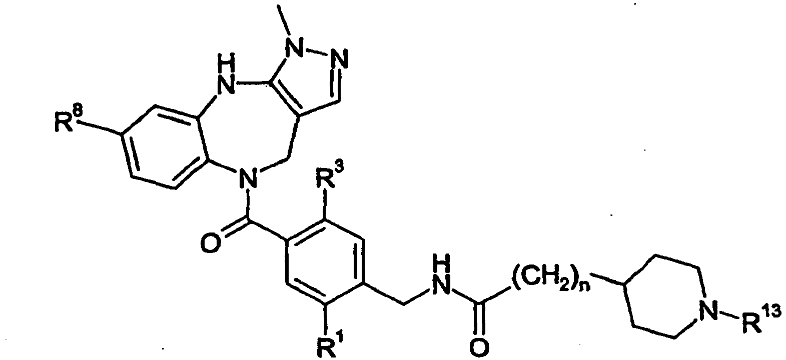

| **Compound number** | **R¹** | **R³** | **R⁸** | **R¹³** | **n** | **MS** | **¹H NMR: δ (ppm)** |
|---|---|---|---|---|---|---|---|
| 1113 E92 | Me | H | H | CH₂CH₂C(CH₃)₃ | 1 | (APCI )+:[M +H]+= 557.5 | 0.86 (9H, s), 1.11-1.29 (2H, m), 1.34-1.41 (2H, m), 1.58-1.64 (2H, m), 1.65-1.86 (1H, m), 1.89-2.01 (2H, m), 2.04 (3H, s), 2.29-2.36 (2H, m), 2.80-2.91 (4H, m), 3.64 (3H, s), 3.94 (1H, d, J=14.6Hz), 4.19 (2H, d, J=4.0Hz), 5.83 (1 H, d, J=14.6Hz), 6.51-6.68 (3H, m), 6.75-6.81 (2H, m), 6.91-7.02 (3H, m), 7.10-7.17 (2H, m) |
| 1114 | Me | H | H | CH₂CH₂C(CH₃)₃ | 2 | (APCI )+:[M +H]+= 571.5 | 0.87 (9H, s), 1.13-1.28 (3H, m), 1.34-1.40 (2H, m), 1.43-1.68 (4H, m), 1.78-1.89 (2H, m), 2.06 (3H, s), 2.15 (2H, t, J=7.4Hz), 2.23-2.30 (2H, m), 2.88 (2H, d, J=10.9Hz), 3.66 (3H, s), 3.95 (1H, d, J=14.6Hz), 4.22 (2H, d, J=5.2Hz), 5.87 (1H, d, J=14.6Hz), 6.08 (1H, t, J=5.2Hz), 6.61-6.68 (2H, m), 6.76-6.87 (3H, m), 6.95-7.05 (2H, m), 7.07 (1 H, s), 7.20 (1 H, s) |
| 1115 | Me | H | H | CH₂CH₂C(CH₃)₃ | 0 | (ESI)+: [M+H] += 543.4 | 0.88 (9H, s), 1.35-1.42 (2H, m), 1.57-2.00 (6H, m), 2.12 (3H, s), 2.29-2.32 (2H, m), 2.92-3.03 (2H, m), 3.14-3.15 (1H, m), 3.80 (3H, s), 3.97 (1H, d, J=14.6Hz), 4.28 (2H, d, J=5.2Hz), 5.58-5.72 (1H, m), 5.90 (1H, d, J=14.6Hz), 6.11 (1H, s), 6.70 (2H, d, J=4.2Hz), 6.82-6.96 (3H, m), 7.03-7.14 (1H, m), 7.14 (1H, s) |
| 1116 | Me | H | H | 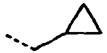 | 0 | (ESI)+: [M+H] + = 513.3 | 0.12-0.14 (2H, m), 0.53-0.56 (2H, m), 0.78-0.97 (1H, m), 1.48-2.01 (6H, m), 2.17 (3H, s), 2.18-2.24 (2H, m), 2.32 (2H, d, J=6.7Hz), 3.12-3.16 (2H, m), 3.80 (3H, s), 3.96 (1H, d, J=15.1Hz) 5.88 (1H, d, J=15.1Hz), 6.05-6.18 (1H, m), 6.42-6.43 (1H, m), 6.68 (2H, d, J=4.0Hz), 6.80-6.89 (2H, m), 6.97-7.02 (1 H, m), 7.03-7.13 (2H, m) |

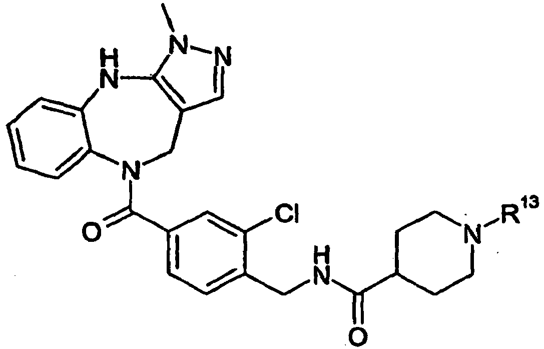

| **Compound number** | **R¹³** | **MS** |
|---|---|---|
| 1117 E93 | CH₂CH₂CH₃ | (ESI)+: [M+H]+= 521.4 |
| 1118 | 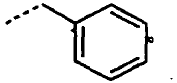 | (ESI)+: [M+H]+= 569.3 |
| 1119 | 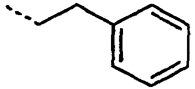 | (ESI)+: [M+H]+= 583.4 |
| 1120 | 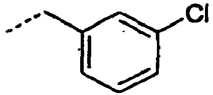 | (ESI)+: [M+H]+= 603.3 |
| 1121 | 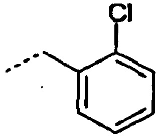 | (ESI)+: [M+H]+= 603.4 |
| 1122 | CH₂CH₂CH₂CH₂CH₃ | (ESI)+: [M+H]+= 549.4 |
| 1123 | 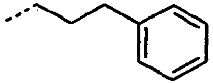 | (ESI)+: [M+H]+= 597.4 |
| 1124 | CH₂CH₂CH(CH₃)₂ | (ESI)+: [M+H]+= 549.4 |
| 1125 | CH₂C(CH₃)₃ | (ESI)+: [M+H]+= 549.4 |
| 1126 | 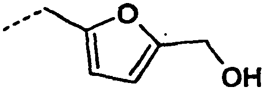 | (ESI)+: [M+H]+= 589.4 |
| 1127 | 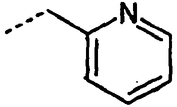 | (ESI)+: [M+H]+= 570.3 |
| 1128 | 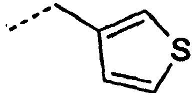 | (ESI)+: [M+H]+= 575.3 |
| 1129 | 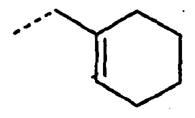 | (ESI)+: [M+H]+= 573.4 |
| 1130 | 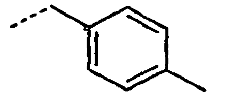 | (ESI)+: [M+H]+= 583.3 |
| 1131 | 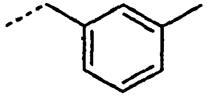 | (ESI)+: [M+H]+= 583.4 |
| 1132 | 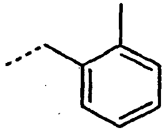 | (ESI)+: [M+H]+= 583.4 |
| 1133 | 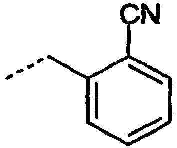 | (ESI)+: [M+H]+= 594.4 |
| 1134 | 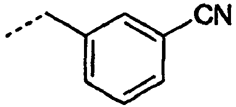 | (ESI)+: [M+H]+= 594.4 |
| 1135 | 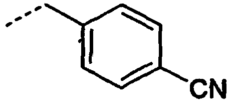 | (ESI)+: [M+H]+= 594.2 |
| 1136 | 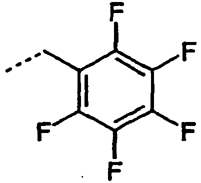 | (ESI)+: [M+H]+= 659.4 |
| 1137 | 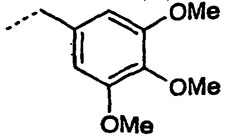 | (ESI)+: [M+H]+= 659.5 |

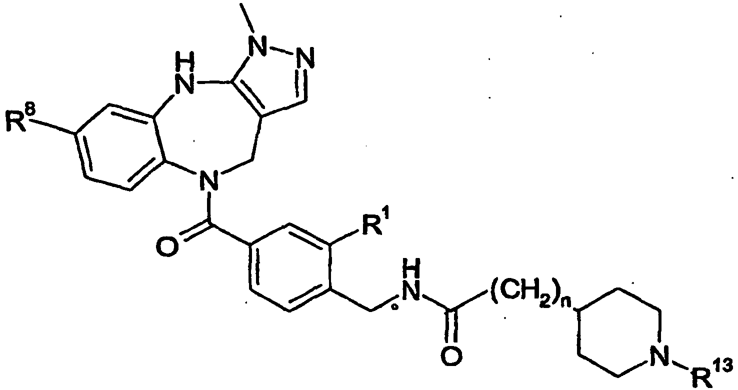

| **Compound number** | **R¹** | **R⁸** | **R¹³** | **n** | **MS** |
|---|---|---|---|---|---|
| 1138 E94 | F | Me | CH₂(CH₂)₄CH₃ | 1 | (ESI)+: [M+H]+= 575.7 |
| 1139 | Cl | H | CH₂(CH₂)₄CH₃ | 0 | (ESI)+: [M+H]+= 563.3 |
| 1140 | Cl | H | CH₂(CH₂)₂CH₃ | 0 | (ESI)+: [M+H]+= 535.3 |
| 1141 | Cl | H | CH₂(₄CH₂)₃OH | 0 | (ESI)+: [M+H]+= 551.3 |
| 1142 | Cl | H | CH₂(CH₂)₂CN | 0 | (ESI)+: [M+H]+= 546.3 |
| 1143 | Cl | H | 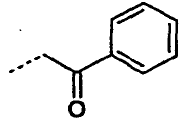 | 0 | (ESI)+: [M+H]+= 597.3 |
| 1144 | Cl | H | 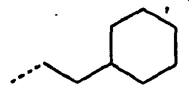 | 0 | (ESI)+: [M+H]+= 589.3 |
| 1145 | Cl | H | 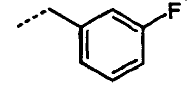 | 0 | (ESI)+: [M+H]+= 587.3 |
| 1146 | Cl | H | 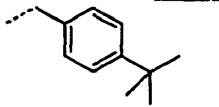 | 0 | (ESI)+: [M+H]+= 625.4 |
| 1147 | Cl | H | 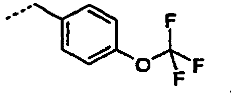 | 0 | (ESI)+: [M+H]+= 653.3 |
| 1148 | Cl | H | 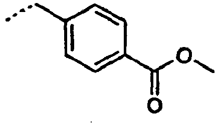 | 0 | (ESI)+: [M+H]+= 627.3 |
| 1149 | Cl | H | 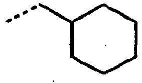 | 0 | (ESI)+: [M+H]+= 575.3 |
| 1150 | Cl | H | CH₂CO₂CH(CH₃)₂ | 0 | (ESI)+: [M+H]+= 579.3 |
| 1151 | Cl | H | CH₂CH₂CN | 0 | (ESI)+: [M+H]+= 532.2 |
| 1152 | Cl | H | CH₂CH₂CH₂OH | 0 | (ESI)+: [M+H]+= 537.3 |
| 1153 | Cl | H | CH₂CN | 0 | (ESI)+: [M+H]+= 518.2 |
| 1154 | Cl | H | 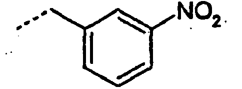 | 0 | (ESI)+: [M+H]+= 614.3 |
| 1155 | Cl | H | 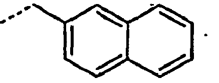 | 0 | (ESI)+: [M+H]+= 619.3 |
| 1156 | Cl | H | 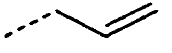 | 0 | (ESI)+**:** [M+H]+= 519.2 |
| 1157 | Cl | H | 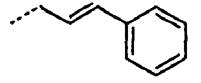 | 0 | (ESI)+: [M+H]+= 595.3 |
| 1158 | Cl | H | 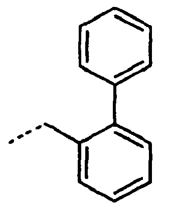 | 0 | (ESI)+: [M+H]+= 645.4 |
| 1159 | Cl | H | 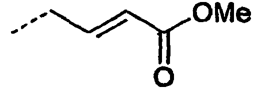 | 0 | (ESI)+: [M+H]+= 577.3 |
| 1160 | Cl | H | CH₂COC(CH₃)₃ | 0 | (ESI)+: [M+H]+= 577.3 |
| 1161 | Cl | H | CH₂CONH₂ | 0 | (ESI)+: [M+H]+= 536.2 |
| 1162 | Cl | H | 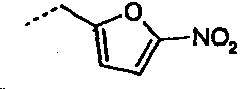 | 0 | (ESI)+: [M+H]+= 604.3 |
| 1163 | Cl | H | 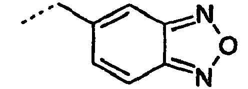 | 0 | (ESI)+: [M+H]+= 611.3 |
| 1164 | F | Me | CH₂(CH₂)₂CH₃ | 1 | (ESI)+: [M+H]+= 547.6 |
| 1165 | F | Me | CH₂(CH₂)₃OH | 1 | (ESI)+: [M+H]+= 563.7 |
| 1166 | F | Me | CH₂(CH₂)₂CN | 1 | (ESI)+: [M+H]+= 558.8 |
| 1167 | F | Me | 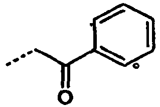 | 1 | (ESI)+: [M+H]+= 609.8 |
| 1168 | F | Me | 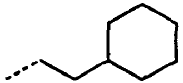 | 1 | (ESI)+: [M+H]+= 601.8 |
| 1169 | F | Me | 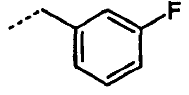 | 1 | (ESI)+: [M+H]+= 599.7 |
| 1170 | F | Me | 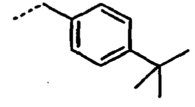 | 1 | (ESI)+: [M+H]+= 637.9 |
| 1171 | F | Me | 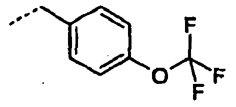 | 1 | (ESI)+: [M+H]+= 665.7 |
| 1172 | F | Me | 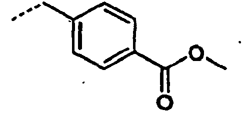 | 1 | (ESI)+: [M+H]+= 639.8 |
| 1173 | F | Me | 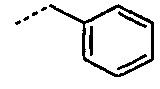 | 1 | (ESI)+: [M+H]+= 581.7 |
| 1174 | F | Me | 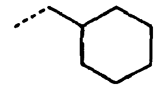 | 1 | (ESI)+: [M+H]+= 587.8 |
| 1175 | F | Me | CH₂CO₂CH(CH₃)₂ | 1 | (ESI)+: [M+H]+= 591.7 |
| 1176 | F | Me | CH₂CH₂CH(CH₃)₂ | 1 | (ESI)+: [M+H]+= 561.6 |
| 1177 | F | Me | CH₂CH₂CH₂OH | 1 | (ESI)+: [M+H]+= 549.6 |
| 1178 | F | Me | CH₂CH₂OCH₂CH₃ | 1 | (ESI)+: [M+H]+= 563.5 |
| 1179 | F | Me | CH₂CH₂CH₂F | 1 | (ESI)+: [M+H]+= 551.5 |
| 1180 | F | Me | 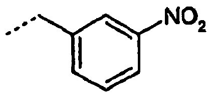 | 1 | (ESI)+: (M+H]+= 626.8 |
| 1181 | F | Me | 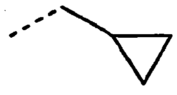 | 1 | (ESI)+: [M+H]+= 545.6 |
| 1182 | F | Me | 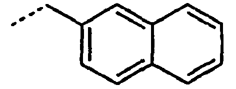 | 1 | (ESI)+: [M+H]+= 631.8 |
| 1183 | F | Me | 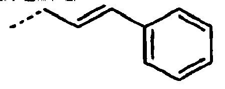 | 1 | (ESI)+: [M+H]+= 607.7 |
| 1184 | F | Me | 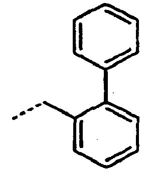 | 1 | (ESI)+: [M+H]+= 657.7 |
| 1185 | F | Me | 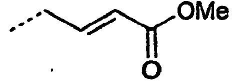 | 1 | (ESI)+: [M+H]+= |
| 1186 | F | Me | 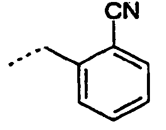 | 1 | (ESI)+: [M+H]+= 606.8 |
| 1187 | F | Me | CH₂COC(CH₃)₃ | 1 | (ESI)+: [M+H]+= 589.7 |
| 1188 | F | Me | CH₂CONH₂ | 1 | (ESI)+: [M+H]+= 548.5 |
| 1189 | F | Me | 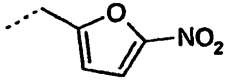 | 1 | (ESI)+: [M+H]+= 616.7 |
| 1190 | F | Me | 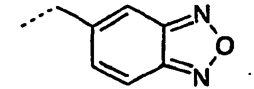 | 1 | (ESI)+: [M+H]+= 623.6 |

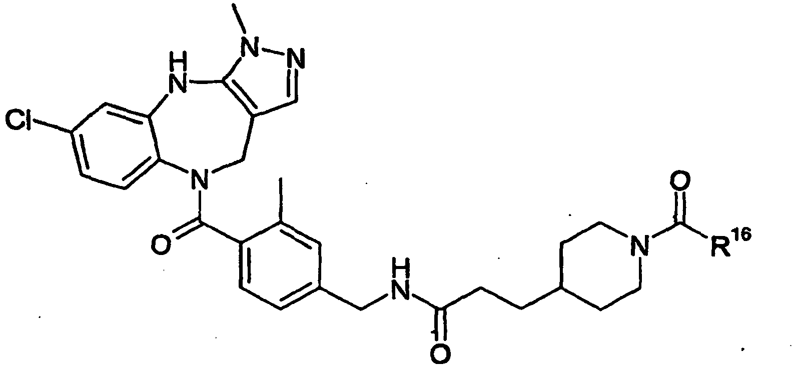

| **Compound number** | **R¹⁶** | **MS** |
|---|---|---|
| 1191 E95 | CH₂CH₃ | (ESI)+: [M+H]+= 577.4 |
| 1192 | 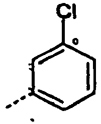 | (ESI)+: [M+H]+= 660.7 |
| 1193 | 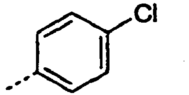 | (ESI)+: [M+H]+= 660.4 |
| 1194 | 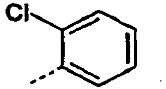 | (ESI)+: [M+H]+= 659.5 |
| 1195 | 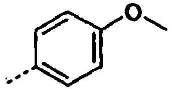 | (ESI)+: [M+H]+= 655.5 |
| 1196 | 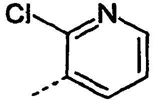 | (ESI)+: [M+H]+= 660.6 |
| 1197 | 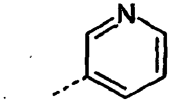 | (ESI)+: [M+H]+= 626.3 |
| 1198 | 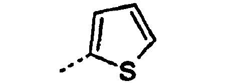 | (ESI)+: [M+H]+= 631.4 |
| 1199 | 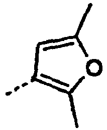 | (ESI)+: [M+H]+= 643.3 |
| 1200 | CH₂CH₂CH₃ | (ESI)+: [M+H]+= 591.3 |
| 1201 | 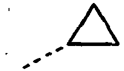 | (ESI)+: [M+H]+= 589.4 |
| 1202 | C(CH₃)₃ | (ESI)+: [M+H]+= 605.2 |
| 1203 | CH₂(CH₂)₃CH₃ | (ESI)+: [M+H]+= 619.3 |
| 1204 | CH(CH₂CH₃)CH₂CH₂CH₂C H₃ | (ESI)+: [M+H]+= 648.7 |
| 1205 | 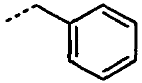 | (ESI)+: [M+H]+= 639.4 |
| 1206 | 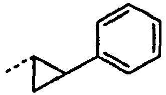 | (ESI)+: [M+H]+= 665.5 |
| 1207 | 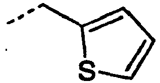 | (ESI)+: [M+H]+= 645.3 |
| 1208 | 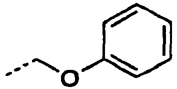 | (ESI)+: [M+H]+= 655.4 |
| 1209 | CH₂CH₂CH₂CO₂CH₂CH₃ | (ESI)+: [M+H]+= 663.5 |

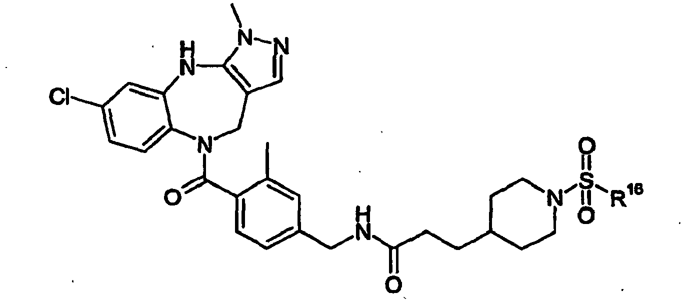

| **Compound number** | **R¹⁶** | **MS** |
|---|---|---|
| 1210 E96 | CH₂CH₃ | |
| 1211 | 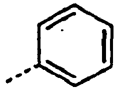 | (ESI)+: [M+H]+= 661.2 |
| 1212 | 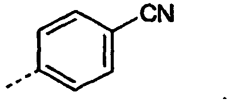 | (ESI)+: [M+H]+= 686.2 |
| 1213 | 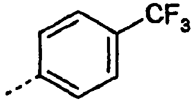 | (ESI)+: [M+H]+= 729.3 |
| 1214 | 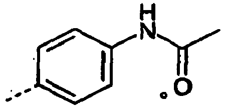 | (ESI)+: [M+H]+= 718.4 |
| 1215 | 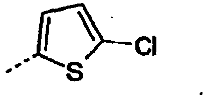 | (ESI)+: [M+H]+= 701.2 |
| 1216 | 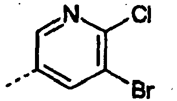 | (ESI)+: [M+H]+= 776.4 |
| 1217 | CH₂CH₂CH₂CH₃ | (ESI)+: [M+H]+= 641.4 |

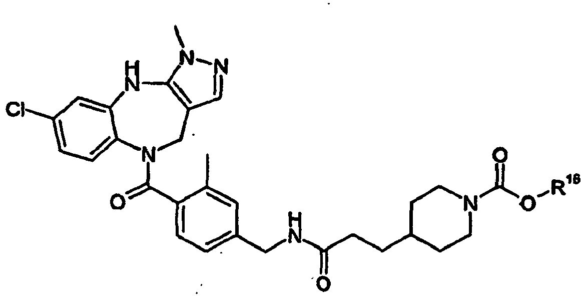

| **Compound** number | **R¹⁶** | **MS** |
|---|---|---|
| 1218 E97 | 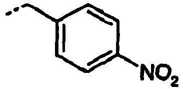 | |
| 1219 | 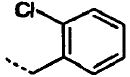 | (ESI)+: [M+H]+ = 689.4, 691.4 |
| 1220 | 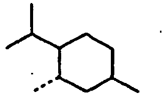 | (ESI)+: [M+H]+ = 703.6, 705.6 |
| 1221 | 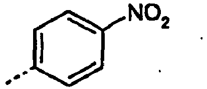 | (ESI)+: [M+H]+ = 686.3, 688.3 |
| 1222 | 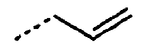 | (ESI)+: [M+H]+ = 605.4, 607.4 |
| 1223 | 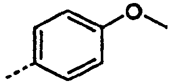 | (ESI)+: [M+H]+ = 671.3 |
| 1224 | 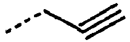 | (ESI)+: [M+H]+ = 603.4, 605.4 |
| 1225 | 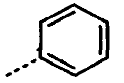 | (ESI)+: [M+H]+ = 641.5 |
| 1226 | 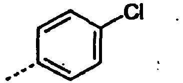 | (ESI)+: [M+H]+ = 675.4, 677.4 |
| 1227 | 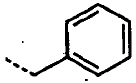 | (ESI)+: [M+H]+ = 655.5, 657.5 |
| 1228 | 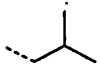 | (ESI)+: [M+H]+ = 621.3, 623.3 |
| 1229 | 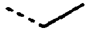 | (ESI)+: [M+H]+ = 593.4 |
| 1230 | 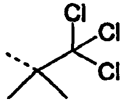 | (ESI)+: [M+H]+ = 725.4, |
| 1231 | CH₃ | (ESI)+: [M+H]+ = 579.4, 581.4 |
| 1232 | 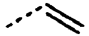 | (ESI)+: [M+H]+ = 591.1 |
| 1233 | 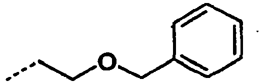 | (ESI)+: [M+H]+ = 699.5, 701.5 |

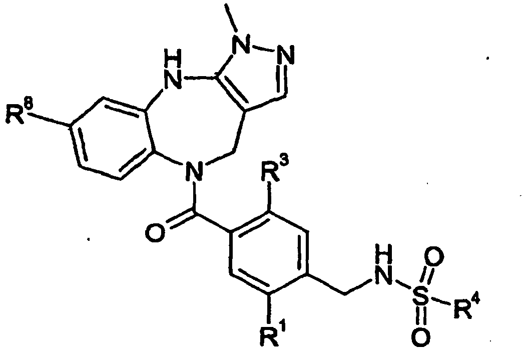

| **Compound number** | **R¹** | **R³** | **R⁴** | **R⁸** | **MS** | **¹H NMR: δ (ppm)** |
|---|---|---|---|---|---|---|
| 1234 E98 | F | H | CH₂CH₂CH₃ | Me | (APCI )+:[M +H]+= 472.4 | 0.96 (3H, t, J=7.4Hz), 1.65-1.78 (2H, m), 2.21 (3H, s), 2.83 (2H, t, J=7.7Hz), 3.75 (3H, s), 3.95 (1H, d, J=14.6Hz), 4.21 (2H, d, J=6.2Hz), 4.90 (1H, brs), 5.83 (1 H, d, J=14.6Hz), 6.24 (1H, s), 6.48-6.58 (2H, m), 6.77 (1H, s), 6.93-7.02 (2H, m), 7.06-7.12 (1H, m), 7.21 (1H, s) |
| 1235 | Cl | H | CH₂CH₂CH₃ | Cl | (APCI )+:[M +H]+= 508.3 | 0.97 (3H, t, J=7.4Hz), 1.68-1.83 (2H, m), 2.86 (2H, t, J=7.4Hz), 3.69 (3H, s), 3.95 (1H, d, J=14.6Hz), 4.26 (2H, d, J=6.4Hz), 5.56 (1H, t, J=6.4Hz), 5.80 (1H, d, J=14.6Hz), 6.58-6.62 (2H, m), 6.94 (1 H, dd, J=1.5, 7.9Hz), 7.04 (1H, s), 7.09 (1H, s), 7.16-7.20 (2H, m), 7.33 (1H, d, J=1.5Hz) |
| 1236 | F | H | CH₂CH₂CH₃ | Cl | (APCI )+:[M +H]+= 492.3, 494.3 | 0.97 (3H, t, J=7.4Hz), 1.68-1.79 (2H, m), 2.85, (2H, t, J=7.4Hz), 3.77 (3H, s), 3.95 (1H, d, J=14.6Hz), 4.25 (2H, d, J=6.2Hz), 4.80 (1H, t, J=6.2Hz), 5.84 (1H, d, J=14.6Hz), 6.38 (1H, s), 6.60-6.66 (2H, m), 6.94-7.03 (3H, m), 7.17-7.24 (2H, m) |

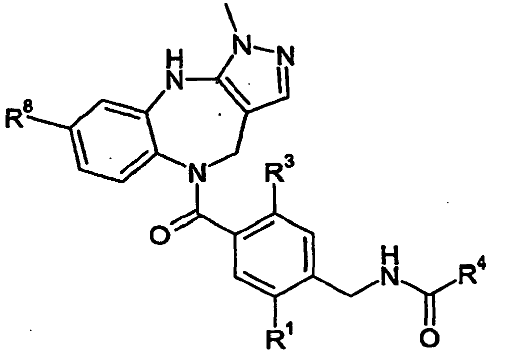

| **Compound number** | **R¹** | **R³** | **R⁴** | **R⁸** | **MS** | **¹H NMR: δ (ppm)** |
|---|---|---|---|---|---|---|
| 1237 E99 | F | H | H | Me | (ESI)+ :[M+H ]+= 394.2 | 2.20 (3H, s), 3.75 (3H, s), 3.94 (1H, d, J=14.6Hz), 4.30-4.40 (2H, m), 5.82 (1H, d, J=14.6Hz), 6.31-6.42 (1H, m), 6.46 (1H, s), 6.47-6.59 (2H, m), 6.78 (1H, s), 6.88-7.03 (3H, m), 7.21 (1H, s), 8.16 (1H, s) |
| 1238 | Cl | H | H | Cl | (ESI)+ [M+H] += 430.2 | 3.69 (3H, s), 3.95 (1H, d, J=14.6Hz), 4.40-4.55 (2H, m), 5.83 (1H, d, J=14.6Hz), 6.28-6.41 (1H, m), 6.56-6.68 (2H, m), 6.78 (1H, s), 6.90-7.14 (3H, m), 7.23 (1H, s), 7.30 (1H, s), 8.20 (1H, s) |
| 1239 | F | H | H | Cl | (APCI )+: [M+H] += 414.2, 416.2 | 3.72 (3H, s), 3.96 (1H, d, J=14.6Hz), 4.33-4.44 (2H, m), 5.81 (1H, d, J=14.6Hz), 6.52-6.56 (1H, m), 6.61 (2H, s), 6.87-6.94 (2H, m), 7.01-7.07 (2H, m), 7.13 (1H, s), 7.21 (1H, s), 8.16 (1H, s) |

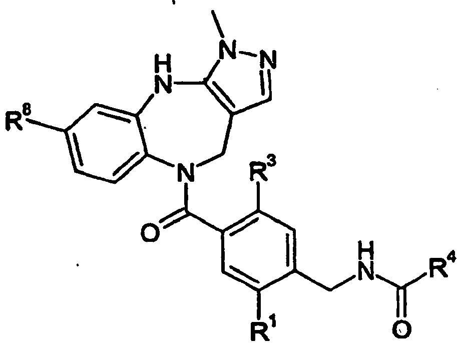

| **Compound number** | **R¹** | **R³** | **R⁴** | **R⁸** | **MS** | **¹H NMR: δ (ppm)** |
|---|---|---|---|---|---|---|
| 1240 E100 | F | H | 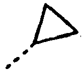 | Me | (APCI )+:[M +H]+= 434.2 | 0.65-0.69 (2H, m), 0.85-0.88 (2H, m), 1.37-1.43 (1H, m), 2.08 (3H, s), 3.64 (3H, s), 3.94 (1H, d, J=14.6Hz), 4.28-4.31 (2H, m), 5.81 (1H, d, J=14.6Hz), 6.46 (1H, d, J=8.4Hz), 6.53 (1H, d, J=7.9Hz), 6.78-6.96 (4H, m), 7.15 (1H, s), 7.17 (1H, s) |
| 1241 E101 | Me | H | 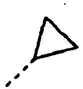 | Me | (ESI)+ :[M+H ]+= 430.4 | 0.62-0.71 (2H, m), 0.81-0.89 (2H, m), 1.39-1.50 (1H, m), 2.04 (3H, s), 2.12 (3H, s), 3.66 (3H, s), 3.91 (1H, d, J=14.6Hz), 4.22 (2H, d, J=2.0Hz), 5.82 (1 H, d, J=14.6Hz), 6.46 (2H, q, J=7.9 Hz), 6.68-6.87 (4H, m), 7.06 (2H, s) |
| 1242 E102 | F | H | 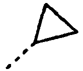 | Cl | (APCI )+:[M +H]+= 454.2, 456.2 | 0.62-0.80 (2H, m), 0.88-1.00 (2H, m), 1.31-1.47 (1H, m), 3.69 (3H, s), 3.95 (1H, d, J=14.6Hz), 4.35 (2H, d, J=5.4Hz), 5.82 (1H, d, J=14.6Hz), 6.51 (1 H, t, J=5.4Hz), 6.61 (2H, s), 6.82-7.08 (4H, m), 7.17 (1H, s), 7.21 (1H, s) |
| 1243 E103 | F | H | CH₂CH₃ | Cl | (ESI)+ :[M+H ]+= 442.1, 444.2 | 1.12 (3H, t, J=7.7Hz), 2.21 (2H, q, J=7.7Hz), 3.76 (3H, s), 3.95 (1H, d, J=14.6Hz), 4.37 (2H, d, J=5.7Hz), 5.83 (1H, d, J=14.6Hz), 5.94 (1H, t, J=5.7Hz), 6.58-6.67 (3H, m), 6.89-7.00 (3H, m), 7.07 (1 H, t, J=7.4Hz), 7.23 (1H, s) |
| 1244 E104 | **F** | H | CH₂CH₂CH₃ | Cl | (APCI )+:[M +H]+= 456.3, 458.3 | 0.88 (3H, t, J=7.4Hz), 1.56-1.69 (2H, m), 2.15 (2H, t, J=7.2Hz), 3.71 (3H, s), 3.96 (1 H, d, J=14.6Hz), 4.35 (2H, d, J=5.7Hz), 5.83 (1H, d, J=14.6Hz), 6.23 (1H, t, J=5.7Hz), 6.59-6.64 (2H, m), 6.87-6.94 (2H, m), 7.01-7.06 (2H, m), 7.19 (1H, s), 7.22 (1H, s) |
| 1245 E105 | **F** | H | CH(CH₃)₂ | Cl | (APCI )+:[M +H]+= 456.3, 458.2 | 1.11 (6H, d, J=6.9Hz), 2.29-2.42 (1H, m), 3.73 (3H, s), 3.95 (1 H, d, J=14.6Hz), 4.35 (2H, d, J=5.7Hz), 5.83 (1H, d, J=14.6Hz), 6.06 (1H, t, J=5.7Hz), 6.60-6.63 (2H, m), 6.88-7.08 (5H, m), 7.22 (1H, s) |
| 1246 E106 | Cl | H | 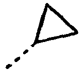 | Cl | (APCI )+:[M +H]+= 470.2 | 0.71-0.74 (2H, m), 0.90-0.91 (2H, m), 1.40-1.43 (1H, m), 3.67 (3H, s), 3.94 (1H, d, J=14.6Hz), 4.38 (2H, d, J=5.4Hz), 5.81 (1H, d, J=14.6Hz), 6.56 (3H, m), 6.90-7.04 (4H, m), 7.21 (1H, s), 7.26 (1H, s) |
| 1247 E107 | Cl | H | CH₂CH₂CH₃ | Cl | (APCI )+:[M +H]+= 472.3 | 0.87 (3H, t, J=7.4Hz), 1.60-1.65 (2H, m), 2.16 (2H, t, J=7.7Hz), 3.67 (3H, s), 3.95 (1H, d, J=14.6Hz), 4.36 (2H, t, J=3.5Hz), 5.79 (1H, d, J=14.6Hz), 6.59 (2H, s), 6.91 (1H, d, J=7.7Hz), 6.98 (1H, d, J=7.7Hz), 7.03 (2H, s), 7.20 (1H, s), 7.23 (1H, s), 7.60 (1H, s) |
| 1248 E108 | Cl | H | CH(CH₃)₂ | Cl | (APCI )+:[M +H]+= 472.3 | 1.09-1.13 (6H, m), 2.30-2.50 (1H, m), 3.70 (3H, s), 3.95 (1H, d, J=14.6Hz), 4.37 (2H, d, J=4.0Hz), 5.81 (1H, d, J=14.6Hz), 6.25 (1H, t, J=5.9Hz), 6.60 (2H, s), 6.85-7.04 (3H, m), 7.22 (1H, s), 7.26 (2H, s) |
| 1249 | Me | H | CH(CH₃)₂ | Cl | (ESI)+ [M+H] + = 452.2, 454.2 | 1.14 (6H, d, J=6.7Hz), 2.14 (3H, s), 2.27-2.42 (1H, m), 3.74.(3H, s), 3.94 (1H, d, J=14.5Hz), 4.30 (2H, d, J=5.2Hz), 5.70 (1H, s), 5.87 (1H, d, J=14.5Hz), 6.57-6.67, (3H, m), 6.88 (2H, s), 6.98 (2H, s), 7.13(1H, s) |
| 1250 | Me | H | CH₂CH₂CH₃ | Cl | (ESI)+ : [M+H] + = 452.2, 454.2 | 0.92 (3H, t, J=7.3Hz), 1.64 (2H, q, J=7.3Hz), 2.12-2.24 (5H, m), 3.75 (3H, s), 3.94 (1H, d, J=14.6Hz), 4.32 (2H, d, J=5.0Hz), 5.64 (1H, s), 5.87 (1H, d, J=14.6Hz), 6.40 (1H, s), 6.62 (2H, s), 6.89 (2H, s), 6.97 (2H, s), 7.14 (1H, s) |
| 1251 | Me | H | CH₂CH₃ | Cl | (ESI)+ : [M+H] + = 438.2, 440.2 | 1.14 (3H, t, J=7.3Hz), 2.15 (3H, s), 2.23 (2H, q, J=7.3Hz), 3.74 (3H, s), 3.93 (1H, d. J=14.9Hz), 4.31 (2H, d, J=5.2Hz), 5.71 (1H, s), 5.87 (1H, d, J=14.9Hz), 6.55 (1H, s), 6.62 (2H, s), 6.88 (2H, s), 6.98 (2H, s), 7.13 (1H, s) |
| 1252 | Me | H | CH₂CH₂CH₃ | Me | (ESI)+ [M+H] += 433.2 | 0.91 (3H, t, J=7.4Hz), 1.65 (2H, q, J=7.4Hz), 2.13 (3H, s), 2.11-2.30 (2H, m), 2.21 (3H, s), 3.76 (3H, s), 3.93 (1H, d, J=14.6Hz), 4.29 (2H, d, J=5.2Hz), 5.55-5.70 (1H, m), 5.87 (1H, d, J=14.6Hz), 6.09 (1H, s), 6.52 (2H, q, J=7.7Hz), 6.74 (1H, s), 6.80-6.93 (2H, m), 7.14 (1H, s), 7.23 (1H, s) |
| 1253 | Me | H | CH(CH₃)₂ | Me | (ESI)+ [M+H] += 433.2 | 1.12 (6H, d, J=6.7Hz), 2.13 (3H, s), 2.21 (3H, s), 2.25-2.42 (1H, m), 3.76 (3H, s), 3.93 (1 H, d, J=14.6Hz), 4.29 (2H, d, J=5.4Hz), 5.55-5.70 (1H, m), 5.88 (1H, d, J=14.6Hz), 6.10 (1H, s), 6.52 (2H, q, J=7.9Hz), 6.74 (1H, s), 6.75-6.93 (2H, m), 7.14 (1H, s), 7.23 (1H, s) |
| 1254 | Me | H | CH₂CH₃ | Me | (ESI)+ [M+H] += 418.2 | 1.13 (3H, t, J=7.4Hz), 2.12 (3H, s), 2.18-2.28 (5H, m), 3.76 (3H, s), 3.93 (1H, d, J=14.6Hz), 4.29 (2H, d, J=5.2Hz), 5.57-5.70 (1H, m), 5.87 (1H, d, J=14.6Hz), 6.07 (1H, s), 6.52 (2H, q, J=7.9Hz), 6.74 (1H, s), 6.80-6.92 (2H, m), 7.14 (1H, s), 7.23 (1H, s) |
| 1255 | Me | H | CH₂CH₃ | F | (APCI )+: [M+H] += 422.4 | 1.13 (3H, t, J=7.4Hz), 2.12 (3H, s), 2.20 (2H, q, J=7.4Hz), 3.70 (3H, s), 3.94 (1H, d, J=14.6Hz), 4.28 (1H, d, J=5.2Hz), 5.83 (1H, s), 5.85 (1H, d, J=14.6Hz), 6.33-6.39 (1H, m), 6.61-6.70 (2H, m), 6.77-6.86 (3H, m), 7.10 (1H, s), 7.23 (1H, s) |
| 1256 | Me | H | CH₂CH₂CH₃ | F | (APCI )+: [M+H] += 436.4 | 0.91 (3H, t, J=7.4Hz), 1.59-1.68 (2H, m), 2.10-2.20 (5H, m), 3.71 (3H, s), 3.94 (1H, d, J=14.6Hz), 4.29 (2H, d, J=5.2Hz), 5.79 (1H, s), 5.86 (1H, d, J=14.6Hz), 6.33-6.42 (1H, m), 6.61-6.72 (3H, m), 6.83-6.88 (2H, m), 7.11 (1H, s), 7.23 (1H, s) |
| 1257 | Me | H | CH(CH₃)₂ | F | (APCI )+: [M+H] +**=** 436.4 | 1.12 (6H, d, J=6.4Hz), 2.12 (3H, s), 2.30-2.41 (1H, m), 3.71 (3H, s), 3.94 (1H, d, J=14.6Hz), 4.28 (2H, d, J=5.2Hz), 5.75 (1H, s), 5.86 (1H, d, J=14.6Hz), 6.33-6.39 (1H, m), 6.62-6.73 (3H, m), 6.80-6.92 (2H, m), 7.10 (1H, s), 7.23 (1H, s) |
| 1258 | Me | H | 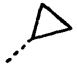 | F | (APCI )+: [M+H] += 434.4 | 0.71-0.74 (2H, m), 0.91-0.96 (2H, m), 1.35-1.37 (1H, m), 2.13 (3H, s), 3.70 (3H, s), 3.94 (1H, d, J=14.6Hz), 4.30 (2H, d, J=5.2Hz), 5.86 (1H, d, J=14.6Hz), 5.99 (1H, s), 6.34-6.40 (1H, m), 6.62-6.70 (3H, m), 6.83-6.91 (2H, m), 7.11 (1H, s) |
| 1259 | **F** | H | 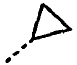 | **F** | (APCI )+: [M+H] += 438.4 | 0.71-0.75 (2H, m), 0.91-0.95 (2H, m), 1.31-1.37 (1H, m), 3.74 (3H, s), 3.96 (1H, d, J=14.6Hz), 4.38 (2H, d, J=5:7Hz), 5.84 (1H, d, J=14.6Hz), 6.14 (1H, s), 6.37-6.43 (1H, m), 6.64-6.69 (3H, m), 6.89-6.95 (2H, m), 7.04-7.10 (1H, m) |
| 1260 | F | H | CH(CH₃)2 | F | (APCI )+: [M+H] += 440.4 | 1.11 (6H, d, J=6.7Hz), 2.34 (1H, septet, J=6.7Hz), 3.76 (3H, s), 3.96 (1H, d, J=14.6Hz), 4.36 (2H, d, J=5.9Hz), 5.84 (1H, d, J=14.6Hz), 5.87 (1H, s), 6.37-6.43 (1H, m), 6.51 (1H, s), 6.64-6.69 (2H, m), 6.91-6.95 (2H, m), 7.04-7.10 (1H, m) |
| 1261 | F | H | CH₂CH₂CH₃ | **F** | (APCI [M+H] += 440.4 | 0.88 (3H, t, J=7.2Hz), 1.58-1.66 (2H, m), 2.14 (2H, t, J=7.4Hz), 3.74 (3H, s), 3.96 (1H, d, J=14.6Hz), 4.36 (2H, d, J=5.2Hz), 5.83 (1 H, d, J=14.6Hz), 6.01 (1 H, s), 6.36-6.41 (1H, m), 6.63-6.71 (2H, m), 6.79 (1H, s), 6.91-6.94 (2H, m), 7.03-7.09 (1H, m) |
| 1262 | F | H | CH₂CH₃ | **F** | (APCI )+: [M+H] += 426.4 | 1.12 (3H, t, J=7.4Hz), 2.20 (2H, q, J=7.4Hz), 3.76 (3H, s), 3.96 (1H, d, J=14.6Hz), 4.37 (2H, d, J=5.4Hz), 5.83 (1H, d, J=14.6Hz), 5.87 (1H, s), 6.37-6.47 (2H, m), 6.64-6.69 (2H, m), 6.92-6.95 (2H, m), 7.05-7.11 (1H, m) |
| 1263 | F | H | 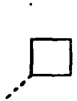 | Me | (APCI )+:[M +H]+= 448.4 | 1.72-1.91 (3H, m), 2.05-2.28 (4H, m), 2.92-3.06 (1H, m), 3.46 (2H, s), 3.72 (3H, s), 3.94 (1H, d, J=14.6Hz), 4.31 (2H, d, J=5.7Hz), 5.83 (1H, d, J=14.6Hz), 6.05 (1H, t, J=5.7Hz), 6.47 (1H, d, J=7.7Hz), 6.54 (1H, d, J=7.7Hz), 6.68 (1H, s), 6.78 (1H, s), 6.82-7.00 (3H, m), 7.20 (1H, s) |
| 1264 | F | H | 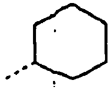 | Me | (APCl )+: [M+H] += 476.4 | 1.10-1.48 (5H, m), 1.60-1.70 (1H, m), 1.70-1.84 (4H, m), 2.00-2.14 (1H, m), 2.19 (3H, s), 3.74 (3H, s), 3.94 (1H, d, J=14.6Hz), 4.32 (2H, d, J=5.9Hz), 5.83 (1H, d, J=14.6Hz), 6.03 (1H, t, J=6.0Hz), 6.44-6.59 (3H, m), 6.77 (1H, s), 6.88-7.02 (3H, m), 7.21 (1H, s) |
| 1265 | F | H | CH₂CH₂CH₃ | Me | (APCI )+: [M+H] += 436.4 | 0.86 (3H, t, J=7.4Hz), 1.53-1.68 (2H, m), 2.13 (2H, q, J=7.4Hz), 2.17 (3H, s), 3.71 (3H, s), 3.94 (1 H, d, J=14.6Hz), 4.31 (2H, d, J=5.4Hz), 5.83 (1H, d, J=14.6Hz), 6.29 (1H, t, J=6.7Hz), 6.47 (1H, d, J=8.2Hz), 6.55 (1H, d, J=8.2Hz), 6.76 (2H, d, J=6.4Hz), 6.87-6.97 (3H, m), 7.20 (1H, s) |
| 1266 | F | H | Me | Me | (APCI )+: [M+H] += 408.4 | 1.92 (3H, s), 2.17 (3H, s), 3.70 (3H, s), 3.94 (1H, d, J=14.6Hz), 4.29 (2H, d, J=5.4Hz), 5.81 (1H, d, J=14.6Hz), 6.42-6.61 (3H, m), 6.85 (2H, d, J=6.9Hz), 6.87-7.00 (3H, m), 7.20 (1H, s) |
| 1267 | F | H | CH(CH₃)₂ | Me | (APCI )+: [M+H] += 436.4 | 1.08-1.11 (6H, m), 2.16 (3H, s), 2.28-2.42 (1H, m), 3.71 (3H, s), 3.94 (1H, d, J=14.6Hz), 4.31 (2H, d, J=4.2Hz), 5.82 (1H, d, J=14.6Hz), 6.23 (1H, t, J=5.9Hz), 6.47 (1H, d, J=7.9Hz), 6.55 (1H, d, J=7.9Hz), 6.77 (2H, d, J=2.5Hz), 6.85-7.03 (3H, m), 7.20 (1H, s) |
| 1268 | F | H | C(CH₃)₃ | Me | (APCI )+: [M+H] += 450.4 | 1.15 (9H, s), 2.16 (3H, s), 3.71 (3H, s), 3.94 (1 H, d, J=14.6Hz), 4.32 (2H, d, J=5.7Hz), 5.83 (1H, d, J=14.6Hz), 6.10-6.23 (1H, m), 6.46 (1H, d, J=7.9Hz), 6.55 (1 H, d, J=7.9Hz), 6.68 (1H, s), 6.76 (1H, s), 6.85-7.05 (3H, m), 7.20 (1H, s) |
| 1269 | Cl | H | 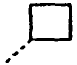 | Cl | (APCI )+: [M+H] += 484.3 | 1.78-2.04 (2H, m), 2.04-2.32 (4H, m), 2.92-3.10 (1H, m), 3.73 (3H, s), 3.95 (1H, d, J=14.6Hz), 4.39 (2H, d, J=3.2Hz), 5.83 (1H, d, J=14.6Hz), 6.01 (1H, t, J=5.7Hz), 6.62 (2H, s), 6.85-7.10 (4H, m), 7.23 (1 H, s), 7.27 (1H, s) |
| 1270 | Cl | H | 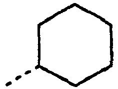 | Cl | (APCI )+: [M+H] += 512.4 | 1.23 (2H, t, J=7.2Hz), 1.26-1.50 (3H, m), 1.59-1.71 (1H, m), 1.71-1.83 (5H, m), 2.12-2.28 (1H, m), 3.79 (3H, s), 3.97 (1H, d, J=14.6Hz), 4.33 (2H, d, J=1.5Hz), 5.74 (1H, d, J=14.6Hz), 6.63-6.68 (2H, m), 7.08-7.13 (2H, m), 7.20 (1H, s), 7.21-7.23 (2H, m) |
| 1271 | Cl | H | 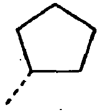 | Cl | (APCI )+: [M+H] += 498.3 | 1.43-1.58 (1H, m), 1.58-1.78 (5H, m), 1.58-1.90 (2H, m), 2.46-2.60 (1 H, m), 3.72 (3H, s), 3.95 (1H, d, J=14.6Hz), 4.30-4.50 (2H, m), 5.83 (1H, d, J=14.6Hz), 6.12 (1H, t, J=5.9Hz), 6.61 (2H, s), 6.84-7.11 (4H, m), 7.22 (1H, s), 7.27 (1H, s) |
| 1272 | Cl | H | Me | Cl | (APCI )+: [M+H] += 444.3 | 1.97 (3H, s), 3.67 (3H, s), 3.94 (1H, d, J=14.6Hz), 4.33 (2H, t, J=5.2Hz), 5.79 (1H, d, J=14.6Hz), 6.59 (2H, s), 6.60-6.70 (1H, m), 6.90 (1H, d, J=7.9Hz), 6.97 (1H, d, J=7.9Hz), 7.04 (1H, s), 7.19 (1H, s), 7.23 (1H, s), 7.58 (1H, s) |
| 1273 | F | H | 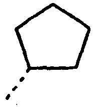 | Me | (APCI )+: [M+H] += 462.4 | 1.41-1.84 (8H, m), 2.13 s), 2.46-2.57 (1H, m), 3.67 (3H, s), 3.94 (1 H, d, J=14.6Hz), 4.28 (2H, d, J=5.2Hz), 5.81 (1H, d, J=14.6Hz), 6.43-6.55 (3H, m), 6.79 (1 H, s), 6.83-6.96 (3H, m), 7.12 (1H, s), 7.17 (1H, s) |
| 1274 | F | H | CH₂CH₃ | Me | (APCI )+: [M+H] += 422.4 | 1.08 (3H, t, J=7.7Hz), 2.15 (3H, s), 2.17 (2H, q, J=7.7Hz), 3.69 (3H, s), 3.93 (1H, d, J=14.6Hz), 4.30 (2H, d, J=5.2Hz), 5.81 (1H, d, J=14.6Hz), 6.38-6.57 (3H, m), 6.80-6.98 (5H, m), 7.19 (1H, s) |
| 1275 | Cl | H | C(CH₃)₃ | Cl | (APCI )+: [M+H] += 486.3 | 1.17 (9H, s), 3.72 (3H, s), 3.95 (1H, d, J=14.6Hz), 4.38 (2H, d, J=5.9Hz), 5.82 (1H, d, J=14.6Hz), 6.21 (1H, t, J=5.7Hz), 6.61 (2H, s), 6.85-7.08 (4H, m), 7.22-7.27 (2H, m) |
| 1276 | Cl | H | CH₂CH₃ | Cl | (APCI )+: [M+H] += 458.2 | 1.11 (3H, t, J=7.7Hz), 2.22 (2H, q, J=7.7Hz), 3.45 (1H, d, J=5.2Hz), 3.67 (3H, s), 3.95 (1H, d, J=14.6Hz), 4.23-4.45 (2H, m), 5.80 (1H, d, J=14.6Hz), 6.53 (1H, t, J=5.9Hz), 6.59 (2H, s), 6.85-6.92 (1H, m), 6.98 (1H, s), 6.99-7.04 (1H, m), 7.20 (1H, s), 7.59 (1H, s) |
| 1277 | F | H | 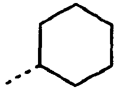 | Cl | (APCI )+: [M+H] += 496.3, 498.3 | 1.17-1.25 (2H, m), 1.25-1.42 (2H, m), 1.65-1.81 (6H, m), 2.02-2.18 (1 H, m), 3.73 (3H, s), 3.96 (1H, d, J=14.6Hz), 4.34 (2H, d, J=5.7Hz), 5.83 (1H, d, J=14.6Hz), 6.08 (1H, t, J=5.7Hz), 6.59-6.64 (2H, m), 6.87-6.94 (2H, m), 7.00-7.04 (2H, m), 7.06 (1H, s), 7.22 (1H, s) |
| 1278 | F | H | 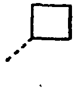 | Cl | (APCI )+: [M+H] += 468.3, 470.3 | 1.80-2.02 (2H, m), 2.03-2.31 (4H, m), 2.95-3.08 (1H, m), 3.71 (3H, s), 3.95 (1 H, d, J=14.6Hz), 4.34 (2H, d, J=5.7Hz), 5.83 (1H, d, J=14.6Hz), 6.06 (1 H, t, J=5.7Hz), 6.59-6.63 (2H, m), 6.87-6.94 (2H, m), 6.99-7.06 (2H, m), 7.22 (2H, s) |
| 1279 | F | H | Me | Cl | (APCI )+: [M+H] += 428.2, 430.2 | 1.96 (3H, s), 3.73 (3H, s), 3.95 (1H, d, J=14.6Hz), 4.35 (2H, d, J=5.7Hz), 5.83 (1H, d, J=14.6Hz), 6.23 (1H, t, J=5.7Hz), 6.58-6.66 (2H, m), 6.87-7.05 (5H, m), 7.22 (1H, |
| 1280 | F | H | C(CH₃)₃ | Cl | (APCI )+: [M+H] += 470.3, 472.3 | 1.16 (9H, s), 3.74 (3H, s), 3.95 (1 H, d, J=14.6Hz), 4.36 (2H, d, J=5.7Hz), 5.84 (1H, d, J=14.6Hz), 6.07 (1H, t, J=5.7Hz), 6.60-6.64 (2H, m), 6.79 (1H, s), 6.90-6.98 (3H, s), 7.02-7.08 (1H, m), 7.23 (1H, s) |
| 1281 | F | H | 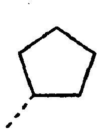 | Cl | (ESI)+ : [M+H] += 482.2, 484.2 | 1.31-1.88 (8H, m), 2.48-2.58 (1H, m), 3.72 (3H, s), 3.95 (1H, d, J=14.6Hz), 4.34 (2H, d, J=5.7Hz), 5.81 (1H, d, J=14.6Hz), 6.25 (1H, t, J=5.7Hz), 6.59 (2H, s), 6.87-6.93 (2H, m), 7.01 (1H, t, J=7.4Hz), 7.08 (1H, s), 7.21 (1H, s), 7.44 (1H, s) |
| 1282 | H | Cl | 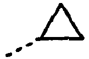 | Me | (APCI )+: [M+H] += 450.2, 452.3 | 0.70-0.74 (2H, m), 0.91-0.94 (2H, m), 1.39 (1H, m), 2.13 (3H, s), 3.70 (3H, s), 3.98 (1H, d, J=14.6Hz), 4.25 (2H, m), 5.79 (1H, d, J=14.6Hz), 6.32 (1H, s), 6.45-6.49 (2H, m), 6.65 (1 H, s), 6.74-6.83 (3H, m), 7.04 (1H, s), 7.23 (1H, s) |
| 1283 | H | Me | 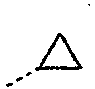 | Cl | (APCI )+: [M+H] += 450.2, 452.2 | 0.68-0.73 (2H, m), 0.90-0.94 (2H, m) 1.31-1.43 (1H, m), 2.25 (3H, s), 3.63 (3H, s), 3.95 (1H, d, J=14.6Hz), 4.23 (2H, d, J=5.6Hz), 4.41 (1H, d, J=5.6Hz), 5.80 (1H, d, J=14.6Hz), 6.39-6.46 (1H, m), 6.56-7.23 (7H, m) |
| 1284 | H | Me | 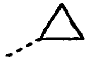 | Me | (APCI )+: [M+H] += 430.2 | 0.69-0.74 (2H, m), 0.93-0.96 (2H, m), 1.28-1.36 (1H, m), 2.15 (3H, s), 2.31 (3H, s), 3.47 (2H, d, J=5.6Hz), 3.72 (3H, s), 3.94 (1H, d, J=14.6Hz), 4.27 (1H, d, J=5.6Hz), 5.83 (1H, d, J=14.6Hz), 6.11 (1H, s), 6.41-6.46 (1H, m), 6.58-6.91 (6H, m) |
| 1285 | H | Cl | 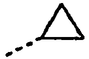 | Cl | (APCI )+: [M+H] += 470.2 | 0.68-0.76 (2H, m), 0.88-0.97 (2H, m), 1.42 (1H, m), 3.60 (3H, s), 3.98 (1H, d, J=14.6Hz), 4.19 (2H, m), 4.38 (1H, m), 5.73 (1H, d, J=14.6Hz), 6.52-6.56 (1H, m), 6.71-7.18 (6H, m), 7.60 (1H, brs) |
| 1286 | H | F | 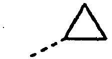 | Cl | (APCI )+: [M+H] += 454.2, 456.3 | 0.69-0.75 (2H, m), 0.91-0.94 (2H, m), 1.32-1.44 (1H, m), 3.66 (3H, s), 3.96 (1H, d, J=14.6Hz), 4.28 (2H, d, J=5.9Hz), 5.80 (1H, d, J=14.6Hz), 6.51-6.62 (2H, m), 6.69 (2H, t, J=8.4, 18.3Hz), 6.79 (1H, d, J=6.4Hz), 6.92 (2H, s), 6.98-7.09 (1H, m), 7.22 (1H, s) |
| 1287 | H | F | 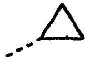 | Me | (APCI )+: [M+H] += 434.2 | 0.69-0.74 (2H, m), 0.91-0.96 (2H, m), 1.31-1.42 (1H, m), 2.15 (3H, s), 3.69 (3H, s), 3.96 (1H, d, J=14.6Hz), 4.27 (2H, t, J=5.7, 11_{.}1Hz), 5.81 (1H, d, J=14.6Hz), 6.32 (1 H, s), 6.42-6.47 (2H, m), 6.62-6.79 (3H, m), 7.02 (1H, t, J=7.4, 14.8Hz), 7.21 (1H, s) |
| 1288 | Cl | H | 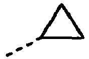 | Me | (APCI )+: [M+H] += 450.3, 452.4 | 0.63-0.75 (2H, m), 0.83-0.94 (2H, m), 1.37-1.46 (1H, m), 2.15 (3H, s), 3.68 (3H, s), 3.95 (1H, d, J=14.6Hz), 4.24-4.42 (2H, m), 5.81 (1H, d, J=14.6Hz), 6.47 (1H, d, J=8.2Hz), 6.54 (1H, d, J=8.2Hz), 6.74-6.79 (2H, m), 6.90-6.96 (2H, m), 7.19-7.23 (1H, m) |
| 1289 | Me | H | 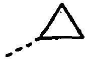 | Cl | (ESI)+ [M+H] += 450.3, 452.4 | 0.66-0.78 (2H, m), 0.82-0.94 (2H, m), 1.34-1.46 (1H, m), 2.08 (3H, s), 3.63 (3H, s), 3.93 (1H, d, J=14.6Hz), 4.26 (2H, d, J=4.0Hz), 5.83 (1H, d, J=14.6Hz), 6.47-6.62 (2H, m), 6.78-6.86 (2H, m), 7.01-7.08 (1H, m), 7.19 (1H, s), 7.39 (1H, s) |

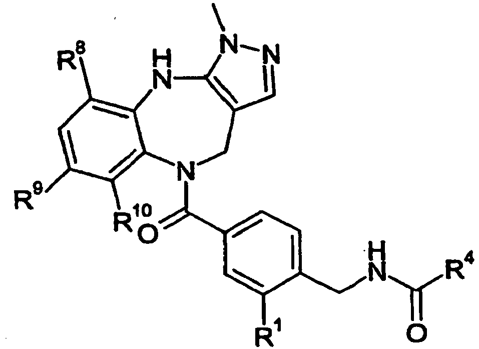

| **Compound number** | **R¹** | **R⁴** | **R⁸** | **R⁹** | **R¹⁰** | **MS** | **¹H NMR: δ (ppm)** |
|---|---|---|---|---|---|---|---|
| 1290 | Me | CH(CH₃)₂ | Me | H | H | (ESI)+ : [M+H] += 433.2 | 1.13 (6H, d, J=6.5Hz), 2.15 (3H, s), 2.26-2.38 (1H, m), 2.39 (3H, s), 3.82 (3H, s), 3.95 (1H, d, J=14.6Hz), 4.29 (2H, d, J=5.2Hz), 5.43 (1H, s), 5.77 (1 H, s), 5.93 (1H, d, J=14.6Hz), 6.62 (2H, s), 6.87 (2H, s), 6.96-7.06 (1H, m), 7.16 (1H, s), 7.25 (1H, s) |
| 1291 | Me | CH₂CH₂CH₃ | Me | H | H | (ESI)+ : [M+H] += 433.2 | 0.91 (3H, t, J=7.2Hz), 1.56-1.74 (2H, m), 2.09 (3H, s), 2.10-2.20 (2H, m), 2.39 (3H, s), 3.81 (3H, s), 3.94 (1H, d, J=14.6Hz), 4.25 (2H, d, J=4.7Hz), 5.65 (1 H, s), 5.85-6.05 (2H, m), 6.60-6.63 (2H, m), 6.82 (2H, s), 7.00 (1H, d, J=5.7Hz), 7.07 (1H, s), 7.20 (1H, s) |
| 1292 | Me | CH₂CH₃ | Me | H | H | (ESI)+ [M+H] += 418.2 | 1.34 (3H, t, J=7.4Hz), 2.12 (3H, s), 2.20 (2H, q, J=7.4Hz), 2.39 (3H, s), 3.82 (3H, s), 3.94 (1H, d, J=14.7Hz), 4.27 (2H, d, J=5.2Hz), 5.52 (1H, s), 5.85 (1H, s), 5.92 (1H, d, J=14.7Hz), 6.56-6.68 (2H, m), 6.86 (2H, s, 7.00 (1H, d, J=5.7Hz), 7.12 (1H, s), 7.25 (1H, s) |
| 1293 | Me | CH(CH₃)₂ | H | Me | H | (ESI)+ [M+H] += 433.2 | 1.08-1.24 (6H, m), 2.02 (3H, s), 2.13 (3H, s), 2.26-2.44 (1H, m), 3.74 (3H, s), 3.94 (1 H, d, J=14.4Hz), 4.28 (2H, d, J=5.2Hz), 5.62 (1H, s), 5.88 (1H, d, J=14.4Hz), 6.12 (1H, s), 6.52 (1H, s), 6.78-6.96 (4H, m), 7.14 (1H, s), 7.22 (1 H, s) |
| 1294 | Me | CH₂CH₂CH₃ | H | Me | H | (ESI)+ : [M+H] += 433.2 | 0.88-1.02 (3H, m), 2.02 (3H, s), 2.08-2.22 (7H, m), 3.72 (3H, s), 3.93 (1H, d, J=14.5Hz), 4.28 (2H, d, J=5.2Hz), 5.73 (1H, s), 5.86 (1 H, d, J=14.5Hz), 6.21 (1H, s), 6.51 (1H, s), 6.79-6.96 (4H, m), 7.12 (1H, s), 7.22 (1H, s) |
| 1295 | Me | CH₂CH₃ | H | Me | H | (ESI)+ [M+H] += 418.2 | 1.12 (3H, t, J=7.4Hz), 2.02 (3H, s), 2.12 (3H, s), 2.19 (2H, q, J=7.4Hz), 3.73 (3H, s), 3.94 (1H, d, J=14.4Hz), 4.28 (2H, d, J=5.0Hz), 5.68 (1H, s), 5.87 (1H, d, J=14.4Hz), 6.15 (1H, s), 6.51 (1H, s), 6.78-6.98 (4H, m), 7.13 (1H, s), 7.22 (1H, s) |
| 1296 | Me | 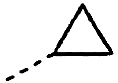 | H | H | Me | (ESI)+ : [M+H] += 430.1 | 0.69-0.79 (2H, m), 0.91-1.03 (2H, m), 1.32-1.41 (1H, m), 1.97 (3H, s), 2.07 (3H, s), 3.71 (3H, s), 3.86 (1H, d, J=14.6Hz), 4.26 (2H, d, J=5.5Hz), 5.89 (1H, d, J=14.6Hz), 5.99-6.08 (1H, m), 6.64 (1H, d, J=7.4Hz), 6.69-6.83 (2H, m), 6.90-7.03 (2H, m), 7.13 (1H, s), 7.23 (1H, s) |
| 1297 | Me | CH(CH₃)₂ | H | H | Me | (ESI)+ : [M+H] += 432.2 | 1.10-1.21 (6H, m), 1.99 (3H, s), 2.10 (3H, s), 2.33-2.42 (1H, m). 3.76 (3H, s), 3.86 (1H, d, J=14.6Hz), 4.27 (2H, d, J=5.5Hz), 5.61 (1H, s), 5.89 (1H, d, J=14.6Hz), 6.65 (1H, d, J=7.4Hz), 6.76-6.85 (2H, m), 6.94-7.04 (2H, m), 7.17 (1 H, s), 7.22 (1H, s) |
| 1298 | Me | CH₂CH₂CH₃ | H | H | Me | (ESI)+ [M+H] += 432.2 | 0.87-0.99 (3H, m), 1.56-1.72 (2H, m), 1.98 (3H, s), 2.10 (3H, s), 2.13-2.25 (2H, m), 3.76 (3H, s), 3.86 (1 H, d, J=14.6Hz), 4.27 (2H, d, J=5.7Hz), 5.66 (1H, s), 5.90 (1 H, d, J=14.6Hz), 6.65 (1H, d, J=7.4Hz), 6.76-6.85 (3H, m), 6.94-7.04 (3H, m), 7.16 (1H, s) |
| 1299 | Me | CH₂CH₃ | H | H | Me | (ESI)+ [M+H] += 418.2 | 1.09-1.22 (3H, m), 1.98 (3H, s), 2.08 (3H, s), 2.14-2.29 (2H, m), 3.74 (3H, s), 3.86 (1H, d, J=14.6Hz), 4.26 (2H, d, J=5.5Hz), 5.75 (1H, s), 5.89 (1H, d, J=14.6Hz), 6.61-6.66 (1H, m), 6.74-6.83 (2H, m), 6.94-7.04 (2H, m), 7.15 (1H, s), 7.22 (1H, s) |
| 1300 | Me | 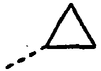 | H | Me | H | (ESI)+ : [M+H] += 430.2 | 0.68-0.79 (2H, m), 0.90-0.98 (2H, m), 1.30-1.40 (1H, m), 2.03 (3H, s), 2.12 (3H, s), 3.73 (3H, s), 3.94 (1H, d, J=14.6Hz), 4.27-4.33 (2H, m), 5.87 (1H, d, J=14.6Hz), 5.90-5.97 (1H, m), 6.24 (1H, s), 6.51 (1H, s), 6.79-6.94 (4H, m), 7.13 (1H, s) |
| 1301 | Me | 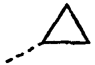 | Me | H | H | (ESI)+ : [M+H] += 430.3 | 0.68-0.80 (2H, m), 0.90-1.02 (2H, m), 1.28-1.40 (1H, m), 2.14 (3H, s), 2.39 (3H, s). 3.82 (3H, m), 3.95 (1H, d, J=14.6Hz), 4.30 (2H, d, J=5.4Hz), 5.69 (1H, brs), 5.82 (1H, s), 5.93 (1H, d, J=14.6Hz), 6.58-6.70 (2H, m), 6.85-6.96 (2H, m), 6.96-7.08 (1H, m), 7.14 (1H, s) |

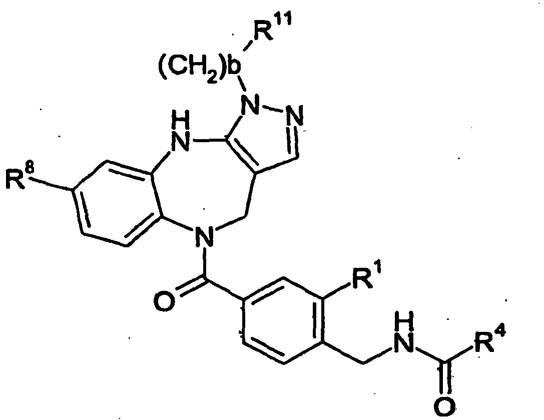

| **Compound number** | **R¹** | **R⁴** | R⁸ | **b** | **R¹¹** | **MS** |
|---|---|---|---|---|---|---|
| 1302 | F | CH2CH2CH3 | F | 0 | 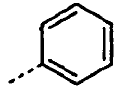 | (ESI)+: [M+H]+ = 502.4 |
| 1303 | F | CH(CH3)2 | F | 0 | 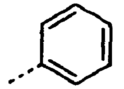 | (ESI)+: [M+H]+ = 502.2 |
| 1304 | F | 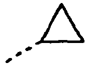 | F | 0 | 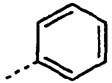 | (ESI)+: [M+H]+ = 500.2 |
| 1305 | F | CH2CH3 | F | 0 | 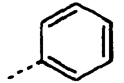 | (ESI)+: [M+H]+ = 488.2 |
| 1306 | F | CH2CH2CH3 | F | 1 | CH3 | (ESI)+: [M+H]+ = 454.3 |
| 1307 | F | CH(CH3)2 | F | 1 | CH3 | (ESI)+: [M+H]+ = 454.3 |
| 1308 | F | 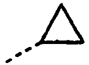 | F | 1 | CH3 | (ESI)+: [M+H]+ = 452.3 |
| 1309 | F | CH2CH3 | F | 1 | CH3 | (ESI)+: [M+H]+ = 440.3 |
| 1310 | Me | CH2CH2CH3 | F | 0 | C(CH3)3 | (ESI)+: [M+H]+ = 478.4 |
| 1311 | Me | CH2CH3 | F | 0 | C(CH3)3 | (ESI)+: [M+H]+ = 464.4 |
| 1312 | Me | CH(CH3)2 | F | 0 | C(CH3)3 | (ESI)+: [M+H]+ = 478.4 |
| 1313 | Me | 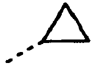 | F | 0 | C(CH3)3 | (ESI)+: [M+H]+ = 476.4 |
| 1314 | F | CH2CH2CH3 | F | 0 | 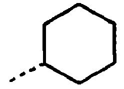 | (ESI)+: [M+H]+ = 508.3 |
| 1315 | F | CH2CH3 | F | 0 | 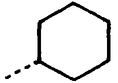 | (ESI)+: [M+H]+ = 494.2 |
| 1316 | F | CH(CH3)2 | F | 0 | 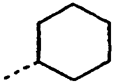 | (ESI)+: [M+H]+ = 508.3 |
| 1317 | F | 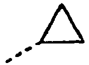 | F | 0 | 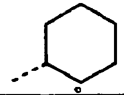 | (ESI)+: [M+H]+ = 506.2 |
| 1318 | F | CH2CH2CH3 | F | 0 | C(CH3)3 | (ESI)+: [M+H]+ = 482.2 |
| 1319 | F | CH2CH3 | F | 0 | C(CH3)3 | (ESI)+: [M+H]+ = 468.2 |
| 1320 | F | CH(CH3)2 | F | 0 | C(CH3)3 | (ESI)+: [M+H]+ = 482.2 |
| 1321 | F | 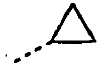 | F | 0 | C(CH3)3 | (ESI)+: [M+H]+ = 480.2 |
| 1322 | F | CH(CH₃)₂ | Cl | 1 | CH₃ | (ESI)+: [M+H]+ = 436.4 |
| 1323 | F | CH₂CH₂CH₃ | Cl | 1 | CH₃ | (ESI)+: [M+H] = 436.4 |
| 1324 | F | CH₂CH₃ | Cl | 1 | CH₃ | (ESI)+: [M+H]+ = 422.4 |
| 1325 | F | 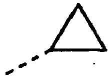 | Cl | 1 | CH₃ | (ESI)+: [M+H]+ = 434.4 |
| 1326 | M e | CH₂CH₃ | H | 2 | CH₂CH₃ | (ESI)+: [M+H]+= 446.3 |
| 1327 | M e | CH₂CH₂CH₃ | H | 2 | CH₂CH₃ | (ESI)+: [M+H]+= 460.3 |
| 1328 | M e | CH(CH₃)₂ | H | 2 | CH₂CH₃ | (ESI)+: [M+H]+= 460.2 |
| 1329 | M e | 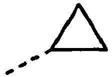 | H | 2 | CH₂CH₃ | (ESI)+: [M+H]+= 458.2 |
| 1330 | F | CH₂CH₃ | H | 2 | CH₂CH₃ | (ESI)+: [M+H]+= 450.2 |
| 1331 | F | CH₂CH₂CH₃ | H | 2 | CH₂CH₃ | (ESI)+: [M+H]+= 464.2 |
| 1332 | F | CH(CH₃)₂ | H | 2 | CH₂CH₃ | (ESI)+: [M+H]+= 464.2 |
| 1333 | F | 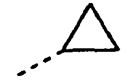 | H | 2 | CH₂CH₃ | (ESI)+: [M+H]+= 462.4 |
| 1334 | F | 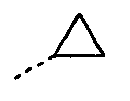 | Cl | 0 | 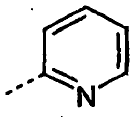 | (ESI)+: [M+H]+= 517.2, 519.2 |
| 1335 | F | CH(CH₃)₂ | Cl | 0 | 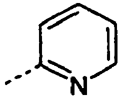 | (ESI)+: [M+H]+= 519.2, 521.2 |
| 1336 | F | CH₂CH₂CH₃ | Cl | 0 | 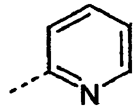 | (ESI)+: [M+H]+= 519.2, 521.2 |
| 1337 | F | CH₂CH₃ | Cl | 0 | 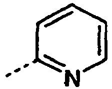 | (ESI)+: [M+H]+= 505.2, 507.2 |
| 1338 | M e | CH(CH₃)₂ | H | 1 | CH₃ | (ESI)+: [M+H]+= 433.2 |
| 1339 | M e | CH₂CH₂CH₃ | H | 1 | CH₃ | (ESI)+: [M+H]+= 433.2 |
| 1340 | M e | CH₂CH₃ | H | 1 | CH₃ | (ESI)+: [M+H]+= 418.2 |
| 1341 | M e | 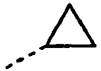 | H | 1 | CH₃ | (ESI)+: [M+H]+= 430.2 |
| 1342 | F | CH₂CH₂CH₃ | Cl | 0 | 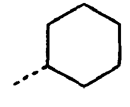 | (ESI)+: [M+H]+= 524.3, 526.3 |
| 1343 | F | CH(CH₃)₂ | Cl | 0 | 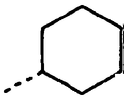 | (ESI)+: [M+H]+= 524.3, 526.3 |
| 1344 | F | 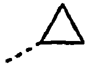 | Cl | 0 | 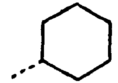 | (ESI)+: [M+H]+= 522.2, 524.2 |
| 1345 | F | CH₂CH₃ | Cl | 0 | 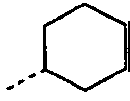 | (ESI)+: [M+H]+= 510.3, 512.3 |
| 1346 | F | CH₂CH₂CH₃ | Cl | 0 | 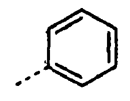 | (ESI)+: [M+H]+= 518.3, 520.3 |
| 1347 | F | 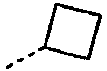 | Cl | 0 | 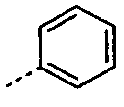 | (ESI)+: [M+H]+= 530.2, 532.2 |
| 1348 | F | CH₂CH₃ | Cl | 0 | 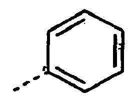 | (ESI)+: [M+H]+= 504.2, 506.2 |
| 1349 | F | CH(CH₃)₂ | Cl | 0 | 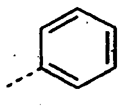 | (ESI)+: [M+H]+= 518.2, 520.2 |
| 1350 | F | CH₂CH₂CH₃ | Cl | 0 | C(CH₃)₃ | (ESI)+: [M+H]+= 498.2, 500.2 |
| 1351 | F | 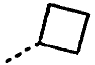 | Cl | 0 | C(CH₃)₃ | (ESI)+: [M+H]+= 510.4, 512.4 |
| 1352 | F | CH₂CH₃ | Cl | 0 | C(CH₃)₃ | (ESI)+: [M+H]+= 484.4, 486.4 |
| 1353 | F | CH(CH₃)₂ | Cl | 0 | C(CH₃)₃ | (ESI)+: [M+H]+= 498.4, 500.4 |
| 1354 | F | CH₂CH₂CH₃ | Cl | 1 | CH₃ | (ESI)+: [M+H]+= 470.3, 472.3 |
| 1355 | F | 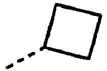 | Cl | 1 | CH₃ | (ESI)+: [M+H]+= 482.3, 484.3 |
| 1356 | F | CH₂CH₃ | Cl | 1 | CH₃ | (ESI)+: [M+H]+= 456.2, 458.2 |
| 1357 | F | CH(CH₃)₂ | Cl | 1 | CH₃ | (ESI)+: [M+H]+= 470.2, 472.2 |
| 1358 | F | CH₂CH₂CH₃ | Cl | 2 | CH₂CH₃ | (ESI)+: [M+H]+= 498.3, 500.3 |
| 1359 | F | 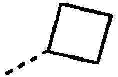 | Cl | 2 | CH₂CH₃ | (ESI)+: [M+H]+= 510.3, 512.3 |
| 1360 | F | CH₂CH₃ | Cl | 2 | CH₂CH₃ | (ESI)+: [M+H]+= 484.3, 486.3 |
| 1361 | F | CH(CH₃)₂ | Cl | 2 | CH₂CH₃ | (ESI)+: [M+H]+= 498.3, 500.3 |
| 1362 | F | 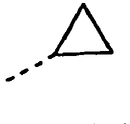 | Cl | 0 | 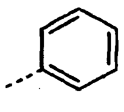 | (ESI)+: [M+H]+= 516.2, 518.2 |
| 1363 | F | 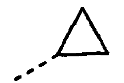 | Cl | 0 | C(CH₃)₃ | (ESI)+: [M+H]+= 496.2, 498.2 |
| 1364 | F | 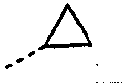 | Cl | 1 | CH₃ | (ESI)+: [M+H]+= 468.1, 470.2 |
| 1365 | F | 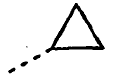 | Cl | 2 | CH₂CH₃ | (ESI)+: [M+H]+= 496.3, 498.3 |

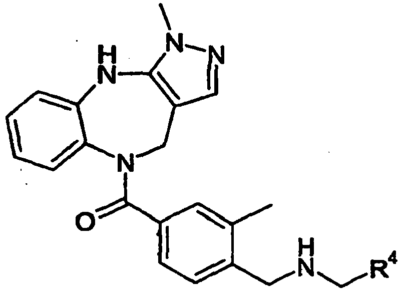

| **Compound number** | **R⁴** | **MS** |
|---|---|---|
| 1366 E109 | 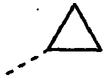 | (ESI)+:[M+H]+= 402.4 |
| 1367 | 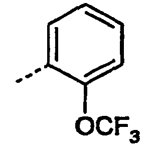 | (APCl)+: [M+H]+= 522.3 |

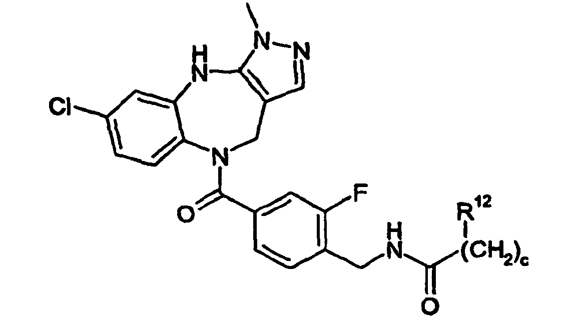

| **Compound number** | **c** | **R¹²** | **MS** |
|---|---|---|---|
| 1368 E113 | 1 | NHCO₂C(CH₃)₃ | (ESI)+:[M+H]+= 543.2, 545.2 |
| 1369 | 1 | N(CH₃)₂ | (ESI)+: [M+H]+= 471.2 |
| 1370 | 1 | N(CH₃)CO₂C(CH₃)₃ | (ESI)+: [M+H]+= 557.1, 559.1 |
| 1371 E114 | 1 | NH₂ | (ESI)+:[M+H]+= 443.0 |
| 1372 | 1 | NHCH₃ | (ESI)+: [M+H]+= 457.1 |

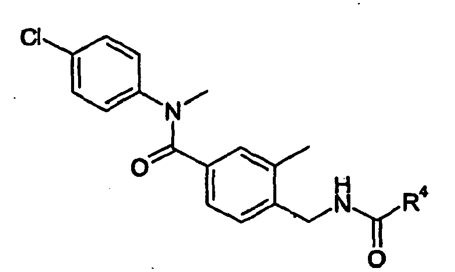

| **Compound number** | **R⁴** | **MS** |
|---|---|---|
| 1373 E115 | 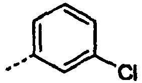 | (ESI)+:[M+H]+= 427.2 |
| 1374 | 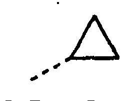 | (APCI)+: [M+H] = 357.3, 359.3 |
| 1375 | 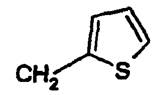 | (APCI)+: [M+H]+= 413.2, 415.2 |
| 1376 | 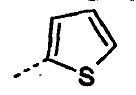 | (APCI)+: [M+N]+= 399.2, 401.2 |
| 1377 | CH₂CH₂CH₂CH₂CH₃ | (APCI)+: [M+H]+= 387.2,389.2 |
| 1378 | 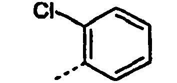 | (APCI)+: [M+H]+= 427.1, 29.1 |
| 1379 | 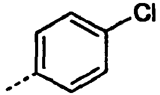 | (APCI)+: [M+H]+= 427.1, 429.1 |
| 1380 | 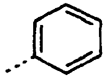 | (APCI)+: [IVI+H]+= 393.2. 395.2 |

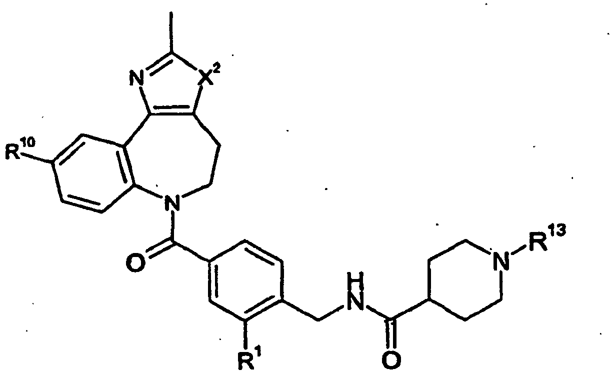

| **Compound number** | **R¹** | **R¹⁰** | **R¹³** | **X²** | **MS** |
|---|---|---|---|---|---|
| 1381 | Me | Me | 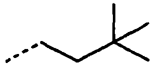 | S | (ESI)+: [M+H]+ = 573.3 |

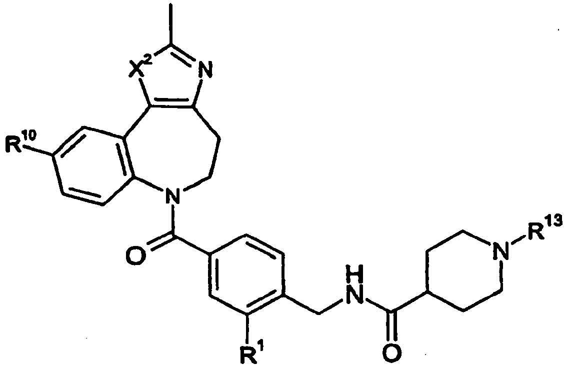

| **Compound number** | **R¹** | **R¹⁰** | **R¹³** | **X²** | **MS** |
|---|---|---|---|---|---|
| 1382 E122 | Me | H | 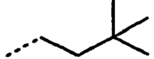 | NH | (APCI)+: [M+H]+ = 542.4 |
| 1383 | Me | Cl | 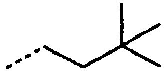 | NH | (ESI)+: [M+H]+ = 576.4 |
| 1384 | Me | Me | 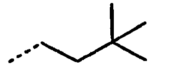 | NH | (ESI)+: [M+H]+ = 556.4 |

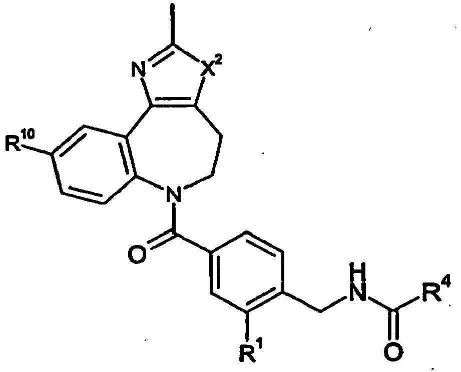

| **Compound number** | **R¹** | **R⁴** | **R¹⁰** | **X²** | **MS** |
|---|---|---|---|---|---|
| 1385 E124 | Me | 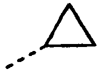 | H | S | (APCl)+: [M+H]+ = 432.3 |
| 1386 | Cl | 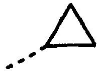 | Me | S | (ESI)+: [M+H]+ = 446.2 |

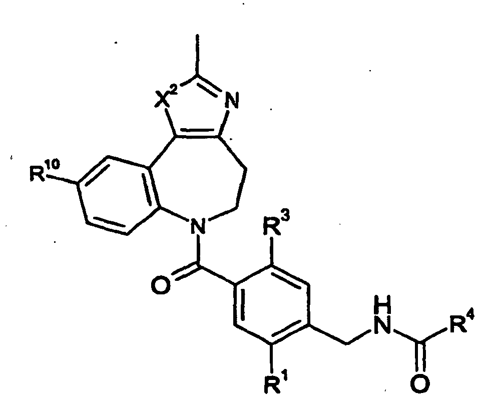

| **Compound number** | **R¹** | **R³** | **R⁴** | **R¹⁰** | **X²** | **MS** |
|---|---|---|---|---|---|---|
| 1387 | H | F | CH₂CH₂CH₃ | Me | NH | (ESI)+: [M+H]+ = 435.2 |

| **Compound number** | **¹H NMR: δ (ppm)** |
|---|---|
| 1314 | 0.89 (3H, t, J=7.4Hz), 1.20-1.53 (3H, m), 1.54-1.70 (2H, m), 1.71-1.82 (1H, m), 1.80-2.10 (6H, m), 2.14 (2H, t, J=7.4Hz), 2.77-3.93 (1H, m), 3.97 (1H, d, J=14.6Hz), 4.38 (2H, d, J=6.2Hz), 5.70-5.79 (1H, m), 5.86 (1H, d, J=94.6Hz), 6.10 (1H, s), 6.34-6.46 (1H, m), 6.62-6.75 (2H, m), 6.95 (2H, d, J=8.6Hz), 7.11 (1H, t, J=7.4Hz), 7.28 (1H, s) |
| 1315 | 1.13 (3H, t, J=7.4Hz), 1.30-1.54 (3H, m), 1.72-1.80 (1H, m), 1.89-2.11 (6H, m), 2.19 (2H, q, J=7.4Hz), 3.78-3.92 (1H, m), 3.97 (1H, d, J=14.6Hz), 4.38 (2H, d, J=5.7Hz), 5.69-5.80 (1H, m), 5.87 (1H, d, J=14.6Hz), 6.07 (1H, s), 6.38-6.48 (1H, m), 6.62-6.71 (2H, m), 6.94 (2H, d, J=8.6Hz), 7.11 (1H, t, J=7.2Hz), 7.28 (1H, s) |
| 1316 | 1.11 (6H, d, J=6.9Hz)_{.} 1.30-1.49 (3H, m), 1.74-1.80 (1H, m), 1.94-1.99 (6H, m). 2.31-2.36 (1H, m), 3.79-3.92 (1H, m), 3.97 (1H, d, J=14.8Hz), 4.37 (2H, d, J=5.9Hz), 5.70-5.81 (1H, m), 5.87 (1H, d, J=14.8Hz), 6.10 (1H, s), 6.36-6.43 (1H, m), 6.62-6.70 (2H, m), 6.95 (2H, d, J=8.9Hz), 7.10 (1H, t. J=7.4Hz), 7.28 (1H, s) |
| 1317 | 0.72-0.77 (2H, m), 0.92-1.00 (2H, m), 1.25-1.50 (4H, m), 1.74-1.80 (1H, m), 1.95-2.00 (6H, m), 3.78-3.90 (1H, m), 3.97 (1H, d, J=14.8Hz), 4.40 (2H, d, J=5.7Hz), 5.84-5.94 (2H, m), 6.04 (1H, s), 6.36-6.45 (1H, m), 6.64-6.70 (2H, m), 6.95 (2H, d, J=8.9Hz), 7.12 (1H, t, J=7.7Hz), 7.28 (1H, s) |
| 1318 | 0.89 (3H, t, J=7.4Hz), 1.59-1.64 (2H, m), 1.74 (9H, s), 2.14 (2H, t, J=7.7Hz), 3.97 (1H, d, J=14.6Hz), 4.38 (2H, d, J=5.7Hz), 5.62-5.77 (1H, m), 5.85 (1H, d, J=14.6Hz), 6.36-6.48 (2H, m), 6.58-6.70 6.92-7.00 7.13 (1H, t, J=7.7Hz), 7.21 (1H, s) |
| 1319 | 1.13 (3H, t, J=7.4Hz), 1.74 (9H, s), 2.18 (2H, q, J=7.4Hz), 3.97 (1H, d, J=14.6Hz), 4.39 (2H, d, J=5.9Hz), 5.63-5.75 (1H, m), 5.85 (1H, d, J=14.6Hz), 6.37-6.45 (2H, m), 6.60-6.70 (2H, m), 6.90-7.00 (2H, m), 7.12 (1H, t, J=7.4Hz), 7.22 (1H, s) |
| 1320 | 1.11 (6H, d, J=6.7Hz), 1.74 (9H, s), 2.22-2.40 (1H, m), 3.97 (1H, d, J=14.8Hz), 4.38 (2H, d, J=5.7Hz), 5.65-5.73 (1H, m), 5.85 (1H, d, J=14.8Hz), 6.34-6.48 (2H, m), 6.56-6.70 (2H, m), 6.90-7.00 12H, m), 7.12 (1H, t, J=7.7Hz), 7.22 (1H, s) |
| 1321 | 0.68-0.81 (2H, m), 0.92-1.01 (2H, m), 1.24-1.35 (1H, m), 1.74 (9H, s), 3.97 (1H, d, J=14.4Hz), 4.40 (2H, d, J=6.2Hz), 5.77-5.95 (2H, m), 6.36 (1H, s), 6.37-6.48 (1H, m), 6.58-6.70 (2H, m), 6.90-7.03 (2H, m), 7.13 (1H, t, J=7.2Hz), 6.22 (1H, s) |
| 1322 | 1.11 (6H, d, J=6.9Hz), 1.47 (3H, t, J=7.2Hz), 2.32 (1 H, septet, J=6.9Hz), 3.99 (1 H, d, J=14.6Hz), 4.09 (2H, q, J=7.2Hz), 4.34-(2H, d, J=5.9Hz) 5.79 (1H, s), 5.88 (1H, d, J=14.6Hz), 6.14 (1H, s), 6.69-6.71, (2H, m), 6.91-7.11 (5H, m), 7.25-7.26 (1H, m) |
| 1323 | 0.89 (3H, t, J=7.4Hz), 1.48 (3H, t, J=7.2.Hz), 1.57-1.60, (2H, m), 2.13 (2H, q, J=7.6Hz), 3.99 (1H, d, J=14.6Hz), 4.09 (2H, q, J=7.2Hz), 4.36 (2H, d, J=5.9Hz), 5.74 (1H, s), 5.87 (1H, d, J=14.6Hz), 6.05 (1H, s), 6.69-6.71 (2H, m), 6.90-6.97 13H, m), 7.03-7.12 (2H, m), 7.25-7.26 (1H, m) |
| 1324 | 1.11 (3H, t, J=7.7Hz), 1.46 (3H, t, J=7.2Hz), 2.18 (2H, q, J=7.7Hz), 3.99 (1 H, d, J=14.6Hz), 4.09 (2H, q, J=7.2Hz), 4.35 (2H, d, J=5.9Hz), 5.82 (1H, s), 5.88 (1H, d, J=14.6Hz), 6.19 (1H, s), 6.69-6.71 (2H, m), 6.91-7.13 (5H, m), 7.25-7.26 (1H, m) |
| 1325 | 0.70-0.74 (2H m), 0.91-0.95 (2H, m), 1.25-1.35 (1H, m), 1.48 (3H, t, J=7.2Hz), 3.98 (1H, d. J=14.6Hz), 4.09 (2H, q, J=7.2Hz), 4.37 (2H, d. J=5.9Hz), 5.89 (1H, d, J=14.6Hz), 5.96 (1H, s), 6.10 (1H, 6.70-6.72, 6.90-7.12 (5H, 7.25-7.26 |
| 1326 | 0.96 (3H, t, J=7.4Hz), 1.12 (3H, t, J=7.4Hz), 1.33-1.44 (2H, m), 1.79-1.88 (2H, m), 2.10-2.25 (5H, m), 3.95-4.04 (3H, m), 4.27 (2H, d, J=5.4Hz), 5.67 (1H, s), 5.90 (1H, d, J=14.6Hz), 6.21 (1H, s), 6.68-6.70 (2H, m), 6.83-7.12 (5H, m), 7.24 (1H, s) |
| 1327 | 0.87-0.99 (6H, m), 1.33-1.44 (2H, m), 1.58-1.67 (2H, m), 1.77-1.87 (2H, m), 2.10-2.16 (5H, m), 3.95-4.04 (3H, m), 4.27 (2H, d, J=5.7Hz), 5.65 (1H, s), 5.90 (1H, d, J=14.6Hz), 6.18 (1H, s), 6.68-6.70 (2H, m), 6.86-7.12 (5H, m), 7.24 (1H, s) |
| 1328 | 0.97 (3H, t, J=7.2Hz), 1.12 (6H, d, J=6.2Hz), 1.37-1.45 2H, m), 1.78-1.87 (2H, m), 2.13 (3H, s), 2.30-2.36 (1H, m), 3.95-4.06 (3H, m), 4.28 (2H, d, J=5.4Hz), 5.53 (1H, s), 5.91 (1H, d, J=14.6Hz), 6.05 1H, s), 6.68-6.70 (2H, m), 6.85-7.14 (5H. m), 7.24 (1H, s) |
| 1329 | 0.70-0.74 (2H, m), 0.94-1.00 (5H, m), 1.30-1.45 (3H, m), 1.81-1.87 (2H, m), 2.13 (3H, s), 3.95-4.05 (3H, m), 4.30 (2H, d, J=5.7Hz), 5.74 (1H, s), 5.91 (1H, d, J=14.6Hz), 6.06 (1H, s), 6.70-6.72 (2H, m), 6.89-6.93 (3H, m), 7.03-7.14 (2H, m), 7.24 (1H, s) |
| 1330 | 0.96 (3H, t, J=7.4Hz), 1.10 (3H, t, J=7.4Hz), 1.35-1.44 (2H, m), 1.77-1.85 (2H, m), 2.17 (2H, q, J=7.4Hz), 3.96-4.06 (3H, m), 4.33 (2H, d, J=5.4Hz), 5.87 (1H, d, J=14.6Hz), 5.90 (1H, s), 6.26 (1H, s), 6.68-6.70 (2H, m), 6.86-7.10 (5H, m), 7.24 (1H, s) |
| 1331 | 0.87 (3H, t, J=7.4Hz), 0.95 (3H, t, J=7.4Hz), 1.35-1.43 (2H, m), 1.56-1.64 (2H, m), 1.76-1.84 (2H, m), 2.09-2.15 (2H, m), 3.96-4.05 |
| | (3H, m), 4.33 (2H, d, J=5.7Hz), 5.87 (1H, d, J=14.6Hz), 5.93 (1H, s), 6.28 (1H, s), 6.68-6.70 (2H, m), 6.86-7.10 (5H, m), 7.24 (1H, s) |
| 1332 | 0.96 (3H, t, J=7.2Hz), 1.09 (6H, d, J=6.2Hz), 1.32-1.46 (2H, m), 1.77-1.88 (2H, m), 2.27-2.37 (1H, m), 3.96-4.06 (3H, m), 4.32 (2H, d, J=5.4Hz), 5.87 (1H, d, J=14.6Hz), 5.90 (1H, s), 6.27 (1H, s), 6.68-6.70 (2H, m), 6.85-7.10 (5H, m), 7.24 (1H, s) |
| 1333 | 0.70-0.74 (2H, m), 0.94-1.01 (5H, m), 1.29-1.45 (3H, m), 1.78-1.87 (2H, m), 3.96-4.05 (3H, m), 4.36 (2H, d, J=5.7Hz), 5.88 (1H, d, J=14.6Hz), 5.97 (1H, s), 6.06 (1H, s), 6.70-6.72 (2H, m), 6.89-6.97 (3H, m), 7.03-7.09 (2H, m), 7.24 (1H, s) |
| 1334 | 0.63-0.80 (2H, m), 0.80-1.08 (2H, m), 1.21-1.45 (1H, m), 4.00 (1H, d, J=15.1Hz), 4.40 (2H, d, J=5.7Hz), 5.80-5.94 (2H, m), 6.55-6.70 (2H, m), 6.89-7.21 (5H, m), 7.46 (1H, s), 7.80-7.90 (1H, m), 8.04 (1H, d, J=8.7Hz), 8.44 (1H, d, J=4.5Hz), 11.26 (1H, s) |
| 1335 | 1.11 (6H, d, J=6.7Hz), 2.32 (1H, sept, J=6.7Hz), 4.00 (1H, d, J=14.6Hz), 4.38 (2H, d, J=5.7Hz), 5.62-5.78 (1H, m), 5.92 (1H, d, J=14.6Hz), 6.50-6.70 (2H, m), 6.90-7.21 (5H, m), 7.46 (1H, s), 7.83-7.89 (1H, m), 8.04 (1H, d, J=8.4Hz), 8.44 (1H, d, J=4.2Hz), 11.26 (1H, s) |
| 1336 | 0.89 (3H, t, J=7.4Hz), 1.62 (2H, m), 2.14 (2H, t, J=7.7Hz), 4.00 (1H, d, J=14.6Hz), 4.39 (2H, d, J=5.7Hz), 5.50-5.65 (1H, m), 5.92 (1H, d, J=14.6Hz), 6.50-6.70 (2H, m), 6.91-7.21 (5H, m), 7.46 (1H, s), 7.82-7.91 (9H, m), 8.05 (1H, d, J=8.4Hz), 8.44 (1H, d, J=4.2Hz), 11.26 (1H, s) |
| 1337 | 1.12 (3H, t, J=7.5Hz), 2.18 (2H, q, J=7.5Hz), 4.00 (1H, d, J=14.8Hz), 4.39 (2H, d, J=5.7Hz), 5.60-5.85 (1H, m), 5.92 (1H, d, J=14.8Hz), 6.58-6.69 (2H, m), 6.98-7.20 (5H, m), 7.46 (1H, s), 7.83-7.90 (1H, m), 8.04 (1H, d, J=8.7Hz), 8.44 (1H, d, J=4.7Hz), 11.26 (1H, s) |
| 1338 | 1.12 (6H, d, J=6.7Hz), 1.47 (3H, t, J=7.0Hz), 2.13 (3H, s), 2.33 (1H, sept, J=6.7Hz), 3.98 (1H, d, J=14.9Hz), 4.08 (2H, q, J=7.0Hz), 4.28 (2H, d, J=5.2Hz), 5.50-5.60 (1H, m), 5.92 (1H, d, J=14.9Hz), 6.10 (1H, s), 6.62-6.76 (2H, m), 6.82-7.00 (3H, m), 7.02-7.10 (1H, m), 7.14 (1H, s) |
| 1339 | 0.90 (3H, t, J=7.3Hz), 1.46 (3H, t, J=7.2Hz), 1.54-1.71 (2H, m), 2.06-2.20 (5H, m), 3.97 (1H, d, J=14.6Hz), 4.07 (2H, q, J=7.2Hz), 4.28 (2H, d, J=4.9Hz), 5.56-5.66 (1H, m), 5.86 (1H, d, J=14.6Hz), 6.17 (1H, s), 6.62-6.74 (2H, m), 6.84-6.98 (3H, m), 7.02-7.12 (1H, m), 7.13.(1H, s) |
| 1340 | 1.13 (3H, t, J=7.7Hz),1:47 (3H, t, J=7.1Hz), 2.13 (3H, s), 2.19 (2H, q, J=7.7Hz), 3.97 (1H, d, J=14.9Hz), 4.08 (2H, q, J=7.1Hz), 4.28 (2H, d, J=5.4Hz), 5.54-5.64 (1H, m), 5.91 (1H, d, J=14.9Hz), 6.14 (1H, s), 6.66-6.76 (2H, m), 6.82-6.98 (3H, m), 7.02-7.12 (9H, m), 7.14 (1H, s) |
| 1341 | 0.68-0.78 (2H, m), 0.94 (2H, s), 1.26-1.38 (1H, m), 1.46 (3H, t, J=7.2Hz), 2.13 (3H, s), 3.97 (1H, d, J=14.6Hz), 4.07 (2H, q, J=7.2Hz), 4.29 (2H, d, J=5.2Hz), 5.76-5.86 (1H, m), 5.92 (1H, d, J=14.6Hz), 6.18 (1H, s), 6.64-6.74 (2H, m), 6.86-6.96 (3H, m), 7.02-7.12 (1H, m), 7.14 (1H, s) |
| 1342 | 0.90 (3H, t, J=7.4Hz), 1.20-1.71 (5H, m), 1.70 (1H, m), 1.81-2.10 (6H, m), 2.15 (2H, t, J=7.4Hz), 3.76-3.90 (1H, m), 3.95 (1H, d, J=14.4Hz), 4.39 (2H, d, J=5.9Hz), 5.65-5.79 (1H, m), 5.87 (1H, d, |
| | J=14.4Hz), 6.64 (2H, q, J=7.2Hz), 6.58-6.70 (3H, m). 7.12 (1H, t, J=7.7Hz), 7.27 (1H, s) |
| 1343 | 1.12 (6H, d, J=6.7Hz), 1.14-1.50 (3H, m), 1.77 (1H, m), 1.81-2.11 (6H, m), 2.28-2.40 (1H, m), 3.73-3.91 (1H, m), 3.95 (1H, d, J=14.6Hz), 4.38 (2H, d, J=6.2Hz), 5.64-5.76 (1H, m), 5.98 (1H, d, J=14.6Hz), 6.64 (2H, q, J=6.9Hz), 6.95-6.98 (3H, m), 7.12 (1H, t, J=7.9Hz), 7.28 (1H, s) |
| 1344 | 0.60-0.81 (2H, m), 0.90-1.00 (2H, m), 1.20-1.48 (4H, m), 1.76 (1H, m), 1.82-2.10 (6H, m), 3.73-3.90 (1H, m), 3.95 (1H, d, J=14.6Hz), 4.41 (2H, d, J=6.2Hz), 5.84-5.90 (2H, m), 6.60-6.72 (2H, m), 6.88-7.03 (3H, m), 7.12 (1H, t, J=7.2Hz), 7.28 (1H, s) |
| 1345 | 1.13 (3H, t, J=7.5Hz), 1.16-1.63 (3H, m), 1.76 (1H, m), 1.85-2.13 (6H, m), 2.22 (2H, q, J=7.5Hz), 3.72-3.90 (1H, m), 3.95 (1H, d, J=15.1 Hz), 4.39 (2H, d, J=5.9Hz), 5.66-5.80 (1H, m), 5.86 (1 H, d, J=15.1Hz), 6.60-6.87 (2H, m), 6.92-7.05 (3H, m), 7.12 (1H, t, J=7.2Hz), 7.27 (1H, s) |
| 1346 | 0.88-0.98 (3H, m), 1.60-1.71 (2H, m), 2.10-2.21 (2H, m), 4.08 (1 H, d, J=14.6Hz), 4.40 (2H, d, J=5.4Hz), 5.70 (1H, brs), 5.95 (1H, d, J=14.6Hz), 6.27 (1H, s), 6.62-6.70 (2H, m), 6.80 (1H, s), 6.85-7.08 (2H, m), 7.12-7.19 (1H, m), 7.45-7.65 (6H, m) |
| 1347 | 1.80-2.02 (2H, m), 2.08-2.30 (4H, m), 2.97 (1 H, t, J=9.2Hz), 4.01 (1H, d, J=14.9Hz), 4.39 (2H, d, J=6.2Hz), 5.60 (1H, brs), 5.96 (1H, d, J=14.9Hz), 6.27 (1H, s), 6.62-6.72 (2H, m), 6.80 (1H, s), 6.96-7.06 (2H, m), 7.10-7.20 (1H, m), 7.45-7.65 (6H, m) |
| 1348 | 1.13 (3H, t, J=7.5Hz), 2.19 (2H, q, J=7.5Hz), 4.04 (1H, d, J=14.9Hz), 4.39 (2H, d, J=5.9Hz), 5.69 (1H, brs), 5.96 (1H, d, J=14.9Hz), 6.27 (1H, s), 6.61-6.71 (2H, m), 6.80 (1H, s), 6.96-7.02 (2H, m), 7.10-7.18 (1H, m), 7.42-7.66 (6H, m) |
| 1349 | 1.20 (6H, d, J=6.7Hz), 2.30-2.40 (1H, m), 4.05 (1H, d, J=14.6Hz), 4.39 (1H, d, J=5.7Hz), 5.72 (1H, brs), 5.96 (1H, d, J=14.6Hz), 6.29 (1H, s), 6.61-6.72 (2H, m), 6.72 (1H, s), 6.95-7.15 (2H, m), 7.10-7.20 (1H, m), 7.45-7.70 (6H, m) |
| 1350 | 0.88-1.00 (3H, m), 1.59-1.71 (2H, m), 1.74 (9H, s), 2.12-2.20 (2H, m), 3.95 (1H, d, J=14.6Hz), 4.39 (2H, d, J=5.2Hz), 5.70 (1 H, brs), 5.84 (1H, d, J=14.6Hz), 6.36 (1H, s), 6.58-6.62 (2H, m), 6.88-7.02 (3H, m), 7.10-7.20 (1H, m), 7.20-7.22 (1H, s) |
| 1351 | 1.74 (9H, s), 1.82-2.03 (2H, m), 2.04-2.35 (4H, m), 2.90-3.05 (1H, m), 3.95 (1H, d, J=14.6Hz), 4.39 (2H, d, J=5.9Hz), 5.59 (1H, brs), 5.85 (1H, d, J=14.6Hz), 6.36 (1H, s), 6.60-6.72 (2H, m), 6.90-7.08 (3H, m), 7.10-7.20 (1H, m), 7.25 (1H, s) |
| 1352 | 1.13 (3H, t, J=7.4Hz), 1.74 (9H, s), 2.19 (2H, q, J=7.4Hz), 3.98 (1H, d, J=14.6Hz), 4.40 (2H, d, J=5.4Hz), 5.70 (1H, brs), 5.88 (1H, d, J=14.6Hz), 6.36 (1H, s), 6.58-6.70 (2H, m), 6.89-7.04 (3H, m), 7.10-7.20 (1H, m), 7.25 (1H, s) |
| 1353 | 1.12 (6H, d, J=6.7Hz), 1.74 (9H, s), 2.33 (1 H, q, J=6.9Hz), 3.95 (1H, d, J=14.9Hz), 4.39 (2H, d, J=5.9Hz), 5.70 (1H, brs), 5.85 (1H, d, J=14.9Hz), 6.36 (1H, s), 6.59-6.71 (2H, m), 6.90-7.05 (3H, m), 7.21 (1H, s), 7.25 (1H, s) |
| 1354 | 0.89 (3H, t, J=7.4Hz), 1.48 (3H, t, J=7.2Hz), 1.56-1.68 (2H, m), 2.15 (2H, t, J=7.4Hz), 3.95 (1H, d, J=14.8Hz), 4.06 (2H, q, J=7.2Hz), 4.39 (2H, d, J=5.9Hz), 5.72-5.80 (1H, m), 5.85 (1H, d, (1H, s), 6.65 (2H, dd, 6.89-6.98 |
| | (3H, m), 7.12 (1H, t, J=7.7Hz), 7.27 (1H, s) |
| 1355 | 1.45 (3H, t, J=7.2Hz), 1.75-2.03 (2H, m), 2.04-2.31 (4H, m), 2.88-3.07 (1H, m), 3.95 (1H, d, J=14.6Hz), 4.07 (2H, q, J=7.4Hz), 4.38 (2H, d, J=5.9Hz), 5.58-5.72 (1H, m), 5.85 (1H, d, J=14.6Hz), 6.17 (1H, s), 6.65 (2H, dd, J=8.4, 13.6Hz), 6.88-6.98 (3H, m), 7.10 (1H, t, J=8.2Hz), 7.26 s) |
| 1356 | 1.13 (3H, t, J=7.7Hz), 1.47 (3H, t, J=7.4Hz), 2.21 (2H, q, J=7.7Hz), 3.98 (1H, d, J=14.6Hz), 4.07 (2H, q, J=7.4Hz), 4.39 (2H, d, J=7.4Hz), 5.70-5.91 (2H, m), 6.17 (1H, s), 6.65 (2H, dd, J=8.4, 14.1 Hz), 6.88-7.00 (3H, m), 7.11 (1H, t, J=7.7Hz), 7.26 (1H, s) |
| 1357 | 1.12 (6H, d, J=6.9Hz), 1.47 (3H, t, J=7.2Hz), 2.25-2.43 (1H, m), 3.95 (1H, d, J=14.6Hz), 4.06 (2H, q, J=7.2Hz), 4.38 (2H, d, J=5.9Hz), 5.73-5.92 (2H, m), 6.22 (1H, s), 6.64 (2H, dd, J=8.1, 12.4Hz), 6.88-6.99 (3H, m), 7.09 (1H, t, J=7.7Hz), 7.26 (1H, s) |
| 1358 | 0.90 (3H, t, J=7.4Hz), 0.98 (3H, t, J=7.4Hz), 1.33-1.52 (1H, m), 1.56-1.70 (2H, m), 1.73-1.90 (2H, m), 2.15 (2H, t, J=7.7Hz), 3.88-4.04 (3H, m), 4.39 (2H, d, J=7.2Hz), 5.69-5.80 (1H, m), 5.85 (1H, d, J=14.6Hz), 6.06 (1H, s), 6.59-6.72 (2H, m), 6.89-7.01 (3H, m), 7.12 (1H, t, J=7.4Hz), 7.25 (1H, s) |
| 1359 | 0.98 (3H, t, J=7.2Hz), 1.33-1.48 (2H, m), 1.56 (2H, s), 1.78-2.00 (4H, m), 2.03-2.30 (3H, m), 2.90-3.06 (1H, m), 3.88-4.05 (3H, m), 4.38 (2H, d, J=5.7Hz), 5.58-5.68 (1H, m), 5.85 (1H, d, J=14.6Hz) 6.06 (1H, s), 6.57-6.70 (2H, m), 6.90-7.02 (3H, m), 7.11 (1H, t, J=7.7Hz) |
| 1360 | 0.97 (3H, t, J=7.4Hz), 1.13 (3H, t, J=7.4Hz), 1.31-1.49 (2H, m), 1.72-1.91 (2H, m), 2.13-2.28 (2H, m), 3.00-3.15 (1H, m), 3.90-4.10 (3H, m), 4.38 (2H, d, J=6.2Hz), 5.83-5.90 (2H, m), 6.20 (1H, s), 6.56-6.70 (2H, m), 6.90-7.03 (3H, m), 7.07-7.14 (1H, m) |
| 1361 | 0.98 (3H, t, J=7.4Hz), 1.12 (6H, d, J=6.9Hz), 1.33-1.48 (2H, m), 1.77-1.91 (2H, m), 2.28-2.41 (1H, m), 3.90-4.10 (3H, m), 4.38 (2H, d, J=5.9Hz), 5.70-5.90 (2H, m), 6.14 (1H, s), 6.64 (2H, dd, J=7.9, 12.1 Hz), 6.90-7.00 (3H, m), 7.10 (1H, t, J=7.2Hz), 7.25 (1H, s) |
| 1362 | 0.66-0.78 (2H, m), 0.90-0.99 (2H, m), 1.28-1.52 (2H, m), 4.03 (1H, d, J=14.6Hz), 4.40 (2H, d, J=5.9Hz), 5.90-6.00 (2H, m), 6.29 (1H, s), 6.60-6.74 (2H, m), 6.80 (1H, d, J=1.7Hz), 6.91-7.03 (2H, m), 7.13 (1H, t J=7.7Hz), 7.42-7.66 (5H, m) |
| 1363 | 0.66-0.78 (2H, m), 0.91-1.00 (2H, m), 1.21-1.46 (2H, m), 1.74 (9H, s), 3.95 (1H, d, J=14.8Hz), 4.40 (2H, d, J=5.7Hz), 5.85 (1H, d, J=14.8Hz), 5.92-6.00 (1H, m), 6.39 (1H, s), 6.59-6.69 (2H, m), 6.89-7.02 (2H, m), 7.13 (1H, t, J=7.4Hz), 7.25 (1H, s) |
| 1364 | 0.67-0.85 (2H, m), 0.94 (2H, s), 1.25-1.55 (5H, m), 3.95 (1H, d, J=14.6Hz), 4.07 (2H, q, J=7.2Hz), 4.39 (2H, d, J=5.7Hz), 5.85 (1H, d, J=14.6Hz), 6.11 (1H, s), 6.52 (1H, s), 6.65 (2H, dd, J=8.6, 13.3Hz), 6.80-7.00 (3H, m), 7.09 (1H, t, J=7.2Hz) |
| 1365 | 0.67-0.80 (2H, m), 0.86-1.06 (5H, m), 1.28-1.46 (4H, m), 1.75-1.90 (2H, m), 3.89-4.08 (3H, m), 4.39 (2H, d, J=7.2Hz), 5.85 (1H, d, J=14.6Hz), 5.95-6.07 (1H, m), 6.25 (1H, s), 6.64 (2H, dd, J=8.4, 13.1Hz), 6.85-7.00 (3H, m), 7.05-7.15 (1H, m) |
| 1366 | 0.37-0.11 (2H, m), 0.42-0.47 (2H, m), 0.87-1.10 (1H, m), 1.32-1.69 (1H, m), 2.16 (3H, s), 2.37-2.43 (2H, m), 3.64 (2H, s), 3.77 (3H, s), 3.99 (1H, d, J=14.6Hz), 5.90 (1H, d, J=14.6Hz), 6.20-6.34 (1H, m), 6.58-6.72 (2H, m), 6.80-7.09 (4H, m), 7.16 (1H, s), |
| | 7.24(1H, d, J=4.7Hz) |
| 1367 | 1.46-1.63 (2H, m), 2.15 (3H, s), 3.64 (2H, s), 3.79 (4H, s), 3.96 (1H, d, J=14.6Hz), 5.92 (1H, d, J=14.6Hz), 6.07 (1H, s), 6.61-6.73 (2H, m), 6.87-6.96 (2H, m), 6.99-7.10 (2H, m), 7.17 (1H, s), 7.20-7.30 (4H, m), 7.36-7.48 (1H, m) |
| 1368 | 1.38 (9H, s), 3.66 (3H, s), 3.70-3.80 (2H, m), 3.94 (1H, d, J=14.6Hz), 4.28-4.38 (2H, m), 5.52-5.61 (1H, m), 5.78 (1H, d, J=14.6Hz), 6.58 (2H, s), 6.82-7.20 (6H, m), 7.62 (1H, s) |
| 1369 | 2.23 (6H, s), 2.96 (2H, s), 3.78 (3H, s), 3.95 (1H, d, J=14.5Hz), 4.41 (2H, d, J=5.9Hz), 5.85 (1H, d, J=14.5Hz), 6.44 (1H, s), 6.58-6.70 (2H, m), 6.90-7.10 (3H, m), 7.11 (1H, t, J=7.0Hz), 7.51 (1H, s) |
| 1370 | 1.38 (9H, s), 2.85 (3H, s), 3.68 (3H, s), 3.84 (2H, s), 3.93 (1H, d, J=14.6Hz), 4.30-4.41 (2H, m), 5.78 (1H, d, J=14.6Hz), 6.57 (2H, s), 6.75-7.25 (6H, m), 7.70 (1H, s) |
| 1371 | 3.30 (1H, s), 3.68 (2H, s), 4.00 (3H, s), 4.00-4.10 (1H, m), 4.41 (2H, s), 5.84 (1H, d, J=15.1Hz), 6.82-6.92 (2H, m), 7.00 (1H, d, J=8.9Hz), 7.26 (1H, t, J=7.5Hz), 7.43 (1H, s), 7.95 (1H, s), 8.55-8.65 (1H, m) |
| 1372 | 2.70 (3H, s), 3.30 (1 H, s), 3.79 (2H, s), 4.01 (3H, s), 4.01-4.10 (1H, m), 4.41 (2H, s), 5.84 (1H, d, J=15.1Hz), 6.82-6.96 (2H, m), 6.98-7.06 (2H, m), 7.26 (1H, t, J=7.7Hz), 7.44 (1H, s), 7.98 (1H, s), 8.70-8.80 (1H, m) |
| 1373 | 2.21 (3H, s), 3.43 (3H, s), 4.51 (2H, d, J=5.7Hz), 6.40-6.52 (1H, m), 6.87-6.98 (4H, m), 7.17-7.21 (3H, m), 7.28-7.38 (1H, m), 7.42-7.48 (1H, m), 7.61-7.67 (1H, m), 7.76 (1H, s) |
| 1374 | 0.68-0.79 (2H, m), 0.92-1.00 (2H, m), 1.27-1.40 (1H, m), 2.21 (3H, s), 3.44 (3H, s), 4.36 (2H, d, J=5.7Hz), 5.61-5.68 (1H, m), 6.95-7.04 (4H, m), 7.18-7.25 (3H, m) |
| 1375 | 2.12 (3H, s), 3.42 (3H, s), 3.85 (2H, s), 4.31 (2H, d, J=5.7Hz), 5.72-5.77 (1H, m), 6.84-6.99 (6H, m), 7.12-7.25 (4H, m) |
| 1376 | 2.27 (3H, s), 3.44 (3H, s), 4.52 (2H, d, J=5.7Hz), 6.00-6.16 (1H, m), 6.92-6.99 (3H, m), 7.04-7.11 (2H, m), 7.21-7.30 (3H, m), 7.45-7.48 (2H, m) |
| 1377 | 0.85 (3H, t, J=7.2Hz), 1.20-1.37 (4H, m), 1.53-1.70 (2H, m), 2.12-2.29 (2H, m), 2.21 (3H, s), 3.43 (3H, s), 4.34 (2H, d, J=5.4Hz), 5.44-5.54 (1H, m), 6.91-6.99 (4H, m), 7.17-7.24 (3H, m) |
| 1378 | 2.28 (3H, s), 3.44 (3H, s), 4.57 (2H, d, J=5.4Hz), 6.19-6.30 (1H, m), 6.92-7.01 (2H, m), 7.09-7.11 (1H, m), 7.11-7.29 (4H, m), 7.31-7.42 (3H, m), 7.62-7.70 (1H, m) |
| 1379 | 2.21 (3H, s), 3.42 (3H, s), 4.50 (2H, d, J=5.4Hz), 6.41-6.51 (1H, m), 6.87-7.03 (4H, m), 7.13-7.22 (3H, m), 7.37 (2H, d, J=6.7Hz), 7.69 (2H, d, J=6.7Hz) |
| 1380 | 2.23 (3H, s), 3.43 (3H, s), 4.53 (2H, d, J=5.7Hz), 6.29-6.42 (1H, m), 6.89-7.07 (4H, m), 7.13-7.25 (3H, m), 7.37-7.50 (3H, m), 7.74-7.77 (2H, m) |
| 1381 | 0.87 (9H, s), 1.34-1.41 (2H, m), 1.68-2.10 (7H, m), 2.14 (3H, s), 2.27-2.32 (2H, m), 2.33 (3H, s), 2.74 (3H, s), 2.94-3.18 (4H, m), 3.48-3.55 (1H, m), 4.30 (2H, d, J=5.4Hz), 5.12-5.18 (1H, m), 5.51 (1H, s), 6.55 (1H, d, J=7.9Hz), 6.67 (1H, d, J=7.9Hz), 6.76-6.81 (2H, m), 7.16 (1H, s), 8.16 (1H, s) |
| 1382 | 0.86 (9H, s), 1.33-1.40 (2H, m), 1.66-1.92 (6H, m), 2.04-2.11 (1H, m), 2.08 (3H, s), 2.24-2.32 (2H, m), 2.45 (3H, s), 2.69-3.43 (5H, m), 4.25 (2H, d, J=4.4Hz), 4.98-5.06 (1H, brd), 5.76-5.84 (1H, brd), 6.62 (2H, t, J=6.4,13.6Hz), 6.76-6.85 (2H, m), 7.06 (1H, s), 7.07-7.24 (1H, m), 8.11-8.23 (1H, m), 10.04-10.19 (1H, m) |
| 1383 | (CD₃OD): 0.89-0.99 (10H, m), 1.48-1.54 (2H, m), 1.77-1.80 (1H, m), 1.80-1.91 (4H, m), 2.15 (3H, s), 2.30-2.43 (1H, m), 2.43 (3H, s), 2.44-2.50 (1H, m), 2.64-2.73 (1H, m), 2.91-3.10 (2H, m), 3.22-3.39 (4H, m), 4.26, (2H, s), 4.95-5.08 (1H, m), 6.63-6.75 (2H, m), 6.78-6.87 (2H, m), 6.90-7.00 (2H, m), 7.95 (1H, s) |
| 1384 | 0.75-0.98 (11H, m), 1.33-1.50 (2H, m), 1.51-1.67 (1H, m), 1.68-1.90, (4H, m), 2.09 (3H, s), 2.11-2.33 (6H, m), 2.36-2.58 (4H, m), 2.77-2.92 (1H, m), 2.93-3.16 (2H, m), 3.17-3.30 (1H, m), 4.14-4.30 (2H, m), 4.90-5.09 (1H, m), 6.36-6.53 (1H, m), 6.53-6.70 (1H, m), 6.70-6.78 (1H, m), 7.04 (1H, s), 7.20-7.30 (2H, m), 7.88(1Hs) |
| 1385 | 0.69-0.73 (2H, m), 0.81-0.97 (2H, m), 1.23-1.28 (1H, m), 2.14 (3H, s), 2.73 (3H, s), 3.09-3.19 (2H, m), 3.48-3.60 (1H, m), 4.30 (2H, d, J=5.4Hz), 5.13-5.23 (1H, m), 5.72 (1H, brs), 6.67 (2H, m), 6.86 (1H, d, J=7.9Hz), 6.95 (1H, m), 7.12-7.24 (2H, m), 8.37 (1H, dd, J=1.5, 8.1Hz) |
| 1386 | 0.68-0.75 (2H, m), 0.93-0.98 (2H, m), 1.25-1.42 (1H, m), 2.16 (3H, s), 2.33 (3H, s),2.74 (3H, s), 3.04-3.18 (2H, m), 3.48-3.57 (1H, m), 4.32 (2H, d, J=5.4Hz), 5.12-5.19 (1H, m), 5.64 (1H, s), 6.55 (1H, d, J=7.9Hz), 6.69 (1H, d, J=7.6Hz), 6.77 (1H, d, J=7.6Hz), 6.86 (1H, d, J=7.9Hz), 7.16 (1H, s), 8.16(1H, s) |
| 1387 | 0.90 (3H, t, J=7.4Hz), 1.56-1.70 (2H, m), 2.14 (2H, t, J=7.2Hz), 2.25 (3H, s), 2.46 (3H, s), 2.81-2.87 (1H, m), 2.98-3.10 (1H, m), 3.25-3.34 (1H, m), 4.28 (2H, d, J=5.9Hz), 4.93-5.00 (1H, m), 6.06 (1H, bs), 6.57-6.84 (6H. m), 7.82 (1H, bs) |

| **Compound number** | **¹H NMR: δ(ppm)** | **MS** |
|---|---|---|
| 1388 E48 | δ 0.32-0.47 (4H, m), 1.50-1.61 (1H, m), 1.83-2.00 (2H, m), 2.31-2.52 (4H, m), 2.52-2.73 (4H, m), 3.45 (2H, s), 3.76 (3H, s), 3.89-4.06 (3H, m), 5.87 (1H, d, J=14.3Hz), 6.37 (1H, s), 6.61-6.80 (3H, m), 6.86-7.00 (2H, m), 7.00-7.13 (2H, m), 7.20 (1H, s) ppm. | (APCI)+: [M+H]+= 505.4 |
| 1389 E55 | δ 1.10-1.35 (4H, m), 1.62-1.82 (4H, m), 2.11 (3H, s), 2.36-2.50 (2H, m), 2.70-2.90 (2H, m), 3.73 (3H, s), 3.80-4.00 (5H, m), 4.50-4.65 (2H, m), 5.87 (1H, d, J=14.6Hz), 6.60-7.25 (9H, m) ppm. | (ESI)⁺:[M+H ]⁺= 530.3 |
| 1390 E68 | δ 0.88 (9H, s), 1.22-1.46 (7H, m), 1.64-1.77 (4H, m), 1.93 (2H, t, J=10.9Hz), 2.02 (3H, s), 2.31-2.38 (2H, m), 2.95-2.99 (2H, m), 3.78 (3H, s), 3.78-3.92 (2H, m), 3.95 (1H, d, J=14.6Hz), 5.91 (1H, d, J=14.6Hz), | (APCI)+: [M+H]+= 544.6 |
| | 6.16 (1H, s), 6.43-6.49 (1H, m), 6.68-6.73 (2H, m), 6.92-7.11 (4H, m), 7.21 (1H, s) ppm. | |
| 1391 E69 | δ 0.86 (9H, s), 1.33-1.40 (2H, m), 1.84-1.94 (2H, m), 2.26-2.33 (2H, m), 2.42 (3H, s), 2.33-2.51 (10H, m), 2.85 (1H, d, J=15.6Hz), 2.91-3.05 (1H, m), 3.26-3.42 (1H, m), 3.91 (2H, t, J=6.2Hz), 5.06 (1H, dd, J=4.2, 12.6Hz), 6.56 (1H, t, J=8.2Hz), 6.62-6.73 (2H, m), 6.83-6.94 (2H, m), 7.18 (1H, t, J=7.4Hz), 8.08-8.22 (1H, brs), 10.25-10.55 (1H, brs) ppm. | (APCI)+: [M+H]+= 548.4 |
| 1392 E70 | δ 0.87 (9H, s), 1.37-1.45 (2H, m), 1.83-1.94 (2H, m), 2.00 (3H, s), 2.29-2.40 (2H, m), 2.43 (3H, s), 2.44-2.63 (10H, m), 2.83 (1H, d, J=15.3Hz), 2.91-3.08 (1H, m), 3.22-3.41 (1H, m), 3.83 (2H, t, J=5.9Hz), 5.09 (1H, dd, J=4.2, 12.9Hz), 6.35 (1H, d, J=8.4Hz), 6.63-6.69 (2H, m), 6.85 (1H, t, J=7.4Hz), 7.07 (1 H, s), 7.16 (1H, t, J=7.4Hz), 8.10 (1H, d, J=6.4Hz) ppm. | (APCI)+: [M+H]+= 544.5 |
| 1393 E71 | δ 0.89 (9H, s), 1.42-1.49 (2H, m), 1.88-2.02 (2H, m), 2.46-2.58 (4H, m), 2.58-2.78 (8H, m), 3.42 (3H, s), 4.01 (2H, t, J=6.2Hz), 6.72 (1H, t, J=8.4Hz), 6.93-6.99 (3H, m), 7.04 (1H, dd, J=2.0, 11.6Hz), 7.19-7.25 (2H, m) ppm. | (APCI)+: [M+H]+= 490.3/492.3 |
| 1394 E72 | δ 0.89 (9H, s), 1.42-1.53 (2H, m), 1.58-1.82 (4H, m), 2.40-2.58 (3H, m), 2.58-2.79 (4H, m), 3.46 (3H, s), 3.81 (3H, s), 3.88-3.97 (3H, m), 5.87 (1H, d, J=14.8Hz), 6.55 (1H, s), 6.60-6.69 (4H, m), 6.93 (1H, d, J=11.4Hz) 6.99-7.12 (4H, m), 7.21 (1H, s) ppm. | (APCI)+: [M+H]+= 563.4 |
| 1395 E73 | δ 0.88 (9H, s), 1.32-1.46 (2H, m), 1.47-1.83 (2H, m), 2.03 (3H, s), 2.30-2.40 (2H, m), 2.40-2.57 (2H, m), 2.57-2.69 (2H, m), 2.80 (2H, t, J=4.9Hz), 3.80 (3H, s), 3.95-4.08 (2H, m), 5.91-6.01 (2H, m), 6.50 (1H, d, J=7.7Hz), 6.63-7.78 (2H, m), 6.92 (1H, d, J=7.9Hz), 6.92-7.01 (1H, m), 7.06-7.18 (2H, m), 7.21-7.28 (3H, m) ppm. | (ESI)+: [M+H]+= 531.4 |
| 1396 E74 | δ 0.86 (9H, s), 1.33-1.39 (2H, m), 2.09 (3H, s), 2.24-2.37 (6H, m), 3.50-3.60 (4H, m), 3.75 (3H, s), 3.95 (1H, d, J=14.6Hz), 4.60 (2H, s), 5.91 (1H, d, J=14.6Hz), 6.10 (1H, s), 6.51 (1H, d, J=8.1Hz), 6.70 (2H, s), 6.88-6.99 (2H, m), 7.01-7.10 (1H, m), 7.20-7.24 (2H, m) ppm. | (ESI)+: [M+H]+= 545.4 |
| 1397 E75 | δ 0.88 (9H, s), 1.37 (2H, t, J=8.4Hz), 2.24-2.37 (6H, m), 3.50-3.60 (4H, m), 3.75 (3H, s), 3.95 (1H, d, J=14.6Hz), 4.60 (2H, s), 5.93 (1H, d, J=14.6Hz), 6.39 (1H, s), 6.61-6.73 (4H, m), 6.93 (1H, d, J=7.9Hz), 7.01-7.10 (1H, m), 7.20-7.24 (2H, m) ppm. | (ESI)+: [M+H]+= 531.5 |
| 1398 E76 | δ 0.87 (9H, s), 1.30-1.36 (2H, m), 1.86-1.91 (2H, m), 1.99 (3H, s), 2.22-2.28 (6H, m), 2.40-2.45 (2H, | (ESI)+: [M+H]+= |
| | m), 3.34-3.39 (4H, m), 3.77 (3H, s), 3.84-3.90 (3H, m), 5.68 (2H, d, J = 14.3 Hz), 6.63-6.69 (3H, m), 6.91 (1H, d, J = 8.4 Hz), 7.01 (1H, s), 7.13-7.16 (2H, m), 7.30 (1H, d, J = 8.2 Hz), 8.60 (1H, s) ppm. | 573.5 |
| 1399 E77 | δ 1.18-1.38 (2H, m), 1.48-1.76 (2H, m), 1.87 (2H, t, J=10.6Hz), 2.15 (3H, s), 2.42-2.48 (2H, m), 2.71-2.88 (4H, m), 2.91-3.01 (1H, m), 3.78 (3H, s), 3.86-3.90 (2H, m), 3.95 (1H, d, J=14.6Hz), 5.90 (1H, d, J=14.6Hz), 6.68 (2H, brs), 6.79-6.96 (3H, m), 7.02-7.10 (1H, m), 7.13-7.24 (2H, m) ppm. | (ESI)+: [M+H]+= 488.4 |
| 1400 E78 | δ 2.15 (3H, s), 3.72 (3H, s), 3.75 (3H, s), 3.96 (1H, d, J=14.6Hz), 4.50 (2H, s), 5.80 (1H, d, J=14.6Hz), 6.12 (1H, s), 6.45-6.51 (2H, m), 6.64 (1H, s), 6.74-6.92, (3H, m), 7.23(1H, s) | (APCI)+: [M+H]+= 441.2, 443.2 |
| 1401 E79 | 8 0.42-1.38 (3H, m), 2.16 (3H, s), 2.28-2.66 (4H, m), 2.71-3.01 (4H, m), 3.28-3.76 (4H, m), 3.78 (3H, s), 3.96 (1H, d, J=14.6Hz), 4.60-4.73 (1H, m), 5.89 (1H, d, J=14.6Hz), 6.15 (1H, d, J=9.1Hz), 6.71 (2H, s), 6.77-6.98 (3H, m), 7.01-7.21 (3H, m) ppm. | (ESI)+: [M+H)+= 488.5 |
| 1402 E80 | δ 0.88 (9H, s), 1.33-1.40 (2H, m), 2.17-2.58 (11H, m), 2.79-2.98 (2H, m), 3.18-3.41 (5H, m), 3.60 (2H, brs), 3.87 (3H, s), 3.88-4.01 (1H, m), 5.92-6.11 (1H, m), 6.78-7.42 (8H, m) ppm. | (ESI)+: [M+H]+= 557.5 |
| 1403 E82 | δ 0.89 (9H, s), 1.35-1.41 (2H, m), 1.64 (2H, brs), 1.74-1.84 (2H, m), 2.14 (3H, s), 2.23 (2H, t, J=7.4Hz), 2.28-2.38 (6H, m), 2.54 (2H, t, J=7.4Hz), 3.34 (2H, t, J=4.9Hz), 3.59 (2H, t, J=4.9Hz), 6.58-6.78 (4H, m), 6.81-6.92 (1H, m), 7.04-7.09 (3H, m) ppm. | (ESI)+: [M+H]+= 506.5 |
| 1404 E83 | δ 0.87 (9H, s), 1.30-1.70 (4H, m), 2.09 (3H, s), 2.20-2.50 (10H, m), 3.41 (2H, brs), 3.58 (4H, brs), 3.74 (3H, s), 3.88-3.98 (1H, m), 5.85-5.95 (1H, m), 6.60-7.22 (9H, m) ppm. | (ESI)+: [M+H]+= 571.5 |
| 1405 E84 | δ 1.08-1.47 (2H, m), 1.61-1.86 (4H, m), 2.15 (3H, s), 2.35-2.48 (2H, m), 2.78-2.84 (2H, m), 2.94-3.11 (2H, m), 3.44-3.62 (1H, m), 3.77 (3H, s), 3.72-3.88 (1H, m), 3.95 (1H, d, J=14.6Hz), 4.01-4.12 (1H, m), 5.89 (1H, d, J=14.6Hz), 6.34 (1H, d, J=4.7Hz), 6.66-6.71 (2H, m), 6.76-6.94 (3H, m), 7.01-7.18 (2H, m) ppm. | (ESI)+: [M+H]+= 474.5 |
| 1406 E85 | δ 1.36-1.55 (6H, m), 1.71-1.80 (2H, m), 1.82-1.96 (1H, m), 2.02-2.10 (4H, m), 2.68 (3H, s), 2.97-3.10 (2H, m), 3.40-3.55 (4H, m), 3.63 (3H, s), 3.81 (1H, d, J=14.6Hz), 5.83 (1H, d, J=14.6Hz), 6.42-6.56 (1H, m), 6.61-6.89 (2H, m), 6.95-7.05 (1H, m), 7.17-7.25 (3H, m), 7.33 (1H, s) ppm. | (ESI)+: [M+H]+= 513.8 |
| 1407 E86 | δ 0.20-0.27 (2H, m), 0.31-0.42 (2H, m), 0.83-1.10 (2H, m), 1.44-1.83 (4H, m), 2.02-2.10 (1H, m), 2.13 | (ESI)+: [M+H]+= |
| | (3H, s), 2.29-2.58 (6H, m), 2.71-2.89 (2H, m), 3.55-3.74 (2H, m), 3.75 (3H, s), 3.95 (1H, d, J=14.6Hz), 4.50-4.60 (1H, m), 5.90 (1H, d, J=14.6Hz), 6.48-6.75 (3H, m), 6.79-7.22 (5H, m) ppm. | 527.7 |
| 1408 E87 | δ 0.55-0.64 (2H, m), 0.71-0.81 (2H, m), 1.11-1.24 (2H, m), 1.53-1.62 (2H, m), 1.70-1.91 (1H, m), 2.15 (3H, s), 2.62-2.84 (4H, m), 3.12-3.25 (4H, m), 3.86 (3H, s), 3.90-4.15 (5H, m), 5.67 (1H, d, J=14.6Hz), 6.61-6.75 (2H, m), 6.76-6.94 (2H, m), 7.07-7.17 (2H, m), 7.35-7.43 (1H, m), 7.47 (1H, s) ppm. | (ESI)+: [M+H]+= 527.6 |
| 1409 E110 | δ 1.91-1.99 (1H, m), 3.32 (2H, s), 3.52-3.85 (8H, m), 3.93 (1H, d, J=14.6Hz), 5.81 (1H, d, J=14.6Hz), 6.59 (2H, s), 6.80-7.10 (4H, m), 7.10-7.38, (2H, m) ppm | (ESI)⁺:[M+H ]⁺= 530.2, 532.2 |
| 1410 E111 | δ 3.47 (4H, s), 3.67 (6H, s), 3.79 (3H, s), 3.87 (2H, s), 3.95 (1H, d, J=14.6Hz), 5.85 (1H, d, J=14.6Hz), 6.14 (1H, s), 6.60-6.70 (2H, m), 6.90-7.02 (3H, m), 7.20-7.38 (2H, m) ppm | (ESI)⁺: [M+H]⁺= 458.1 |
| 1411 E112 | δ 1.28 (1 H, s), 2.71 (2H, t, J=5.3Hz), 3.64 (2H, t, J=5.3Hz), 3.81 (3H, s), 3.95 (2H, s), 4.00 (1H, d, J=14.7Hz), 5.74 (1H, d, J=14.7Hz), 6.66 (1 H, d, J=8.3Hz), 6.78 (1H, d, J=8.3Hz), 7.02-7.10 (2H, m), 7.22-7.38(3H, m) ppm | (ESI)⁺:[M+H ]⁺= 430.1 |
| 1412 E116 | (270MHz, d4-MeOH): δ 0.37-0.48 (2H, m), 0.70-0.81 (2H, m), 1.02-1.18 (1H, m), 1.86-2.11 (4H, m), 2.22 (3H, s), 2.49-2.64 (1H, m), 3.01 (2H, d, J=7.4 Hz), 3.17-3.30 (2H, m), 3.42 (3H, s), 3.49-3.76 (2H, m), 4.30 (2H, s), 7.06-7.17 (5H, m), 7.23-7.27 (2H, m)ppm. | (APCI)⁺:[M+ H]⁺= 454.3/456.3 |
| 1413 E117 | δ 0.91 (9H, s), 1.48-1.58 (2H, m), 1.60-1.79 (4H, m), 2.16 (3H, s), 2.22-2.35 (1H, m), 2.51-2.65 (2H, m), 2.71-2.82 (2H, m), 3.11-3.27 (2H, m), 3.43 (3H, s), 4.25 (2H, d, J=5.5Hz), 6.52-6.63 (1H, m), 6.95-7.09 (5H, m), 7.20 (2H, d, J=8.6Hz) ppm. | (ESI)+: [M+H]+= 484.4/486.4 |
| 1414 E118 | δ 0.87 (9H, s), 1.34-1.41 (2H, m), 1.62-1.96 (6H, m), 2.04-2.19 (1H, m), 2.17 (3H, s), 2.26-2.33 (2H, m), 2.94-2.99 (2H, m), 3.48 (3H, s), 4.34 (2H, d, J=5.4Hz), 6.95 (2H, d, J=1.0Hz), 7.14 (2H, d, J=8.2Hz), 7.19 (1H, s), 7.49 (2H, d, J=8.4Hz) ppm. | (APCI)⁺:[M+ H]⁺= 518.4 |
| 1415 E119 | δ 0.64-0.73 (2H, m), 0.90-1.00 (2H, m), 1.25-1.40 (1H, m), 2.12 (3H, s), 2.55 (3H, s), 2.80-2.90 (1H, m), 2.90-2.94 (1 H, m), 3.45 (1H, s), 4.30 (2H, d, J=5.4Hz), 5.09-5.22 (1H, m), 5.77-5.88 (1H, m), 6.60-6.73 (2H, m), 6.86-6.93 (2H, m), 7.01 (1H, s), 7.14-7.24 (1H, m), 7.76 (1H, dd, J=1.2, | 7.9Hz) ppm. (ESI)⁺:[M+H ]⁺= 416.3 |
| 1416 E120 | δ 0.87 (9H, s), 1.36-1.45 (2H, m), 1.73-1.82 (2H, m), 1.90-2.07 (2H, m), 2.11 (3H, s), 2.36-2.42 (2H, m), 2.55 (3H, s), 2.80-2.90 (1H, m), 2.90-2.98 (1H, m), 3.10-3.16 (2H, m), 4.28 (2H, d, J=5.2Hz), 5.11-5.19 (1H, m), 5.72-5.82 (1 H, m), 6.49 (2H, dd, J=1.5, 6.7Hz), 6.61-6.72 (2H, m), 6.80-6.97 (2H, m), 7.00 (1H, s), 7.13-7.25 (1H, m), 7.77 (1H, dd, J=1.5, 7.9Hz), 8.15 (2H, dd, J=1.7, 5.2Hz) ppm. | (APCI)⁺:[M+ H]⁺= 543.4 |
| 1417 E121 | δ 0.88 (9H, s), 1.37-1.53 (5H, m), 1.63-1.70 (2H, m), 2.11 (3H, s), 2.45 (3H, s), 2.63-3.46 (8H, m), 2.87-2.94 (2H, m), 4.23 (2H, brd), 5.08 (2H, d, J=7.9Hz), 4.96-5.11 (1H, m), 6.60 (2H, t, J=7.9, 15.1Hz), 6.81-6.86 (2H, m), 6.95 (2H, d, J=7.9Hz), 7.04 (1H, s), 7.12-7.33 (4H, m), 8.22 (1H, dd, J=1.2, 7.9Hz) ppm. | (APCI)⁺:[M+ H]⁺= 632.4 |
| 1418 E123 | δ 0.64-0.71 (2H, m), 0.89-0.95 (2H, m), 1.33-1.39 (1H, m), 2.10 (3H, s), 2.43 (3H, s), 2.74-2.81 (1H, m), 2.92-3.04 (1H, m), 3.07-3.26 (1H, m), 4.18-4.34 (2H, m), 4.97-5.05 (1H, m), 6.60 (2H, d, J=7.9Hz), 6.79-6.84 (2H, m), 7.05 (1H, s), 7.11 (1H, t, J=7.4, 14.8Hz), 7.96-8.21 (1H, brs), 10.10-10.60 (1H, brs) ppm. | (APCI)⁺:[M+ H]⁺= 415.3 |
| 1419 E125 | δ 1.13 (6H, d, J=6.9Hz), 2.15 (3H, s), 2.30-2.40 (1H, m), 2.48 (3H, s), 2.86-3.03 (2H, m), 3.20-3.38 (1H, m), 4.20-4.36 (2H, m), 5.04-5.11 (1H, m), 5.63 (1H, s), 6.53 (1H, d, J=8.2Hz), 6.62 (1H, d, J=7.9Hz), 6.70-6.90 (2H, m), 7.10 (1H, s), 8.21 (1H, s), 9.39 (1H, s) ppm. | (ESI)+: [M+H]+ = 451.2, 453.2 |

## Claims

1. A compound according to general formula 1a, or a compound which is a tautomer or a pharmaceutically acceptable salt thereof, 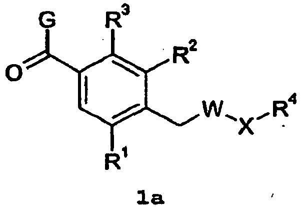 wherein G is a group selected among general formula 2a, 3a, 4a, 5a, and 6a, 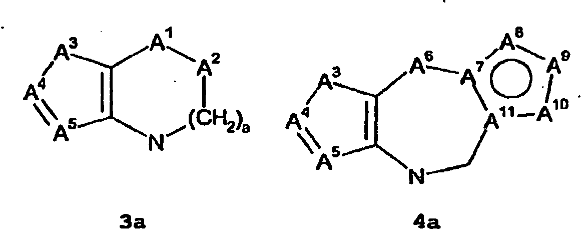 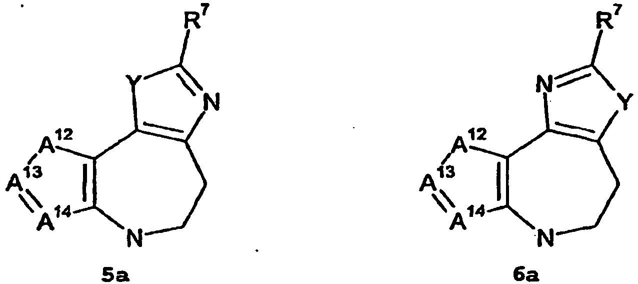 wherein:
A¹ is selected among CH₂, CH(OH), NH, N-alkyl; O and S;
A² is selected among CH₂, CH(OH), C(=O) and NH;
A³ and A¹² are independently selected among S, NH, N-alkyl, -C(R⁸)=CH-, -C(R⁸)=N-, -N=C(R⁸)- and -CH=C(R⁸)-;
A⁴ and A¹³ are independently selected among C(R⁹) and N;
A⁵ and A¹⁴ are independently selected among C(R¹⁰) and N;
A⁶ is selected among CH₂, NH, N-alkyl and O;
A⁷ and A¹¹ are independently selected among C and N;
A⁸ and A⁹ are independently selected among CH, N, NH, N(CH₂)_{b}R¹¹ and S;
A¹⁰ is selected among -CH=CH-, CH, N, NH, N-(CH₂)_{b}-R¹¹ and S;
wherein the ring constituted by A⁷, A⁸, A⁹, A¹⁰ and A¹¹ is aromatic;
R¹, R² and R³ are independently selected among H, alkyl, O-alkyl, NO₂, F, Cl and Br;
R⁴ is selected among H, alkyl, aryl, heteroaryl, -(CH₂)_{c}-R¹² and 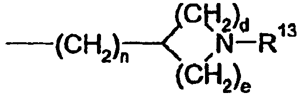
R⁷ is selected among H, alkyl, aryl, heteroaryl and -(CH₂)_{g}-R¹⁴;
R⁸, R⁹ and R¹⁰ are independently selected among H, alkyl, alkoxy, F, Cl, Br, CN, NH₂, NO₂, NH(alkyl), N(alkyl)₂; with the provisos that when G is 3a, and
R⁴ is H, alkyl, aryl, heteroaryl or -(CH₂)_{c}-R¹², then the ring containing A³, A⁴ and A⁵ is substituted in at least one position by alkyl, alkoxy, F, Cl, Br, CN, NH₂, NO₂, NH(alkyl) or N(alkyl)₂; and
when G is 4a, R⁴ is H, alkyl, aryl, heteroaryl or -(CH₂)_{c}-R¹², and A⁸ is NH, NCH₃ or S, then the ring containing A³, A⁴ and A⁵ is substituted in at least one position by alkyl, alkoxy, F, Cl, Br, CN, NH₂, NO₂, NH(alkyl) or N(alkyl)₂;
R¹¹ and R¹² are independently selected among H, alkyl, aryl, heteroaryl, F, OH, O-alkyl, S-alkyl, O-acyl, NH₂, NH-alkyl, N(alkyl)₂, NH-acyl, N(alkyl)-acyl, CO₂H, CO₂-alkyl, CONH₂, CONH-alkyl, CON(alkyl)₂, CN and CF₃;
R¹³ is selected among H, alkyl, aryl, heteroaryl, -(CH₂)ₙ-R¹⁵ and Z-R¹⁶;
R¹⁴ and R¹⁵ are independently selected among H, alkyl, alkenyl, aryl, heteroaryl, F, OH, O-alkyl, S-alkyl, O-acyl, NH₂, NH-alkyl, N(alkyl)₂, NH-acyl, N(alkyl)-acyl, CO₂H, CO₂-alkyl, CO-alkyl, CO-aryl, CONH₂, CONH-alkyl, CON(alkyl)₂, alkenyl-CO₂-alkyl, alkenyl-aryl, CN and CF₃;
R¹⁶ is selected among H, alkyl, alkenyl, aryl, heteroaryl, O-aryl, -(CH₂)₁-R¹⁷, cyclopropyl-aryl and O-(CH₂)₁-R¹⁷;
R¹⁷ is selected among H, alkyl, aryl, heteroaryl, F, OH, O-alkyl, S-alkyl, O-acyl, NH₂, NH-alkyl, N(alkyl)₂, NH-acyl, N(alkyl)-acyl, CO₂H, CO₂-alkyl, CONH₂, CONH-alkyl, CON (alkyl)₂, CN and CF₃;
W is selected among O and NH;
X is selected among (CH₂)ₘ, C(=O) and S(=O)ⱼ Y is selected among O, S, NH and N-alkyl;
Z is selected among -C(=O), -C(=O)-O and -S(=O)ₖ;
wherein alkyl is selected from saturated hydrocarbon residues including linear groups up to 10 atoms (C₁-C₁₀), branched groups of between 3 and 10 atoms (C₃-C₁₀), cyclic groups of between 3 and 8 (C₃-C₈) atoms and combinations of linear, branched and cyclic groups.
a is selected among 1 and 2;
b and c are independently selected among 0, 1, 2 and 3;
d and e are independently selected among 1 and 2;
g, h and i are independently selected among 1, 2 and 3;
j and k are independently selected among 1 and 2; and
m and n are independently selected among 0, 1 and 2;
wherein at least one of R¹, R² and R³ is other than hydrogen and wherein the compound according to general formula 1a is not (4-Aminomethyl-3-methylphenyl)-(5*H*,11*H*-benzo[*e*]pyrrolo[1,2-*a*][1,4]diazepin-10-yl)methanone

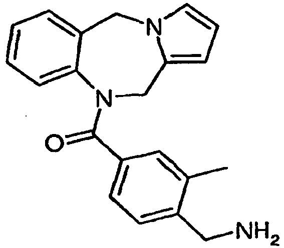

2. The compound according to claim 1, wherein G is selected among:

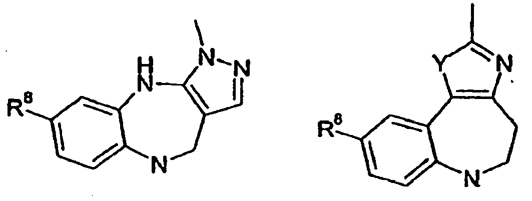

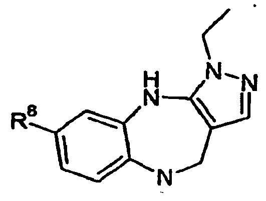

3. The compound according to claim 1, wherein G is selected among:

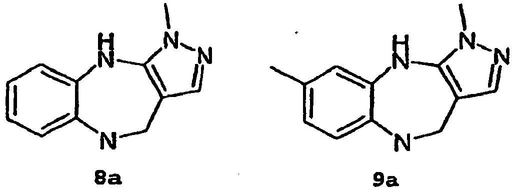

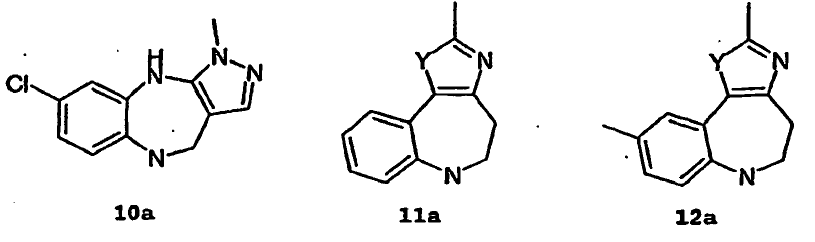

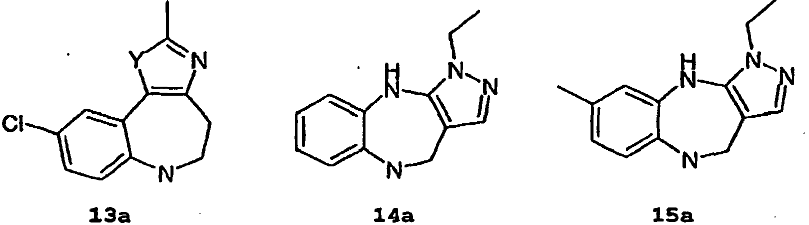

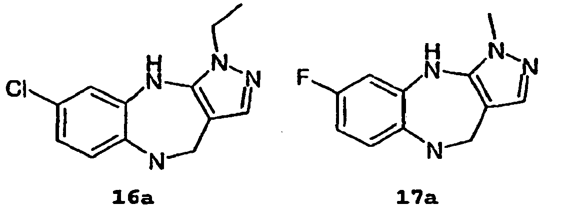

4. The compound according to claim 1, wherein one of R¹, R² and R³ is selected among methyl, chlorine and fluorine, and the others are hydrogen.

5. The compound according to claim 1, wherein W is NH.

6. The compound according to claim 1, wherein R⁴ is alkyl.

7. The compound according to claim 1, wherein d is 2 and e is 2.

8. The compound according to claim 1, wherein R¹³ is alkyl.

9. The compound according to claim 1, wherein n is 0.

10. The compound according to claim 1, wherein R² is H, R⁴ is a piperidine where d is 2 and e is 2, W is NH, X is C(=O) and n is 0, as shown in formula 18a: 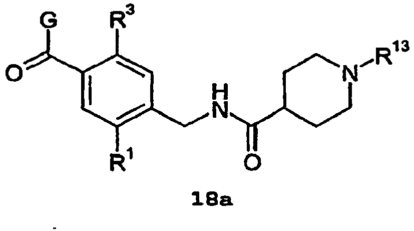 wherein R¹ is selected among methyl, chlorine and fluorine, and R³ is H; or R¹ is H, and R³ is selected among methyl, chlorine and fluorine, and R¹³ is alkyl.

11. The compound according to claim 1, wherein X is C(=O), n is 0 and R⁴ is a piperidine where d is 2 and e is 2, as shown in formula 19a: 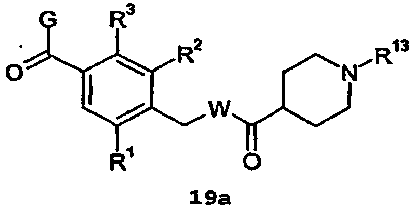 wherein G is selected among general formulae 8a to 17a, and R¹³ is alkyl.

12. The compound according to claim 1, wherein R² is H and W is NH as shown in formula 20a: 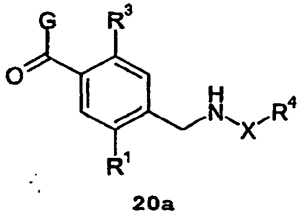 wherein either R¹ is selected among methyl, chlorine and fluorine, and R³ is H; or R¹ is H, and R³ is selected among methyl, chlorine and fluorine; and G is selected from general formulae 8a to 17a.

13. The compound according to claim 1, wherein R² is H, W is NH and X is C(=O) as shown in formula 21a: 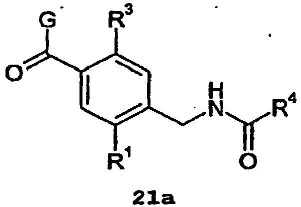 wherein R¹ is selected among methyl, chlorine and fluorine, and R³ is H; or R¹ is H, and R³ is selected among methyl, chlorine and fluorine; and R⁴ is alkyl.

14. The compound according to claim 1, wherein X is C(=O) as shown in formula 22a: 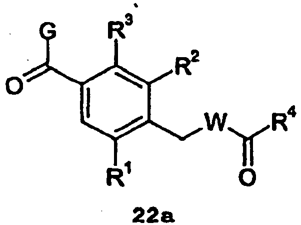 wherein G is selected among general formulae 8a to 17a, and R⁴ is alkyl.

15. The compound according to claim 1, wherein R² and R³ are both H, W is NH and X is C(=O) as shown in formula 23a: 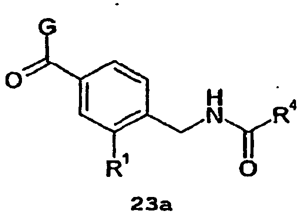 wherein G is selected among general formulae 9a, 10a, 15a, 16a and 17a, R¹ is selected among methyl, chlorine and fluorine, and R⁴ is alkyl.

16. The compound according to claim 1, wherein R² and R³ are both H, R⁴ is a piperidine where d is 2 and e is 2, W is NH, X is C(=O) and n is 0 as shown in formula 24a: 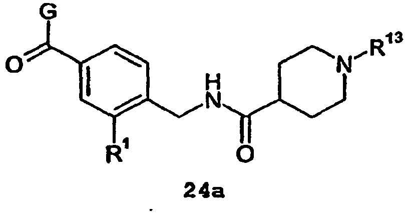 wherein G is selected among general formulae 9a, 10a, 15a, 16a and 17a, R¹ is selected among methyl, chlorine and fluorine, and R¹³ is alkyl.

17. The compound according to claim 1, selected among the group consisting of:
1-(3,3-Dimethyl-butyl)-piperidine-4-carboxylic acid 2-chloro-4-(3,6-dimethyl-4,10-dihydro-3H-2,3,4,9-tetraaza-benzo[f]azulene-9-carbonyl)-benzylamide;
1-(3,3-Dimethyl-butyl)-piperidine-4-carboxylic acid 2-chloro-4-(3-methyl-4,10-dihydro-3H-2,3,4,9-tetraaza-benzo[f]azulene-9-carbonyl)-benzylamide;
1-(3,3-Dimethyl-butyl)-piperidine-4-carboxylic acid 2-chloro-4-(6-chloro-3-methyl-4,10-dihydro-3H-2,3,4,9-tetraaza-benzo[f]azulene-9-carbonyl)-benzylamide;
1-(3,3-Dimethyl-butyl)-piperidine-4-carboxylic acid 2-fluoxo-4-(3-methyl-4,10-dihydro-3H-2,3,4,9-tetraaza-benzo[f]azulene-9-carbonyl)-benzylamide;
1-(3,3-Dimethyl-butyl)-piperidine-4-carboxylic acid 2-methyl-4-(2-methyl-4,5-dihydro-1H-1,3,6-triaza-benzo[e]azulene-6-carbonyl)-benzylamide;
1-(3,3-Dimethyl-butyl)-piperidine-4-carboxylic acid 2-methyl-4-(2-methyl-4,5-dihydro-1-oxa-3,6-diaza-benzo[e]azulene-6-carbonyl)-benzylamide;
1-(3,3-Dimethyl-butyl)-piperidine-4-carboxylic acid 2-methyl-4-(3-methyl-4,10-dihydro-3H-2,3,4,9-tetraaza-benzo[f]azulene-9-carbonyl)-benzylamide;
1-(3,3-Dimethyl-butyl)-piperidine-4-carboxylic acid 3-chloro-4- (3, 6-dimethyl-4,10-dihydro-3H-2,3,4,9-tetraaza-benzo[f]azulene-9-carbonyl)-benzylamide;
1-(3,3-Dimethyl-butyl)-piperidine-4-carboxylic acid 3-chloro-4-(3-methyl-4,10-dihydro-3H-2,3,4,9-tetraaza-benzo[f]azulene-9-carbonyl)-benzylamide;
1-(3,3-Dimethyl-butyl)-piperidine-4-carboxylic acid 3-chloro-4-(6-chloro-3-methyl-4,10-dihydro-3H-2,3,4,9-tetraaza-benzo[f]azulene-9-carbonyl)-benzylamide;
1-(3,3-Dimethyl-butyl)-piperidine-4-carboxylic acid 3-fluoro-4-(3-methyl-4,10-dihydro-3H-2,3,4,9-tetraaza-benzo[f]azulene-9-carbonyl)-benzylamide;
1-(3,3-Dimethyl-butyl)-piperidine-4-carboxylic acid 3-methyl-4-(3-methyl-4,10-dihydro-3H-2,3,4,9-tetraaza-benzo[f]azulene-9-carbonyl)-benzylamide;
1-(3,3-Dimethyl-butyl)-piperidine-4-carboxylic acid 4-(3,6-dimethyl-4,10-dihydro-3H-2,3,4,9-tetraaza-benzo[f]azulene-9-carbonyl)-2-methyl-benzylamide;
1-(3,3-Dimethyl-butyl)-piperidine-4-carboxylic acid 4-(3,6-dimethyl-4,10-dihydro-3H-2,3,4, 9-tetraaza-benzo[f]azulene-9-carbonyl)-3-methyl-benzylamide;
1-(3,3-Dimethyl-butyl)-piperidine-4-carboxylic acid 4-(3,6-dimethyl-4,10-dihydro-3H-2,3,4,9-tetraaza-benzo[f]azulene-9-carbonyl)-2-fluoro-benzylamide;
1-(3,3-Dimethyl-butyl)-piperidine-4-carboxylic acid 4-(3,6-dimethyl-4,10-dihydro-3H-2,3,4,9-tetraaza-benzo[f]azulene-9-carbonyl)-3-fluoro-benzylamide;
1-(3,3-Dimethyl-butyl)-piperidine-4-carboxylic acid 4-(6-chloro-3-methyl-4,10-dihydro-3H-2,3,4,9-tetraaza-benzo[f]azulene-9-carbonyl)-2-methyl-benzylamide;
1-(3,3-Dimethyl-butyl)-piperidine-4-carboxylic acid 4-(6-chloro-3-methyl-4,10-dihydro-3H-2,3,4,9-tetraaza-benzo[f]azulene-9-carbonyl)-2-fluoro-benzylamide;
1-(3,3-Dimethyl-butyl)-piperidine-4-carboxylic acid 4-(6-chloro-3-methyl-4,10-dihydro-3H-2,3,4,9-tetraaza-benzo[f]azulene-9-carbonyl)-3-methyl-benzylamide;
1-(3,3-Dimethyl-butyl)-piperidine-4-carboxylic acid 4-(6-chloro-3-methyl-4,10-dihydro-3H-2,3,4,9-tetraaza-benzo[f]azulene-9-carbonyl)-3-fluoro-benzylamide;
1-(3,3-Dimethyl-butyl)-piperidine-4-carboxylic acid 4-[(4-chloro-phenyl)-methyl-carbamoyl]-2-methyl-benzylamide;
1-Cyclopropylmethyl-piperidine-4-carboxylic acid 2-chloro-4-(3,6-dimethyl-4,10-dihydro-3H-2,3,4,9-tetraaza-benzo[f]azulene-9-carbonyl)-benzylamide;
1-Cyclopropylmethyl-piperidine-4-carboxylic acid 2-chloro-4-(3-methyl-4,10-dihydro-3H-2,3,4,9-tetraaza-benzo[f]azulene-9-carbonyl)-benzylamide;
1-Cyclopropylmethyl-piperidine-4-carboxylic acid 2-chloro-4-(6-chloro-3-methyl-4,10-dihydro-3H-2,3,4,9-tetraaza-benzo[f]azulene-9-carbonyl)-benzylamide;
1-Cyclopropylmethyl-piperidine-4-carboxylic acid 2-fluoro-4-(3-methyl-4,10-dihydro-3H-2,3,4,9-tetraaza-benzo[f]azulene-9-carbonyl)-benzylamide;
1-Cyclopropylmethyl-piperidine-4-carboxylic acid 2-methyl-4-(3-methyl-4,10-dihydro-3H-2,3,4,9-tetraaza-benzo[f]azulene-9-carbonyl)-benzylamide;
1-Cyclopropylmethyl-piperidine-4-carboxylic acid 3-chloro-4-(3,6-dimethyl-4,10-dihydro-3H-2,3,4,9-tetraaza-benzo[f]azulene-9-carbonyl)-benzylamide;
1-Cyclopropylmethyl-piperidine-4-carboxylic acid 3-chloro-4-(3-methyl-4,10-dihydro-3H-2,3,4,9-tetraaza-benzo[f]azulene-9-carbonyl)-benzylamide;
1-Cyclopropylmethyl-piperidine-4-carboxylic acid 3-chloro-4-(6-chloro-3-methyl-4,10-dihydro-3H-2,3,4,9-tetraaza-benzo[f]azulene-9-carbonyl)-benzylamide;
1-Cyclopropylmethyl-piperidine-4-carboxylic acid 3-fluoro-4-(3-methyl-4,10-dihydro-3H-2,3,4,9-tetraaza-benzo[f]azulene-9-carbonyl)-benzylamide;
1-Cyclopropylmethyl-piperidine-4-carboxylic acid 3-methyl-4-(3-methyl-4,10-dihydro-3H-2,3,4,9-tetraaza-benzo[f]azulene-9-carbonyl)-benzylamide;
1-Cyclopropylmethyl-piperidine-4-carboxylic acid 4-(3,6-dimethyl-4,10-dihydro-3H-2,3,4,9-tetraaza-benzo[f]azulene-9-carbonyl)-2-methyl-benzylamide;
1-Cyclopropylmethyl-piperidine-4-carboxylic acid 4-(3,6-dimethyl-4,10-dihydro-3H-2,3,4,9-tetraaza-benzo[f]azulene-9-carbonyl)-3-methyl-benzylamide;
1-Cyclopropylmethyl-piperidine-4-carboxylic acid 4-(3,6-dimethyl-4,10-dihydro-3H-2,3,4,9-tetraaza-benzo[f]azulene-9-carbonyl)-2-fluoro-benzylamide;
1-Cyclopropylmethyl-piperidine-4-carboxylic acid 4-(3,6-dimethyl-4,10-dihydro-3H-2,3,4,9-tetraaza-benzo[f]azulene-9-carbonyl)-3-fluoro-benzylamide;
1-Cyclopropylmethyl-piperidine-4-carboxylic acid 4-(6-chloro-3-methyl-4,10-dihydro-3H-2,3,4,9-tetraaza-benzo[f]azulene-9-carbonyl)-2-fluoro-benzylamide;
1-Cyclopropylmethyl-piperidine-4-carboxylic acid 4-(6-chloro-3-methyl-4,10-dihydro-3H-2,3,4,9-tetraaza-benzo[f]azulene-9-carbonyl)-2-methyl-benzylamide;
1-Cyclopropylmethyl-piperidine-4-carboxylic acid 4-(6-chloro-3-methyl-4,10-dihydro-3H-2,3,4,9-tetraaza-benzo[f]azulene-9-carbonyl)-3-methyl-benzylamide;
1-Cyclopropylmethyl-piperidine-4-carboxylic acid 4-(6-chloro-3-methyl-4,10-dihydro-3H-2,3,4,9-tetraaza-benzo[f]azulene-9-carbonyl)-3-fluoro-benzylamide;
1-Cyclopropylmethyl-piperidine-4-carboxylic acid 4-[(4-chloro-phenyl)-methyl-carbamoyl]-2-methyl-benzylamide;
Cyclobutanecarboxylic acid 2-chloro-4-(6-chloro-3-methyl-4,10-dihydro-3H-2,3,4,9-tetraaza-benzo[f]azulene-9-carbonyl)-benzylamide;
Cyclobutanecarboxylic acid 4-(3,6-dimethyl-4,10-dihydro-3H-2,3,4,9-tetraaza-benzo[f]azulene-9-carbonyl)-2-fluoro-benzylamide;
Cyclobutanecarboxylic acid 4-(6-chloro-3-methyl-4,10-dihydro-3H-2,3,4,9-tetraaza-benzo[f]azulene-9-carbonyl)-2-fluoro-benzylamide;
Cyclopentanecarboxylic acid 2-chloro-4-(6-chloro-3-methyl-4,10-dihydro-3H-2,3,4,9-tetraaza-benzo[f]azulene-9-carbonyl)-benzylamide;
Cyclopentanecarboxylic acid 4-(3,6-dimethyl-4,10-dihydro-3H-2,3,4,9-tetraaza-benzo[f]azulene-9-carbonyl)-2-fluoro-benzylamide;
Cyclopentanecarboxylic acid 4-(6-chloro-3-methyl-4,10-dihydro-3H-2,3,4,9-tetraaza-benzo[f]azulene-9-carbonyl)-2-fluoro-benzylamide;
Cyclopropanecarboxylic acid 2-chloro-4-(3,6-dimethyl-4,10-dihydro-3H-2,3,4,9-tetraaza-benzo[f]azulene-9-carbonyl)-benzylamide;
Cyclopropanecarboxylic acid 2-chloro-4-(6-chloro-3-methyl-4,10-dihydro-3H-2,3,4,9-tetraaza-benzo[f]azulene-9-carbonyl)-benzylamide;
Cyclopropanecarboxylic acid 3-chloro-4-(3,6-dimethyl-4,10-dihydro-3H-2,3,4,9-tetraaza-benzo[f]azulene-9-carbonyl)-benzylamide;
Cyclopropanecarboxylic acid 3-chloro-4-(6-chloro-3-methyl-4,10-dihydro-3H-2,3,4,9-tetraaza-benzo[f]azulene-9-carbonyl)-benzylamide;
Cyclopropanecarboxylic acid 4-(3,6-dimethyl-4,10-dihydro-3H-2,3,4,9-tetraaza-benzo[f]azulene-9-carbonyl)-2-methyl-benzylamide;
Cyclopropanecarboxylic acid 4-(3,6-dimethyl-4,10-dihydro-3H-2,3,4,9-tetraaza-benzo[f]azulene-9-carbonyl)-2-fluoro-benzylamide;
Cyclopropanecarboxylic acid 4-(3,6-dimethyl-4,10-dihydro-3H-2,3,4,9-tetraaza-benzo[f]azulene-9-carbonyl)-3-fluoro-benzylamide;
Cyclopropanecarboxylic acid 4-(3,6-dimethyl-4,10-dihydro-3H-2,3,4,9-tetraaza-benzo[f]azulene-9-carbonyl)-3-methyl-benzylamide;
Cyclopropanecarboxylic acid 4-(6-chloro-3-methyl-4,10-dihydro-3H-2,3,4,9-tetraaza-benzo[f]azulene-9-carbonyl)-2-methyl-benzylamide;
Cyclopropanecarboxylic acid 4-(6-chloro-3-methyl-4,10-dihydro-3H-2,3,4,9-tetraaza-benzo[f]azulene-9-carbonyl)-3-fluoro-benzylamide;
Cyclopropanecarboxylic acid 4-(6-chloro-3-methyl-4,10-dihydro-3H-2,3,4,9-tetraazabenzo[f]azulene-9-carbonyl)-2-fluoro-benzylamide;
Cyclopropanecarboxylic acid 4-(6-chloro-3-methyl-4,10-dihydro-3H-2,3,4,9-tetraaza-benzo[f]azulene-9-carbonyl)-3-methyl-benzylamide;
N-(2-Chloro-4-(6-chloro-3-methyl-4,10-dihydro-3H-2,3,4,9-tetraaza-benzo[f]azulene-9-carbonyl)-benzyl]-propionamide;
N-[2-Chloro-4-(6-chloro-3-methyl-4,10-dihydro-3H-2,3,4,9-tetraaza-benzo[f]azulene-9-carbonyl)-benzyl]-2,2-dimethyl-propionamide;
N-[2-Chloro-4-(6-chloro-3-methyl-4,10-dihydro-3H-2,3,4,9-tetraaza-benzo[f]azulene-9-carbonyl)-benzyl]-isobutyramide;
N-[2-Chloro-4-(6-chloro-3-methyl-4,10-dihydro-3H-2,3,4,9-tetraaza-benzo[f]azulene-9-carbonyl)-benzyl]-butyramide;
N-[4-(3,6-Dimethyl-4,10-dihydro-3H-2,3,4,9-tetraazabenzo[f]azulene-9-carbonyl)-2-fluoro-benzyl]-propionamide;
N-[4-(3,6-Dimethyl-4,10-dihydro-3H-2,3,4,9-tetraazabenzo[f]azulene-9-carbonyl)-2-fluoro-benzyl]-2,2-dimethyl-propionamide;
N-[4-(3,6-Dimethyl-4,10-dihydro-3H-2,3,4,9-tetraazabenzo[f]azulene-9-carbonyl)-2-fluoro-benzyl]-isobutyramide;
N-[4-(3,6-Dimethyl-4,10-dihydro-3H-2,3,4,9-tetraazabenzo[f]azulene-9-carbonyl)-2-fluoro-benzyl]-butyramide;
N-[4-(6-Chloro-3-methyl-4,10-dihydro-3H-2,3,4,9-tetraaza-benzo[f]azulene-9-carbonyl)-2-fluoro-benzyl]-propionamide;
N-[4-(6-Chloro-3-methyl-4,10-dihydro-3H-2,3,4,9-tetraaza-benzo[f]azulene-9-carbonyl)-2-fluoro-benzyl]-2,2-dimethyl-propionamide;
N-[4-(6-Chloro-3-methyl-4,10-dihydro-3H-2,3,4,9-tetraaza-benzo[f]azulene-9-carbonyl)-2-fluoro-benzyl]-isobutyramide;
N-[4-(6-Chloro-3-methyl-4,10-dihydro-3H-2,3,4,9-tetraaza-benzo[f]azulene-9-carbonyl)-2-fluoro-benzyl]-acetamide;
N-[4-(6-Chloro-3-methyl-4,10-dihydro-3H-2,3,4,9-tetraaza-benzo[f]azulene-9-carbonyl)-2-fluoro-benzyl]-butyramide;
N-[4-(6-Chloro-3-methyl-4,10-dihydro-3H-2,3,4,9-tetraaza-benzo[f]azulene-9-carbonyl)-2-fluoro-benzyl]-formamide;
N-[4-(6-Chloro-3-ethyl-4,10-dihydro-3H-2,3,4,9-tetraaza-benzo[f]azulene-9-carbonyl)-2-fluoro-benzyl]-propionamide;
Cyclopropanecarboxylic acid 4-(6-chloro-3-ethyl-4,10-dihydro-3H-2,3,4,9-tetraaza-benzo[f]azulene-9-carbonyl)-2-fluoro-benzylamide;
N-[2-Fluoro-4-(6-fluoro-3-methyl-4,10-dihydro-3H-2,3,4,9-tetraaza-benzo[f]azulene-9-carbonyl)-benzyl]-propionamide;
N-[2-Fluoro-4-(6-fluoro-3-methyl-4,10-dihydro-3H-2,3,4,9-tetraaza-benzo[f]azulene-9-carbonyl)-benzyl]-isobutyramide;
Cyclopropanecarboxylic acid 2-fluoro-4-(6-fluoro-3-methyl-4,10-dihydro-3H-2,3,4,9-tetraazabenzo[f]azulene-9-carbonyl)-benzylamide; and
N-(4-(9-Chloro-2-methyl-4,5-dihydro-1H-1,3,6-triazabenzo[e]azulene-6-carbonyl)-2-methyl-benzyl]-isobutyramide.

18. A compound according to claim 1, which is 1-(3,3-dimethyl-butyl)-piperidine-4-carboxylic acid 4-(3,6-dimethyl-4,10-dihydro-3H-2,3,4,9-tetraaza-benzo[f]azulene-9-carbonyl)-2-fluoro-benzylamide or a tautomer or a pharmaceutically acceptable salt thereof.

19. A compound according to claim 1, which is 1-(3,3-dimethyl-butyl)-piperidine-4-carboxylic acid 4-(3,6-dimethyl-4,10-dihydro-3H-2,3,4,9-tetraaza-benzo[f]azulene-9-carbonyl)-2-methyl-benzylamide or a tautomer or a pharmaceutically acceptable salt thereof.

20. A compound according to claim 1, which is cyclopropanecarboxylic acid 4-(6-chloro-3-methyl-4,10-dihydro-3H-2,3,4,9-tetraazabenzo[f]azulene-9-carbonyl)-2-fluorobenzylamide or a tautomer or a pharmaceutically acceptable salt thereof.

21. A compound according to claim 1, which is N-[2-chloro-4-(6-chloro-3-methyl-4,10-dihydro-3H-2,3,4,9-tetraaza-benzo[f]azulene-9-carbonyl)-benzyl]-butyramide or a pharmaceutically acceptable salt thereof.

22. A compound according to claim 1, which is cyclopropanecarboxylic acid 4-(6-chloro-3-methyl-4,10-dihydro-3H-2,3,4,9-tetraaza-benzo[f]azulene-9-carbonyl)-2-methyl-benzylamide or a pharmaceutically acceptable salt thereof.

23. A pharmaceutical composition comprising a compound according to any of the preceding claims.

24. The pharmaceutical composition according to claim 23, formulated for oral administration, preferably as a tablet, a capsule or a sachet.

25. The pharmaceutical composition according to claim 23 or 24, for the treatment of a condition selected among dysmenorrhoea, pre-term labour, hypertension, Raynaud's disease, brain oedema, motion sickness, hyperlipemia, small cell lung cancer, depression, anxiety, hyponatremia, liver cirrhosis and congestive heart failure.

26. Use of a compound of formula 1b 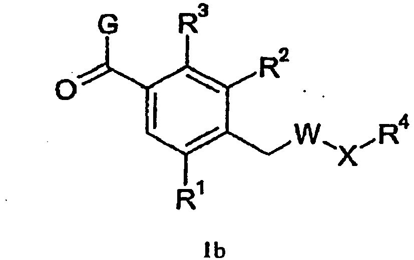 wherein G is a group selected among general formula 2a, 3a, 4a, 5a, and 6a, 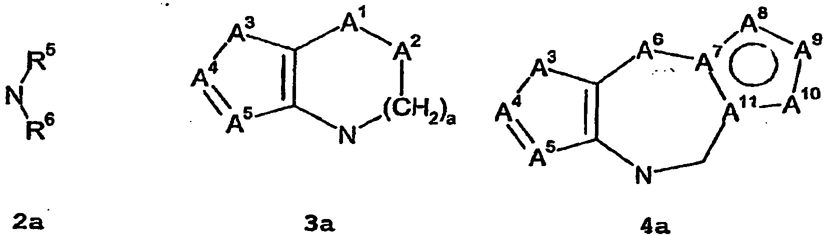 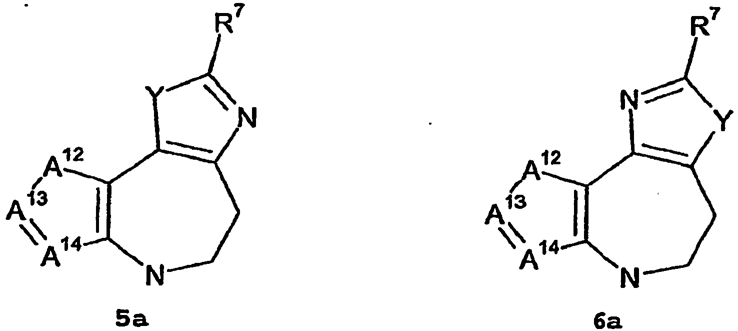 wherein:
A¹ is selected among CH₂, CH(OH), NH, N-alkyl, O and S;
A² is selected among CH₂, CH(OH), C(=O) and NH;
A³ and A¹² are independently selected among S, NH, N-alkyl, -C(R⁸)=CH-, -C(R⁸)=N-, -N=C(R⁸)- and -CH=C(R⁸)-;
A⁴ and A¹³ are independently selected among C(R⁹) and N;
A⁵ and A¹⁴ are independently selected among C(R¹⁰) and N;
A⁶ is selected among CH₂, NH, N-alkyl and O;
A⁷ and A¹¹ are independently selected among C and N;
A⁸ and A⁹ are independently selected among CH, N, NH, N(CH₂)_{b}R¹¹ and S;
A¹⁰ is selected among -CH=CH-, CH, N, NH, N-(CH₂)_{b}-R¹¹ and S;
wherein the ring constituted by A⁷, A⁸, A⁹, A¹⁰ and A¹¹ is aromatic;
R¹, R² and R³ are independently selected among H, alkyl, O-alkyl, NO₂, F, Cl and Br;
R⁴ is selected among H, alkyl, aryl, heteroaryl, -(CH₂)_{c}-R¹² and 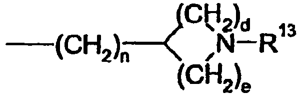
R⁵ and R⁶ are independently selected among alkyl, aryl, -(CH₂)_{f}-aryl and -(CH₂)_{f}-heteroaryl;
R⁷ is selected among H, alkyl, aryl, heteroaryl and -(CH₂)_{g}-R¹⁴;
R⁸, R⁹ and R¹⁰ are independently selected among H, alkyl, alkoxy, F, Cl, Br, CN, NH₂, NO₂, NH(alkyl), N(alkyl)₂; with the provisos that when G is 3a, and R⁴ is H, alkyl, aryl, heteroaryl or -(CH₂)_{c}-R¹², then the ring containing A³, A⁴ and A⁵ is substituted in at least one position by alkyl, alkoxy, F, Cl, Br, CN, NH₂, NO₂, NH(alkyl) or N(alkyl)₂; and
when G is 4a, R⁴ is H, alkyl, aryl, heteroaryl or -(CH₂)_{c}-R¹², and A⁸ is NH, NCH₃ or S, then the ring containing A³, A⁴ and A⁵ is substituted in at least one position by alkyl, alkoxy, F, Cl, Br, CN, NH₂, NO₂, NH(alkyl) or N(alkyl)₂;
R¹¹ and R¹² are independently selected among H, alkyl, aryl, heteroaryl, F, OH, O-alkyl, S-alkyl, O-acyl, NH₂, NH-alkyl, N(alkyl)₂, NH-acyl, N (alkyl)-acyl, CO₂H, CO₂-alkyl, CONH₂, CONH-alkyl, CON(alkyl)₂, CN and CF₃;
R¹³ is selected among H, alkyl, aryl, heteroaryl, -(CH₂)ₕ-R¹⁵ and Z-R¹⁶;
R¹⁴ and R¹⁵ are independently selected among H, alkyl, alkenyl, aryl, heteroaryl, F, OH, O-alkyl, S-alkyl, O-acyl, NH₂, NH-alkyl, N(alkyl)₂, NH-acyl, N(alkyl)-acyl, CO₂H, CO₂-alkyl, CO-alkyl, CO-aryl, CONH₂, CONH-alkyl, CON(alkyl)₂, alkenyl-CO₂-alkyl, alkenyl-aryl, CN and CF₃;
R¹⁶ is selected among H, alkyl, alkenyl, aryl, heteroaryl, O-aryl, -(CH₂)₁-R¹⁷, cyclopropyl-aryl and O-(CH₂)₁-R¹⁷;
R¹⁷ is selected among H, alkyl, aryl, heteroaryl, F, OH, O-alkyl, S-alkyl, O-acyl, NH₂, NH-alkyl, N(alkyl)₂, NH-acyl, N(alkyl)-acyl, CO₂H, CO₂-alkyl, CONH₂, CONH-alkyl, CON(alkyl)₂, CN and CF₃;
W is selected among O and NH;
X is selected among (CH₂)ₘ, C(=O) and S(=O)ⱼ Y is selected among O, S, NH and N-alkyl;
Z is selected among -C(=O), -C(=O)-O and -S(=O)ₖ;
a is selected among 1 and 2;
b and c are independently selected among 0, 1, 2 and 3;
d, e and f are independently selected among 1 and 2;
g, h and i are independently selected among 1, 2 and 3;
j and k are independently selected among 1 and 2; and
m and n are independently selected among 0, 1 and 2,
or a compound which is a tautomer or a pharmaceutically acceptable salt thereof, for the manufacture of a medicament for the treatment of a condition selected among dysmenorrhoea, pre-term labour, hypertension, Raynaud's disease, brain oedema, motion sickness, hyperlipemia, small cell lung cancer, depression, anxiety, hyponatremia, liver cirrhosis and congestive heart failure.

27. The use according to claim 26, wherein said compound is 1-(3,3-dimethyl-butyl)-piperidine-4-carboxylic acid 4-(3,6-dimethyl-4,10-dihydro-3H-2,3,4,9-tetraazabenzo[f]azulene-9-carbonyl)-2-fluoro-benzylamide or a tautomer or a pharmaceutically acceptable salt thereof.

28. The use according to claim 26, wherein said compound is 1-(3,3-dimethyl-butyl)-piperidine-4-carboxylic acid 4-(3,6-dimethyl-4,10-dihydro-3H-2,3,4,9-tetraazabenzo[f]azulene-9-carbonyl)-2-methyl-benzylamide or a tautomer or a pharmaceutically acceptable salt thereof:

29. The use according to claim 26, wherein said compound is cyclopropanecarboxylic acid 4-(6-chloro-3-methyl-4,10-dihydro-3H-2,3,4,9-tetraazabenzo[f]azulene-9-carbonyl)-2-fluoro-benzylamide or a tautomer or a pharmaceutically acceptable salt thereof.

30. The use according to claim 26, wherein said compound is N-[2-chloro-4-(6-chloro-3-methyl-4,10-dihydro-3H-2,3,4,9-tetraaza-benzo[f]azulene-9-carbonyl)-benzyl]-butyramide or a pharmaceutically acceptable salt thereof.

31. The use according to claim 26, wherein said compound is, which is cyclopropanecarboxylic acid 4-(6-chloro-3-methyl-4,10-dihydro-3H-2,3,4,9-tetraazabenzo[f]azulene-9-carbonyl)-2-methyl-benzylamide or a pharmaceutically acceptable salt thereof.

32. A compound of formula 1b, as defined in claim 26, for use in the treatment of a condition selected among dysmenorrhoea, pre-term labour, hypertension, Raynaud's disease, brain oedema, motion sickness, hyperlipemia, small cell lung cancer, depression, anxiety, hyponatremia, liver cirrhosis and congestive heart failure.

33. The compound for use according to claim 32, wherein said compound is 1-(3,3-dimethyl-butyl)-piperidine-4-carboxylic acid 4-(3,6-dimethyl-4,10-dihydro-3H-2,3,4,9-tetraaza-benzo[f]azulene-9-carbonyl)-2-fluoro-benzylamide or a tautomer or a pharmaceutically acceptable salt thereof.

34. The compound for use according to claim 32, wherein said compound is 1-(3,3-dimethyl-butyl)-piperidine-4-carboxylic acid 4-(3,6-dimethyl-4,10-dihydro-3H-2,3,4,9-tetraaza-benzo[f]azulene-9-carbonyl)-2-methyl-benzylamide or a tautomer or a pharmaceutically acceptable salt thereof.

35. The compound for use according to claim 32, wherein said compound is cyclopropanecarboxylic acid 4-(6-chloro-3-methyl-4,10-dihydro-3H-2,3,4,9-tetraazabenzo[f]azulene-9-carbonyl)-2-fluoro-benzylamide or a tautomer or a pharmaceutically acceptable salt thereof.

36. The compound for use according to claim 32, wherein said compound is N-[2-chloro-4-(6-chloro-3-methyl-4,10-dihydro-3H-2,3,4,9-tetraaza-benzo[f]azulene-9-carbonyl)-benzyl]-butyramide or a pharmaceutically acceptable salt thereof.

37. The compound for use according to claim 32, wherein said compound is, which is cyclopropanecarboxylic acid 4-(6-chloro-3-methyl-4,10-dihydro-3H-2,3,4,9-tetraazabenzo[f]azulene-9-carbonyl)-2-methyl-benzylamide or a pharmaceutically acceptable salt thereof.

## Patentansprüche

1. Verbindung der allgemeinen Formel **1a**, oder eine Verbindung, bei der es sich um ein Tautomer oder ein pharmazeutisch unbedenkliches Salz davon handelt, 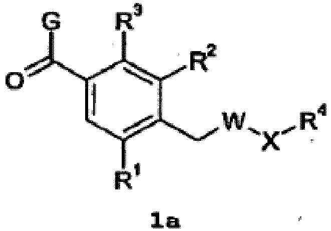 wobei G für eine Gruppe ausgewählt aus den allgemeinen Formeln **2a**, **3a**, **4a**, **5a** und **6a** steht, 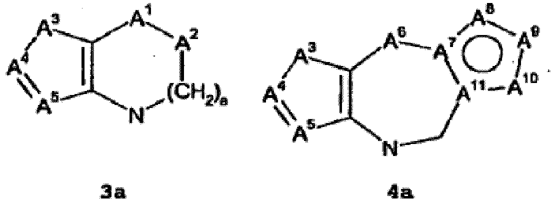 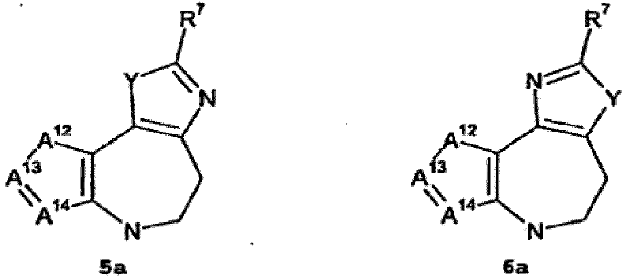 wobei:
A¹ aus CH₂, CH(OH), NH, N-Alkyl, O und S ausgewählt ist,
A² aus CH₂, CH(OH), C(=O) und NH ausgewählt ist,
A³ und A¹² unabhängig voneinander aus S, NH, N-Alkyl, -C(R⁸)=CH-, -C(R⁸)=N-, -N=C(R⁸)- und -CH=C(R⁸)- ausgewählt sind,
A⁴ und A¹³ unabhängig voneinander aus C(R⁹) und N ausgewählt sind,
A⁵ und A¹⁴ unabhängig voneinander aus C(R¹⁰) und N ausgewählt sind,
A⁶ aus CH₂, NH, N-Alkyl und 0 ausgewählt ist,
A⁷ und A¹¹ unabhängig voneinander aus C und N ausgewählt sind,
A⁸ und A⁹ unabhängig aus voneinander aus CH, N, NH, N(CH₂)_{b}R¹¹ und S ausgewählt sind,
A¹⁰ aus -CH=CH-, CH, N, NH, N-(CH₂)_{b}-R¹¹ und S ausgewählt ist,
wobei der von A⁷, A⁸, A⁹, A¹⁰ und A¹¹ gebildete Ring aromatisch ist,
R¹, R² und R³ unabhängig voneinander aus H, Alkyl, O-Alkyl, NO₂, F, Cl und Br ausgewählt sind,
R⁴ aus H, Alkyl, Aryl, Heteroaryl, -(CH₂)_{c}-R¹² und 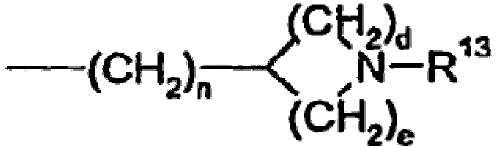 ausgewählt ist,
R⁷ aus H, Alkyl, Aryl, Heteroaryl und - (CH₂)₉-R¹⁴ ausgewählt ist,
R⁸, R⁹ und R¹⁰ unabhängig voneinander aus H, Alkyl, Alkoxy, F, Cl, Br, CN, NH₂, NO₂, NH (Alkyl), N(Alkyl)₂ ausgewählt sind, mit den Maßgaben, dass, wenn G für **3a** steht und R⁴ für H, Alkyl, Aryl, Heteroaryl oder -(CH₂)_{c}-R¹² steht, der A³, A⁴ und A⁵ enthaltende Ring an mindestens einer Position durch Alkyl, Alkoxy, F, Cl, Br, CN, NH₂, NO₂, NH (Alkyl) oder N(Alkyl)₂ substituiert ist, und
wenn G für **4a** steht, R⁴ für H, Alkyl, Aryl, Heteroaryl oder -(CH₂)_{c}-R¹² steht und A⁸ für NH, NCH₃ oder S steht, der A³, A⁴ und A⁵ enthaltende Ring an mindestens einer Position durch Alkyl, Alkoxy, F, Cl, Br, CN, NH₂, NO₂, NH(Alkyl) oder N(Alkyl)₂ substituiert ist,
R¹¹ und R¹² unabhängig voneinander aus H, Alkyl, Aryl, Heteroaryl, F, OH, O-Alkyl, S-Alkyl, O-Acyl, NH₂, NH-Alkyl, N(Alkyl)₂, NH-Acyl, N(Alkyl)-acyl, CO₂H, CO₂-Alkyl, CONH₂, CONH-Alkyl, CON(Alkyl)₂, CN und CF₃ ausgewählt sind,
R¹³ aus H, Alkyl, Aryl, Heteroaryl, -(CH₂)ₙ-R¹⁵ und Z-R¹⁶ ausgewählt ist,
R¹⁴ und R¹⁵ unabhängig voneinander aus H, Alkyl, Alkenyl, Aryl, Heteroaryl, F, OH, O-Alkyl, S-Alkyl, O-Acyl, NH₂, NH-Alkyl, N(Alkyl)₂, NH-Acyl, N(Alkyl)-acyl, CO₂H, CO₂-Alkyl, CO-Alkyl, CO-Aryl, CONH₂, CONH-Alkyl, CON(Alkyl)₂, Alkenyl-CO₂-alkyl, Alkenyl-aryl, CN und CF₃ ausgewählt sind,
R¹⁶ aus H, Alkyl, Alkenyl, Aryl, Heteroaryl, O-Aryl, -(CH₂)ᵢ-R¹⁷, Cyclopropyl-aryl und O-(CH₂)ᵢ-R¹⁷ ausgewählt ist,
R¹⁷ aus H, Alkyl, Aryl, Heteroaryl, F, OH, O-Alkyl, S-Alkyl, O-Acyl, NH₂, NH-Alkyl, N(Alkyl)₂, NH-Acyl, N(Alkyl)-acyl, CO₂H, CO₂-Alkyl, CONH₂, CONH-Alkyl, CON(Alkyl)₂, CN und CF₃ ausgewählt ist,
W aus O und NH ausgewählt ist,
X aus (CH₂)ₘ, C(=O) und S(=O)ⱼ ausgewählt ist,
Y aus O, S, NH und N-Alkyl ausgewählt ist,
Z aus -C(=O), -C(=O)-O und -S(=O)ₖ ausgewählt ist,
wobei Alkyl aus gesättigten Kohlenwasserstoffresten einschließlich geradkettigen Gruppen mit bis zu 10 Atomen (C₁-C₁₀), verzweigten Gruppen mit 3 bis 10 Atomen (C₃-C₁₀), cyclischen Gruppen mit 3 bis 8 Atomen (C₃-C₈) und Kombinationen geradkettiger, verzweigter und cyclischer Gruppen ausgewählt ist,
a aus 1 und 2 ausgewählt ist,
b und c unabhängig voneinander aus 0, 1, 2 und 3 ausgewählt sind,
d und e unabhängig voneinander aus 1 und 2 ausgewählt sind,
g, h und i unabhängig voneinander aus 1, 2 und 3 ausgewählt sind,
j und k unabhängig voneinander aus 1 und 2 ausgewählt sind und
m und n unabhängig voneinander aus 0, 1 und 2 ausgewählt sind,
wobei mindestens einer der Reste R¹, R² und R³ nicht für Wasserstoff steht und wobei es sich bei der Verbindung der allgemeinen Formel **1a** nicht um (4-Aminomethyl-3-methylphenyl)-(5*H*,11*H*-benzo[*e*]pyrrolo[1,2-*a*][1,4]diazepin-1-yl)methanon 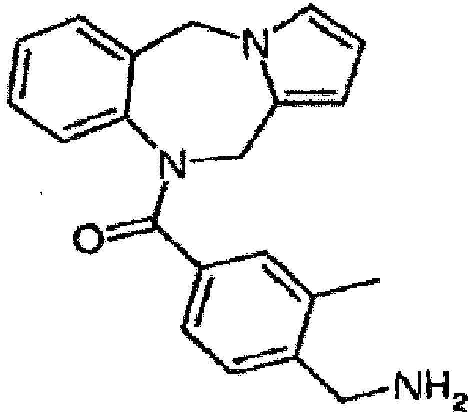 handelt.

2. Verbindung nach Anspruch 1, wobei G ausgewählt ist aus:

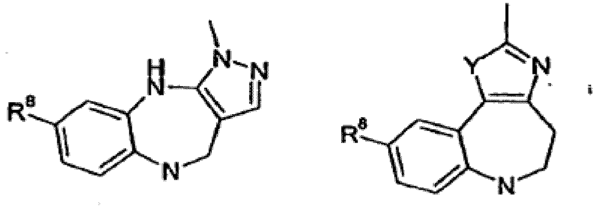

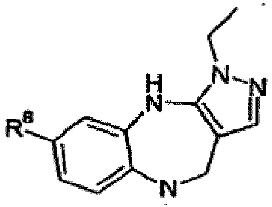

3. Verbindung nach Anspruch 1, wobei G ausgewählt ist aus:

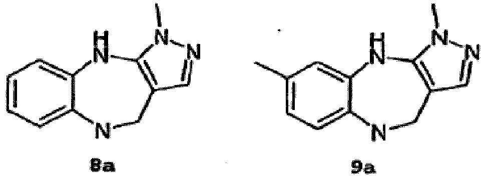

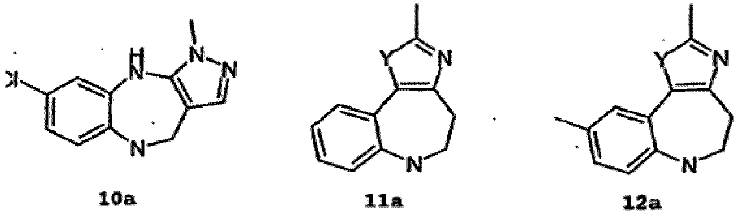

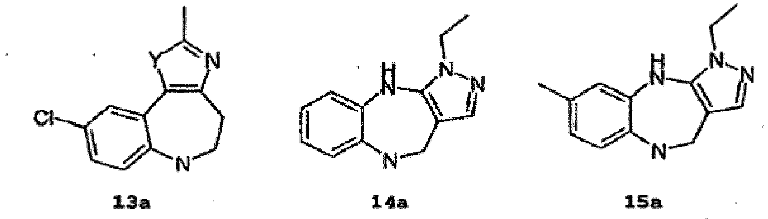

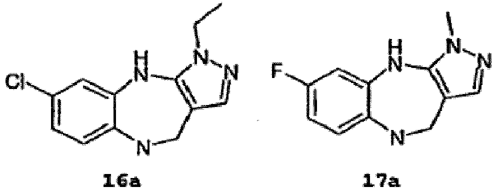

4. Verbindung nach Anspruch 1, wobei einer der Reste R¹, R² und R³ aus Methyl, Chlor und Fluor ausgewählt ist und die anderen für Wasserstoff stehen.

5. Verbindung nach Anspruch 1, wobei W für NH steht.

6. Verbindung nach Anspruch 1, wobei R⁴ für Alkyl steht.

7. Verbindung nach Anspruch 1, wobei d für 2 steht und e für 2 steht.

8. Verbindung nach Anspruch 1, wobei R¹³ für Alkyl steht.

9. Verbindung nach Anspruch 1, wobei n für 0 steht.

10. Verbindung nach Anspruch 1, wobei R² für H steht, R⁴ für ein Piperidin steht, wobei d für 2 steht und e für 2 steht, W für NH steht, X für C(=O) und n für 0 steht, wie in Formel **18a** gezeigt: 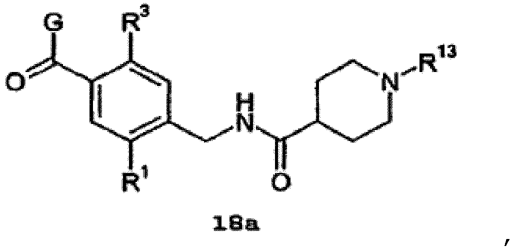 wobei R¹ aus Methyl, Chlor und Fluor ausgewählt ist und R³ für H steht oder R¹ für H steht und R³ aus Methyl, Chlor und Fluor ausgewählt ist und R¹³ für Alkyl steht.

11. Verbindung nach Anspruch 1, wobei X für C(=O) steht, n für 0 steht und R⁴ für ein Piperidin steht, wobei d für 2 steht und e für 2 steht, wie in Formel **19a** gezeigt: 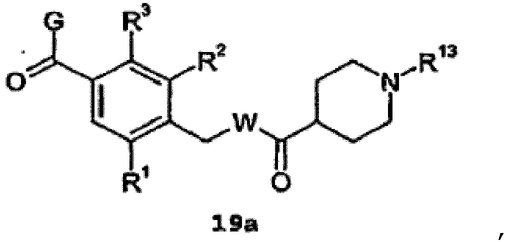 wobei G aus den allgemeinen Formeln **8a** bis **17a** ausgewählt ist und R¹³ für Alkyl steht.

12. Verbindung nach Anspruch 1, wobei R² für H steht und W für NH steht, wie in Formel **20a** gezeigt: 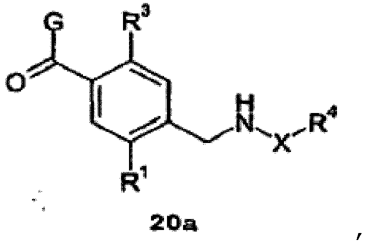 wobei entweder R¹ aus Methyl, Chlor und Fluor ausgewählt ist und R³ für H steht oder R¹ für H steht und R³ aus Methyl, Chlor und Fluor ausgewählt ist und G aus den allgemeinen Formeln **8a** bis **17a** ausgewählt ist.

13. Verbindung nach Anspruch 1, wobei R² für H steht, W für NH steht und X für C(=0) steht, wie in Formel **21a** gezeigt: 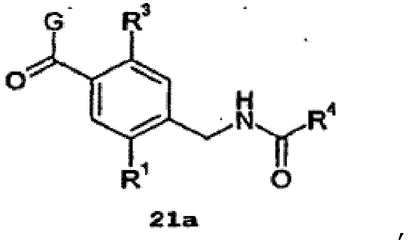 wobei R¹ aus Methyl, Chlor und Fluor ausgewählt ist und R³ für H steht oder R¹ für H steht und R³ aus Methyl, Chlor und Fluor ausgewählt ist und R⁴ für Alkyl steht.

14. Verbindung nach Anspruch 1, wobei X für C(=O) steht, wie in Formel 22a gezeigt: 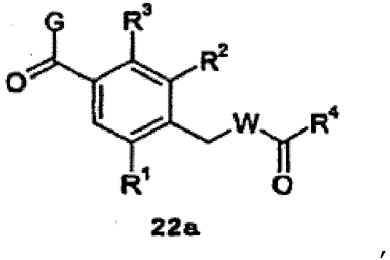 wobei G aus den allgemeinen Formeln **8a** bis **17a** ausgewählt ist und R⁴ für Alkyl steht.

15. Verbindung nach Anspruch 1, wobei R² und R³ beide für H stehen, W für NH steht und X für C(=O) steht, wie in Formel **23a** gezeigt: 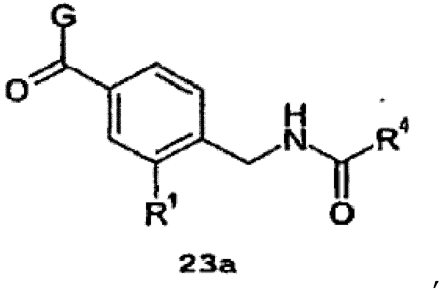 wobei G aus den allgemeinen Formeln **9a**, **10a**, **15a**, **16a** und **17a** ausgewählt ist, R¹ aus Methyl, Chlor und Fluor ausgewählt ist und R⁴ für Alkyl steht.

16. Verbindung nach Anspruch 1, wobei R² und R³ beide für H stehen, R⁴ für ein Piperidin steht, wobei d für 2 steht und e für 2 steht, W für NH steht, X für C(=O) steht und n für 0 steht, wie in Formel **24a** gezeigt: 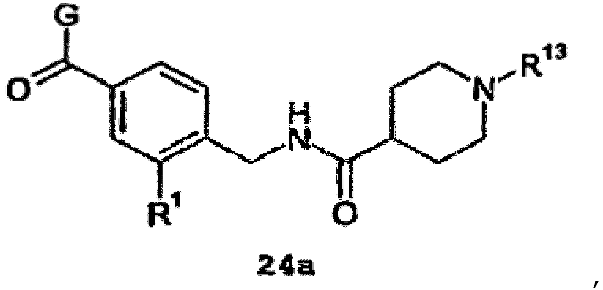 wobei G aus den allgemeinen Formeln **9a**, **10a, 15a**, **16a** und **17a** ausgewählt ist, R¹ aus Methyl, Chlor und Fluor ausgewählt ist und R¹³ für Alkyl steht.

17. Verbindung nach Anspruch 1, ausgewählt aus der Gruppe bestehend aus:
1-(3,3-Dimethylbutyl)piperidin-4-carbonsäure-2-chlor-4-(3,6-dimethyl-4,10-dihydro-3H-2,3,4,9-tetraazabenzo[f]azulen-9-carbonyl)benzylamid,
1-(3,3-Dimethylbutyl)piperidin-4-carbonsäure-2-chlor-4-(3-methyl-4,10-dihydro-3H-2,3,4,9-tetraazabenzo[f]azulen-9-carbonyl)benzylamid,
1-(3,3-Dimethylbutyl)piperidin-4-carbonsäure-2-chlor-4-(6-chlor-3-methyl-4,10-dihyydro-3H-2,3,4,9-tetraazabenzo[f]azulen-9-carbonyl)benzylamid,
1-(3,3-Dimethylbutyl)piperidin-4-carbonsäure-2-fluor-4-(3-methyl-4,10-dihydro-3H-2,3,4,9-tetraazabenzo[f]azulen-9-carbonyl)benzylamid,
1-(3,3-Dimethylbutyl)piperidin-4-carbonsäure-2-methyl-4-(2-methyl-4,5-dihydro-1H-1,3,6-triazabenzo[e]azulen-6-carbonyl)benzylamid,
1-(3,3-Dimethylbutyl)piperidin-4-carbonsäure-2-methyl-4-(2-methyl-4,5-dihydro-1-oxa-3,6-diazabenzo[e]azulen-6-carbonyl)benzylamid,
1-(3,3-Dimethylbutyl)piperidin-4-carbonsäure-2-methyl-4-(3-methyl-4,10-dihydro-3H-2,3,4,9-tetraazabenzo[f]azulen-9-carbonyl)benzylamid,
1-(3,3-Dimethylbutyl)piperidin-4-carbonsäure-3-chlor-4-(3,6-dimethyl-4,10-dihydro-3H-2,3,4,9-tetraazabenzo[f]azulen-9-carbonyl)benzylamid,
1-(3,3-Dimethylbutyl)piperidin-4-carbonsäure-3-chlor-4-(3-methyl-4,10-dihydro-3H-2,3,4,9-tetraazabenzo[f]azulen-9-carbonyl)benzylamid,
1-(3,3-Dimethylbutyl)piperidin-4-carbonsäure-3-chlor-4-(6-chlor-3-methyl-4,10-dihydro-3H-2,3,4,9-tetraazabenzo[f]azulen-9-carbonyl)benzylamid,
1-(3,3-Dimethylbutyl)piperidin-4-carbonsäure-3-fluor-4-(3-methyl-4,10-dihydro-3H-2,3,4,9-tetraazabenzo[f]azulen-9-carbonyl)benzylamid,
1-(3,3-Dimethylbutyl)piperidin-4-carbonsäure-3-methyl-4-(3-methyl-4,10-dihydro-3H-2,3,4,9-tetraazabenzo[f]azulen-9-carbonyl)benzylamid,
1-(3,3-Dimethylbutyl)piperidin-4-carbonsäure-4-(3,6-dimethyl-4,10-dihydro-3H-2,3,4,9-tetraazabenzo[f]azulen-9-carbonyl)-2-methylbenzylamid,
1-(3,3-Dimethylbutyl)piperidin-4-carbonsäure-4-(3,6-dimethyl-4,10-dihydro-3H-2,3,4,9-tetraazabenzo[f]azulen-9-carbonyl)-3-methylbenzylamid,
1-(3,3-Dimethylbutyl)piperidin-4-carbonsäure-4-(3,6-dimethyl-4,10-dihydro-3H-2,3,4,9-tetraazabenzo[f]azulen-9-carbonyl)-2-fluorbenzylamid,
1-(3,3-Dimethylbutyl)piperidin-4-carbonsäure-4-(3,6-dimethyl-4,10-dihydro-3H-2,3,4,9-tetraazabenzo[f]azulen-9-carbonyl)-3-fluorbenzylamid,
1-(3,3-Dimethylbutyl)piperidin-4-carbonsäure-4-(6-chlor-3-methyl-4,10-dihydro-3H-2,3,4,9-tetraazabenzo[f]azulen-9-carbonyl)-2-methylbenzylamid,
1-(3,3-Dimethylbutyl)piperidin-4-carbonsäure-4-(6-chlor-3-methyl-4,10-dihydro-3H-2,3,4,9-tetraazabenzo[f]azulen-9-carbonyl)-2-fluorbenzylamid,
1-(3,3-Dimethylbutyl)piperidin-4-carbonsäure-4-(6-chlor-3-methyl-4,10-dihydro-3H-2,3,4,9-tetraazabenzo[f]azulen-9-carbonyl)-3-methylbenzylamid,
1-(3,3-Dimethylbutyl)piperidin-4-carbonsäure-4-(6-chlor-3-methyl-4,10-dihydro-3H-2,3,4,9-tetraazabenzo[f]azulen-9-carbonyl)-3-fluorbenzylamid,
1-(3,3-Dimethylbutyl)piperidin-4-carbonsäure-4-[(4-chlorphenyl)methylcarbamoyl]-2-methylbenzylamid,
1-Cyclopropylmethylpiperidin-4-carbonsäure-2-chlor-4-(3,6-dimethyl-4,10-dihydro-3H-2,3,4,9-tetraazabenzo[f]azulen-9-carbonyl)benzylamid,
1-Cyclopropylmethylpiperidin-4-carbonsäure-2-chlor-4-(3-methyl-4,10-dihydro-3H-2,3,4,9-tetraazabenzo[f]azulen-9-carbonyl)benzylamid,
1-Cyclopropylmethylpiperidin-4-carbonsäure-2-chlor-4-(6-chlor-3-methyl-4,10-dihydro-3H-2,3,4,9-tetraazabenzo[f]azulen-9-carbonyl)benzylamid,
1-Cyclopropylmethylpiperidin-4-carbonsäure-2-fluor-4-(3-methyl-4,10-dihydro-3H-2,3,4,9-tetraazabenzo[f]azulen-9-carbonyl)benzylamid,
1-Cyclopropylmethylpiperidin-4-carbonsäure-2-methyl-4-(3-methyl-4,10-dihydro-3H-2,3,4,9-tetraazabenzo[f]azulen-9-carbonyl)benzylamid,
1-Cyclopropylmethylpiperidin-4-carbonsäure-3-chlor-4-(3,6-dimethyl-4,10-dihydro-3H-2,3,4,9-tetraazabenzo[f]azulen-9-carbonyl)benzylamid,
1-Cyclopropylmethylpiperidin-4-carbonsäure-3-chlor-4-(3-methyl-4,10-dihydro-3H-2,3,4,9-tetraazabenzo[f]azulen-9-carbonyl)benzylamid,
1-Cyclopropylmethylpiperidin-4-carbonsäure-3-chlor-4-(6-chlor-3-methyl-4,10-dihydro-3H-2,3,4,9-tetraazabenzo[f]azulen-9-carbonyl)benzylamid,
1-Cyclopropylmethylpiperidin-4-carbonsäure-3-fluor-4-(3-methyl-4,10-dihydro-3H-2,3,4,9-tetraazabenzo[f]azulen-9-carbonyl)benzylamid,
1-Cyclopropylmethylpiperidin-4-carbonsäure-3-methyl-4-(3-methyl-4,10-dihydro-3H-2,3,4,9-tetraazabenzo[f]azulen-9-carbonyl)benzylamid,
1-Cyclopropylmethylpiperidin-4-carbonsäure-4-(3,6-dimethyl-4,10-dihydro-3H-2,3,4,9-tetraazabenzo[f]azulen-9-carbonyl)-2-methylbenzylamid,
1-Cyclopropylmethylpiperidin-4-carbonsäure-4-(3,6-dimethyl-4,10-dihydro-3H-2,3,4,9-tetraazabenzo[f]azulen-9-carbonyl)-3-methylbenzylamid,
1-Cyclopropylmethylpiperidin-4-carbonsäure-4-(3,6-dimethyl-4,10-dihydro-3H-2,3,4,9-tetraazabenzo[f]azulen-9-carbonyl)-2-fluorbenzylamid,
1-Cyclopropylmethylpiperidin-4-carbonsäure-4-(3,6-dimethyl-4,10-dihydro-3H-2,3,4,9-tetraazabenzo[f]azulen-9-carbonyl)-3-fluorbenzylamid,
1-Cyclopropylmethylpiperidin-4-carbonsäure-4-(6-chlor-3-methyl-4,10-dihydro-3H-2,3,4,9-tetraazabenzo[f]azulen-9-carbonyl)-2-fluorbenzylamid,
1-Cyclopropylmethylpiperidin-4-carbonsäure-4-(6-chlor-3-methyl-4,10-dihydro-3H-2,3,4,9-tetraazabenzo[f]azulen-9-carbonyl)-2-methylbenzylamid,
1-Cyclopropylmethylpiperidin-4-carbonsäure-4-(6-chlor-3-methyl-4,10-dihydro-3H-2,3,4,9-tetraazabenzo[f]azulen-9-carbonyl)-3-methylbenzylamid,
1-Cyclopropylmethylpiperidin-4-carbonsäure-4-(6-chlor-3-methyl-4,10-dihydro-3H-2,3,4,9-tetraazabenzo[f]azulen-9-carbonyl)-3-fluorbenzylamid,
1-Cyclopropylmethylpiperidin-4-carbonsäure-4-[(4-chlorphenyl)methylcarbamoyl]-2-methylbenzylamid,
Cyclobutancarbonsäure-2-chlor-4-(6-chlor-3-methyl-4,10-dihydro-3H-2,3,4,9-tetraazabenzo[f]azulen-9-carbonyl)benzylamid,
Cyclobutancarbonsäure-4-(3,6-dimethyl-4,10-dihydro-3H-2,3,4,9-tetraazabenzo[f]azulen-9-carbonyl)-2-fluorbenzylamid,
Cyclobutancarbonsäure-4-(6-chlor-3-methyl-4,10-dihydro-3H-2,3,4,9-tetraazabenzo[f]azulen-9-carbonyl)-2-fluorbenzylamid,
Cyclopentancarbonsäure-2-chlor-4-(6-chlor-3-methyl-4,10-dihydro-3H-2,3,4,9-tetraazabenzo[f]azulen-9-carbonyl)benzylamid,
Cyclopentancarbonsäure-4-(3,6-dimethyl-4,10-dihydro-3H-2,3,4,9-tetraazabenzo[f]azulen-9-carbonyl)-2-fluorbenzylamid,
Cyclopentancarbonsäure-4-(6-chlor-3-methyl-4,10-dihydro-3H-2,3,4,9-tetraazabenzo[f]azulen-9-carbonyl)-2-fluorbenzylamid,
Cyclopropancarbonsäure-2-chlor-4-(3,6-dimethyl-4,10-dihydro-3H-2,3,4,9-tetraazabenzo[f]azulen-9-carbonyl)benzylamid,
Cyclopropancarbonsäure-2-chlor-4-(6-chlor-3-methyl-4,10-dihydro-3H-2,3,4,9-tetraazabenzo[f]azulen-9-carbonyl)benzylamid,
Cyclopropancarbonsäure-3-chlor-4-(3,6-dimethyl-4,10-dihydro-3H-2,3,4,9-tetraazabenzo[f]azulen-9-carbonyl)benzylamid,
Cyclopropancarbonsäure-3-chlor-4-(6-chlor-3-methyl-4,10-dihydro-3H-2,3,4,9-tetraazabenzo[f]azulen-9-carbonyl)benzylamid,
Cyclopropancarbonsäure-4-(3,6-dimethyl-4,10-dihydro-3H-2,3,4,9-tetraazabenzo[f]azulen-9-carbonyl)-2-methylbenzylamid,
Cyclopropancarbonsäure-4-(3,6-dimethyl-4,10-dihydro-3H-2,3,4,9-tetraazabenzo[f]azulen-9-carbonyl)-2-fluorbenzylamid,
Cyclopropancarbonsäure-4-(3,6-dimethyl-4,10-dihydro-3H-2,3,4,9-tetraazabenzo[f]azulen-9-carbonyl)-3-fluorbenzylamid,
Cyclopropancarbonsäure-4-(3,6-dimethyl-4,10-dihydro-3H-2,3,4,9-tetraazabenzo[f]azulen-9-carbonyl)-3-methylbenzylamid,
Cyclopropancarbonsäure-4-(6-chlor-3-methyl-4,10-dihydro-3H-2,3,4,9-tetraazabenzo[f]azulen-9-carbonyl)-2-methylbenzylamid,
Cyclopropancarbonsäure-4-(6-chlor-3-methyl-4,10-dihydro-3H-2,3,4,9-tetraazabenzo[f]azulen-9-carbonyl)-3-fluorbenzylamid,
Cyclopropancarbonsäure-4-(6-chlor-3-methyl-4,10-dihydro-3H-2,3,4,9-tetraazabenzo[f]azulen-9-carbonyl)-2-fluorbenzylamid,
Cyclopropancarbonsäure-4-(6-chlor-3-methyl-4,10-dihydro-3H-2,3,4,9-tetraazabenzo[f]azulen-9-carbonyl)-3-methylbenzylamid,
N-[2-Chlor-4-(6-chlor-3-methyl-4,10-dihydro-3H-2,3,4,9-tetraazabenzo[f]azulen-9-carbonyl)benzyl]propionamid, N-[2-Chlor-4-(6-chlor-3-methyl-4,10-dihydro-3H-2,3,4,9-tetraazabenzo[f]azulen-9-carbonyl)benzyl]-2,2-dimethylpropionamid,
N-[2-Chlor-4-(6-chlor-3-methyl-4,10-dihydro-3H-2,3,4,9-tetraazabenzo[f]azulen-9-carbonyl)benzyl]isobutyramid, N-[2-Chlor-4-(6-chlor-3-methyl-4,10-dihydro-3H-2,3,4,9-tetraazabenzo[f]azulen-9-carbonyl)benzyl]butyramid,
N-[4-(3,6-Dimethyl-4,10-dihydro-3H-2,3,4,9-tetraazabenzo[f]azulen-9-carbonyl)-2-fluorbenzyl]propionamid,
N-[4-(3,6-Dimethyl-4,10-dihydro-3H-2,3,4,9-tetraazabenzo[f]azulen-9-carbonyl)-2-fluorbenzyl]-2,2-dimethylpropionamid,
N-[4-(3,6-Dimethyl-4,10-dihydro-3H-2,3,4,9-tetraazabenzo[f]azulen-9-carbonyl)-2-fluorbenzyl]isobutyramid,
N-[4-(3,6-Dimethyl-4,10-dihydro-3H-2,3,4,9-tetraazabenzo[f]azulen-9-carbonyl)-2-fluorbenzyl]butyramid,
N-[4-(6-Chlor-3-methyl-4,10-dihydro-3H-2,3,4,9-tetraazabenzo[f]azulen-9-carbonyl)-2-fluorbenzyl]propionamid,
N-[4-(6-Chlor-3-methyl-4,10-dihydro-3H-2,3,4,9-tetraazabenzo[f]azulen-9-carbonyl)-2-fluorbenzyl]-2,2-dimethylpropionamid,
N-[4-(6-Chlor-3-methyl-4,10-dihydro-3H-2,3,4,9-tetraazabenzo[f]azulen-9-carbonyl)-2-fluorbenzyl]isobutyramid,
N-[4-(6-Chlor-3-methyl-4,10-dihydro-3H-2,3,4,9-tetraazabenzo[f]azulen-9-carbonyl)-2-fluorbenzyl]acetamid,
N-[4-(6-Chlor-3-methyl-4,10-dihydro-3H-2,3,4,9-tetraazabenzo[f]azulen-9-carbonyl)-2-fluorbenzyl]butyramid,
N-[4-(6-Chlor-3-methyl-4,10-dihydro-3H-2,3,4,9-tetraazabenzo[f]azulen-9-carbonyl)-2-fluorbenzyl]formamid,
N-[4-(6-Chlor-3-ethyl-4,10-dihydro-3H-2,3,4,9-tetraazabenzo[f]azulen-9-carbonyl)-2-fluorbenzyl]propionamid,
Cyclopropancarbonsäure-4-(6-chlor-3-ethyl-4,10-dihydro-3H-2,3,4,9-tetraazabenzo[f]azulen-9-carbonyl)-2-fluorbenzylamid,
N-[2-Fluor-4-(6-fluor-3-methyl-4,10-dihydro-3H-2,3,4,9-tetraazabenzo[f]azulen-9-carbonyl)benzyl]propionamid,
N-[2-Fluor-4-(6-fluor-3-methyl-4,10-dihydro-3H-2,3,4,9-tetraazabenzo[f]azulen-9-carbonyl)benzyl]isobutyramid,
Cyclopropancarbonsäure-2-fluor-4-(6-fluor-3-methyl-4,10-dihydro-3H-2,3,4,9-tetraazabenzo[f]azulen-9-carbonyl)benzylamid und
N-[4-(9-Chlor-2-methyl-4,5-dihydro-1H-1,3,6-triazabenzo[e]azulen-6-carbonyl)-2-methylbenzyl]isobutyramid.

18. Verbindung nach Anspruch 1, bei der es sich um 1-(3,3-Dimethylbutyl)piperidin-4-carbonsäure-4-(3,6-dimethyl-4,10-dihydro-3*H*-2,3,4,9-tetraazabenzo[*f*]azulen-9-carbonyl)-2-fluorbenzylamid handelt, oder ein Tautomer oder ein pharmazeutisch unbedenkliches Salz davon.

19. Verbindung nach Anspruch 1, bei der es sich um 1-(3,3-Dimethylbutyl)piperidin-4-carbonsäure-4-(3,6-dimethyl-4,10-dihydro-3*H*-2,3,4,9-tetraazabenzo[*f*]azulen-9-carbonyl)-2-methylbenzamid handelt, oder ein Tautomer oder ein pharmazeutisch unbedenkliches Salz davon.

20. Verbindung nach Anspruch 1, bei der es sich um Cyclopropancarbonsäure-4-(6-chlor-3-methyl-4,10-dihydro-3*H*-2,3,4,9-tetraazabenzo[*f*]azulen-9-carbonyl)-2-fluorbenzylamid handelt, oder ein Tautomer oder ein pharmazeutisch unbedenkliches Salz davon.

21. Verbindung nach Anspruch 1, bei der es sich um *N-*[2-Chlor-4-(6-chlor-3-methyl-4,10-dihydro-3*H*-2,3,4,9-tetraazabenzo[*f*]azulen-9-carbonyl)benzyl]butyramid handelt, oder ein pharmazeutisch unbedenkliches Salz davon.

22. Verbindung nach Anspruch 1, bei der es sich um Cyclopropancarbonsäure-4-(6-chlor-3-methyl-4,10-dihydro-3*H*-2,3,4,9-tetraazabenzo[*f*]azulen-9-carbonyl)-2-methylbenzylamid handelt, oder ein pharmazeutisch unbedenkliches Salz davon.

23. Pharmazeutische Zusammensetzung, umfassend eine Verbindung nach einem der vorhergehenden Ansprüche.

24. Pharmazeutische Zusammensetzung nach Anspruch 23, formuliert für die orale Verabreichung, vorzugsweise als eine Tablette, eine Kapsel oder ein Beutel.

25. Pharmazeutische Zusammensetzung nach Anspruch 23 oder 24 zur Behandlung eines Leidens ausgewählt aus Dysmenorrhoea, einem vorzeitigen Einsetzen der Wehen, Bluthochdruck, Raynaud-Krankheit, Hirnödem, Reisekrankheit, Hyperlipämie, kleinzelligem Lungenkrebs, Depression, Angst, Hyponatriämie, Leberzirrhose und dekompensierter Herzinsuffizienz.

26. Verwendung einer Verbindung der Formel **1b** 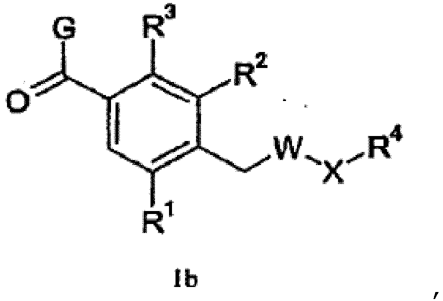 in welcher G für eine Gruppe ausgewählt aus den allgemeinen Formeln **2a**, **3a**, **4a**, **5a** und **6a** steht, 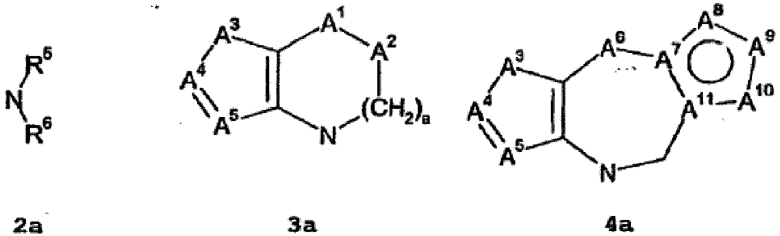 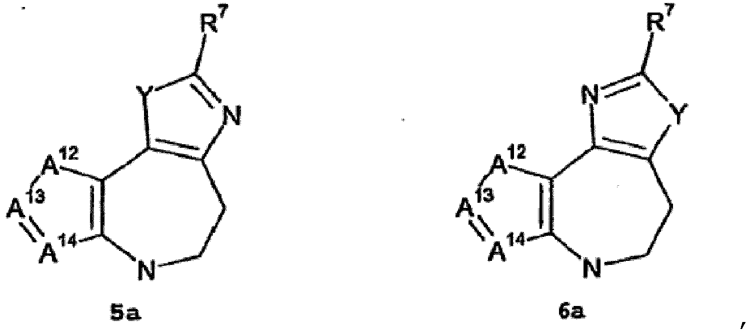 wobei
A¹ aus CH₂, CH(OH), NH, N-Alkyl, 0 und S ausgewählt ist,
A² aus CH₂, CH(OH), C(=O) und NH ausgewählt ist,
A³ und A¹² unabhängig voneinander aus S, NH, N-Alkyl, -C(R⁸)=CH-, -C(R⁸)=N-, -N=C(R⁸)- und -CH=C(R⁸)- ausgewählt sind,
A⁴ und A¹³ unabhängig voneinander aus C(R⁹) und N ausgewählt sind,
A⁵ und A¹⁴ unabhängig voneinander aus C(R¹⁰) und N ausgewählt sind,
A⁶ aus CH₂, NH, N-Alkyl und 0 ausgewählt ist,
A⁷ und A¹¹ unabhängig voneinander aus C und N ausgewählt sind,
A⁸ und A⁹ unabhängig aus voneinander aus CH, N, NH, N(CH₂)_{b}R¹¹ und S ausgewählt sind,
A¹⁰ aus -CH=CH-, CH, N, NH, N-(CH₂)_{b}-R¹¹ und S ausgewählt ist,
wobei der von A⁷, A⁸, A⁹, A¹⁰ und A¹¹ gebildete Ring aromatisch ist,
R¹, R² und R³ unabhängig voneinander aus H, Alkyl, O-Alkyl, NO₂, F, Cl und Br ausgewählt sind,
R⁴ aus H, Alkyl, Aryl, Heteroaryl, -(CH₂)_{c}-R¹² und 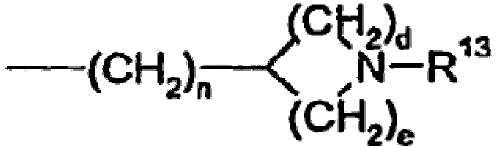 ausgewählt ist,
R⁵ und R⁶ unabhängig voneinander aus Alkyl, Aryl, -(CH₂)_{f}-Aryl und -(CH₂)_{f}-Heteroaryl ausgewählt sind,
R⁷ aus H, Alkyl, Aryl, Heteroaryl und -(CH₂)₉-R¹⁴ ausgewählt ist,
R⁸, R⁹ und R¹⁰ unabhängig voneinander aus H, Alkyl, Alkoxy, F, Cl, Br, CN, NH₂, NO₂, NH (Alkyl), N(Alkyl)₂ ausgewählt sind, mit den Maßgaben, dass, wenn G für **3a** steht und R⁴ für H, Alkyl, Aryl, Heteroaryl oder -(CH₂)_{c}-R¹² steht, der A³, A⁴ und A⁵ enthaltende Ring an mindestens einer Position durch Alkyl, Alkoxy, F, Cl, Br, CN, NH₂, NO₂, NH (Alkyl) oder N(Alkyl)₂ substituiert ist und,
wenn G für **4a** steht, R⁴ für H, Alkyl, Aryl, Heteroaryl oder -(CH₂)_{c}-R¹² steht und A⁸ für NH, NCH₃ oder S steht, der A³, A⁴ und A⁵ enthaltende Ring an mindestens einer Position durch Alkyl, Alkoxy, F, Cl, Br, CN, NH₂, NO₂, NH(Alkyl) oder N(Alkyl)₂ substituiert ist,
R¹¹ und R¹² unabhängig voneinander aus H, Alkyl, Aryl, Heteroaryl, F, OH, O-Alkyl, S-Alkyl, O-Acyl, NH₂, NH-Alkyl, N(Alkyl)₂, NH-Acyl, N(Alkyl)-acyl, CO₂H, CO₂-Alkyl, CONH₂, CONH-Alkyl, CON(Alkyl)₂, CN und CF₃ ausgewählt sind,
R¹³ aus H, Alkyl, Aryl, Heteroaryl, -(CH₂)ₙ-R¹⁵ und Z-R¹⁶ ausgewählt ist,
R¹⁴ und R¹⁵ unabhängig voneinander aus H, Alkyl, Alkenyl, Aryl, Heteroaryl, F, OH, O-Alkyl, S-Alkyl, O-Acyl, NH₂, NH-Alkyl, N(Alkyl)₂, NH-Acyl, N(Alkyl)-acyl, CO₂H, CO₂-Alkyl, CO-Alkyl, CO-Aryl, CONH₂, CONH-Alkyl, CON(Alkyl)₂, Alkenyl-CO₂-alkyl, Alkenyl-aryl, CN und CF₃ ausgewählt sind,
R¹⁶ aus H, Alkyl, Alkenyl, Aryl, Heteroaryl, O-Aryl, -(CH₂)ᵢ-R¹⁷, Cyclopropyl-aryl und O-(CH₂)ᵢ-R¹⁷ ausgewählt ist,
R¹⁷ aus H, Alkyl, Aryl, Heteroaryl, F, OH, O-Alkyl, S-Alkyl, O-Acyl, NH₂, NH-Alkyl, N(Alkyl)₂, NH-Acyl, N(Alkyl)-acyl, CO₂H, CO₂-Alkyl, CONH₂, CONH-Alkyl, CON(Alkyl)₂, CN und CF₃ ausgewählt ist,
W aus O und NH ausgewählt ist,
X aus (CH₂)ₘ, C(=O) und S(=O)ⱼ ausgewählt ist,
Y aus 0, S, NH und N-Alkyl ausgewählt ist,
Z aus -C(=O), -C(=O)-O und -S(=O)ₖ ausgewählt ist,
a aus 1 und 2 ausgewählt ist,
b und c unabhängig voneinander aus 0, 1, 2 und 3 ausgewählt sind,
d, e und f unabhängig voneinander aus 1 und 2 ausgewählt sind,
g, h und i unabhängig voneinander aus 1, 2 und 3 ausgewählt sind,
j und k unabhängig voneinander aus 1 und 2 ausgewählt sind und
m und n unabhängig voneinander aus 0, 1 und 2 ausgewählt sind,
oder einer Verbindung, bei der es sich um ein Tautomer oder ein pharmazeutisch unbedenkliches Salz davon handelt, zur Herstellung eines Medikaments zur Behandlung eines Leidens ausgewählt aus Dysmenorrhoea, einem vorzeitigen Einsetzen der Wehen, Bluthochdruck,
Raynaud-Krankheit, Hirnödem, Reisekrankheit, Hyperlipämie, kleinzelligem Lungenkrebs, Depression, Angst, Hyponatriämie, Leberzirrhose und dekompensierter Herzinsuffizienz.

27. Verwendung nach Anspruch 26, wobei es sich bei der Verbindung um 1-(3,3-Dimethylbutyl)piperidin-4-carbonsäure-4-(3,6-dimethyl-4,10-dihydro-3*H*-2,3,4,9-tetraazabenzo[*f*]azulen-9-carbonyl)-2-fluorbenzylamid handelt, oder ein Tautomer oder ein pharmazeutisch unbedenkliches Salz davon.

28. Verwendung nach Anspruch 26, wobei es sich bei der Verbindung um 1-(3,3-Dimethylbutyl)piperidin-4-carbonsäure-4-(3,6-dimethyl-4,10-dihydro-3*H*-2,3,4,9-tetraazabenzo[*f*]azulen-9-carbonyl)-2-methylbenzamid handelt, oder ein Tautomer oder ein pharmazeutisch unbedenkliches Salz davon.

29. Verwendung nach Anspruch 26, wobei es sich bei der Verbindung um Cyclopropancarbonsäure-4-(6-chlor-3-methyl-4,10-dihydro-3*H*-2,3,4,9-tetraazabenzo[*f*]azulen-9-carbonyl)-2-fluorbenzylamid handelt, oder ein Tautomer oder ein pharmazeutisch unbedenkliches Salz davon.

30. Verwendung nach Anspruch 26, wobei es sich bei der Verbindung um *N*-[2-Chlor-4-(6-chlor-3-methyl-4,10-dihydro-3*H*-2,3,4,9-tetraazabenzo[*f*]azulen-9-carbonyl)benzyl]butyramid handelt, oder ein pharmazeutisch unbedenkliches Salz davon.

31. Verwendung nach Anspruch 26, wobei es sich bei der Verbindung um Cyclopropancarbonsäure-4-(6-chlor-3-methyl-4,10-dihydro-3*H*-2,3,4,9-tetraazabenzo[*f*]azulen-9-carbonyl)-2-methylbenzylamid handelt, oder ein pharmazeutisch unbedenkliches Salz davon.

32. Verbindung der Formel **1b**, wie in Anspruch 26 definiert, zur Verwendung bei der Behandlung eines Leidens ausgewählt aus Dysmenorrhoea, einem vorzeitigen Einsetzen der Wehen, Bluthochdruck, Raynaud-Krankheit, Hirnödem, Reisekrankheit, Hyperlipämie, kleinzelligem Lungenkrebs, Depression, Angst, Hyponatriämie, Leberzirrhose und dekompensierter Herzinsuffizienz.

33. Verbindung zur Verwendung nach Anspruch 32, wobei es sich bei der Verbindung um 1-(3,3-Dimethylbutyl)piperidin-4-carbonsäure-4-(3,6-dimethyl-4,10-dihydro-3*H*-2,3,4,9-tetraazabenzo[*f*]azulen-9-carbonyl)-2-fluorbenzylamid handelt, oder ein Tautomer oder ein pharmazeutisch unbedenkliches Salz davon.

34. Verbindung zur Verwendung nach Anspruch 32, wobei es sich bei der Verbindung um 1-(3,3-Dimethylbutyl)piperidin-4-carbonsäure-4-(3,6-dimethyl-4,10-dihydro-3*H*-2,3,4,9-tetraazabenzo[*f*]azulen-9-carbonyl)-2-methylbenzamid handelt, oder ein Tautomer oder ein pharmazeutisch unbedenkliches Salz davon.

35. Verbindung zur Verwendung nach Anspruch 32, wobei es sich bei der Verbindung um Cyclopropancarbonsäure-4-(6-chlor-3-methyl-4,10-dihydro-3*H*-2,3,4,9-tetraazabenzo[*f*]azulen-9-carbonyl)-2-fluorbenzylamid handelt, oder ein Tautomer oder ein pharmazeutisch unbedenkliches Salz davon.

36. Verbindung zur Verwendung nach Anspruch 32, wobei es sich bei der Verbindung um *N*-[2-Chlor-4-(6-chlor-3-methyl-4,10-dihydro-3*H*-2,3,4,9-tetraazabenzo[*f*]azulen-9-carbonyl)benzyl]butyramid handelt, oder ein pharmazeutisch unbedenkliches Salz davon.

37. Verbindung zur Verwendung nach Anspruch 32, wobei es sich bei der Verbindung um Cyclopropancarbonsäure-4-(6-chlor-3-methyl-4,10-dihydro-3*H*-2,3,4,9-tetraazabenzo[*f*]azulen-9-carbonyl)-2-methylbenzylamid handelt, oder ein pharmazeutisch unbedenkliches Salz davon.

## Revendications

1. Composé selon la formule générale 1a, ou un composé qui est un tautomère ou un sel pharmaceutiquement acceptable de celui-ci, 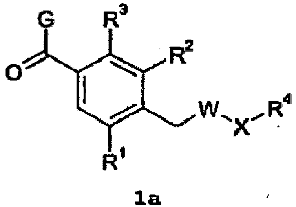 dans laquelle G est un groupe choisi parmi les formules générales 2a, 3a, 4a, 5a et 6a, 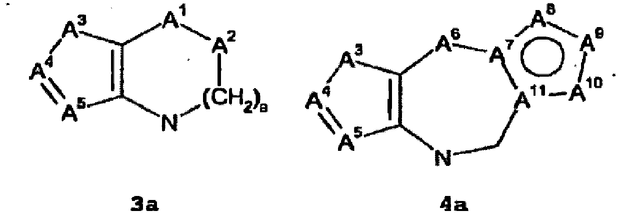 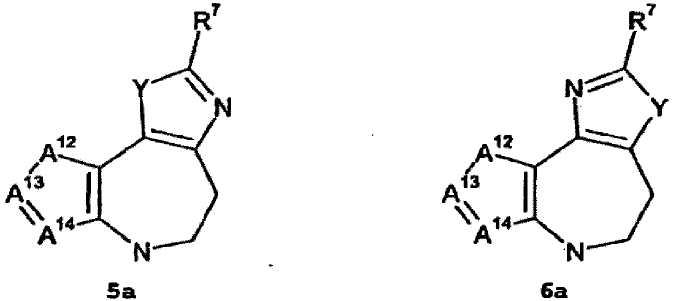 dans lesquelles :
A¹ est choisi parmi CH₂, CH(OH), NH, N-alkyle, 0 et S ;
A² est choisi parmi CH₂, CH(OH), C(=O) et NH ;
A³ et A¹² sont choisis indépendamment parmi S, NH, N-alkyle, -C(R⁸)=CH-, -C(R⁸)=N-, -N=C(R⁸)- et -CH=C(R⁸)- ;
A⁴ et A¹³ sont choisis indépendamment parmi C(R⁹) et N ;
A⁵ et A¹⁴ sont choisis indépendamment parmi C(R¹⁰) et N ;
A⁶ est choisi parmi CH₂, NH, N-alkyle et 0 ;
A⁷ et A¹¹ sont choisis indépendamment parmi C et N ;
A⁸ et A⁹ sont choisis indépendamment parmi CH, N, NH,
N(CH₂)_{b}R¹¹ et S ;
A¹⁰ est choisi parmi -CH=CH-, CH, N, NH, N-(CH₂)_{b}-R¹¹ et S ;
le cycle constitué par A⁷, A⁸, A⁹, A¹⁰ et A¹¹ étant aromatique ;
R¹, R² et R³ sont choisis indépendamment parmi H, alkyle, O-alkyle, NO₂, F, Cl et Br ;
R⁴ est choisi parmi H, alkyle, aryle, hétéroaryle, -(CH₂)_{c}-R¹² et 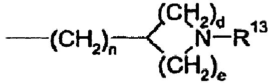
R⁷ est choisi parmi H, alkyle, aryle, hétéroaryle et -(CH₂)_{g}-R¹⁴ ;
R⁸, R⁹ et R¹⁰ sont choisis indépendamment parmi H, alkyle, alcoxy, F, Cl, Br, CN, NH₂, NO₂, NH (alkyle) et N(alkyle)₂ ; à condition que lorsque G est 3a et R⁴ est H, alkyle, aryle, hétéroaryle ou -(CH₂)_{c}-R¹², alors le cycle contenant A³, A⁴ et A⁵ soit substitué en au moins une position par alkyle, alcoxy, F, Cl, Br, CN, NH₂, NO₂, NH(alkyle) ou N(alkyle)₂ ; et
lorsque G est 4a, R⁴ est H, alkyle, aryle, hétéroaryle ou -(CH₂)_{c}-R¹² et A⁸ est NH, NCH₃ ou S, alors le cycle contenant A³, A⁴ et A⁵ soit substitué en au moins une position par alkyle, alcoxy, F, Cl, Br, CN, NH₂, NO₂, NH(alkyle) ou N(alkyle)₂ ;
R¹¹ et R¹² sont choisis indépendamment parmi H, alkyle, aryle, hétéroaryle, F, OH, O-alkyle, S-alkyle, O-acyle, NH₂, NH-alkyle, N(alkyle)₂, NH-acyle, N(alkyl)-acyle, CO₂H, CO₂-alkyle, CONH₂, CONH-alkyle, CON(alkyle)₂, CN et CF₃ ;
R¹³ est choisi parmi H, alkyle, aryle, hétéroaryle, -(CH₂)ₕ-R¹⁵ et Z-R¹⁶ ;
R¹⁴ et R¹⁵ sont choisis indépendamment parmi H, alkyle, alcényle, aryle, hétéroaryle, F, OH, O-alkyle, S-alkyle, O-acyle, NH₂, NH-alkyle, N(alkyle)₂, NH-acyle, N(alkyl)-acyle, CO₂H, CO₂-alkyle, CO-alkyle, CO-aryle, CONH₂, CONH-alkyle, CON(alkyle)₂, alcényl-CO₂-alkyle, alcényl-aryle, CN et CF₃ ;
R¹⁶ est choisi parmi H, alkyle, alcényle, aryle, hétéroaryle, O-aryle, -(CH₂)ᵢ-R¹⁷, cyclopropyl-aryle et O-(CH₂)ᵢ-R¹⁷ ;
R¹⁷ est choisi parmi H, alkyle, aryle, hétéroaryle, F, OH, O-alkyle, S-alkyle, O-acyle, NH₂, NH-alkyle, N(alkyle)₂, NH-acyle, N(alkyl)-acyle, CO₂H, CO-alkyle, CONH₂, CONH-alkyle, CON(alkyle)₂, CN et CF₃ ;
W est choisi parmi 0 et NH ;
X est choisi parmi (CH₂)ₘ, C(=O) et S(=O)ⱼ Y est choisi parmi 0, S, NH et N-alkyle ;
Z est choisi parmi -C(=O), -C(=O)-O et -S(=O)ₖ ;
les groupes alkyle étant choisis parmi des résidus hydrocarbonés saturés notamment des groupes linéaires ayant jusqu' à 10 atomes (en C₁-C₁₀), des groupes ramifiés ayant entre 3 et 10 atomes (en C₃-C₁₀), des groupes cycliques ayant entre 3 et 8 atomes (en C₃-C₈) et des combinaisons de groupes linéaires, ramifiés et cycliques,
a est choisi parmi 1 et 2 ;
b et c sont choisis indépendamment parmi 0, 1, 2 et 3 ;
d et e sont choisis indépendamment parmi 1 et 2 ;
g, h et i sont choisis indépendamment parmi 1, 2 et 3 ;
j et k sont choisis indépendamment parmi 1 et 2 ; et
m et n sont choisis indépendamment parmi 0, 1 et 2 ; et
au moins l'un de R¹, R² et R³ étant autre que hydrogène et
le composé selon la formule générale 1a n'étant pas la (4-aminométhyl-3-méthylphényl)-(5*H*, 11*H-*benzo[*e*]pyrrolo[1,2-*a*][1,4]diazépin-10-yl)méthanone

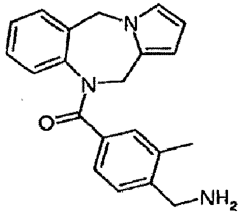

2. Composé selon la revendication 1, **caractérisé en ce que** G est choisi parmi :

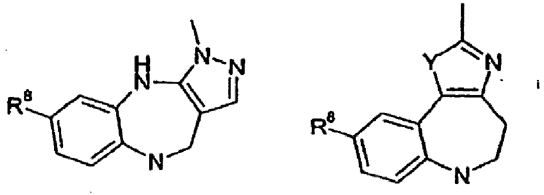

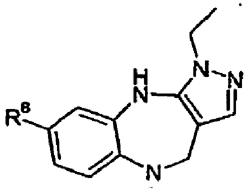

3. Composé selon la revendication 1, **caractérisé en ce que** G est choisi parmi :

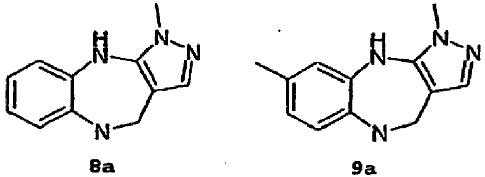

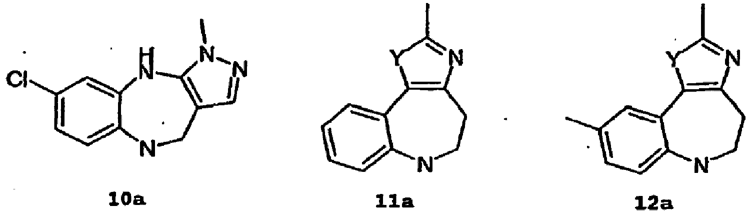

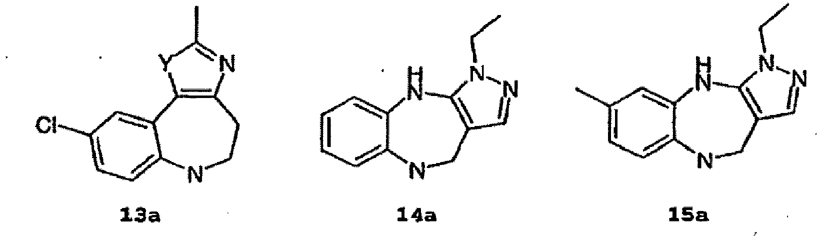

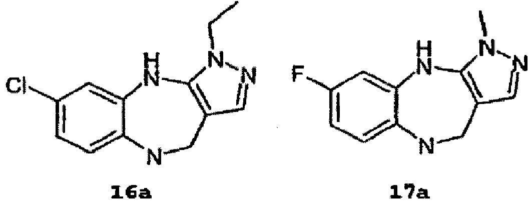

4. Composé selon la revendication 1, **caractérisé en ce que** l'un de R¹, R² et R³ est choisi parmi méthyle, chlore et fluor et les autres sont hydrogène.

5. Composé selon la revendication 1, **caractérisé en ce que** W est NH.

6. Composé selon la revendication 1, **caractérisé en ce que** R⁴ est alkyle.

7. Composé selon la revendication 1, **caractérisé en ce que** d est 2 et e est 2.

8. Composé selon la revendication 1, **caractérisé en ce que** R¹³ est alkyle.

9. Composé selon la revendication 1, **caractérisé en ce que** n est 0.

10. Composé selon la revendication 1, **caractérisé en ce que** R² est H, R⁴ est une pipéridine quand d est 2 et e est 2, W est NH, X est C(=0) et n est 0, comme représenté dans la formule 18a : 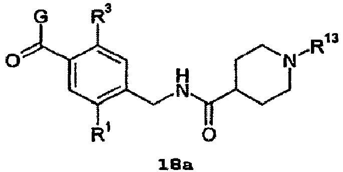 dans laquelle R¹ est choisi parmi méthyle, chlore et fluor et R³ est H ; ou R¹ est H et R³ est choisi parmi méthyle, chlore et fluor, et R¹³ est alkyle.

11. Composé selon la revendication 1, **caractérisé en ce que** X est C(=0), n est 0 et R⁴ est une pipéridine quand d est 2 et e est 2, comme représenté dans la formule 19a : 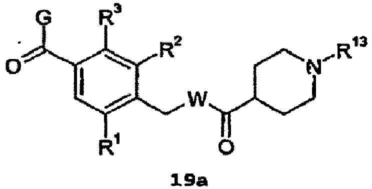 dans laquelle G est choisi parmi les formules générales 8a à 17a et R¹³ est alkyle.

12. Composé selon la revendication 1, **caractérisé en ce que** R² est H et W est NH comme représenté dans la formule 20a : 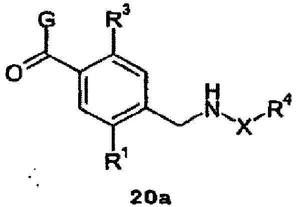 dans laquelle soit R¹ est choisi parmi méthyle, chlore et fluor et R³ est H ; soit R¹ est H et R³ est choisi parmi méthyle, chlore et fluor ; et G est choisi parmi les formules générales 8a à 17a.

13. Composé selon la revendication 1, **caractérisé en ce que** R² est H, W est NH et X est C(=0) comme représenté dans la formule 21a : 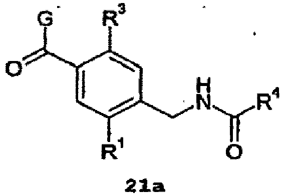 dans laquelle R¹ est choisi parmi méthyle, chlore et fluor et R³ est H ; ou R¹ est H et R³ est choisi parmi méthyle, chlore et fluor ; et R⁴ est alkyle.

14. Composé selon la revendication 1, **caractérisé en ce que** X est C(=O) comme représenté dans la formule 22a : 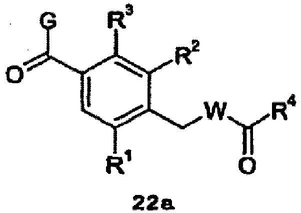 dans laquelle G est choisi parmi les formules générales 8a à 17a et R⁴ est alkyle.

15. Composé selon la revendication 1, **caractérisé en ce que** R² et R³ sont tous deux H, W est NH et X est C(=O) comme représenté dans la formule 23a : 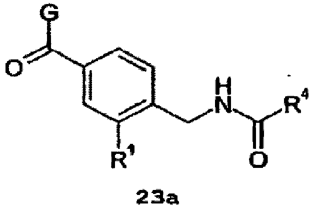 dans laquelle G est choisi parmi les formules générales 9a, 10a, 15a, 16a et 17a, R¹ est choisi parmi méthyle, chlore et fluor et R⁴ est alkyle.

16. Composé selon la revendication 1, **caractérisé en ce que** R² et R³ sont tous deux H, R⁴ est une pipéridine quand d est 2 et e est 2, W est NH, X est C(=O) et n est 0 comme représenté dans la formule 24a : 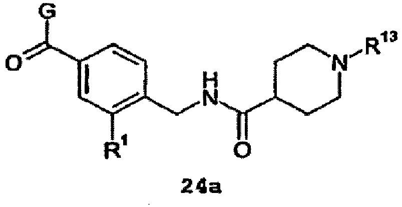 dans laquelle G est choisi parmi les formules générales 9a, 10a, 15a, 16a et 17a, R¹ est choisi parmi méthyle, chlore et fluor et R¹³ est alkyle.

17. Composé selon la revendication 1, choisi dans le groupe constitué par :
le 2-chloro-4-(3,6-diméthyl-4,10-dihydro-3*H*-2,3,4,9-tétraazabenzo[*f*]azulène-9-carbonyl)benzylamide d'acide 1-(3,3-diméthylbutyl)pipéridine-4-carboxylique ;
le 2-chloro-4-(3-méthyl-4,10-dihydro-3*H*-2,3,4,9-tétraazabenzo[*f*]azulène-9-carbonyl)benzylamide d'acide 1-(3,3-diméthylbutyl)pipéridine-4-carboxylique ;
le 2-chloro-4-(6-chloro-3-méthyl-4,10-dihydro-3*H-*2,3,4,9-tétraazabenzo[*f*]azulène-9-carbonyl)benzylamide d'acide 1-(3,3-diméthylbutyl)pipéridine-4-carboxylique ;
le 2-fluoro-4-(3-méthyl-4,10-dihydro-3*H*-2,3,4,9-tétraazabenzo[*f*]azulène-9-carbonyl)benzylamide d'acide 1-(3,3-diméthylbutyl)pipéridine-4-carboxylique ;
le 2-méthyl-4-(2-méthyl-4,5-dihydro-1*H*-1,3,6-triazabenzo[e]azulène-6-carbonyl)benzylamide d'acide 1-(3,3-diméthylbutyl)pipéridine-4-carboxylique ;
le 2-méthyl-4-(2-méthyl-4,5-dihydro-1-oxa-3,6-diazabenzo[*e*]azulène-6-carbonyl)benzylamide d'acide 1-(3,3-diméthylbutyl)pipéridine-4-carboxylique ;
le 2-méthyl-4-(3-méthyl-4,10-dihydro-3*H*-2,3,4,9-tétraazabenzo[*f*]azulène-9-carbonyl)benzylamide d'acide 1-(3,3-diméthylbutyl)pipéridine-4-carboxylique ;
le 3-chloro-4-(3,6-diméthyl-4,10-dihydro-3*H*-2,3,4,9-tétraazabenzo[*f*]azulène-9-carbonyl)benzylamide d'acide 1-(3,3-diméthylbutyl)pipéridine-4-carboxylique ;
le 3-chloro-4-(3-méthyl-4,10-dihydro-3*H*-2,3,4,9-tétraazabenzo[*f*]azulène-9-carbonyl)benzylamide d'acide 1-(3,3-diméthylbutyl)pipéridine-4-carboxylique ;
le 3-chloro-4-(6-chloro-3-méthyl-4,10-dihydro-3*H-*2,3,4,9-tétraazabenzo[*f*]azulène-9-carbonyl)benzylamide d'acide 1-(3,3-diméthylbutyl)pipéridine-4-carboxylique ;
le 3-fluoro-4-(3-méthyl-4,10-dihydro-3*H*-2,3,4,9-tétraazabenzo[*f*]azulène-9-carbonyl)benzylamide d'acide 1-(3,3-diméthylbutyl)pipéridine-4-carboxylique ;
le 3-méthyl-4-(3-méthyl-4,10-dihydro-3*H*-2,3,4,9-tétraazabenzo[*f*]azulène-9-carbonyl)benzylamide d'acide 1-(3,3-diméthylbutyl)pipéridine-4-carboxylique ;
le 4-(3,6-diméthyl-4,10-dihydro-3*H*-2,3,4,9-tétraazabenzo[*f*]azulène-9-carbonyl)-2-méthylbenzylamide d'acide 1-(3,3-diméthylbutyl)pipéridine-4-carboxylique ;
le 4-(3,6-diméthyl-4,10-dihydro-3*H*-2,3,4,9-tétraazabenzo[*f*]azulène-9-carbonyl)-3-méthylbenzylamide d'acide 1-(3,3-diméthylbutyl)pipéridine-4-carboxylique ;
le 4-(3,6-diméthyl-4,10-dihydro-3*H*-2,3,4,9-tétraazabenzo[*f*]azulène-9-carbonyl)-2-fluorobenzylamide d'acide 1-(3,3-diméthylbutyl)pipéridine-4-carboxylique ;
le 4-(3,6-diméthyl-4,10-dihydro-3H-2,3,4,9-tétraazabenzo[*f*]azulène-9-carbonyl)-3-fluorobenzylamide d'acide 1-(3,3-diméthylbutyl)pipéridine-4-carboxylique ;
le 4-(6-chloro-3-méthyl-4,10-dihydro-3*H*-2,3,4,9-tétraazabenzo[*f*]azulène-9-carbonyl)-2-méthylbenzylamide d'acide 1-(3,3-diméthylbutyl)pipéridine-4-carboxylique ;
le 4-(6-chloro-3-méthyl-4,10-dihydro-3*H*-2,3,4,9-tétraazabenzo[*f*]azulène-9-carbonyl)-2-fluorobenzylamide d'acide 1-(3,3-diméthylbutyl)pipéridine-4-carboxylique ;
le 4-(6-chloro-3-méthyl-4,10-dihydro-3*H*-2,3,4,9-tétraazabenzo[*f*]azulène-9-carbonyl)-3-méthylbenzylamide d'acide 1-(3,3-diméthylbutyl)pipéridine-4-carboxylique ;
le 4-(6-chloro-3-méthyl-4,10-dihydro-3*H*-2,3,4,9-tétraazabenzo[*f*]azulène-9-carbonyl)-3-fluorobenzylamide d'acide 1-(3,3-diméthylbutyl)pipéridine-4-carboxylique ;
le 4-[(4-chlorophényl)méthylcarbamoyl]-2-méthylbenzylamide d'acide 1-(3,3-diméthylbutyl)pipéridine-4-carboxylique ;
le 2-chloro-4-(3,6-diméthyl-4,10-dihydro-3*H*-2,3,4,9-tétraazabenzo[*f*]azulène-9-carbonyl)benzylamide d'acide 1-cyclopropylméthylpipéridine-4-carboxylique ;
le 2-chloro-4-(3-méthyl-4,10-dihydro-3*H*-2,3,4,9-tétraazabenzo[*f*]azulène-9-carbonyl)benzylamide d'acide 1-cyclopropylméthylpipéridine-4-carboxylique ;
le 2-chloro-4-(6-chloro-3-méthyl-4,10-dihydro-3*H-*2,3,4,9-tétraazabenzo[*f*]azulène-9-carbonyl)benzylamide d'acide 1-cyclopropylméthylpipéridine-4-carboxylique ;
le 2-fluoro-4-(3-méthyl-4,10-dihydro-3*H*-2,3,4,9-tétraazabenzo[*f*]azulène-9-carbonyl)benzylamide d'acide 1-cyclopropylméthylpipéridine-4-carboxylique ;
le 2-méthyl-4-(3-méthyl-4,10-dihydro-3*H*-2,3,4,9-tétraazabenzo[*f*]azulène-9-carbonyl)benzylamide d'acide 1-cyclopropylméthylpipéridine-4-carboxylique ;
le 3-chloro-4-(3,6-diméthyl-4,10-dihydro-3*H*-2,3,4,9-tétraazabenzo[*f*]azulène-9-carbonyl)benzylamide d'acide 1-cyclopropylméthylpipéridine-4-carboxylique ;
le 3-chloro-4-(3-méthyl-4,10-dihydro-3*H*-2,3,4,9-tétraazabenzo[*f*]azulène-9-carbonyl)benzylamide d'acide 1-cyclopropylméthylpipéridine-4-carboxylique ;
le 3-chloro-4-(6-chloro-3-méthyl-4,10-dihydro-3*H-*2,3,4,9-tétraazabenzo[*f*]azulène-9-carbonyl)benzylamide d'acide 1-cyclopropylméthylpipéridine-4-carboxylique ;
le 3-fluoro-4-(3-méthyl-4,10-dihydro-3*H*-2,3,4,9-tétraazabenzo[*f*]azulène-9-carbonyl)benzylamide d'acide 1-cyclopropylméthylpipéridine-4-carboxylique ;
le 3-méthyl-4-(3-méthyl-4,10-dihydro-3*H*-2,3,4,9-tétraazabenzo[*f*]azulène-9-carbonyl)benzylamide d'acide 1-cyclopropylméthylpipéridine-4-carboxylique ;
le 4-(3,6-diméthyl-4,10-dihydro-3*H*-2,3,4,9-tétraazabenzo[*f*]azulène-9-carbonyl)-2-méthylbenzylamide d'acide 1-cyclopropylméthylpipéridine-4-carboxylique ;
le 4-(3,6-diméthyl-4,10-dihydro-3*H*-2,3,4,9-tétraazabenzo[*f*]azulène-9-carbonyl)-3-méthylbenzylamide d'acide 1-cyclopropylméthylpipéridine-4-carboxylique ;
le 4-(3,6-diméthyl-4,10-dihydro-3*H*-2,3,4,9-tétraazabenzo[*f*]azulène-9-carbonyl)-2-fluorobenzylamide d'acide 1-cyclopropylméthylpipéridine-4-carboxylique ;
le 4-(3,6-diméthyl-4,10-dihydro-3*H*-2,3,4,9-tétraazabenzo[*f*]azulène-9-carbonyl)-3-fluorobenzylamide d'acide 1-cyclopropylméthylpipéridine-4-carboxylique ;
le 4-(6-chloro-3-méthyl-4,10-dihydro-3*H*-2,3,4,9-tétraazabenzo[*f*]azulène-9-carbonyl)-2-fluorobenzylamide d'acide 1-cyclopropylméthylpipéridine-4-carboxylique ;
le 4-(6-chloro-3-méthyl-4,10-dihydro-3*H*-2,3,4,9-tétraazabenzo[*f*]azulène-9-carbonyl)-2-méthylbenzylamide d'acide 1-cyclopropylméthylpipéridine-4-carboxylique ;
le 4-(6-chloro-3-méthyl-4,10-dihydro-3*H*-2,3,4,9-tétraazabenzo[*f*]azulène-9-carbonyl)-3-méthylbenzylamide d'acide 1-cyclopropylméthylpipéridine-4-carboxylique ;
le 4-(6-chloro-3-méthyl-4,10-dihydro-3*H*-2,3,4,9-tétraazabenzo[*f*]azulène-9-carbonyl)-3-fluorobenzylamide d'acide 1-cyclopropylméthylpipéridine-4-carboxylique ;
le 4-[(4-chlorophényl)méthylcarbamoyl]-2-méthylbenzylamide d'acide 1-cyclopropylméthylpipéridine-4-carboxylique ;
le 2-chloro-4-(6-chloro-3-méthyl-4,10-dihydro-3*H-*2,3,4,9-tétraazabenzo[*f*]azulène-9-carbonyl)benzylamide d'acide cyclobutanecarboxylique ;
le 4-(3,6-diméthyl-4,10-dihydro-3*H*-2,3,4,9-tétraazabenzo[*f*]azulène-9-carbonyl)-2-fluorobenzylamide d'acide cyclobutanecarboxylique ;
le 4-(6-chloro-3-méthyl-4,10-dihydro-3*H*-2,3,4,9-tétraazabenzo[*f*]azulène-9-carbonyl)-2-fluorobenzylamide d'acide cyclobutanecarboxylique ;
le 2-chloro-4-(6-chloro-3-méthyl-4,10-dihydro-3*H-*2,3,4,9-tétraazabenzo[*f*]azulène-9-carbonyl)benzylamide d'acide cyclopentanecarboxylique ;
le 4-(3,6-diméthyl-4,10-dihydro-3*H*-2,3,4,9-tétraazabenzo[*f*]azulène-9-carbonyl)-2-fluorobenzylamide d'acide cyclopentanecarboxylique ;
le 4-(6-chloro-3-méthyl-4,10-dihydro-3*H*-2,3,4,9-tétraazabenzo[*f*]azulène-9-carbonyl)-2-fluorobenzylamide d'acide cyclopentanecarboxylique ;
le 2-chloro-4-(3,6-diméthyl-4,10-dihydro-3*H*-2,3,4,9-tétraazabenzo[*f*]azulène-9-carbonyl)benzylamide d'acide cyclopropanecarboxylique ;
le 2-chloro-4-(6-chloro-3-méthyl-4,10-dihydro-3*H-*2,3,4,9-tétraazabenzo[*f*]azulène-9-carbonyl)benzylamide d'acide cyclopropanecarboxylique ;
le 3-chloro-4-(3,6-diméthyl-4,10-dihydro-3*H*-2,3,4,9-tétraazabenzo[*f*]azulène-9-carbonyl)benzylamide d'acide cyclopropanecarboxylique ;
le 3-chloro-4-(6-chloro-3-méthyl-4,10-dihydro-3*H-*2,3,4,9-tétraazabenzo[*f*]azulène-9-carbonyl)benzylamide d'acide cyclopropanecarboxylique ;
le 4-(3,6-diméthyl-4,10-dihydro-3*H*-2,3,4,9-tétraazabenzo[*f*]azulène-9-carbonyl)-2-méthylbenzylamide d'acide cyclopropanecarboxylique ;
le 4-(3,6-diméthyl-4,10-dihydro-3*H*-2,3,4,9-tétraazabenzo[*f*]azulène-9-carbonyl)-2-fluorobenzylamide d'acide cyclopropanecarboxylique ;
le 4-(3,6-diméthyl-4,10-dihydro-3*H*-2,3,4,9-tétraazabenzo[*f*]azulène-9-carbonyl)-3-fluorobenzylamide d'acide cyclopropanecarboxylique ;
le 4-(3,6-diméthyl-4,10-dihydro-3*H*-2,3,4,9-tétraazabenzo[*f*]azulène-9-carbonyl)-3-méthylbenzylamide d'acide cyclopropanecarboxylique ;
le 4-(6-chloro-3-méthyl-4,10-dihydro-3*H*-2,3,4,9-tétraazabenzo[*f*]azulène-9-carbonyl)-2-méthylbenzylamide d'acide cyclopropanecarboxylique ;
le 4-(6-chloro-3-méthyl-4,10-dihydro-3*H*-2,3,4,9-tétraazabenzo[*f*]azulène-9-carbonyl)-3-fluorobenzylamide d'acide cyclopropanecarboxylique ;
le 4-(6-chloro-3-méthyl-4,10-dihydro-3*H*-2,3,4,9-tétraazabenzo[*f*]azulène-9-carbonyl)-2-fluorobenzylamide d'acide cyclopropanecarboxylique ;
le 4-(6-chloro-3-méthyl-4,10-dihydro-3*H*-2,3,4,9-tétraazabenzo[*f*]azulène-9-carbonyl)-3-méthylbenzylamide d'acide cyclopropanecarboxylique ;
le *N*-[2-chloro-4-(6-chloro-3-méthyl-4,10-dihydro-3*H-*2,3,4,9-tétraazabenzo[*f*]azulène-9-carbonyl)benzyl]propionamide ;
le *N*-[2-chloro-4-(6-chloro-3-méthyl-4,10-dihydro-3*H-*2,3,4,9-tétraazabenzo[*f*]azulène-9-carbonyl)benzyl]-2,2-diméthylpropionamide ;
le *N*-[2-chloro-4-(6-chloro-3-méthyl-4,10-dihydro-3*H-*2,3,4,9-tétraazabenzo[*f*]azulène-9-carbonyl)benzyl]isobutyramide ;
le *N*-[2-chloro-4-(6-chloro-3-méthyl-4,10-dihydro-3*H-*2,3,4,9-tétraazabenzo[*f*]azulène-9-carbonyl)benzyl]butyramide ;
le *N*-[4-(3,6-diméthyl-4,10-dihydro-3*H*-2,3,4,9-tétraazabenzo[*f*]azulène-9-carbonyl)-2-fluorobenzyl]propionamide ;
le *N*-[4-(3,6-diméthyl-4,10-dihydro-3*H*-2,3,4,9-tétraazabenzo[*f*]azulène-9-carbonyl)-2-fluorobenzyl]-2,2-diméthylpropionamide ;
le *N*-[4-(3,6-diméthyl-4,10-dihydro-3*H*-2,3,4,9-tétraazabenzo[*f*]azulène-9-carbonyl)-2-fluorobenzyl]isobutyramide ;
le *N*-[4-(3,6-diméthyl-4,10-dihydro-3*H*-2,3,4,9-tétraazabenzo[*f*]azulène-9-carbonyl)-2-fluorobenzyl]butyramide ;
le *N*-[4-(6-chloro-3-méthyl-4,10-dihydro-3*H*-2,3,4,9-tétraazabenzo[*f*]azulène-9-carbonyl)-2-fluorobenzyl]propionamide ;
le *N*-[4-(6-chloro-3-méthyl-4,10-dihydro-3*H*-2,3,4,9-tétraazabenzo[*f*]azulène-9-carbonyl)-2-fluorobenzyl]-2,2-diméthylpropionamide ;
le *N*-[4-(6-chloro-3-méthyl-4,10-dihydro-3*H*-2,3,4,9-tétraazabenzo[*f*]azulène-9-carbonyl)-2-fluorobenzyl]isobutyramide ;
le *N*-[4-(6-chloro-3-méthyl-4,10-dihydro-3*H*-2,3,4,9-tétraazabenzo[*f*]azulène-9-carbonyl)-2-fluorobenzyl]acétamide ;
le *N*-[4-(6-chloro-3-méthyl-4,10-dihydro-3*H*-2,3,4,9-tétraazabenzo[*f*]azulène-9-carbonyl)-2-fluorobenzyl]butyramide ;
le *N*-[4-(6-chloro-3-méthyl-4,10-dihydro-3*H*-2,3,4,9-tétraazabenzo[*f*]azulène-9-carbonyl)-2-fluorobenzyl]formamide ;
le *N*-[4-(6-chloro-3-éthyl-4,10-dihydro-3H-2,3,4,9-tétraazabenzo[*f*]azulène-9-carbonyl)-2-fluorobenzyl]propionamide ;
le 4-(6-chloro-3-éthyl-4,10-dihydro-3*H*-2,3,4,9-tétraazabenzo[*f*]azulène-9-carbonyl)-2-fluorobenzylamide d'acide cyclopropanecarboxylique ;
le *N*-[2-fluoro-4-(6-fluoro-3-méthyl-4,10-dihydro-3*H-*2,3,4,9-tétraazabenzo[*f*]azulène-9-carbonyl)benzyl]propionamide ;
le *N*-[2-fluoro-4-(6-fluoro-3-méthyl-4,10-dihydro-3*H-*2,3,4,9-tétraazabenzo[*f*]azulène-9-carbonyl)benzyl]isobutyramide ;
le 2-fluoro-4-(6-fluoro-3-méthyl-4,10-dihydro-3*H-*2,3,4,9-tétraazabenzo[*f*]azulène-9-carbonyl)benzylamide d'acide cyclopropanecarboxylique ; et
le *N*-[4-(9-chloro-2-méthyl-4,5-dihydro-1*H*-1,3,6-triazabenzo[*e*]azulène-6-carbonyl)-2-méthylbenzyl]isobutyramide.

18. Composé selon la revendication 1, qui est le 4-(3,6-diméthyl-4,10-dihydro-3*H*-2,3,4,9-tétraazabenzo[*f*]azulène-9-carbonyl)-2-fluorobenzylamide d'acide 1-(3,3-diméthylbutyl)pipéridine-4-carboxylique ou un tautomère ou un sel pharmaceutiquement acceptable de celui-ci.

19. Composé selon la revendication 1, qui est le 4-(3,6-diméthyl-4,10-dihydro-3*H*-2,3,4,9-tétraazabenzo[*f*]azulène-9-carbonyl)-2-méthylbenzylamide d'acide 1-(3,3-diméthylbutyl)pipéridine-4-carboxylique ou un tautomère ou un sel pharmaceutiquement acceptable de celui-ci.

20. Composé selon la revendication 1, qui est le 4-(6-chloro-3-méthyl-4,10-dihydro-3*H*-2,3,4,9-tétraazabenzo[*f*]azulène-9-carbonyl)-2-fluorobenzylamide d'acide cyclopropanecarboxylique ou un tautomère ou un sel pharmaceutiquement acceptable de celui-ci.

21. Composé selon la revendication 1, qui est le *N*-[2-chloro-4-(6-chloro-3-méthyl-4,10-dihydro-3*H*-2,3,4,9-tétraazabenzo[*f*]azulène-9-carbonyl)benzyl]butyramide ou un sel pharmaceutiquement acceptable de celui-ci.

22. Composé selon la revendication 1, qui est le 4-(6-chloro-3-méthyl-4,10-dihydro-3*H*-2,3,4,9-tétraazabenzo[*f*]azulène-9-carbonyl)-2-méthylbenzylamide d'acide cyclopropanecarboxylique ou un sel pharmaceutiquement acceptable de celui-ci.

23. Composition pharmaceutique comprenant un composé selon l'une quelconque des revendications précédentes.

24. Composition pharmaceutique selon la revendication 23, formulée pour l'administration par voie orale, de préférence sous forme de comprimé, de capsule ou de sachet.

25. Composition pharmaceutique selon la revendication 23 ou 24, destinée au traitement d'une affection choisie parmi la dysménorrhée, le travail préterme, l'hypertension, la maladie de Raynaud, l'oedème du cerveau, le mal du transport, l'hyperlipémie, le cancer du poumon à petites cellules, la dépression, l'anxiété, l'hyponatrémie, la cirrhose du foie et l'insuffisance cardiaque congestive.

26. Utilisation d'un composé de formule 1b 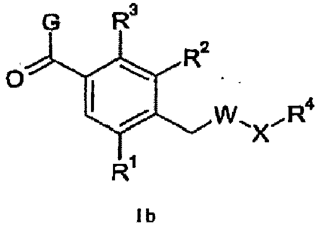 dans laquelle G est un groupe choisi parmi les formules générales 2a, 3a, 4a, 5a et 6a, 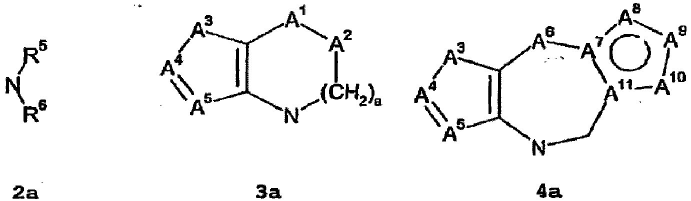 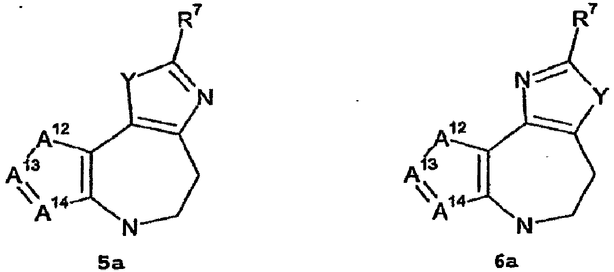 dans lesquelles:
A¹ est choisi parmi CH₂, CH(OH), NH, N-alkyle, 0 et S ;
A² est choisi parmi CH₂, CH(OH), C(=O) et NH ;
A³ et A¹² sont choisis indépendamment parmi S, NH, N-alkyle, -C(R⁸)=CH-, -C(R⁸)=N-, -N=C(R⁸)- et -CH=C(R⁸)- ;
A⁴ et A¹³ sont choisis indépendamment parmi C(R⁹) et N ;
A⁵ et A¹⁴ sont choisis indépendamment parmi C(R¹⁰) et N ;
A⁶ est choisi parmi CH₂, NH, N-alkyle et 0 ;
A⁷ et A¹¹ sont choisis indépendamment parmi C et N ;
A⁸ et A⁹ sont choisis indépendamment parmi CH, N, NH, N(CH₂)_{b}R¹¹ et S ;
A¹⁰ est choisi parmi -CH=CH-, CH, N, NH, N-(CH₂)_{b}-R¹¹ et S ;
le cycle constitué par A⁷, A⁸, A⁹, A¹⁰ et A¹¹ étant aromatique ;
R¹, R² et R³ sont choisis indépendamment parmi H, alkyle, O-alkyle, NO₂, F, Cl et Br ;
R⁴ est choisi parmi H, alkyle, aryle, hétéroaryle, -(CH₂)_{c}-R¹² et 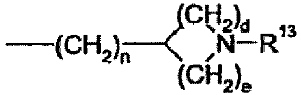
R¹ et R⁶ sont choisis indépendamment parmi alkyle, aryle, - (CH₂)_{f}-aryle et -(CH₂)_{f}-hétéroaryle ;
R⁷ est choisi parmi H, alkyle, aryle, hétéroaryle et -(CH₂)_{g}-R¹⁴ ;
R⁸, R⁹ et R¹⁰ sont choisis indépendamment parmi H, alkyle, alcoxy, F, Cl, Br, CN, NH₂, NO₂, NH (alkyle), N(alkyle)₂ ; à condition que lorsque G est 3a et R⁴ est H, alkyle, aryle, hétéroaryle ou -(CH₂)_{c}-R¹², alors le cycle contenant A³, A⁴ et A⁵ soit substitué en au moins une position par alkyle, alcoxy, F, Cl, Br, CN, NH₂, NO₂, NH(alkyle) ou N(alkyle)₂ ; et
lorsque G est 4a, R⁴ est H, alkyle, aryle, hétéroaryle ou -(CH₂)_{c}-R¹² et A⁸ est NH, NCH₃ ou S, alors le cycle contenant A³, A⁴ et A⁵ soit substitué en au moins une position par alkyle, alcoxy, F, Cl, Br, CN, NH₂, NO₂, NH(alkyle) ou N(alkyle)₂ ;
R¹¹ et R¹² sont choisis indépendamment parmi H, alkyle, aryle, hétéroaryle, F, OH, O-alkyle, S-alkyle, O-acyle, NH₂, NH-alkyle, N(alkyle)₂, NH-acyle, N(alkyl)-acyle, CO₂H, CO₂-alkyle, CONH₂, CONH-alkyle, CON(alkyle)₂, CN et CF₃ ;
R¹³ est choisi parmi H, alkyle, aryle, hétéroaryle, -(CH₂)ₕ-R¹⁵ et Z-R¹⁶ ;
R¹⁴ et R¹⁵ sont choisis indépendamment parmi H, alkyle, alcényle, aryle, hétéroaryle, F, OH, O-alkyle, S-alkyle, O-acyle, NH₂, NH-alkyle, N(alkyle)₂, NH-acyle, N(alkyl)-acyle, CO₂H, CO₂-alkyle, CO-alkyle, CO-aryle, CONH₂, CONH-alkyle, CON(alkyle)₂, alcényl-CO₂-alkyle, alcényl-aryle, CN et CF₃ ;
R¹⁶ est choisi parmi H, alkyle, alcényle, aryle, hétéroaryle, O-aryle, -(CH₂)ᵢ-R¹⁷, cyclopropyl-aryle et O-(CH₂)ᵢ-R¹⁷ ;
R¹⁷ est choisi parmi H, alkyle, aryle, hétéroaryle, F, OH, O-alkyle, S-alkyle, O-acyle, NH₂, NH-alkyle, N(alkyle)₂, NH-acyle, N(alkyl)-acyle, CO₂H, CO₂-alkyle, CONH₂, CONH-alkyle, CON(alkyle)₂, CN et CF₃ ;
W est choisi parmi 0 et NH ;
X est choisi parmi (CH₂)ₘ, C(=O) et S(=O)ⱼ
Y est choisi parmi 0, S, NH et N-alkyle ;
Z est choisi parmi -C(=O), -C(=O)-O et -S(=O)ₖ ;
a est choisi parmi 1 et 2 ;
b et c sont choisis indépendamment parmi 0, 1, 2 et 3 ;
d, e et f sont choisis indépendamment parmi 1 et 2 ;
g, h et i sont choisis indépendamment parmi 1, 2 et 3 ;
j et k sont choisis indépendamment parmi 1 et 2 ; et
m et n sont choisis indépendamment parmi 0, 1 et 2,
ou d'un composé qui est un tautomère ou un sel pharmaceutiquement acceptable de celui-ci, pour la fabrication d'un médicament destiné au traitement d'une affection choisie parmi la dysménorrhée, le travail préterme, l'hypertension, la maladie de Raynaud, l'oedème du cerveau, le mal du transport, l'hyperlipémie, le cancer du poumon à petites cellules, la dépression, l'anxiété, l'hyponatrémie, la cirrhose du foie et l'insuffisance cardiaque congestive.

27. Utilisation selon la revendication 26, **caractérisée en ce que** ledit composé est le 4-(3,6-diméthyl-4,10-dihydro-3*H*-2,3,4,9-tétraazabenzo[*f*]azulène-9-carbonyl)-2-fluorobenzylamide d'acide 1-(3,3-diméthylbutyl)pipéridine-4-carboxylique ou un tautomère ou un sel pharmaceutiquement acceptable de celui-ci.

28. Utilisation selon la revendication 26, **caractérisée en ce que** ledit composé est le 4-(3,6-diméthyl-4,10-dihydro-3*H*-2,3,4,9-tétraazabenzo[*f*]azulène-9-carbonyl)-2-méthylbenzylamide d'acide 1-(3,3-diméthylbutyl)pipéridine-4-carboxylique ou un tautomère ou un sel pharmaceutiquement acceptable de celui-ci.

29. Utilisation selon la revendication 26, **caractérisée en ce que** ledit composé est le 4-(6-chloro-3-méthyl-4,10-dihydro-3*H*-2,3,4,9-tétraazabenzo[*f*]azulène-9-carbonyl)-2-fluorobenzylamide d'acide cyclopropanecarboxylique ou un tautomère ou un sel pharmaceutiquement acceptable de celui-ci.

30. Utilisation selon la revendication 26, **caractérisée en ce que** ledit composé est le *N*-[2-chloro-4-(6-chloro-3-méthyl-4,10-dihydro-3*H*-2,3,4,9-tétraazabenzo[*f*]azulène-9-carbonyl)benzyl]butyramide ou un sel pharmaceutiquement acceptable de celui-ci.

31. Utilisation selon la revendication 26, **caractérisée en ce que** ledit composé est le 4-(6-chloro-3-méthyl-4,10-dihydro-3*H*-2,3,4,9-tétraazabenzo[*f*]azulène-9-carbonyl)-2-méthylbenzylamide d'acide cyclopropanecarboxylique ou un sel pharmaceutiquement acceptable de celui-ci.

32. Composé de formule 1b, tel que défini dans la revendication 26, destiné à être utilisé dans le traitement d'une affection choisie parmi la dysménorrhée, le travail préterme, l'hypertension, la maladie de Raynaud, l'oedème du cerveau, le mal du transport, l'hyperlipémie, le cancer du poumon à petites cellules, la dépression, l'anxiété, l'hyponatrémie, la cirrhose du foie et l'insuffisance cardiaque congestive.

33. Composé destiné à être utilisé selon la revendication 32, ledit composé étant le 4-(3,6-diméthyl-4,10-dihydro-3*H*-2,3,4,9-tétraazabenzo[*f*]azulène-9-carbonyl)-2-fluorobenzylamide d'acide 1-(3,3-diméthylbutyl)pipéridine-4-carboxylique ou un tautomère ou un sel pharmaceutiquement acceptable de celui-ci.

34. Composé destiné à être utilisé selon la revendication 32, ledit composé étant le 4-(3,6-diméthyl-4,10-dihydro-3*H*-2,3,4,9-tétraazabenzo[*f*]azulène-9-carbonyl)-2-méthylbenzylamide d'acide 1-(3,3-diméthylbutyl)pipéridine-4-carboxylique ou un tautomère ou un sel pharmaceutiquement acceptable de celui-ci.

35. Composé destiné à être utilisé selon la revendication 32, ledit composé étant le 4-(6-chloro-3-méthyl-4,10-dihydro-3*H*-2,3,4,9-tétraazabenzo[*f*]azulène-9-carbonyl)-2-fluorobenzylamide d'acide cyclopropanecarboxylique ou un tautomère ou un sel pharmaceutiquement acceptable de celui-ci.

36. Composé destiné à être utilisé selon la revendication 32, ledit composé étant le *N*-[2-chloro-4-(6-chloro-3-méthyl-4,10-dihydro-3*H*-2,3,4,9-tétraazabenzo[*f*]azulène-9-carbonyl)benzyl]butyramide ou un sel pharmaceutiquement acceptable de celui-ci.

37. Composé destiné à être utilisé selon la revendication 32, ledit composé étant le 4-(6-chloro-3-méthyl-4,10-dihydro-3*H*-2,3,4,9-tétraazabenzo[*f*]azulène-9-carbonyl)-2-méthylbenzylamide d'acide cyclopropanecarboxylique ou un sel pharmaceutiquement acceptable de celui-ci.
